(19) Europäisches Patentamt
European Patent Office
Office européen des brevets

(11) EP 1 975 234 A2

(12) # EUROPEAN PATENT APPLICATION

(43) Date of publication:
**01.10.2008 Bulletin 2008/40**

(51) Int Cl.:
***C12N 15/12*** *(2006.01)* ***C07K 14/47*** *(2006.01)*

(21) Application number: **08006605.3**

(22) Date of filing: **31.10.1997**

(84) Designated Contracting States:
**AT BE CH DE DK ES FI FR GB GR IE IT LI LU MC NL PT SE**
Designated Extension States:
**AL LT LV RO SI**

(30) Priority: **01.11.1996 AU PO338496**
**14.02.1997 AU PO511797**

(62) Document number(s) of the earlier application(s) in accordance with Art. 76 EPC:
**97909070.1 / 0 948 522**

(71) Applicant: **THE WALTER AND ELIZA HALL INSTITUTE OF MEDICAL RESEARCH**
**Parkville, VIC 3052 (AU)**

(72) Inventors:
- **Hilton, Douglas J.**
  **Warrandyte, VIC 3133 (AU)**
- **Alexander, Warren S.**
  **Moonee Ponds, VIC 3039 (AU)**
- **Viney, Elizabeth M.**
  **Bundoora, VIC 3083 (AU)**
- **Willson, Tracy A.**
  **North Balwyn, VIC 3104 (AU)**
- **Richardson, Rachael T.**
  **East Brighton, VIC 3187 (AU)**
- **Starr, Robyn**
  **Carlton, VIC 3053 (AU)**
- **Nicholson, Sandra E.**
  **Newport, VIC 3015 (AU)**
- **Metcalf, Donald**
  **Balwyn, VIC 3103 (AU)**
- **Nicola, Nicos A.**
  **Mont Albert, VIC 3127 (AU)**

(74) Representative: **Dzieglewska, Hanna Eva**
**Frank B. Dehn & Co.**
**St Bride's House**
**10 Salisbury Square**
**London**
**EC4Y 8JD (GB)**

Remarks:
This application was filed on 31-03-2008 as a divisional application to the application mentioned under INID code 62.

(54) **Therapeutic and diagnotics agents capable of modulating cellular responsiveness to cytokines**

(57) The present invention relates generally to therapeutic and diagnostic agents. More particularly, the present invention provides therapeutic molecules capable of modulating signal transduction such as but not limited to cytokine-mediated signal transduction. The molecules of the present invention are useful, therefore, in modulating cellular responsiveness to cytokines as well as other mediators of signal transduction such as endogenous or exogenous molecules, antigens, microbes and microbial products, viruses or components thereof, ions, hormones and parasites.

EP 1 975 234 A2

SEQUENCE LISTING

(1) GENERAL INFORMATION:

(i) APPLICANT: THE WALTER AND ELIZA HALL INSTITUTE OF MEDICAL RESEARCH

(ii) TITLE OF INVENTION: THERAPEUTIC AND DIAGNOSTIC AGENTS

(iii) NUMBER OF SEQUENCES: 49

(iv) CORRESPONDENCE ADDRESS:
(A) ADDRESSEE: FRANK B. DEHN & CO.
(B) STREET: ST. BRIDE'S HOUSE, 10 SALISBURY SQUARE
(C) CITY: LONDON
(D) STATE: LONDON
(E) COUNTRY: UNITED KINGDOM
(F) ZIP: EC4Y 8JD

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: PCT INTERNATIONAL
(B) FILING DATE: 31-OCT-1997

(vi) PRIOR APPLICATION DATA:
(A) APPLICATION NUMBER: PO5117
(B) FILING DATE: 14-FEB-1997

(vii) PRIOR APPLICATION DATA:
(A) APPLICATION NUMBER: PO 3384
(B) FILING DATE: 01-NOV-1996

(viii) ATTORNEY/AGENT INFORMATION:
(A) NAME: DZIEGLEWSKA DR, HANNA EVA
(C) REFERENCE/DOCKET NUMBER: 27.33.69072/25

(ix) TELECOMMUNICATION INFORMATION:
(A) TELEPHONE: +44 (0)20 7632 7200
(B) TELEFAX: +44 (0)20 7353 8895

(2) INFORMATION FOR SEQ ID NO:1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:1:

CACGCCGCCC ACGTGAAGGC 20

(2) INFORMATION FOR SEQ ID NO:2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:2:

```
TTCGCCAATG ACAAGACGCT                              20
```

(2) INFORMATION FOR SEQ ID NO:3:

(i) SEQUENCE CHARACTERISTICS:
(A) LENGTH: 1236 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 1..636

(xi) SEQUENCE DESCRIPTION: SEQ ID NO:3:

```
CGAGGCTCAA GCTCCGGGCG GATTCTGCGT GCCGCTCTCG CTCCTTGGGG TCTGTTGGCC    -101

GGCCTGTGCC ACCCGGACGC CCGGCTCACT GCCTCTGTCT CCCCCATCAG CGCAGCCCCG     -41

GACGCTATGG CCCACCCCTC CAGCTGGCCC CTCGAGTAGG                            -1


ATG GTA GCA CGC AAC CAG GTG GCA GCC GAC AAT GCG ATC TCC CCG GCA       48
Met Val Ala Arg Asn Gln Val Ala Ala Asp Asn Ala Ile Ser Pro Ala
  1               5                  10                  15

GCA GAG CCC CGA CGG CGG TCA GAG CCC TCC TCG TCC TCG TCT TCG TCC       96
Ala Glu Pro Arg Arg Arg Ser Glu Pro Ser Ser Ser Ser Ser Ser Ser
             20                  25                  30

TCG CCA GCG GCC CCC GTG CGT CCC CGG CCC TGC CCG GCG GTC CCA GCC      144
Ser Pro Ala Ala Pro Val Arg Pro Arg Pro Cys Pro Ala Val Pro Ala
         35                  40                  45

CCA GCC CCT GGC GAC ACT CAC TTC CGC ACC TTC CGC TCC CAC TCC GAT      192
Pro Ala Pro Gly Asp Thr His Phe Arg Thr Phe Arg Ser His Ser Asp
     50                  55                  60

TAC CGG CGC ATC ACG CGG ACC AGC GCG CTC CTG GAC GCC TGC GGC TTC      240
Tyr Arg Arg Ile Thr Arg Thr Ser Ala Leu Leu Asp Ala Cys Gly Phe
 65                  70                  75                  80

TAT TGG GGA CCC CTG AGC GTG CAC GGG GCG CAC GAG CGG CTG CGT GCC      288
Tyr Trp Gly Pro Leu Ser Val His Gly Ala His Glu Arg Leu Arg Ala
                 85                  90                  95

GAG CCC GTG GGC ACC TTC TTG GTG CGC GAC AGT CGT CAA CGG AAC TGC      336
Glu Pro Val Gly Thr Phe Leu Val Arg Asp Ser Arg Gln Arg Asn Cys
            100                 105                 110

TTC TTC GCG CTC AGC GTG AAG ATG GCT TCG GGC CCC ACG AGC ATC CGC      384
Phe Phe Ala Leu Ser Val Lys Met Ala Ser Gly Pro Thr Ser Ile Arg
        115                 120                 125

GTG CAC TTC CAG GCC GGC CGC TTC CAC TTG GAC GGC AGC CGC GAG ACC      432
Val His Phe Gln Ala Gly Arg Phe His Leu Asp Gly Ser Arg Glu Thr
    130                 135                 140
```

```
TTC GAC TGC CTT TTC GAG CTG CTG GAG CAC TAC GTG GCG GCG CCG CGC       480
Phe Asp Cys Leu Phe Glu Leu Leu Glu His Tyr Val Ala Ala Pro Arg
145                 150                 155                 160

CGC ATG TTG GGG GCC CCG CTG CGC CAG CGC CGC GTG CGG CCG CTG CAG       528
Arg Met Leu Gly Ala Pro Leu Arg Gln Arg Arg Val Arg Pro Leu Gln
                165                 170                 175

GAG CTG TGT CGC CAG CGC ATC GTG GCC GCC GTG GGT CGC GAG AAC CTG       576
Glu Leu Cys Arg Gln Arg Ile Val Ala Ala Val Gly Arg Glu Asn Leu
            180                 185                 190

GCG CGC ATC CCT CTT AAC CCG GTA CTC CGT GAC TAC CTG AGT TCC TTC       624
Ala Arg Ile Pro Leu Asn Pro Val Leu Arg Asp Tyr Leu Ser Ser Phe
        195                 200                 205

CCC TTC CAG ATC TGA CCGGCTG CCGCTGTGCC GCAGCATTAA GTGGGGGCGC          676
Pro Phe Gln Ile  *
    210

CTTATTATTT CTTATTATTA ATTATTATTA TTTTTCTGGA ACCACGTGGG AGCCCTCCCC     736

GCCTGGGTCG GAGGGAGTGG TTGTGGAGGG TGAGATGCCT CCCACTTCTG GCTGGAGACC     796

TCATCCCACC TCTCAGGGGT GGGGGTGCTC CCCTCCTGGT GCTCCCTCCG GGTCCCCCCT     856

GGTTGTAGCA GCTTGTGTCT GGGGCCAGGA CCTGAATTCC ACTCCTACCT CTCCATGTTT     916

ACATATTCCC AGTATCTTTG CACAAACCAG GGGTCGGGGA GGGTCTCTGG CTTCATTTTT     976

CTGCTGTGCA GAATATCCTA TTTTATATTT TTACAGCCAG TTTAGGTAAT AAACTTTATT    1036

ATGAAAGTTT TTTTTTAAAA GAAAAAAAAA AAAAAAAAA                           1075
```

(2) INFORMATION FOR SEQ ID NO:4:

(i) SEQUENCE CHARACTERISTICS:
(A) LENGTH: 212 amino acids
(B) TYPE: amino acid
(D) TOPOLOGY: linear

(ii) MOLECULE TYPE: protein

(xi) SEQUENCE DESCRIPTION: SEQ ID NO:4:

```
Met Val Ala Arg Asn Gln Val Ala Ala Asp Asn Ala Ile Ser Pro Ala
 1               5                  10                  15

Ala Glu Pro Arg Arg Arg Ser Glu Pro Ser Ser Ser Ser Ser Ser Ser
            20                  25                  30

Ser Pro Ala Ala Pro Val Arg Pro Arg Pro Cys Pro Ala Val Pro Ala
        35                  40                  45

Pro Ala Pro Gly Asp Thr His Phe Arg Thr Phe Arg Ser His Ser Asp
    50                  55                  60

Tyr Arg Arg Ile Thr Arg Thr Ser Ala Leu Leu Asp Ala Cys Gly Phe
 65                  70                  75                  80

Tyr Trp Gly Pro Leu Ser Val His Gly Ala His Glu Arg Leu Arg Ala
                85                  90                  95

Glu Pro Val Gly Thr Phe Leu Val Arg Asp Ser Arg Gln Arg Asn Cys
            100                 105                 110
```

```
Phe Phe Ala Leu Ser Val Lys Met Ala Ser Gly Pro Thr Ser Ile Arg
        115                 120                 125

Val His Phe Gln Ala Gly Arg Phe His Leu Asp Gly Ser Arg Glu Thr
    130                 135                 140

Phe Asp Cys Leu Phe Glu Leu Leu Glu His Tyr Val Ala Ala Pro Arg
145                 150                 155                 160

Arg Met Leu Gly Ala Pro Leu Arg Gln Arg Arg Val Arg Pro Leu Gln
                165                 170                 175

Glu Leu Cys Arg Gln Arg Ile Val Ala Ala Val Gly Arg Glu Asn Leu
            180                 185                 190

Ala Arg Ile Pro Leu Asn Pro Val Leu Arg Asp Tyr Leu Ser Ser Phe
        195                 200                 205

Pro Phe Gln Ile
    210
```

(2) INFORMATION FOR SEQ ID NO:5:

(i) SEQUENCE CHARACTERISTICS:
(A) LENGTH: 1121 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 223..819

(xi) SEQUENCE DESCRIPTION: SEQ ID NO:5:

```
GCGATCTGTG GGTGACAGTG TCTGCGAGAG ACTTTGCCAC ACCATTCTGC CGGAATTTGG       60

AGAAAAAGAA CCAGCCGCTT CCAGTCCCCT CCCCCTCCGC CACCATTTCG GACACCCTGC      120

ACACTCTCGT TTTGGGGTAC CCTGTGACTT CCAGGCAGCA CGCGAGGTCC ACTGGCCCCA      180

GCTCGGGCGA CCAGCTGTCT GGGACGTGTT GACTCATCTC CC ATG ACC CTG CGG         234
                                              Met Thr Leu Arg
                                                1

TGC CTG GAG CCC TCC GGG AAT GGA GCG GAC AGG ACG CGG AGC CAG TGG       282
Cys Leu Glu Pro Ser Gly Asn Gly Ala Asp Arg Thr Arg Ser Gln Trp
  5                  10                  15                  20

GGG ACC GCG GGG TTG CCG GAG GAA CAG TCC CCC GAG GCG GCG CGT CTG       330
Gly Thr Ala Gly Leu Pro Glu Glu Gln Ser Pro Glu Ala Ala Arg Leu
                 25                  30                  35

GCG AAA GCC CTG CGC GAG CTC AGT CAA ACA GGA TGG TAC TGG GGA AGT       378
Ala Lys Ala Leu Arg Glu Leu Ser Gln Thr Gly Trp Tyr Trp Gly Ser
             40                  45                  50

ATG ACT GTT AAT GAA GCC AAA GAG AAA TTA AAA GAG GCT CCA GAA GGA       426
Met Thr Val Asn Glu Ala Lys Glu Lys Leu Lys Glu Ala Pro Glu Gly
         55                  60                  65
```

```
ACT TTC TTG ATT AGA GAT AGT TCG CAT TCA GAC TAC CTA CTA ACT ATA      474
Thr Phe Leu Ile Arg Asp Ser Ser His Ser Asp Tyr Leu Leu Thr Ile
     70                  75                  80

TCC GTT AAG ACG TCA GCT GGA CCG ACT AAC CTG CGG ATT GAG TAC CAA      522
Ser Val Lys Thr Ser Ala Gly Pro Thr Asn Leu Arg Ile Glu Tyr Gln
 85                  90                  95                 100

GAT GGG AAA TTC AGA TTG GAT TCT ATC ATA TGT GTC AAG TCC AAG CTT      570
Asp Gly Lys Phe Arg Leu Asp Ser Ile Ile Cys Val Lys Ser Lys Leu
                105                 110                 115

AAA CAG TTT GAC AGT GTG GTT CAT CTG ATT GAC TAC TAT GTC CAG ATG      618
Lys Gln Phe Asp Ser Val Val His Leu Ile Asp Tyr Tyr Val Gln Met
            120                 125                 130

TGC AAG GAT AAA CGG ACA GGC CCA GAA GCC CCA CGG AAT GGG ACT GTT      666
Cys Lys Asp Lys Arg Thr Gly Pro Glu Ala Pro Arg Asn Gly Thr Val
        135                 140                 145

CAC CTG TAC CTG ACC AAA CCT CTG TAT ACA TCA GCA CCC ACT CTG CAG      714
His Leu Tyr Leu Thr Lys Pro Leu Tyr Thr Ser Ala Pro Thr Leu Gln
    150                 155                 160

CAT TTC TGT CGA CTC GCC ATT AAC AAA TGT ACC GGT ACG ATC TGG GGA      762
His Phe Cys Arg Leu Ala Ile Asn Lys Cys Thr Gly Thr Ile Trp Gly
165                 170                 175                 180

CTG CCT TTA CCA ACA AGA CTA AAA GAT TAC TTG GAA GAA TAT AAA TTC      810
Leu Pro Leu Pro Thr Arg Leu Lys Asp Tyr Leu Glu Glu Tyr Lys Phe
                185                 190                 195

CAG GTA TAAGTATTTC TCTCTCTTTT TCGTTTTTTT TTAAAAAAAA AAAAACACAT       866
Gln Val

GCCTCATATA GACTATCTCC GAATGCAGCT ATGTGAAAGA GAACCCAGAG GCCCTCCTCT     926

GGATAACTGC GCAGAATTCT CTCTTAAGGA CAGTTGGGCT CAGTCTAACT TAAAGGTGTG     986

AAGATGTAGC TAGGTATTTT AAAGTTCCCC TTAGGTAGTT TTAGCTGAAT GATGCTTTCT    1046

TTCCTATGGC TGCTCAAGAT CAAATGGCCC TTTTAAATGA AACAAAACAA AACAAAACAA    1106

AAAAAAAAAA AAAAA                                                     1121
```

(2) INFORMATION FOR SEQ ID NO:6:

(i) SEQUENCE CHARACTERISTICS:
(A) LENGTH: 198 amino acids
(B) TYPE: amino acid
(D) TOPOLOGY: linear

(ii) MOLECULE TYPE: protein

(xi) SEQUENCE DESCRIPTION: SEQ ID NO:6:

```
Met Thr Leu Arg Cys Leu Glu Pro Ser Gly Asn Gly Ala Asp Arg Thr
  1               5                  10                  15

Arg Ser Gln Trp Gly Thr Ala Gly Leu Pro Glu Glu Gln Ser Pro Glu
            20                  25                  30

Ala Ala Arg Leu Ala Lys Ala Leu Arg Glu Leu Ser Gln Thr Gly Trp
        35                  40                  45

Tyr Trp Gly Ser Met Thr Val Asn Glu Ala Lys Glu Lys Leu Lys Glu
```

```
    50                  55                  60

Ala Pro Glu Gly Thr Phe Leu Ile Arg Asp Ser Ser His Ser Asp Tyr
65                  70                  75                  80

Leu Leu Thr Ile Ser Val Lys Thr Ser Ala Gly Pro Thr Asn Leu Arg
                85                  90                  95

Ile Glu Tyr Gln Asp Gly Lys Phe Arg Leu Asp Ser Ile Ile Cys Val
            100                 105                 110

Lys Ser Lys Leu Lys Gln Phe Asp Ser Val Val His Leu Ile Asp Tyr
        115                 120                 125

Tyr Val Gln Met Cys Lys Asp Lys Arg Thr Gly Pro Glu Ala Pro Arg
    130                 135                 140

Asn Gly Thr Val His Leu Tyr Leu Thr Lys Pro Leu Tyr Thr Ser Ala
145                 150                 155                 160

Pro Thr Leu Gln His Phe Cys Arg Leu Ala Ile Asn Lys Cys Thr Gly
                165                 170                 175

Thr Ile Trp Gly Leu Pro Leu Pro Thr Arg Leu Lys Asp Tyr Leu Glu
            180                 185                 190

Glu Tyr Lys Phe Gln Val
        195
```

(2) INFORMATION FOR SEQ ID NO:7:

(i) SEQUENCE CHARACTERISTICS:
(A) LENGTH: 2187 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 18..695

(xi) SEQUENCE DESCRIPTION: SEQ ID NO:7:

```
CGCTGGCTCC GTGCGCC ATG GTC ACC CAC AGC AAG TTT CCC GCC GCC GGG        50
                   Met Val Thr His Ser Lys Phe Pro Ala Ala Gly
                    1               5                  10

ATG AGC CGC CCC CTG GAC ACC AGC CTG CGC CTC AAG ACC TTC AGC TCC       98
Met Ser Arg Pro Leu Asp Thr Ser Leu Arg Leu Lys Thr Phe Ser Ser
            15                  20                  25

AAA AGC GAG TAC CAG CTG GTG GTG AAC GCC GTG CGC AAG CTG CAG GAG      146
Lys Ser Glu Tyr Gln Leu Val Val Asn Ala Val Arg Lys Leu Gln Glu
        30                  35                  40

AGC GGA TTC TAC TGG AGC GCC GTG ACC GGC GGC GAG GCG AAC CTG CTG      194
Ser Gly Phe Tyr Trp Ser Ala Val Thr Gly Gly Glu Ala Asn Leu Leu
    45                  50                  55

CTC AGC GCC GAG CCC GCG GGC ACC TTT CTT ATC CGC GAC AGC TCG GAC      242
Leu Ser Ala Glu Pro Ala Gly Thr Phe Leu Ile Arg Asp Ser Ser Asp
60                  65                  70                  75
```

```
CAG CGC CAC TTC TTC ACG TTG AGC GTC AAG ACC CAG TCG GGG ACC AAG     290
Gln Arg His Phe Phe Thr Leu Ser Val Lys Thr Gln Ser Gly Thr Lys
                80                  85                  90

AAC CTA CGC ATC CAG TGT GAG GGG GGC AGC TTT TCG CTG CAG AGT GAC     338
Asn Leu Arg Ile Gln Cys Glu Gly Gly Ser Phe Ser Leu Gln Ser Asp
            95                  100                 105

CCC CGA AGC ACG CAG CCA GTT CCC CGC TTC GAC TGT GTA CTC AAG CTG     386
Pro Arg Ser Thr Gln Pro Val Pro Arg Phe Asp Cys Val Leu Lys Leu
        110                 115                 120

GTG CAC CAC TAC ATG CCG CCT CCA GGG ACC CCC TCC TTT TCT TTG CCA     434
Val His His Tyr Met Pro Pro Pro Gly Thr Pro Ser Phe Ser Leu Pro
    125                 130                 135

CCC ACG GAA CCC TCG TCC GAA GTT CCG GAG CAG CCA CCT GCC CAG GCA     482
Pro Thr Glu Pro Ser Ser Glu Val Pro Glu Gln Pro Pro Ala Gln Ala
140                 145                 150                 155

CTC CCC GGG AGT ACC CCC AAG AGA GCT TAC TAC ATC TAT TCT GGG GGC     530
Leu Pro Gly Ser Thr Pro Lys Arg Ala Tyr Tyr Ile Tyr Ser Gly Gly
                160                 165                 170

GAG AAG ATT CCG CTG GTA CTG AGC CGA CCT CTC TCC TCC AAC GTG GCC     578
Glu Lys Ile Pro Leu Val Leu Ser Arg Pro Leu Ser Ser Asn Val Ala
            175                 180                 185

ACC CTC CAG CAT CTT TGT CGG AAG ACT GTC AAC GGC CAC CTG GAC TCC     626
Thr Leu Gln His Leu Cys Arg Lys Thr Val Asn Gly His Leu Asp Ser
        190                 195                 200

TAT GAG AAA GTG ACC CAG CTG CCT GGA CCC ATT CGG GAG TTC CTG GAT     674
Tyr Glu Lys Val Thr Gln Leu Pro Gly Pro Ile Arg Glu Phe Leu Asp
    205                 210                 215

CAG TAT GAT GCT CCA CTT TAAGGAGCAA AAGGGTCAGA GGGGGGCCTG            722
Gln Tyr Asp Ala Pro Leu
220                 225

GGTCGGTCGG TCGCCTCTCC TCCGAGGCAC ATGGCACAAG CACAAAAATC CAGCCCCAAC   782
GGTCGGTAGC TCCCAGTGAG CCAGGGGCAG ATTGGCTTCT TCCTCAGGCC CTCCACTCCC   842
GCAGAGTAGA GCTGGCAGGA CCTGGAATTC GTCTGAGGGG AGGGGGAGCT GCCACCTGCT   902
TTCCCCCCTC CCCCAGCTCC AGCTTCTTTC AAGTGGAGCC AGCCGGCCTG GCCTGGTGGG   962
ACAATACCTT TGACAAGCGG ACTCTCCCCT CCCCTTCCTC CACACCCCCT CTGCTTCCCA  1022
AGGGAGGTGG GGACACCTCC AAGTGTTGAA CTTAGAACTG CAAGGGGAAT CTTCAAACTT  1082
TCCCGCTGGA ACTTGTTTGC GCTTTGATTT GGTTTGATCA AGAGCAGGCA CCTGGGGGAA  1142
GGATGGAAGA GAAAAGGGTG TGTGAAGGGT TTTTATGCTG GCCAAAGAAA TAACCACTCC  1202
CACTGCCCAA CCTAGGTGAG GAGTGGTGGC TCCTGGCTCT GGGGAGAGTG GCAAGGGGTG  1262
ACCTGAAGAG AGCTATACTG GTGCCAGGCT CCTCTCCATG GGGCAGCTAA TGAAACCTCG  1322
CAGATCCCTT GCACCCCAGA ACCCTCCCCG TTGTGAAGAG GCAGTAGCAT TTAGAAGGGA  1382
GACAGATGAG GCTGGTGAGC TGGCCGCCTT TTCCAACACC GAAGGGAGGC AGATCAACAG  1442
ATGAGCCATC TTGGAGCCCA GGTTTCCCCT GGAGCAGATG GAGGGTTCTG CTTTGTCTCT  1502
CCTATGTGGG GCTAGGAGAC TCGCCTTAAA TGCCCTCTGT CCCAGGGATG GGATTGGCA   1562
```

```
CACAAGGAGC CAAACACAGC CAATAGGCAG AGAGTTGAGG GATTCACCCA GGTGGCTACA   1622
GGCCAGGGGA AGTGGCTGCA GGGGAGAGAC CCAGTCACTC CAGGAGACTC CTGAGTTAAC   1682
ACTGGGAAGA CATTGGCCAG TCCTAGTCAT CTCTCGGTCA GTAGGTCCGA GAGCTTCCAG   1742
GCCCTGCACA GCCCTCCTTT CTCACCTGGG GGGAGGCAGG AGGTGATGGA GAAGCCTTCC   1802
CATGCCGCTC ACAGGGGCCT CACGGGAATG CAGCAGCCAT GCAATTACCT GGAACTGGTC   1862
CTGTGTTGGG GAGAAACAAG TTTTCTGAAG TCAGGTATGG GGCTGGGTGG GGCAGCTGTG   1922
TGTTGGGGTG GCTTTTTTCT CTCTGTTTTG AATAATGTTT ACAATTTGCC TCAATCACTT   1982
TTATAAAAAT CCACCTCCAG CCCGCCCCTC TCCCCACTCA GGCCTTCGAG GCTGTCTGAA   2042
GATGCTTGAA AAACTCAACC AAATCCCAGT TCAACTCAGA CTTTGCACAT ATATTTATAT   2102
TTATACTCAG AAAAGAAACA TTTCAGTAAT TTATAATAAA AGAGCACTAT TTTTTAATGA   2162
AAAAAAAAAA AAAAAAAAAA AAAAA                                         2187
```

(2) INFORMATION FOR SEQ ID NO:8:

(i) SEQUENCE CHARACTERISTICS:
(A) LENGTH: 225 amino acids
(B) TYPE: amino acid
(D) TOPOLOGY: linear

(ii) MOLECULE TYPE: protein

(xi) SEQUENCE DESCRIPTION: SEQ ID NO:8:

```
Met Val Thr His Ser Lys Phe Pro Ala Ala Gly Met Ser Arg Pro Leu
 1               5                  10                  15

Asp Thr Ser Leu Arg Leu Lys Thr Phe Ser Ser Lys Ser Glu Tyr Gln
            20                  25                  30

Leu Val Val Asn Ala Val Arg Lys Leu Gln Glu Ser Gly Phe Tyr Trp
        35                  40                  45

Ser Ala Val Thr Gly Gly Glu Ala Asn Leu Leu Leu Ser Ala Glu Pro
    50                  55                  60

Ala Gly Thr Phe Leu Ile Arg Asp Ser Ser Asp Gln Arg His Phe Phe
65                  70                  75                  80

Thr Leu Ser Val Lys Thr Gln Ser Gly Thr Lys Asn Leu Arg Ile Gln
                85                  90                  95

Cys Glu Gly Gly Ser Phe Ser Leu Gln Ser Asp Pro Arg Ser Thr Gln
            100                 105                 110

Pro Val Pro Arg Phe Asp Cys Val Leu Lys Leu Val His His Tyr Met
        115                 120                 125

Pro Pro Pro Gly Thr Pro Ser Phe Ser Leu Pro Pro Thr Glu Pro Ser
    130                 135                 140

Ser Glu Val Pro Glu Gln Pro Pro Ala Gln Ala Leu Pro Gly Ser Thr
145                 150                 155                 160

Pro Lys Arg Ala Tyr Tyr Ile Tyr Ser Gly Gly Glu Lys Ile Pro Leu
                165                 170                 175

Val Leu Ser Arg Pro Leu Ser Ser Asn Val Ala Thr Leu Gln His Leu
```

```
            180                      185                      190

Cys Arg Lys Thr Val Asn Gly His Leu Asp Ser Tyr Glu Lys Val Thr
        195                     200                     205

Gln Leu Pro Gly Pro Ile Arg Glu Phe Leu Asp Gln Tyr Asp Ala Pro
    210                     215                     220

Leu
225
```

(2) INFORMATION FOR SEQ ID NO:9:

(i) SEQUENCE CHARACTERISTICS:
(A) LENGTH: 1094 base pairs
(B) TYPE: nucleic acid
(D) TOPOLOGY: linear

(ii) MOLECULE TYPE: protein

(xi) SEQUENCE DESCRIPTION: SEQ ID NO:9:

```
CTCCGGCTGG CCCCTTCTGT AGGATGGTAG CACACAACCA GGTGGCAGCC GACAATGCAG60
TCTCCACAGC AGCAGAGCCC CGACGGCGGC CAGAACCTTC CTCCTCTTCC TCCTCCTCGC120
CCGCGGCCCC CGCGCGCCCG CGGCCGTGCC CCGCGGTCCC GGCCCCGGCC CCCGGCGACA180
CGCACTTCCG CACATTCCGT TCGCACGCCG ATTACCGGCG CATCACGCGC GCCAGCGCGC240
TCCTGGACGC CTGCGGATTC TACTGGGGGC CCCTGAGCGT GCACGGGGCG CACGAGCGGC300
TGCGCGCCGA GCCCGTGGGC ACCTTCCTGG TGCGCGACAG CCGCCAGCGG AACTGCTTTT360
TCGCCCTTAG CGTGAAGATG GCCTCGGGAC CCACGAGCAT CCGCGTGCAC TTTCAGGCCG420
GCCGCTTTCA CCTGGATGGC AGCCGCGAGA GCTTCGACTG CCTCTTCGAG CTGCTGGAGC480
ACTACGTGGC GGCGCCGCGC CGCATGCTGG GGGCCCCGCT GCGCCAGCGC CGCGTGCGGC540
CGCTGCAGGA GCTGTGCCGC CAGCGCATCG TGGCCACCGT GGGCCGCGAG AACCTGGCTC600
GCATCCCCCT CAACCCCGTC CTCCGCGACT ACCTGAGCTC CTTCCCCTTC CAGATTTGAC660
CGGCAGCGCC CGCCGTGCAC GCAGCATTAA CTGGGATGCC GTGTTATTTT GTTATTACTT720
GCCTGGAACC ATGTGGGTAC CCTCCCCGGC CTGGGTTGGA GGGAGCGGAT GGGTGTAGGG780
GCGAGGCGCC TCCCGCCCTC GGCTGGAGAC GAGGCCGCAG ACCCCTTCTC ACCTCTTGAG840
GGGGTCCTCC CCCTCCTGGT GCTCCCTCTG GGTCCCCCTG GTTGTTGTAG CAGCTTAACT900
GTATCTGGAG CCAGGACCTG AACTCGCACC TCCTACCTCT TCATGTTTAC ATATACCCAG960
TATCTTTGCA CAAACCAGGG GTTGGGGGAG GGTCTCTGGC TTTATTTTTC TGCTGTGCAG1020
AATCCTATTT TATATTTTTT AAAGTCAGTT TAGGTAATAA ACTTTATTAT GAAAGTTTTT1080
TTTTTTAAAA AAAA                                                  1094
```

(2) INFORMATION FOR SEQ ID NO:10:

(i) SEQUENCE CHARACTERISTICS:
(A) LENGTH: 211 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:10:

```
Met Val Ala His Asn Gln Val Ala Ala Asp Asn Ala Val Ser Thr Ala
1               5                   10                  15

Ala Glu Pro Arg Arg Arg Pro Glu Pro Ser Ser Ser Ser Ser Ser Ser
            20                  25                  30

Pro Ala Ala Pro Ala Arg Pro Arg Pro Cys Pro Ala Val Pro Ala Pro
```

```
            35                  40                  45
Ala Pro Gly Asp Thr His Phe Arg Thr Phe Arg Ser His Ala Asp Tyr
    50                  55                  60
Arg Arg Ile Thr Arg Ala Ser Ala Leu Leu Asp Ala Cys Gly Phe Tyr
65                  70                  75                  80
Trp Gly Pro Leu Ser Val His Gly Ala His Glu Arg Leu Arg Ala Glu
                85                  90                  95
Pro Val Gly Thr Phe Leu Val Arg Asp Ser Arg Gln Arg Asn Cys Phe
            100                 105                 110
Phe Ala Leu Ser Val Lys Met Ala Ser Gly Pro Thr Ser Ile Arg Val
        115                 120                 125
His Phe Gln Ala Gly Arg Phe His Leu Asp Gly Ser Arg Glu Ser Phe
    130                 135                 140
Asp Cys Leu Phe Glu Leu Leu Glu His Tyr Val Ala Ala Pro Arg Arg
145                 150                 155                 160
Met Leu Gly Ala Pro Leu Arg Gln Arg Arg Val Arg Pro Leu Gln Glu
                165                 170                 175
Leu Cys Arg Gln Arg Ile Val Ala Thr Val Gly Arg Glu Asn Leu Ala
            180                 185                 190
Arg Ile Pro Leu Asn Pro Val Leu Arg Asp Tyr Leu Ser Ser Phe Pro
        195                 200                 205
Phe Gln Ile
    210
```

(2) INFORMATION FOR SEQ ID NO:11:

(i) SEQUENCE CHARACTERISTICS:
(A) LENGTH: 2807 base pairs
(B) TYPE: nucleic acid
(D) TOPOLOGY: linear

(ii) MOLECULE TYPE: protein

(xi) SEQUENCE DESCRIPTION: SEQ ID NO:11:

```
GGAAACCGAG GCGGGGAGAC CAGGAGGCCT TGGCCTCAGA GCTTCAGAGT CGCGTGGCAG60
CAAACAGAGA AACCTGTAGA GGGCAGTGTG CGTCACTTAG CTCAGGGAAG CTGCACGCGA120
AACTCACCCG CCTTCATTCA TAAACATCGT CAGCTAGGCA CCTACTCCTG GGCTTTCAGG180
ACAAACTGAA TCACGAAACC ACAGTGTCCT TAAAATAGGT CTGACCGCCT GAATCCCTGG240
CCAAGGTGTG TACGGGGCAT GGGAGCCCTT GTGCAGAGAT GCTTGCAGGA GCCTTGAGGG300
GCTCTGTAAG ACAGAGGCTA GGAAGACAAA GTTGGGGGCT ACAGCTTCTT GTCCTGCCCG360
GGGCCTCAGT TTCTTCGGTT GCCCACGTAG GAGTGCAGAG AGTCCAGCCC CTGGGGACCC420
AACCCAACCC CGCCCAGTTT CCGAGGAACT CGTCCGGGAG CGGGGGCGCC CCTCCCGCAC480
CGCCTTAGGC TTCCTTTGAA GCCTCTGCGG TCAGGCCACC GCTTCCTGGG AAGCCCAAGC540
CAAGGCCAGG CCGAGTGGCC AACGGGAGGG GCCCGCGCGC GATTCTGGAG GAGGGCGGCG600
GCCCCACAGG TCTCCAGGGC TGGCTAGCCG GGCTCCTAGA GCGGAGACTG CCAAGGCCTT660
CGGGTCCTGG GCAGGAAGGA TCCTGGCAGG GAGGAGTTGC TTGGGGGGTG GGGGGGAAAG720
GCTCCAGGCG CGGTGGAGCT CTGACCAGGA GAATGCACAC ACTCGGAGGG GAGGAGGCGT780
GTCAGCCCCA AGCTAGCATC CCACCCGGGG AGCAGCGATG TGGGGCGAAG GTAGCCAGAG840
CAAAAGAGCA GGCACCAGGT GACACGAAAC AGAAGATTCC GGGTAGAGCC AGAACCCCAG900
AAGTCCCATT CAGGGAAGGT GCGAGGCGAG AACGAGTTAG GTGGACCCTC TCCAGGGGCA960
GCCAAAGAAA TCTAAAGAGA ACCCGAAGGA CTTGCCGGAA AGAGAAACCG AAAGCGGCGG1020
TGGGCGGGAT CGGTGGGCGG GGCCTCCCTG GTTTAAGAGC TTGATGCAGG GGCGGGCAGC1080
AGCAGAGAGA ACTGCGGCCG TGGCAGCGGC ACGGCTCCCG GCCCCGGAGC ATGCGCGACA1140
GCAGCCCCGG AACCCCCAGC CGCGGCGCCC CGCGTCCCGC CGCCAGGTGA GCCGAGGCAG1200
CTGCGAAGGA GCAGGCGGGA GGGGATGGGA GGAAGGGGAG CAGAGCCTGG CAGGACTATC1260
```

```
CTCGCAGACT GCATGGCGGG GTCGTGGATG CTATGCCTCT GGCGCCCGCC CCACCGGCTG1320
GCCCAGGCGG CCCCTCGCGC GCGCGGGGCG CCGTCAGCCC CTCCTCTCCG GCCCTGAGCC1380
CGGATCGTCC GCCCGGGTTC CAGTTCCCGG CGTGGCCAGT AGGCGGCAAC CGCGAGGCGG1440
CAAGCCACCC AGCGGGGACG GCCTGGAGTC GGGCCCCTCT CCACGCCCCC TTCTCCACGC1500
GCGCGGGGAG GCAGGGCTCC ACCGCCAGTC TGGAAGGGTT CCACATACAG GAACGGCCTA1560
CTTCGCAGAT GAGCCCACCG AGGCTCAGGC TCCGGGCGGA TTCTGCGTGT CACCCTCGCT1620
CCTTGGGGTC CGCTGGCCGG CCTGTGCCAC CCGGACGCCC GGTTCACTGC CTCTGTCTCC1680
CCCATCAGCG CAGCCCCGGA CGCTATGGCC CACCCCTCCA GCTGGCCCCT CGAGTAGGAT1740
GGTAGCACGT AACCAGGTGG AAGCCGACAA TGCGATCTCC CCGGCATCAG AGCCCCGACG1800
GCGGCCAGAG CCATCCTCGT CCTCGTCTTC GTCCTCGCCG GCGGCCCCGG CGCGTCCCCG1860
GCCCTGCCCG GTGGTCCCGG CCCCGGCTCC GGGCGACACT CACTTCCGCA CCTTCCGCTC1920
CCACTCTGAT TACCGGCGCA TCACGCGGAC CAGCGCTCTC CTGGACGCCT GCGGCTTCTA1980
CTGGGGACCC CTGAGCGTGC ATGGGGCGCA CGAACGGCTG CGTTCCGAAC CCGTGGGCAC2040
CTTCTTGGTG CGCGACAGTC GCCAGCGGAA CTGCTTCTTC GCGCTCAGCG TGAAGATGGC2100
TTCGGGCCCC ACGAGCATTC GTGTGCACTT CCAGGCCGGC CGCTTCCACC TGGACGGCAA2160
CCGCGAGACC TTCGACTGCC TCTTCGAGCT GCTGGAGCAC TACGTGGCGG CGCCGCGCCG2220
CATGTTGGGG GCCCCACTGC GCCAGCGCCG CGTGCGGCCG CTGCAGGAGC TGTGTCGCCA2280
GCGCATCGTG GCCGCCGTGG GTCGCGAGAA CCTGGCACGC ATCCCTCTTA ACCCGGTACT2340
CCGTGACTAC CTGAGTTCCT TCCCCTTCCA GATCTGACCG GCTGCCGCCG TGCCCGCAGA2400
ATTAAGTGGG AGCGCCTTAT TATTTCTTAT TATTAATTAT TATTATTTTT CTGGAACCAC2460
GTGGGAGCCC TCCCCGCCTA GGTCGGAGGG AGTGGGTGTG GAGGGTGAGA TCCCTCCCAC2520
TTCTGGCTGG AGACCTTATC CCGCCTCTCG GGGGGCCTCC CCTCCTGGTG CTCCCTCCCG2580
GTCCCCCTGG TTGTAGCAGC TTGTGTCTGG GGCCAGGACC TGAACTCCAC GCCTACCTCT2640
CCATGTTTAC ATGTTCCCAG TATCTTTGCA CAAACCAGGG GTGGGGGAGG GTCTCTGGCT2700
TCATTTTTCT GCTGTGCAGA ATATTCTATT TTATATTTTT ACATCCAGTT TAGATAATAA2760
ACTTTATTAT GAAAGTTTTT TTTTTTAAAG AAACAAAGAT TTCTAGA          2807
```

(2) INFORMATION FOR SEQ ID NO:12:

(i) SEQUENCE CHARACTERISTICS:
(A) LENGTH: 212 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:12:

```
Met Val Ala Arg Asn Gln Val Glu Ala Asp Asn Ala Ile Ser Pro Ala
1               5                   10                  15

Ser Glu Pro Arg Arg Arg Pro Glu Pro Ser Ser Ser Ser Ser Ser Ser
            20                  25                  30

Ser Pro Ala Ala Pro Ala Arg Pro Arg Pro Cys Pro Val Val Pro Ala
        35                  40                  45

Pro Ala Pro Gly Asp Thr His Phe Arg Thr Phe Arg Ser His Ser Asp
    50                  55                  60

Tyr Arg Arg Ile Thr Arg Thr Ser Ala Leu Leu Asp Ala Cys Gly Phe
65                  70                  75                  80

Tyr Trp Gly Pro Leu Ser Val His Gly Ala His Glu Arg Leu Arg Ser
                85                  90                  95

Glu Pro Val Gly Thr Phe Leu Val Arg Asp Ser Arg Gln Arg Asn Cys
            100                 105                 110

Phe Phe Ala Leu Ser Val Lys Met Ala Ser Gly Pro Thr Ser Ile Arg
        115                 120                 125

Val His Phe Gln Ala Gly Arg Phe His Leu Asp Gly Asn Arg Glu Thr
```

```
    130                 135                 140

Phe Asp Cys Leu Phe Glu Leu Leu Glu His Tyr Val Ala Ala Pro Arg
145                 150                 155                 160

Arg Met Leu Gly Ala Pro Leu Arg Gln Arg Arg Val Arg Pro Leu Gln
                165                 170                 175

Glu Leu Cys Arg Gln Arg Ile Val Ala Ala Val Gly Arg Glu Asn Leu
            180                 185                 190

Ala Arg Ile Pro Leu Asn Pro Val Leu Arg Asp Tyr Leu Ser Ser Phe
        195                 200                 205

Pro Phe Gln Ile
    210
```

(2) INFORMATION FOR SEQ ID NO:13:

(i) SEQUENCE CHARACTERISTICS:
(A) LENGTH: 1611 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 263..1529

(xi) SEQUENCE DESCRIPTION: SEQ ID NO:13:

```
CGAATTCCGG GCGGGCTGTG TGAGTCTGTG AGTGGAAGGC GCGCCGGCTC TTTTGTCTGA       60

GTGTGACCCG GTGGCTTTGT TCCAGGCATT CCGGTGATTT CCTCCGGGCA GTCCGCAGAA      120

GCCGCAGCGG CCGCCCGCGC TCTCTCTGCA GTCTCCACAC CCGGGAGAGC CTGAGCCCGC      180

GTCACGCCCC TCAGCCCCCG CTGAGTCCCT TCTCTGTTGT CGCGTCCGAA TCGAGTTCCC      240

GGAATCAGAC GGTGCCCCAT AG ATG GCC AGC TTT CCC CCG AGG GTT AAC GAG       292
                         Met Ala Ser Phe Pro Pro Arg Val Asn Glu
                          1               5                  10

AAA GAG ATC GTG AGA TCA CGT ACT ATA GGG GAA CTC TTG GCT CCA GCA        340
Lys Glu Ile Val Arg Ser Arg Thr Ile Gly Glu Leu Leu Ala Pro Ala
                15                  20                  25

GCT CCT TTT GAC AAG AAA TGT GGT GGT GAG AAC TGG ACG GTT GCT TTT        388
Ala Pro Phe Asp Lys Lys Cys Gly Gly Glu Asn Trp Thr Val Ala Phe
            30                  35                  40

GCT CCT GAT GGT TCC TAC TTT GCG TGG TCA CAA GGA TAT CGC ATA GTG        436
Ala Pro Asp Gly Ser Tyr Phe Ala Trp Ser Gln Gly Tyr Arg Ile Val
        45                  50                  55

AAG CTT GTC CCG TGG TCC CAG TGC CGT AAG AAC TTT CTT TTG CAT GGT        484
Lys Leu Val Pro Trp Ser Gln Cys Arg Lys Asn Phe Leu Leu His Gly
    60                  65                  70

TCC AAA AAT GTT ACC AAT TCA AGC TGT CTA AAA TTG GCA AGA CAA AAC        532
Ser Lys Asn Val Thr Asn Ser Ser Cys Leu Lys Leu Ala Arg Gln Asn
 75                  80                  85                  90
```

```
AGT AAT GGT GGT CAG AAA AAC AAG CCT CCT GAG CAC GTT ATA GAC TGT    580
Ser Asn Gly Gly Gln Lys Asn Lys Pro Pro Glu His Val Ile Asp Cys
                95                  100                 105

GGA GAC ATA GTC TGG AGT CTT GCT TTT GGG TCT TCA GTT CCA GAA AAA    628
Gly Asp Ile Val Trp Ser Leu Ala Phe Gly Ser Ser Val Pro Glu Lys
            110                 115                 120

CAG AGT CGT TGC GTT AAT ATA GAA TGG CAT CGG TTC CGA TTT GGA CAG    676
Gln Ser Arg Cys Val Asn Ile Glu Trp His Arg Phe Arg Phe Gly Gln
        125                 130                 135

GAT CAG CTA CTC CTT GCC ACA GGA TTA AAC AAT GGT CGC ATC AAA ATC    724
Asp Gln Leu Leu Leu Ala Thr Gly Leu Asn Asn Gly Arg Ile Lys Ile
    140                 145                 150

TGG GAT GTA TAT ACA GGA AAA CTC CTC CTT AAT TTG GTA GAC CAC ATT    772
Trp Asp Val Tyr Thr Gly Lys Leu Leu Leu Asn Leu Val Asp His Ile
155                 160                 165                 170

GAA ATG GTT AGA GAT TTA ACT TTT GCT CCA GAT GGG AGC TTA CTC CTT    820
Glu Met Val Arg Asp Leu Thr Phe Ala Pro Asp Gly Ser Leu Leu Leu
                175                 180                 185

GTA TCA GCT TCA AGA GAC AAA ACT CTA AGA GTG TGG GAC CTG AAA GAT    868
Val Ser Ala Ser Arg Asp Lys Thr Leu Arg Val Trp Asp Leu Lys Asp
            190                 195                 200

GAT GGA AAC ATG GTG AAA GTA TTG CGG GCA CAT CAG AAT TGG GTG TAC    916
Asp Gly Asn Met Val Lys Val Leu Arg Ala His Gln Asn Trp Val Tyr
        205                 210                 215

AGT TGT GCA TTC TCT CCC GAC TGT TCT ATG CTG TGT TCA GTG GGC GCC    964
Ser Cys Ala Phe Ser Pro Asp Cys Ser Met Leu Cys Ser Val Gly Ala
    220                 225                 230

AGT AAA GCA GTT TTC CTT TGG AAT ATG GAT AAA TAC ACC ATG ATT AGG   1012
Ser Lys Ala Val Phe Leu Trp Asn Met Asp Lys Tyr Thr Met Ile Arg
235                 240                 245                 250

AAG CTG GAA GGT CAT CAC CAT GAT GTT GTA GCT TGT GAC TTT TCT CCT   1060
Lys Leu Glu Gly His His His Asp Val Val Ala Cys Asp Phe Ser Pro
                255                 260                 265

GAT GGA GCA TTG CTA GCT ACT GCA TCC TAT GAC ACT CGT GTG TAT GTC   1108
Asp Gly Ala Leu Leu Ala Thr Ala Ser Tyr Asp Thr Arg Val Tyr Val
            270                 275                 280

TGG GAT CCA CAC AAT GGA GAC CTT CTG ATG GAG TTT GGG CAC CTG TTT   1156
Trp Asp Pro His Asn Gly Asp Leu Leu Met Glu Phe Gly His Leu Phe
        285                 290                 295

CCC TCG CCC ACT CCA ATA TTT GCT GGA GGA GCA AAT GAC CGA TGG GTG   1204
Pro Ser Pro Thr Pro Ile Phe Ala Gly Gly Ala Asn Asp Arg Trp Val
    300                 305                 310

AGA GCT GTG TCT TTC AGT CAT GAT GGA CTG CAT GTT GCC AGC CTT GCT   1252
Arg Ala Val Ser Phe Ser His Asp Gly Leu His Val Ala Ser Leu Ala
315                 320                 325                 330

GAT GAT AAA ATG GTG AGG TTC TGG AGA ATC GAT GAG GAT TGT CCG GTA   1300
Asp Asp Lys Met Val Arg Phe Trp Arg Ile Asp Glu Asp Cys Pro Val
                335                 340                 345

CAA GTT GCA CCT TTG AGC AAT GGT CTT TGC TGT GCC TTT TCT ACT GAT   1348
Gln Val Ala Pro Leu Ser Asn Gly Leu Cys Cys Ala Phe Ser Thr Asp
            350                 355                 360
```

```
GGC AGT GTT TTA GCT GCT GGG ACA CAT GAT GGA AGT GTG TAT TTT TGG      1396
Gly Ser Val Leu Ala Ala Gly Thr His Asp Gly Ser Val Tyr Phe Trp
        365                 370                 375

GCC ACT CCA AGG CAA GTC CCT AGC CTT CAA CAT ATA TGT CGC ATG TCA      1444
Ala Thr Pro Arg Gln Val Pro Ser Leu Gln His Ile Cys Arg Met Ser
    380                 385                 390

ATC CGA AGA GTG ATG TCC ACC CAA GAA GTC CAA AAA CTG CCT GTT CCT      1492
Ile Arg Arg Val Met Ser Thr Gln Glu Val Gln Lys Leu Pro Val Pro
395                 400                 405                 410

TCC AAA ATA TTG GCG TTT CTC TCC TAC CGC GGT TAG A CTGAAGACTG         1539
Ser Lys Ile Leu Ala Phe Leu Ser Tyr Arg Gly  *
                415                 420

CCTTTCCTGG TAGGCCTGCC AGACAGAGCG CCCTTTACAA GACACACCTC AAGCTTTACC    1599

TCGTGCCGAA TT                                                        1611
```

(2) INFORMATION FOR SEQ ID NO:14:

(i) SEQUENCE CHARACTERISTICS:
(A) LENGTH: 422 amino acids
(B) TYPE: amino acid
(D) TOPOLOGY: linear

(ii) MOLECULE TYPE: protein

(xi) SEQUENCE DESCRIPTION: SEQ ID NO:14:

```
Met Ala Ser Phe Pro Pro Arg Val Asn Glu Lys Glu Ile Val Arg Ser
 1               5                  10                  15

Arg Thr Ile Gly Glu Leu Leu Ala Pro Ala Ala Pro Phe Asp Lys Lys
            20                  25                  30

Cys Gly Gly Glu Asn Trp Thr Val Ala Phe Ala Pro Asp Gly Ser Tyr
        35                  40                  45

Phe Ala Trp Ser Gln Gly Tyr Arg Ile Val Lys Leu Val Pro Trp Ser
    50                  55                  60

Gln Cys Arg Lys Asn Phe Leu Leu His Gly Ser Lys Asn Val Thr Asn
65                  70                  75                  80

Ser Ser Cys Leu Lys Leu Ala Arg Gln Asn Ser Asn Gly Gly Gln Lys
                85                  90                  95

Asn Lys Pro Pro Glu His Val Ile Asp Cys Gly Asp Ile Val Trp Ser
            100                 105                 110

Leu Ala Phe Gly Ser Ser Val Pro Glu Lys Gln Ser Arg Cys Val Asn
        115                 120                 125

Ile Glu Trp His Arg Phe Arg Phe Gly Gln Asp Gln Leu Leu Leu Ala
    130                 135                 140

Thr Gly Leu Asn Asn Gly Arg Ile Lys Ile Trp Asp Val Tyr Thr Gly
145                 150                 155                 160

Lys Leu Leu Leu Asn Leu Val Asp His Ile Glu Met Val Arg Asp Leu
                165                 170                 175

Thr Phe Ala Pro Asp Gly Ser Leu Leu Leu Val Ser Ala Ser Arg Asp
            180                 185                 190
```

```
Lys Thr Leu Arg Val Trp Asp Leu Lys Asp Asp Gly Asn Met Val Lys
        195                 200                 205

Val Leu Arg Ala His Gln Asn Trp Val Tyr Ser Cys Ala Phe Ser Pro
    210                 215                 220

Asp Cys Ser Met Leu Cys Ser Val Gly Ala Ser Lys Ala Val Phe Leu
225                 230                 235                 240

Trp Asn Met Asp Lys Tyr Thr Met Ile Arg Lys Leu Glu Gly His His
                245                 250                 255

His Asp Val Val Ala Cys Asp Phe Ser Pro Asp Gly Ala Leu Leu Ala
            260                 265                 270

Thr Ala Ser Tyr Asp Thr Arg Val Tyr Val Trp Asp Pro His Asn Gly
        275                 280                 285

Asp Leu Leu Met Glu Phe Gly His Leu Phe Pro Ser Pro Thr Pro Ile
    290                 295                 300

Phe Ala Gly Gly Ala Asn Asp Arg Trp Val Arg Ala Val Ser Phe Ser
305                 310                 315                 320

His Asp Gly Leu His Val Ala Ser Leu Ala Asp Asp Lys Met Val Arg
                325                 330                 335

Phe Trp Arg Ile Asp Glu Asp Cys Pro Val Gln Val Ala Pro Leu Ser
            340                 345                 350

Asn Gly Leu Cys Cys Ala Phe Ser Thr Asp Gly Ser Val Leu Ala Ala
        355                 360                 365

Gly Thr His Asp Gly Ser Val Tyr Phe Trp Ala Thr Pro Arg Gln Val
    370                 375                 380

Pro Ser Leu Gln His Ile Cys Arg Met Ser Ile Arg Arg Val Met Ser
385                 390                 395                 400

Thr Gln Glu Val Gln Lys Leu Pro Val Pro Ser Lys Ile Leu Ala Phe
                405                 410                 415

Leu Ser Tyr Arg Gly  *
            420
```

(2) INFORMATION FOR SEQ ID NO:15:

(i) SEQUENCE CHARACTERISTICS:
(A) LENGTH: 783 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:15:

```
CTGTCTTCCT CCGCAGCGCG AGGCTGGGTA CAGGGTCTAT TGTCTGTGGT TGACTCCGTA    60
CTTTGGTCTG AGGCCTTCGG GAGCTTTCCC GAGGCAGTTA GCAGAAGCCG CAGCGACCGC   120
CCCCGCCCGT CTCCTCTGTC CCTGGGCCCG GGAGACAAAC TTGGCGTCAC GCCCTCAGCG   180
GTCGCCACTC TCTTCTCTGT TGTTGGGTCC GCATCGTATT CCCGGAATCA GACGGTGCCC   240
CATAGATGGC CAGCTTTCCC CCGAGGGTCA ACGAGAAAGA GATCGTGAGA TCACGTACTA   300
```

TAGGTGAACT TTTAGCTCCT GCAGCTCCTT TTGACAAGAA ATGTGGTCGT GAAAATTGGA 360

CTGTTGCTTT TGCTCCAGAT GGTTCATACT TTGCTTGGTC ACAAGGACAT CGCACAGTAA 420

AGCTTGTTCC GTGGTCCCAG TGCCTTCAGA ACTTTCTCTT GCATGGCACC AAGAATGTTA 480

CCAATTCAAG CAGTTTAAGA TTGCCAAGAC AAAATAGTGA TGGTGGTCAG AAAAATAAGC 540

CTCGTGACAT ATTATAGACT GTGGAGATAT AGTCTGGAGT CTTGCTTTTG GGTCATCAGT 600

TCCAGAAAAA CAGAGTCGCT GTGTAAATAT AGAATGGCAT CGCTTCAGAT TTGGACAAGA 660

TCAGCTACTT CTTGCTACAG GGTTGAACAA TGGGCGTATC AAAATATGGG ATGTATATCA 720

GGAAACTCCT CCTTAACTTG GTAGATCATA CTGAAGTGGT CAGAGATTTA ACTTTTGCTC 780

CAG 783

(2) INFORMATION FOR SEQ ID NO:16:

(i) SEQUENCE CHARACTERISTICS:
(A) LENGTH: 1122 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:16:

CTCTGTATGT CTGAATGAAG CTATAACATT TGCCTTTTTA TTGCAGGTTT TCCTTTGGAA 60

TATGGATAAA TACACCATGA TACGGAAACT AGAAGGACAT CACCATGATG TGGTAGCTTG 120

TGACTTTTCT CCTGATGGAG CATTACTGGC TACTGCATCT TATGATACTC GAGTATATAT 180

CTGGGATCCA CATAATGGAG ACATTCTGAT GGAATTTGGG CACCTGTTTC CCCCACCTAC 240

TCCAATATTT GCTGGAGGAG CAAATGACCG GTGGGTACGA TCTGTATCTT TTAGCCATGA 300

TGGACTGCAT GTTGCAAGCC TTGCTGATGA TAAAATGGTG AGGTTCTGGA GAATTGATGA 360

GGATTATCCA GTGCAAGTTG CACCTTTGAG CAATGGTCTT TGCTGTGCCT TCTCTACTGA 420

TGGCAGTGTT TTAGCTGCTG GGACACATGA CGGAAGTGTG TATTTTTGGG CCACTCCACG 480

GCAGGTCCCT AGCCTGCAAC ATTTATGTCG CATGTCAATC CGAAGAGTGA TGCCCACCCA 540

AGAAGTTCAG GAGCTGCCGA TTCCTTCCAA GCTTTTGGAG TTTCTCTCGT ATCGTATTTA 600

GAAGATTCTG CCTTCCCTAG TAGTAGGGAC TGACAGAATA CACTTAACAC AAACCTCAAG 660

CTTTACTGAC TTCAATTATC TGTTTTTAAA GACGTAGAAG ATTTATTTAA TTTGATATGT 720

TCTTGTACTG CATTTTGATC AGTTGAGCTT TTAAAATATT ATTTATAGAC AATAGAAGTA 780

TTTCTGAACA TATCAAATAT AAATTTTTTT AAAGATCTAA CTGTGAAAAC ATACATACCT 840

GTACATATTT AGATATAAGC TGCTATATGT TGAATGGACC CTTTTGCTTT TCTGATTTTT 900

AGTTCTGACA TGTATATATT GCTTCAGTAG AGCCACAATA TGTATCTTTG CTGTAAAGTG 960

CAAGGAAATT TTAAATTCTG GGACACTGAG TTAGATGGTA AATACTGACT TACGAAAGTT 1020

GAATTGGGTG AGGCGGGCAA ATCACCTGAG GTCAGCAGTT TGAGACTAGC CTGGCAAACA 1080

```
TGATGAAACC CTGTCTCTAC TAAAAATACA AAAAAAAAAA AA                        1122

(2) INFORMATION FOR SEQ ID NO:17:

     (i) SEQUENCE CHARACTERISTICS:
          (A) LENGTH: 2544 base pairs
          (B) TYPE: nucleic acid
          (C) STRANDEDNESS: single
          (D) TOPOLOGY: linear

    (ii) MOLECULE TYPE: DNA


    (ix) FEATURE:
          (A) NAME/KEY: CDS
          (B) LOCATION: 423..2030


    (xi) SEQUENCE DESCRIPTION: SEQ ID NO:17:

CGGCACGAGC CGGGCTCCGT CCGGAGGAAG CGAGGCTGCG CCGCCGGCCC GGCAGGAGCG       60

GAGGACGGGA MGCGCGGGCG GTCGCGCTCG CCCTGTCGCT GACTGCGCTG CCCCGGCCCA      120

TCCTTGCCTG GCCGCAGGTG CCCTGGATGA GGCCGCCGCG CGTGTCCCGG CCGCTGAGTG      180

TCCCCCGCGG TCGCCCGGCG CCTGCCCTCA AGCGGCCGCC TCTCCTTGCC CGGGTCCCCG      240

TTTTCCCCCG GCGCAGTCCT CCTCCGGTGG GCGCCTCCGC ACCTCGGCGC AGGCGGCACG      300

GCCCTCGGGC CGGGATGGAT CCGCCGGGAA GAGGAAGACA AGCCGGGGCG TTGAGCCCCT      360

GCGCACGGTG CCGCCGCGCG TAGTGGGAGC TTACTCGCAG TAGGCTCTCG CTCTTCTAAT      420

CA ATG GAT AAA GTG GGG AAA ATG TGG AAC AAC TTA AAA TAC AGA TGC         467
   Met Asp Lys Val Gly Lys Met Trp Asn Asn Leu Lys Tyr Arg Cys
     1               5                  10                  15

CAG AAT CTC TTC AGC CAC GAG GGA GGA AGC CGT AAT GAG AAC GTG GAG        515
Gln Asn Leu Phe Ser His Glu Gly Gly Ser Arg Asn Glu Asn Val Glu
                 20                  25                  30

ATG AAC CCC AAC AGA TGT CCG TCT GTC AAA GAG AAA AGC ATC AGT CTG        563
Met Asn Pro Asn Arg Cys Pro Ser Val Lys Glu Lys Ser Ile Ser Leu
             35                  40                  45

GGA GAG GCA GCT CCC CAG CAA GAG AGC AGT CCC TTA AGA GAA AAT GTT        611
Gly Glu Ala Ala Pro Gln Gln Glu Ser Ser Pro Leu Arg Glu Asn Val
         50                  55                  60

GCC TTA CAG CTG GGA CTG AGC CCT TCC AAG ACC TTT TCC AGG CGG AAC        659
Ala Leu Gln Leu Gly Leu Ser Pro Ser Lys Thr Phe Ser Arg Arg Asn
     65                  70                  75

CAA AAC TGT GCC GCA GAG ATC CCT CAA GTG GTT GAA ATC AGC ATC GAG        707
Gln Asn Cys Ala Ala Glu Ile Pro Gln Val Val Glu Ile Ser Ile Glu
 80                  85                  90                  95

AAA GAC AGT GAC TCG GGT GCC ACC CCA GGA ACG AGG CTT GCA CGG AGA        755
Lys Asp Ser Asp Ser Gly Ala Thr Pro Gly Thr Arg Leu Ala Arg Arg
                100                 105                 110

GAC TCC TAC TCG CGG CAC GCC CCG TGG GGA GGA AAG AAG AAA CAT TCC        803
Asp Ser Tyr Ser Arg His Ala Pro Trp Gly Gly Lys Lys Lys His Ser
            115                 120                 125
```

```
TGT TCC ACA AAG ACC CAG AGT TCA TTG GAT ACC GAG AAA AAG TTT GGT      851
Cys Ser Thr Lys Thr Gln Ser Ser Leu Asp Thr Glu Lys Lys Phe Gly
        130                 135                 140

AGA ACT CGA AGC GGC CTT CAG AGG CGA GAG CGG CGC TAT GGA GTC AGC      899
Arg Thr Arg Ser Gly Leu Gln Arg Arg Glu Arg Arg Tyr Gly Val Ser
    145                 150                 155

TCC ATG CAG GAC ATG GAC AGC GTT TCT AGC CGC GCG GTC GGG AGC CGC      947
Ser Met Gln Asp Met Asp Ser Val Ser Ser Arg Ala Val Gly Ser Arg
160                 165                 170                 175

TCC CTG AGG CAG AGG CTC CAG GAC ACG GTG GGT TTG TGT TTT CCC ATG      995
Ser Leu Arg Gln Arg Leu Gln Asp Thr Val Gly Leu Cys Phe Pro Met
                180                 185                 190

AGA ACT TAC AGC AAG CAG TCA AAG CCA CTC TTT TCC AAT AAA AGA AAA     1043
Arg Thr Tyr Ser Lys Gln Ser Lys Pro Leu Phe Ser Asn Lys Arg Lys
            195                 200                 205

ATA CAT CTT TCT GAA TTA ATG CTG GAG AAA TGC CCT TTT CCT GCT GGC     1091
Ile His Leu Ser Glu Leu Met Leu Glu Lys Cys Pro Phe Pro Ala Gly
        210                 215                 220

TCG GAT TTA GCA CAA AAG TGG CAT TTG ATT AAA CAG CAT ACC GCC CCT     1139
Ser Asp Leu Ala Gln Lys Trp His Leu Ile Lys Gln His Thr Ala Pro
    225                 230                 235

GTG AGC CCA CAC TCA ACA TTT TTT GAT ACA TTT GAT CCA TCA CTG GTG     1187
Val Ser Pro His Ser Thr Phe Phe Asp Thr Phe Asp Pro Ser Leu Val
240                 245                 250                 255

TCT ACA GAA GAT GAA GAA GAT AGG CTT CGC GAG AGA AGA CGG CTT AGT     1235
Ser Thr Glu Asp Glu Glu Asp Arg Leu Arg Glu Arg Arg Arg Leu Ser
                260                 265                 270

ATC GAA GAA GGG GTG GAT CCC CCT CCC AAC GCA CAA ATA CAC ACC TTT     1283
Ile Glu Glu Gly Val Asp Pro Pro Pro Asn Ala Gln Ile His Thr Phe
            275                 280                 285

GAA GCT ACT GCA CAG GTC AAC CCA TTG TAT AAG CTG GGA CCA AAG TTA     1331
Glu Ala Thr Ala Gln Val Asn Pro Leu Tyr Lys Leu Gly Pro Lys Leu
        290                 295                 300

GCT CCT GGG ATG ACA GAG ATA AGT GGA GAT GGT TCT GCA ATT CCA CAA     1379
Ala Pro Gly Met Thr Glu Ile Ser Gly Asp Gly Ser Ala Ile Pro Gln
    305                 310                 315

GCS AAT TGT GAC TCA GAA GAG GAT TCA ACC ACC CTA TGT CTG CAG TCA     1427
Xaa Asn Cys Asp Ser Glu Glu Asp Ser Thr Thr Leu Cys Leu Gln Ser
320                 325                 330                 335

CGG AGG CAG AAG CAG CGC CAG GTG TCC GGG GAC AGC CAC GCG CAC GTT     1475
Arg Arg Gln Lys Gln Arg Gln Val Ser Gly Asp Ser His Ala His Val
                340                 345                 350

AGC AGA CAG GGA GCT TGG AAA GTT CAT ACG CAG ATC GAT TAC ATA CAC     1523
Ser Arg Gln Gly Ala Trp Lys Val His Thr Gln Ile Asp Tyr Ile His
            355                 360                 365

TGC CTC GTG CCA GAT TTG CTT CAG ATC ACA GGG AAT CCC TGT TAC TGG     1571
Cys Leu Val Pro Asp Leu Leu Gln Ile Thr Gly Asn Pro Cys Tyr Trp
        370                 375                 380

GGC GTG ATG GAC CGA TAC GAG GCC GAA GCC CTT CTA GAA GGG AAA CCG     1619
Gly Val Met Asp Arg Tyr Glu Ala Glu Ala Leu Leu Glu Gly Lys Pro
    385                 390                 395
```

```
GAA GGC ACG TTC TTG CTC AGG GAC TCT GCA CAG GAG GAC TAC CTC TTC      1667
Glu Gly Thr Phe Leu Leu Arg Asp Ser Ala Gln Glu Asp Tyr Leu Phe
400                 405                 410                 415

TCT GTG AGC TTC CGC CGC TAC AAC AGG TCT CTG CAC GCC CGG ATC GAG      1715
Ser Val Ser Phe Arg Arg Tyr Asn Arg Ser Leu His Ala Arg Ile Glu
                420                 425                 430

CAG TGG AAC CAC AAC TTC AGC TTC GAT GCC CAT GAC CCC TGC GTG TTT      1763
Gln Trp Asn His Asn Phe Ser Phe Asp Ala His Asp Pro Cys Val Phe
            435                 440                 445

CAC TCC TCC ACW GTC ACG GGG CTT CTC GAA CAC TAT AAA GAC CCC AGC      1811
His Ser Ser Xaa Val Thr Gly Leu Leu Glu His Tyr Lys Asp Pro Ser
        450                 455                 460

TCT TGC ATG TTT TTT GAA CCG TTG CTA ACG ATA TCA CTG AAT AGA ACT      1859
Ser Cys Met Phe Phe Glu Pro Leu Leu Thr Ile Ser Leu Asn Arg Thr
    465                 470                 475

TTC CCT TTC AGC CTG CAG TAT ATC TGC CGC GCA GTG ATC TGC AGA TGC      1907
Phe Pro Phe Ser Leu Gln Tyr Ile Cys Arg Ala Val Ile Cys Arg Cys
480                 485                 490                 495

ACT ACG TAT GAT GGG ATT GAC GGG CTC CCG CTA CCG TCG ATG TTA CAG      1955
Thr Thr Tyr Asp Gly Ile Asp Gly Leu Pro Leu Pro Ser Met Leu Gln
                500                 505                 510

GAT TTT TTA AAA GAG TAT CAT TAT AAA CAA AAA GTT AGG GTT CGC TGG      2003
Asp Phe Leu Lys Glu Tyr His Tyr Lys Gln Lys Val Arg Val Arg Trp
            515                 520                 525

TTA GAA CGA GAR CCA GTC AAA GCA AAG TAACTCCTGT CCCCAAAGGG            2050
Leu Glu Arg Xaa Pro Val Lys Ala Lys
        530                 535

CACTAACTAA GTCTGCTCCT CCCGTGCATC MGAACTGCAC CCATAGGRAG GCAGTCAGCT    2110

GCTAGGATTT CCCACCCAGA ATGGGAGCTT AGTCATTAGC CTCTGCCCTA TGGGGTCCGC    2170

TGTTCCTCAG ACAAAGGTGC CTAGGGACAG CAAGATGGCT TGCAGGTGTT CGGTGGGCTG    2230

TGACAACTGA GGGAGGCAAC TCTGGGGCAT TTGCTATGAA GAATTCTATT TCTTACCGAA    2290

GAACAAATTA TTAATATTGG ATGGGTATTT CAATAGTGTG ACTAATGTTT GAAATTATTT    2350

TTTCTAAGAA TTTTTCTATA ACCTTCAGAA AAAGTAGTGA TGTTTGTAGT TACTATAAAT    2410

CAAGCTTTGA AAGTTCAAAA CAAACAAGTT AAATAAAAGA CTACCTTCCT TTTAGAGAAA    2470

ACAAATGCAA GTTTTCCCAG CCACAGGCAT TGTGCACTGT TAATGTTAGC TTGTTATCAG    2530

CTCCTTTCTC CTCC                                                      2544
```

(2) INFORMATION FOR SEQ ID NO:18:

(i) SEQUENCE CHARACTERISTICS:
(A) LENGTH: 536 amino acids
(B) TYPE: amino acid
(D) TOPOLOGY: linear

(ii) MOLECULE TYPE: protein

(xi) SEQUENCE DESCRIPTION: SEQ ID NO:18:

```
Met Asp Lys Val Gly Lys Met Trp Asn Asn Leu Lys Tyr Arg Cys Gln
  1               5                  10                  15
```

```
Asn Leu Phe Ser His Glu Gly Gly Ser Arg Asn Glu Asn Val Glu Met
            20                  25                  30

Asn Pro Asn Arg Cys Pro Ser Val Lys Glu Lys Ser Ile Ser Leu Gly
        35                  40                  45

Glu Ala Ala Pro Gln Gln Glu Ser Ser Pro Leu Arg Glu Asn Val Ala
    50                  55                  60

Leu Gln Leu Gly Leu Ser Pro Ser Lys Thr Phe Ser Arg Arg Asn Gln
65                  70                  75                  80

Asn Cys Ala Ala Glu Ile Pro Gln Val Val Glu Ile Ser Ile Glu Lys
                85                  90                  95

Asp Ser Asp Ser Gly Ala Thr Pro Gly Thr Arg Leu Ala Arg Arg Asp
            100                 105                 110

Ser Tyr Ser Arg His Ala Pro Trp Gly Gly Lys Lys Lys His Ser Cys
        115                 120                 125

Ser Thr Lys Thr Gln Ser Ser Leu Asp Thr Glu Lys Lys Phe Gly Arg
    130                 135                 140

Thr Arg Ser Gly Leu Gln Arg Arg Glu Arg Arg Tyr Gly Val Ser Ser
145                 150                 155                 160

Met Gln Asp Met Asp Ser Val Ser Ser Arg Ala Val Gly Ser Arg Ser
                165                 170                 175

Leu Arg Gln Arg Leu Gln Asp Thr Val Gly Leu Cys Phe Pro Met Arg
            180                 185                 190

Thr Tyr Ser Lys Gln Ser Lys Pro Leu Phe Ser Asn Lys Arg Lys Ile
        195                 200                 205

His Leu Ser Glu Leu Met Leu Glu Lys Cys Pro Phe Pro Ala Gly Ser
    210                 215                 220

Asp Leu Ala Gln Lys Trp His Leu Ile Lys Gln His Thr Ala Pro Val
225                 230                 235                 240

Ser Pro His Ser Thr Phe Phe Asp Thr Phe Asp Pro Ser Leu Val Ser
                245                 250                 255

Thr Glu Asp Glu Glu Asp Arg Leu Arg Glu Arg Arg Arg Leu Ser Ile
            260                 265                 270

Glu Glu Gly Val Asp Pro Pro Pro Asn Ala Gln Ile His Thr Phe Glu
        275                 280                 285

Ala Thr Ala Gln Val Asn Pro Leu Tyr Lys Leu Gly Pro Lys Leu Ala
    290                 295                 300

Pro Gly Met Thr Glu Ile Ser Gly Asp Gly Ser Ala Ile Pro Gln Xaa
305                 310                 315                 320

Asn Cys Asp Ser Glu Glu Asp Ser Thr Thr Leu Cys Leu Gln Ser Arg
                325                 330                 335

Arg Gln Lys Gln Arg Gln Val Ser Gly Asp Ser His Ala His Val Ser
            340                 345                 350

Arg Gln Gly Ala Trp Lys Val His Thr Gln Ile Asp Tyr Ile His Cys
        355                 360                 365

Leu Val Pro Asp Leu Leu Gln Ile Thr Gly Asn Pro Cys Tyr Trp Gly
```

```
370                 375                 380

Val Met Asp Arg Tyr Glu Ala Glu Ala Leu Leu Glu Gly Lys Pro Glu
385                 390                 395                 400

Gly Thr Phe Leu Leu Arg Asp Ser Ala Gln Glu Asp Tyr Leu Phe Ser
                405                 410                 415

Val Ser Phe Arg Arg Tyr Asn Arg Ser Leu His Ala Arg Ile Glu Gln
            420                 425                 430

Trp Asn His Asn Phe Ser Phe Asp Ala His Asp Pro Cys Val Phe His
        435                 440                 445

Ser Ser Xaa Val Thr Gly Leu Leu Glu His Tyr Lys Asp Pro Ser Ser
    450                 455                 460

Cys Met Phe Phe Glu Pro Leu Leu Thr Ile Ser Leu Asn Arg Thr Phe
465                 470                 475                 480

Pro Phe Ser Leu Gln Tyr Ile Cys Arg Ala Val Ile Cys Arg Cys Thr
                485                 490                 495

Thr Tyr Asp Gly Ile Asp Gly Leu Pro Leu Pro Ser Met Leu Gln Asp
            500                 505                 510

Phe Leu Lys Glu Tyr His Tyr Lys Gln Lys Val Arg Val Arg Trp Leu
        515                 520                 525

Glu Arg Xaa Pro Val Lys Ala Lys
    530                 535
```

(2) INFORMATION FOR SEQ ID NO:19:

(i) SEQUENCE CHARACTERISTICS:
  (A) LENGTH: 1221 base pairs
  (B) TYPE: nucleic acid
  (C) STRANDEDNESS: single
  (D) TOPOLOGY: linear

(ii) MOLECULE TYPE: DNA

(xi) SEQUENCE DESCRIPTION: SEQ ID NO:19:

```
GATTAAACAG CATACAGCTC CTGTGAGCCC ACATTCAACA TTTTTTGATA CTTTGATCCA    60
TCTTTGGTTT CTACAGAAGA TGAAGAAGAT AGGCTTAGAG AGAGAAGGCG GCTTAGTATT   120
GAAGAAGGGG TTGATCCCCC TCCCAATGCA CAAATACATA CATTTGAAGC TACTGCACAG   180
GTTAATCCAT TATTAAACTG GGACCAAAAT TAGCTCCTGG AATGACTGAA ATAAGTGGGG   240
ACAGTTCTGC AATTCCACAA GCTAATTGTG ACTCGGAAGA GGATACAACC ACCCTGTGTT   300
GCAGTCACGG AGGCAGAAGC AGCGTCAGAT ATCTGGAGAC AGCCATACCC ATGTTAGCAG   360
ACAGGGAGCT TGGAAAGTCC ACACACAGAT TGATTACATA CACTGCTTCG TGCCTGATTT   420
GCTTCAAATT ACAGGGAATC CCTGTTACTG GGAGTGATGG GACCGTTATG AAGCAGAAGC   480
CCTTCTCGAA GGGAAACCTG AAGGCACGTT TTTGCTCAGG GACTCTGCGC AAGAGGACTA   540
CTTCTTCTCT GTGAGCTTCC GCCGATACAA CAGATCCCTG CATGCCCGAA TTGAGCAGTG   600
GAATCACAAC TTTAGTTTCG ACGCCCATGA CCCGTGTGTA TTTCACTCCT CCACTGTAAC   660
```

```
GGGACTTTTA GAACATTATA AAGATCCCAG TTCGTGCATG TTTTTTGAAC CATTGCTTAC    720
TATATCACTA AATAGGACTT TCCCTTTTAG CCTGCAGTAT ATCTGTCGCG CGGTAATCTG    780
CAGGTGCACT ACGTATGATG GAATTGATGG GCTCCCTCTA CCCTCAATGT TACAGGATTT    840
TTTAAAAGAG TATCATTATA AACAAAAAGT TAGAGTTCGC TGGTTGGAAC GAGAACCAGT    900
CAAGGCAAAG TAAACTCTCC GGTCCCCAAA GGGTGTTAAC TAGGTCCGCT TTCATGTGCA    960
TCAGACAGTA CACCTATAGC AAGCACACGT AGCAGTGTTA GGCTTTTTCA TACAGTATGT   1020
AAGCTTAGTG TTAGTATCTG TCAGATGCTA CCTGCTGTTA CTTATTCAGA TAAACATGGT   1080
GCCTATTGGA ACAATAGCGG ATAGAGCTAC AGGTGTTCAG TAAGACTACA AAAACATTTT   1140
GCCTATTTCG CTAACAGTTT GGTTTTTAAT GGCTGTGGTA TTTGAGTGAG GCAACTCTGG   1200
GGCATTTGTT ATGAAGAAAT G                                             1221
```

(2) INFORMATION FOR SEQ ID NO:20:

(i) SEQUENCE CHARACTERISTICS:
(A) LENGTH: 2369 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 116..1330

(xi) SEQUENCE DESCRIPTION: SEQ ID NO:20:

```
GGCACGAGGC GGTGGTGGCG GCGGCGGGCG CGGCCGCGGC GGGGCGGGCG CGGAATGAAG     60

GCCCACGGCC CTGGGGGCTG AGGCGCCCGC CGCCTGGGGC GGGCCGCGCG TCCTC ATG    118
                                                             Met
                                                               1

GAG GCC GGA GAG GAG CCG CTG CTG CTG GCT GAA CTC AAG CCT GGG CGC    166
Glu Ala Gly Glu Glu Pro Leu Leu Leu Ala Glu Leu Lys Pro Gly Arg
              5                  10                  15

CCC CAC CAG TTC GAC TGG AAG TCA AGC TGC GAG ACC TGG AGC GTG GCC    214
Pro His Gln Phe Asp Trp Lys Ser Ser Cys Glu Thr Trp Ser Val Ala
         20                  25                  30

TTC TCG CCA GAC GGT TCC TGG TTC GCC TGG TCT CAA GGA CAC TGC GTG    262
Phe Ser Pro Asp Gly Ser Trp Phe Ala Trp Ser Gln Gly His Cys Val
     35                  40                  45

GTC AAG CTG GTC CCC TGG CCC TTA GAG GAA CAG TTC ATC CCT AAA GGA    310
Val Lys Leu Val Pro Trp Pro Leu Glu Glu Gln Phe Ile Pro Lys Gly
 50                  55                  60                  65

TTC GAA GCC AAG AGC CGA AGC AGC AAG AAT GAC CCA AAA GGA CGG GGC    358
Phe Glu Ala Lys Ser Arg Ser Ser Lys Asn Asp Pro Lys Gly Arg Gly
                 70                  75                  80

AGT CTG AAG GAG AAG ACG CTG GAC TGT GGC CAG ATT GTG TGG GGG CTG    406
```

```
Ser Leu Lys Glu Lys Thr Leu Asp Cys Gly Gln Ile Val Trp Gly Leu
            85                  90                  95

GCC TTC AGC CCG TGG CCC TCT CCA CCC AGC AGG AAA CTC TGG GCA CGT      454
Ala Phe Ser Pro Trp Pro Ser Pro Pro Ser Arg Lys Leu Trp Ala Arg
        100                 105                 110

CAC CAT CCC CAG GCG CCT GAT GTT TCT TGC CTG ATC CTG GCC ACA GGT      502
His His Pro Gln Ala Pro Asp Val Ser Cys Leu Ile Leu Ala Thr Gly
    115                 120                 125

CTC AAC GAT GGG CAG ATC AAG ATT TGG GAG GTA CAG ACA GGC CTC CTG      550
Leu Asn Asp Gly Gln Ile Lys Ile Trp Glu Val Gln Thr Gly Leu Leu
130                 135                 140                 145

CTT CTG AAT CTT TCT GGC CAC CAA GAC GTC GTG AGA GAT CTG AGC TTC      598
Leu Leu Asn Leu Ser Gly His Gln Asp Val Val Arg Asp Leu Ser Phe
                150                 155                 160

ACG CCC AGC GGC AGT TTG ATT TTG GTC TCT GCA TCC CGG GAT AAG ACA      646
Thr Pro Ser Gly Ser Leu Ile Leu Val Ser Ala Ser Arg Asp Lys Thr
            165                 170                 175

CTT CGA ATT TGG GAC CTG AAT AAA CAC GGT AAG CAG ATC CAG GTG TTA      694
Leu Arg Ile Trp Asp Leu Asn Lys His Gly Lys Gln Ile Gln Val Leu
        180                 185                 190

TCC GGC CAT CTG CAG TGG GTT TAC TGC TGC TCC ATC TCC CCT GAC TGT      742
Ser Gly His Leu Gln Trp Val Tyr Cys Cys Ser Ile Ser Pro Asp Cys
    195                 200                 205

AGC ATG CTG TGC TCT GCA GCT GGG GAG AAG TCG GTC TTT CTG TGG AGC      790
Ser Met Leu Cys Ser Ala Ala Gly Glu Lys Ser Val Phe Leu Trp Ser
210                 215                 220                 225

ATG CGG TCC TAC ACA CTA ATC CGG AAA CTA GAA GGC CAC CAA AGC AGT      838
Met Arg Ser Tyr Thr Leu Ile Arg Lys Leu Glu Gly His Gln Ser Ser
                230                 235                 240

GTT GTC TCC TGT GAT TTC TCT CCT GAT TCA GCC TTG CTT GTC ACA GCT      886
Val Val Ser Cys Asp Phe Ser Pro Asp Ser Ala Leu Leu Val Thr Ala
            245                 250                 255

TCG TAT GAC ACC AGT GTG ATT ATG TGG GAC CCC TAC ACC GGC GCG AGG      934
Ser Tyr Asp Thr Ser Val Ile Met Trp Asp Pro Tyr Thr Gly Ala Arg
        260                 265                 270

CTG AGG TCA CTT CAT CAC ACA CAA CTT GAA CCC ACC ATG GAT GAC AGT      982
Leu Arg Ser Leu His His Thr Gln Leu Glu Pro Thr Met Asp Asp Ser
    275                 280                 285

GAC GTC CAC ATG AGC TCC CTG AGG TCC GTG TGC TTC TCA CCT GAA GGC     1030
Asp Val His Met Ser Ser Leu Arg Ser Val Cys Phe Ser Pro Glu Gly
290                 295                 300                 305

TTG TAT CTC GCT ACG GTG GCA GAT GAC AGG CTG CTC AGG ATC TGG GCT     1078
Leu Tyr Leu Ala Thr Val Ala Asp Asp Arg Leu Leu Arg Ile Trp Ala
                310                 315                 320

CTG GAA CTG AAG GCT CCG GTT GCC TTT GCT CCG ATG ACC AAT GGT CTT     1126
Leu Glu Leu Lys Ala Pro Val Ala Phe Ala Pro Met Thr Asn Gly Leu
            325                 330                 335

TGC TGC ACG TTC TTC CCA CAC GGT GGA ATT ATT GCC ACA GGG ACG AGA     1174
Cys Cys Thr Phe Phe Pro His Gly Gly Ile Ile Ala Thr Gly Thr Arg
        340                 345                 350

GAT GGC CAT GTC CAG TTC TGG ACA GCT CCC CGG GTC CTG TCC TCA CTG     1222
```

```
Asp Gly His Val Gln Phe Trp Thr Ala Pro Arg Val Leu Ser Ser Leu
    355                 360                 365

AAG CAC TTA TGC AGG AAA GCC CTC CGA AGT TTC CTG ACA ACG TAT CAA      1270
Lys His Leu Cys Arg Lys Ala Leu Arg Ser Phe Leu Thr Thr Tyr Gln
370                 375                 380                 385

GTC CTA GCA CTG CCA ATC CCC AAG AAG ATG AAA GAG TTC CTC ACA TAC      1318
Val Leu Ala Leu Pro Ile Pro Lys Lys Met Lys Glu Phe Leu Thr Tyr
                390                 395                 400

AGG ACT TTC TAGCAGTGCC GGCTCCCCCA CCTCCTGCAG CAGCAGCAGT              1367
Arg Thr Phe
        405

ACAAGGGACT GGCTAGGATG GAGTCAGGCA GCTCACACTG GACCAGTGTG GACCTTCCTT    1427

CCTCCCATGG CATGTGCAAG TAGGTCTGCG TGACCCCACT TCTGTGGTGC CGGCCTTACC    1487

TCGTCTTCAT CCGTGGTGAG CAGCCTTCGT CAGTCTAGTT GTGTTGAAGC CAAGTGCAGT    1547

TGTGGATGTT GCTGGGGTAA TAAAGGCAAG CGGGCTCCAG AGCCTCTCTG GTGGCGGCCA    1607

AGCCACACTC CCTTAACTGG GAAGTACCTG CCACGTAGGG CATTTCTGCT GCCTATTTCC    1667

AGCCAGCGGC TGCATGGTTT GAAGTTCCTC CGTTGTGGTC AGAAGAACTC TGGTGTTTGG    1727

TTCCCTGCTC AGCTGCGCGT GGACTGGGCT GAGCTCCTCA CCATACACTA GTGCCGGCTT    1787

TTGTTTCCTG TAAACAGTGG TTGCATGTGT AGAGAAGTAA CAAGCGAGTA TTCAGATCAT    1847

ACGAGGAGGC GTTCCTCGGT GCATGACGGT CAGATGGCCA TTTATCAGCA TATTTATTTG    1907

TATTTTCTCA GCACATAGTA AGGTACAACT GTGTTTTCTC AATTGTCTCG AAAAAACAGA    1967

GTTCTTAAGT GGCCCAGTTG TGGAGCCAAG TCTAAGTCGT GTGGAGTCAG TGCTGACATC    2027

ACTGGCTTGT GCTGTCTGTC ACATGTGTTT GTCTCTGCTG CTTGACCTCA TGGGATGTAC    2087

CCTCCAGTTC AACTGCCCAA AACAGACAGC CCCTTCCAAG CACCGTTCTT TGACAGCGGT    2147

AGCAGCTACC TATTCAAGAC GCCTCACACA AAATCTGCCT TAGAAAGTTA ATATATTTTA    2207

AATTATTTTA AAAGAAACTC AACATCTTAT TCTTTGGCCT TTCTTAATTG ATGCTTTATG    2267

GAGGCAGTGT TAACATTGTA CAGTGTATGC ATAGAGGAGT CTCCTCTATT TGAAGAACAA    2327

TGCAAAATGA GGCTTTCATT GAAGGGAAAA AAAAAAAAAA AA                       2369
```

(2) INFORMATION FOR SEQ ID NO:21:

(i) SEQUENCE CHARACTERISTICS:
(A) LENGTH: 404 amino acids
(B) TYPE: amino acid
(D) TOPOLOGY: linear

(ii) MOLECULE TYPE: protein

(xi) SEQUENCE DESCRIPTION: SEQ ID NO:21:

```
Met Glu Ala Gly Glu Glu Pro Leu Leu Leu Ala Glu Leu Lys Pro Gly
  1               5                  10                  15

Arg Pro His Gln Phe Asp Trp Lys Ser Ser Cys Glu Thr Trp Ser Val
             20                  25                  30

Ala Phe Ser Pro Asp Gly Ser Trp Phe Ala Trp Ser Gln Gly His Cys
```

35 40 45

Val Val Lys Leu Val Pro Trp Pro Leu Glu Glu Gln Phe Ile Pro Lys
50 55 60

Gly Phe Glu Ala Lys Ser Arg Ser Ser Lys Asn Asp Pro Lys Gly Arg
65 70 75 80

Gly Ser Leu Lys Glu Lys Thr Leu Asp Cys Gly Gln Ile Val Trp Gly
85 90 95

Leu Ala Phe Ser Pro Trp Pro Ser Pro Pro Ser Arg Lys Leu Trp Ala
100 105 110

Arg His His Pro Gln Ala Pro Asp Val Ser Cys Leu Ile Leu Ala Thr
115 120 125

Gly Leu Asn Asp Gly Gln Ile Lys Ile Trp Glu Val Gln Thr Gly Leu
130 135 140

Leu Leu Leu Asn Leu Ser Gly His Gln Asp Val Val Arg Asp Leu Ser
145 150 155 160

Phe Thr Pro Ser Gly Ser Leu Ile Leu Val Ser Ala Ser Arg Asp Lys
165 170 175

Thr Leu Arg Ile Trp Asp Leu Asn Lys His Gly Lys Gln Ile Gln Val
180 185 190

Leu Ser Gly His Leu Gln Trp Val Tyr Cys Cys Ser Ile Ser Pro Asp
195 200 205

Cys Ser Met Leu Cys Ser Ala Ala Gly Glu Lys Ser Val Phe Leu Trp
210 215 220

Ser Met Arg Ser Tyr Thr Leu Ile Arg Lys Leu Glu Gly His Gln Ser
225 230 235 240

Ser Val Val Ser Cys Asp Phe Ser Pro Asp Ser Ala Leu Leu Val Thr
245 250 255

Ala Ser Tyr Asp Thr Ser Val Ile Met Trp Asp Pro Tyr Thr Gly Ala
260 265 270

Arg Leu Arg Ser Leu His His Thr Gln Leu Glu Pro Thr Met Asp Asp
275 280 285

Ser Asp Val His Met Ser Ser Leu Arg Ser Val Cys Phe Ser Pro Glu
290 295 300

Gly Leu Tyr Leu Ala Thr Val Ala Asp Asp Arg Leu Leu Arg Ile Trp
305 310 315 320

Ala Leu Glu Leu Lys Ala Pro Val Ala Phe Ala Pro Met Thr Asn Gly
325 330 335

Leu Cys Cys Thr Phe Phe Pro His Gly Gly Ile Ile Ala Thr Gly Thr
340 345 350

Arg Asp Gly His Val Gln Phe Trp Thr Ala Pro Arg Val Leu Ser Ser
355 360 365

Leu Lys His Leu Cys Arg Lys Ala Leu Arg Ser Phe Leu Thr Thr Tyr
370 375 380

Gln Val Leu Ala Leu Pro Ile Pro Lys Lys Met Lys Glu Phe Leu Thr
385 390 395 400

Tyr Arg Thr Phe

(2) INFORMATION FOR SEQ ID NO:22:

(i) SEQUENCE CHARACTERISTICS:
(A) LENGTH: 1246 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:22:

GACACTGCAT CGTCAAACTG ATCCCCTGGC CGTTGGAGGA GCAGTTCATC CCTAAAGGGT 60
TTGAAGCCAA AAGCCGAAGT AGCAAAAATG AGACGAAAGG GCGGGGCAGC CCAAAAGAGA 120
AGACGCTGGA CTGTGGTCAG ATTGTCTGGG GGCTGGCCTT CAGCCTGTGC TTTCCCCACC 180
CAGCAGGAAG CTCTGGGCAC GCCACCACCC CCAAGTGCCC GATGTCTCTT GCCTGGTTCT 240
TGCTACGGGA CTCAACGATG GGCAGATCAA GATCTGGGAG GTGCAGACAG GGCTCCTGCT 300
TTTGAATCTT TCCGGCCACC AAGATGTCGT GAGAGATCTG AGCTTCACAC CCAGTGGCAG 360
TTTGATTTTG GTCTCCGCGT CACGGGATAA GACTCTTCGC ATCTGGGACC TGAATAAACA 420
CGGTAAACAG ATTCAAGTGT TATCGGGCCA CCTGCAGTGG GTTTACTGCT GTTCCATCTC 480
CCCAGACTGC AGCATGCTGT GCTCTGCAGC TGGAGAGAAG TCGGTCTTTC TATGGAGCAT 540
GAGGTCCTAC ACGTTAATTC GGAAGCTAGA GGGCCATCAA AGCAGTGTTG TCTCTTGTGA 600
CTTCTCCCCC GACTCTGCCC TGCTTGTCAC GGCTTCTTAC GATACCAATG TGATTATGTG 660
GGACCCCTAC ACCGGCGAAA GGCTGAGGTC ACTCCACCAC ACCCAGGTTG ACCCCGCCAT 720
GGATGACAGT GACGTCCACA TTAGCTCACT GAGATCTGTG TGCTTCTCTC CAGAAGGCTT 780
GTACCTTGCC ACGGTGGCAG ATGACAGACT CCTCAGGATC TGGGCCCTGG AACTGAAAAC 840
TCCCATTGCA TTTGCTCCTA TGACCAATGG GCTTTGCTGG CACATTTTTT CCACATGGTG 900
GAGTCATTGC CACAGGGACA AGAGATGGCC ACGTCCAGTT CTGGACAGCT CCTAGGGTCC 960
TGTCCTCACT GAAGCACTTA TGCCGGAAAG CCCTTCGAAG TTTCCTAACA ACTTACCAAG 1020
TCCTAGCACT GCCAATCCCC AAGAAAATGA AAGAGTTCCT CACATACAGG ACTTTTTAAG 1080
CAACACCACA TCTTGTGCTT CTTTGTAGCA GGGTAAATCG TCCTGTCAAA GGGAGTTGCT 1140
GGAATAATGG GCCAAACATC TGGTCTTGCA TTGAAATAGC ATTTCTTTGG GATTGTGAAT 1200
AGAATGTAGC AAAACCAGAT TCCAGTGTAC TAGTCATGGA TTTTTC 1246

(2) INFORMATION FOR SEQ ID NO:23:

(i) SEQUENCE CHARACTERISTICS:
(A) LENGTH: 422 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:23:

ACCATGGTTC CAAGTCCTCT CCCCTGTGGT CAAGTTGCCC GAATGTTGGG CCCAAGTGCC 60
TTTTCCTCCT TGGGCCTCCC CTTCTGACCT GCAGGACAGT TTTCCGGAGC CCATTTGGTA 120
TGAGGTATTA ATTAGCCTTA ACTAAATTAC AGGGGACTCA GAGGCCGTGC TCCTGACCGA 180
TCCAGACACT ATTTTTTTTT TTTTTTTTTA ACAATGGTGT GCATGTGCAG GAAATGACAA 240
ATTTGTATGT CAGATTATAC AAGGATGTAT TCTTAAACCG CATGACTATT CAGATGGCTA 300
CTGAGTTATC AGTGGCCATT TATTAGCATC ATATTTATTT GTATTTTCTC AACAGATGTT 360
AAGGTACAAC TGTGTTTTTC TCGATTATCT AAAAACCATA GTACTTAAAT TGAAAAAAAA 420
AA 422

(2) INFORMATION FOR SEQ ID NO:24:

(i) SEQUENCE CHARACTERISTICS:
(A) LENGTH: 2019 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:24:

GGCACGAGGC GGGGTCAGGG CGGAGGCTGA GGACCAAGTA GGCATGGCGG AGGGCGGGAC 60
CGGCCCCGAT GGACGGGCCG GCCCGGGACC CGCAGGTCCT AATCTGAAGG AGTGGCTGAG 120
GGAGCAGTTC TGTGACCATC CACTGGAGCA CTGTGACGAT ACAAGACTCC ATGATGCAGC 180
CTATGTAGGG GACCTCCAGA CCCTCAGGAA CCTACTGCAA GAGGAGAGCT ACCGGAGCCG 240
CATCAATGAG AAGTCTGTCT GGTGCTGCGG CTGGCTTCCC TGCACACCAC TGAGGATCGC 300
AGCCACTGCA GGCCATGGGA ACTGTGTGGA CTTCCTCATA CGCAAAGGGG CCGAGGTGGA 360
CCTGGTGGAT GTCAAGGGGC AGACTGCCCT GTATGTGGCT GTAGTGAACG GGCACTTGGA 420
GAGCACTGAG ATCCTTTTGG AAGCTGGTGC TGATCCCAAC GGCAGCCGGC ACCACCGCAG 480
CACTCCTGTG TACCATGCCT YTCGTGTGGG TAGGGACGAC ATCCTGAAGG CTCTTATCAG 540
GTATGGGGCA GATGTTGATG TCAACCATCA TCTGAATTCT GACACCCGGC CCCCTTTTTC 600
ACGGCGGCTA ACCTCCTTGG TGGTCTGTCC TCTATACATC AGTGCTGCCT ACCATAACCT 660
TCAGTGCTTC AGGCTGCTCT TGCAGGCTGG GGCAAATCCT GACTTCAATT GCAATGGCCC 720
TGTCAACACC CAGGAGTTCT ACAGGGGATC CCCTGGGTGT GTCATGGATG CTGTCCTGCG 780
CCATGGCTGT GAAGCAGCCT TCGTGAGTCT GTTGGTAGAG TTTGGAGCCA ACCTGAACCT 840

```
GGTGAAGTGG GAATCCCTGG GCCCAGAGGC AAGAGGCAGA AGAAAGATGG ATCCTGAGGC     900
CTTGCAGGTC TTTAAAGAGG CCAGAAGTAT TCCCAGGACC TTGCTGAGTT TGTGCCGGGT     960
GGCTGTGAGA AGAGCTCTTG GCAAATACCG ACTGCATCTG GTTCCCTCGC TGCCGCTGCC    1020
AGACCCCATA AAGAAGTTTT TGCTTTATGA GTAGCATTCA CATGCAGTGC TGACTGCAAT    1080
GTGGAAGCCG ATCACCTGCA GTGAAAACTG ACACAGACTC TGGCATCCTG GGAACCATGG    1140
CCTGTGCTGC CAGCTTGATC CTTGGCTGTC AGTGAAGAAA AAACGGCTGT GTTCTCTTGG    1200
ACTGTGATTC TATCTCAGGT GCTTGGGCCA TCGAACGCTC CTTGAGTCAT TGTCAACTGA    1260
GAGGCACATA CAAACTTAAT TTTGTTCCTC TTCAGTCTCT CTGTTTTGGA TTCTTCCTGG    1320
CAATGTGTGC AGCATGGGCT GAGCCTGGTG ATTGCCCTAG TGGGGAAGGC TTTTTTCTCC    1380
AGGCTATGCA TCTATTTATG TTCCTACTTT GCAATTTATT GTTCTTTTAA GGCTTGATAT    1440
CAAAACAGAA AGAGGTTTGT TAAGAAAAGA TATAGGGAGA AAGGAATTCC GGTTCCGTGC    1500
ACTTGCTAGC CTGCTTTCCT TGCCTGGGTT TGTCTGTCTA TGCTGCCTGG TGCACATCCC    1560
TTCTCTTTGC TGCCACTGTT CTATTTTGGG AGTTGTCTTC CGTCTAAGAT GGCTTCTGGG    1620
GTTCTATCTT ATTGCACAGA GGTCCCAGAA CAGTGTTCAT AGGGCACCAT CTGCTCTGCC    1680
AAGGGTTTTC TGATGTCTTA CCCTGGGGAT CTTCAGACAG TGGTTACCTT TAGGAGACCC    1740
ACCTGGAACT AACCATTAAG TGACTGCCCA CATTCAGATC AGGGACCATC TTAATAGTAC    1800
TCACTGCCAG TCCTCACAAG AGAAGATGAC ACGGGTGCTC TCTTCAGACA CTCCCATACA    1860
GGAAGTTGGA AAATGTCTTG GTCACCTGGG TTGTTCCCAG GCTACAACTT CTTGGTGTTC    1920
CACTAARACC AGRATATCCT AGTTTTTTGG GTTGACTGTT CCCTCCCCAC TTTCCTTGAA    1980
NCCCAATGCC CNTTTGTKTN GGTTGCTTCC CTAAAAKTT                           2019
```

(2) INFORMATION FOR SEQ ID NO:25:

(i) SEQUENCE CHARACTERISTICS:
(A) LENGTH: 350 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:25:

```
Ala Arg Gly Gly Val Arg Ala Glu Ala Glu Asp Gln Val Gly Met Ala
1               5                   10                  15

Glu Gly Gly Thr Gly Pro Asp Gly Arg Ala Gly Pro Gly Pro Ala Gly
            20                  25                  30

Pro Asn Leu Lys Glu Trp Leu Arg Glu Gln Phe Cys Asp His Pro Leu
        35                  40                  45

Glu His Cys Asp Asp Thr Arg Leu His Asp Ala Ala Tyr Val Gly Asp
    50                  55                  60
```

```
Leu Gln Thr Leu Arg Asn Leu Leu Gln Glu Glu Ser Tyr Arg Ser Arg
65                  70                  75                  80

Ile Asn Glu Lys Ser Val Trp Cys Cys Gly Trp Leu Pro Cys Thr Pro
                85                  90                  95

Leu Arg Ile Ala Ala Thr Ala Gly His Gly Asn Cys Val Asp Phe Leu
            100                 105                 110

Ile Arg Lys Gly Ala Glu Val Asp Leu Val Asp Val Lys Gly Gln Thr
        115                 120                 125

Ala Leu Tyr Val Ala Val Val Asn Gly His Leu Glu Ser Thr Glu Ile
    130                 135                 140

Leu Leu Glu Ala Gly Ala Asp Pro Asn Gly Ser Arg His His Arg Ser
145                 150                 155                 160

Thr Pro Val Tyr His Ala Xaa Arg Val Gly Arg Asp Asp Ile Leu Lys
                165                 170                 175

Ala Leu Ile Arg Tyr Gly Ala Asp Val Asp Val Asn His His Leu Asn
            180                 185                 190

Ser Asp Thr Arg Pro Pro Phe Ser Arg Arg Leu Thr Ser Leu Val Val
        195                 200                 205

Cys Pro Leu Tyr Ile Ser Ala Ala Tyr His Asn Leu Gln Cys Phe Arg
    210                 215                 220

Leu Leu Leu Gln Ala Gly Ala Asn Pro Asp Phe Asn Cys Asn Gly Pro
225                 230                 235                 240

Val Asn Thr Gln Glu Phe Tyr Arg Gly Ser Pro Gly Cys Val Met Asp
                245                 250                 255

Ala Val Leu Arg His Gly Cys Glu Ala Ala Phe Val Ser Leu Leu Val
            260                 265                 270

Glu Phe Gly Ala Asn Leu Asn Leu Val Lys Trp Glu Ser Leu Gly Pro
        275                 280                 285

Glu Ala Arg Gly Arg Arg Lys Met Asp Pro Glu Ala Leu Gln Val Phe
    290                 295                 300

Lys Glu Ala Arg Ser Ile Pro Arg Thr Leu Leu Ser Leu Cys Arg Val
305                 310                 315                 320

Ala Val Arg Arg Ala Leu Gly Lys Tyr Arg Leu His Leu Val Pro Ser
                325                 330                 335

Leu Pro Leu Pro Asp Pro Ile Lys Lys Phe Leu Leu Tyr Glu
            340                 345                 350
```

(2) INFORMATION FOR SEQ ID NO:26:

(i) SEQUENCE CHARACTERISTICS:
(A) LENGTH: 419 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:26:

GCATCCATGG CGGAGGGCGG CAGCACGACG GGCGGGCAGG GCCGGGCTCC GCAGGTCGTA 60

ATCTGAAGGA GTGGCTGAGG GAGCAATTTT GTGATCATCC GCTGGAGCAC TGTGAGGACA 120

CGAGGCTCCA TGATGCAGCT TACGTCGGGG ACCTCCAGAC CCTCAGGAGC CTATTGCAAG 180

AGGAGAGCTA CCGGAGCCGC ATCAACGAGA AGTCTGTCTG GTGCTGTGGC TGGCTCCCCT 240

GCACACCGTT GCGAATCGCG GCCACTGCAG GCCATGGGAG CTGTGTGGAC TTCCTCATCC 300

GGAAGGGGGC CGAGGTGGAT CTGGTGGACG TAAAAGGACA GACGGCCCTG TATGTGGCTG 360

TGGTGAACGG GCACCTAGAG AGTACCCAGA TCCTTCTCGA AGCTGGCGCG GACCCCAAC 419

(2) INFORMATION FOR SEQ ID NO:27:

(i) SEQUENCE CHARACTERISTICS:
(A) LENGTH: 595 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:27:

GAGGAAGAAG AAAAGTGGAC CCTGAGGCCT TGCAGGTCTT TAAAGAGGCC AGAAGTGTTC 60

CCAGAACCTT GCTGTGTCTG TGCCGTGTGG CTGTGAGAAG AGCTCTTGGC AAAACCGGCT 120

TCATCTGATT CCTTCGCTGC CTCTGCCAGA CCCCATAAAG AAGTTTCTAC TCCATGAGTA 180

GACTCCAAGT GCTGCGGTTG ATTCCAGTGA GGGAGAAAGT GATCTGCAGG GAGGTGGACA 240

CCGAGCCCTG AGTGCTGTGC TGCTGCTGGT CTCCTGATGG CTGTTGCTGC AGAAGATGTC 300

CTCGTAGACT GTCATTGCTC CTCAGGTGCC TGGGCCGCTG AACAGTCCTT GGGTCATTGT 360

CAGCTGAGAG GCTTATACTA AAGTTATTAT TGTTTTTCCC AAGTTCTCTG TTCTGGATTT 420

TCAGTTGCAT ATTAATGTAA CGGGCCATGG GGTATGTACA TGTAGGGGCT GAGGTTGGAG 480

GCCTACTAAT TTCCTGTAGG GAAGACTCCC AGCACTTCTG GAACTGTGCT TCTCTTTATT 540

TTTCTACTTC TCAATTTGAT GGTTCGATTA AAGCCTTCTA GTATCTCAAT GAAAA 595

(2) INFORMATION FOR SEQ ID NO:28:

(i) SEQUENCE CHARACTERISTICS:
(A) LENGTH: 896 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 4..396

(xi) SEQUENCE DESCRIPTION: SEQ ID NO:28:

```
CTG ATG TCC GCA ATT CTG AAG GTT GGA CAC CAC TGC TGG CTG CCT GTG      48
    Met Ser Ala Ile Leu Lys Val Gly His His Cys Trp Leu Pro Val
      1               5                  10                  15

ACA TCC GCT GTC AAT CCC CAA AGG ATG CTG AGG CCA CCA CCA ACC GCT      96
Thr Ser Ala Val Asn Pro Gln Arg Met Leu Arg Pro Pro Pro Thr Ala
                 20                  25                  30

GTT TTC AAC TGT GCC GCT TGC TGC TGT CTG TGG GGG CAG ATG CTG ATG     144
Val Phe Asn Cys Ala Ala Cys Cys Cys Leu Trp Gly Gln Met Leu Met
             35                  40                  45

AAT ACA TAC CGT GTA GTT CAG CTT CCT GAG GAG GCC AAG GGC TTG GTG     192
Asn Thr Tyr Arg Val Val Gln Leu Pro Glu Glu Ala Lys Gly Leu Val
         50                  55                  60

CCA CCA GAG ATT CTA CAG AAG TAC CAT GGA TTC TAC TCT TCC CTC TTT     240
Pro Pro Glu Ile Leu Gln Lys Tyr His Gly Phe Tyr Ser Ser Leu Phe
     65                  70                  75

GCC TTG GTG AGG CAG CCC AGG TCG CTG CAG CAT CTC TGC CGT TGT GCG     288
Ala Leu Val Arg Gln Pro Arg Ser Leu Gln His Leu Cys Arg Cys Ala
 80                  85                  90                  95

CTC CGC AGT CAC CTG GAG GGC TGT CTG CCC CAT GCA CTA CCG CGC CTT     336
Leu Arg Ser His Leu Glu Gly Cys Leu Pro His Ala Leu Pro Arg Leu
                100                 105                 110

CCC CTG CCA CCG CGC ATG CTC CGC TTT CTG CAG CTG GAC TTT GAG GAT     384
Pro Leu Pro Pro Arg Met Leu Arg Phe Leu Gln Leu Asp Phe Glu Asp
            115                 120                 125

CTG CTC TAC TAGGCTTGCT GCCCTGTGAA CAAAGCAGAC CCCACCCCCA             433
Leu Leu Tyr
        130

CCCCAAGGGC ATCTCTCAGC AATGAATGAT GCAAGGCGGT CTGTCTTCAA GTCAGGAGTG   493

GACGCCTTGA TCCACACTTG AGAGAAGAGG CCAGATCAGC ACCYGGCTGG TAGTGATNGC   553

AGAGGGCACC TGTGCAGATC TGTGTGCGCA CTGGAAATCT CTAGGCTGAA GGCYAGAGCA   613

AATGGTGCAR GTGTTAGTCC TTGGGANGAG AGACAGANGG TGAGAAAGCA AGACAGAGGT   673

GAGAGTGCAC ATGTCAAGTG GTAGATTGCC TTAAAAGAAA GCTAAAAAAA GAAAAAGATT   733

CGGGCGAACT TCTTTAGGGG TAATGCTGCA GCGTGTTAAA CTGACTGACC AGCGTCCATA   793

TCTTTGGACC CTTCCCGGGT GAAAAAGCCC CTTCATCCTC CAGCGCTCCC CAAGGGTGCT   853

TAGCAATACC GGGTGCTTTT CTGCCGCAAA GTGAGTTACC AAA                     896
```

(2) INFORMATION FOR SEQ ID NO:29:

(i) SEQUENCE CHARACTERISTICS:
(A) LENGTH: 130 amino acids
(B) TYPE: amino acid
(D) TOPOLOGY: linear

(ii) MOLECULE TYPE: protein

(xi) SEQUENCE DESCRIPTION: SEQ ID NO:29:

```
Met Ser Ala Ile Leu Lys Val Gly His His Cys Trp Leu Pro Val Thr
  1               5                  10                  15
```

```
Ser Ala Val Asn Pro Gln Arg Met Leu Arg Pro Pro Pro Thr Ala Val
            20                  25                  30

Phe Asn Cys Ala Ala Cys Cys Cys Leu Trp Gly Gln Met Leu Met Asn
        35                  40                  45

Thr Tyr Arg Val Val Gln Leu Pro Glu Glu Ala Lys Gly Leu Val Pro
    50                  55                  60

Pro Glu Ile Leu Gln Lys Tyr His Gly Phe Tyr Ser Ser Leu Phe Ala
65                  70                  75                      80

Leu Val Arg Gln Pro Arg Ser Leu Gln His Leu Cys Arg Cys Ala Leu
                85                  90                  95

Arg Ser His Leu Glu Gly Cys Leu Pro His Ala Leu Pro Arg Leu Pro
            100                 105                 110

Leu Pro Pro Arg Met Leu Arg Phe Leu Gln Leu Asp Phe Glu Asp Leu
        115                 120                 125

Leu Tyr
    130
```

(2) INFORMATION FOR SEQ ID NO:30:

(i) SEQUENCE CHARACTERISTICS:
(A) LENGTH: 436 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:30:

```
GTGGGGGCGT CATCATGACC TCCTCTAGGG CTCTGCAACA TGACTCCTGT GGTGCAAATC    60
AACAAATTGT TCACTGATGA ATCCACAAGG ATCTCTGGGC CTACAACCAG GTCCTGGTCC   120
ACATGACTGT CGTCTTCGGA GAAGGCACCA CTCGCCCCCG GCAGGTACGG CTGACACCTC   180
CATGGGAGAA GACGTATCCA GGCAGCAGCT GCGCGGCCCT TCAAGAGGGC ACATCCCGTC   240
ATCTAAAGGC ACGGTGTACT GAAGGTAGTC CTGAGACATG AGTCCGATTA CTACAGGCAC   300
GTGTTCCTCC AGGTGGAGGC TCAGGTCCCC GGGTGAGCTG GGGCTGCAGC GGGACTCAGG   360
GCGCGGCTCT GGCTGCAGGT CTCGCAGCTC CCTGGGCTGT AGCTCCCGCA GATCCTTGCG   420
CACACCGTTG ACTGGT                                                   436
```

(2) INFORMATION FOR SEQ ID NO:31:

(i) SEQUENCE CHARACTERISTICS:
(A) LENGTH: 2180 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:31:

```
TTAATAGTAC CTACATAGTA GAAAATTATA ACTCCACTTT AAAACAATGT TTTCTTTCTA 60
TTCAAATCAA TTTAAAACTT TTTATAAACA TTAATGTTGC AAGAGAATCC AGTCCATTTA 120
TGAAAATTAG TTGACAATCA AGTTCACCCA AGAAAATGTT GACTAAGCTA AAGAAATCAC 180
AGATAAAACA TTTTACCAAA AGGATAGGTA ACACACAAAA AAATGCTATC ACAGGAAGCT 240
ATGATCATCT AATATTTCTT TAATAATAAT TCTAGTTCCA TAGGTTTTCA TGTTATGCCA 300
ATTTGTACCC GAGTTTAATT ACAGAAAAGG CAACAATTTC TAAATTGGTG GTATACATTT 360
CTTTACAATT TTTTAATGTA AGGCCATTTA TTAAAATAGA CAAACTAGAA GATGAAAACG 420
AAGGCAACAG AAAAATTCAA CTTTTCACAA CCAAAAGAAT TAGCACAACC TTAGAAATAA 480
TTTAGAAAAA AGTGTTGTTA AAAGATATGT TGCAGATCTC CGTTCCATTA CCCAAGATTA 540
TGTCAATTCA CGATTCTAAA TAAATCTTTT TAAAGTAAGA GATTAAAAAC TCATCTTCAG 600
TGTATATGTA AATTCCGTGG TTTTATCACA CAGGTATGTT TATTCAACAC TGCTTTGGAA 660
ATGGACCATT TAAAAGGACA TGGCAATTTC CATTCTGTTA AGTTTCATTC AACCTTTACT 720
TAGGGGTTGA TTACCACATG AAATGTGCTT TTAATGCATA AAAATCACAG TGGATTAGCC 780
AGCAAAAGGG ACTGGGCGGG GGGGGCATTG AGGAGAATTT GATAATTCAC ATTGTGATTA 840
TTCTGCACAT TGATGAAACA TAATTCACAC CTCTAAAACC TCAAGACTTC CCTTTTTTAA 900
AGAACCAAAA TAAACCCAAG ACACCTTGCT GACACTTCCC CACCCCTAAA CAAACTGATG 960
ACTCTTTTAC ACATAAAACT GAAATAGTTA TGGCAGCAAA AGATTTTGAT GGCAATGAAA 1020
GTTTGTAAAC TGTATTTCAA TCTCTTGTTC TTATTCCCAA AGTGCAAGAT GCAGGGTTCT 1080
CAATCTTTCA GTAGTGCTTC TCCTGTAAAT AATCCTTCAT TTTGTTTGGC AAAGGCAGTT 1140
TCTGAATTAA GTCTATTCTG GTATACTGAC GTATAACAAA ACGACACAGG TACTGCAACG 1200
AGCGCACCTA TGAACCCCGG AACACTGGTT GGCAAGTTCT GACGGAAGTG CAGATTCCAG 1260
GCAGCGAGAC CTTGAATAAC AAAAAGCTCC CATTTTCAGA GTCCCTGATT GAATGCTCCA 1320
ATTAGATCAA CTATGGACGT ATGTCCTTCC ACATCGGCTG TTCATAAAAG CTAAACCTAC 1380
CATTTGAGTG CTCAATTCTA GTGTGAAGTG TTTTACCATG GGAGCGAAAG TCACAGCTTA 1440
AAAGGTAACG GTCGTCAGAA CTGTCCCGAA CAAGAAAAGA ACCATCTGGC ACGTTTGCTA 1500
GCTTCCCTTC TGCCTCCCAA CGTGTGATTG GTCCCCAGTA CCATCCTTGC TTTGCAAGTT 1560
TTTTCAGCTC CTCTGTAAGG CTTGTCACAA CCATGGGACC ACTACTTTGC ACTGAGTCAT 1620
AAACTCTTGC AACCCCAGGA GCAGAGTTCG GATCAAAATT CAAATGACAG CGCATAACTT 1680
TCAGCCACGT GGGGCTTTCT GTCCAGTGAG TCCACTGAAA GTTCCCCTTT GGGATTTGGA 1740
TTATTCCTGC ATTGGAGTAA CCAATGGTGA AGATTGGAGG GACATCCATC GTGAACCCGC 1800
TCTCCGGGGT TCTGCAACAT GACTCCCGTG GTGCCAATCA ACAAGCCATT CACCGGACTG 1860
ATCCACGAAG ATCTCTGGGG CGACAACTAG GTCCTGGTCT ACCTGACTCT CATCCTCGGG 1920
GAAAGCGCGC CCTCCCACTT GAGGAGGAAC CGCAGAGACT TCCATGGGAG AAGAGCTGTC 1980
```

CAGACAATAG CTCCGTGATC CTTCCAAAGG ATACATCCCC TCATCTAAAG GCACAGTATA 2040
CTGAATGTAG TCCTGAGGCA TAAGTCCAAT AACGACAGGC ACATGTTCAT CCAGGTGAAG 2100
ATGCAGGTCT CCATTATGAG AAGCCGAGCT CTTCAGTGAA TTGGCTTGCT CCTGGCACGT 2160
GGTCTCAGAC TGGAGGTCGT 2180

(2) INFORMATION FOR SEQ ID NO:32:

(i) SEQUENCE CHARACTERISTICS:
(A) LENGTH: 2649 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:32:

GGCACGAGGC TGTGTCCAGC ACACAGAGAG GGCCCGGCCA TCTGCTTTGG TTCAGAGCCC 60
TGTGTCTGTC TGTCACTTAG ACTCTTCCTC CCGGCTCGCA GCTCACCCTC CATCCTCCTT 120
ACTGGCTCCA GCATGACTCG CTTCTCTTAT GCAGAGTACT TTGCTCTGTT TCACTCTGGC 180
TCTGCACCTT CCAGGTCCCC TTCGTCTCCC GAGAACCCAC CGGCCCGCGC ACCCCTGGGT 240
CTGTTCCAAG GGGTCATGCA GAAGTATAGC AGCAACCTGT TCAAGACCTC CCAGATGGCG 300
GCTATGGACC CCGTGCTGAA GGCCATCAAG GAAGGGGATG AAGAGGCCTT GAAGATCATG 360
ATCCAGGATG GGAAGAATCT TGCAGAGCCC AACAAGGAGG GCTGGCTGCC GCTCCACGAG 420
GCTGCCTACT ATGGCCAGCT GGGCTGCCTG AAAGTCCTGC AGCAAGCCTA CCCAGGGACC 480
ATTGACCAAC GCACACTGCA GGAAGAGACA GCATTATACC TGGCCACATG CAGAGAACAC 540
CTGGATTGCC TCCTGTCGCT GCTCCAGGCG GGGGCAGAGC CTGACATCTC TAACAAATCC 600
AGGGAGACTC CACTTTACAA AGCCTGTGAG CGCAAGAACG CGGAGGCGGT GAGGATATTG 660
GTGCGATACA ACGCAGACGC CAACCACCGC TGTAACAGGG GCTGGACCGC ACTGCACGAG 720
TCTGTCTCCC GCAATGACCT GGAGGTCATG GAGATCCTAG TGAGTGGCGG GGCCAAGGTG 780
GAGGCCAAGA ATGTCTACAG CATCACCCCT TTGTTTGTGG CTGCCCAGAG TGGGCAGCTG 840
GAGGCCCTGA GGTTCCTGGC CAAGCATGGT GCAGACATCA ACACGCAGGC CAGTGACAGT 900
GCATCAGCCC TCTACGAGGC CAGCAAGAAT GAGCATGAAG ACGTGGTAGA GTTTCTTCTC 960
TCTCAGGGCG CCGATGCTAA CAAAGCCAAC AAGGACGGCC TGCTCCCCCT GCATGTTGCC 1020
TCCAAGAAGG GCAACTATAG AATAGTGCAG ATGCTGCTGC CTGTGACCAG CCGCACGCGC 1080
GTGCGCCGTA GCGGCATCAG CCCGCTGCAC CTAGCGGCCG AGCGCAACCA CGACGCGGTG 1140
CTGGAGGCGC TGCTGGCCGC GCGCTTCGAC GTGAACGCAC CTCTGGCTCC CGAGCGCGCC 1200
CGCCTCTACG AGGACCGCCG CAGTTCTGCG CTCTACTTCG CTGTGGTCAA CAACAATGTG 1260
TACGCCACCG AGCTGTTGCT GCTGGCGGGC GCGGACCCCA ACCGCGATGT CATCAGCCCT 1320

CTGCTCGTGG CCATCCGCCA CGGCTGCCTG CGCACCATGC AGCTGCTGTT GGACCATGGC 1380

GCCAACATCG ACGCCTACAT CGCCACTCAC CCCACCGCCT TTCCAGCCAC CATCATGTTT 1440

GCCATGAAGT GCCTGTCGTT ACTCAAGTTC CTTATGGACC TCGGCTGCGA TGGCGAGCCC 1500

TGCTTCTCCT GCCTGTACGG CAACGGGCCG CACCACCCGC CCCGCGACCT GGCCGCTTCC 1560

ACGACGCACC CGTGGACGAC AAGGCACCTA GCGTGGTGCA GTTCTGTGAG TTCCTGTCGG 1620

CCCCGGAAGT GAGCCGCTGG GCGGGACCCA TCATCGATGT CCTCCTGGAC TATGTGGGCA 1680

ACGTGCAGCT GTGCTCCCGG CTGAAGGAGC ACATCGACAG CTTTGAGGAC TGGGCTGTCA 1740

TCAAGGAGAA GGCAGAACCT CCGAGACCTC TGGCTCACCT CTGCCGGCTG CGGGTTCGGA 1800

AGGCCATAGG AAAATACCGG ATAAAACTCC TGGACACACT GCCGCTTCCC GGCAGGCTAA 1860

TCAGATACTT GAAATATGAG AATACACAGT AACCAGCCTG GAGAGGAGAT GTGGCCTTCA 1920

GACTGTTTCC GGGACGCCCC AGGTGGCCTG CATCCAGGAC CCCCTGGGGT CAGAACAGGT 1980

GTGACCTTGC TGGTTCTTTG CTGGAGCTTC ACCCAAAGTG AGAACCTGAT GTGGGGAGTG 2040

GACGTGGAAC CTCTGCTTTC ACACTGTCAG CGGATCGCAG ACCCGCTCTG CTTCTGGCCA 2100

TAGCCAGAGA CCTTCAACCT GGGGCCAGGG GAGAGCTGGT CTGGGCAAGG TGGCCCAGGC 2160

AGGAATCCTG GCCTTAAGCT GGAGAACTTG TAGGAATCCC TCACTGGACC CTCAGCTTTC 2220

AGGCTGCGAG GGAGACGCCC AGCCCAAGTA TTTTATTTCC GTGACACAAT AACGTTGTAT 2280

CAGAAAAAAA AAAAAACATG GGCGCAGCTT ATTCCTTAGT AGGGTATTTA CTTGCATGCG 2340

CGCTTAAAGC TACTGGAAAC ATGCGTTCCA CTATGCTTGA GAATCCCCTT GCACTGGTAA 2400

ACGAGAGCCG ACGTGCTTCA AGGTTGGATT TTTGGTTGCC CCTTTGGCGT TCCGCGGGTT 2460

TGTCCGACGT AATTGACCCC GTGTTTTGTC ACTTTCGAGT GTTCCGACTA TTGGGGGGCT 2520

TTTGGTTGTC CCCAAAATTG TGGGTGGTGT GCGGACGCCA CGAGAAGTGG TTCATGGGCG 2580

ATAATCATTA CTGGAGAATG TAGAGCGGCG GTTTTACGAA TAAATATTTT TTAAGCCGCC 2640

TTCCCAAAA 2649

(2) INFORMATION FOR SEQ ID NO:33:

(i) SEQUENCE CHARACTERISTICS:
(A) LENGTH: 495 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:33:

CCTCCTGAGA GTTCGCCGGC CCGGGCCCAA TGGGTTGTTC CAAGGGGTCA TGCAGAAATA 60

CAGCAGCAGC TTGTTCAAGA CCTCCCAGCT GGCGCCTGCG GACCCCTTGA TAAAGGCCAT 120

CAAGGATGCG ATGAAGAGGC CTTGAAGACC ATGATCAAGG AAGGGAAGAA TCTCGCAGAG 180

CCCAACAAGG AGGGCTGGCT GCCGCTGCAC GAGGCCGCAT ACTATGGCCA GGTGGGCTGC 240

CTGAAAGTCC TGCAGCGAGC GTACCCAGGG ACCATCGACC AGCGCACCCT GCAGGAGGAA 300

ACAGCCGTTT ACTTGGCAAC GTGCAGGGGC CACCTGGACT GTCTCCTGTC ACTGCTCCAA 360

GCAGGGGCAG AGCGGGACAT CTCCAACAAA TCCCGAGAGA ACCGCTCTAC AAAGCCTGTG 420

AGCGCAAGAA CGCGGAAGCC GTGAAGATTC TTGGTGCAGC ACAACGCAGA CACCAACAAC 480

GCTGCAACCG GGCTG 495

(2) INFORMATION FOR SEQ ID NO:34:

(i) SEQUENCE CHARACTERISTICS:
(A) LENGTH: 709 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:34:

GTGCAGCTCT GCTCGCGGCT GAAGGAACAC ATCGACAGCT TTGAGGACTG GGCCGTCATC 60

AAGGAGAAGG CAGAACCTCC AAGACCTCTG GCTCACCTTT GCCGACTGCG GGTTCGAAAG 120

GCCATTGGGA AATACCGTAT AAAACTCCTA GACACCTTGC CGCTCCCAGG CAGGCTGATT 180

AGATACCTGA AATACGAGAA CACCCAGTAA CTGGGGCCAC GGGGAGAGAG GAGTAGCCCC 240

TCAGACTCTT CTTACTAAGT CTCAGGACGT CGGTGTTCCC AACTCCAAGG GGACCTGGTG 300

ACAGACGAGG CTGCAGGCTG CCTCCCTCTC AGCCTGGACA GCTACCAGGA TCTCACTGGG 360

TCTCAGGGCC CAGAGCTTTG GCCAGAGCAG AGAACAGAAT GTGTCAAGGA GAAGAATCAT 420

TTGTTTACAA ACTGATGAGC AGATCCCAGA CCTTCTCTAC CTTCAGGAAT GGCAGAAACC 480

TCTATTCCTG GGGCCAGGGC AGAGCTTGAG GTGTTCTGGG GAAGGTGGTG CTCAGAGCCT 540

TCCCTGTGCC CCTCCACTTG TTCTGGAAAA CTCACCACTT GACTTCAGAG CTTTCTCTCC 600

AAAGACTAAG ATGAAGACGT GGCCCAAGGT AGGGGGTAGG GGGAGCCTGG GTCTTGGAGG 660

GCTTTGTTAA GTATTAATAT AATAAATGTT ACACATGTGA AAAAAAAAA 709

(2) INFORMATION FOR SEQ ID NO:35:

(i) SEQUENCE CHARACTERISTICS:
(A) LENGTH: 848 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 1..624

(xi) SEQUENCE DESCRIPTION: SEQ ID NO:35:

```
TTG GAG AAG TGT GGT TGG TAT TGG GGG CCA ATG AAT TGG GAA GAT GCA       48
Leu Glu Lys Cys Gly Trp Tyr Trp Gly Pro Met Asn Trp Glu Asp Ala
 1               5                  10                  15

GAG ATG AAG CTG AAA GGG AAA CCA GAT GGT TCT TTC CTG GTA CGA GAC       96
Glu Met Lys Leu Lys Gly Lys Pro Asp Gly Ser Phe Leu Val Arg Asp
            20                  25                  30

AGT TCT GAT CCT CGT TAC ATC CTG AGC CTC AGT TTC CGA TCA CAG GGT      144
Ser Ser Asp Pro Arg Tyr Ile Leu Ser Leu Ser Phe Arg Ser Gln Gly
        35                  40                  45

ATC ACC CAC CAC ACT AGA ATG GAG CAC TAC AGA GGA ACC TTC AGC CTG      192
Ile Thr His His Thr Arg Met Glu His Tyr Arg Gly Thr Phe Ser Leu
    50                  55                  60

TGG TGT CAT CCC AAG TTT GAG GAC CGC TGT CAA TCT GTT GTA GAG TTT      240
Trp Cys His Pro Lys Phe Glu Asp Arg Cys Gln Ser Val Val Glu Phe
65                  70                  75                  80

ATT AAG AGA GCC ATT ATG CAC TCC AAG AAT GGA AAG TTT CTC TAT TTC      288
Ile Lys Arg Ala Ile Met His Ser Lys Asn Gly Lys Phe Leu Tyr Phe
                85                  90                  95

TTA AGA TCC AGG GTT CCA GGA CTG CCA CCA ACT CCT GTC CAG CTG CTC      336
Leu Arg Ser Arg Val Pro Gly Leu Pro Pro Thr Pro Val Gln Leu Leu
            100                 105                 110

TAT CCA GTG TCC CGA TTC AGC AAT GTC AAA TCC CTC CAG CAC CTT TGC      384
Tyr Pro Val Ser Arg Phe Ser Asn Val Lys Ser Leu Gln His Leu Cys
        115                 120                 125

AGA TTC CGG ATA CGA CAG CTC GTC AGG ATA GAT CAC ATC CCA GAT CTC      432
Arg Phe Arg Ile Arg Gln Leu Val Arg Ile Asp His Ile Pro Asp Leu
    130                 135                 140

CCA CTG CCT AAA CCT CTG ATC TCT TAT ATC CGA AAG TTC TAC TAC TAT      480
Pro Leu Pro Lys Pro Leu Ile Ser Tyr Ile Arg Lys Phe Tyr Tyr Tyr
145                 150                 155                 160

GAT CCT CAG GAA GAG GTA TAC CTG TCT CTA AAG GAA GCG CAG CGT CAG      528
Asp Pro Gln Glu Glu Val Tyr Leu Ser Leu Lys Glu Ala Gln Arg Gln
                165                 170                 175

TTT CCA AAC AGA AGC AAG AGG TGG AAC CCT CCA CGT AGC GAG GGG CTC      576
Phe Pro Asn Arg Ser Lys Arg Trp Asn Pro Pro Arg Ser Glu Gly Leu
            180                 185                 190

CCT GCT GGT CAC CAC CAA GGG CAT TTG GTT GCC AAG CTC CAG CTT TGAAGAACCA   631
Pro Ala Gly His His Gln Gly His Leu Val Ala Lys Leu Gln Leu
        195                 200                 205

AATTAAGCTA CCATGAAAAG AAGAGGAAAA GTGAGGGAAC AGGAAGGTTG GGATTCTCTG    691

TGCAGAGACT TTGGTTCCCC ACGCAAGCCC TGGGGCTTGG AAGAAGCACA TGACCGTACT    751

CTGCGTGGGG CTCCACCTCA CACCCACCCC TGGGCATCTT AGGACTGGAG GGCTCCTTG     811

GAAAACTGGA AGAAGTCTCA ACACTGTTTC TTTTTCA                             848
```

(2) INFORMATION FOR SEQ ID NO:36:

(i) SEQUENCE CHARACTERISTICS:
(A) LENGTH: 207 amino acids
(B) TYPE: amino acid
(D) TOPOLOGY: linear

(ii) MOLECULE TYPE: protein

(xi) SEQUENCE DESCRIPTION: SEQ ID NO:36:

```
Leu Glu Lys Cys Gly Trp Tyr Trp Gly Pro Met Asn Trp Glu Asp Ala
 1               5                  10                  15

Glu Met Lys Leu Lys Gly Lys Pro Asp Gly Ser Phe Leu Val Arg Asp
            20                  25                  30

Ser Ser Asp Pro Arg Tyr Ile Leu Ser Leu Ser Phe Arg Ser Gln Gly
        35                  40                  45

Ile Thr His His Thr Arg Met Glu His Tyr Arg Gly Thr Phe Ser Leu
    50                  55                  60

Trp Cys His Pro Lys Phe Glu Asp Arg Cys Gln Ser Val Val Glu Phe
65                  70                  75                  80

Ile Lys Arg Ala Ile Met His Ser Lys Asn Gly Lys Phe Leu Tyr Phe
                85                  90                  95

Leu Arg Ser Arg Val Pro Gly Leu Pro Pro Thr Pro Val Gln Leu Leu
            100                 105                 110

Tyr Pro Val Ser Arg Phe Ser Asn Val Lys Ser Leu Gln His Leu Cys
        115                 120                 125

Arg Phe Arg Ile Arg Gln Leu Val Arg Ile Asp His Ile Pro Asp Leu
    130                 135                 140

Pro Leu Pro Lys Pro Leu Ile Ser Tyr Ile Arg Lys Phe Tyr Tyr Tyr
145                 150                 155                 160

Asp Pro Gln Glu Glu Val Tyr Leu Ser Leu Lys Glu Ala Gln Arg Gln
                165                 170                 175

Phe Pro Asn Arg Ser Lys Arg Trp Asn Pro Pro Arg Ser Glu Gly Leu
            180                 185                 190

Pro Ala Gly His His Gln Gly His Leu Val Ala Lys Leu Gln Leu
        195                 200                 205
```

(2) INFORMATION FOR SEQ ID NO:37:

(i) SEQUENCE CHARACTERISTICS:
(A) LENGTH: 464 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:37:

```
GTTCCAAGCC TAACCCATCT TTGTCGTTTG GAAATTCGGG CCAGTCTAAA AGCAGAGCAC    60
CTTCACTCTG ACATTTTCAT CCATCAGTTG CCACTTCCCA GAAGTCTGCA GAACTATTTG   120
CTCTATGAAG AGGTTTTAAG AATGAATGAG ATTCTAGAAC CAGCAGCTAA TCAGGATGGA   180
GAAACCAGCA AGGCCACCTG ACACAGGTCC TTTAATTCTG TTTAGTCACA AAAGACGGCT   240
TGTGTGACTG TTTGGATTTG GTGATCAAAT GTCCATGTTT ACAGTTGCTT TTCCCAGTTT   300
```

GTGTCTTTCC CAATATTGTG AACCTTATCC ATCTTGCCTT ACTCAGTTTT ATTTCTAGTG 360

CACTTTGTTG TGTATTATTT GTTTACCTGA CCATTTTCTA CTTTATTCTG CTAATAAACT 420

GTAATTCTGA AAAAAAAAAA AAAAAAAAAA AAAAAAAAAA AAAA 464

(2) INFORMATION FOR SEQ ID NO:38:

(i) SEQUENCE CHARACTERISTICS:
(A) LENGTH: 747 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:38:

GGGGATCGAA AGCGGGGGCT TCTGGGACGC AGCTCTGGAG ACGCGGCCTC GGACCAGCCA 60

TTTCGGTGTA GAAGTGGCAG CACGGCAGAC TGGTCAAACA AATGGATTTT ACAGAGGCTT 120

ACGCGGACAC GTGCTCTACA GTTGGACTTG CTGCCAGGGA AGGCAATGTT AAAGTCTTAA 180

GGAAACTGCT CAAAAAGGGC CGAAGTGTCG ATGTTGCTGA TAACAGGGGA TGGATGCCAA 240

TTCATGAAGC AGCTTATCAC AACTCTGTAG AATGTTTGCA AATGTTAATT AATGCAGATT 300

CATCTGAAAA CTACATTAAG ATGAAGACCT TTGAAGGTTT CTGTGCTTTG CATCTCGCTG 360

CAAGTCAAGG ACATTGGAAA ATCGTACAGA TTCTTTTAGA AGCTGGGGCA GATCCTAATG 420

CAACTACTTT AGAAGAAACG ACACCATTGT TTTTAGCTGT TGAAAATGGA CAGATAGATG 480

TGTTAAGGCT GTTGCTTCAA CACGGAGCAA ATGTTAATGG ATCCCATTCT ATGTGTGGAT 540

GGAACTCCTT GCACCAGGCT TCTTTTCAGG AAAATGCTGA GATCATAAAA TTGCTTCTTA 600

GAAAAGGAGC AAACAAGGAA TGCCAGGATG ACTTTGGAAT CACACCTTTA TTTGTGGCTG 660

CTCAGTATGG CCAAGCTAGA AAGCTTTGAA GCATACTTAT TTCATCCGGG TGCAAATGTC 720

AATTGTCAAG CCTTGGACAA AGCTACC 747

(2) INFORMATION FOR SEQ ID NO:39:

(i) SEQUENCE CHARACTERISTICS:
(A) LENGTH: 1018 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:39:

CACAAATGGG ACCATACAAA AATCTTGGAC TTGTTAATAA CCACTTACTA ACCGGGACCT 60

GTGACACTGG GCTAAACAAA GTAAGTCCCT GTTTACTCAG CAGTGTTTGG GGGACATGAA 120

GGATTGCCTA GAAATATTAC TCCGGAATGG TCTACAGCCC AGACGCCCAG GCGTGCCTTG 180

TTTTTGGATT CAGTTCTCCT GTGTGCATGG CTTTCCAAAA GGAGGTGGAG CTGTAGTTCT 240

TTGGAATTGT GAACATTCTT TTGAAATATG GAGCCCAGAT AAATGAACTT CATTTGGCAT 300

ACTGCCTGAA GTACGAGAAG TTTTCGATAT TTCGCTACTT TTTGAGGAAA GGTTGCTCAT 360

TGGGACCATG GAACCATATA TATGAATTTG TAAATCATGC AATTAAAGCA CAAGCAAAAT 420

ATAAGGAGTG GTTGCCACAT CTTCTGGTTG CTGGATTTGA CCCACTGATT CTACTGTGCA 480

ATTCTTGGAT TGACTCAGTC AGCATTGACA CCCTTATCTT CACTTTGGAG TTTACTAATT 540

GGAAGACACT TGCACCAGCT GTTGAAAGGA TGCTCTCTGC TCGTGCCTCA AACGCTTGGA 600

TTCTACAGCA ACATATTGCC CACTGTTCCA TCCCTGACCC ATCTTTGTCG TTTGGAAATT 660

CGGTCCAGTC TAAAATCAGA ACGTCTACGG TCTGACAGTT ATATTAGTCA GCTGCCACTT 720

CCCAGAAGCC TACATAATTA TTTGCTCTAT GAAGACGTTC TGAGGATGTA TGAAGTTCCA 780

GAACTGGCAG CTATTCAAGA TGGATAAATC AGTGAAACTA CTTAACACAG CTAATTTTTT 840

TCTCTGAAAA ATCATCGAGA CAAAAGAGCC ACAGAGTACA AGTTTTTATG ATTTTATAGT 900

CAAAAGATGA TTATTGATTG TCAGATAGGT TAGGTTTTGG GGGGCCAGTA GTTCAGTGAG 960

AATGTTTATG TTTACAACTA GCCTTCCCAG TAAAAAAAAA AAAAAAAAAA AAAAAAAA 1018

(2) INFORMATION FOR SEQ ID NO:40:

(i) SEQUENCE CHARACTERISTICS:
(A) LENGTH: 1897 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:40:

CGGGGGGCTG GGACCTGGGG CGTAACCGTC TCTACCACGA CGGCAAGAAC CAGCCAAGTA 60

AAACATACCC AGCCTTTCTG GAGCCGGACG AGACATTCAT TGTCCCTGAC TCCTTTTTCG 120

TGGCCCTGGA CATGRATGAT GGGACCTTAA GTTTCATCGT GGATGGACAG TACATGGGAG 180

TGGCTTTCCG GGGACTCAAG GGTAAAAAGC TGTATCCTGT AGTGAGTGCC GTCTGGGGCC 240

ACTGTGAGAT CCGCATGCGC TACTTGAACG GACTTGATCC TGAGCCCCTG CCACTCATGG 300

ACCTGTGCCG GCGTTCGGTG CGCCTAGCGC TGGGAAAAGA GCGCCTGGGT GCCATCCCCG 360

CTCTGCCGCT ACCTGCCTCC CTCAAAGCCT ACCTCCTCTA CCAGTGATCC ACATCCCAGG 420

ACCGCCATAC GACAGCCATC TGGTGCCAAR TCACTGAGCC CGTTGGGGTC CGCCGACCCC 480

TGCGCCTGGG ATGGAAGCCC ACCTCAGCCA TGGGCAGACG TGCCCCCTCA TCCTACCGGC 540

TGCCTCTGCT GGGGGAACCT ATGCCAACGG ACTTCTCCCT TCCCAACACT GGCTGAAGCA 600

GCAGCACCCA GGCCCTTCCC TGAACCAGAT GCAGAGAATA AACTATGAAA ACCTCTCTCA 660

GGCGCCTTCT GCTCTCAGGT GGAGTGGGCT GCCCCCCACT CTCTGCAGAG AGAGGCTACA 720

CCCACCTGGG GGGTCCTGGG AGGTAAGACT AGTAGGAGGT GCCAGGGCTG ARTCCAAAAG 780

```
CAGGAATGGC CAGGAMCAGG CCATACAGAT GAAGCTCAGG ATGTCACATA CCATGGACAM     840
TGAGACAGAA CCCCAGGTTG GAMTTCCCTT GGGCCAACGA GTGCCAGCTT TAATGTCAGC     900
TGCMGGTGCT CTGTGGCCTG TATTTATTCT TTAAACAGTA GCAAAGGCCA TTTATTTATT     960
CCACTTAGAA AGGAAACCTT GGTGGGTGGY TTCCCTCGAT GTGCTTTCCC CCACCTCCCT    1020
GGAATGTGTG TGCCACACCT GTCCTTGTCC CAGGCCAGGA CTGTGGCACA TGAGCTGGTG    1080
TGCACAGATA CACGTATGTC GTCGTGCATG ACCCCTGACT AGTTCCTAAG TAGCCCTGCA    1140
CCAAGCACCA GAGCAGACCC CAAGAGAGGC CCGTGCAAGT CCCCATGTCC CCAGGTCCCT    1200
GCTTCTGTTG CCTTGGGACT CATACACCGG CACACGTGTT TCAGCCTCTT GACTTCCATG    1260
AGCTTCGAAT TTTGCCCCCG ATTCTTCTGA TATTTCCCAT TGGCATCCTC CAAAGCTCTG    1320
GGCCTGGAGG GCATTAGGAC ACATGGAATG AGTGGGGTCT CCAGCCCCTG GGAAAGCCAC    1380
TGGCAAGGCA GGATTAGAAA GACCAAGAGC AGGGTGGGGC GCCATGAAGC CTGTATGCCT    1440
CTCAGGCTCA AGACCCCGCC ACACACCCAC TCAAGCCTCA GAAGTGGTGT GTAGGGCAGC    1500
CCCAGGAGAG GAATGCCTGT CCTAGCAGCA CGTACATGGA GCACCCCACA TGTGCTCCAG    1560
CCCTCTGGCT GTTTCTCTTG CTCTAGAATC AACTCCCTAC ATTGGGAATG TAGCCATTTG    1620
GTAGAGGACT TGCCTAGCCT GCAGGAAGCT CACGTTCCAT CCCCTGCACC AAGGAGAATC    1680
AAAGCTCAGG AGGCTGAGGC AGGAGGATTG CTGTCAGTGG TGTACAGAGG TCATGGCCAT    1740
CCTGGGCTAT ATTAAACCTT GTCCTTTAAG AAAAAGAAAA GAAATCAACT TCCATTGAAT    1800
CTGAGTTCTG CTCATTTCTG CACAGGTACA ATAGATGACT TKATTTGTTG AAAAATGKTT    1860
AATATATTTA CMTATATATA TATTTGTAAG AAGCATT                             1897
```

(2) INFORMATION FOR SEQ ID NO:41:

(i) SEQUENCE CHARACTERISTICS:
(A) LENGTH: 134 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:41:

```
Gly Gly Trp Asp Leu Gly Arg Asn Arg Leu Tyr His Asp Gly Lys Asn
1               5                   10                  15

Gln Pro Ser Lys Thr Tyr Pro Ala Phe Leu Glu Pro Asp Glu Thr Phe
            20                  25                  30

Ile Val Pro Asp Ser Phe Phe Val Ala Leu Asp Met Xaa Asp Gly Thr
        35                  40                  45

Leu Ser Phe Ile Val Asp Gly Gln Tyr Met Gly Val Ala Phe Arg Gly
    50                  55                  60

Leu Lys Gly Lys Lys Leu Tyr Pro Val Val Ser Ala Val Trp Gly His
65                  70                  75                  80
```

```
Cys Glu Ile Arg Met Arg Tyr Leu Asn Gly Leu Asp Pro Glu Pro Leu
                85                  90                  95

Pro Leu Met Asp Leu Cys Arg Arg Ser Val Arg Leu Ala Leu Gly Lys
            100                 105                 110

Glu Arg Leu Gly Ala Ile Pro Ala Leu Pro Leu Pro Ala Ser Leu Lys
        115                 120                 125

Ala Tyr Leu Leu Tyr Gln
    130
```

(2) INFORMATION FOR SEQ ID NO:42:

(i) SEQUENCE CHARACTERISTICS:
(A) LENGTH: 265 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:42:

```
AAGGGTAAAA AACTGTATCC TGTAGTGAGT GCCGTCTGGG GCCACTGTAG ATCCGAATGC     60
GCTACTTGAA CGGACTCGAT CCCGAGACTG CCGCTCATGG ATTTGTGCCG TCGCTCGGTG    120
CGCCTGGCCC TGGGGAGGGA GCGCCTGGGG GAGAACCACA CCTGCCGCTG CCGGCTTCCC    180
TCAAGGCCTA CCTCCTCTAC CAGTGACGTT CGCCATCATA CCGCCAGCGC GACAGCCACC    240
TGGTGCCAAC TCACTGAGCC GCCTG                                          265
```

(2) INFORMATION FOR SEQ ID NO:43:

(i) SEQUENCE CHARACTERISTICS:
(A) LENGTH: 2438 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:43:

```
AAGTGGCGGC GGTCCCTGGA GAGCAGGCGG AGGCAGCGGC AAGTCTGACT CTGGGCTGAC     60
CGTGGAGCCG GGGCGGGGGC TGACAGCCAG GCCTCCGCCT GGCGGGAGCC GCACGAGGAG    120
CGGGAGTGGC CGGGCCTCTC TTCCGCGCTT GAGCGAGCGC CGGGTGATGG CGGTGGTGAT    180
GGCGGCAGGC GCTCGGACAG CTCCGCTTGA GCTGAGCTCG GAGAGATCCG TCCAGAAAGT    240
GCCCAGAAGA AACTTCCTCT TAGAAAAGCT GAAAAACACA RTATTTATAA CACTGGAAAT    300
TGTAAAGAAT TTGTTTAAAA TGGCTGAAAA CAATAGTAAA AATGTAGATG TACGGCCTAA    360
AACAAGTCGG AGTCGAAGTG CTGACAGGAA GGATGGTTAT GTGTGGAGTG GAAAGAAGTT    420
```

GTCTTGGTCC AAAAAGAGTG AGAGTTGTTC TGAATCTGAA GCCATAGGTA CTGTTGAGAA 480
TGTTGAAATT CCTCTAAGAA GCCAAGAAAG GCAGCTTAGC TGTTCGTCCA TTGAGTTGGA 540
CTTAGATCAT TCCTGTGGGC ATAGATTTTT AGGCCGATCC CTTAAACAGA AACTGCAAGA 600
TGCGGTGGGG CAGTGTTTTC CAATAAAGAA TTGTAGTGGC CGACACTCTC CAGGGCTTCC 660
ATCTAAAAGA AAGATTCATA TCAGTGAACT CATGTTAGAT AAGTGCCCTT TCCCACCTCG 720
CTCAGATTTA GCCTTTAGGT GGCATTTTAT TAAACGACAC ACTGTTCCTA TGAGTCCCAA 780
CTCAGATGAA TGGGTGAGTG CAGACCTGTC TGAGAGGAAA CTGAGAGATG CTCAGCTGAA 840
ACGAAGAAAC ACAGAAGATG ACATACCCTG TTTCTCACAT ACCAATGGCC AGCCTTGTGT 900
CATAACTGCC AACAGTGCTT CGTGTACAGG TGGTCACATA ACTGGTTCTA TGATGAACTT 960
GGTCACAAAC AACAGCATAG AAGACAGTGA CATGGATTCA GAGGATGAAA TTATAACGCT 1020
GTGCACAAGC TCCAGAAAAA GGAATAAGCC CAGGTGGGAA ATGGAAGAGG AGATCCTGCA 1080
GTTGGAGGCA CCTCCTAAGT TCCACACCCA GATCGACTAC GTCCACTGCC TTGTTCCAGA 1140
CCTCCTTCAG ATCAGTAACA ATCCGTGCTA CTGGGGTGTC ATGGACAAAT ATGCAGCCGA 1200
AGCTCTGCTG GAAGGAAAGC CAGAGGGCAC CTTTTTACTT CGAGATTCAG CGCAGGAAGA 1260
TTATTTATTC TCTGTTAGTT TTAGACGCTA CAGTCGTTCT CTTCATGCTA GAATTGAGCA 1320
GTGGAATCAT AACTTTAGCT TTGATGCCCA TGATCCTTGT GTCTTCCATT CTCCTGATAT 1380
TACTGGGCTC CTGGAACACT ATAAGGACCC CAGTGCCTGT ATGTTCTTTG AGCCGCTCTT 1440
GTCCACTCCC TTAATCCGGA CGTTCCCCTT TTCCTTGCAG CATATTTGCA GAACGGTTAT 1500
TTGTAATTGT ACGACTTACG ATGGCATCGA TGCCCTTCCC ATTCCTTCGC CTATGAAATT 1560
GTATCTGAAG GAATACCATT ATAAATCAAA AGTTAGGTTA CTCAGGATTG ATGTGCCAGA 1620
GCAGCAGTGA TGCGGAGAGG TTAGAATGTC GACCTGCATA CATATTTTCA TTTAATATTT 1680
TATTTTTCTT ATGCCTCTTT GAATTTTTGT ACAAAGGCAG TTGAATCAAA TAAAACTGTG 1740
CCCTAAGTTT TAATTCCAGA TCAATTTATT TTTTTTATGA TACACTTGTT ATATATTTTT 1800
AAGCAGGTGT TTGGTTTTGT TTTTACCATA TAAATTTACA TATGGTCCAG GCATATTTAC 1860
AATTTCAAGG CATTGCATAT ACATTTGAAT ATTCTGTATT TTTTAAATAA TCTTTTGTTC 1920
TTTCCTATGT GTGAAATATT TTGCTAATCT ATGCTATCAG TATTCTTGTA TGACCGAATA 1980
GTTACCTATT CTCTTTTCAT CTTGAAGATT TTCAGTAAAG AGTGTTGTAA TCAATCCATT 2040
ATAATGTAAT TGACTTTTGT AATTTGCCAA TAGGAGTGTT AAACAACAAA ATGATTTAAA 2100
ATGAAACTTA ATGTATTTTC ATTTTAAATA TTAACTAAAC CAAGTTTGTT TGTTAGTTAT 2160
TCTAGCCAAT AAGAAAAGAG AATGTAGCAT CCTAGAGGTG TATTTGTTCT GCAGTTTGGC 2220
AGGACCGTCA GTTAGTCCAA ATAAACATCC CCTCAGCGTG GAGGCGAATG GAACCTGTGC 2280
TCCTTTCTTA CGGGAAGCTT TGCAAAGCAA AATAGCAGGG TTACAAGCTT GGAGTTGTTA 2340
AGGCAACTAG AGTTTTCTCT ATTAATTTAT AGACTGTTGT TGCACCTACT TAGCTCTTTT 2400
TTGGGAACTC TAGTTCCCAG GGGAAAATAC CTCGTGCC 2438

(2) INFORMATION FOR SEQ ID NO:44:

(i) SEQUENCE CHARACTERISTICS:
(A) LENGTH: 542 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:44:

```
Ser Gly Gly Gly Pro Trp Arg Ala Gly Gly Gly Ser Gly Lys Ser Asp
1               5                   10                  15

Ser Gly Leu Thr Val Glu Pro Gly Arg Gly Leu Thr Ala Arg Pro Pro
            20                  25                  30

Pro Gly Gly Ser Arg Thr Arg Ser Gly Ser Gly Arg Ala Ser Leu Pro
        35                  40                  45

Arg Leu Ser Glu Arg Arg Val Met Ala Val Val Met Ala Ala Gly Ala
    50                  55                  60

Arg Thr Ala Pro Leu Glu Leu Ser Ser Glu Arg Ser Val Gln Lys Val
65                  70                  75                  80

Pro Arg Arg Asn Phe Leu Leu Glu Lys Leu Lys Asn Thr Xaa Phe Ile
                85                  90                  95

Thr Leu Glu Ile Val Lys Asn Leu Phe Lys Met Ala Glu Asn Asn Ser
            100                 105                 110

Lys Asn Val Asp Val Arg Pro Lys Thr Ser Arg Ser Arg Ser Ala Asp
        115                 120                 125

Arg Lys Asp Gly Tyr Val Trp Ser Gly Lys Lys Leu Ser Trp Ser Lys
    130                 135                 140

Lys Ser Glu Ser Cys Ser Glu Ser Glu Ala Ile Gly Thr Val Glu Asn
145                 150                 155                 160

Val Glu Ile Pro Leu Arg Ser Gln Glu Arg Gln Leu Ser Cys Ser Ser
                165                 170                 175

Ile Glu Leu Asp Leu Asp His Ser Cys Gly His Arg Phe Leu Gly Arg
            180                 185                 190

Ser Leu Lys Gln Lys Leu Gln Asp Ala Val Gly Gln Cys Phe Pro Ile
        195                 200                 205

Lys Asn Cys Ser Gly Arg His Ser Pro Gly Leu Pro Ser Lys Arg Lys
    210                 215                 220

Ile His Ile Ser Glu Leu Met Leu Asp Lys Cys Pro Phe Pro Pro Arg
225                 230                 235                 240

Ser Asp Leu Ala Phe Arg Trp His Phe Ile Lys Arg His Thr Val Pro
                245                 250                 255

Met Ser Pro Asn Ser Asp Glu Trp Val Ser Ala Asp Leu Ser Glu Arg
            260                 265                 270

Lys Leu Arg Asp Ala Gln Leu Lys Arg Arg Asn Thr Glu Asp Asp Ile
        275                 280                 285
```

```
Pro Cys Phe Ser His Thr Asn Gly Gln Pro Cys Val Ile Thr Ala Asn
    290                 295                 300

Ser Ala Ser Cys Thr Gly Gly His Ile Thr Gly Ser Met Met Asn Leu
305                 310                 315                 320

Val Thr Asn Asn Ser Ile Glu Asp Ser Asp Met Asp Ser Glu Asp Glu
                325                 330                 335

Ile Ile Thr Leu Cys Thr Ser Ser Arg Lys Arg Asn Lys Pro Arg Trp
            340                 345                 350

Glu Met Glu Glu Glu Ile Leu Gln Leu Glu Ala Pro Pro Lys Phe His
        355                 360                 365

Thr Gln Ile Asp Tyr Val His Cys Leu Val Pro Asp Leu Leu Gln Ile
    370                 375                 380

Ser Asn Asn Pro Cys Tyr Trp Gly Val Met Asp Lys Tyr Ala Ala Glu
385                 390                 395                 400

Ala Leu Leu Glu Gly Lys Pro Glu Gly Thr Phe Leu Leu Arg Asp Ser
                405                 410                 415

Ala Gln Glu Asp Tyr Leu Phe Ser Val Ser Phe Arg Arg Tyr Ser Arg
            420                 425                 430

Ser Leu His Ala Arg Ile Glu Gln Trp Asn His Asn Phe Ser Phe Asp
        435                 440                 445

Ala His Asp Pro Cys Val Phe His Ser Pro Asp Ile Thr Gly Leu Leu
    450                 455                 460

Glu His Tyr Lys Asp Pro Ser Ala Cys Met Phe Phe Glu Pro Leu Leu
465                 470                 475                 480

Ser Thr Pro Leu Ile Arg Thr Phe Pro Phe Ser Leu Gln His Ile Cys
                485                 490                 495

Arg Thr Val Ile Cys Asn Cys Thr Thr Tyr Asp Gly Ile Asp Ala Leu
            500                 505                 510

Pro Ile Pro Ser Pro Met Lys Leu Tyr Leu Lys Glu Tyr His Tyr Lys
        515                 520                 525

Ser Lys Val Arg Leu Leu Arg Ile Asp Val Pro Glu Gln Gln
    530                 535                 540
```

(2) INFORMATION FOR SEQ ID NO:45:

(i) SEQUENCE CHARACTERISTICS:
(A) LENGTH: 4999 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:45:

```
CCCTCTGGGC AAGCCGCCCC CCCCCCACCC ATCTACCACA CACACACACA CACACACACA     60
CACACATTCA GACCTTGGGG CAAAAACAAA GCAAAATAAC AACAACAAAA ACACTGCCTG    120
```

TGGAAAGTCC TTACTTCAGG AAGGTTGGCA GATGAGGAGC AAGGGAACAT TTTATCAGGA 180
CTGCCACAAA GGAGTCTTTT TTTTTAATGG TTTTTCAAGA CAGGGTTTCT CTGTATAGCC 240
CTGGCTGTCC TGGAGCTCAC TTTGTAGACC AGGCTGGCCT CGAACTCAGA AATTCGCCTG 300
CCTCTGCCTC CTGAGTGCTG GGATTAAAGG CGTGCAGCAC CATGTCCAAC TGGCATTTTC 360
TCAATTAAGG TTCGTTCCTT TCAGATAACT CTAGGTTCTG GGTCAAGCTG ACACAAGGCT 420
ACACAGCACA GTTTGTATGC CACATTCAGT TCAGAAGACA CCCAACCTCC CTGGAACTGG 480
AACTTATGCA CATTTGTGAG CTTCCACTTG GGAGTGGGAA CCTGAACTGG GTCCTCTGCA 540
AGAGCAGCCG TGCTCTTAAC TGCTGAGCCA TTTCAGCAGC CTCACATCAG AATTAAGTTA 600
GAAATTAGCCG GGTATGAATC ATACCCTTAG AATCCTAGCA TCTGAAAGCA GAGCTAAGAG 660
AAACAGGGAT TCAAGACCAG CTCTTGGCTA CAGAGCCCGT CCTGTCCTAG GATGGGCTAC 720
AAGAGACTAT TTCAAAGCCA TCCAAACAAC AATAACTACA ACAACAACAA GGTTAAAATT 780
AGGCTGGGCA CAGGGTACAC ACCTTTAATG CCAACACTCA GGAGGCAGAG GCAGGCTGAT 840
CAGTGTGAGT TTGAGTTCAA CGTGGTCTAC ATAGGGAGTT CTAGGCCAGC AGAGGTTACA 900
GTCTCTCTCT CTCTCTCTCT CTCTCTCTCT CTCTCACACA CACACACACA CACACACACA 960
CACACACACA CACACACGGT GGCATTATGG GATTTTTTTG GGATAAGGTT TCTCTGTCTA 1020
GCCCTGGCAT AGATTCACTC TGTAGACTAG GCTAGCCTTG AACTCAGAGA TCCGCCTGCC 1080
TCTGCCTCCC AAGTGCTGGG ATTATAGGTG TTGCACCACC ACTGCCCAGC CACTTTGGGA 1140
TTTTTGAACT GTTATCAAGA GGCTTTCGAG GAGGTCAAAC TTCAACAGCA ACCTCTCCAT 1200
GATAATGTAG CTAATGATCA AACGACACTC AAAACTTAAC CCTTAAAGCA CACATCCACC 1260
AGACAGCGTG CCCACTCGTA GTTCCATTAC TCAGGAGGCT GAAGCAGGAG GATGAAGGAC 1320
TAAGGCTTCA GCAACCTAGG GAGCCGCAGG GGACAGTAGT CTCAATCCCT ACATTCTCCT 1380
GAACACAGGA GCAGGAGTTC AGGAAGGGTG TCAAGGCCGC TTACTGATCT TAGGGCCTCA 1440
GGAATGACTA GCTCAGGCAG AGAGAGCAAA GGTCTCCAGT GGAGAAGTCT ACACACACAC 1500
ACACACACAC ACACACACAC ACACACACAC AGAATCCAAG GCGATGACGT CATCAAAGGG 1560
TTAATTCTAG TCTGGGATGG GGGGGAGGGT GGGGCACGCA GCTGTCAGGT GGCTTTGGAA 1620
AAATAAACTG CTGAAGAGTC TGACGCCAGG GAGTCCTGGG AGGGACAAGA GGTTACCCAC 1680
TCAAAGAGTG TGCTCCACAA AGCATGCGCG CTTGTCCACG TCTGGAGTCG TCACTTATTT 1740
TTTGCCTGGA TTCTTTGTAG CCGGTGGGTT CTCAAGGCGG TAAGTGGTGT GGCCGCCGTG 1800
GTCTGGGAGG TGACGATAGG GTTAATCGTC CACAGAGCCC AGGGGCGGAG CGCGGGCGGG 1860
CGTCCGCAGC CCCGCTGGAG CCGGAAGCAG TGGCTGGTCA GGGGCGCTTC TAGCCTTCCC 1920
TATCTGTACT TCCACAGAGG TCTCTGCGAG CTAGGGGGAC AGTGAGGTGC GGGGTAGGGG 1980
CCCGGCGTTA GAGCCAGCAA GGGGACGGTT CACGGTAAGG TCTGAGGGAG AGAGAGCTCC 2040
TGAGAAACTT GGGGGGCGCG ACACAGATAG GGTGAAAGCA GAGTGATAGA CCTGGGATGG 2100
TTAGGGGACC AAGGGAAGAC CAGGCTGGTT GGCATACACC GGTGAACGGA TGGGAGTCCT 2160

AGGGAAAGAT GATGCGCCTA ACAGTCCTTT CTGTCTCCAC ACCACTCCAG GGGACGATCC 2220
GGAGCTCAAC TTTCAAAAGC GAGACGCCCC AGCAAGCCTG TTTTGAGAAG TTCTTCAGCG 2280
GCTCTCCTCA TGGGCCAGAC GGCCCTGGCA AGGGGCAGCA GCAGCACCCC TACCTCGCAG 2340
GCTCTGTACT CGGACTTCTC TCCTCCCGAG GGCTTGGAGG AGCTCCTGTC TGCTCCCCCT 2400
CCTGACCTGG TTGCCCAACG GCACCACGGC TGGAACCCCA AGGATTGCTC CGAGAACATC 2460
GATGTCAAGG AAGGGGGTCT GTGCTTTGAG CGGCGCCCTG TGGCCCAGAG CACTGATGGA 2520
GTCCGGGGGA AACGGGGCTA TTCGAGAGGT CTGCACGCCT GGGAGATCAG CTGGCCCCTG 2580
GAGCAAAGGG GCACACACGC CGTGGTGGGC GTGGCCACCG CCCTCGCCCC GCTGCAGGCT 2640
GACCACTATG CGGCGCTTTT GGGCAGCAAC AGCGAGTCCT GGGGCTGGGA TATTGGGCGG 2700
GGAAAATTGT ATCATCAGAG TAAGGGCCTC GAGGCCCCCC AGTATCCAGC TGGACCTCAG 2760
GGTGAGCAGC TAGTGGTGCC AGAGAGACTG CTGGTGGTTC TGGACATGGA GGAGGGGACT 2820
CTTGGCTACT CTATTGGGGG CACGTACCTG GGACCAGCCT TCCGTGGACT GAAGGGGAGG 2880
ACCCTCTATC CCTCTGTAAG TGCTGTTTGG GGCCAGTGCC AGGTCCGCAT CCGCTACATG 2940
GGCGAAAGAA GAGGTGAGAT ACGGACTAGG TGTGGGGAGA TCACTACTCT TGGCAATGGT 3000
TTGGGCTGGA AACTCATGGT TGGAGCACAG GAAGTAGGCT TCTTGTCACT TTGGCCTGTC 3060
ACTTAGATGG CCTTGGATCT AGCTTCACTC CCAATCCCTA TTGGATGTGA TGCACAAATT 3120
CAGAGCCTTT GGGTCTCCCT CAGCTGAGGT GGCGGTGGAA ATGGAGGAAG AAGGAAGGGT 3180
GCCTGAGCAG GATCTCAAGT TCAAGGATGC CTGGAGTTGC TTACTTACCT TGTCTTCCTT 3240
CTCTCTCCGC AGTGGAGGAA CCACAATCCC TTCTGCACCT GAGCCGCCTG TGTGTGCGCC 3300
ATGCTCTGGG GGACACCCGG CTGGGTCAAA TATCCACTCT GCCTTTGCCC CCTGCCATGA 3360
AGCGCTATCT GCTCTACAAA TGACCCAGTA GTACAGGGTG TGCTGGCACC CTACCGTGGG 3420
GACAGGTGGA GAGGCACCCG CTGGCCTAGA CAACTTTAAA AAGCTGGTGA AGCTGGGGGG 3480
GGGGGGCTGG ACCCCTTCAC CTCCCCTTCT CACAGGAGCA AGACATATAG AAATGATATT 3540
AAACACCATG GCAGCCTGGG ACAAAGAGGT TTTTGAAGTA AAAAATGAGA TGTATTGTCA 3600
CAACCTGTTT CATTATTGTT TTTTGTTTTG TTTTACACTC CCCCACCCCA GGCTAGAGCC 3660
CCATCACTGT CTTAAGGAAT TATGACAACC CACAAAGCTC AGGCCCAGGT GTTTATTTCC 3720
CTTACATGTA GGATGGTTCA CAAACACAAT ACAGGGGCTT TGGCACCGTG GGGGAGGGGA 3780
CTATCCCAGG CCTCTTAGGG TCTCATGTAT ACCGAATTCA GACCCGAAAG CTCTGAATTT 3840
CTGCATCAGA CATCCAGTAG AACTTGGGAG TGAAGCTAGA GCCAAGGCCA TCTAAGTGAC 3900
AGGCCAAAGT GACACGAAGC CCACTTCCTG TGCTCCAACC ATGAGTTTCC AGCCCAAACC 3960
AATGGAAGGT GATTTCACTT GTCAGGGCCC AAAGGGACAG TCAGTTCTAC TCCCTCCCCT 4020
CACTAGGAGC CACCTTGGTG ACAGTTGATT CTACCCACTG TAAGTGGTAA AGGGATTGGC 4080
CTGGTCCCAA CCATAATAGG GCGGTGGAAA CGGCTCAGGA GGGTACAGCG TGGATTAGGC 4140
CACAAGATGG GGCAGATGAT GTCATCAGAA GCATGTGACC GGTGGGAGCA GTTACTAAAC 4200

```
TTCTGGGCAA CCTAGTCCAT GCTATGCAGG CAGGTAGAGG GATGGGCAGT GCTCATTGTT    4260
TGGCATTGAT GATGTCCACA AATTCAGGCT TGAGAGATGC GCCACCCACA AGGAAGCCGT    4320
CCACGTCAGG CTGGCTTGCC AGCTCTTTGC AGGTTGCTCC AGTCACAGAA CCTGTACCAG    4380
GAACAAGAAG ACAGTTTGGT CAGGTCTATG ATCAGAACAC TTAAGCCCCA CCTCTCTGTG    4440
CAAGGCAGCC TCAGTCTGTC TTAGCCCATT TCCGTCTTAG CTAGAGCCAA AGCCACTCAC    4500
CTCCATAAAT GATCCGGGTG CTCTGAGCCA CCCCATCATT GACATTGGAT TTCAGCCATC    4560
CCCGGAGCTT CTCGTGTACT TCCTGTGCCT AGAAGGAGGA GGCAGAGCTA CTAAGTAAGC    4620
TCCTTCCTAT CTATCATTCA AGGAGTAAAA ACCACTGGTT CTCACATAGA GTTGAGTTTC    4680
CAGAAAAGCC CCGGGACCAG AGAGTGGCAA GGCTCCAATC CCACCAGGCT TGGAATGAAC    4740
ATTTTTGGCA AAGTCACTCT CCTTGGTGAG TTTGGGGGCC CTCTGTCTCT AAAGGGGCTT    4800
GGATGGGCTC CATAGCTGTG TGAGTCTGTT AAAGCCGGAC AGGCTGAGGA GCTCTGGGTA    4860
GTTACCTGCT GAGGGGTTGC CGTCTTGCCA GTCCCAATGG CCCACACAGG TTCATAGGCC    4920
AGGACCACCT TGCTCCAGTC TTTCACATTA TCTGTGGGGC AGAGAGGAGA GTGAGTAGGA    4980
AGGAGCTGAC CCGCCAAGC                                                 4999
```

(2) INFORMATION FOR SEQ ID NO:46:

(i) SEQUENCE CHARACTERISTICS:
(A) LENGTH: 264 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:46:

```
Met Gly Gln Thr Ala Leu Ala Arg Gly Ser Ser Ser Thr Pro Thr Ser
1               5                   10                  15

Gln Ala Leu Tyr Ser Asp Phe Ser Pro Pro Glu Gly Leu Glu Glu Leu
            20                  25                  30

Leu Ser Ala Pro Pro Pro Asp Leu Val Ala Gln Arg His His Gly Trp
        35                  40                  45

Asn Pro Lys Asp Cys Ser Glu Asn Ile Asp Val Lys Glu Gly Gly Leu
    50                  55                  60

Cys Phe Glu Arg Arg Pro Val Ala Gln Ser Thr Asp Gly Val Arg Gly
65                  70                  75                  80

Lys Arg Gly Tyr Ser Arg Gly Leu His Ala Trp Glu Ile Ser Trp Pro
                85                  90                  95

Leu Glu Gln Arg Gly Thr His Ala Val Val Gly Val Ala Thr Ala Leu
            100                 105                 110

Ala Pro Leu Gln Ala Asp His Tyr Ala Ala Leu Leu Gly Ser Asn Ser
        115                 120                 125

Glu Ser Trp Gly Trp Asp Ile Gly Arg Gly Lys Leu Tyr His Gln Ser
```

```
        130                 135                 140

    Lys Gly Leu Glu Ala Pro Gln Tyr Pro Ala Gly Pro Gln Gly Glu Gln
    145                 150                 155                 160

    Leu Val Val Pro Glu Arg Leu Leu Val Val Leu Asp Met Glu Glu Gly
                    165                 170                 175

    Thr Leu Gly Tyr Ser Ile Gly Gly Thr Tyr Leu Gly Pro Ala Phe Arg
                180                 185                 190

    Gly Leu Lys Gly Arg Thr Leu Tyr Pro Ser Val Ser Ala Val Trp Gly
            195                 200                 205

    Gln Cys Gln Val Arg Ile Arg Tyr Met Gly Glu Arg Arg Val Glu Glu
        210                 215                 220

    Pro Gln Ser Leu Leu His Leu Ser Arg Leu Cys Val Arg His Ala Leu
    225                 230                 235                 240

    Gly Asp Thr Arg Leu Gly Gln Ile Ser Thr Leu Pro Leu Pro Pro Ala
                    245                 250                 255

    Met Lys Arg Tyr Leu Leu Tyr Lys
                260
```

(2) INFORMATION FOR SEQ ID NO:47:

(i) SEQUENCE CHARACTERISTICS:
(A) LENGTH: 5615 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:47:

```
GTACTTTCTT TATATCTCCA TAATTTTATT TACTATTACT ACATGATACA TTATTTTATA    60
AAAGTCTTTG TAACCTCCTT AAGGATTCAC TGCTTAATCT CCAGTGCTTA GCACAAATCA   120
TTAAATGCGA ACCAGAAACT CTTCCAAATG TGTTACATCT ATAACCTCAT TGGATTCTCA   180
CTACCAACCC CATGCAATAG ATACTAATGT GATCTCTGTC TTACAGAGGA AGAAACAGGC   240
ACAGGGAGGT TCAGTAATTT GCCCAAGGTC ATACACACAC TGGCCTTCAG GTATTCATGC   300
CCGGGGAGTC TGGTCCCACA GCTGGCATGT TTGCCATTAT ATTATATTGC CTCCTTATAG   360
TGTCGGCACT CATTAAGCAC ATTGACAGCT ATGCTTGGTG AGTGACTACT ATGTACCCAG   420
CTCTGTGCTA CATGCTTTAC CTGGATTATT TCAACTGCAC AACAACCCTG TGAGGTAACT   480
ACCATCATTG CTCCTATTTT ACATAACAGA AAACTACAGA AATCTGGGGC TGGGCGTAGT   540
GGCTCATGCC TGAAATCCCA GCACTTTGGG AGACCCTGTC TCTAAAAAAA ATTTTTTTTT   600
GGCCGGACGT GGTGGCTCAC ACCTGTAATC TCAGCACTTT GGGAGGCTAA GGCAGGCAGA   660
TCACAAGGTC AGGAGTTCTA GACCAGCCTG GCCAACATGG CAAAACCCTG TGTCTACTAA   720
AAATACAAAA AATAGCTAGG CGTGGTGGCA GGTGCCTGTA ATCCCAGCTA CTCAGGAGGC   780
TGAGGCAGGA GAATCCCCTG AACCTGGGAG ATGGAGGTTA CAGAGAGCCG AGATCGTGCC   840
```

GCTGCACTCC AGCCTGGGCA ACAAGAGCAA GACTCTGTCT CGAAAAAAAT AAAAATAAAA 900
ATAAAAATAT TTTTTTAAAA ATTAGCTGGG TGTGGTAGCA CATGCCTGTA GTCCCAGCTA 960
CTTGGGAGGC TGAGGTAGGA GGATCACTTG AGCCCAGGAG GTCAAGGCTG CAGTGGGCTG 1020
TGATGGCGCC ACTGCACTCT AGCCTTGGTG ACAGCAAGAC CCTGTCTCAA AAAAAAAAAA 1080
AAGAGAAATC GGGCAACTTC CCCAAGATCG CGCAGTTAAC TAGTGGCATA GCTTCACTCA 1140
AACTCGAAGT CTTAATCAGG ACACTCTACC AAATGAGATC AACGGCTCAG TAATGGATTG 1200
GCATCCAGTA TGAAGACTGG ACCAGCAGGG AGAACTATGA TGCGTACAGC CTAGAGCCTG 1260
AAGCAGATTT CACAGCCTCA GAGGTGGCAC AGGCTGACTC ACAACCCGGG GCAGAAAGGG 1320
ACCAGCCCAG AAACAGTGAC CCAGAATCAC AGGGAAGTAG AAATGGGATT CGGCACAATG 1380
AAGCCCCTCC TTGACCCCAT GCTCCTTACC CTCAGGGGCG CAGGAGTTAG TCGCTCAGGC 1440
GGCTCAAAGG TCTTGACGGT GGAGAACACC ATCCCCAGGG ATTCCCGACG CGGTGATGCC 1500
ATCAAAGCGT TAATTCTGAG ATGGGCCTGC CCGGGTGCGG ACTCTGCCGC AGCAAGAGAA 1560
GGGTTAACTG CCCCGGGCCT TCGCCGTGGG GGCGGGGCCT CGGGGAGGGT CACAGCCCGG 1620
GACTGAGACC CGAGGTTAAC CGCCCGGGGT GGGCTCCACG GGGGCGGGGC ATGCTCTCCG 1680
CGGCTGCTGC CGGTATAGAG CGGTAACTGC CCAGGAGGGG GCGGGGCCCC ACAGGGGCGT 1740
GGCCTCGGAG CTGCACGGCC GTGGGCGGCG ATGAGAGGGT TAAGCCCCAG AGGGCCCTGG 1800
AGGGGCGGGG CCGCGGGACG GGCTCGGCCC AAGGGAGGAG CTGGGGGCGG AAGCGGCCGG 1860
CGGTCTGCGC CCTGCGCGCC TCGGCTTCTT TCCGCCCGGC TCCTTCAGAG GCCCGGCGAC 1920
CTCCAGGGCT GGGAAGTCAA CCGAGGTTCG GGGGCAGCGG CGAGGGCTCC GGGCGAGTAA 1980
GGGGGATGGT CCATGCTGAG GCCCAAATGG GGCGAACTCG CGAGAGTCTC TGGCGACCTG 2040
GATCAGATGG GGCGAGGGCA GATGAAGGGC CCAGGAGCTT TGGGGCAGCG AGGAGGGAGG 2100
AGCGGGCCCG TTGGCAAACT TGGGTGAAAG GATGGGGTAC CTGGGTGACG AGCCCCCGCC 2160
AGGATTCTGC TCTTCACGCC CCTTTTCTCC CAGCTCCCTT CCAGGTCAAT CCAAACTGGA 2220
GCTCAACTTT CAGAAGAGAA AGACGCCCCA GCAAGCCTCT TTCGGGGAGT CCTCTAGCTC 2280
CTCACCTCCA TGGGCCAGAC AGCTCTGGCA GGGGGCAGCA GCAGCACCCC CACGCCACAG 2340
GCCCTGTACC CTGACCTCTC CTGTCCCGAG GGCTTGGAAG AGCTGCTGTC TGCACCCCCT 2400
CCTGACCTGG GGGCCCAGCG GCGCCACGGT TGGAACCCCA AAGACTGTTC AGAGAACATC 2460
GAGGTCAAGG AAGGAGGGTT GTACTTTGAG CGGCGGCCCG TGGCCCAGAG CACTGATGGG 2520
GCCCGGGGTA AGAGGGGCTA TTCAAGGGGC CTGCACGCCT GGGAGATCAG CTGGCCCCTA 2580
GAGCAGAGGG GCACGCATGC CGTGGTGGGC GTGGCCACGG CCCTCGCCCC GCTGCAGACT 2640
GACCACTACG CGGCGCTGCT GGGCAGCAAC AGCGAGTCGT GGGGCTGGGA CATCGGGCGG 2700
GGGAAGCTGT ACCATCAGAG CAAGGGGCCC GGAGCCCCCC AGTATCCAGC GGGAACTCAG 2760
GGTGAGCAGC TGGAGGTGCC AGAGAGACTG CTGGTGGTTC TGGACATGGA GGAGGGAACT 2820
CTGGGCTACG CTATTGGGGG CACCTACCTG GGGCCAGCAT TCCGCGGACT GAAGGGCAGG 2880

ACCCTCTATC CGGCAGTAAG CGCTGTCTGG GGCCAGTGCC AGGTCCGCAT CCGCTACCTG 2940
GGCGAAAGGA GAGGTGAGGC CTGGGGCAGA CGTGGGGAGA ACTTTCTGTC CCTGGTGGCA 3000
GTGGTTTGGG ATGGAAACTC TTCTGACAAG AGCAGAGGGG ATGGACCTTC ATCCAGCCTG 3060
CCTCAACCTC TGTTCAGTGC TGGGAAAGGC TAGGGGTCTT CACAGCTGTT ATTTAATTTA 3120
ACCCAACAGC AATAGAGGTG AAACAGGCTT GAGAAAGCAA CTTTCTCAAG TTCTCTTGGC 3180
CAGTAAATGG TGAACCTTCA GAATGGAGGG AGGAACTGCA GGGATGAGAG AATTCAGGAG 3240
ATATCAACCC CTGAGCAAGA GGTGCAAAGC GTTAGGTACT GGGTTTGATG TACAGGTCCA 3300
AAAGAAGGAT GGGCAGAGCC AGGTACCCAG GCTGTATACC GGATTCCCTG GGCTCTAACC 3360
TGTCTCTGTG CCACATACCT ACTTCCTTCC TCAGCCACAC CTCTGGATGG AGACACTGGG 3420
GCCCTGGGCA CCAGGGAGGA GAGCAGTGGA GGAGGCAGGG CCTTAGGGTG GGGCAGCAGG 3480
GGAGGAGCCT CCCCAGGAAC TGACTGGGTC CAGGGCTTGG AGCTGCTCTC TGCAGTTGTG 3540
TGGGCTGTAG AGTGGAGGGC CATCCCTCCT CACCTCAGCC CCAGCTCCCA AGCCTCTGGA 3600
GTCAAAGCCT GGGCCAGCTC CACCACTGTC AGAGCCACCT TGGCCTGTTG TTTAGAGGGC 3660
CTTAGCCAGC TCTTCACCCC CAGCTCTGAC TAGGGATGTG TGAAATCTTA TCTGGGAGGC 3720
AGAACTTCCG GGTATCTCAA ATTCCCCTTT CAGCCAGGTG GGCACACTCG AAGCAGGAAA 3780
GCAGAAAGGC ATCTGAGTAG GACCCCGTAG TTTGAGGACA TCTGGCTGGT GGCTGCACCC 3840
ATACTTACAT TCCCCTCCTT CTCTCTCCCA GCGGAGCCAC ACTCCCTTCT GCACCTGAGC 3900
CGCCTGTGTG TGCGCCACAA CCTGGGGGAT ACCCGGCTCG GCCAGGTGTC TGCCCTGCCC 3960
TTGCCCCCTG CCATGAAGCG CTACCTGCTC TACCAGTGAG CCCTGTGATA CCACAGACTG 4020
TGCTGAGGTC TTGCCACCAC CCCTCCCCTT GGGGAGGTGG GGAGGCACTG CTGGCCTAGA 4080
CCAGCTGCTG AAAGCTGGTG AGGCTGAGCC CCTACCCCAA CCCAAGCTCT GCGGAAATCA 4140
ACAGCCCCAG AGCCACTTGG AGGGAGGAAG AAAGGGAGCC GGCGTTCAAG GCTATGACAG 4200
TCTGCTACGC AAAACATTTT TTCAAGTAAA AATAGTAAGA GATGTTGTTA TAGAAACCTG 4260
TTCTTGTTTT TTTTTTTTTC TTGCACAAAT GATCATTTAT ATAGCTGCCT CAAAAAGGAA 4320
GATTATCTGG GCAAGTCCAG TGAAGGCAGA CAAACCACAA GACCTAGTGC CAGGTTTATT 4380
CCCTCACATG GGTGGTTCAC ATACACAGCA CAGAGGCACG GGCACCATGG GAGAGGGCAG 4440
CACTCCTGCC TTCTGAGGGG ATCTTGGCCT CACGGTGTAA GAAGGGAGAG GATGGTTTCT 4500
CTTCTGCCCT CACTAGGGCC TAGGGAACCC AGGAGCAAAT CCCACCACGC CTTCCATCTC 4560
TCAGCCAAGG AGAAGCCACC TTGGTGACGT TTAGTTCCAA CCATTATAGT AAGTGGAGAA 4620
GGGATTGGCC TGGTCCCAAC CATTACAGGG TGAAGATATA AACAGTAAAG GAAGATACAG 4680
TTTGGATGAG GCCACAGGAA GGAGCAGATG ACACCATCAG AAGCATATGC AGGGAAAGGG 4740
CAGTTACTGG GCTTCTGGGC TGCTTAGTCC CTGGCTTGGC AGGAAGGGTA GGGAAGATGG 4800
ATGGGGCTCA TTGTTTGGCA TTGATGATGT CCACGAATTC GGGCTTGAGG GAAGCACCAC 4860
CCACAAGGAA GCCATCCACA TCAGGCTGGC TGGCCAGCTC CTTGCAGGTT GCCCCAGTCA 4920

CAGAGCCTGG GAAGGGAGCA GAACAAGGGC TTGGTCAAGA ATGGGATGAG TCTGCCCCAT 4980

CCCCACCTCC ATGTCCGAGG GCTCAGTCTA GTCCTCAGCC CACTCCACCT CAGCCGGGAA 5040

CCAAAGCCAC TCACCTCCAT AAATGATACG GGTGCTCTGA GCCACCGCAT CAGAGACGTT 5100

GGACTTCAGC CATCCTCGGA GCTTCTCGTG TACTTCCTGG GCCTAGAACA AGAAGCTGGC 5160

CTAAGTAAGA CCTTTTCTGC CTCTCTAAGA GGAAAAATCA CTGGCACCAG TGGACACTTA 5220

GTGTGGTTTC TGACTGAGTC AGAGTACCAG GGCTCTGATC CAAGCCAGGC CCTGGACTGG 5280

ATGCCCTTGG ACAAGTCACT GTCTCTGGGT TCAAGGTCTC TGTGTCTTTG AAATAAGGGG 5340

TTGCCCCATG TGGGCTGTGT CTGTCCAAAC CTATTGAGGC AGGCTGGGAT GAGGGCAGGG 5400

CTCCTGGGCC CGGTTACCTG TTGGGGTGTT GCAGTCTTGC CAGTACCAAT GGCCCACACA 5460

GGCTCATAGG CCAGGACGAC CTTGCTCCAG TCCTTCACGT TATCTGCAGG GCAGAGATAC 5520

AGATGGAGGG AAGGGTGAAC AAGAAAGAGC TCTCCAGCCA GGTTCTCCGG AGTACGAAGA 5580

ACGGTGGCCT ACTGCCCCCT AGTGGACATT GGGGG 5615

(2) INFORMATION FOR SEQ ID NO:48:

(i) SEQUENCE CHARACTERISTICS:
(A) LENGTH: 263 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:48:

```
Met Gly Gln Thr Ala Leu Ala Gly Gly Ser Ser Ser Thr Pro Thr Pro
1               5                   10                  15

Gln Ala Leu Tyr Pro Asp Leu Ser Cys Pro Glu Gly Leu Glu Glu Leu
            20                  25                  30

Leu Ser Ala Pro Pro Pro Asp Leu Gly Ala Gln Arg Arg His Gly Trp
        35                  40                  45

Asn Pro Lys Asp Cys Ser Glu Asn Ile Glu Val Lys Glu Gly Gly Leu
    50                  55                  60

Tyr Phe Glu Arg Arg Pro Val Ala Gln Ser Thr Asp Gly Ala Arg Gly
65                  70                  75                  80

Lys Arg Gly Tyr Ser Arg Gly Leu His Ala Trp Glu Ile Ser Trp Pro
                85                  90                  95

Leu Glu Gln Arg Gly Thr His Ala Val Val Gly Val Ala Thr Ala Leu
            100                 105                 110

Ala Pro Leu Gln Thr Asp His Tyr Ala Ala Leu Leu Gly Ser Asn Ser
        115                 120                 125

Glu Ser Trp Gly Trp Asp Ile Gly Arg Gly Lys Leu Tyr His Gln Ser
    130                 135                 140
```

```
Lys Gly Pro Gly Ala Pro Gln Tyr Pro Ala Gly Thr Gln Gly Glu Gln
145                 150                 155                 160

Leu Glu Val Pro Glu Arg Leu Leu Val Val Leu Asp Met Glu Glu Gly
                165                 170                 175

Thr Leu Gly Tyr Ala Ile Gly Gly Thr Tyr Leu Gly Pro Ala Phe Arg
            180                 185                 190

Gly Leu Lys Gly Arg Thr Leu Tyr Pro Ala Val Ser Ala Val Trp Gly
        195                 200                 205

Gln Cys Gln Val Arg Ile Arg Tyr Leu Gly Glu Arg Arg Ala Glu Pro
    210                 215                 220

His Ser Leu Leu His Leu Ser Arg Leu Cys Val Arg His Asn Leu Gly
225                 230                 235                 240

Asp Thr Arg Leu Gly Gln Val Ser Ala Leu Pro Leu Pro Pro Ala Met
                245                 250                 255

Lys Arg Tyr Leu Leu Tyr Gln
            260
```

(2) INFORMATION FOR SEQ ID NO:49:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:49:

AGCTAGATCT GGACCCTACA ATGGCAGC 28

(2) INFORMATION FOR SEQ ID NO:50:

(i) SEQUENCE CHARACTERISTICS:
(A) LENGTH: base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:50:

AGCTAGATCT GCCATCCTAC TCGAGGGGCC AGCTGG 36

SEQUENCE LISTING

(1) GENERAL INFORMATION:

(i) APPLICANT: (Other than US) THE WALTER AND ELIZA HALL INSTITUTE OF MEDICAL RESEARCH
(US Only)

(ii) TITLE OF INVENTION: THERAPEUTIC AND DIAGNOSTIC AGENTS

(iii) NUMBER OF SEQUENCES: 49

(iv) CORRESPONDENCE ADDRESS:
(A) ADDRESSEE: DAVIES COLLISON CAVE
(B) STREET: 1 LITTLE COLLINS STREET
(C) CITY: MELBOURNE
(D) STATE: VICTORIA
(E) COUNTRY: AUSTRALIA
(F) ZIP: 3000

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: PCT INTERNATIONAL
(B) FILING DATE: 31-OCT-1997

(vi) PRIOR APPLICATION DATA:
(A) APPLICATION NUMBER: PO5117
(B) FILING DATE: 14-FEB-1997

(vii) PRIOR APPLICATION DATA:
(A) APPLICATION NUMBER: PO 3384
(B) FILING DATE: 01-NOV-1996

(viii) ATTORNEY/AGENT INFORMATION:
(A) NAME: HUGHES DR, E JOHN L
(C) REFERENCE/DOCKET NUMBER: EJH/EK

(ix) TELECOMMUNICATION INFORMATION:
(A) TELEPHONE: +61 3 9254 2777
(B) TELEFAX: +61 3 9254 2770

(2) INFORMATION FOR SEQ ID NO:1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:1:

CACGCCGCCC ACGTGAAGGC 20

(2) INFORMATION FOR SEQ ID NO:2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:2:

TTCGCCAATG ACAAGACGCT 20

(2) INFORMATION FOR SEQ ID NO:3:

(i) SEQUENCE CHARACTERISTICS:
(A) LENGTH: 1236 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 1..636

(xi) SEQUENCE DESCRIPTION: SEQ ID NO:3:

```
CGAGGCTCAA GCTCCGGGCG GATTCTGCGT GCCGCTCTCG CTCCTTGGGG TCTGTTGGCC    -101

GGCCTGTGCC ACCCGGACGC CCGGCTCACT GCCTCTGTCT CCCCCATCAG CGCAGCCCCG     -41

GACGCTATGG CCCACCCCTC CAGCTGGCCC CTCGAGTAGG                            -1

ATG GTA GCA CGC AAC CAG GTG GCA GCC GAC AAT GCG ATC TCC CCG GCA        48
Met Val Ala Arg Asn Gln Val Ala Ala Asp Asn Ala Ile Ser Pro Ala
  1               5                  10                  15

GCA GAG CCC CGA CGG CGG TCA GAG CCC TCC TCG TCC TCG TCT TCG TCC        96
Ala Glu Pro Arg Arg Arg Ser Glu Pro Ser Ser Ser Ser Ser Ser Ser
            20                  25                  30

TCG CCA GCG GCC CCC GTG CGT CCC CGG CCC TGC CCG GCG GTC CCA GCC       144
```

```
Ser Pro Ala Ala Pro Val Arg Pro Arg Pro Cys Pro Ala Val Pro Ala
        35                  40                  45

CCA GCC CCT GGC GAC ACT CAC TTC CGC ACC TTC CGC TCC CAC TCC GAT      192
Pro Ala Pro Gly Asp Thr His Phe Arg Thr Phe Arg Ser His Ser Asp
    50                  55                  60

TAC CGG CGC ATC ACG CGG ACC AGC GCG CTC CTG GAC GCC TGC GGC TTC      240
Tyr Arg Arg Ile Thr Arg Thr Ser Ala Leu Leu Asp Ala Cys Gly Phe
65                  70                  75                  80

TAT TGG GGA CCC CTG AGC GTG CAC GGG GCG CAC GAG CGG CTG CGT GCC      288
Tyr Trp Gly Pro Leu Ser Val His Gly Ala His Glu Arg Leu Arg Ala
                85                  90                  95

GAG CCC GTG GGC ACC TTC TTG GTG CGC GAC AGT CGT CAA CGG AAC TGC      336
Glu Pro Val Gly Thr Phe Leu Val Arg Asp Ser Arg Gln Arg Asn Cys
            100                 105                 110

TTC TTC GCG CTC AGC GTG AAG ATG GCT TCG GGC CCC ACG AGC ATC CGC      384
Phe Phe Ala Leu Ser Val Lys Met Ala Ser Gly Pro Thr Ser Ile Arg
        115                 120                 125

GTG CAC TTC CAG GCC GGC CGC TTC CAC TTG GAC GGC AGC CGC GAG ACC      432
Val His Phe Gln Ala Gly Arg Phe His Leu Asp Gly Ser Arg Glu Thr
    130                 135                 140

TTC GAC TGC CTT TTC GAG CTG CTG GAG CAC TAC GTG GCG GCG CCG CGC      480
Phe Asp Cys Leu Phe Glu Leu Leu Glu His Tyr Val Ala Ala Pro Arg
145                 150                 155                 160

CGC ATG TTG GGG GCC CCG CTG CGC CAG CGC CGC GTG CGG CCG CTG CAG      528
Arg Met Leu Gly Ala Pro Leu Arg Gln Arg Arg Val Arg Pro Leu Gln
                165                 170                 175

GAG CTG TGT CGC CAG CGC ATC GTG GCC GCC GTG GGT CGC GAG AAC CTG      576
Glu Leu Cys Arg Gln Arg Ile Val Ala Ala Val Gly Arg Glu Asn Leu
            180                 185                 190

GCG CGC ATC CCT CTT AAC CCG GTA CTC CGT GAC TAC CTG AGT TCC TTC      624
Ala Arg Ile Pro Leu Asn Pro Val Leu Arg Asp Tyr Leu Ser Ser Phe
        195                 200                 205

CCC TTC CAG ATC TGA CCGGCTG CCGCTGTGCC GCAGCATTAA GTGGGGGCGC         676
Pro Phe Gln Ile  *
    210

CTTATTATTT CTTATTATTA ATTATTATTA TTTTTCTGGA ACCACGTGGG AGCCCTCCCC    736

GCCTGGGTCG GAGGGAGTGG TTGTGGAGGG TGAGATGCCT CCCACTTCTG GCTGGAGACC    796

TCATCCCACC TCTCAGGGGT GGGGGTGCTC CCCTCCTGGT GCTCCCTCCG GGTCCCCCCT    856

GGTTGTAGCA GCTTGTGTCT GGGGCCAGGA CCTGAATTCC ACTCCTACCT CTCCATGTTT    916

ACATATTCCC AGTATCTTTG CACAAACCAG GGGTCGGGGA GGGTCTCTGG CTTCATTTTT    976

CTGCTGTGCA GAATATCCTA TTTTATATTT TTACAGCCAG TTTAGGTAAT AAACTTTATT   1036

ATGAAAGTTT TTTTTTAAAA GAAAAAAAAA AAAAAAAAA                          1075
```

(2) INFORMATION FOR SEQ ID NO:4:

(i) SEQUENCE CHARACTERISTICS:
(A) LENGTH: 212 amino acids

```
            (B) TYPE: amino acid
            (D) TOPOLOGY: linear

      (ii) MOLECULE TYPE: protein

      (xi) SEQUENCE DESCRIPTION: SEQ ID NO:4:

Met Val Ala Arg Asn Gln Val Ala Ala Asp Asn Ala Ile Ser Pro Ala
 1               5                  10                  15

Ala Glu Pro Arg Arg Arg Ser Glu Pro Ser Ser Ser Ser Ser Ser Ser
            20                  25                  30

Ser Pro Ala Ala Pro Val Arg Pro Arg Pro Cys Pro Ala Val Pro Ala
        35                  40                  45

Pro Ala Pro Gly Asp Thr His Phe Arg Thr Phe Arg Ser His Ser Asp
    50                  55                  60

Tyr Arg Arg Ile Thr Arg Thr Ser Ala Leu Leu Asp Ala Cys Gly Phe
65                  70                  75                  80

Tyr Trp Gly Pro Leu Ser Val His Gly Ala His Glu Arg Leu Arg Ala
                85                  90                  95

Glu Pro Val Gly Thr Phe Leu Val Arg Asp Ser Arg Gln Arg Asn Cys
            100                 105                 110

Phe Phe Ala Leu Ser Val Lys Met Ala Ser Gly Pro Thr Ser Ile Arg
        115                 120                 125

Val His Phe Gln Ala Gly Arg Phe His Leu Asp Gly Ser Arg Glu Thr
    130                 135                 140

Phe Asp Cys Leu Phe Glu Leu Leu Glu His Tyr Val Ala Ala Pro Arg
145                 150                 155                 160

Arg Met Leu Gly Ala Pro Leu Arg Gln Arg Arg Val Arg Pro Leu Gln
                165                 170                 175

Glu Leu Cys Arg Gln Arg Ile Val Ala Ala Val Gly Arg Glu Asn Leu
            180                 185                 190

Ala Arg Ile Pro Leu Asn Pro Val Leu Arg Asp Tyr Leu Ser Ser Phe
        195                 200                 205

Pro Phe Gln Ile
    210


(2) INFORMATION FOR SEQ ID NO:5:

     (i) SEQUENCE CHARACTERISTICS:
          (A) LENGTH: 1121 base pairs
          (B) TYPE: nucleic acid
          (C) STRANDEDNESS: single
          (D) TOPOLOGY: linear

     (ii) MOLECULE TYPE: DNA

     (ix) FEATURE:
          (A) NAME/KEY: CDS
          (B) LOCATION: 223..819
```

(xi) SEQUENCE DESCRIPTION: SEQ ID NO:5:

```
GCGATCTGTG GGTGACAGTG TCTGCGAGAG ACTTTGCCAC ACCATTCTGC CGGAATTTGG      60

AGAAAAAGAA CCAGCCGCTT CCAGTCCCCT CCCCCTCCGC CACCATTTCG GACACCCTGC     120

ACACTCTCGT TTTGGGGTAC CCTGTGACTT CCAGGCAGCA CGCGAGGTCC ACTGGCCCCA     180

GCTCGGGCGA CCAGCTGTCT GGGACGTGTT GACTCATCTC CC ATG ACC CTG CGG        234
                                              Met Thr Leu Arg
                                                1

TGC CTG GAG CCC TCC GGG AAT GGA GCG GAC AGG ACG CGG AGC CAG TGG       282
Cys Leu Glu Pro Ser Gly Asn Gly Ala Asp Arg Thr Arg Ser Gln Trp
  5                  10                  15                  20

GGG ACC GCG GGG TTG CCG GAG GAA CAG TCC CCC GAG GCG GCG CGT CTG       330
Gly Thr Ala Gly Leu Pro Glu Glu Gln Ser Pro Glu Ala Ala Arg Leu
                 25                  30                  35

GCG AAA GCC CTG CGC GAG CTC AGT CAA ACA GGA TGG TAC TGG GGA AGT       378
Ala Lys Ala Leu Arg Glu Leu Ser Gln Thr Gly Trp Tyr Trp Gly Ser
             40                  45                  50

ATG ACT GTT AAT GAA GCC AAA GAG AAA TTA AAA GAG GCT CCA GAA GGA       426
Met Thr Val Asn Glu Ala Lys Glu Lys Leu Lys Glu Ala Pro Glu Gly
         55                  60                  65

ACT TTC TTG ATT AGA GAT AGT TCG CAT TCA GAC TAC CTA CTA ACT ATA       474
Thr Phe Leu Ile Arg Asp Ser Ser His Ser Asp Tyr Leu Leu Thr Ile
     70                  75                  80

TCC GTT AAG ACG TCA GCT GGA CCG ACT AAC CTG CGG ATT GAG TAC CAA       522
Ser Val Lys Thr Ser Ala Gly Pro Thr Asn Leu Arg Ile Glu Tyr Gln
 85                  90                  95                 100

GAT GGG AAA TTC AGA TTG GAT TCT ATC ATA TGT GTC AAG TCC AAG CTT       570
Asp Gly Lys Phe Arg Leu Asp Ser Ile Ile Cys Val Lys Ser Lys Leu
                105                 110                 115

AAA CAG TTT GAC AGT GTG GTT CAT CTG ATT GAC TAC TAT GTC CAG ATG       618
Lys Gln Phe Asp Ser Val Val His Leu Ile Asp Tyr Tyr Val Gln Met
            120                 125                 130

TGC AAG GAT AAA CGG ACA GGC CCA GAA GCC CCA CGG AAT GGG ACT GTT       666
Cys Lys Asp Lys Arg Thr Gly Pro Glu Ala Pro Arg Asn Gly Thr Val
        135                 140                 145

CAC CTG TAC CTG ACC AAA CCT CTG TAT ACA TCA GCA CCC ACT CTG CAG       714
His Leu Tyr Leu Thr Lys Pro Leu Tyr Thr Ser Ala Pro Thr Leu Gln
    150                 155                 160

CAT TTC TGT CGA CTC GCC ATT AAC AAA TGT ACC GGT ACG ATC TGG GGA       762
His Phe Cys Arg Leu Ala Ile Asn Lys Cys Thr Gly Thr Ile Trp Gly
165                 170                 175                 180

CTG CCT TTA CCA ACA AGA CTA AAA GAT TAC TTG GAA GAA TAT AAA TTC       810
Leu Pro Leu Pro Thr Arg Leu Lys Asp Tyr Leu Glu Glu Tyr Lys Phe
                185                 190                 195

CAG GTA TAAGTATTTC TCTCTCTTTT TCGTTTTTTT TTAAAAAAAA AAAAACACAT        866
Gln Val

GCCTCATATA GACTATCTCC GAATGCAGCT ATGTGAAAGA GAACCCAGAG GCCCTCCTCT     926

GGATAACTGC GCAGAATTCT CTCTTAAGGA CAGTTGGGCT CAGTCTAACT TAAAGGTGTG     986
```

```
AAGATGTAGC TAGGTATTTT AAAGTTCCCC TTAGGTAGTT TTAGCTGAAT GATGCTTTCT    1046
TTCCTATGGC TGCTCAAGAT CAAATGGCCC TTTTAAATGA AACAAAACAA AACAAAACAA    1106
AAAAAAAAAA AAAAA                                                     1121
```

(2) INFORMATION FOR SEQ ID NO:6:

(i) SEQUENCE CHARACTERISTICS:
(A) LENGTH: 198 amino acids
(B) TYPE: amino acid
(D) TOPOLOGY: linear

(ii) MOLECULE TYPE: protein

(xi) SEQUENCE DESCRIPTION: SEQ ID NO:6:

```
Met Thr Leu Arg Cys Leu Glu Pro Ser Gly Asn Gly Ala Asp Arg Thr
 1               5                  10                  15

Arg Ser Gln Trp Gly Thr Ala Gly Leu Pro Glu Glu Gln Ser Pro Glu
            20                  25                  30

Ala Ala Arg Leu Ala Lys Ala Leu Arg Glu Leu Ser Gln Thr Gly Trp
        35                  40                  45

Tyr Trp Gly Ser Met Thr Val Asn Glu Ala Lys Glu Lys Leu Lys Glu
    50                  55                  60

Ala Pro Glu Gly Thr Phe Leu Ile Arg Asp Ser Ser His Ser Asp Tyr
65                  70                  75                  80

Leu Leu Thr Ile Ser Val Lys Thr Ser Ala Gly Pro Thr Asn Leu Arg
                85                  90                  95

Ile Glu Tyr Gln Asp Gly Lys Phe Arg Leu Asp Ser Ile Ile Cys Val
            100                 105                 110

Lys Ser Lys Leu Lys Gln Phe Asp Ser Val Val His Leu Ile Asp Tyr
        115                 120                 125

Tyr Val Gln Met Cys Lys Asp Lys Arg Thr Gly Pro Glu Ala Pro Arg
    130                 135                 140

Asn Gly Thr Val His Leu Tyr Leu Thr Lys Pro Leu Tyr Thr Ser Ala
145                 150                 155                 160

Pro Thr Leu Gln His Phe Cys Arg Leu Ala Ile Asn Lys Cys Thr Gly
                165                 170                 175

Thr Ile Trp Gly Leu Pro Leu Pro Thr Arg Leu Lys Asp Tyr Leu Glu
            180                 185                 190

Glu Tyr Lys Phe Gln Val
        195
```

(2) INFORMATION FOR SEQ ID NO:7:

(i) SEQUENCE CHARACTERISTICS:
(A) LENGTH: 2187 base pairs
(B) TYPE: nucleic acid
(C) STRANDEDNESS: single
(D) TOPOLOGY: linear

(ii) MOLECULE TYPE: DNA

```
(ix) FEATURE:
     (A) NAME/KEY: CDS
     (B) LOCATION: 18..695

(xi) SEQUENCE DESCRIPTION: SEQ ID NO:7:

CGCTGGCTCC GTGCGCC ATG GTC ACC CAC AGC AAG TTT CCC GCC GCC GGG      50
                   Met Val Thr His Ser Lys Phe Pro Ala Ala Gly
                     1               5                  10

ATG AGC CGC CCC CTG GAC ACC AGC CTG CGC CTC AAG ACC TTC AGC TCC     98
Met Ser Arg Pro Leu Asp Thr Ser Leu Arg Leu Lys Thr Phe Ser Ser
             15                  20                  25

AAA AGC GAG TAC CAG CTG GTG GTG AAC GCC GTG CGC AAG CTG CAG GAG    146
Lys Ser Glu Tyr Gln Leu Val Val Asn Ala Val Arg Lys Leu Gln Glu
         30                  35                  40

AGC GGA TTC TAC TGG AGC GCC GTG ACC GGC GGC GAG GCG AAC CTG CTG    194
Ser Gly Phe Tyr Trp Ser Ala Val Thr Gly Gly Glu Ala Asn Leu Leu
     45                  50                  55

CTC AGC GCC GAG CCC GCG GGC ACC TTT CTT ATC CGC GAC AGC TCG GAC    242
Leu Ser Ala Glu Pro Ala Gly Thr Phe Leu Ile Arg Asp Ser Ser Asp
 60                  65                  70                  75

CAG CGC CAC TTC TTC ACG TTG AGC GTC AAG ACC CAG TCG GGG ACC AAG    290
Gln Arg His Phe Phe Thr Leu Ser Val Lys Thr Gln Ser Gly Thr Lys
                 80                  85                  90

AAC CTA CGC ATC CAG TGT GAG GGG GGC AGC TTT TCG CTG CAG AGT GAC    338
Asn Leu Arg Ile Gln Cys Glu Gly Gly Ser Phe Ser Leu Gln Ser Asp
             95                 100                 105

CCC CGA AGC ACG CAG CCA GTT CCC CGC TTC GAC TGT GTA CTC AAG CTG    386
Pro Arg Ser Thr Gln Pro Val Pro Arg Phe Asp Cys Val Leu Lys Leu
        110                 115                 120

GTG CAC CAC TAC ATG CCG CCT CCA GGG ACC CCC TCC TTT TCT TTG CCA    434
Val His His Tyr Met Pro Pro Pro Gly Thr Pro Ser Phe Ser Leu Pro
    125                 130                 135

CCC ACG GAA CCC TCG TCC GAA GTT CCG GAG CAG CCA CCT GCC CAG GCA    482
Pro Thr Glu Pro Ser Ser Glu Val Pro Glu Gln Pro Pro Ala Gln Ala
140                 145                 150                 155

CTC CCC GGG AGT ACC CCC AAG AGA GCT TAC TAC ATC TAT TCT GGG GGC    530
Leu Pro Gly Ser Thr Pro Lys Arg Ala Tyr Tyr Ile Tyr Ser Gly Gly
                160                 165                 170

GAG AAG ATT CCG CTG GTA CTG AGC CGA CCT CTC TCC TCC AAC GTG GCC    578
Glu Lys Ile Pro Leu Val Leu Ser Arg Pro Leu Ser Ser Asn Val Ala
            175                 180                 185

ACC CTC CAG CAT CTT TGT CGG AAG ACT GTC AAC GGC CAC CTG GAC TCC    626
Thr Leu Gln His Leu Cys Arg Lys Thr Val Asn Gly His Leu Asp Ser
        190                 195                 200

TAT GAG AAA GTG ACC CAG CTG CCT GGA CCC ATT CGG GAG TTC CTG GAT    674
Tyr Glu Lys Val Thr Gln Leu Pro Gly Pro Ile Arg Glu Phe Leu Asp
    205                 210                 215

CAG TAT GAT GCT CCA CTT TAAGGAGCAA AAGGGTCAGA GGGGGGCCTG           722
Gln Tyr Asp Ala Pro Leu
220                 225
```

```
GGTCGGTCGG TCGCCTCTCC TCCGAGGCAC ATGGCACAAG CACAAAAATC CAGCCCCAAC     782
GGTCGGTAGC TCCCAGTGAG CCAGGGGCAG ATTGGCTTCT TCCTCAGGCC CTCCACTCCC     842
GCAGAGTAGA GCTGGCAGGA CCTGGAATTC GTCTGAGGGG AGGGGGAGCT GCCACCTGCT     902
TTCCCCCCTC CCCCAGCTCC AGCTTCTTTC AAGTGGAGCC AGCCGGCCTG GCCTGGTGGG     962
ACAATACCTT TGACAAGCGG ACTCTCCCCT CCCCTTCCTC CACACCCCCT CTGCTTCCCA    1022
AGGGAGGTGG GGACACCTCC AAGTGTTGAA CTTAGAACTG CAAGGGGAAT CTTCAAACTT    1082
TCCCGCTGGA ACTTGTTTGC GCTTTGATTT GGTTTGATCA AGAGCAGGCA CCTGGGGGAA    1142
GGATGGAAGA GAAAAGGGTG TGTGAAGGGT TTTTATGCTG GCCAAAGAAA TAACCACTCC    1202
CACTGCCCAA CCTAGGTGAG GAGTGGTGGC TCCTGGCTCT GGGGAGAGTG GCAAGGGGTG    1262
ACCTGAAGAG AGCTATACTG GTGCCAGGCT CCTCTCCATG GGGCAGCTAA TGAAACCTCG    1322
CAGATCCCTT GCACCCCAGA ACCCTCCCCG TTGTGAAGAG GCAGTAGCAT TTAGAAGGGA    1382
GACAGATGAG GCTGGTGAGC TGGCCGCCTT TTCCAACACC GAAGGGAGGC AGATCAACAG    1442
ATGAGCCATC TTGGAGCCCA GGTTTCCCCT GGAGCAGATG GAGGGTTCTG CTTTGTCTCT    1502
CCTATGTGGG GCTAGGAGAC TCGCCTTAAA TGCCCTCTGT CCCAGGGATG GGGATTGGCA    1562
CACAAGGAGC CAAACACAGC CAATAGGCAG AGAGTTGAGG GATTCACCCA GGTGGCTACA    1622
GGCCAGGGGA AGTGGCTGCA GGGGAGAGAC CCAGTCACTC CAGGAGACTC CTGAGTTAAC    1682
ACTGGGAAGA CATTGGCCAG TCCTAGTCAT CTCTCGGTCA GTAGGTCCGA GAGCTTCCAG    1742
GCCCTGCACA GCCCTCCTTT CTCACCTGGG GGGAGGCAGG AGGTGATGGA GAAGCCTTCC    1802
CATGCCGCTC ACAGGGGCCT CACGGGAATG CAGCAGCCAT GCAATTACCT GGAACTGGTC    1862
CTGTGTTGGG GAGAAACAAG TTTTCTGAAG TCAGGTATGG GGCTGGGTGG GGCAGCTGTG    1922
TGTTGGGGTG GCTTTTTTCT CTCTGTTTTG AATAATGTTT ACAATTTGCC TCAATCACTT    1982
TTATAAAAAT CCACCTCCAG CCCGCCCCTC TCCCCACTCA GGCCTTCGAG GCTGTCTGAA    2042
GATGCTTGAA AAACTCAACC AAATCCCAGT TCAACTCAGA CTTTGCACAT ATATTTATAT    2102
TTATACTCAG AAAAGAAACA TTTCAGTAAT TTATAATAAA AGAGCACTAT TTTTTAATGA    2162
AAAAAAAAAA AAAAAAAAAA AAAAA                                          2187
```

(2) INFORMATION FOR SEQ ID NO:8:

(i) SEQUENCE CHARACTERISTICS:
(A) LENGTH: 225 amino acids
(B) TYPE: amino acid
(D) TOPOLOGY: linear

(ii) MOLECULE TYPE: protein

(xi) SEQUENCE DESCRIPTION: SEQ ID NO:8:

```
Met Val Thr His Ser Lys Phe Pro Ala Ala Gly Met Ser Arg Pro Leu
  1               5                  10                  15

Asp Thr Ser Leu Arg Leu Lys Thr Phe Ser Ser Lys Ser Glu Tyr Gln
             20                  25                  30
```

```
Leu Val Val Asn Ala Val Arg Lys Leu Gln Glu Ser Gly Phe Tyr Trp
        35                  40                  45

Ser Ala Val Thr Gly Gly Glu Ala Asn Leu Leu Leu Ser Ala Glu Pro
    50                  55                  60

Ala Gly Thr Phe Leu Ile Arg Asp Ser Ser Asp Gln Arg His Phe Phe
65                  70                  75                  80

Thr Leu Ser Val Lys Thr Gln Ser Gly Thr Lys Asn Leu Arg Ile Gln
                85                  90                  95

Cys Glu Gly Gly Ser Phe Ser Leu Gln Ser Asp Pro Arg Ser Thr Gln
            100                 105                 110

Pro Val Pro Arg Phe Asp Cys Val Leu Lys Leu Val His His Tyr Met
        115                 120                 125

Pro Pro Pro Gly Thr Pro Ser Phe Ser Leu Pro Pro Thr Glu Pro Ser
    130                 135                 140

Ser Glu Val Pro Glu Gln Pro Pro Ala Gln Ala Leu Pro Gly Ser Thr
145                 150                 155                 160

Pro Lys Arg Ala Tyr Tyr Ile Tyr Ser Gly Gly Glu Lys Ile Pro Leu
                165                 170                 175

Val Leu Ser Arg Pro Leu Ser Ser Asn Val Ala Thr Leu Gln His Leu
            180                 185                 190

Cys Arg Lys Thr Val Asn Gly His Leu Asp Ser Tyr Glu Lys Val Thr
        195                 200                 205

Gln Leu Pro Gly Pro Ile Arg Glu Phe Leu Asp Gln Tyr Asp Ala Pro
    210                 215                 220

Leu
225
```

(2) INFORMATION FOR SEQ ID NO:9:

(i) SEQUENCE CHARACTERISTICS:
(A) LENGTH: 1094 base pairs
(B) TYPE: nucleic acid
(D) TOPOLOGY: linear

(ii) MOLECULE TYPE: protein

(xi) SEQUENCE DESCRIPTION: SEQ ID NO:9:

```
CTCCGGCTGG CCCCTTCTGT AGGATGGTAG CACACAACCA GGTGGCAGCC GACAATGCAG     60
TCTCCACAGC AGCAGAGCCC CGACGGCGGC CAGAACCTTC CTCCTCTTCC TCCTCCTCGC    120
CCGCGGCCCC CGCGCGCCCG CGGCCGTGCC CCGCGGTCCC GGCCCCGGCC CCCGGCGACA    180
CGCACTTCCG CACATTCCGT TCGCACGCCG ATTACCGGCG CATCACGCGC GCCAGCGCGC    240
TCCTGGACGC CTGCGGATTC TACTGGGGGC CCCTGAGCGT GCACGGGGCG CACGAGCGGC    300
TGCGCGCCGA GCCCGTGGGC ACCTTCCTGG TGCGCGACAG CCGCCAGCGG AACTGCTTTT    360
TCGCCCTTAG CGTGAAGATG GCCTCGGGAC CCACGAGCAT CCGCGTGCAC TTTCAGGCCG    420
GCCGCTTTCA CCTGGATGGC AGCCGCGAGA GCTTCGACTG CCTCTTCGAG CTGCTGGAGC    480
```

```
ACTACGTGGC GGCGCCGCGC CGCATGCTGG GGGCCCCGCT GCGCCAGCGC CGCGTGCGGC    540
CGCTGCAGGA GCTGTGCCGC CAGCGCATCG TGGCCACCGT GGGCCGCGAG AACCTGGCTC    600
GCATCCCCCT CAACCCCGTC CTCCGCGACT ACCTGAGCTC CTTCCCCTTC CAGATTTGAC    660
CGGCAGCGCC CGCCGTGCAC GCAGCATTAA CTGGGATGCC GTGTTATTTT GTTATTACTT    720
GCCTGGAACC ATGTGGGTAC CCTCCCCGGC CTGGGTTGGA GGGAGCGGAT GGGTGTAGGG    780
GCGAGGCGCC TCCCGCCCTC GGCTGGAGAC GAGGCCGCAG ACCCCTTCTC ACCTCTTGAG    840
GGGGTCCTCC CCCTCCTGGT GCTCCCTCTG GGTCCCCCTG GTTGTTGTAG CAGCTTAACT    900
GTATCTGGAG CCAGGACCTG AACTCGCACC TCCTACCTCT TCATGTTTAC ATATACCCAG    960
TATCTTTGCA CAAACCAGGG GTTGGGGGAG GGTCTCTGGC TTTATTTTTC TGCTGTGCAG   1020
AATCCTATTT TATATTTTTT AAAGTCAGTT TAGGTAATAA ACTTTATTAT GAAAGTTTTT   1080
TTTTTTAAAA AAAA                                                     1094
```

(2) INFORMATION FOR SEQ ID NO:10:

(i) SEQUENCE CHARACTERISTICS:
(A) LENGTH: 211 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:10:

```
Met Val Ala His Asn Gln Val Ala Ala Asp Asn Ala Val Ser Thr Ala
1               5                   10                  15

Ala Glu Pro Arg Arg Arg Pro Glu Pro Ser Ser Ser Ser Ser Ser Ser
            20                  25                  30

Pro Ala Ala Pro Ala Arg Pro Arg Pro Cys Pro Ala Val Pro Ala Pro
        35                  40                  45

Ala Pro Gly Asp Thr His Phe Arg Thr Phe Arg Ser His Ala Asp Tyr
    50                  55                  60

Arg Arg Ile Thr Arg Ala Ser Ala Leu Leu Asp Ala Cys Gly Phe Tyr
65                  70                  75                  80

Trp Gly Pro Leu Ser Val His Gly Ala His Glu Arg Leu Arg Ala Glu
                85                  90                  95
```

Pro Val Gly Thr Phe Leu Val Arg Asp Ser Arg Gln Arg Asn Cys Phe
100 105 110

Phe Ala Leu Ser Val Lys Met Ala Ser Gly Pro Thr Ser Ile Arg Val
115 120 125

His Phe Gln Ala Gly Arg Phe His Leu Asp Gly Ser Arg Glu Ser Phe
130 135 140

Asp Cys Leu Phe Glu Leu Leu Glu His Tyr Val Ala Ala Pro Arg Arg
145 150 155 160

Met Leu Gly Ala Pro Leu Arg Gln Arg Arg Val Arg Pro Leu Gln Glu
165 170 175

Leu Cys Arg Gln Arg Ile Val Ala Thr Val Gly Arg Glu Asn Leu Ala
180 185 190

Arg Ile Pro Leu Asn Pro Val Leu Arg Asp Tyr Leu Ser Ser Phe Pro
195 200 205

Phe Gln Ile
210

(2) INFORMATION FOR SEQ ID NO:11:

(i) SEQUENCE CHARACTERISTICS:
(A) LENGTH: 2807 base pairs
(B) TYPE: nucleic acid
(D) TOPOLOGY: linear

(ii) MOLECULE TYPE: protein

(xi) SEQUENCE DESCRIPTION: SEQ ID NO:11:

GGAAACCGAG GCGGGGAGAC CAGGAGGCCT TGGCCTCAGA GCTTCAGAGT CGCGTGGCAG 60
CAAACAGAGA AACCTGTAGA GGGCAGTGTG CGTCACTTAG CTCAGGGAAG CTGCACGCGA 120
AACTCACCCG CCTTCATTCA TAAACATCGT CAGCTAGGCA CCTACTCCTG GGCTTTCAGG 180
ACAAACTGAA TCACGAAACC ACAGTGTCCT TAAAATAGGT CTGACCGCCT GAATCCCTGG 240
CCAAGGTGTG TACGGGGCAT GGGAGCCCTT GTGCAGAGAT GCTTGCAGGA GCCTTGAGGG 300
GCTCTGTAAG ACAGAGGCTA GGAAGACAAA GTTGGGGGCT ACAGCTTCTT GTCCTGCCCG 360
GGGCCTCAGT TTCTTCGGTT GCCCACGTAG GAGTGCAGAG AGTCCAGCCC CTGGGGACCC 420
AACCCAACCC CGCCCAGTTT CCGAGGAACT CGTCCGGGAG CGGGGGCGCC CCTCCCGCAC 480
CGCCTTAGGC TTCCTTTGAA GCCTCTGCGG TCAGGCCACC GCTTCCTGGG AAGCCCAAGC 540

CAAGGCCAGG CCGAGTGGCC AACGGGAGGG GCCCGCGCGC GATTCTGGAG GAGGGCGGCG 600
GCCCCACAGG TCTCCAGGGC TGGCTAGCCG GGCTCCTAGA GCGGAGACTG CCAAGGCCTT 660
CGGGTCCTGG GCAGGAAGGA TCCTGGCAGG GAGGAGTTGC TTGGGGGGTG GGGGGGAAAG 720
GCTCCAGGCG CGGTGGAGCT CTGACCAGGA GAATGCACAC ACTCGGAGGG GAGGAGGCGT 780
GTCAGCCCCA AGCTAGCATC CCACCCGGGG AGCAGCGATG TGGGGCGAAG GTAGCCAGAG 840
CAAAAGAGCA GGCACCAGGT GACACGAAAC AGAAGATTCC GGGTAGAGCC AGAACCCCAG 900
AAGTCCCATT CAGGGAAGGT GCGAGGCGAG AACGAGTTAG GTGGACCCTC TCCAGGGGCA 960
GCCAAAGAAA TCTAAAGAGA ACCCGAAGGA CTTGCCGGAA AGAGAAACCG AAAGCGGCGG 1020
TGGGCGGGAT CGGTGGGCGG GGCCTCCCTG GTTTAAGAGC TTGATGCAGG GGCGGGCAGC 1080
AGCAGAGAGA ACTGCGGCCG TGGCAGCGGC ACGGCTCCCG GCCCCGGAGC ATGCGCGACA 1140
GCAGCCCCGG AACCCCCAGC CGCGGCGCCC CGCGTCCCGC CGCCAGGTGA GCCGAGGCAG 1200
CTGCGAAGGA GCAGGCGGGA GGGGATGGGA GGAAGGGGAG CAGAGCCTGG CAGGACTATC 1260
CTCGCAGACT GCATGGCGGG GTCGTGGATG CTATGCCTCT GGCGCCCGCC CCACCGGCTG 1320
GCCCAGGCGG CCCCTCGCGC GCGCGGGGCG CCGTCAGCCC CTCCTCTCCG GCCCTGAGCC 1380
CGGATCGTCC GCCCGGGTTC CAGTTCCCGG CGTGGCCAGT AGGCGGCAAC CGCGAGGCGG 1440
CAAGCCACCC AGCGGGGACG GCCTGGAGTC GGGCCCCTCT CCACGCCCCC TTCTCCACGC 1500
GCGCGGGGAG GCAGGGCTCC ACCGCCAGTC TGGAAGGGTT CCACATACAG GAACGGCCTA 1560
CTTCGCAGAT GAGCCCACCG AGGCTCAGGC TCCGGGCGGA TTCTGCGTGT CACCCTCGCT 1620
CCTTGGGGTC CGCTGGCCGG CCTGTGCCAC CCGGACGCCC GGTTCACTGC CTCTGTCTCC 1680
CCCATCAGCG CAGCCCCGGA CGCTATGGCC CACCCCTCCA GCTGGCCCCT CGAGTAGGAT 1740
GGTAGCACGT AACCAGGTGG AAGCCGACAA TGCGATCTCC CCGGCATCAG AGCCCCGACG 1800
GCGGCCAGAG CCATCCTCGT CCTCGTCTTC GTCCTCGCCG GCGGCCCCGG CGCGTCCCCG 1860
GCCCTGCCCG GTGGTCCCGG CCCCGGCTCC GGGCGACACT CACTTCCGCA CCTTCCGCTC 1920
CCACTCTGAT TACCGGCGCA TCACGCGGAC CAGCGCTCTC CTGGACGCCT GCGGCTTCTA 1980
CTGGGGACCC CTGAGCGTGC ATGGGGCGCA CGAACGGCTG CGTTCCGAAC CCGTGGGCAC 2040
CTTCTTGGTG CGCGACAGTC GCCAGCGGAA CTGCTTCTTC GCGCTCAGCG TGAAGATGGC 2100
TTCGGGCCCC ACGAGCATTC GTGTGCACTT CCAGGCCGGC CGCTTCCACC TGGACGGCAA 2160
CCGCGAGACC TTCGACTGCC TCTTCGAGCT GCTGGAGCAC TACGTGGCGG CGCCGCGCCG 2220
CATGTTGGGG GCCCCACTGC GCCAGCGCCG CGTGCGGCCG CTGCAGGAGC TGTGTCGCCA 2280
GCGCATCGTG GCCGCCGTGG GTCGCGAGAA CCTGGCACGC ATCCCTCTTA ACCCGGTACT 2340
CCGTGACTAC CTGAGTTCCT TCCCCTTCCA GATCTGACCG GCTGCCGCCG TGCCCGCAGA 2400
ATTAAGTGGG AGCGCCTTAT TATTTCTTAT TATTAATTAT TATTATTTTT CTGGAACCAC 2460
GTGGGAGCCC TCCCCGCCTA GGTCGGAGGG AGTGGGTGTG GAGGGTGAGA TCCCTCCCAC 2520
TTCTGGCTGG AGACCTTATC CCGCCTCTCG GGGGGCCTCC CCTCCTGGTG CTCCCTCCCG 2580
GTCCCCCTGG TTGTAGCAGC TTGTGTCTGG GGCCAGGACC TGAACTCCAC GCCTACCTCT 2640
CCATGTTTAC ATGTTCCCAG TATCTTTGCA CAAACCAGGG GTGGGGGAGG GTCTCTGGCT 2700
TCATTTTTCT GCTGTGCAGA ATATTCTATT TTATATTTTT ACATCCAGTT TAGATAATAA 2760
ACTTTATTAT GAAAGTTTTT TTTTTTAAAG AAACAAAGAT TTCTAGA 2807

(2) INFORMATION FOR SEQ ID NO:12:

(i) SEQUENCE CHARACTERISTICS:

(A) LENGTH: 212 amino acids

(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:12:

```
Met Val Ala Arg Asn Gln Val Glu Ala Asp Asn Ala Ile Ser Pro Ala
1               5                   10                  15

Ser Glu Pro Arg Arg Arg Pro Glu Pro Ser Ser Ser Ser Ser Ser Ser
            20                  25                  30

Ser Pro Ala Ala Pro Ala Arg Pro Arg Pro Cys Pro Val Val Pro Ala
        35                  40                  45

Pro Ala Pro Gly Asp Thr His Phe Arg Thr Phe Arg Ser His Ser Asp
    50                  55                  60

Tyr Arg Arg Ile Thr Arg Thr Ser Ala Leu Leu Asp Ala Cys Gly Phe
65                  70                  75                  80

Tyr Trp Gly Pro Leu Ser Val His Gly Ala His Glu Arg Leu Arg Ser
                85                  90                  95

Glu Pro Val Gly Thr Phe Leu Val Arg Asp Ser Arg Gln Arg Asn Cys
            100                 105                 110

Phe Phe Ala Leu Ser Val Lys Met Ala Ser Gly Pro Thr Ser Ile Arg
        115                 120                 125

Val His Phe Gln Ala Gly Arg Phe His Leu Asp Gly Asn Arg Glu Thr
    130                 135                 140

Phe Asp Cys Leu Phe Glu Leu Leu Glu His Tyr Val Ala Ala Pro Arg
145                 150                 155                 160

Arg Met Leu Gly Ala Pro Leu Arg Gln Arg Arg Val Arg Pro Leu Gln
                165                 170                 175

Glu Leu Cys Arg Gln Arg Ile Val Ala Ala Val Gly Arg Glu Asn Leu
            180                 185                 190
```

```
Ala Arg Ile Pro Leu Asn Pro Val Leu Arg Asp Tyr Leu Ser Ser Phe
        195                 200                 205

Pro Phe Gln Ile
    210
```

(2) INFORMATION FOR SEQ ID NO:13:

(i) SEQUENCE CHARACTERISTICS:
(A) LENGTH: 1611 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 263..1529

(xi) SEQUENCE DESCRIPTION: SEQ ID NO:13:

```
CGAATTCCGG GCGGGCTGTG TGAGTCTGTG AGTGGAAGGC GCGCCGGCTC TTTTGTCTGA      60

GTGTGACCCG GTGGCTTTGT TCCAGGCATT CCGGTGATTT CCTCCGGGCA GTCCGCAGAA     120

GCCGCAGCGG CCGCCCGCGC TCTCTCTGCA GTCTCCACAC CCGGGAGAGC CTGAGCCCGC     180

GTCACGCCCC TCAGCCCCCG CTGAGTCCCT TCTCTGTTGT CGCGTCCGAA TCGAGTTCCC     240

GGAATCAGAC GGTGCCCCAT AG ATG GCC AGC TTT CCC CCG AGG GTT AAC GAG     292
                         Met Ala Ser Phe Pro Pro Arg Val Asn Glu
                           1               5                  10

AAA GAG ATC GTG AGA TCA CGT ACT ATA GGG GAA CTC TTG GCT CCA GCA      340
Lys Glu Ile Val Arg Ser Arg Thr Ile Gly Glu Leu Leu Ala Pro Ala
                15                  20                  25

GCT CCT TTT GAC AAG AAA TGT GGT GGT GAG AAC TGG ACG GTT GCT TTT      388
Ala Pro Phe Asp Lys Lys Cys Gly Gly Glu Asn Trp Thr Val Ala Phe
            30                  35                  40
```

```
GCT CCT GAT GGT TCC TAC TTT GCG TGG TCA CAA GGA TAT CGC ATA GTG      436
Ala Pro Asp Gly Ser Tyr Phe Ala Trp Ser Gln Gly Tyr Arg Ile Val
        45                  50                  55

AAG CTT GTC CCG TGG TCC CAG TGC CGT AAG AAC TTT CTT TTG CAT GGT      484
Lys Leu Val Pro Trp Ser Gln Cys Arg Lys Asn Phe Leu Leu His Gly
    60                  65                  70

TCC AAA AAT GTT ACC AAT TCA AGC TGT CTA AAA TTG GCA AGA CAA AAC      532
Ser Lys Asn Val Thr Asn Ser Ser Cys Leu Lys Leu Ala Arg Gln Asn
 75                  80                  85                  90

AGT AAT GGT GGT CAG AAA AAC AAG CCT CCT GAG CAC GTT ATA GAC TGT      580
Ser Asn Gly Gly Gln Lys Asn Lys Pro Pro Glu His Val Ile Asp Cys
                 95                 100                 105

GGA GAC ATA GTC TGG AGT CTT GCT TTT GGG TCT TCA GTT CCA GAA AAA      628
Gly Asp Ile Val Trp Ser Leu Ala Phe Gly Ser Ser Val Pro Glu Lys
            110                 115                 120

CAG AGT CGT TGC GTT AAT ATA GAA TGG CAT CGG TTC CGA TTT GGA CAG      676
Gln Ser Arg Cys Val Asn Ile Glu Trp His Arg Phe Arg Phe Gly Gln
        125                 130                 135

GAT CAG CTA CTC CTT GCC ACA GGA TTA AAC AAT GGT CGC ATC AAA ATC      724
Asp Gln Leu Leu Leu Ala Thr Gly Leu Asn Asn Gly Arg Ile Lys Ile
    140                 145                 150

TGG GAT GTA TAT ACA GGA AAA CTC CTC CTT AAT TTG GTA GAC CAC ATT      772
Trp Asp Val Tyr Thr Gly Lys Leu Leu Leu Asn Leu Val Asp His Ile
155                 160                 165                 170

GAA ATG GTT AGA GAT TTA ACT TTT GCT CCA GAT GGG AGC TTA CTC CTT      820
Glu Met Val Arg Asp Leu Thr Phe Ala Pro Asp Gly Ser Leu Leu Leu
                175                 180                 185

GTA TCA GCT TCA AGA GAC AAA ACT CTA AGA GTG TGG GAC CTG AAA GAT      868
Val Ser Ala Ser Arg Asp Lys Thr Leu Arg Val Trp Asp Leu Lys Asp
            190                 195                 200

GAT GGA AAC ATG GTG AAA GTA TTG CGG GCA CAT CAG AAT TGG GTG TAC      916
Asp Gly Asn Met Val Lys Val Leu Arg Ala His Gln Asn Trp Val Tyr
        205                 210                 215

AGT TGT GCA TTC TCT CCC GAC TGT TCT ATG CTG TGT TCA GTG GGC GCC      964
```

Ser Cys Ala Phe Ser Pro Asp Cys Ser Met Leu Cys Ser Val Gly Ala
220 225 230

AGT AAA GCA GTT TTC CTT TGG AAT ATG GAT AAA TAC ACC ATG ATT AGG 1012
Ser Lys Ala Val Phe Leu Trp Asn Met Asp Lys Tyr Thr Met Ile Arg
235 240 245 250

AAG CTG GAA GGT CAT CAC CAT GAT GTT GTA GCT TGT GAC TTT TCT CCT 1060
Lys Leu Glu Gly His His His Asp Val Val Ala Cys Asp Phe Ser Pro
255 260 265

GAT GGA GCA TTG CTA GCT ACT GCA TCC TAT GAC ACT CGT GTG TAT GTC 1108
Asp Gly Ala Leu Leu Ala Thr Ala Ser Tyr Asp Thr Arg Val Tyr Val
270 275 280

TGG GAT CCA CAC AAT GGA GAC CTT CTG ATG GAG TTT GGG CAC CTG TTT 1156
Trp Asp Pro His Asn Gly Asp Leu Leu Met Glu Phe Gly His Leu Phe
285 290 295

CCC TCG CCC ACT CCA ATA TTT GCT GGA GGA GCA AAT GAC CGA TGG GTG 1204
Pro Ser Pro Thr Pro Ile Phe Ala Gly Gly Ala Asn Asp Arg Trp Val
300 305 310

AGA GCT GTG TCT TTC AGT CAT GAT GGA CTG CAT GTT GCC AGC CTT GCT 1252
Arg Ala Val Ser Phe Ser His Asp Gly Leu His Val Ala Ser Leu Ala
315 320 325 330

GAT GAT AAA ATG GTG AGG TTC TGG AGA ATC GAT GAG GAT TGT CCG GTA 1300
Asp Asp Lys Met Val Arg Phe Trp Arg Ile Asp Glu Asp Cys Pro Val
335 340 345

CAA GTT GCA CCT TTG AGC AAT GGT CTT TGC TGT GCC TTT TCT ACT GAT 1348
Gln Val Ala Pro Leu Ser Asn Gly Leu Cys Cys Ala Phe Ser Thr Asp
350 355 360

GGC AGT GTT TTA GCT GCT GGG ACA CAT GAT GGA AGT GTG TAT TTT TGG 1396
Gly Ser Val Leu Ala Ala Gly Thr His Asp Gly Ser Val Tyr Phe Trp
365 370 375

GCC ACT CCA AGG CAA GTC CCT AGC CTT CAA CAT ATA TGT CGC ATG TCA 1444
Ala Thr Pro Arg Gln Val Pro Ser Leu Gln His Ile Cys Arg Met Ser
380 385 390

ATC CGA AGA GTG ATG TCC ACC CAA GAA GTC CAA AAA CTG CCT GTT CCT 1492
Ile Arg Arg Val Met Ser Thr Gln Glu Val Gln Lys Leu Pro Val Pro

```
395                 400                 405                 410

TCC AAA ATA TTG GCG TTT CTC TCC TAC CGC GGT TAG A CTGAAGACTG          1539
Ser Lys Ile Leu Ala Phe Leu Ser Tyr Arg Gly  *
                415                 420

CCTTTCCTGG TAGGCCTGCC AGACAGAGCG CCCTTTACAA GACACACCTC AAGCTTTACC     1599

TCGTGCCGAA TT                                                         1611
```

(2) INFORMATION FOR SEQ ID NO:14:

(i) SEQUENCE CHARACTERISTICS:
(A) LENGTH: 422 amino acids
(B) TYPE: amino acid
(D) TOPOLOGY: linear

(ii) MOLECULE TYPE: protein

(xi) SEQUENCE DESCRIPTION: SEQ ID NO:14:

```
Met Ala Ser Phe Pro Pro Arg Val Asn Glu Lys Glu Ile Val Arg Ser
 1               5                  10                  15

Arg Thr Ile Gly Glu Leu Leu Ala Pro Ala Ala Pro Phe Asp Lys Lys
            20                  25                  30

Cys Gly Gly Glu Asn Trp Thr Val Ala Phe Ala Pro Asp Gly Ser Tyr
        35                  40                  45

Phe Ala Trp Ser Gln Gly Tyr Arg Ile Val Lys Leu Val Pro Trp Ser
    50                  55                  60

Gln Cys Arg Lys Asn Phe Leu Leu His Gly Ser Lys Asn Val Thr Asn
65                  70                  75                  80

Ser Ser Cys Leu Lys Leu Ala Arg Gln Asn Ser Asn Gly Gly Gln Lys
                85                  90                  95

Asn Lys Pro Pro Glu His Val Ile Asp Cys Gly Asp Ile Val Trp Ser
            100                 105                 110

Leu Ala Phe Gly Ser Ser Val Pro Glu Lys Gln Ser Arg Cys Val Asn
        115                 120                 125
```

Ile Glu Trp His Arg Phe Arg Phe Gly Gln Asp Gln Leu Leu Leu Ala
    130                 135                 140

Thr Gly Leu Asn Asn Gly Arg Ile Lys Ile Trp Asp Val Tyr Thr Gly
145                 150                 155                 160

Lys Leu Leu Leu Asn Leu Val Asp His Ile Glu Met Val Arg Asp Leu
                165                 170                 175

Thr Phe Ala Pro Asp Gly Ser Leu Leu Leu Val Ser Ala Ser Arg Asp
            180                 185                 190

Lys Thr Leu Arg Val Trp Asp Leu Lys Asp Asp Gly Asn Met Val Lys
        195                 200                 205

Val Leu Arg Ala His Gln Asn Trp Val Tyr Ser Cys Ala Phe Ser Pro
    210                 215                 220

Asp Cys Ser Met Leu Cys Ser Val Gly Ala Ser Lys Ala Val Phe Leu
225                 230                 235                 240

Trp Asn Met Asp Lys Tyr Thr Met Ile Arg Lys Leu Glu Gly His His
                245                 250                 255

His Asp Val Val Ala Cys Asp Phe Ser Pro Asp Gly Ala Leu Leu Ala
            260                 265                 270

Thr Ala Ser Tyr Asp Thr Arg Val Tyr Val Trp Asp Pro His Asn Gly
        275                 280                 285

Asp Leu Leu Met Glu Phe Gly His Leu Phe Pro Ser Pro Thr Pro Ile
    290                 295                 300

Phe Ala Gly Gly Ala Asn Asp Arg Trp Val Arg Ala Val Ser Phe Ser
305                 310                 315                 320

His Asp Gly Leu His Val Ala Ser Leu Ala Asp Asp Lys Met Val Arg
                325                 330                 335

Phe Trp Arg Ile Asp Glu Asp Cys Pro Val Gln Val Ala Pro Leu Ser
            340                 345                 350

Asn Gly Leu Cys Cys Ala Phe Ser Thr Asp Gly Ser Val Leu Ala Ala
        355                 360                 365

Gly Thr His Asp Gly Ser Val Tyr Phe Trp Ala Thr Pro Arg Gln Val
370 375 380

Pro Ser Leu Gln His Ile Cys Arg Met Ser Ile Arg Arg Val Met Ser
385 390 395 400

Thr Gln Glu Val Gln Lys Leu Pro Val Pro Ser Lys Ile Leu Ala Phe
405 410 415

Leu Ser Tyr Arg Gly *
420

(2) INFORMATION FOR SEQ ID NO:15:

(i) SEQUENCE CHARACTERISTICS:
(A) LENGTH: 783 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:15:

CTGTCTTCCT CCGCAGCGCG AGGCTGGGTA CAGGGTCTAT TGTCTGTGGT TGACTCCGTA 60

CTTTGGTCTG AGGCCTTCGG GAGCTTTCCC GAGGCAGTTA GCAGAAGCCG CAGCGACCGC 120

CCCCGCCCGT CTCCTCTGTC CCTGGGCCCG GGAGACAAAC TTGGCGTCAC GCCCTCAGCG 180

GTCGCCACTC TCTTCTCTGT TGTTGGGTCC GCATCGTATT CCCGGAATCA GACGGTGCCC 240

CATAGATGGC CAGCTTTCCC CCGAGGGTCA ACGAGAAAGA GATCGTGAGA TCACGTACTA 300

TAGGTGAACT TTTAGCTCCT GCAGCTCCTT TTGACAAGAA ATGTGGTCGT GAAAATTGGA 360

CTGTTGCTTT TGCTCCAGAT GGTTCATACT TTGCTTGGTC ACAAGGACAT CGCACAGTAA 420

AGCTTGTTCC GTGGTCCCAG TGCCTTCAGA ACTTTCTCTT GCATGGCACC AAGAATGTTA 480

CCAATTCAAG CAGTTTAAGA TTGCCAAGAC AAAATAGTGA TGGTGGTCAG AAAAATAAGC 540

CTCGTGACAT ATTATAGACT GTGGAGATAT AGTCTGGAGT CTTGCTTTTG GGTCATCAGT 600

TCCAGAAAAA CAGAGTCGCT GTGTAAATAT AGAATGGCAT CGCTTCAGAT TTGGACAAGA 660

TCAGCTACTT CTTGCTACAG GGTTGAACAA TGGGCGTATC AAAATATGGG ATGTATATCA 720

GGAAACTCCT CCTTAACTTG GTAGATCATA CTGAAGTGGT CAGAGATTTA ACTTTTGCTC 780

CAG 783

(2) INFORMATION FOR SEQ ID NO:16:

(i) SEQUENCE CHARACTERISTICS:
(A) LENGTH: 1122 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:16:

CTCTGTATGT CTGAATGAAG CTATAACATT TGCCTTTTTA TTGCAGGTTT TCCTTTGGAA 60

TATGGATAAA TACACCATGA TACGGAAACT AGAAGGACAT CACCATGATG TGGTAGCTTG 120

TGACTTTTCT CCTGATGGAG CATTACTGGC TACTGCATCT TATGATACTC GAGTATATAT 180

CTGGGATCCA CATAATGGAG ACATTCTGAT GGAATTTGGG CACCTGTTTC CCCCACCTAC 240

TCCAATATTT GCTGGAGGAG CAAATGACCG GTGGGTACGA TCTGTATCTT TTAGCCATGA 300

TGGACTGCAT GTTGCAAGCC TTGCTGATGA TAAAATGGTG AGGTTCTGGA GAATTGATGA 360

GGATTATCCA GTGCAAGTTG CACCTTTGAG CAATGGTCTT TGCTGTGCCT TCTCTACTGA 420

TGGCAGTGTT TTAGCTGCTG GGACACATGA CGGAAGTGTG TATTTTTGGG CCACTCCACG 480

GCAGGTCCCT AGCCTGCAAC ATTTATGTCG CATGTCAATC CGAAGAGTGA TGCCCACCCA 540

AGAAGTTCAG GAGCTGCCGA TTCCTTCCAA GCTTTTGGAG TTTCTCTCGT ATCGTATTTA 600

GAAGATTCTG CCTTCCCTAG TAGTAGGGAC TGACAGAATA CACTTAACAC AAACCTCAAG 660

CTTTACTGAC TTCAATTATC TGTTTTTAAA GACGTAGAAG ATTTATTTAA TTTGATATGT 720

TCTTGTACTG CATTTTGATC AGTTGAGCTT TTAAAATATT ATTTATAGAC AATAGAAGTA 780

TTTCTGAACA TATCAAATAT AAATTTTTTT AAAGATCTAA CTGTGAAAAC ATACATACCT 840

GTACATATTT AGATATAAGC TGCTATATGT TGAATGGACC CTTTTGCTTT TCTGATTTTT 900

AGTTCTGACA TGTATATATT GCTTCAGTAG AGCCACAATA TGTATCTTTG CTGTAAAGTG 960

CAAGGAAATT TTAAATTCTG GGACACTGAG TTAGATGGTA AATACTGACT TACGAAAGTT 1020

GAATTGGGTG AGGCGGGCAA ATCACCTGAG GTCAGCAGTT TGAGACTAGC CTGGCAAACA 1080

TGATGAAACC CTGTCTCTAC TAAAAATACA AAAAAAAAAA AA 1122

(2) INFORMATION FOR SEQ ID NO:17:

(i) SEQUENCE CHARACTERISTICS:
(A) LENGTH: 2537 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 422..2029

(xi) SEQUENCE DESCRIPTION: SEQ ID NO:17:

CGGCACGAGC CGGGCTCCGT CCGGAGGAAG CGAGGCTGCG CCGCCGGCCC GGCAGGAGCG 60

GAGGACGGGA GCGCGGGCGG TCGCGCTCGC CCTGTCGCTG ACTGCGCTGC CCCGGCCCAT 120

CCTTGCCTGG CCGCAGGTGC CCTGGATGAG GCCGCCGCGC GTGTCCCGGC CGCTGAGTGT 180

CCCCCGCGGT CGCCCGGCGC CTGCCCTCAA GCGGCCGCCT CTCCTTGCCC GGGTCCCCGT 240

TTTCCCCCGG CGCAGTCCTC CTCCGGTGGG CGCCTCCGCA CCTCGGCGCA GGCGGCACGG 300

CCCTCGGGCC GGGATGGATC CGCCGGGAAG AGGAAGACAA GCCGGGGCGT TGAGCCCCTG 360

```
CGCACGGTGC CGCCGCGCGT AGTGGGAGCT TACTCGCAGT AGGCTCTCGC TCTTCTAATC     420

A ATG GAT AAA GTG GGG AAA ATG TGG AAC AAC TTA AAA TAC AGA TGC         466
  Met Asp Lys Val Gly Lys Met Trp Asn Asn Leu Lys Tyr Arg Cys
   1               5                  10                  15

CAG AAT CTC TTC AGC CAC GAG GGA GGA AGC CGT AAT GAG AAC GTG GAG       514
Gln Asn Leu Phe Ser His Glu Gly Gly Ser Arg Asn Glu Asn Val Glu
                20                  25                  30

ATG AAC CCC AAC AGA TGT CCG TCT GTC AAA GAG AAA AGC ATC AGT CTG       562
Met Asn Pro Asn Arg Cys Pro Ser Val Lys Glu Lys Ser Ile Ser Leu
            35                  40                  45

GGA GAG GCA GCT CCC CAG CAA GAG AGC AGT CCC TTA AGA GAA AAT GTT       610
Gly Glu Ala Ala Pro Gln Gln Glu Ser Ser Pro Leu Arg Glu Asn Val
        50                  55                  60

GCC TTA CAG CTG GGA CTG AGC CCT TCC AAG ACC TTT TCC AGG CGG AAC       658
Ala Leu Gln Leu Gly Leu Ser Pro Ser Lys Thr Phe Ser Arg Arg Asn
    65                  70                  75

CAA AAC TGT GCC GCA GAG ATC CCT CAA GTG GTT GAA ATC AGC ATC GAG       706
Gln Asn Cys Ala Ala Glu Ile Pro Gln Val Val Glu Ile Ser Ile Glu
80                  85                  90                  95

AAA GAC AGT GAC TCG GGT GCC ACC CCA GGA ACG AGG CTT GCA CGG AGA       754
Lys Asp Ser Asp Ser Gly Ala Thr Pro Gly Thr Arg Leu Ala Arg Arg
                100                 105                 110

GAC TCC TAC TCG CGG CAC GCC CCG TGG GGA GGA AAG AAG AAA CAT TCC       802
Asp Ser Tyr Ser Arg His Ala Pro Trp Gly Gly Lys Lys Lys His Ser
            115                 120                 125

TGT TCC ACA AAG ACC CAG AGT TCA TTG GAT ACC GAG AAA AAG TTT GGT       850
Cys Ser Thr Lys Thr Gln Ser Ser Leu Asp Thr Glu Lys Lys Phe Gly
        130                 135                 140

AGA ACT CGA AGC GGC CTT CAG AGG CGA GAG CGG CGC TAT GGA GTC AGC       898
Arg Thr Arg Ser Gly Leu Gln Arg Arg Glu Arg Arg Tyr Gly Val Ser
    145                 150                 155

TCC ATG CAG GAC ATG GAC AGC GTT TCT AGC CGC GCG GTC GGG AGC CGC       946
Ser Met Gln Asp Met Asp Ser Val Ser Ser Arg Ala Val Gly Ser Arg
160                 165                 170                 175
```

```
TCC CTG AGG CAG AGG CTC CAG GAC ACG GTG GGT TTG TGT TTT CCC ATG      994
Ser Leu Arg Gln Arg Leu Gln Asp Thr Val Gly Leu Cys Phe Pro Met
                180                 185                 190

AGA ACT TAC AGC AAG CAG TCA AAG CCA CTC TTT TCC AAT AAA AGA AAA     1042
Arg Thr Tyr Ser Lys Gln Ser Lys Pro Leu Phe Ser Asn Lys Arg Lys
            195                 200                 205

ATA CAT CTT TCT GAA TTA ATG CTG GAG AAA TGC CCT TTT CCT GCT GGC     1090
Ile His Leu Ser Glu Leu Met Leu Glu Lys Cys Pro Phe Pro Ala Gly
        210                 215                 220

TCG GAT TTA GCA CAA AAG TGG CAT TTG ATT AAA CAG CAT ACC GCC CCT     1138
Ser Asp Leu Ala Gln Lys Trp His Leu Ile Lys Gln His Thr Ala Pro
    225                 230                 235

GTG AGC CCA CAC TCA ACA TTT TTT GAT ACA TTT GAT CCA TCA CTG GTG     1186
Val Ser Pro His Ser Thr Phe Phe Asp Thr Phe Asp Pro Ser Leu Val
240                 245                 250                 255

TCT ACA GAA GAT GAA GAA GAT AGG CTT CGC GAG AGA AGA CGG CTT AGT     1234
Ser Thr Glu Asp Glu Glu Asp Arg Leu Arg Glu Arg Arg Arg Leu Ser
                260                 265                 270

ATC GAA GAA GGG GTG GAT CCC CCT CCC AAC GCA CAA ATA CAC ACC TTT     1282
Ile Glu Glu Gly Val Asp Pro Pro Pro Asn Ala Gln Ile His Thr Phe
            275                 280                 285

GAA GCT ACT GCA CAG GTC AAC CCA TTG TAT AAG CTG GGA CCA AAG TTA     1330
Glu Ala Thr Ala Gln Val Asn Pro Leu Tyr Lys Leu Gly Pro Lys Leu
        290                 295                 300

GCT CCT GGG ATG ACA GAG ATA AGT GGA GAT GGT TCT GCA ATT CCA CAA     1378
Ala Pro Gly Met Thr Glu Ile Ser Gly Asp Gly Ser Ala Ile Pro Gln
    305                 310                 315

GCA ATT GTG ACT CAG AAG AGG ATT CAA CCA CCC TAT GTC TGC AGT CAC     1426
Ala Ile Val Thr Gln Lys Arg Ile Gln Pro Pro Tyr Val Cys Ser His
320                 325                 330                 335

GGA GGC AGA AGC AGC GCC AGG TGT CCG GGG ACA GCC ACG CGC ACG TTA     1474
Gly Gly Arg Ser Ser Ala Arg Cys Pro Gly Thr Ala Thr Arg Thr Leu
                340                 345                 350

GCA GAC AGG GAG CTT GGA AAG TTC ATA CGC AGA TCG ATT ACA TAC ACT     1522
```

```
Ala Asp Arg Glu Leu Gly Lys Phe Ile Arg Arg Ser Ile Thr Tyr Thr
            355                 360                 365

GCC TCG TGC CAG ATT TGC TTC AGA TCA CAG GGA ATC CCT GTT ACT GGG      1570
Ala Ser Cys Gln Ile Cys Phe Arg Ser Gln Gly Ile Pro Val Thr Gly
        370                 375                 380

GCG TGA TGG ACC GAT ACG AGG CCG AAG CCC TTC TAG AAG GGA AAC CGG      1618
Ala  *  Trp Thr Asp Thr Arg Pro Lys Pro Phe  *  Lys Gly Asn Arg
    385                 390                 395

AAG GCA CGT TCT TGC TCA GGG ACT CTG CAC AGG AGG ACT ACC TCT TCT      1666
Lys Ala Arg Ser Cys Ser Gly Thr Leu His Arg Arg Thr Thr Ser Ser
400                 405                 410                 415

CTG TGA GCT TCC GCC GCT ACA ACA GGT CTC TGC ACG CCC GGA TCG AGC      1714
Leu  *  Ala Ser Ala Ala Thr Thr Gly Leu Cys Thr Pro Gly Ser Ser
                420                 425                 430

AGT GGA ACC ACA ACT TCA GCT TCG ATG CCC ATG ACC CCT GCG TGT TTC      1762
Ser Gly Thr Thr Thr Ser Ala Ser Met Pro Met Thr Pro Ala Cys Phe
            435                 440                 445

ACT CCT CCA CGT CAC GGG GCT TCT CGA ACA CTA TAA AGA CCC CAG CTC      1810
Thr Pro Pro Arg His Gly Ala Ser Arg Thr Leu  *  Arg Pro Gln Leu
        450                 455                 460

TTG CAT GTT TTT TGA ACC GTT GCT AAC GAT ATC ACT GAA TAG AAC TTT      1858
Leu His Val Phe  *  Thr Val Ala Asn Asp Ile Thr Glu  *  Asn Phe
    465                 470                 475

CCC TTT CAG CCT GCA GTA TAT CTG CCG CGC AGT GAT CTG CAG ATG CAC      1906
Pro Phe Gln Pro Ala Val Tyr Leu Pro Arg Ser Asp Leu Gln Met His
480                 485                 490                 495

TAC GTA TGA TGG GAT TGA CGG GCT CCC GCT ACC GTC GAT GTT ACA GGA      1954
Tyr Val  *  Trp Asp  *  Arg Ala Pro Ala Thr Val Asp Val Thr Gly
                500                 505                 510

TTT TTT AAA AGA GTA TCA TTA TAA ACA AAA AGT TAG GGT TCG CTG GTT      2002
Phe Phe Lys Arg Val Ser Leu  *  Thr Lys Ser  *  Gly Ser Leu Val
            515                 520                 525

AGA ACG AGA CCA GTC AAA GCA AAG TAACTCCTGT CCCCAAAGGG CACTAACTAA     2056
Arg Thr Arg Pro Val Lys Ala Lys
```

530 535

GTCTGCTCCT CCCGTGCATC GAACTGCACC CATAGGAGGC AGTCAGCTGC TAGGATTTCC 2116

CACCCAGAAT GGGAGCTTAG TCATTAGCCT CTGCCCTATG GGGTCCGCTG TTCCTCAGAC 2176

AAAGGTGCCT AGGGACAGCA AGATGGCTTG CAGGTGTTCG GTGGGCTGTG ACAACTGAGG 2236

GAGGCAACTC TGGGGCATTT GCTATGAAGA ATTCTATTTC TTACCGAAGA ACAAATTATT 2296

AATATTGGAT GGGTATTTCA ATAGTGTGAC TAATGTTTGA AATTATTTTT TCTAAGAATT 2356

TTTCTATAAC CTTCAGAAAA AGTAGTGATG TTTGTAGTTA CTATAAATCA AGCTTTGAAA 2416

GTTCAAAACA AACAAGTTAA ATAAAAGACT ACCTTCCTTT TAGAGAAAAC AAATGCAAGT 2476

TTTCCCAGCC ACAGGCATTG TGCACTGTTA ATGTTGCTTG TTATCAGCTC CTTTCTCCTC 2536

C 2537

(2) INFORMATION FOR SEQ ID NO:18:

(i) SEQUENCE CHARACTERISTICS:
(A) LENGTH: 535 amino acids
(B) TYPE: amino acid
(D) TOPOLOGY: linear

(ii) MOLECULE TYPE: protein

(xi) SEQUENCE DESCRIPTION: SEQ ID NO:18:

Met Asp Lys Val Gly Lys Met Trp Asn Asn Leu Lys Tyr Arg Cys Gln
1 5 10 15

Asn Leu Phe Ser His Glu Gly Gly Ser Arg Asn Glu Asn Val Glu Met
20 25 30

Asn Pro Asn Arg Cys Pro Ser Val Lys Glu Lys Ser Ile Ser Leu Gly
35 40 45

Glu Ala Ala Pro Gln Gln Glu Ser Ser Pro Leu Arg Glu Asn Val Ala
50 55 60

Leu Gln Leu Gly Leu Ser Pro Ser Lys Thr Phe Ser Arg Arg Asn Gln

```
65                  70                  75                  80

Asn Cys Ala Ala Glu Ile Pro Gln Val Val Glu Ile Ser Ile Glu Lys
                85                  90                  95

Asp Ser Asp Ser Gly Ala Thr Pro Gly Thr Arg Leu Ala Arg Arg Asp
            100                 105                 110

Ser Tyr Ser Arg His Ala Pro Trp Gly Gly Lys Lys Lys His Ser Cys
        115                 120                 125

Ser Thr Lys Thr Gln Ser Ser Leu Asp Thr Glu Lys Lys Phe Gly Arg
    130                 135                 140

Thr Arg Ser Gly Leu Gln Arg Arg Glu Arg Arg Tyr Gly Val Ser Ser
145                 150                 155                 160

Met Gln Asp Met Asp Ser Val Ser Ser Arg Ala Val Gly Ser Arg Ser
                165                 170                 175

Leu Arg Gln Arg Leu Gln Asp Thr Val Gly Leu Cys Phe Pro Met Arg
            180                 185                 190

Thr Tyr Ser Lys Gln Ser Lys Pro Leu Phe Ser Asn Lys Arg Lys Ile
        195                 200                 205

His Leu Ser Glu Leu Met Leu Glu Lys Cys Pro Phe Pro Ala Gly Ser
    210                 215                 220

Asp Leu Ala Gln Lys Trp His Leu Ile Lys Gln His Thr Ala Pro Val
225                 230                 235                 240

Ser Pro His Ser Thr Phe Phe Asp Thr Phe Asp Pro Ser Leu Val Ser
                245                 250                 255

Thr Glu Asp Glu Glu Asp Arg Leu Arg Glu Arg Arg Arg Leu Ser Ile
            260                 265                 270

Glu Glu Gly Val Asp Pro Pro Pro Asn Ala Gln Ile His Thr Phe Glu
        275                 280                 285

Ala Thr Ala Gln Val Asn Pro Leu Tyr Lys Leu Gly Pro Lys Leu Ala
    290                 295                 300

Pro Gly Met Thr Glu Ile Ser Gly Asp Gly Ser Ala Ile Pro Gln Ala
```

305 310 315 320

Ile Val Thr Gln Lys Arg Ile Gln Pro Pro Tyr Val Cys Ser His Gly
325 330 335

Gly Arg Ser Ser Ala Arg Cys Pro Gly Thr Ala Thr Arg Thr Leu Ala
340 345 350

Asp Arg Glu Leu Gly Lys Phe Ile Arg Arg Ser Ile Thr Tyr Thr Ala
355 360 365

Ser Cys Gln Ile Cys Phe Arg Ser Gln Gly Ile Pro Val Thr Gly Ala
370 375 380

* Trp Thr Asp Thr Arg Pro Lys Pro Phe * Lys Gly Asn Arg Lys
385 390 395 400

Ala Arg Ser Cys Ser Gly Thr Leu His Arg Arg Thr Thr Ser Ser Leu
405 410 415

* Ala Ser Ala Ala Thr Thr Gly Leu Cys Thr Pro Gly Ser Ser Ser
420 425 430

Gly Thr Thr Thr Ser Ala Ser Met Pro Met Thr Pro Ala Cys Phe Thr
435 440 445

Pro Pro Arg His Gly Ala Ser Arg Thr Leu * Arg Pro Gln Leu Leu
450 455 460

His Val Phe * Thr Val Ala Asn Asp Ile Thr Glu * Asn Phe Pro
465 470 475 480

Phe Gln Pro Ala Val Tyr Leu Pro Arg Ser Asp Leu Gln Met His Tyr
485 490 495

Val * Trp Asp * Arg Ala Pro Ala Thr Val Asp Val Thr Gly Phe
500 505 510

Phe Lys Arg Val Ser Leu * Thr Lys Ser * Gly Ser Leu Val Arg
515 520 525

Thr Arg Pro Val Lys Ala Lys
530 535

(2) INFORMATION FOR SEQ ID NO:19:

(i) SEQUENCE CHARACTERISTICS:
(A) LENGTH: 1221 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:19:

GATTAAACAG CATACAGCTC CTGTGAGCCC ACATTCAACA TTTTTTGATA CTTTGATCCA 60

TCTTTGGTTT CTACAGAAGA TGAAGAAGAT AGGCTTAGAG AGAGAAGGCG GCTTAGTATT 120

GAAGAAGGGG TTGATCCCCC TCCCAATGCA CAAATACATA CATTTGAAGC TACTGCACAG 180

GTTAATCCAT TATTAAACTG GGACCAAAAT TAGCTCCTGG AATGACTGAA ATAAGTGGGG 240

ACAGTTCTGC AATTCCACAA GCTAATTGTG ACTCGGAAGA GGATACAACC ACCCTGTGTT 300

GCAGTCACGG AGGCAGAAGC AGCGTCAGAT ATCTGGAGAC AGCCATACCC ATGTTAGCAG 360

ACAGGGAGCT TGGAAAGTCC ACACACAGAT TGATTACATA CACTGCTTCG TGCCTGATTT 420

GCTTCAAATT ACAGGGAATC CCTGTTACTG GGGAGTGATG GACCGTTATG AAGCAGAAGC 480

CCTTCTCGAA GGGAAACCTG AAGGCACGTT TTTGCTCAGG GACTCTGCGC AAGAGGACTA 540

CTTCTTCTCT GTGAGCTTCC GCCGATACAA CAGATCCCTG CATGCCCGAA TTGAGCAGTG 600

GAATCACAAC TTTAGTTTCG ACGCCCATGA CCCGTGTGTA TTTCACTCCT CCACTGTAAC 660

GGGACTTTTA GAACATTATA AAGATCCCAG TTCGTGCATG TTTTTTGAAC CATTGCTTAC 720

TATATCACTA AATAGGACTT TCCCTTTTAG CCTGCAGTAT ATCTGTCGCG CGGTAATCTG 780

CAGGTGCACT ACGTATGATG GAATTGATGG GCTCCCTCTA CCCTCAATGT TACAGGATTT 840

TTTAAAAGAG TATCATTATA AACAAAAAGT TAGAGTTCGC TGGTTGGAAC GAGAACCAGT 900

CAAGGCAAAG TAAACTCTCC GGTCCCCAAA GGGTGTTAAC TAGGTCCGCT TTCATGTGCA 960

TCAGACAGTA CACCTATAGC AAGCACACGT AGCAGTGTTA GGCTTTTTCA TACAGTATGT 1020

AAGCTTAGTG TTAGTATCTG TCAGATGCTA CCTGCTGTTA CTTATTCAGA TAAACATGGT 1080

GCCTATTGGA ACAATAGCGG ATAGAGCTAC AGGTGTTCAG TAAGACTACA AAAACATTTT 1140

GCCTATTTCG CTAACAGTTT GGTTTTTAAT GGCTGTGGTA TTTGAGTGAG GCAACTCTGG 1200

GGCATTTGTT ATGAAGAAAT G 1221

(2) INFORMATION FOR SEQ ID NO:20:

(i) SEQUENCE CHARACTERISTICS:
(A) LENGTH: 2369 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 116..1330

(xi) SEQUENCE DESCRIPTION: SEQ ID NO:20:

GGCACGAGGC GGTGGTGGCG GCGGCGGGCG CGGCCGCGGC GGGGCGGGCG CGGAATGAAG 60

GCCCACGGCC CTGGGGGCTG AGGCGCCCGC CGCCTGGGGC GGGCCGCGCG TCCTC ATG 118
Met
1

GAG GCC GGA GAG GAG CCG CTG CTG CTG GCT GAA CTC AAG CCT GGG CGC 166
Glu Ala Gly Glu Glu Pro Leu Leu Leu Ala Glu Leu Lys Pro Gly Arg
5 10 15

CCC CAC CAG TTC GAC TGG AAG TCA AGC TGC GAG ACC TGG AGC GTG GCC 214
Pro His Gln Phe Asp Trp Lys Ser Ser Cys Glu Thr Trp Ser Val Ala
20 25 30

TTC TCG CCA GAC GGT TCC TGG TTC GCC TGG TCT CAA GGA CAC TGC GTG 262

```
Phe Ser Pro Asp Gly Ser Trp Phe Ala Trp Ser Gln Gly His Cys Val
     35                  40                  45

GTC AAG CTG GTC CCC TGG CCC TTA GAG GAA CAG TTC ATC CCT AAA GGA      310
Val Lys Leu Val Pro Trp Pro Leu Glu Glu Gln Phe Ile Pro Lys Gly
 50                  55                  60                  65

TTC GAA GCC AAG AGC CGA AGC AGC AAG AAT GAC CCA AAA GGA CGG GGC      358
Phe Glu Ala Lys Ser Arg Ser Ser Lys Asn Asp Pro Lys Gly Arg Gly
                 70                  75                  80

AGT CTG AAG GAG AAG ACG CTG GAC TGT GGC CAG ATT GTG TGG GGG CTG      406
Ser Leu Lys Glu Lys Thr Leu Asp Cys Gly Gln Ile Val Trp Gly Leu
             85                  90                  95

GCC TTC AGC CCG TGG CCC TCT CCA CCC AGC AGG AAA CTC TGG GCA CGT      454
Ala Phe Ser Pro Trp Pro Ser Pro Pro Ser Arg Lys Leu Trp Ala Arg
        100                 105                 110

CAC CAT CCC CAG GCG CCT GAT GTT TCT TGC CTG ATC CTG GCC ACA GGT      502
His His Pro Gln Ala Pro Asp Val Ser Cys Leu Ile Leu Ala Thr Gly
    115                 120                 125

CTC AAC GAT GGG CAG ATC AAG ATT TGG GAG GTA CAG ACA GGC CTC CTG      550
Leu Asn Asp Gly Gln Ile Lys Ile Trp Glu Val Gln Thr Gly Leu Leu
130                 135                 140                 145

CTT CTG AAT CTT TCT GGC CAC CAA GAC GTC GTG AGA GAT CTG AGC TTC      598
Leu Leu Asn Leu Ser Gly His Gln Asp Val Val Arg Asp Leu Ser Phe
                150                 155                 160

ACG CCC AGC GGC AGT TTG ATT TTG GTC TCT GCA TCC CGG GAT AAG ACA      646
Thr Pro Ser Gly Ser Leu Ile Leu Val Ser Ala Ser Arg Asp Lys Thr
            165                 170                 175

CTT CGA ATT TGG GAC CTG AAT AAA CAC GGT AAG CAG ATC CAG GTG TTA      694
Leu Arg Ile Trp Asp Leu Asn Lys His Gly Lys Gln Ile Gln Val Leu
        180                 185                 190

TCC GGC CAT CTG CAG TGG GTT TAC TGC TGC TCC ATC TCC CCT GAC TGT      742
Ser Gly His Leu Gln Trp Val Tyr Cys Cys Ser Ile Ser Pro Asp Cys
    195                 200                 205

AGC ATG CTG TGC TCT GCA GCT GGG GAG AAG TCG GTC TTT CTG TGG AGC      790
Ser Met Leu Cys Ser Ala Ala Gly Glu Lys Ser Val Phe Leu Trp Ser
```

```
210                215                220                225

ATG CGG TCC TAC ACA CTA ATC CGG AAA CTA GAA GGC CAC CAA AGC AGT      838
Met Arg Ser Tyr Thr Leu Ile Arg Lys Leu Glu Gly His Gln Ser Ser
                230                235                240

GTT GTC TCC TGT GAT TTC TCT CCT GAT TCA GCC TTG CTT GTC ACA GCT      886
Val Val Ser Cys Asp Phe Ser Pro Asp Ser Ala Leu Leu Val Thr Ala
            245                250                255

TCG TAT GAC ACC AGT GTG ATT ATG TGG GAC CCC TAC ACC GGC GCG AGG      934
Ser Tyr Asp Thr Ser Val Ile Met Trp Asp Pro Tyr Thr Gly Ala Arg
        260                265                270

CTG AGG TCA CTT CAT CAC ACA CAA CTT GAA CCC ACC ATG GAT GAC AGT      982
Leu Arg Ser Leu His His Thr Gln Leu Glu Pro Thr Met Asp Asp Ser
    275                280                285

GAC GTC CAC ATG AGC TCC CTG AGG TCC GTG TGC TTC TCA CCT GAA GGC     1030
Asp Val His Met Ser Ser Leu Arg Ser Val Cys Phe Ser Pro Glu Gly
290                295                300                305

TTG TAT CTC GCT ACG GTG GCA GAT GAC AGG CTG CTC AGG ATC TGG GCT     1078
Leu Tyr Leu Ala Thr Val Ala Asp Asp Arg Leu Leu Arg Ile Trp Ala
                310                315                320

CTG GAA CTG AAG GCT CCG GTT GCC TTT GCT CCG ATG ACC AAT GGT CTT     1126
Leu Glu Leu Lys Ala Pro Val Ala Phe Ala Pro Met Thr Asn Gly Leu
            325                330                335

TGC TGC ACG TTC TTC CCA CAC GGT GGA ATT ATT GCC ACA GGG ACG AGA     1174
Cys Cys Thr Phe Phe Pro His Gly Gly Ile Ile Ala Thr Gly Thr Arg
        340                345                350

GAT GGC CAT GTC CAG TTC TGG ACA GCT CCC CGG GTC CTG TCC TCA CTG     1222
Asp Gly His Val Gln Phe Trp Thr Ala Pro Arg Val Leu Ser Ser Leu
    355                360                365

AAG CAC TTA TGC AGG AAA GCC CTC CGA AGT TTC CTG ACA ACG TAT CAA     1270
Lys His Leu Cys Arg Lys Ala Leu Arg Ser Phe Leu Thr Thr Tyr Gln
370                375                380                385

GTC CTA GCA CTG CCA ATC CCC AAG AAG ATG AAA GAG TTC CTC ACA TAC     1318
Val Leu Ala Leu Pro Ile Pro Lys Lys Met Lys Glu Phe Leu Thr Tyr
                390                395                400
```

```
AGG ACT TTC TAGCAGTGCC GGCTCCCCCA CCTCCTGCAG CAGCAGCAGT              1367
Arg Thr Phe
            405

ACAAGGGACT GGCTAGGATG GAGTCAGGCA GCTCACACTG GACCAGTGTG GACCTTCCTT    1427

CCTCCCATGG CATGTGCAAG TAGGTCTGCG TGACCCCACT TCTGTGGTGC CGGCCTTACC    1487

TCGTCTTCAT CCGTGGTGAG CAGCCTTCGT CAGTCTAGTT GTGTTGAAGC CAAGTGCAGT    1547

TGTGGATGTT GCTGGGGTAA TAAAGGCAAG CGGGCTCCAG AGCCTCTCTG GTGGCGGCCA    1607

AGCCACACTC CCTTAACTGG GAAGTACCTG CCACGTAGGG CATTTCTGCT GCCTATTTCC    1667

AGCCAGCGGC TGCATGGTTT GAAGTTCCTC CGTTGTGGTC AGAAGAACTC TGGTGTTTGG    1727

TTCCCTGCTC AGCTGCGCGT GGACTGGGCT GAGCTCCTCA CCATACACTA GTGCCGGCTT    1787

TTGTTTCCTG TAAACAGTGG TTGCATGTGT AGAGAAGTAA CAAGCGAGTA TTCAGATCAT    1847

ACGAGGAGGC GTTCCTCGGT GCATGACGGT CAGATGGCCA TTTATCAGCA TATTTATTTG    1907

TATTTTCTCA GCACATAGTA AGGTACAACT GTGTTTTCTC AATTGTCTCG AAAAAACAGA    1967

GTTCTTAAGT GGCCCAGTTG TGGAGCCAAG TCTAAGTCGT GTGGAGTCAG TGCTGACATC    2027

ACTGGCTTGT GCTGTCTGTC ACATGTGTTT GTCTCTGCTG CTTGACCTCA TGGGATGTAC    2087

CCTCCAGTTC AACTGCCCAA AACAGACAGC CCCTTCCAAG CACCGTTCTT TGACAGCGGT    2147

AGCAGCTACC TATTCAAGAC GCCTCACACA AAATCTGCCT TAGAAAGTTA ATATATTTTA    2207

AATTATTTTA AAAGAAACTC AACATCTTAT TCTTTGGCCT TTCTTAATTG ATGCTTTATG    2267

GAGGCAGTGT TAACATTGTA CAGTGTATGC ATAGAGGAGT CTCCTCTATT TGAAGAACAA    2327

TGCAAAATGA GGCTTTCATT GAAGGGAAAA AAAAAAAAAA AA                       2369
```

(2) INFORMATION FOR SEQ ID NO:21:

(i) SEQUENCE CHARACTERISTICS:
- (A) LENGTH: 404 amino acids
- (B) TYPE: amino acid
- (D) TOPOLOGY: linear

(ii) MOLECULE TYPE: protein

(xi) SEQUENCE DESCRIPTION: SEQ ID NO:21:

```
Met Glu Ala Gly Glu Glu Pro Leu Leu Leu Ala Glu Leu Lys Pro Gly
 1               5                  10                  15

Arg Pro His Gln Phe Asp Trp Lys Ser Ser Cys Glu Thr Trp Ser Val
            20                  25                  30

Ala Phe Ser Pro Asp Gly Ser Trp Phe Ala Trp Ser Gln Gly His Cys
        35                  40                  45

Val Val Lys Leu Val Pro Trp Pro Leu Glu Glu Gln Phe Ile Pro Lys
    50                  55                  60

Gly Phe Glu Ala Lys Ser Arg Ser Ser Lys Asn Asp Pro Lys Gly Arg
65                  70                  75                  80

Gly Ser Leu Lys Glu Lys Thr Leu Asp Cys Gly Gln Ile Val Trp Gly
                85                  90                  95

Leu Ala Phe Ser Pro Trp Pro Ser Pro Pro Ser Arg Lys Leu Trp Ala
            100                 105                 110

Arg His His Pro Gln Ala Pro Asp Val Ser Cys Leu Ile Leu Ala Thr
        115                 120                 125

Gly Leu Asn Asp Gly Gln Ile Lys Ile Trp Glu Val Gln Thr Gly Leu
    130                 135                 140

Leu Leu Leu Asn Leu Ser Gly His Gln Asp Val Val Arg Asp Leu Ser
145                 150                 155                 160

Phe Thr Pro Ser Gly Ser Leu Ile Leu Val Ser Ala Ser Arg Asp Lys
                165                 170                 175

Thr Leu Arg Ile Trp Asp Leu Asn Lys His Gly Lys Gln Ile Gln Val
            180                 185                 190

Leu Ser Gly His Leu Gln Trp Val Tyr Cys Cys Ser Ile Ser Pro Asp
        195                 200                 205

Cys Ser Met Leu Cys Ser Ala Ala Gly Glu Lys Ser Val Phe Leu Trp
    210                 215                 220
```

Ser Met Arg Ser Tyr Thr Leu Ile Arg Lys Leu Glu Gly His Gln Ser
225 230 235 240

Ser Val Val Ser Cys Asp Phe Ser Pro Asp Ser Ala Leu Leu Val Thr
245 250 255

Ala Ser Tyr Asp Thr Ser Val Ile Met Trp Asp Pro Tyr Thr Gly Ala
260 265 270

Arg Leu Arg Ser Leu His His Thr Gln Leu Glu Pro Thr Met Asp Asp
275 280 285

Ser Asp Val His Met Ser Ser Leu Arg Ser Val Cys Phe Ser Pro Glu
290 295 300

Gly Leu Tyr Leu Ala Thr Val Ala Asp Asp Arg Leu Leu Arg Ile Trp
305 310 315 320

Ala Leu Glu Leu Lys Ala Pro Val Ala Phe Ala Pro Met Thr Asn Gly
325 330 335

Leu Cys Cys Thr Phe Phe Pro His Gly Gly Ile Ile Ala Thr Gly Thr
340 345 350

Arg Asp Gly His Val Gln Phe Trp Thr Ala Pro Arg Val Leu Ser Ser
355 360 365

Leu Lys His Leu Cys Arg Lys Ala Leu Arg Ser Phe Leu Thr Thr Tyr
370 375 380

Gln Val Leu Ala Leu Pro Ile Pro Lys Lys Met Lys Glu Phe Leu Thr
385 390 395 400

Tyr Arg Thr Phe

(2) INFORMATION FOR SEQ ID NO:22:

(i) SEQUENCE CHARACTERISTICS:
(A) LENGTH: 1246 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:22:

```
GACACTGCAT CGTCAAACTG ATCCCCTGGC CGTTGGAGGA GCAGTTCATC CCTAAAGGGT     60
TTGAAGCCAA AAGCCGAAGT AGCAAAAATG AGACGAAAGG GCGGGGCAGC CCAAAAGAGA    120
AGACGCTGGA CTGTGGTCAG ATTGTCTGGG GGCTGGCCTT CAGCCTGTGC TTTCCCCACC    180
CAGCAGGAAG CTCTGGGCAC GCCACCACCC CCAAGTGCCC GATGTCTCTT GCCTGGTTCT    240
TGCTACGGGA CTCAACGATG GGCAGATCAA GATCTGGGAG GTGCAGACAG GGCTCCTGCT    300
TTTGAATCTT TCCGGCCACC AAGATGTCGT GAGAGATCTG AGCTTCACAC CCAGTGGCAG    360
TTTGATTTTG GTCTCCGCGT CACGGGATAA GACTCTTCGC ATCTGGGACC TGAATAAACA    420
CGGTAAACAG ATTCAAGTGT TATCGGGCCA CCTGCAGTGG GTTTACTGCT GTTCCATCTC    480
CCCAGACTGC AGCATGCTGT GCTCTGCAGC TGGAGAGAAG TCGGTCTTTC TATGGAGCAT    540
GAGGTCCTAC ACGTTAATTC GGAAGCTAGA GGGCCATCAA AGCAGTGTTG TCTCTTGTGA    600
CTTCTCCCCC GACTCTGCCC TGCTTGTCAC GGCTTCTTAC GATACCAATG TGATTATGTG    660
GGACCCCTAC ACCGGCGAAA GGCTGAGGTC ACTCCACCAC ACCCAGGTTG ACCCCGCCAT    720
GGATGACAGT GACGTCCACA TTAGCTCACT GAGATCTGTG TGCTTCTCTC CAGAAGGCTT    780
GTACCTTGCC ACGGTGGCAG ATGACAGACT CCTCAGGATC TGGGCCCTGG AACTGAAAAC    840
TCCCATTGCA TTTGCTCCTA TGACCAATGG GCTTTGCTGG CACATTTTTT CCACATGGTG    900
GAGTCATTGC CACAGGGACA AGAGATGGCC ACGTCCAGTT CTGGACAGCT CCTAGGGTCC    960
TGTCCTCACT GAAGCACTTA TGCCGGAAAG CCCTTCGAAG TTTCCTAACA ACTTACCAAG   1020
TCCTAGCACT GCCAATCCCC AAGAAAATGA AAGAGTTCCT CACATACAGG ACTTTTTAAG   1080
CAACACCACA TCTTGTGCTT CTTTGTAGCA GGGTAAATCG TCCTGTCAAA GGGAGTTGCT   1140
GGAATAATGG GCCAAACATC TGGTCTTGCA TTGAAATAGC ATTTCTTTGG GATTGTGAAT   1200
```

AGAATGTAGC AAAACCAGAT TCCAGTGTAC TAGTCATGGA TTTTTC 1246

(2) INFORMATION FOR SEQ ID NO:23:

(i) SEQUENCE CHARACTERISTICS:
(A) LENGTH: 422 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:23:

ACCATGGTTC CAAGTCCTCT CCCCTGTGGT CAAGTTGCCC GAATGTTGGG CCCAAGTGCC 60

TTTTCCTCCT TGGGCCTCCC CTTCTGACCT GCAGGACAGT TTTCCGGAGC CCATTTGGTA 120

TGAGGTATTA ATTAGCCTTA ACTAAATTAC AGGGGACTCA GAGGCCGTGC TCCTGACCGA 180

TCCAGACACT ATTTTTTTTT TTTTTTTTTA ACAATGGTGT GCATGTGCAG GAAATGACAA 240

ATTTGTATGT CAGATTATAC AAGGATGTAT TCTTAAACCG CATGACTATT CAGATGGCTA 300

CTGAGTTATC AGTGGCCATT TATTAGCATC ATATTTATTT GTATTTTCTC AACAGATGTT 360

AAGGTACAAC TGTGTTTTTC TCGATTATCT AAAAACCATA GTACTTAAAT TGAAAAAAAA 420

AA 422

(2) INFORMATION FOR SEQ ID NO:24:

(i) SEQUENCE CHARACTERISTICS:
(A) LENGTH: 2019 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:24:

GGCACGAGGC GGGGTCAGGG CGGAGGCTGA GGACCAAGTA GGCATGGCGG AGGGCGGGAC 60

CGGCCCCGAT GGACGGGCCG GCCCGGGACC CGCAGGTCCT AATCTGAAGG AGTGGCTGAG 120

GGAGCAGTTC TGTGACCATC CACTGGAGCA CTGTGACGAT ACAAGACTCC ATGATGCAGC 180

CTATGTAGGG GACCTCCAGA CCCTCAGGAA CCTACTGCAA GAGGAGAGCT ACCGGAGCCG 240

CATCAATGAG AAGTCTGTCT GGTGCTGCGG CTGGCTTCCC TGCACACCAC TGAGGATCGC 300

AGCCACTGCA GGCCATGGGA ACTGTGTGGA CTTCCTCATA CGCAAAGGGG CCGAGGTGGA 360

CCTGGTGGAT GTCAAGGGGC AGACTGCCCT GTATGTGGCT GTAGTGAACG GGCACTTGGA 420

GAGCACTGAG ATCCTTTTGG AAGCTGGTGC TGATCCCAAC GGCAGCCGGC ACCACCGCAG 480

CACTCCTGTG TACCATGCCT YTCGTGTGGG TAGGGACGAC ATCCTGAAGG CTCTTATCAG 540

GTATGGGGCA GATGTTGATG TCAACCATCA TCTGAATTCT GACACCCGGC CCCCTTTTTC 600

ACGGCGGCTA ACCTCCTTGG TGGTCTGTCC TCTATACATC AGTGCTGCCT ACCATAACCT 660

TCAGTGCTTC AGGCTGCTCT TGCAGGCTGG GGCAAATCCT GACTTCAATT GCAATGGCCC 720

TGTCAACACC CAGGAGTTCT ACAGGGGATC CCCTGGGTGT GTCATGGATG CTGTCCTGCG 780

CCATGGCTGT GAAGCAGCCT TCGTGAGTCT GTTGGTAGAG TTTGGAGCCA ACCTGAACCT 840

GGTGAAGTGG GAATCCCTGG GCCCAGAGGC AAGAGGCAGA AGAAAGATGG ATCCTGAGGC 900

CTTGCAGGTC TTTAAAGAGG CCAGAAGTAT TCCCAGGACC TTGCTGAGTT TGTGCCGGGT 960

GGCTGTGAGA AGAGCTCTTG GCAAATACCG ACTGCATCTG GTTCCCTCGC TGCCGCTGCC 1020

AGACCCCATA AAGAAGTTTT TGCTTTATGA GTAGCATTCA CATGCAGTGC TGACTGCAAT 1080

GTGGAAGCCG ATCACCTGCA GTGAAAACTG ACACAGACTC TGGCATCCTG GGAACCATGG 1140

CCTGTGCTGC CAGCTTGATC CTTGGCTGTC AGTGAAGAAA AAACGGCTGT GTTCTCTTGG 1200

ACTGTGATTC TATCTCAGGT GCTTGGGCCA TCGAACGCTC CTTGAGTCAT TGTCAACTGA 1260

```
GAGGCACATA CAAACTTAAT TTTGTTCCTC TTCAGTCTCT CTGTTTTGGA TTCTTCCTGG    1320
CAATGTGTGC AGCATGGGCT GAGCCTGGTG ATTGCCCTAG TGGGGAAGGC TTTTTTCTCC    1380
AGGCTATGCA TCTATTTATG TTCCTACTTT GCAATTTATT GTTCTTTTAA GGCTTGATAT    1440
CAAAACAGAA AGAGGTTTGT TAAGAAAAGA TATAGGGAGA AAGGAATTCC GGTTCCGTGC    1500
ACTTGCTAGC CTGCTTTCCT TGCCTGGGTT TGTCTGTCTA TGCTGCCTGG TGCACATCCC    1560
TTCTCTTTGC TGCCACTGTT CTATTTTGGG AGTTGTCTTC CGTCTAAGAT GGCTTCTGGG    1620
GTTCTATCTT ATTGCACAGA GGTCCCAGAA CAGTGTTCAT AGGGCACCAT CTGCTCTGCC    1680
AAGGGTTTTC TGATGTCTTA CCCTGGGGAT CTTCAGACAG TGGTTACCTT TAGGAGACCC    1740
ACCTGGAACT AACCATTAAG TGACTGCCCA CATTCAGATC AGGGACCATC TTAATAGTAC    1800
TCACTGCCAG TCCTCACAAG AGAAGATGAC ACGGGTGCTC TCTTCAGACA CTCCCATACA    1860
GGAAGTTGGA AAATGTCTTG GTCACCTGGG TTGTTCCCAG GCTACAACTT CTTGGTGTTC    1920
CACTAARACC AGRATATCCT AGTTTTTTGG GTTGACTGTT CCCTCCCCAC TTTCCTTGAA    1980
NCCCAATGCC CNTTTGTKTN GGTTGCTTCC CTAAAAKTT                           2019
```

(2) INFORMATION FOR SEQ ID NO:25:

(i) SEQUENCE CHARACTERISTICS:
(A) LENGTH: 350 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:25:

```
Ala Arg Gly Gly Val Arg Ala Glu Ala Glu Asp Gln Val Gly Met Ala
1               5                   10                  15
```

Glu Gly Gly Thr Gly Pro Asp Gly Arg Ala Gly Pro Gly Pro Ala Gly
20 25 30

Pro Asn Leu Lys Glu Trp Leu Arg Glu Gln Phe Cys Asp His Pro Leu
35 40 45

Glu His Cys Asp Asp Thr Arg Leu His Asp Ala Ala Tyr Val Gly Asp
50 55 60

Leu Gln Thr Leu Arg Asn Leu Leu Gln Glu Glu Ser Tyr Arg Ser Arg
65 70 75 80

Ile Asn Glu Lys Ser Val Trp Cys Cys Gly Trp Leu Pro Cys Thr Pro
85 90 95

Leu Arg Ile Ala Ala Thr Ala Gly His Gly Asn Cys Val Asp Phe Leu
100 105 110

Ile Arg Lys Gly Ala Glu Val Asp Leu Val Asp Val Lys Gly Gln Thr
115 120 125

Ala Leu Tyr Val Ala Val Val Asn Gly His Leu Glu Ser Thr Glu Ile
130 135 140

Leu Leu Glu Ala Gly Ala Asp Pro Asn Gly Ser Arg His His Arg Ser
145 150 155 160

Thr Pro Val Tyr His Ala Xaa Arg Val Gly Arg Asp Asp Ile Leu Lys
165 170 175

Ala Leu Ile Arg Tyr Gly Ala Asp Val Asp Val Asn His His Leu Asn
180 185 190

Ser Asp Thr Arg Pro Pro Phe Ser Arg Arg Leu Thr Ser Leu Val Val
195 200 205

Cys Pro Leu Tyr Ile Ser Ala Ala Tyr His Asn Leu Gln Cys Phe Arg
210 215 220

Leu Leu Leu Gln Ala Gly Ala Asn Pro Asp Phe Asn Cys Asn Gly Pro
225 230 235 240

Val Asn Thr Gln Glu Phe Tyr Arg Gly Ser Pro Gly Cys Val Met Asp
245 250 255

Ala Val Leu Arg His Gly Cys Glu Ala Ala Phe Val Ser Leu Leu Val
260 265 270

Glu Phe Gly Ala Asn Leu Asn Leu Val Lys Trp Glu Ser Leu Gly Pro
275 280 285

Glu Ala Arg Gly Arg Arg Lys Met Asp Pro Glu Ala Leu Gln Val Phe
290 295 300

Lys Glu Ala Arg Ser Ile Pro Arg Thr Leu Leu Ser Leu Cys Arg Val
305 310 315 320

Ala Val Arg Arg Ala Leu Gly Lys Tyr Arg Leu His Leu Val Pro Ser
325 330 335

Leu Pro Leu Pro Asp Pro Ile Lys Lys Phe Leu Leu Tyr Glu
340 345 350

(2) INFORMATION FOR SEQ ID NO:26:

(i) SEQUENCE CHARACTERISTICS:
(A) LENGTH: 419 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:26:

GCATCCATGG CGGAGGGCGG CAGCACGACG GGCGGGCAGG GCCGGGCTCC GCAGGTCGTA 60

ATCTGAAGGA GTGGCTGAGG GAGCAATTTT GTGATCATCC GCTGGAGCAC TGTGAGGACA 120

CGAGGCTCCA TGATGCAGCT TACGTCGGGG ACCTCCAGAC CCTCAGGAGC CTATTGCAAG 180

AGGAGAGCTA CCGGAGCCGC ATCAACGAGA AGTCTGTCTG GTGCTGTGGC TGGCTCCCCT 240

GCACACCGTT GCGAATCGCG GCCACTGCAG GCCATGGGAG CTGTGTGGAC TTCCTCATCC 300

GGAAGGGGGC CGAGGTGGAT CTGGTGGACG TAAAAGGACA GACGGCCCTG TATGTGGCTG 360

TGGTGAACGG GCACCTAGAG AGTACCCAGA TCCTTCTCGA AGCTGGCGCG GACCCCAAC 419

(2) INFORMATION FOR SEQ ID NO:27:

(i) SEQUENCE CHARACTERISTICS:
(A) LENGTH: 595 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:27:

GAGGAAGAAG AAAAGTGGAC CCTGAGGCCT TGCAGGTCTT TAAAGAGGCC AGAAGTGTTC 60

CCAGAACCTT GCTGTGTCTG TGCCGTGTGG CTGTGAGAAG AGCTCTTGGC AAAACCGGCT 120

TCATCTGATT CCTTCGCTGC CTCTGCCAGA CCCCATAAAG AAGTTTCTAC TCCATGAGTA 180

GACTCCAAGT GCTGCGGTTG ATTCCAGTGA GGGAGAAAGT GATCTGCAGG GAGGTGGACA 240

CCGAGCCCTG AGTGCTGTGC TGCTGCTGGT CTCCTGATGG CTGTTGCTGC AGAAGATGTC 300

CTCGTAGACT GTCATTGCTC CTCAGGTGCC TGGGCCGCTG AACAGTCCTT GGGTCATTGT 360

CAGCTGAGAG GCTTATACTA AAGTTATTAT TGTTTTTCCC AAGTTCTCTG TTCTGGATTT 420

TCAGTTGCAT ATTAATGTAA CGGGCCATGG GGTATGTACA TGTAGGGGCT GAGGTTGGAG 480

GCCTACTAAT TTCCTGTAGG GAAGACTCCC AGCACTTCTG GAACTGTGCT TCTCTTTATT 540

TTTCTACTTC TCAATTTGAT GGTTCGATTA AAGCCTTCTA GTATCTCAAT GAAAA 595

(2) INFORMATION FOR SEQ ID NO:28:

(i) SEQUENCE CHARACTERISTICS:
(A) LENGTH: 896 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 4..396

(xi) SEQUENCE DESCRIPTION: SEQ ID NO:28:

```
CTG ATG TCC GCA ATT CTG AAG GTT GGA CAC CAC TGC TGG CTG CCT GTG      48
    Met Ser Ala Ile Leu Lys Val Gly His His Cys Trp Leu Pro Val
     1               5                  10                  15

ACA TCC GCT GTC AAT CCC CAA AGG ATG CTG AGG CCA CCA CCA ACC GCT      96
Thr Ser Ala Val Asn Pro Gln Arg Met Leu Arg Pro Pro Pro Thr Ala
                20                  25                  30

GTT TTC AAC TGT GCC GCT TGC TGC TGT CTG TGG GGG CAG ATG CTG ATG     144
Val Phe Asn Cys Ala Ala Cys Cys Cys Leu Trp Gly Gln Met Leu Met
            35                  40                  45

AAT ACA TAC CGT GTA GTT CAG CTT CCT GAG GAG GCC AAG GGC TTG GTG     192
Asn Thr Tyr Arg Val Val Gln Leu Pro Glu Glu Ala Lys Gly Leu Val
        50                  55                  60

CCA CCA GAG ATT CTA CAG AAG TAC CAT GGA TTC TAC TCT TCC CTC TTT     240
Pro Pro Glu Ile Leu Gln Lys Tyr His Gly Phe Tyr Ser Ser Leu Phe
    65                  70                  75

GCC TTG GTG AGG CAG CCC AGG TCG CTG CAG CAT CTC TGC CGT TGT GCG     288
Ala Leu Val Arg Gln Pro Arg Ser Leu Gln His Leu Cys Arg Cys Ala
 80                  85                  90                  95

CTC CGC AGT CAC CTG GAG GGC TGT CTG CCC CAT GCA CTA CCG CGC CTT     336
Leu Arg Ser His Leu Glu Gly Cys Leu Pro His Ala Leu Pro Arg Leu
               100                 105                 110

CCC CTG CCA CCG CGC ATG CTC CGC TTT CTG CAG CTG GAC TTT GAG GAT     384
Pro Leu Pro Pro Arg Met Leu Arg Phe Leu Gln Leu Asp Phe Glu Asp
           115                 120                 125

CTG CTC TAC TAGGCTTGCT GCCCTGTGAA CAAAGCAGAC CCCACCCCCA             433
Leu Leu Tyr
        130
```

```
CCCCAAGGGC ATCTCTCAGC AATGAATGAT GCAAGGCGGT CTGTCTTCAA GTCAGGAGTG      493
GACGCCTTGA TCCACACTTG AGAGAAGAGG CCAGATCAGC ACCYGGCTGG TAGTGATNGC      553
AGAGGGCACC TGTGCAGATC TGTGTGCGCA CTGGAAATCT CTAGGCTGAA GGCYAGAGCA      613
AATGGTGCAR GTGTTAGTCC TTGGGANGAG AGACAGANGG TGAGAAAGCA AGACAGAGGT      673
GAGAGTGCAC ATGTCAAGTG GTAGATTGCC TTAAAAGAAA GCTAAAAAAA GAAAAAGATT      733
CGGGCGAACT TCTTTAGGGG TAATGCTGCA GCGTGTTAAA CTGACTGACC AGCGTCCATA      793
TCTTTGGACC CTTCCCGGGT GAAAAAGCCC CTTCATCCTC CAGCGCTCCC CAAGGGTGCT      853
TAGCAATACC GGGTGCTTTT CTGCCGCAAA GTGAGTTACC AAA                        896
```

(2) INFORMATION FOR SEQ ID NO:29:

(i) SEQUENCE CHARACTERISTICS:
(A) LENGTH: 130 amino acids
(B) TYPE: amino acid
(D) TOPOLOGY: linear

(ii) MOLECULE TYPE: protein

(xi) SEQUENCE DESCRIPTION: SEQ ID NO:29:

```
Met Ser Ala Ile Leu Lys Val Gly His His Cys Trp Leu Pro Val Thr
  1               5                  10                  15

Ser Ala Val Asn Pro Gln Arg Met Leu Arg Pro Pro Pro Thr Ala Val
             20                  25                  30

Phe Asn Cys Ala Ala Cys Cys Cys Leu Trp Gly Gln Met Leu Met Asn
         35                  40                  45

Thr Tyr Arg Val Val Gln Leu Pro Glu Glu Ala Lys Gly Leu Val Pro
     50                  55                  60

Pro Glu Ile Leu Gln Lys Tyr His Gly Phe Tyr Ser Ser Leu Phe Ala
 65                  70                  75                  80

Leu Val Arg Gln Pro Arg Ser Leu Gln His Leu Cys Arg Cys Ala Leu
                 85                  90                  95
```

```
Arg Ser His Leu Glu Gly Cys Leu Pro His Ala Leu Pro Arg Leu Pro
            100                 105                 110

Leu Pro Pro Arg Met Leu Arg Phe Leu Gln Leu Asp Phe Glu Asp Leu
        115                 120                 125

Leu Tyr
    130
```

(2) INFORMATION FOR SEQ ID NO:30:

(i) SEQUENCE CHARACTERISTICS:
(A) LENGTH: 436 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:30:

```
GTGGGGGCGT CATCATGACC TCCTCTAGGG CTCTGCAACA TGACTCCTGT GGTGCAAATC    60

AACAAATTGT TCACTGATGA ATCCACAAGG ATCTCTGGGC CTACAACCAG GTCCTGGTCC   120

ACATGACTGT CGTCTTCGGA GAAGGCACCA CTCGCCCCCG GCAGGTACGG CTGACACCTC   180

CATGGGAGAA GACGTATCCA GGCAGCAGCT GCGCGGCCCT TCAAGAGGGC ACATCCCGTC   240

ATCTAAAGGC ACGGTGTACT GAAGGTAGTC CTGAGACATG AGTCCGATTA CTACAGGCAC   300

GTGTTCCTCC AGGTGGAGGC TCAGGTCCCC GGGTGAGCTG GGGCTGCAGC GGGACTCAGG   360

GCGCGGCTCT GGCTGCAGGT CTCGCAGCTC CCTGGGCTGT AGCTCCCGCA GATCCTTGCG   420

CACACCGTTG ACTGGT                                                   436
```

(2) INFORMATION FOR SEQ ID NO:31:

(i) SEQUENCE CHARACTERISTICS:

(A) LENGTH: 2180 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:31:

TTAATAGTAC CTACATAGTA GAAAATTATA ACTCCACTTT AAAACAATGT TTTCTTTCTA 60
TTCAAATCAA TTTAAAACTT TTTATAAACA TTAATGTTGC AAGAGAATCC AGTCCATTTA 120
TGAAAATTAG TTGACAATCA AGTTCACCCA AGAAAATGTT GACTAAGCTA AAGAAATCAC 180
AGATAAAACA TTTTACCAAA AGGATAGGTA ACACACAAAA AAATGCTATC ACAGGAAGCT 240
ATGATCATCT AATATTTCTT TAATAATAAT TCTAGTTCCA TAGGTTTTCA TGTTATGCCA 300
ATTTGTACCC GAGTTTAATT ACAGAAAAGG CAACAATTTC TAAATTGGTG GTATACATTT 360
CTTTACAATT TTTTAATGTA AGGCCATTTA TTAAAATAGA CAAACTAGAA GATGAAAACG 420
AAGGCAACAG AAAAATTCAA CTTTTCACAA CCAAAAGAAT TAGCACAACC TTAGAAATAA 480
TTTAGAAAAA AGTGTTGTTA AAAGATATGT TGCAGATCTC CGTTCCATTA CCCAAGATTA 540
TGTCAATTCA CGATTCTAAA TAAATCTTTT TAAAGTAAGA GATTAAAAAC TCATCTTCAG 600
TGTATATGTA AATTCCGTGG TTTTATCACA CAGGTATGTT TATTCAACAC TGCTTTGGAA 660
ATGGACCATT TAAAAGGACA TGGCAATTTC CATTCTGTTA AGTTTCATTC AACCTTTACT 720
TAGGGGTTGA TTACCACATG AAATGTGCTT TTAATGCATA AAAATCACAG TGGATTAGCC 780
AGCAAAAGGG ACTGGGCGGG GGGGGCATTG AGGAGAATTT GATAATTCAC ATTGTGATTA 840
TTCTGCACAT TGATGAAACA TAATTCACAC CTCTAAAACC TCAAGACTTC CCTTTTTTAA 900
AGAACCAAAA TAAACCCAAG ACACCTTGCT GACACTTCCC CACCCCTAAA CAAACTGATG 960
ACTCTTTTAC ACATAAAACT GAAATAGTTA TGGCAGCAAA AGATTTTGAT GGCAATGAAA 1020

```
GTTTGTAAAC TGTATTTCAA TCTCTTGTTC TTATTCCCAA AGTGCAAGAT GCAGGGTTCT   1080
CAATCTTTCA GTAGTGCTTC TCCTGTAAAT AATCCTTCAT TTTGTTTGGC AAAGGCAGTT   1140
TCTGAATTAA GTCTATTCTG GTATACTGAC GTATAACAAA ACGACACAGG TACTGCAACG   1200
AGCGCACCTA TGAACCCCGG AACACTGGTT GGCAAGTTCT GACGGAAGTG CAGATTCCAG   1260
GCAGCGAGAC CTTGAATAAC AAAAAGCTCC CATTTTCAGA GTCCCTGATT GAATGCTCCA   1320
ATTAGATCAA CTATGGACGT ATGTCCTTCC ACATCGGCTG TTCATAAAAG CTAAACCTAC   1380
CATTTGAGTG CTCAATTCTA GTGTGAAGTG TTTTACCATG GGAGCGAAAG TCACAGCTTA   1440
AAAGGTAACG GTCGTCAGAA CTGTCCCGAA CAAGAAAAGA ACCATCTGGC ACGTTTGCTA   1500
GCTTCCCTTC TGCCTCCCAA CGTGTGATTG GTCCCCAGTA CCATCCTTGC TTTGCAAGTT   1560
TTTTCAGCTC CTCTGTAAGG CTTGTCACAA CCATGGGACC ACTACTTTGC ACTGAGTCAT   1620
AAACTCTTGC AACCCCAGGA GCAGAGTTCG GATCAAAATT CAAATGACAG CGCATAACTT   1680
TCAGCCACGT GGGGCTTTCT GTCCAGTGAG TCCACTGAAA GTTCCCCTTT GGGATTTGGA   1740
TTATTCCTGC ATTGGAGTAA CCAATGGTGA AGATTGGAGG GACATCCATC GTGAACCCGC   1800
TCTCCGGGGT TCTGCAACAT GACTCCCGTG GTGCCAATCA ACAAGCCATT CACCGGACTG   1860
ATCCACGAAG ATCTCTGGGG CGACAACTAG GTCCTGGTCT ACCTGACTCT CATCCTCGGG   1920
GAAAGCGCGC CCTCCCACTT GAGGAGGAAC CGCAGAGACT TCCATGGGAG AAGAGCTGTC   1980
CAGACAATAG CTCCGTGATC CTTCCAAAGG ATACATCCCC TCATCTAAAG GCACAGTATA   2040
CTGAATGTAG TCCTGAGGCA TAAGTCCAAT AACGACAGGC ACATGTTCAT CCAGGTGAAG   2100
ATGCAGGTCT CCATTATGAG AAGCCGAGCT CTTCAGTGAA TTGGCTTGCT CCTGGCACGT   2160
GGTCTCAGAC TGGAGGTCGT                                               2180
```

(2) INFORMATION FOR SEQ ID NO:32:

(i) SEQUENCE CHARACTERISTICS:

(A) LENGTH: 2649 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:32:

```
GGCACGAGGC TGTGTCCAGC ACACAGAGAG GGCCCGGCCA TCTGCTTTGG TTCAGAGCCC    60
TGTGTCTGTC TGTCACTTAG ACTCTTCCTC CCGGCTCGCA GCTCACCCTC CATCCTCCTT   120
ACTGGCTCCA GCATGACTCG CTTCTCTTAT GCAGAGTACT TTGCTCTGTT TCACTCTGGC   180
TCTGCACCTT CCAGGTCCCC TTCGTCTCCC GAGAACCCAC CGGCCCGCGC ACCCCTGGGT   240
CTGTTCCAAG GGGTCATGCA GAAGTATAGC AGCAACCTGT TCAAGACCTC CCAGATGGCG   300
GCTATGGACC CCGTGCTGAA GGCCATCAAG GAAGGGGATG AAGAGGCCTT GAAGATCATG   360
ATCCAGGATG GGAAGAATCT TGCAGAGCCC AACAAGGAGG GCTGGCTGCC GCTCCACGAG   420
GCTGCCTACT ATGGCCAGCT GGGCTGCCTG AAAGTCCTGC AGCAAGCCTA CCCAGGGACC   480
ATTGACCAAC GCACACTGCA GGAAGAGACA GCATTATACC TGGCCACATG CAGAGAACAC   540
CTGGATTGCC TCCTGTCGCT GCTCCAGGCG GGGGCAGAGC CTGACATCTC TAACAAATCC   600
AGGGAGACTC CACTTTACAA AGCCTGTGAG CGCAAGAACG CGGAGGCGGT GAGGATATTG   660
GTGCGATACA ACGCAGACGC CAACCACCGC TGTAACAGGG GCTGGACCGC ACTGCACGAG   720
TCTGTCTCCC GCAATGACCT GGAGGTCATG GAGATCCTAG TGAGTGGCGG GGCCAAGGTG   780
GAGGCCAAGA ATGTCTACAG CATCACCCCT TTGTTTGTGG CTGCCCAGAG TGGGCAGCTG   840
GAGGCCCTGA GGTTCCTGGC CAAGCATGGT GCAGACATCA ACACGCAGGC CAGTGACAGT   900
GCATCAGCCC TCTACGAGGC CAGCAAGAAT GAGCATGAAG ACGTGGTAGA GTTTCTTCTC   960
TCTCAGGGCG CCGATGCTAA CAAAGCCAAC AAGGACGGCC TGCTCCCCCT GCATGTTGCC  1020
```

TCCAAGAAGG GCAACTATAG AATAGTGCAG ATGCTGCTGC CTGTGACCAG CCGCACGCGC 1080
GTGCGCCGTA GCGGCATCAG CCCGCTGCAC CTAGCGGCCG AGCGCAACCA CGACGCGGTG 1140
CTGGAGGCGC TGCTGGCCGC GCGCTTCGAC GTGAACGCAC CTCTGGCTCC CGAGCGCGCC 1200
CGCCTCTACG AGGACCGCCG CAGTTCTGCG CTCTACTTCG CTGTGGTCAA CAACAATGTG 1260
TACGCCACCG AGCTGTTGCT GCTGGCGGGC GCGGACCCCA ACCGCGATGT CATCAGCCCT 1320
CTGCTCGTGG CCATCCGCCA CGGCTGCCTG CGCACCATGC AGCTGCTGTT GGACCATGGC 1380
GCCAACATCG ACGCCTACAT CGCCACTCAC CCCACCGCCT TTCCAGCCAC CATCATGTTT 1440
GCCATGAAGT GCCTGTCGTT ACTCAAGTTC CTTATGGACC TCGGCTGCGA TGGCGAGCCC 1500
TGCTTCTCCT GCCTGTACGG CAACGGGCCG CACCACCCGC CCCGCGACCT GGCCGCTTCC 1560
ACGACGCACC CGTGGACGAC AAGGCACCTA GCGTGGTGCA GTTCTGTGAG TTCCTGTCGG 1620
CCCCGGAAGT GAGCCGCTGG GCGGGACCCA TCATCGATGT CCTCCTGGAC TATGTGGGCA 1680
ACGTGCAGCT GTGCTCCCGG CTGAAGGAGC ACATCGACAG CTTTGAGGAC TGGGCTGTCA 1740
TCAAGGAGAA GGCAGAACCT CCGAGACCTC TGGCTCACCT CTGCCGGCTG CGGGTTCGGA 1800
AGGCCATAGG AAAATACCGG ATAAAACTCC TGGACACACT GCCGCTTCCC GGCAGGCTAA 1860
TCAGATACTT GAAATATGAG AATACACAGT AACCAGCCTG GAGAGGAGAT GTGGCCTTCA 1920
GACTGTTTCC GGGACGCCCC AGGTGGCCTG CATCCAGGAC CCCCTGGGGT CAGAACAGGT 1980
GTGACCTTGC TGGTTCTTTG CTGGAGCTTC ACCCAAAGTG AGAACCTGAT GTGGGGAGTG 2040
GACGTGGAAC CTCTGCTTTC ACACTGTCAG CGGATCGCAG ACCCGCTCTG CTTCTGGCCA 2100
TAGCCAGAGA CCTTCAACCT GGGGCCAGGG GAGAGCTGGT CTGGGCAAGG TGGCCCAGGC 2160
AGGAATCCTG GCCTTAAGCT GGAGAACTTG TAGGAATCCC TCACTGGACC CTCAGCTTTC 2220
AGGCTGCGAG GGAGACGCCC AGCCCAAGTA TTTTATTTCC GTGACACAAT AACGTTGTAT 2280
CAGAAAAAAA AAAAAACATG GGCGCAGCTT ATTCCTTAGT AGGGTATTTA CTTGCATGCG 2340
CGCTTAAAGC TACTGGAAAC ATGCGTTCCA CTATGCTTGA GAATCCCCTT GCACTGGTAA 2400

ACGAGAGCCG ACGTGCTTCA AGGTTGGATT TTTGGTTGCC CCTTTGGCGT TCCGCGGGTT 2460

TGTCCGACGT AATTGACCCC GTGTTTTGTC ACTTTCGAGT GTTCCGACTA TTGGGGGGCT 2520

TTTGGTTGTC CCCAAAATTG TGGGTGGTGT GCGGACGCCA CGAGAAGTGG TTCATGGGCG 2580

ATAATCATTA CTGGAGAATG TAGAGCGGCG GTTTTACGAA TAAATATTTT TTAAGCCGCC 2640

TTCCCAAAA 2649

(2) INFORMATION FOR SEQ ID NO:33:

(i) SEQUENCE CHARACTERISTICS:
(A) LENGTH: 495 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:33:

CCTCCTGAGA GTTCGCCGGC CCGGGCCCAA TGGGTTGTTC CAAGGGGTCA TGCAGAAATA 60

CAGCAGCAGC TTGTTCAAGA CCTCCCAGCT GGCGCCTGCG GACCCCTTGA TAAAGGCCAT 120

CAAGGATGCG ATGAAGAGGC CTTGAAGACC ATGATCAAGG AAGGGAAGAA TCTCGCAGAG 180

CCCAACAAGG AGGGCTGGCT GCCGCTGCAC GAGGCCGCAT ACTATGGCCA GGTGGGCTGC 240

CTGAAAGTCC TGCAGCGAGC GTACCCAGGG ACCATCGACC AGCGCACCCT GCAGGAGGAA 300

ACAGCCGTTT ACTTGGCAAC GTGCAGGGGC CACCTGGACT GTCTCCTGTC ACTGCTCCAA 360

GCAGGGGCAG AGCGGGACAT CTCCAACAAA TCCCGAGAGA ACCGCTCTAC AAAGCCTGTG 420

AGCGCAAGAA CGCGGAAGCC GTGAAGATTC TTGGTGCAGC ACAACGCAGA CACCAACAAC 480

GCTGCAACCG GGCTG 495

(2) INFORMATION FOR SEQ ID NO:34:

(i) SEQUENCE CHARACTERISTICS:

(A) LENGTH: 709 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:34:

```
GTGCAGCTCT GCTCGCGGCT GAAGGAACAC ATCGACAGCT TTGAGGACTG GGCCGTCATC    60
AAGGAGAAGG CAGAACCTCC AAGACCTCTG GCTCACCTTT GCCGACTGCG GGTTCGAAAG   120
GCCATTGGGA AATACCGTAT AAAACTCCTA GACACCTTGC CGCTCCCAGG CAGGCTGATT   180
AGATACCTGA AATACGAGAA CACCCAGTAA CTGGGGCCAC GGGGAGAGAG GAGTAGCCCC   240
TCAGACTCTT CTTACTAAGT CTCAGGACGT CGGTGTTCCC AACTCCAAGG GGACCTGGTG   300
ACAGACGAGG CTGCAGGCTG CCTCCCTCTC AGCCTGGACA GCTACCAGGA TCTCACTGGG   360
TCTCAGGGCC CAGAGCTTTG GCCAGAGCAG AGAACAGAAT GTGTCAAGGA GAAGAATCAT   420
TTGTTTACAA ACTGATGAGC AGATCCCAGA CCTTCTCTAC CTTCAGGAAT GGCAGAAACC   480
TCTATTCCTG GGGCCAGGGC AGAGCTTGAG GTGTTCTGGG GAAGGTGGTG CTCAGAGCCT   540
TCCCTGTGCC CCTCCACTTG TTCTGGAAAA CTCACCACTT GACTTCAGAG CTTTCTCTCC   600
AAAGACTAAG ATGAAGACGT GGCCCAAGGT AGGGGGTAGG GGGAGCCTGG GTCTTGGAGG   660
GCTTTGTTAA GTATTAATAT AATAAATGTT ACACATGTGA AAAAAAAAA               709
```

(2) INFORMATION FOR SEQ ID NO:35:

(i) SEQUENCE CHARACTERISTICS:
(A) LENGTH: 848 base pairs
(B) TYPE: nucleic acid
(C) STRANDEDNESS: single
(D) TOPOLOGY: linear

(ii) MOLECULE TYPE: DNA

(ix) FEATURE:
(A) NAME/KEY: CDS
(B) LOCATION: 1..624

(xi) SEQUENCE DESCRIPTION: SEQ ID NO:35:

```
TTG GAG AAG TGT GGT TGG TAT TGG GGG CCA ATG AAT TGG GAA GAT GCA      48
Leu Glu Lys Cys Gly Trp Tyr Trp Gly Pro Met Asn Trp Glu Asp Ala
  1               5                  10                  15

GAG ATG AAG CTG AAA GGG AAA CCA GAT GGT TCT TTC CTG GTA CGA GAC      96
Glu Met Lys Leu Lys Gly Lys Pro Asp Gly Ser Phe Leu Val Arg Asp
             20                  25                  30

AGT TCT GAT CCT CGT TAC ATC CTG AGC CTC AGT TTC CGA TCA CAG GGT     144
Ser Ser Asp Pro Arg Tyr Ile Leu Ser Leu Ser Phe Arg Ser Gln Gly
         35                  40                  45

ATC ACC CAC CAC ACT AGA ATG GAG CAC TAC AGA GGA ACC TTC AGC CTG     192
Ile Thr His His Thr Arg Met Glu His Tyr Arg Gly Thr Phe Ser Leu
     50                  55                  60

TGG TGT CAT CCC AAG TTT GAG GAC CGC TGT CAA TCT GTT GTA GAG TTT     240
Trp Cys His Pro Lys Phe Glu Asp Arg Cys Gln Ser Val Val Glu Phe
 65                  70                  75                      80

ATT AAG AGA GCC ATT ATG CAC TCC AAG AAT GGA AAG TTT CTC TAT TTC     288
Ile Lys Arg Ala Ile Met His Ser Lys Asn Gly Lys Phe Leu Tyr Phe
                 85                  90                  95

TTA AGA TCC AGG GTT CCA GGA CTG CCA CCA ACT CCT GTC CAG CTG CTC     336
Leu Arg Ser Arg Val Pro Gly Leu Pro Pro Thr Pro Val Gln Leu Leu
            100                 105                 110

TAT CCA GTG TCC CGA TTC AGC AAT GTC AAA TCC CTC CAG CAC CTT TGC     384
Tyr Pro Val Ser Arg Phe Ser Asn Val Lys Ser Leu Gln His Leu Cys
        115                 120                 125

AGA TTC CGG ATA CGA CAG CTC GTC AGG ATA GAT CAC ATC CCA GAT CTC     432
Arg Phe Arg Ile Arg Gln Leu Val Arg Ile Asp His Ile Pro Asp Leu
    130                 135                 140
```

```
CCA CTG CCT AAA CCT CTG ATC TCT TAT ATC CGA AAG TTC TAC TAC TAT        480
Pro Leu Pro Lys Pro Leu Ile Ser Tyr Ile Arg Lys Phe Tyr Tyr Tyr
145                 150                 155                 160

GAT CCT CAG GAA GAG GTA TAC CTG TCT CTA AAG GAA GCG CAG CGT CAG        528
Asp Pro Gln Glu Glu Val Tyr Leu Ser Leu Lys Glu Ala Gln Arg Gln
                165                 170                 175

TTT CCA AAC AGA AGC AAG AGG TGG AAC CCT CCA CGT AGC GAG GGG CTC        576
Phe Pro Asn Arg Ser Lys Arg Trp Asn Pro Pro Arg Ser Glu Gly Leu
            180                 185                 190

CCT GCT GGT CAC CAC CAA GGG CAT TTG GTT GCC AAG CTC CAG CTT TGAAGAACCA
631
Pro Ala Gly His His Gln Gly His Leu Val Ala Lys Leu Gln Leu
        195                 200                 205

AATTAAGCTA CCATGAAAAG AAGAGGAAAA GTGAGGGAAC AGGAAGGTTG GGATTCTCTG      691

TGCAGAGACT TTGGTTCCCC ACGCAAGCCC TGGGGCTTGG AAGAAGCACA TGACCGTACT      751

CTGCGTGGGG CTCCACCTCA CACCCACCCC TGGGCATCTT AGGACTGGAG GGGCTCCTTG      811

GAAAACTGGA AGAAGTCTCA ACACTGTTTC TTTTTCA                               848
```

(2) INFORMATION FOR SEQ ID NO:36:

(i) SEQUENCE CHARACTERISTICS:
- (A) LENGTH: 207 amino acids
- (B) TYPE: amino acid
- (D) TOPOLOGY: linear

(ii) MOLECULE TYPE: protein

(xi) SEQUENCE DESCRIPTION: SEQ ID NO:36:

```
Leu Glu Lys Cys Gly Trp Tyr Trp Gly Pro Met Asn Trp Glu Asp Ala
  1               5                  10                  15

Glu Met Lys Leu Lys Gly Lys Pro Asp Gly Ser Phe Leu Val Arg Asp
             20                  25                  30

Ser Ser Asp Pro Arg Tyr Ile Leu Ser Leu Ser Phe Arg Ser Gln Gly
         35                  40                  45
```

```
Ile Thr His His Thr Arg Met Glu His Tyr Arg Gly Thr Phe Ser Leu
     50                  55                  60

Trp Cys His Pro Lys Phe Glu Asp Arg Cys Gln Ser Val Val Glu Phe
 65                  70                  75                  80

Ile Lys Arg Ala Ile Met His Ser Lys Asn Gly Lys Phe Leu Tyr Phe
                 85                  90                  95

Leu Arg Ser Arg Val Pro Gly Leu Pro Pro Thr Pro Val Gln Leu Leu
            100                 105                 110

Tyr Pro Val Ser Arg Phe Ser Asn Val Lys Ser Leu Gln His Leu Cys
        115                 120                 125

Arg Phe Arg Ile Arg Gln Leu Val Arg Ile Asp His Ile Pro Asp Leu
    130                 135                 140

Pro Leu Pro Lys Pro Leu Ile Ser Tyr Ile Arg Lys Phe Tyr Tyr Tyr
145                 150                 155                 160

Asp Pro Gln Glu Glu Val Tyr Leu Ser Leu Lys Glu Ala Gln Arg Gln
                165                 170                 175

Phe Pro Asn Arg Ser Lys Arg Trp Asn Pro Pro Arg Ser Glu Gly Leu
            180                 185                 190

Pro Ala Gly His His Gln Gly His Leu Val Ala Lys Leu Gln Leu
        195                 200                 205
```

(2) INFORMATION FOR SEQ ID NO:37:

(i) SEQUENCE CHARACTERISTICS:
- (A) LENGTH: 464 base pairs
- (B) TYPE: nucleic acid
- (C) STRANDEDNESS: single
- (D) TOPOLOGY: linear

(ii) MOLECULE TYPE: DNA

(xi) SEQUENCE DESCRIPTION: SEQ ID NO:37:

GTTCCAAGCC TAACCCATCT TTGTCGTTTG GAAATTCGGG CCAGTCTAAA AGCAGAGCAC 60

CTTCACTCTG ACATTTTCAT CCATCAGTTG CCACTTCCCA GAAGTCTGCA GAACTATTTG 120

CTCTATGAAG AGGTTTTAAG AATGAATGAG ATTCTAGAAC CAGCAGCTAA TCAGGATGGA 180

GAAACCAGCA AGGCCACCTG ACACAGGTCC TTTAATTCTG TTTAGTCACA AAAGACGGCT 240

TGTGTGACTG TTTGGATTTG GTGATCAAAT GTCCATGTTT ACAGTTGCTT TTCCCAGTTT 300

GTGTCTTTCC CAATATTGTG AACCTTATCC ATCTTGCCTT ACTCAGTTTT ATTTCTAGTG 360

CACTTTGTTG TGTATTATTT GTTTACCTGA CCATTTTCTA CTTTATTCTG CTAATAAACT 420

GTAATTCTGA AAAAAAAAAA AAAAAAAAAA AAAAAAAAAA AAAA 464

(2) INFORMATION FOR SEQ ID NO:38:

(i) SEQUENCE CHARACTERISTICS:
(A) LENGTH: 747 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:38:

GGGGATCGAA AGCGGGGGCT TCTGGGACGC AGCTCTGGAG ACGCGGCCTC GGACCAGCCA 60

TTTCGGTGTA GAAGTGGCAG CACGGCAGAC TGGTCAAACA AATGGATTTT ACAGAGGCTT 120

ACGCGGACAC GTGCTCTACA GTTGGACTTG CTGCCAGGGA AGGCAATGTT AAAGTCTTAA 180

GGAAACTGCT CAAAAAGGGC CGAAGTGTCG ATGTTGCTGA TAACAGGGGA TGGATGCCAA 240

TTCATGAAGC AGCTTATCAC AACTCTGTAG AATGTTTGCA AATGTTAATT AATGCAGATT 300

CATCTGAAAA CTACATTAAG ATGAAGACCT TTGAAGGTTT CTGTGCTTTG CATCTCGCTG 360

CAAGTCAAGG ACATTGGAAA ATCGTACAGA TTCTTTTAGA AGCTGGGGCA GATCCTAATG 420

CAACTACTTT AGAAGAAACG ACACCATTGT TTTTAGCTGT TGAAAATGGA CAGATAGATG 480

TGTTAAGGCT GTTGCTTCAA CACGGAGCAA ATGTTAATGG ATCCCATTCT ATGTGTGGAT 540

GGAACTCCTT GCACCAGGCT TCTTTTCAGG AAAATGCTGA GATCATAAAA TTGCTTCTTA 600

GAAAAGGAGC AAACAAGGAA TGCCAGGATG ACTTTGGAAT CACACCTTTA TTTGTGGCTG 660

CTCAGTATGG CCAAGCTAGA AAGCTTTGAA GCATACTTAT TTCATCCGGG TGCAAATGTC 720

AATTGTCAAG CCTTGGACAA AGCTACC 747

(2) INFORMATION FOR SEQ ID NO:39:

(i) SEQUENCE CHARACTERISTICS:
(A) LENGTH: 1018 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:39:

CACAAATGGG ACCATACAAA AATCTTGGAC TTGTTAATAA CCACTTACTA ACCGGGACCT 60

GTGACACTGG GCTAAACAAA GTAAGTCCCT GTTTACTCAG CAGTGTTTGG GGGACATGAA 120

GGATTGCCTA GAAATATTAC TCCGGAATGG TCTACAGCCC AGACGCCCAG GCGTGCCTTG 180

TTTTTGGATT CAGTTCTCCT GTGTGCATGG CTTTCCAAAA GGAGGTGGAG CTGTAGTTCT 240

TTGGAATTGT GAACATTCTT TTGAAATATG GAGCCCAGAT AAATGAACTT CATTTGGCAT 300

ACTGCCTGAA GTACGAGAAG TTTTCGATAT TTCGCTACTT TTTGAGGAAA GGTTGCTCAT 360

TGGGACCATG GAACCATATA TATGAATTTG TAAATCATGC AATTAAAGCA CAAGCAAAAT 420

ATAAGGAGTG GTTGCCACAT CTTCTGGTTG CTGGATTTGA CCCACTGATT CTACTGTGCA 480

ATTCTTGGAT TGACTCAGTC AGCATTGACA CCCTTATCTT CACTTTGGAG TTTACTAATT 540

GGAAGACACT TGCACCAGCT GTTGAAAGGA TGCTCTCTGC TCGTGCCTCA AACGCTTGGA 600

TTCTACAGCA ACATATTGCC CACTGTTCCA TCCCTGACCC ATCTTTGTCG TTTGGAAATT 660

CGGTCCAGTC TAAAATCAGA ACGTCTACGG TCTGACAGTT ATATTAGTCA GCTGCCACTT 720

CCCAGAAGCC TACATAATTA TTTGCTCTAT GAAGACGTTC TGAGGATGTA TGAAGTTCCA 780

GAACTGGCAG CTATTCAAGA TGGATAAATC AGTGAAACTA CTTAACACAG CTAATTTTTT 840

TCTCTGAAAA ATCATCGAGA CAAAAGAGCC ACAGAGTACA AGTTTTTATG ATTTTATAGT 900

CAAAAGATGA TTATTGATTG TCAGATAGGT TAGGTTTTGG GGGGCCAGTA GTTCAGTGAG 960

AATGTTTATG TTTACAACTA GCCTTCCCAG TAAAAAAAAA AAAAAAAAAA AAAAAAAA 1018

(2) INFORMATION FOR SEQ ID NO:40:

(i) SEQUENCE CHARACTERISTICS:
(A) LENGTH: 1897 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:40:

CGGGGGGCTG GGACCTGGGG CGTAACCGTC TCTACCACGA CGGCAAGAAC CAGCCAAGTA 60

AAACATACCC AGCCTTTCTG GAGCCGGACG AGACATTCAT TGTCCCTGAC TCCTTTTTCG 120

TGGCCCTGGA CATGRATGAT GGGACCTTAA GTTTCATCGT GGATGGACAG TACATGGGAG 180

TGGCTTTCCG GGGACTCAAG GGTAAAAAGC TGTATCCTGT AGTGAGTGCC GTCTGGGGCC 240

ACTGTGAGAT CCGCATGCGC TACTTGAACG GACTTGATCC TGAGCCCCTG CCACTCATGG 300

ACCTGTGCCG GCGTTCGGTG CGCCTAGCGC TGGGAAAAGA GCGCCTGGGT GCCATCCCCG 360

CTCTGCCGCT ACCTGCCTCC CTCAAAGCCT ACCTCCTCTA CCAGTGATCC ACATCCCAGG 420

ACCGCCATAC GACAGCCATC TGGTGCCAAR TCACTGAGCC CGTTGGGGTC CGCCGACCCC 480

TGCGCCTGGG ATGGAAGCCC ACCTCAGCCA TGGGCAGACG TGCCCCCTCA TCCTACCGGC 540

```
TGCCTCTGCT GGGGGAACCT ATGCCAACGG ACTTCTCCCT TCCCAACACT GGCTGAAGCA    600
GCAGCACCCA GGCCCTTCCC TGAACCAGAT GCAGAGAATA AACTATGAAA ACCTCTCTCA    660
GGCGCCTTCT GCTCTCAGGT GGAGTGGGCT GCCCCCCACT CTCTGCAGAG AGAGGCTACA    720
CCCACCTGGG GGGTCCTGGG AGGTAAGACT AGTAGGAGGT GCCAGGGCTG ARTCCAAAAG    780
CAGGAATGGC CAGGAMCAGG CCATACAGAT GAAGCTCAGG ATGTCACATA CCATGGACAM    840
TGAGACAGAA CCCCAGGTTG GAMTTCCCTT GGGCCAACGA GTGCCAGCTT TAATGTCAGC    900
TGCMGGTGCT CTGTGGCCTG TATTTATTCT TTAAACAGTA GCAAAGGCCA TTTATTTATT    960
CCACTTAGAA AGGAAACCTT GGTGGGTGGY TTCCCTCGAT GTGCTTTCCC CCACCTCCCT   1020
GGAATGTGTG TGCCACACCT GTCCTTGTCC CAGGCCAGGA CTGTGGCACA TGAGCTGGTG   1080
TGCACAGATA CACGTATGTC GTCGTGCATG ACCCCTGACT AGTTCCTAAG TAGCCCTGCA   1140
CCAAGCACCA GAGCAGACCC CAAGAGAGGC CCGTGCAAGT CCCCATGTCC CCAGGTCCCT   1200
GCTTCTGTTG CCTTGGGACT CATACACCGG CACACGTGTT TCAGCCTCTT GACTTCCATG   1260
AGCTTCGAAT TTTGCCCCCG ATTCTTCTGA TATTTCCCAT TGGCATCCTC CAAAGCTCTG   1320
GGCCTGGAGG GCATTAGGAC ACATGGAATG AGTGGGGTCT CCAGCCCCTG GGAAAGCCAC   1380
TGGCAAGGCA GGATTAGAAA GACCAAGAGC AGGGTGGGGC GCCATGAAGC CTGTATGCCT   1440
CTCAGGCTCA AGACCCCGCC ACACACCCAC TCAAGCCTCA GAAGTGGTGT GTAGGGCAGC   1500
CCCAGGAGAG GAATGCCTGT CCTAGCAGCA CGTACATGGA GCACCCCACA TGTGCTCCAG   1560
CCCTCTGGCT GTTTCTCTTG CTCTAGAATC AACTCCCTAC ATTGGGAATG TAGCCATTTG   1620
GTAGAGGACT TGCCTAGCCT GCAGGAAGCT CACGTTCCAT CCCCTGCACC AAGGAGAATC   1680
AAAGCTCAGG AGGCTGAGGC AGGAGGATTG CTGTCAGTGG TGTACAGAGG TCATGGCCAT   1740
CCTGGGCTAT ATTAAACCTT GTCCTTTAAG AAAAAGAAAA GAAATCAACT TCCATTGAAT   1800
CTGAGTTCTG CTCATTTCTG CACAGGTACA ATAGATGACT TKATTTGTTG AAAAATGKTT   1860
AATATATTTA CMTATATATA TATTTGTAAG AAGCATT                            1897
```

(2) INFORMATION FOR SEQ ID NO:41:

(i) SEQUENCE CHARACTERISTICS:
(A) LENGTH: 134 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:41:

```
Gly Gly Trp Asp Leu Gly Arg Asn Arg Leu Tyr His Asp Gly Lys Asn
1               5                   10                  15

Gln Pro Ser Lys Thr Tyr Pro Ala Phe Leu Glu Pro Asp Glu Thr Phe
            20                  25                  30

Ile Val Pro Asp Ser Phe Phe Val Ala Leu Asp Met Xaa Asp Gly Thr
        35                  40                  45

Leu Ser Phe Ile Val Asp Gly Gln Tyr Met Gly Val Ala Phe Arg Gly
    50                  55                  60

Leu Lys Gly Lys Lys Leu Tyr Pro Val Val Ser Ala Val Trp Gly His
65                  70                  75                  80

Cys Glu Ile Arg Met Arg Tyr Leu Asn Gly Leu Asp Pro Glu Pro Leu
                85                  90                  95

Pro Leu Met Asp Leu Cys Arg Arg Ser Val Arg Leu Ala Leu Gly Lys
            100                 105                 110

Glu Arg Leu Gly Ala Ile Pro Ala Leu Pro Leu Pro Ala Ser Leu Lys
        115                 120                 125

Ala Tyr Leu Leu Tyr Gln
    130
```

(2) INFORMATION FOR SEQ ID NO:42:

(i) SEQUENCE CHARACTERISTICS:
(A) LENGTH: 265 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:42:

AAGGGTAAAA AACTGTATCC TGTAGTGAGT GCCGTCTGGG GCCACTGTAG ATCCGAATGC 60

GCTACTTGAA CGGACTCGAT CCCGAGACTG CCGCTCATGG ATTTGTGCCG TCGCTCGGTG 120

CGCCTGGCCC TGGGGAGGGA GCGCCTGGGG GAGAACCACA CCTGCCGCTG CCGGCTTCCC 180

TCAAGGCCTA CCTCCTCTAC CAGTGACGTT CGCCATCATA CCGCCAGCGC GACAGCCACC 240

TGGTGCCAAC TCACTGAGCC GCCTG 265

(2) INFORMATION FOR SEQ ID NO:43:

(i) SEQUENCE CHARACTERISTICS:
(A) LENGTH: 2438 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:43:

AAGTGGCGGC GGTCCCTGGA GAGCAGGCGG AGGCAGCGGC AAGTCTGACT CTGGGCTGAC 60

CGTGGAGCCG GGGCGGGGGC TGACAGCCAG GCCTCCGCCT GGCGGGAGCC GCACGAGGAG 120

CGGGAGTGGC CGGGCCTCTC TTCCGCGCTT GAGCGAGCGC CGGGTGATGG CGGTGGTGAT 180

GGCGGCAGGC GCTCGGACAG CTCCGCTTGA GCTGAGCTCG GAGAGATCCG TCCAGAAAGT 240

GCCCAGAAGA AACTTCCTCT TAGAAAAGCT GAAAAACACA RTATTTATAA CACTGGAAAT 300
TGTAAAGAAT TTGTTTAAAA TGGCTGAAAA CAATAGTAAA AATGTAGATG TACGGCCTAA 360
AACAAGTCGG AGTCGAAGTG CTGACAGGAA GGATGGTTAT GTGTGGAGTG GAAAGAAGTT 420
GTCTTGGTCC AAAAAGAGTG AGAGTTGTTC TGAATCTGAA GCCATAGGTA CTGTTGAGAA 480
TGTTGAAATT CCTCTAAGAA GCCAAGAAAG GCAGCTTAGC TGTTCGTCCA TTGAGTTGGA 540
CTTAGATCAT TCCTGTGGGC ATAGATTTTT AGGCCGATCC CTTAAACAGA AACTGCAAGA 600
TGCGGTGGGG CAGTGTTTTC CAATAAAGAA TTGTAGTGGC CGACACTCTC CAGGGCTTCC 660
ATCTAAAAGA AAGATTCATA TCAGTGAACT CATGTTAGAT AAGTGCCCTT TCCCACCTCG 720
CTCAGATTTA GCCTTTAGGT GGCATTTTAT TAAACGACAC ACTGTTCCTA TGAGTCCCAA 780
CTCAGATGAA TGGGTGAGTG CAGACCTGTC TGAGAGGAAA CTGAGAGATG CTCAGCTGAA 840
ACGAAGAAAC ACAGAAGATG ACATACCCTG TTTCTCACAT ACCAATGGCC AGCCTTGTGT 900
CATAACTGCC AACAGTGCTT CGTGTACAGG TGGTCACATA ACTGGTTCTA TGATGAACTT 960
GGTCACAAAC AACAGCATAG AAGACAGTGA CATGGATTCA GAGGATGAAA TTATAACGCT 1020
GTGCACAAGC TCCAGAAAAA GGAATAAGCC CAGGTGGGAA ATGGAAGAGG AGATCCTGCA 1080
GTTGGAGGCA CCTCCTAAGT TCCACACCCA GATCGACTAC GTCCACTGCC TTGTTCCAGA 1140
CCTCCTTCAG ATCAGTAACA ATCCGTGCTA CTGGGGTGTC ATGGACAAAT ATGCAGCCGA 1200
AGCTCTGCTG GAAGGAAAGC CAGAGGGCAC CTTTTTACTT CGAGATTCAG CGCAGGAAGA 1260
TTATTTATTC TCTGTTAGTT TTAGACGCTA CAGTCGTTCT CTTCATGCTA GAATTGAGCA 1320
GTGGAATCAT AACTTTAGCT TTGATGCCCA TGATCCTTGT GTCTTCCATT CTCCTGATAT 1380
TACTGGGCTC CTGGAACACT ATAAGGACCC CAGTGCCTGT ATGTTCTTTG AGCCGCTCTT 1440
GTCCACTCCC TTAATCCGGA CGTTCCCCTT TTCCTTGCAG CATATTTGCA GAACGGTTAT 1500
TTGTAATTGT ACGACTTACG ATGGCATCGA TGCCCTTCCC ATTCCTTCGC CTATGAAATT 1560
GTATCTGAAG GAATACCATT ATAAATCAAA AGTTAGGTTA CTCAGGATTG ATGTGCCAGA 1620

GCAGCAGTGA TGCGGAGAGG TTAGAATGTC GACCTGCATA CATATTTTCA TTTAATATTT 1680

TATTTTTCTT ATGCCTCTTT GAATTTTTGT ACAAAGGCAG TTGAATCAAA TAAAACTGTG 1740

CCCTAAGTTT TAATTCCAGA TCAATTTATT TTTTTATGA TACACTTGTT ATATATTTTT 1800

AAGCAGGTGT TTGGTTTTGT TTTTACCATA TAAATTTACA TATGGTCCAG GCATATTTAC 1860

AATTTCAAGG CATTGCATAT ACATTTGAAT ATTCTGTATT TTTTAAATAA TCTTTTGTTC 1920

TTTCCTATGT GTGAAATATT TTGCTAATCT ATGCTATCAG TATTCTTGTA TGACCGAATA 1980

GTTACCTATT CTCTTTTCAT CTTGAAGATT TTCAGTAAAG AGTGTTGTAA TCAATCCATT 2040

ATAATGTAAT TGACTTTTGT AATTTGCCAA TAGGAGTGTT AAACAACAAA ATGATTTAAA 2100

ATGAAACTTA ATGTATTTTC ATTTTAAATA TTAACTAAAC CAAGTTTGTT TGTTAGTTAT 2160

TCTAGCCAAT AAGAAAAGAG AATGTAGCAT CCTAGAGGTG TATTTGTTCT GCAGTTTGGC 2220

AGGACCGTCA GTTAGTCCAA ATAAACATCC CCTCAGCGTG GAGGCGAATG GAACCTGTGC 2280

TCCTTTCTTA CGGGAAGCTT TGCAAAGCAA AATAGCAGGG TTACAAGCTT GGAGTTGTTA 2340

AGGCAACTAG AGTTTTCTCT ATTAATTTAT AGACTGTTGT TGCACCTACT TAGCTCTTTT 2400

TTGGGAACTC TAGTTCCCAG GGGAAAATAC CTCGTGCC 2438

(2) INFORMATION FOR SEQ ID NO:44:

(i) SEQUENCE CHARACTERISTICS:
(A) LENGTH: 542 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:44:

Ser Gly Gly Gly Pro Trp Arg Ala Gly Gly Gly Ser Gly Lys Ser Asp
1 5 10 15

Ser Gly Leu Thr Val Glu Pro Gly Arg Gly Leu Thr Ala Arg Pro Pro
            20                  25                  30

Pro Gly Gly Ser Arg Thr Arg Ser Gly Ser Gly Arg Ala Ser Leu Pro
        35                  40                  45

Arg Leu Ser Glu Arg Arg Val Met Ala Val Val Met Ala Ala Gly Ala
    50                  55                  60

Arg Thr Ala Pro Leu Glu Leu Ser Ser Glu Arg Ser Val Gln Lys Val
65                  70                  75                  80

Pro Arg Arg Asn Phe Leu Leu Glu Lys Leu Lys Asn Thr Xaa Phe Ile
                85                  90                  95

Thr Leu Glu Ile Val Lys Asn Leu Phe Lys Met Ala Glu Asn Asn Ser
            100                 105                 110

Lys Asn Val Asp Val Arg Pro Lys Thr Ser Arg Ser Arg Ser Ala Asp
        115                 120                 125

Arg Lys Asp Gly Tyr Val Trp Ser Gly Lys Lys Leu Ser Trp Ser Lys
    130                 135                 140

Lys Ser Glu Ser Cys Ser Glu Ser Glu Ala Ile Gly Thr Val Glu Asn
145                 150                 155                 160

Val Glu Ile Pro Leu Arg Ser Gln Glu Arg Gln Leu Ser Cys Ser Ser
                165                 170                 175

Ile Glu Leu Asp Leu Asp His Ser Cys Gly His Arg Phe Leu Gly Arg
            180                 185                 190

Ser Leu Lys Gln Lys Leu Gln Asp Ala Val Gly Gln Cys Phe Pro Ile
        195                 200                 205

Lys Asn Cys Ser Gly Arg His Ser Pro Gly Leu Pro Ser Lys Arg Lys
    210                 215                 220

Ile His Ile Ser Glu Leu Met Leu Asp Lys Cys Pro Phe Pro Pro Arg
225                 230                 235                 240

Ser Asp Leu Ala Phe Arg Trp His Phe Ile Lys Arg His Thr Val Pro
                245                 250                 255

Met Ser Pro Asn Ser Asp Glu Trp Val Ser Ala Asp Leu Ser Glu Arg
260 265 270

Lys Leu Arg Asp Ala Gln Leu Lys Arg Arg Asn Thr Glu Asp Asp Ile
275 280 285

Pro Cys Phe Ser His Thr Asn Gly Gln Pro Cys Val Ile Thr Ala Asn
290 295 300

Ser Ala Ser Cys Thr Gly Gly His Ile Thr Gly Ser Met Met Asn Leu
305 310 315 320

Val Thr Asn Asn Ser Ile Glu Asp Ser Asp Met Asp Ser Glu Asp Glu
325 330 335

Ile Ile Thr Leu Cys Thr Ser Ser Arg Lys Arg Asn Lys Pro Arg Trp
340 345 350

Glu Met Glu Glu Glu Ile Leu Gln Leu Glu Ala Pro Pro Lys Phe His
355 360 365

Thr Gln Ile Asp Tyr Val His Cys Leu Val Pro Asp Leu Leu Gln Ile
370 375 380

Ser Asn Asn Pro Cys Tyr Trp Gly Val Met Asp Lys Tyr Ala Ala Glu
385 390 395 400

Ala Leu Leu Glu Gly Lys Pro Glu Gly Thr Phe Leu Leu Arg Asp Ser
405 410 415

Ala Gln Glu Asp Tyr Leu Phe Ser Val Ser Phe Arg Arg Tyr Ser Arg
420 425 430

Ser Leu His Ala Arg Ile Glu Gln Trp Asn His Asn Phe Ser Phe Asp
435 440 445

Ala His Asp Pro Cys Val Phe His Ser Pro Asp Ile Thr Gly Leu Leu
450 455 460

Glu His Tyr Lys Asp Pro Ser Ala Cys Met Phe Phe Glu Pro Leu Leu
465 470 475 480

Ser Thr Pro Leu Ile Arg Thr Phe Pro Phe Ser Leu Gln His Ile Cys
485 490 495

```
Arg Thr Val Ile Cys Asn Cys Thr Thr Tyr Asp Gly Ile Asp Ala Leu
            500                 505                 510

Pro Ile Pro Ser Pro Met Lys Leu Tyr Leu Lys Glu Tyr His Tyr Lys
        515                 520                 525

Ser Lys Val Arg Leu Leu Arg Ile Asp Val Pro Glu Gln Gln
    530                 535                 540
```

(2) INFORMATION FOR SEQ ID NO:45:

(i) SEQUENCE CHARACTERISTICS:
(A) LENGTH: 4999 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:45:

```
CCCTCTGGGC AAGCCGCCCC CCCCCCACCC ATCTACCACA CACACACACA CACACACACA    60
CACACATTCA GACCTTGGGG CAAAAACAAA GCAAAATAAC AACAACAAAA ACACTGCCTG   120
TGGAAAGTCC TTACTTCAGG AAGGTTGGCA GATGAGGAGC AAGGGAACAT TTTATCAGGA   180
CTGCCACAAA GGAGTCTTTT TTTTTAATGG TTTTTCAAGA CAGGGTTTCT CTGTATAGCC   240
CTGGCTGTCC TGGAGCTCAC TTTGTAGACC AGGCTGGCCT CGAACTCAGA AATTCGCCTG   300
CCTCTGCCTC CTGAGTGCTG GGATTAAAGG CGTGCAGCAC CATGTCCAAC TGGCATTTTC   360
TCAATTAAGG TTCGTTCCTT TCAGATAACT CTAGGTTCTG GGTCAAGCTG ACACAAGGCT   420
ACACAGCACA GTTTGTATGC CACATTCAGT TCAGAAGACA CCCAACCTCC CTGGAACTGG   480
AACTTATGCA CATTTGTGAG CTTCCACTTG GGAGTGGGAA CCTGAACTGG GTCCTCTGCA   540
AGAGCAGCCG TGCTCTTAAC TGCTGAGCCA TTTCAGCAGC CTCACATCAG AATTAAGTTA   600
```

GAAATTAGCCG GGTATGAATC ATACCCTTAG AATCCTAGCA TCTGAAAGCA GAGCTAAGAG 660

AAACAGGGAT TCAAGACCAG CTCTTGGCTA CAGAGCCCGT CCTGTCCTAG GATGGGCTAC 720

AAGAGACTAT TTCAAAGCCA TCCAAACAAC AATAACTACA ACAACAACAA GGTTAAAATT 780

AGGCTGGGCA CAGGGTACAC ACCTTTAATG CCAACACTCA GGAGGCAGAG GCAGGCTGAT 840

CAGTGTGAGT TTGAGTTCAA CGTGGTCTAC ATAGGGAGTT CTAGGCCAGC AGAGGTTACA 900

GTCTCTCTCT CTCTCTCTCT CTCTCTCTCT CTCTCACACA CACACACACA CACACACACA 960

CACACACACA CACACACGGT GGCATTATGG GATTTTTTTG GGATAAGGTT TCTCTGTCTA 1020

GCCCTGGCAT AGATTCACTC TGTAGACTAG GCTAGCCTTG AACTCAGAGA TCCGCCTGCC 1080

TCTGCCTCCC AAGTGCTGGG ATTATAGGTG TTGCACCACC ACTGCCCAGC CACTTTGGGA 1140

TTTTTGAACT GTTATCAAGA GGCTTTCGAG GAGGTCAAAC TTCAACAGCA ACCTCTCCAT 1200

GATAATGTAG CTAATGATCA AACGACACTC AAAACTTAAC CCTTAAAGCA CACATCCACC 1260

AGACAGCGTG CCCACTCGTA GTTCCATTAC TCAGGAGGCT GAAGCAGGAG GATGAAGGAC 1320

TAAGGCTTCA GCAACCTAGG GAGCCGCAGG GGACAGTAGT CTCAATCCCT ACATTCTCCT 1380

GAACACAGGA GCAGGAGTTC AGGAAGGGTG TCAAGGCCGC TTACTGATCT TAGGGCCTCA 1440

GGAATGACTA GCTCAGGCAG AGAGAGCAAA GGTCTCCAGT GGAGAAGTCT ACACACACAC 1500

ACACACACAC ACACACACAC ACACACACAC AGAATCCAAG GCGATGACGT CATCAAAGGG 1560

TTAATTCTAG TCTGGGATGG GGGGGAGGGT GGGGCACGCA GCTGTCAGGT GGCTTTGGAA 1620

AAATAAACTG CTGAAGAGTC TGACGCCAGG GAGTCCTGGG AGGGACAAGA GGTTACCCAC 1680

TCAAAGAGTG TGCTCCACAA AGCATGCGCG CTTGTCCACG TCTGGAGTCG TCACTTATTT 1740

TTTGCCTGGA TTCTTTGTAG CCGGTGGGTT CTCAAGGCGG TAAGTGGTGT GGCCGCCGTG 1800

GTCTGGGAGG TGACGATAGG GTTAATCGTC CACAGAGCCC AGGGGCGGAG CGCGGGCGGG 1860

CGTCCGCAGC CCCGCTGGAG CCGGAAGCAG TGGCTGGTCA GGGGCGCTTC TAGCCTTCCC 1920

TATCTGTACT TCCACAGAGG TCTCTGCGAG CTAGGGGGAC AGTGAGGTGC GGGGTAGGGG 1980
CCCGGCGTTA GAGCCAGCAA GGGGACGGTT CACGGTAAGG TCTGAGGGAG AGAGAGCTCC 2040
TGAGAAACTT GGGGGGCGCG ACACAGATAG GGTGAAAGCA GAGTGATAGA CCTGGGATGG 2100
TTAGGGGACC AAGGGAAGAC CAGGCTGGTT GGCATACACC GGTGAACGGA TGGGAGTCCT 2160
AGGGAAAGAT GATGCGCCTA ACAGTCCTTT CTGTCTCCAC ACCACTCCAG GGGACGATCC 2220
GGAGCTCAAC TTTCAAAAGC GAGACGCCCC AGCAAGCCTG TTTTGAGAAG TTCTTCAGCG 2280
GCTCTCCTCA TGGGCCAGAC GGCCCTGGCA AGGGGCAGCA GCAGCACCCC TACCTCGCAG 2340
GCTCTGTACT CGGACTTCTC TCCTCCCGAG GGCTTGGAGG AGCTCCTGTC TGCTCCCCCT 2400
CCTGACCTGG TTGCCCAACG GCACCACGGC TGGAACCCCA AGGATTGCTC CGAGAACATC 2460
GATGTCAAGG AAGGGGGTCT GTGCTTTGAG CGGCGCCCTG TGGCCCAGAG CACTGATGGA 2520
GTCCGGGGGA AACGGGGCTA TTCGAGAGGT CTGCACGCCT GGGAGATCAG CTGGCCCCTG 2580
GAGCAAAGGG GCACACACGC CGTGGTGGGC GTGGCCACCG CCCTCGCCCC GCTGCAGGCT 2640
GACCACTATG CGGCGCTTTT GGGCAGCAAC AGCGAGTCCT GGGGCTGGGA TATTGGGCGG 2700
GGAAAATTGT ATCATCAGAG TAAGGGCCTC GAGGCCCCCC AGTATCCAGC TGGACCTCAG 2760
GGTGAGCAGC TAGTGGTGCC AGAGAGACTG CTGGTGGTTC TGGACATGGA GGAGGGGACT 2820
CTTGGCTACT CTATTGGGGG CACGTACCTG GGACCAGCCT TCCGTGGACT GAAGGGGAGG 2880
ACCCTCTATC CCTCTGTAAG TGCTGTTTGG GGCCAGTGCC AGGTCCGCAT CCGCTACATG 2940
GGCGAAAGAA GAGGTGAGAT ACGGACTAGG TGTGGGGAGA TCACTACTCT TGGCAATGGT 3000
TTGGGCTGGA AACTCATGGT TGGAGCACAG GAAGTAGGCT TCTTGTCACT TTGGCCTGTC 3060
ACTTAGATGG CCTTGGATCT AGCTTCACTC CCAATCCCTA TTGGATGTGA TGCACAAATT 3120
CAGAGCCTTT GGGTCTCCCT CAGCTGAGGT GGCGGTGGAA ATGGAGGAAG AAGGAAGGGT 3180
GCCTGAGCAG GATCTCAAGT TCAAGGATGC CTGGAGTTGC TTACTTACCT TGTCTTCCTT 3240
CTCTCTCCGC AGTGGAGGAA CCACAATCCC TTCTGCACCT GAGCCGCCTG TGTGTGCGCC 3300

ATGCTCTGGG GGACACCCGG CTGGGTCAAA TATCCACTCT GCCTTTGCCC CCTGCCATGA 3360
AGCGCTATCT GCTCTACAAA TGACCCAGTA GTACAGGGTG TGCTGGCACC CTACCGTGGG 3420
GACAGGTGGA GAGGCACCCG CTGGCCTAGA CAACTTTAAA AAGCTGGTGA AGCTGGGGGG 3480
GGGGGGCTGG ACCCCTTCAC CTCCCCTTCT CACAGGAGCA AGACATATAG AAATGATATT 3540
AAACACCATG GCAGCCTGGG ACAAAGAGGT TTTTGAAGTA AAAAATGAGA TGTATTGTCA 3600
CAACCTGTTT CATTATTGTT TTTTGTTTTG TTTTACACTC CCCCACCCCA GGCTAGAGCC 3660
CCATCACTGT CTTAAGGAAT TATGACAACC CACAAAGCTC AGGCCCAGGT GTTTATTTCC 3720
CTTACATGTA GGATGGTTCA CAAACACAAT ACAGGGGCTT TGGCACCGTG GGGGAGGGGA 3780
CTATCCCAGG CCTCTTAGGG TCTCATGTAT ACCGAATTCA GACCCGAAAG CTCTGAATTT 3840
CTGCATCAGA CATCCAGTAG AACTTGGGAG TGAAGCTAGA GCCAAGGCCA TCTAAGTGAC 3900
AGGCCAAAGT GACACGAAGC CCACTTCCTG TGCTCCAACC ATGAGTTTCC AGCCCAAACC 3960
AATGGAAGGT GATTTCACTT GTCAGGGCCC AAAGGGACAG TCAGTTCTAC TCCCTCCCCT 4020
CACTAGGAGC CACCTTGGTG ACAGTTGATT CTACCCACTG TAAGTGGTAA AGGGATTGGC 4080
CTGGTCCCAA CCATAATAGG GCGGTGGAAA CGGCTCAGGA GGGTACAGCG TGGATTAGGC 4140
CACAAGATGG GGCAGATGAT GTCATCAGAA GCATGTGACC GGTGGGAGCA GTTACTAAAC 4200
TTCTGGGCAA CCTAGTCCAT GCTATGCAGG CAGGTAGAGG GATGGGCAGT GCTCATTGTT 4260
TGGCATTGAT GATGTCCACA AATTCAGGCT TGAGAGATGC GCCACCCACA AGGAAGCCGT 4320
CCACGTCAGG CTGGCTTGCC AGCTCTTTGC AGGTTGCTCC AGTCACAGAA CCTGTACCAG 4380
GAACAAGAAG ACAGTTTGGT CAGGTCTATG ATCAGAACAC TTAAGCCCCA CCTCTCTGTG 4440
CAAGGCAGCC TCAGTCTGTC TTAGCCCATT TCCGTCTTAG CTAGAGCCAA AGCCACTCAC 4500
CTCCATAAAT GATCCGGGTG CTCTGAGCCA CCCCATCATT GACATTGGAT TTCAGCCATC 4560
CCCGGAGCTT CTCGTGTACT TCCTGTGCCT AGAAGGAGGA GGCAGAGCTA CTAAGTAAGC 4620
TCCTTCCTAT CTATCATTCA AGGAGTAAAA ACCACTGGTT CTCACATAGA GTTGAGTTTC 4680

CAGAAAAGCC CCGGGACCAG AGAGTGGCAA GGCTCCAATC CCACCAGGCT TGGAATGAAC 4740

ATTTTTGGCA AAGTCACTCT CCTTGGTGAG TTTGGGGGCC CTCTGTCTCT AAAGGGGCTT 4800

GGATGGGCTC CATAGCTGTG TGAGTCTGTT AAAGCCGGAC AGGCTGAGGA GCTCTGGGTA 4860

GTTACCTGCT GAGGGGTTGC CGTCTTGCCA GTCCCAATGG CCCACACAGG TTCATAGGCC 4920

AGGACCACCT TGCTCCAGTC TTTCACATTA TCTGTGGGGC AGAGAGGAGA GTGAGTAGGA 4980

AGGAGCTGAC CCGCCAAGC 4999

(2) INFORMATION FOR SEQ ID NO:46:

(i) SEQUENCE CHARACTERISTICS:
(A) LENGTH: 264 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:46:

Met Gly Gln Thr Ala Leu Ala Arg Gly Ser Ser Ser Thr Pro Thr Ser
1 5 10 15

Gln Ala Leu Tyr Ser Asp Phe Ser Pro Pro Glu Gly Leu Glu Glu Leu
20 25 30

Leu Ser Ala Pro Pro Pro Asp Leu Val Ala Gln Arg His His Gly Trp
35 40 45

Asn Pro Lys Asp Cys Ser Glu Asn Ile Asp Val Lys Glu Gly Gly Leu
50 55 60

Cys Phe Glu Arg Arg Pro Val Ala Gln Ser Thr Asp Gly Val Arg Gly
65 70 75 80

Lys Arg Gly Tyr Ser Arg Gly Leu His Ala Trp Glu Ile Ser Trp Pro
85 90 95

Leu Glu Gln Arg Gly Thr His Ala Val Val Gly Val Ala Thr Ala Leu

```
            100                 105                 110

Ala Pro Leu Gln Ala Asp His Tyr Ala Ala Leu Leu Gly Ser Asn Ser
        115                 120                 125

Glu Ser Trp Gly Trp Asp Ile Gly Arg Gly Lys Leu Tyr His Gln Ser
    130                 135                 140

Lys Gly Leu Glu Ala Pro Gln Tyr Pro Ala Gly Pro Gln Gly Glu Gln
145                 150                 155                 160

Leu Val Val Pro Glu Arg Leu Leu Val Val Leu Asp Met Glu Glu Gly
                165                 170                 175

Thr Leu Gly Tyr Ser Ile Gly Gly Thr Tyr Leu Gly Pro Ala Phe Arg
            180                 185                 190

Gly Leu Lys Gly Arg Thr Leu Tyr Pro Ser Val Ser Ala Val Trp Gly
        195                 200                 205

Gln Cys Gln Val Arg Ile Arg Tyr Met Gly Glu Arg Arg Val Glu Glu
    210                 215                 220

Pro Gln Ser Leu Leu His Leu Ser Arg Leu Cys Val Arg His Ala Leu
225                 230                 235                 240

Gly Asp Thr Arg Leu Gly Gln Ile Ser Thr Leu Pro Leu Pro Pro Ala
                245                 250                 255

Met Lys Arg Tyr Leu Leu Tyr Lys
            260
```

(2) INFORMATION FOR SEQ ID NO:47:

(i) SEQUENCE CHARACTERISTICS:
- (A) LENGTH: 5615 base pairs
- (B) TYPE: nucleic acid
- (C) STRANDEDNESS: single
- (D) TOPOLOGY: linear

(ii) MOLECULE TYPE: DNA

(xi) SEQUENCE DESCRIPTION: SEQ ID NO:47:

GTACTTTCTT TATATCTCCA TAATTTTATT TACTATTACT ACATGATACA TTATTTTATA 60

AAAGTCTTTG TAACCTCCTT AAGGATTCAC TGCTTAATCT CCAGTGCTTA GCACAAATCA 120

TTAAATGCGA ACCAGAAACT CTTCCAAATG TGTTACATCT ATAACCTCAT TGGATTCTCA 180

CTACCAACCC CATGCAATAG ATACTAATGT GATCTCTGTC TTACAGAGGA AGAAACAGGC 240

ACAGGGAGGT TCAGTAATTT GCCCAAGGTC ATACACACAC TGGCCTTCAG GTATTCATGC 300

CCGGGGAGTC TGGTCCCACA GCTGGCATGT TTGCCATTAT ATTATATTGC CTCCTTATAG 360

TGTCGGCACT CATTAAGCAC ATTGACAGCT ATGCTTGGTG AGTGACTACT ATGTACCCAG 420

CTCTGTGCTA CATGCTTTAC CTGGATTATT TCAACTGCAC AACAACCCTG TGAGGTAACT 480

ACCATCATTG CTCCTATTTT ACATAACAGA AAACTACAGA AATCTGGGGC TGGGCGTAGT 540

GGCTCATGCC TGAAATCCCA GCACTTTGGG AGACCCTGTC TCTAAAAAAA ATTTTTTTTT 600

GGCCGGACGT GGTGGCTCAC ACCTGTAATC TCAGCACTTT GGGAGGCTAA GGCAGGCAGA 660

TCACAAGGTC AGGAGTTCTA GACCAGCCTG GCCAACATGG CAAAACCCTG TGTCTACTAA 720

AAATACAAAA AATAGCTAGG CGTGGTGGCA GGTGCCTGTA ATCCCAGCTA CTCAGGAGGC 780

TGAGGCAGGA GAATCCCCTG AACCTGGGAG ATGGAGGTTA CAGAGAGCCG AGATCGTGCC 840

GCTGCACTCC AGCCTGGGCA ACAAGAGCAA GACTCTGTCT CGAAAAAAAT AAAAATAAAA 900

ATAAAAATAT TTTTTTAAAA ATTAGCTGGG TGTGGTAGCA CATGCCTGTA GTCCCAGCTA 960

CTTGGGAGGC TGAGGTAGGA GGATCACTTG AGCCCAGGAG GTCAAGGCTG CAGTGGGCTG 1020

TGATGGCGCC ACTGCACTCT AGCCTTGGTG ACAGCAAGAC CCTGTCTCAA AAAAAAAAAA 1080

AAGAGAAATC GGGCAACTTC CCCAAGATCG CGCAGTTAAC TAGTGGCATA GCTTCACTCA 1140

AACTCGAAGT CTTAATCAGG ACACTCTACC AAATGAGATC AACGGCTCAG TAATGGATTG 1200

GCATCCAGTA TGAAGACTGG ACCAGCAGGG AGAACTATGA TGCGTACAGC CTAGAGCCTG 1260

AAGCAGATTT CACAGCCTCA GAGGTGGCAC AGGCTGACTC ACAACCCGGG GCAGAAAGGG 1320

ACCAGCCCAG AAACAGTGAC CCAGAATCAC AGGGAAGTAG AAATGGGATT CGGCACAATG 1380
AAGCCCCTCC TTGACCCCAT GCTCCTTACC CTCAGGGGCG CAGGAGTTAG TCGCTCAGGC 1440
GGCTCAAAGG TCTTGACGGT GGAGAACACC ATCCCCAGGG ATTCCCGACG CGGTGATGCC 1500
ATCAAAGCGT TAATTCTGAG ATGGGCCTGC CCGGGTGCGG ACTCTGCCGC AGCAAGAGAA 1560
GGGTTAACTG CCCCGGGCCT TCGCCGTGGG GGCGGGGCCT CGGGGAGGGT CACAGCCCGG 1620
GACTGAGACC CGAGGTTAAC CGCCCGGGGT GGGCTCCACG GGGGCGGGGC ATGCTCTCCG 1680
CGGCTGCTGC CGGTATAGAG CGGTAACTGC CCAGGAGGGG GCGGGGCCCC ACAGGGGCGT 1740
GGCCTCGGAG CTGCACGGCC GTGGGCGGCG ATGAGAGGGT TAAGCCCCAG AGGGCCCTGG 1800
AGGGGCGGGG CCGCGGGACG GGCTCGGCCC AAGGGAGGAG CTGGGGGCGG AAGCGGCCGG 1860
CGGTCTGCGC CCTGCGCGCC TCGGCTTCTT TCCGCCCGGC TCCTTCAGAG GCCCGGCGAC 1920
CTCCAGGGCT GGGAAGTCAA CCGAGGTTCG GGGGCAGCGG CGAGGGCTCC GGGCGAGTAA 1980
GGGGGATGGT CCATGCTGAG GCCCAAATGG GGCGAACTCG CGAGAGTCTC TGGCGACCTG 2040
GATCAGATGG GGCGAGGGCA GATGAAGGGC CCAGGAGCTT TGGGGCAGCG AGGAGGGAGG 2100
AGCGGGCCCG TTGGCAAACT TGGGTGAAAG GATGGGGTAC CTGGGTGACG AGCCCCCGCC 2160
AGGATTCTGC TCTTCACGCC CCTTTTCTCC CAGCTCCCTT CCAGGTCAAT CCAAACTGGA 2220
GCTCAACTTT CAGAAGAGAA AGACGCCCCA GCAAGCCTCT TTCGGGGAGT CCTCTAGCTC 2280
CTCACCTCCA TGGGCCAGAC AGCTCTGGCA GGGGGCAGCA GCAGCACCCC CACGCCACAG 2340
GCCCTGTACC CTGACCTCTC CTGTCCCGAG GGCTTGGAAG AGCTGCTGTC TGCACCCCCT 2400
CCTGACCTGG GGGCCCAGCG GCGCCACGGT TGGAACCCCA AAGACTGTTC AGAGAACATC 2460
GAGGTCAAGG AAGGAGGGTT GTACTTTGAG CGGCGGCCCG TGGCCCAGAG CACTGATGGG 2520
GCCCGGGGTA AGAGGGGCTA TTCAAGGGGC CTGCACGCCT GGGAGATCAG CTGGCCCCTA 2580
GAGCAGAGGG GCACGCATGC CGTGGTGGGC GTGGCCACGG CCCTCGCCCC GCTGCAGACT 2640
GACCACTACG CGGCGCTGCT GGGCAGCAAC AGCGAGTCGT GGGGCTGGGA CATCGGGCGG 2700

GGGAAGCTGT ACCATCAGAG CAAGGGGCCC GGAGCCCCCC AGTATCCAGC GGGAACTCAG 2760

GGTGAGCAGC TGGAGGTGCC AGAGAGACTG CTGGTGGTTC TGGACATGGA GGAGGGAACT 2820

CTGGGCTACG CTATTGGGGG CACCTACCTG GGGCCAGCAT TCCGCGGACT GAAGGGCAGG 2880

ACCCTCTATC CGGCAGTAAG CGCTGTCTGG GGCCAGTGCC AGGTCCGCAT CCGCTACCTG 2940

GGCGAAAGGA GAGGTGAGGC CTGGGGCAGA CGTGGGGAGA ACTTTCTGTC CCTGGTGGCA 3000

GTGGTTTGGG ATGGAAACTC TTCTGACAAG AGCAGAGGGG ATGGACCTTC ATCCAGCCTG 3060

CCTCAACCTC TGTTCAGTGC TGGGAAAGGC TAGGGGTCTT CACAGCTGTT ATTTAATTTA 3120

ACCCAACAGC AATAGAGGTG AAACAGGCTT GAGAAAGCAA CTTTCTCAAG TTCTCTTGGC 3180

CAGTAAATGG TGAACCTTCA GAATGGAGGG AGGAACTGCA GGGATGAGAG AATTCAGGAG 3240

ATATCAACCC CTGAGCAAGA GGTGCAAAGC GTTAGGTACT GGGTTTGATG TACAGGTCCA 3300

AAAGAAGGAT GGGCAGAGCC AGGTACCCAG GCTGTATACC GGATTCCCTG GGCTCTAACC 3360

TGTCTCTGTG CCACATACCT ACTTCCTTCC TCAGCCACAC CTCTGGATGG AGACACTGGG 3420

GCCCTGGGCA CCAGGGAGGA GAGCAGTGGA GGAGGCAGGG CCTTAGGGTG GGGCAGCAGG 3480

GGAGGAGCCT CCCCAGGAAC TGACTGGGTC CAGGGCTTGG AGCTGCTCTC TGCAGTTGTG 3540

TGGGCTGTAG AGTGGAGGGC CATCCCTCCT CACCTCAGCC CCAGCTCCCA AGCCTCTGGA 3600

GTCAAAGCCT GGGCCAGCTC CACCACTGTC AGAGCCACCT TGGCCTGTTG TTTAGAGGGC 3660

CTTAGCCAGC TCTTCACCCC CAGCTCTGAC TAGGGATGTG TGAAATCTTA TCTGGGAGGC 3720

AGAACTTCCG GGTATCTCAA ATTCCCCTTT CAGCCAGGTG GGCACACTCG AAGCAGGAAA 3780

GCAGAAAGGC ATCTGAGTAG GACCCCGTAG TTTGAGGACA TCTGGCTGGT GGCTGCACCC 3840

ATACTTACAT TCCCCTCCTT CTCTCTCCCA GCGGAGCCAC ACTCCCTTCT GCACCTGAGC 3900

CGCCTGTGTG TGCGCCACAA CCTGGGGGAT ACCCGGCTCG GCCAGGTGTC TGCCCTGCCC 3960

TTGCCCCCTG CCATGAAGCG CTACCTGCTC TACCAGTGAG CCCTGTGATA CCACAGACTG 4020

TGCTGAGGTC TTGCCACCAC CCCTCCCCTT GGGGAGGTGG GGAGGCACTG CTGGCCTAGA 4080

CCAGCTGCTG AAAGCTGGTG AGGCTGAGCC CCTACCCCAA CCCAAGCTCT GCGGAAATCA 4140

ACAGCCCCAG AGCCACTTGG AGGGAGGAAG AAAGGGAGCC GGCGTTCAAG GCTATGACAG 4200

TCTGCTACGC AAAACATTTT TTCAAGTAAA AATAGTAAGA GATGTTGTTA TAGAAACCTG 4260

TTCTTGTTTT TTTTTTTTTC TTGCACAAAT GATCATTTAT ATAGCTGCCT CAAAAAGGAA 4320

GATTATCTGG GCAAGTCCAG TGAAGGCAGA CAAACCACAA GACCTAGTGC CAGGTTTATT 4380

CCCTCACATG GGTGGTTCAC ATACACAGCA CAGAGGCACG GGCACCATGG GAGAGGGCAG 4440

CACTCCTGCC TTCTGAGGGG ATCTTGGCCT CACGGTGTAA GAAGGGAGAG GATGGTTTCT 4500

CTTCTGCCCT CACTAGGGCC TAGGGAACCC AGGAGCAAAT CCCACCACGC CTTCCATCTC 4560

TCAGCCAAGG AGAAGCCACC TTGGTGACGT TTAGTTCCAA CCATTATAGT AAGTGGAGAA 4620

GGGATTGGCC TGGTCCCAAC CATTACAGGG TGAAGATATA AACAGTAAAG GAAGATACAG 4680

TTTGGATGAG GCCACAGGAA GGAGCAGATG ACACCATCAG AAGCATATGC AGGGAAAGGG 4740

CAGTTACTGG GCTTCTGGGC TGCTTAGTCC CTGGCTTGGC AGGAAGGGTA GGGAAGATGG 4800

ATGGGGCTCA TTGTTTGGCA TTGATGATGT CCACGAATTC GGGCTTGAGG GAAGCACCAC 4860

CCACAAGGAA GCCATCCACA TCAGGCTGGC TGGCCAGCTC CTTGCAGGTT GCCCCAGTCA 4920

CAGAGCCTGG GAAGGGAGCA GAACAAGGGC TTGGTCAAGA ATGGGATGAG TCTGCCCCAT 4980

CCCCACCTCC ATGTCCGAGG GCTCAGTCTA GTCCTCAGCC CACTCCACCT CAGCCGGGAA 5040

CCAAAGCCAC TCACCTCCAT AAATGATACG GGTGCTCTGA GCCACCGCAT CAGAGACGTT 5100

GGACTTCAGC CATCCTCGGA GCTTCTCGTG TACTTCCTGG GCCTAGAACA AGAAGCTGGC 5160

CTAAGTAAGA CCTTTTCTGC CTCTCTAAGA GGAAAAATCA CTGGCACCAG TGGACACTTA 5220

GTGTGGTTTC TGACTGAGTC AGAGTACCAG GGCTCTGATC CAAGCCAGGC CCTGGACTGG 5280

ATGCCCTTGG ACAAGTCACT GTCTCTGGGT TCAAGGTCTC TGTGTCTTTG AAATAAGGGG 5340

TTGCCCCATG TGGGCTGTGT CTGTCCAAAC CTATTGAGGC AGGCTGGGAT GAGGGCAGGG 5400

CTCCTGGGCC CGGTTACCTG TTGGGGTGTT GCAGTCTTGC CAGTACCAAT GGCCCACACA 5460

GGCTCATAGG CCAGGACGAC CTTGCTCCAG TCCTTCACGT TATCTGCAGG GCAGAGATAC 5520

AGATGGAGGG AAGGGTGAAC AAGAAAGAGC TCTCCAGCCA GGTTCTCCGG AGTACGAAGA 5580

ACGGTGGCCT ACTGCCCCCT AGTGGACATT GGGGG 5615

(2) INFORMATION FOR SEQ ID NO:48:

(i) SEQUENCE CHARACTERISTICS:
(A) LENGTH: 263 amino acids
(B) TYPE: amino acid
(C) STRANDEDNESS: single
(D) TOPOLOGY: linear

(ii) MOLECULE TYPE: DNA

(xi) SEQUENCE DESCRIPTION: SEQ ID NO:48:

Met Gly Gln Thr Ala Leu Ala Gly Gly Ser Ser Ser Thr Pro Thr Pro
1 5 10 15

Gln Ala Leu Tyr Pro Asp Leu Ser Cys Pro Glu Gly Leu Glu Glu Leu
20 25 30

Leu Ser Ala Pro Pro Pro Asp Leu Gly Ala Gln Arg Arg His Gly Trp
35 40 45

Asn Pro Lys Asp Cys Ser Glu Asn Ile Glu Val Lys Glu Gly Gly Leu
50 55 60

Tyr Phe Glu Arg Arg Pro Val Ala Gln Ser Thr Asp Gly Ala Arg Gly
65 70 75 80

Lys Arg Gly Tyr Ser Arg Gly Leu His Ala Trp Glu Ile Ser Trp Pro
85 90 95

Leu Glu Gln Arg Gly Thr His Ala Val Val Gly Val Ala Thr Ala Leu
100 105 110

Ala Pro Leu Gln Thr Asp His Tyr Ala Ala Leu Leu Gly Ser Asn Ser
115 120 125

```
Glu Ser Trp Gly Trp Asp Ile Gly Arg Gly Lys Leu Tyr His Gln Ser
    130                 135                 140

Lys Gly Pro Gly Ala Pro Gln Tyr Pro Ala Gly Thr Gln Gly Glu Gln
145                 150                 155                 160

Leu Glu Val Pro Glu Arg Leu Leu Val Val Leu Asp Met Glu Glu Gly
                165                 170                 175

Thr Leu Gly Tyr Ala Ile Gly Gly Thr Tyr Leu Gly Pro Ala Phe Arg
            180                 185                 190

Gly Leu Lys Gly Arg Thr Leu Tyr Pro Ala Val Ser Ala Val Trp Gly
        195                 200                 205

Gln Cys Gln Val Arg Ile Arg Tyr Leu Gly Glu Arg Arg Ala Glu Pro
    210                 215                 220

His Ser Leu Leu His Leu Ser Arg Leu Cys Val Arg His Asn Leu Gly
225                 230                 235                 240

Asp Thr Arg Leu Gly Gln Val Ser Ala Leu Pro Leu Pro Pro Ala Met
                245                 250                 255

Lys Arg Tyr Leu Leu Tyr Gln
            260
```

(2) INFORMATION FOR SEQ ID NO:49:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:49:

AGCTAGATCT GGACCCTACA ATGGCAGC 28

(2) INFORMATION FOR SEQ ID NO:50:

(i) SEQUENCE CHARACTERISTICS:

(A) LENGTH: base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:50:

```
AGCTAGATCT GCCATCCTAC TCGAGGGGCC AGCTGG                                36
```

## Description

## FIELD OF THE INVENTION

The present invention relates generally to therapeutic and diagnostic agents. More particularly, the present invention provides therapeutic molecules capable of modulating signal transduction such as but not limited to cytokine-mediated signal transduction. The molecules of the present invention are useful, therefore, in modulating cellular responsiveness to cytokines as well as other mediators of signal transduction such as endogenous or exogenous molecules, antigens, microbes and microbial products, viruses or components thereof, ions, hormones and parasites.

Bibliographic details of the publications referred to in this specification by author are collected at the end of the description. Sequence Identity Numbers (SEQ ID NOs.) for the nucleotide and amino acid sequences referred to in the specification are defined after the bibliography. A summary of the SEQ ID NOs is given in Table 1.

Throughout this specification and the claims which follow, unless the context requires otherwise, the word "comprise", or variations such as "comprises" or "comprising", will be understood to imply the inclusion of a stated integer or group of integers but not the exclusion of any other integer or group of integers.

## BACKGROUND OF THE INVENTION

Cells continually monitor their environment in order to modulate physiological and biochemical processes which in turn affects future behaviour. Frequently, a cell's initial interaction with its surroundings occurs via receptors expressed on the plasma membrane. Activation of these receptors, whether through binding endogenous ligands (such as cytokines) or exogenous ligands (such as antigens), triggers a biochemical cascade from the membrane through the cytoplasm to the nucleus.

Of the endogenous ligands, cytokines represent a particularly important and versatile group.

Cytokines are proteins which regulate the survival, proliferation, differentiation and function of a variety of cells within the body [Nicola, 1994]. The haemopoietic cytokines have in common a four-alpha helical bundle structure and the vast majority interact with a structurally related family of cell surface receptors, the type I and type II cytokine receptors [Bazan, 1990; Sprang, 1993]. In all cases, ligand-induced receptor aggregation appears to be a critical event in initiating intracellular signal transduction cascades. Some cytokines, for example growth hormone, erythropoietin (Epo) and granulocyte-colony-stimulating factor (G-CSF), trigger receptor homodimerisation, while for other cytokines, receptor heterodimerisation or heterotrimerisation is crucial. In the latter cases, several cytokines share common receptor subunits and on this basis can be grouped into three subfamilies with similar patterns of intracellular activation and similar biological effects [Hilton, 1994]. Interleukin-3 (IL-3), IL-5 and granulocyte-macrophage colony-stimulating factor (GM-CSF) use the common β-receptor subunit (βc) and each cytokine stimulates the production and functional activity of granulocytes and macrophages. IL-2, IL-4, IL-7, IL-9, and IL-15 each use the common y-chain (yc), while IL-4 and IL-13 share an alternative γ-chain (γ'c or IL-13 receptor α-chain). Each of these cytokines plays an important role in regulating acquired immunity in the lymphoid system. Finally, IL-6, IL-11, leukaemia inhibitory factor (LEF), oncostatin-M (OSM), ciliary neurotrophic factor (CNTF) and cardiotrophin (CT) share the receptor subunit gpl30. Each of these cytokines appears to be highly pleiotropic, having effects both within and outside the haemopoietic system [Nicola, 1994].

In all of the above cases at least one subunit of each receptor complex contains the conserved sequence elements, termed box1 and box2, in their cytoplasmic tails [Murakami, 1991]. Box1 is a proline-rich motif which is located more proximal to the transmembrane domain than the acidic box 2 element. The box-1 region serves as the binding site for a class of cytoplasmic tyrosine kinases termed JAKs (Janus kinases). Ligand-induced receptor dimerisation serves to increase the catalytic activity of the associated JAKs through cross-phosphorylation. Activated JAKs then tyrosine phosphorylate several substrates, including the receptors themselves. Specific phosphotyrosine residues on the receptor then serve as docking sites for SH2-containing proteins, the best characterised of which are the signal transducers and activators of transcription (STATs) and the adaptor protein, shc. The STATs are then phosphorylated on tyrosines, probably by JAKs, dissociate from the receptor and form either homodimers or heterodimers through the interaction of the SH2 domain of one STAT with the phosphotyrosine residue of the other. STAT dimers then translocate to the nucleus where they bind to specific cytokine-responsive promoters and activate transcription [Darnell, 1994; Ihle, 1995; Ihle, 1995]. In a separate pathway, tyrosine phosphorylated shc interacts with another SH2 domain-containing protein, Grb-2, leading ultimately to activation of members of the MAP kinase family and in turn transcription factors such as fos and jun [Sato, 1993; Cutler, 1993]. These pathways are not unique to members of the cytokine receptor family since cytokines that bind receptor tyrosine kinases also being able to activate STATs and members of the MAP kinase family [David, 1996; Leaman, 1996; Shual, 1993; Sato, 1993; Cutler, 1993].

Four members of the JAK family of cytoplasmic tyrosine kinases have been described, JAK1, JAK2, JAK3 and TYK2, each of which binds to a specific subset of cytokine receptor subunits. Six STATs have been described (STAT1 through STAT6), and these too are activated by distinct cytokine/receptor complexes. For example, STAT1 appears to be functionally specific to the interferon system, STAT4 appears to be specific to IL-12, while STAT6 appears to be specific for IL-4 and IL-13. Thus, despite common activation mechanisms some degree of cytokine specificity may be achieved through the use of specific JAKs and STATs [Thierfelder, 1996; Kaplan, 1996; Takeda, 1996; Shimoda, 1996; Meraz, 1996; Durbin, 1996].

In addition to those described above, there are clearly other mechanisms of activation of these pathways. For example, the JAK/STAT pathway appears to be able to activate MAP kinases independent of the shc-induced pathway [David, 1995] and the STATs themselves can be activated without binding to the receptor, possibly by direct interaction with JAKs [Gupta, 1996]. Conversely, full activation of STATS may require the action of MAP kinase in addition to that of JAKs [David, 1995; Wen, 1995].

While the activation of these signalling pathways is becoming better understood, little is known of the regulation of these pathways, including employment of negative or positive feedback loops. This is important since once a cell has begun to respond to a stimulus, it is critical that the intensity and duration of the response is regulated and that signal transduction is switched off. It is likewise desirable to increase the intensity of a response systemically or even locally as the situation requires.

In work leading up to the present invention, the inventors sought to isolate negative regulators of signal transduction. The inventors have now identified a new family of proteins which are capable of acting as regulators of signalling. The new family of proteins is defined as the suppressor of cytokine signalling (SOCS) family based on the ability of the initially identified SOCS molecules to suppress cytokine-mediated signalling. It should be noted, however, that not all members of the SOCS family need necessarily share suppressor function nor target solely cytokine mediated signalling. The SOCS family comprises at least three classes of protein molecules based on amino acid sequence motifs located N-terminal of a C-terminal motif called the SOCS box. The identification of this new family of regulatory molecules permits the generation of a range of effector or modulator molecules capable of modulating signal transduction and, hence, cellular responsiveness to a range of molecules including cytokines. The present invention, therefore, provides therapeutic and diagnostic agents based on SOCS proteins, derivatives, homologues, analogues and mimetics thereof as well as agonists and antagonists of SOCS proteins.

## SUMMARY OF THE INVENTION

The present invention provides *inter alia* nucleic acid molecules encoding members of the SOCS family of proteins as well as the proteins themselves. Reference hereinafter to "SOCS" encompasses any or all members of the SOCS family. Specific SOCS molecules are defined numerically such as, for example, SOCS1, SOCS2 and SOCS3. The species from which the SOCS has been obtained may be indicated by a preface of a single letter abbreviation where "h" is human, "m" is murine and "r" is rat. Accordingly, "mSOCS1" is a specific SOCS from a murine animal. Reference herein to "SOCS" is not to imply that the protein solely suppresses cytokine-mediated signal transduction, as the molecule may modulate other effector-mediated signal transductions such as by hormones or other endogenous or exogenous molecules, antigens, microbes and microbial products, viruses or components thereof, ions, hormones and parasites. The term "modulates" encompasses up-regulation, down-regulation as well as maintenance of particular levels.

One aspect of the present invention provides a nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein comprises a SOCS box in its C-terminal region .

Another aspect of the present invention provides a nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein comprises a SOCS box in its C-terminal region and a protein:molecule interacting region.

Yet another aspect of the present invention is directed to a nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein comprises a C-terminal region and a protein:molecule interacting region located in a region N-terminal of the SOCS box.

Preferably, the protein:molecule interacting region is a protein:DNA or protein:protein binding region.

Still a further aspect of the present invention provides a nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein comprises a SOCS box in its C-terminal region and one or more of an SH2 domain, WD-40 repeats or ankyrin repeats N-terminal of the SOCS box.

Even still a further aspect of the present invention is directed to a nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein comprises a SOCS box in its C-terminal region wherein the SOCS box comprises the amino acid sequence:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20}$$
$$X_{21} X_{22} X_{23} [Xj]_n X_{24} X_{25} X_{26} X_{27} X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F;
$X_{28}$ is L, I, V, M, A or P;

and a protein:molecule interacting region such as but not limited to one or more of an SH2 domain, WD-40 repeats and/or ankyrin repeats N-terminal of the SOCS box.

Another aspect of the present invention is directed to a nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein exhibits the following characteristics:

(i) comprises a SOCS box in its C-terminal region having the amino acid sequence:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20}$$
$$X_{21} X_{22} X_{23} [Xj]_n X_{24} X_{25} X_{26} X_{27} X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F;
$X_{28}$ is L, I, V, M, A or P; and

(ii) comprises at least one of a SH2 domain, WD-40 repeats and/or ankyrin repeats or other protein:molecule interacting domain in a region N-terminal of the SOCS box.

Preferably, the SOCS molecules modulate signal transduction such as from a cytokine or hormone or other endogenous or exogenous molecule, a microbe or microbial product, an antigen or a parasite.
More preferably, the SOCS molecule modulate cytokine mediated signal transduction.
Still another aspect of the present invention comprises a nucleic acid molecule comprising a sequence of nucleotides encoding or complementary to a sequence encoding a protein or a derivative, homologue, analogue or mimetic thereof or comprises a nucleotide sequence capable of hybridizing thereto under low stringency conditions at 42°C wherein said protein exhibits the following characteristics;

(i) is capable of modulating signal transduction;
(ii) comprises a SOCS box in its C-terminal region having the amino acid sequence:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20}$$
$$X_{21} X_{22} X_{23} [Xj]_n X_{24} X_{25} X_{26} X_{27} X_{28}$$

wherein:

$X_1$ is L, I, V, M, A or P;

$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W ;
$X_8$ is C, T or S ;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F;
$X_{28}$ is L, I, V, M, A or P; and

(iii) comprises at least one of a SH2 domain, WD-40 repeats and/or ankyrin repeats or other protein:molecule interacting domain in a region N-terminal of the SOCS box.

Preferably, the signal transduction is mediated by a cytokine such as one or more of EPO, TPO, G-CSF, GM-CSF, IL-3, IL-2, IL-4, IL-7, IL-13, IL-6, LIF, IL-12, IFN$\alpha$, TNF$\alpha$, IL-1 and/or M-CSF.
Preferably, the signal transduction is mediated by one or more of Interleukin 6 (IL-6), Leukaemia Inhibitory Factor (LIF), Oncostatin M (OSM), Interferon (IFN)-$\alpha$ and/or thrombopoietin.
Preferably, the signal transduction is mediated by IL-6.
Particularly preferred nucleic acid molecules comprise nucleotide sequences substantially set forth in SEQ ID NO:3 (mSOCS1), SEQ ID NO:5 (mSOCS2), SEQ ID NO:7 (mSOCS3), SEQ ID NO:9 (hSOCS1), SEQ ID NO:11 (rSOCS1), SEQ ID NO:13 (mSOCS4), SEQ ID NO:15 and SEQ ID NO:16 (hSOCS4), SEQ ID NO:17 (mSOCS5), SEQ ID NO:19 (hSOCS5), SEQ ID NO:20 (mSOCS6), SEQ ID NO:22 and SEQ ID NO:23 (hSOCS6), SEQ ID NO:24 (mSOCS7), SEQ ID NO:26 and SEQ ID NO:27 (hSOCS7), SEQ ID NO:28 (mSOCS8), SEQ ID NO:30 (mSOCS9), SEQ ID NO:31 (hSOCS9), SEQ ID NO:32 (mSOCS10), SEQ ID NO:33 and SEQ ID NO:34 (hSOCS10), SEQ ID NO:35 (hSOCS11), SEQ ID NO:37 (mSOCS12), SEQ ID NO:38 and SEQ ID NO:39 (hSOCS12), SEQ ID NO:40 (mSOCS13), SEQ ID NO:42 (hSOCS13), SEQ ID NO: 43 (mSOCS14), SEQ ID NO:45 (mSOCS15) and SEQ ID NO:47 (hSOCS15) or a nucleotide sequence having at least about 15% similarity to all or a region of any of the listed sequences or a nucleotide acid molecule capable of hybridizing to any one of the listed sequences under low stringency conditions at 42°C.
Another aspect of the present invention relates to a protein or a derivative, homologue, analogue or mimetic thereof comprising a SOCS box in its C-terminal region.
Yet another aspect of the present invention is directed to a protein or a derivative, homologue, analogue or mimetic thereof comprising a SOCS box in its C-terminal region and a protein: molecule interacting region.
Even yet another aspect of the present invention provides a protein or a derivative, homologue, analogue or mimetic thereof comprising an interacting region located in a region N-terminal of the SOCS box.
Preferably, the protein:molecule interacting region is a protein:DNA or a protein:protein binding region.
Another aspect of the present invention contemplates a protein or a derivative, homologue, analogue or mimetic thereof comprising a SOCS box in its C-terminal region and a SH2 domain, WD-40 repeats or ankyrin repeats N-terminal of the SOCS box.

Still yet another aspect of the present invention provides a protein or a derivative, homologue, analogue or mimetic thereof exhibiting the following characteristics:

(i) comprises a SOCS box in its C-terminal region having the amino acid sequence:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20} X_{21} X_{22} X_{23} [Xj]_n X_{24} X_{25} X_{26} X_{27} X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F;
$X_{28}$ is L, I, V, M, A or P; and

(ii) comprises at least one of a SH2 domain, WD-40 repeats and/or ankyrin repeats or other protein:molecule interacting domain in a region N-terminal of the SOCS box.

Preferably, the proteins modulate signal transduction such as cytokine-mediated signal transduction.

Preferred cytokines are EPO, TPO, G-CSF, GM-CSF, IL-3, IL-2, IL-4, IL-7, IL-13, IL-6, LIF, IL-12, IFNγ, TNFα, IL-1 and/or M-CSF.

A particularly preferred cytokine is IL-6.

Even yet another aspect of the present invention provides a protein or derivative, homologue, analogue or mimetic thereof exhibiting the following characteristics:

(i) is capable of modulating signal transduction such as cytokine-mediated signal transduction;
(ii) comprises a SOCS box in its C-terminal region having the amino acid sequence:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20}$$
$$X_{21} X_{22} X_{23} [Xj]_n X_{24} X_{25} X_{26} X_{27} X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino, acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F;
$X_{28}$ is L, I, V, M, A or P; and

(iii) comprises at least one of a SH2 domain, WD-40 repeats and/or ankyrin repeats or other protein-molecule interacting domain in a region N-terminal of the SOCS box.

Particularly preferred SOCS proteins comprise an amino acid sequence substantially as set forth in SEQ ID NO:4 (mSOCS1), SEQ ID NO:6 (mSOCS2), SEQ ID NO:8.(mSOCS3), SEQ ID NO:10 (hSOCS1), SEQ ID NO:12 (rSOCS1), SEQ ID NO:14 (mSOCS4), SEQ ID NO:18 (mSOCS5), SEQ ID NO:21 (mSOCS6), SEQ ID NO:25 (mSOCS7), SEQ ID NO:29 (mSOCS8), SEQ ID NO:36 (hSOCS11), SEQ ID NO:41 (mSOCS13), SEQ ID NO:44 (mSOCS14), SEQ ID NO:46 (mSOCS15) and SEQ ID NO:48 (hSOCS15) or an amino acid sequence having at least 15% similarity to all or a region of any one of the listed sequences.

Another aspect of the present invention contemplates a method of modulating levels of a SOCS protein in a cell said method comprising contacting a cell containing a SOCS gene with an effective amount of a modulator of SOCS gene expression or SOCS protein activity for a time and under conditions sufficient to modulate levels of said SOCS protein.

A related aspect of the present invention provides a method of modulating signal transduction in a cell containing a SOCS gene comprising contacting said cell with an effective amount of a modulator of SOCS gene expression or SOCS protein activity for a time sufficient to modulate signal transduction.

Yet a further related aspect of the present invention is directed to a method of influencing interaction between cells wherein at least one cell carries a SOCS gene, said method comprising contacting the cell carrying the SOCS gene with an effective amount of a modulator of SOCS gene expression or SOCS protein activity for a time sufficient to modulate signal transduction.

In accordance with the present invention, n in $[X_i]_n$ and $[X_j]_n$ may, in addition from being 1-50, be from 1-30, 1-20, 1-10 and 1-5.

A summary of the SEQ ID NOs referred to in the subject specification is given in Table 1.

TABLE 1

**SUMMARY OF SEQUENCE IDENTITY NUMBERS**

| **SEQUENCE** | **SEQ ID NO.** |
|---|---|
| PCR Primer | 1 |
| PCR Primer | 2 |
| Mouse SOCS1 (nucleotide) | 3 |
| Mouse SOCS1 (amino acid) | 4 |
| Mouse SOCS2 (nucleotide) | 5 |
| Mouse SOCS2 (amino acid) | 6 |
| Mouse SOCS3 (nucleotide) | 7 |
| Mouse SOCS3 (amino acid) | 8 |
| Human SOCS1 (nucleotide) | 9 |
| Human SOCS1 (amino acid) | 10 |
| Rat SOCS1 (nucleotide) | 11 |
| Rat SOCS1 (amino acid) | 12 |
| nucleotide sequence of murine SOCS4 | 13 |
| amino acid sequence of murine SOCS4 | 14 |
| nucleotide sequence of SOCS4 cDNA human contig 4.1 | 15 |
| nucleotide sequence of SOCS4 cDNA human contig 4.2 | 16 |
| nucleotide sequence of murine SOCS5 | 17 |
| amino acid sequence of murine SOCS5 | 18 |
| nucleotide sequence of human SOCS5 | 19 |
| nucleotide sequence of murine SOCS6 | 20 |
| amino acid of murine SOCS6 | 21 |
| nucleotide sequence of human SOCS6 contig h6.1 | 22 |
| nucleotide sequence of human SOCS6 contig h6.2 | 23 |
| nucleotide sequence of murine SOCS7 | 24 |
| amino acid sequence of murine SOCS7 | 25 |
| nucleotide sequence of human SOCS7 contig h7.1 | 26 |
| nucleotide sequence of human SOCS7 contig 17.2 | 27 |
| nucleotide sequence of murine SOCS8 | 28 |
| amino acid sequence of murine SOCS8 | 29 |
| nucleotide sequence of murine SOCS9 | 30 |
| nucleotide sequence of human SOCS9 | 31 |
| nucleotide sequence of murine SOCS10 | 32 |
| nucleotide sequence of human SOCS10 contig h10.1 | 33 |
| nucleotide sequence of human SOCS10 contig h10.2 | 34 |
| nucleotide sequence of human SOCS11 | 35 |
| amino acid sequence of human SOCS11 | 36 |
| nucleotide sequence of mouse SOCS12 | 37 |
| nucleotide sequence of human SOCS12 contig h12.1 | 38 |
| nucleotide sequence of human SOCS12 contig h12.2 | 39 |
| nucleotide sequence of murine SOCS13 | 40 |

(continued)

**SUMMARY OF SEQUENCE IDENTITY NUMBERS**

| SEQUENCE | SEQ ID NO. |
|---|---|
| amino acid sequence of murine SOCS13 | 41 |
| nucleotide sequence of human SOCS13 cDNA contig h13.1 | 42 |
| nucleotide sequence of murine SOCS14 cDNA | 43 |
| amino acid sequence of murine SOCS14 | 44 |
| nucleotide sequence of murine SOCS15 cDNA | 45 |
| amino acid sequence of murine SOCS15 | 46 |
| nucleotide sequence of human SOCS15 | 47 |
| amino acid sequence of human SOCS15 | 48 |

Single and three letter abbreviations are used to denote amino acid residues and these are summarized in Table 2.

**TABLE 2**

| Amino Acid | Three-letter Abbreviation | One-letter Symbol |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any residue | Xaa | X |

## BRIEF DESCRIPTION OF THE DRAWINGS

In some of the Figures, abbreviations are used to denote SOCS proteins with certain binding motifs. SOCS proteins which contain WD-40 repeats are referred to as WSB1-WSB4. SOCS proteins with ankyrin repeats are referred to as ASB1-ASB3.

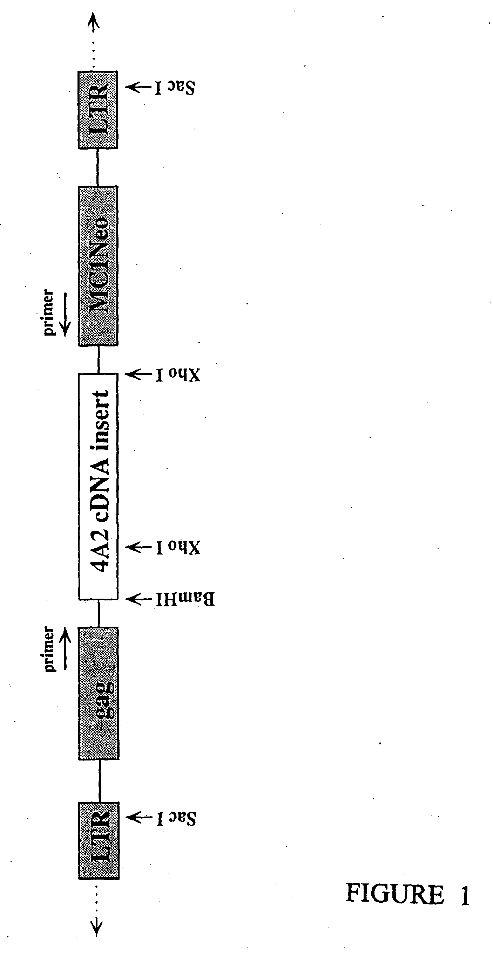

**Figure 1** is a diagrammatic representation showing generation of an IL-6-unresponsive M1 clone by retroviral infection. The RUFneo retrovirus, showing the position of landmark restriction endonuclease cleavage sites, the 4A2 cDNA insert and the position of PCR primer sequences.

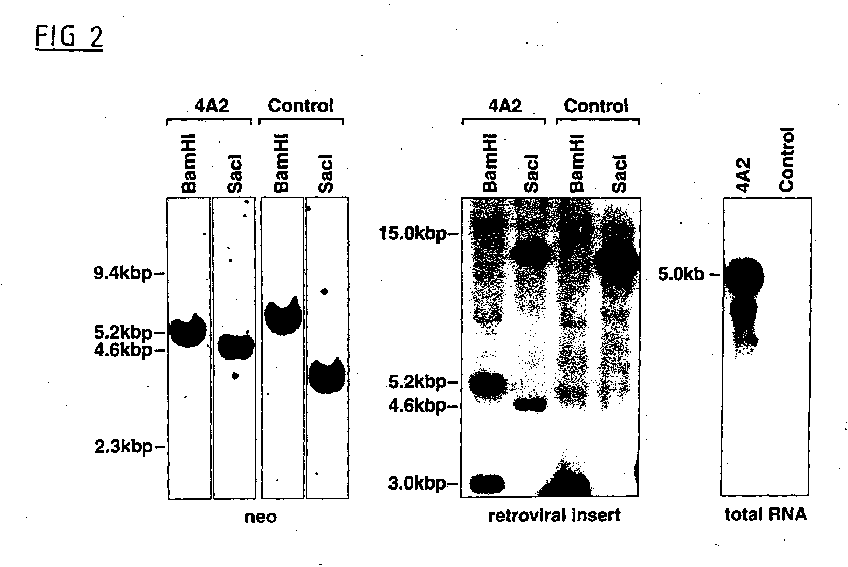

**Figure 2** is a photographic representation of Southern and Northern analysis. (Left and Middle Panels) Southern blot analysis of genomic DNA from clone 4A2 and a control infected M1 clone. DNA was digested with BamH I, to reveal the number of retroviruses carried by each clone, and Sac I, to estimate the size of the retroviral cDNA insert. Left panel; probed with neo. Right panel; probed with the Xho I-digested 4A2 PCR product. (Right Panel). Northern blot analysis of total RNA from clone 4A2 and a control infected M1 clone, probed with the Xho I-digested 4A2 PCR product. The two bands represent unspliced and spliced retroviral transcripts, resulting from splice donor and acceptor sites in the retroviral genome.

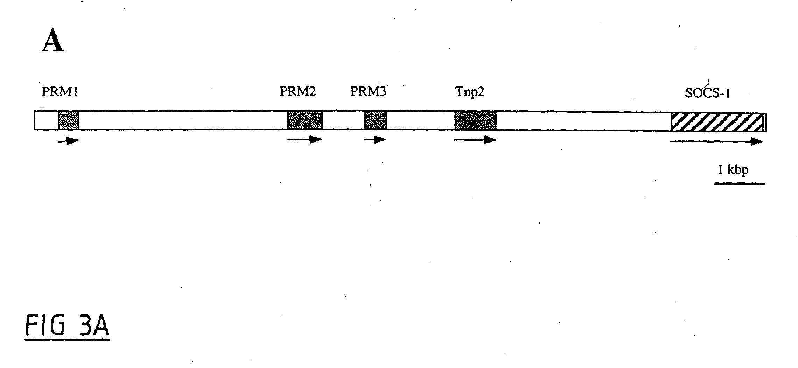

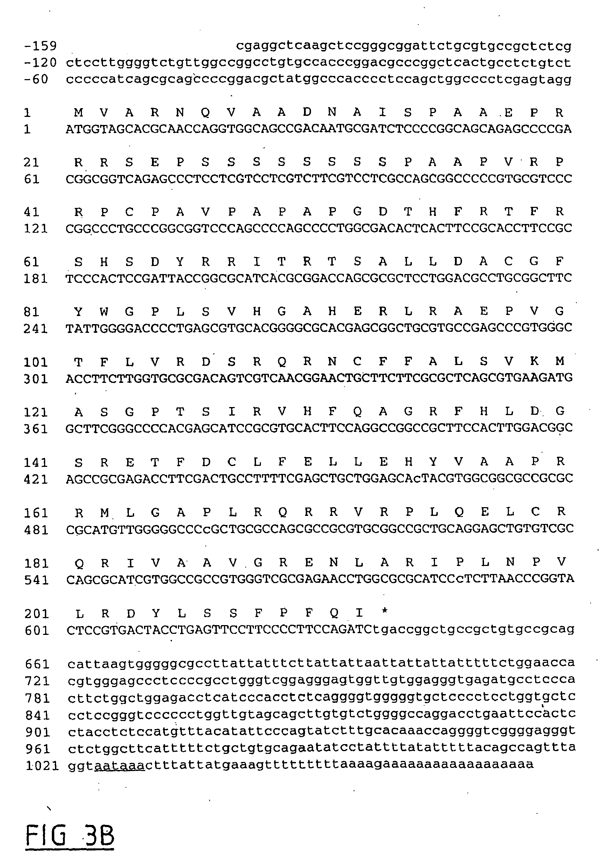

**Figure 3** is a representation of the nucleotide sequence and structure of the SOCS1 gene. A. The genomic context of SOCS1 in relation to the protamine gene cluster on murine chromosome 16. The accession number of this locus is MMPRMGNS (direct submission; G. Schlueter, 1995) for the mouse and BTPRMTNP2 for the rat (direct submission; G. Schlueter, 1996). B. The nucleotide sequence of the sacs cDNA and deduced amino acid sequence. Conventional one letter abbreviations are used for the amino acid sequence and the asterisk indicates the stop codon. The polyadenylation signal sequence is underlined. The coding region is shown in uppercase and the untranslated region is shown in lower case.

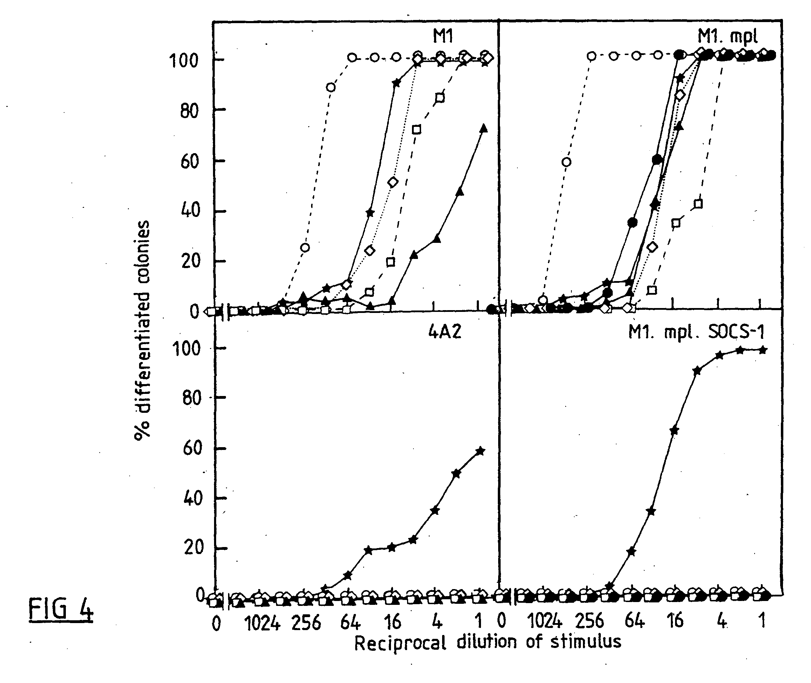

**Figure 4** is a graphical representation of cell differentiation in the presence of cytokines. Semi-solid agar cultures of parental M1 cells (M1 and M1.mpl) and M1 cells expressing SOCS1 (4A2 and M1.mpl.SOCS1), were used and the percentage of colonies which differentiated in response to a titration of 1 mg/ml IL-6 (●), 100 ng/ml LIF (◇), 1 mg/ml OSM (D), 100 ng/ml IFN-γ (▲), 500 ng/ml TPO (●), or $3x10^{-6}$ M dexamethasone (*) determined.

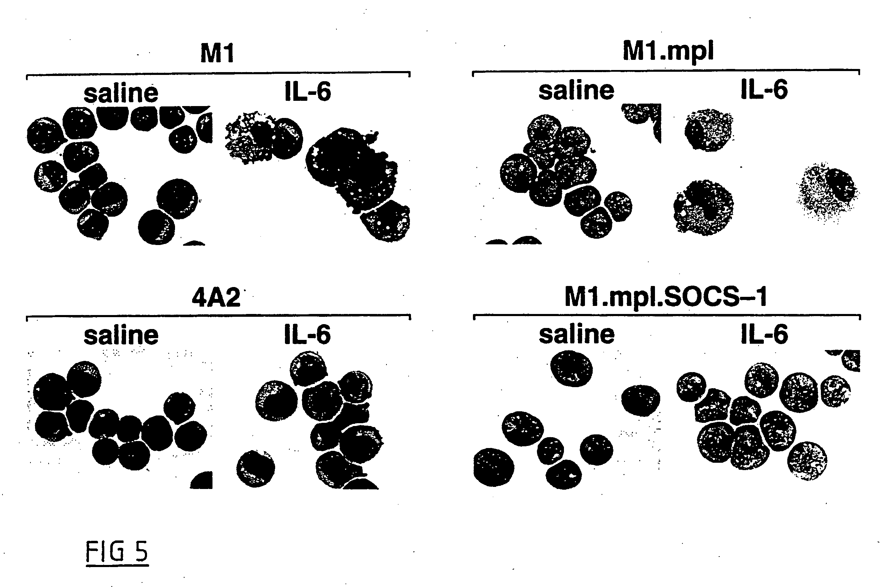

**Figure 5** is a photographic representation of cytospins of liquid cultures of parental M1 cells (M1 and M1.mpl) ) and M1 cells expressing SOCS1 (4A2 and M1.mpl.SOCS1) cultured for 4 days in the presence of 10 ng/ml IL-6 or saline. Unlike parental M1 cells, morphological features consistent with macrophage differentiation are not observed in M1 cells constitutively expressing SOCS1 (4A2 and M1.mpl.SOCS1) when cultured in IL-6.

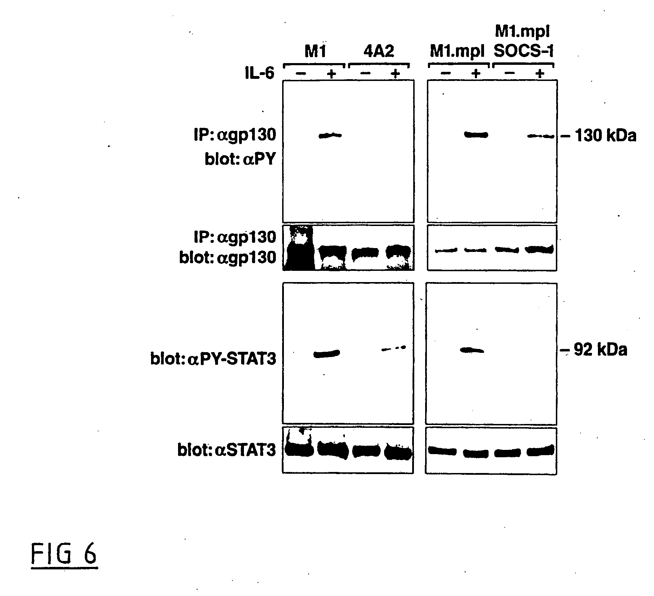

**Figure 6** is a photographic representation showing inhibition of phosphorylation of signalling molecules by SOCS1. Parental M1 cells (M1 and M1.mpl) and M1 cells expressing SOCS1 (4A2 and M1.mpl.SOCS1) were incubated in the absence (-) or presence (+) of 10 ng/ml of IL-6 for 4 minutes at 37°C. Cells were then lysed and extracts were either immunopreciptated using anti-mouse gp130 antibody prior to SDS-PAGE (two upper panels) or were electrophoresed directly (two lower panels). Gels were blotted and the filters were then probed with anti-phosphotyrosine (upper panel), anti-gp130 antibody (second top panel), anti-phospho-STAT3 (second bottom panel) or anti-STAT3 (lower panel). Blots were visualised using peroxidase-conjugated secondary antibodies and Enhanced Chemiluminescence (ECL) reagents.

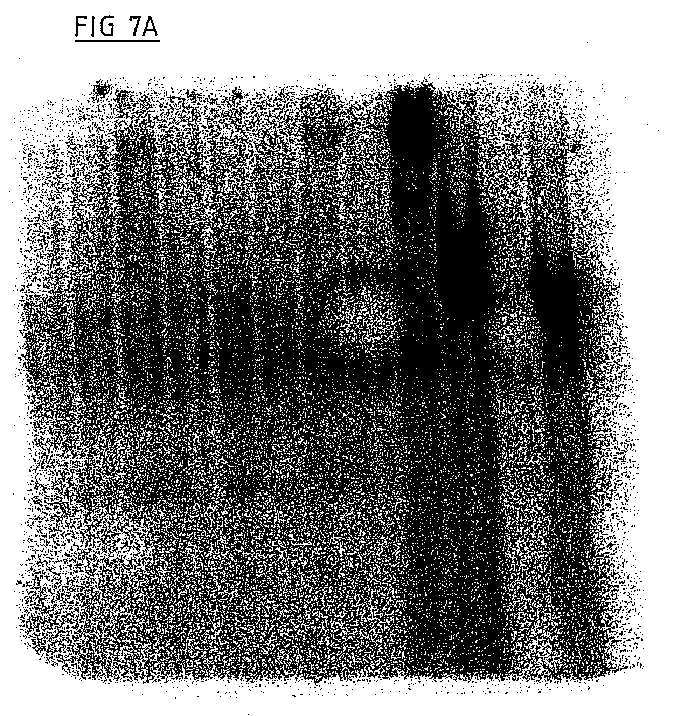

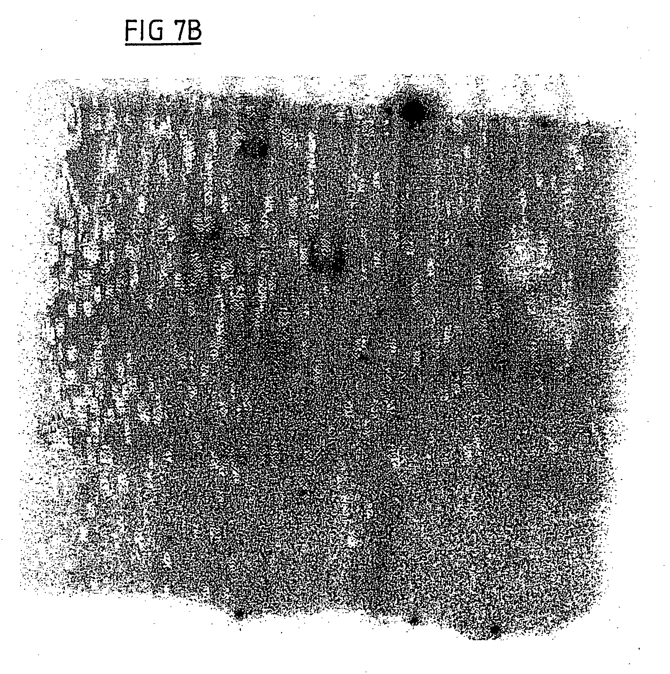

**Figure 7** is a representation of protein extracts prepared from (A) M1 cells or M1 cells expressing SOCS1 (4A2) and (B) M1.mpl cells or M1.mpl.SOCS1 cells incubated for 10 min at 37°C in 10 ml serum-free DME containing either saline, 100 ng/ml IL-6 or 100 ng/ml IFN-γ. The binding reactions contained 4-6 μg protein (constant within a given experiment), 5 ng $^{32}$P-labelled m67 oligonucleotide encoding the high affinity SIF (c-*sis*-inducible factor) binding site, and 800 ng sonicated salmon sperm DNA. For certain experiments, protein samples were preincubated with an excess of unlabelled m67 oligonucleotide, or antibodies specific for either STAT1 or STAT3.

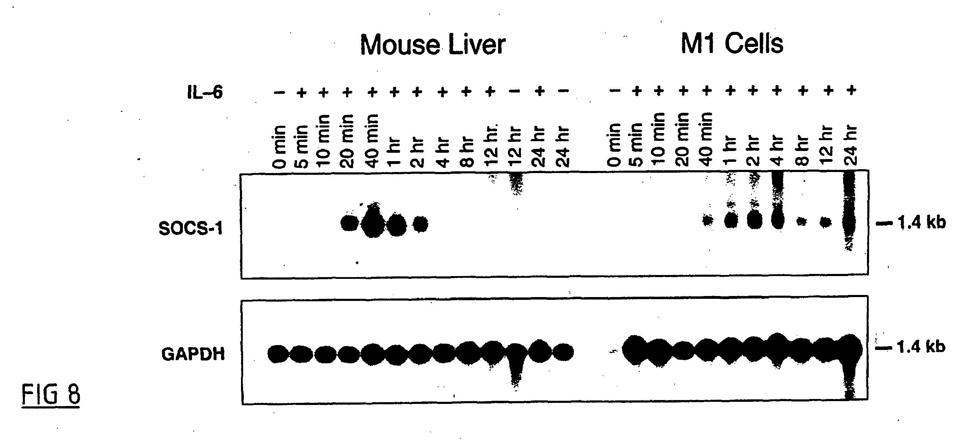

**Figure 8** is a photographic representation of Northern hybridisation. Mice were injected intravenously with 2 μg and after various periods of time, the livers were removed and polyA+ mRNA was purified. M1 cells were stimulated for various lengths of time with 500 ng/ml of IL-6, after which polyA+ mRNA was isolated. mRNA was fractionated by electrophoresis and immobilized on nylon filters. Northern blots were prehybridized, hybridized with random-primed $^{32}$P-labelled SOCS1 or GAPDH DNA fragments, washed and exposed to film overnight.

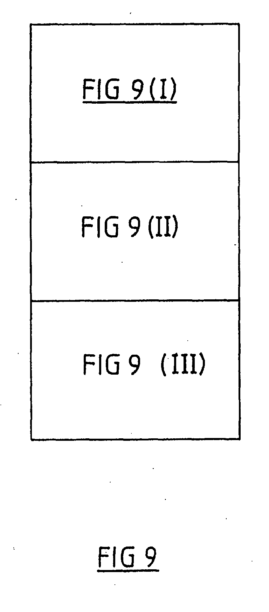

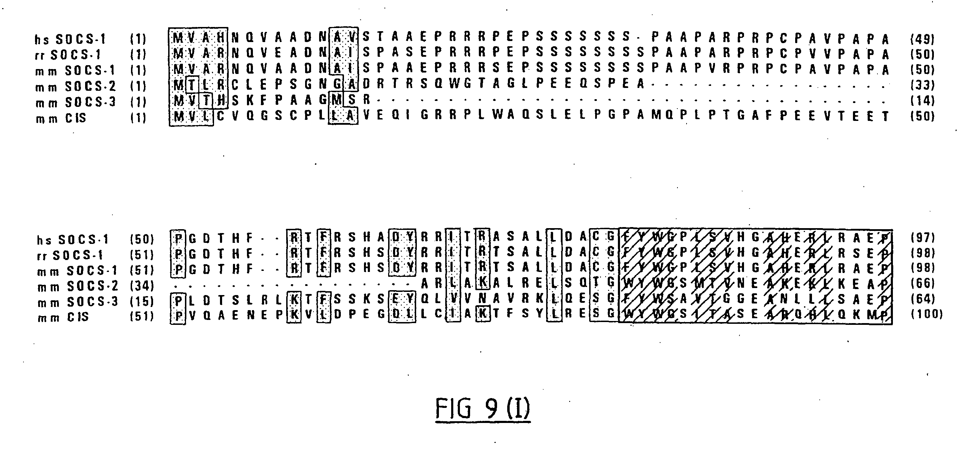

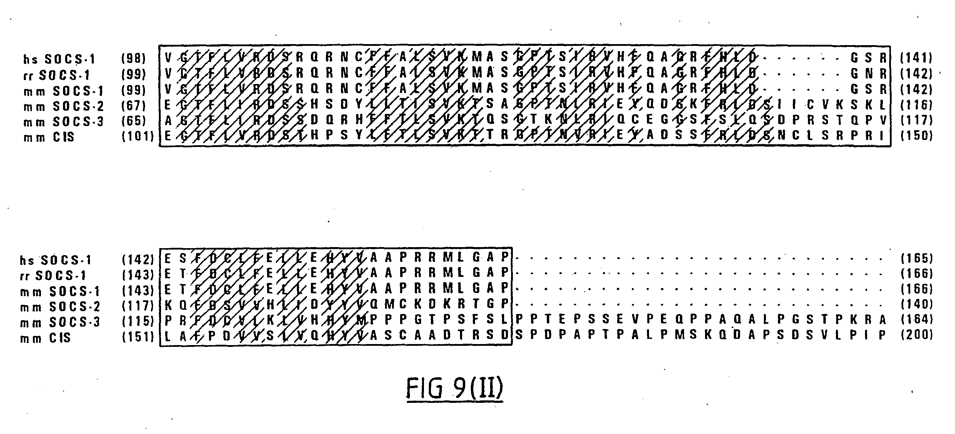

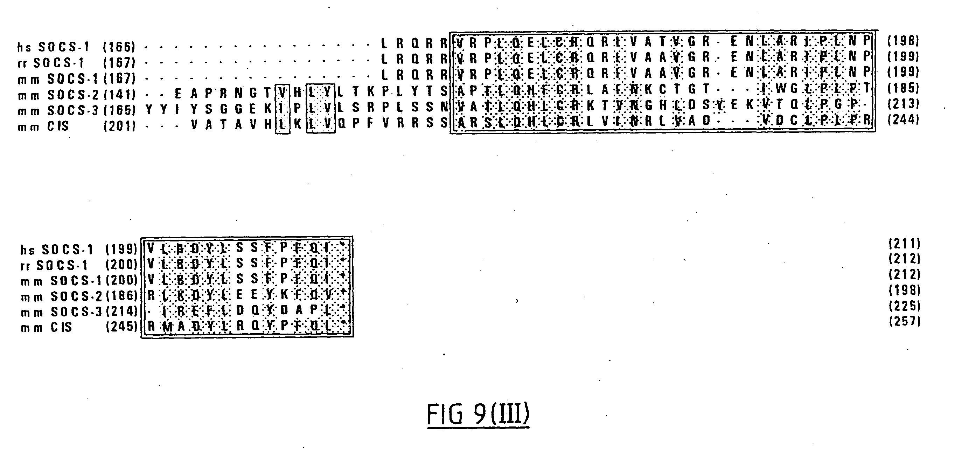

**Figure 9** is a representation of a comparison of the amino acid sequences of SOCS1, SOCS2, SOCS3 and CIS. Alignment of the predicted amino acid sequence of mouse (mm), human (hs) and rat (rr) SOCS1, SOCS2, SOCS3 and CIS. Those residues shaded are conserved in three or four mouse SOCS family members. The SH2 domain is boxed in solid lines, while the SOCS box is bounded by double lines.

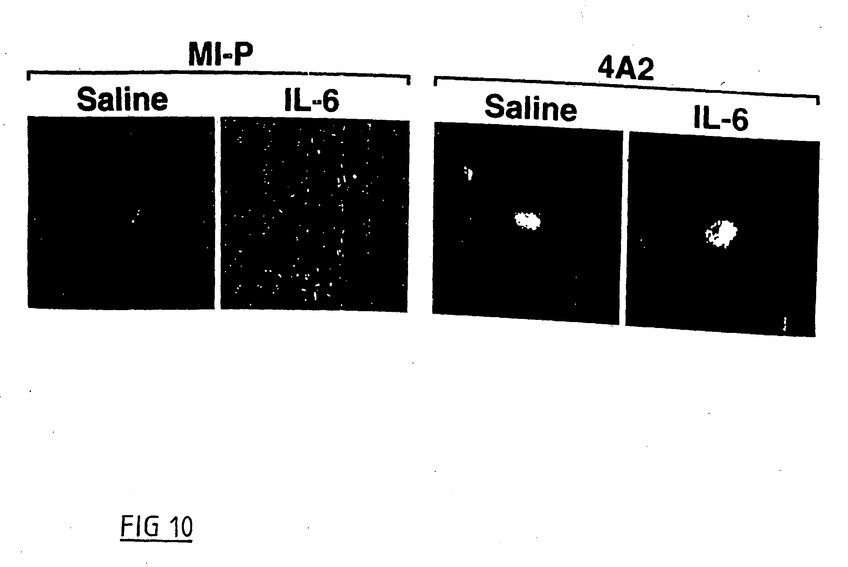

**Figure 10** is a photographic representation showing the phenotype of IL-6 unresponsive M1 cell clone, 4A2. Colonies of parental M1 cells (left panel) and clone 4A2 (right panel) cultured in semi-solid agar for 7 days in saline or 100 ng/ml IL-6.

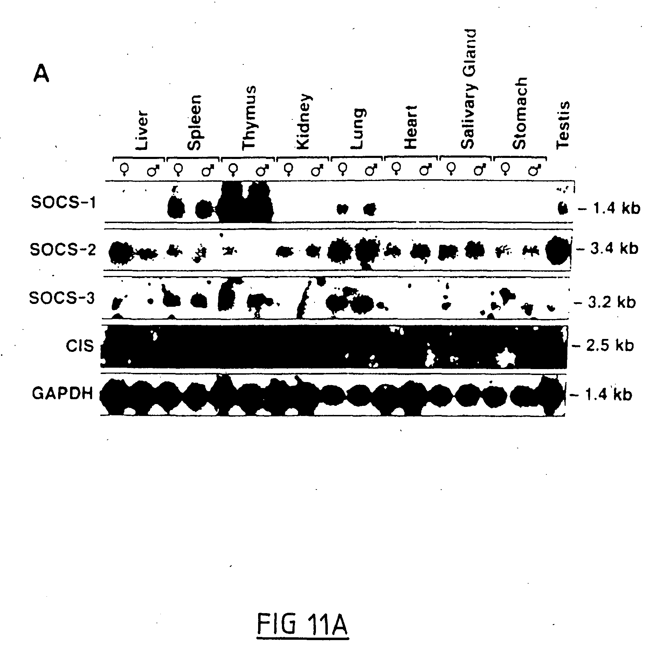

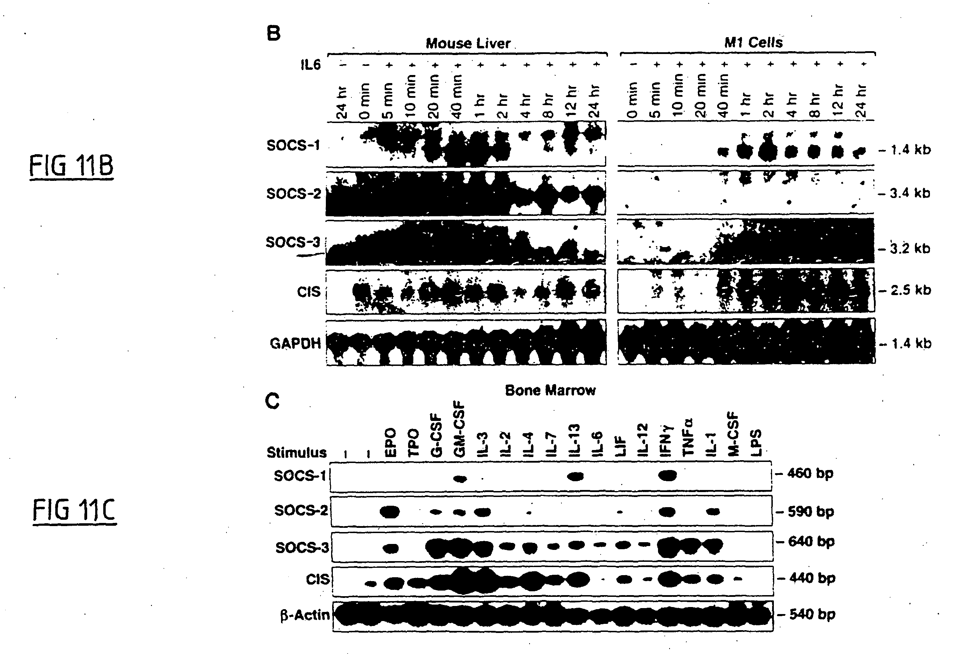

**Figure 11** is a photographic representation showing expression of mRNA for SOCS family members *in vitro* and *in vivo.*

(A) Northern analysis of mRNA from a range of mouse organs showing constitutive expression of SOCS family members in a limited number of tissues.

(B) Norther analysis of mRNA from liver and M1 cells showing induction of expression of SOCS family members following exposure to IL-6.

(C) Reverse transcriptase PCR analysis of mRNA from bone marrow showing induction of expression of SOCS family members by a range of cytokines.

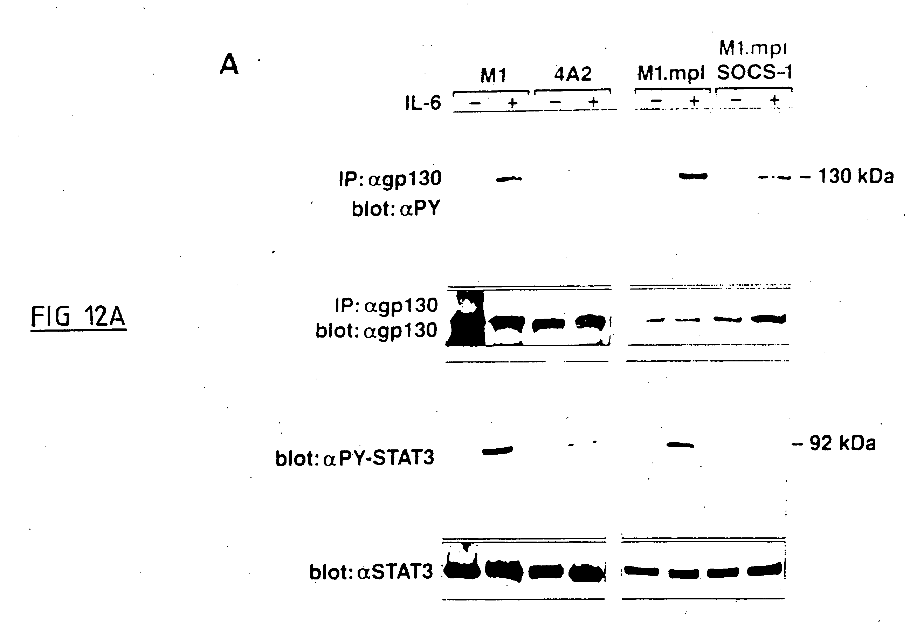

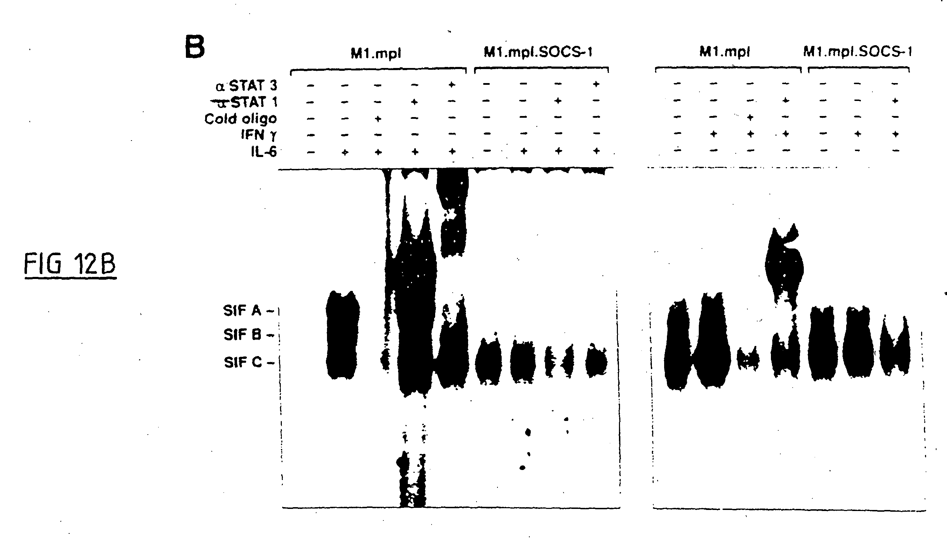

**Figure 12** is a photographic representation showing SOCS1 suppresses the phosphorylation and activation of gp130 and STAT-3.

(A) Western blots of extracts from parental M1 cells (M1 and M1.mpl) and M1 cells expressing SOCS1 (4A2 and M1.mpl.SOCS1) stimulated with (+) or without (-) 100 ng/ml IL-6. Top: Extracts immunoprecipitated with antu-gp130 (αgp130) and immunoblotted with anti-phosphotyrosine (αPY-STAT3), or for STAT3 (αSTAT3) to demonstrate equal loading of protein. The molecular weights of the bands are shown on the right.

(B) EMSA of M1.mpl and M1.mpl.SOCS1 cells stimulated with (+) and without (-) 100 ng/ml IL-6 or 100 ng/ml IFNγ. The DNA-binding complexes SIF A, B, and C are indicated at the left.

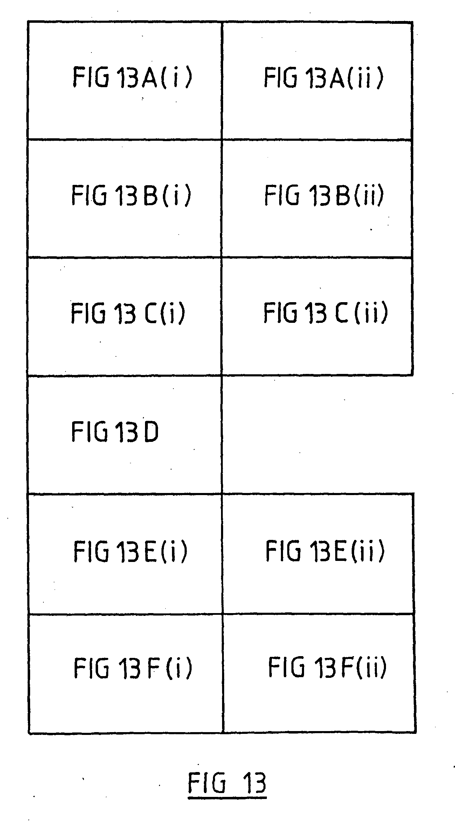

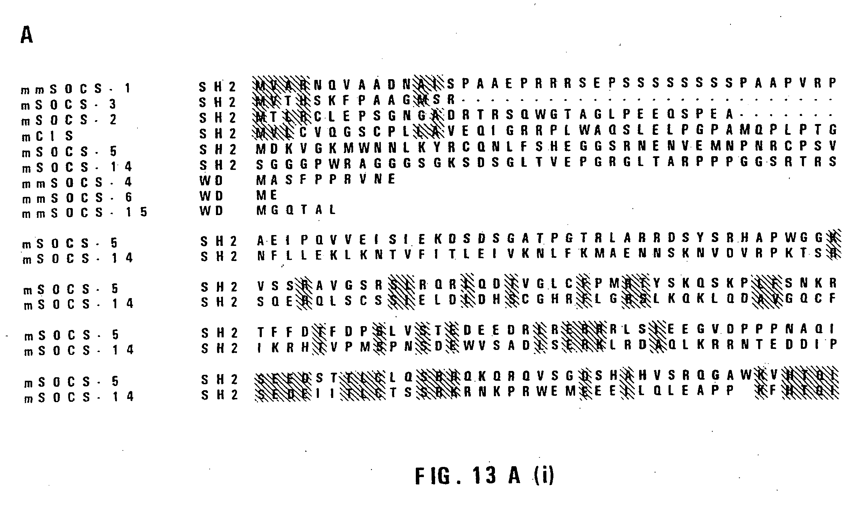

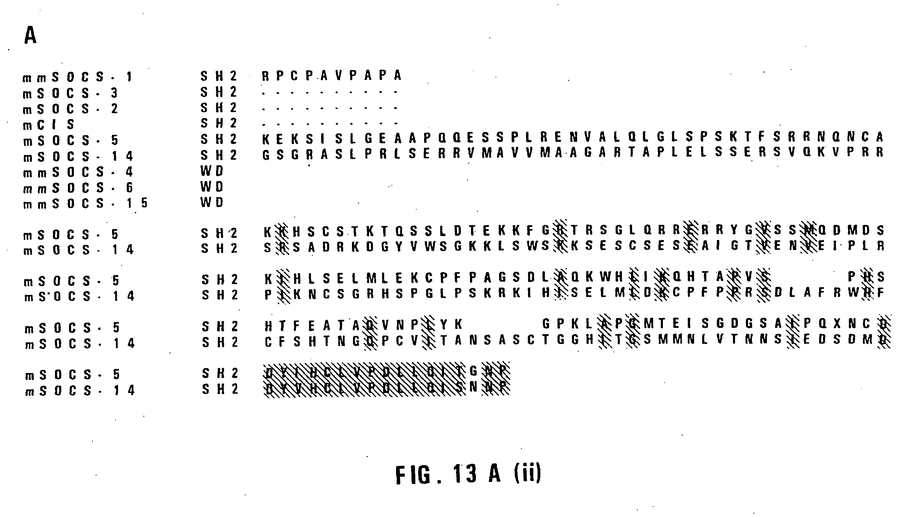

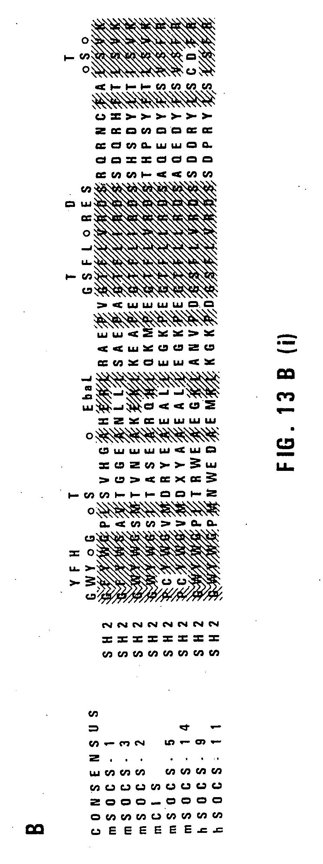

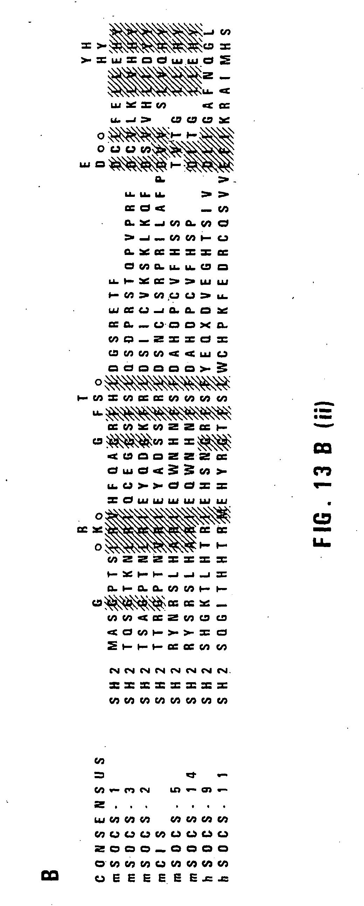

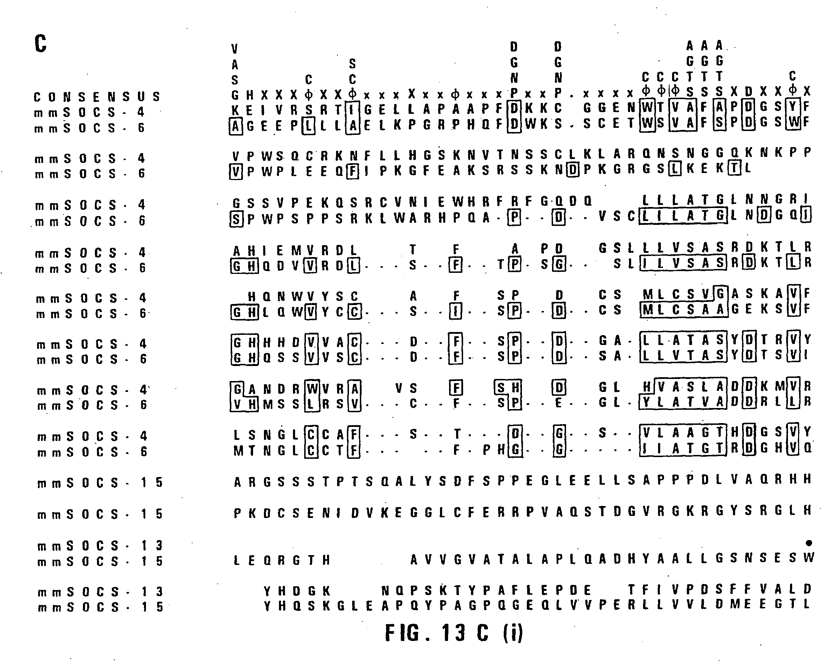

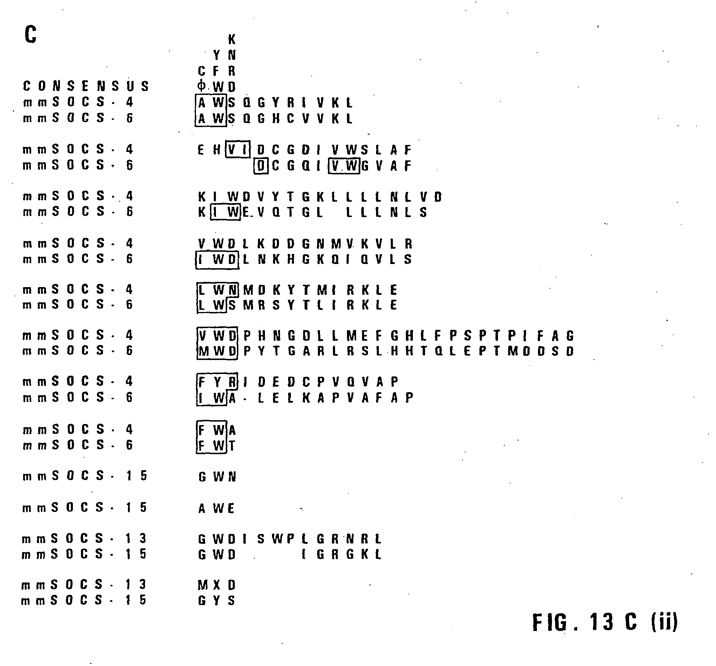

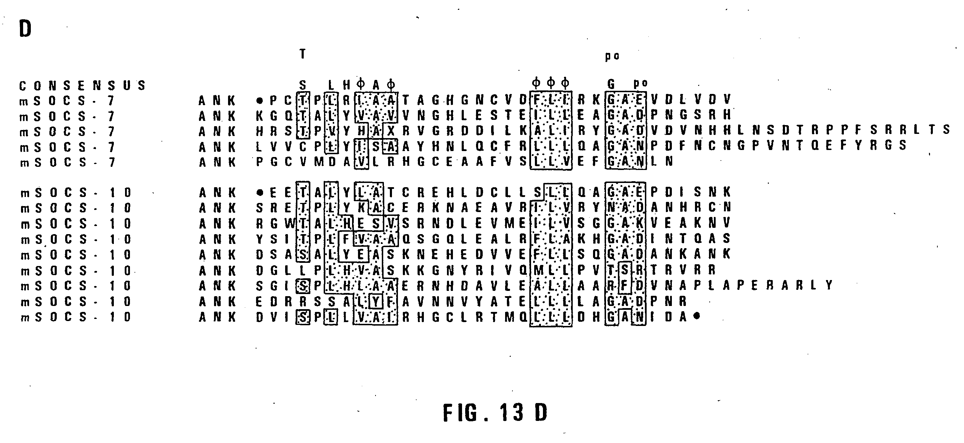

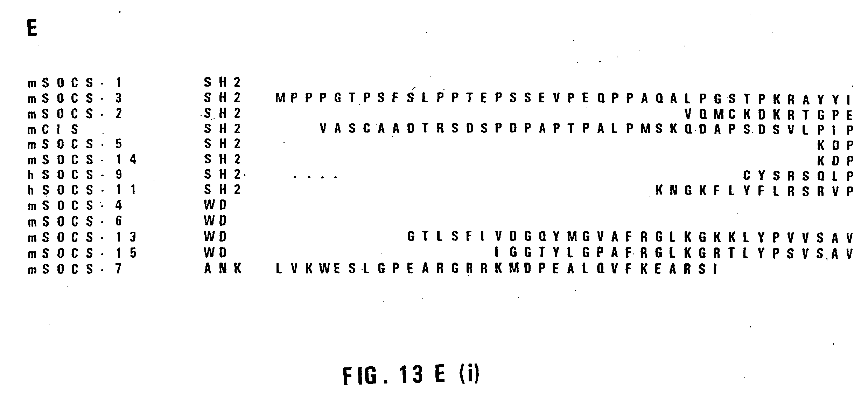

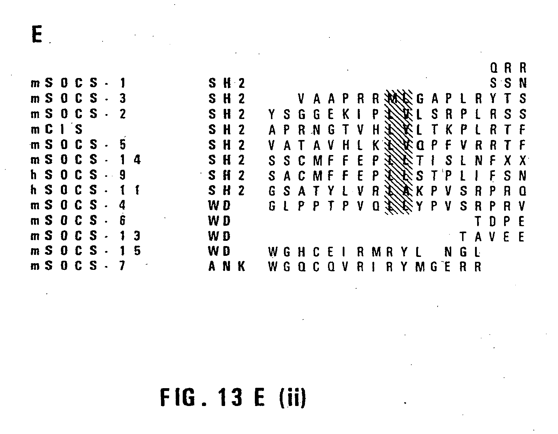

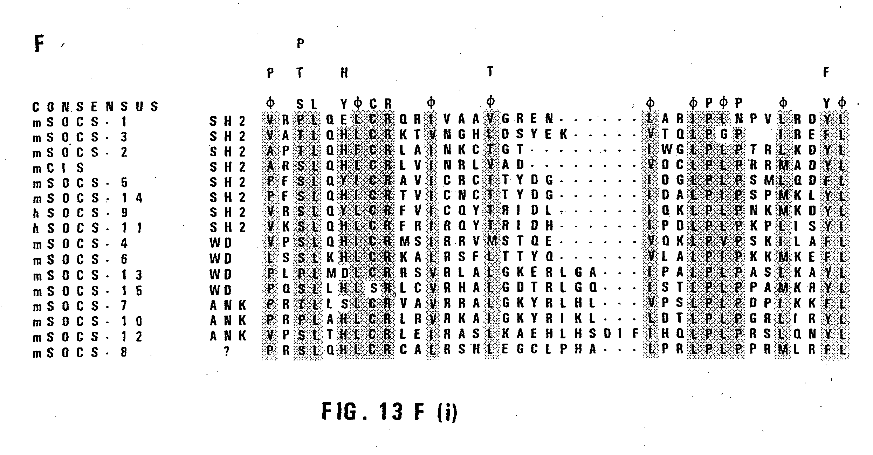

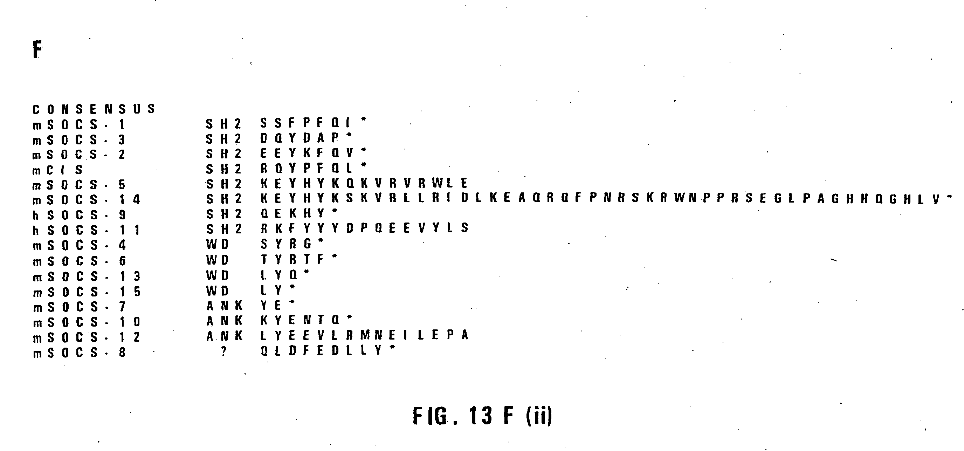

**Figure 13** is a representation of a comparison of the amino acid sequence of the SOCS proteins (A) Schematic representation of structures of SOCS proteins including proteins which contain WD-40 repeats (WSB) and ankyrin repeats (ASB). (B) Alignment of N-terminal regions of SOCS proteins. (C) Alignment of the SH2 domains of CIS, SOCS1, 2, 3, 5, 9, 11 and 14. (D) Alignment of the WD-40 repeats of SOCS4, SOCS6, SOCS13 and SOCS15. (E) Alignment of the ankyrin repeats of SOCS7 and SOCS10. (F) Alignment of the regions between SH2, WD-40 and ankyrin repeats and the SOCS box. (G) Alignment of the SOCS box. In each case the conventional one letter abbreviations for amino acids are used, with X denoting residues of uncertain identity and OOO denoting the beginning and the end of contigs: Amino acid sequence obtained from conceptual translation of nucleic acid sequence derived from isolated cDNAs is shown in upper case while amino acid sequence obtained by conceptual translation of ESTs is shown in lower case and is approximate only. Conserved residues, defined as (LIVMA), (FYW), (DE), (QN), (C, S, T), (KRH), (PG) are shaded in the SH2 domain, WD-40 repeats, ankyrin repeats and the SOCS box. For the alignment of SH2 domains, WD-40 repeats and ankyrin repeats a consensus sequence is shown above. In each case this has been derived from examination of a large and diverse set of domains (Neer *et al,* 1994; Bork, 1993).

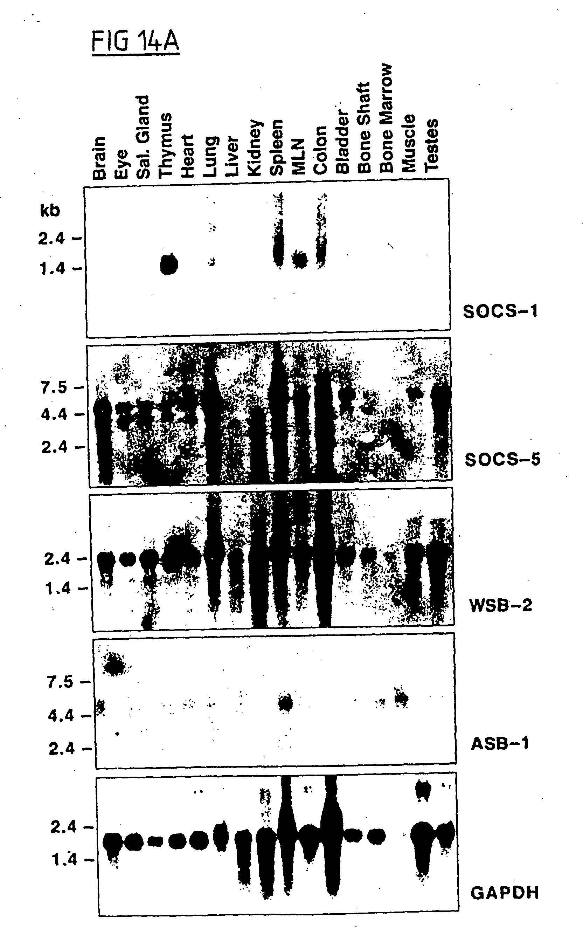

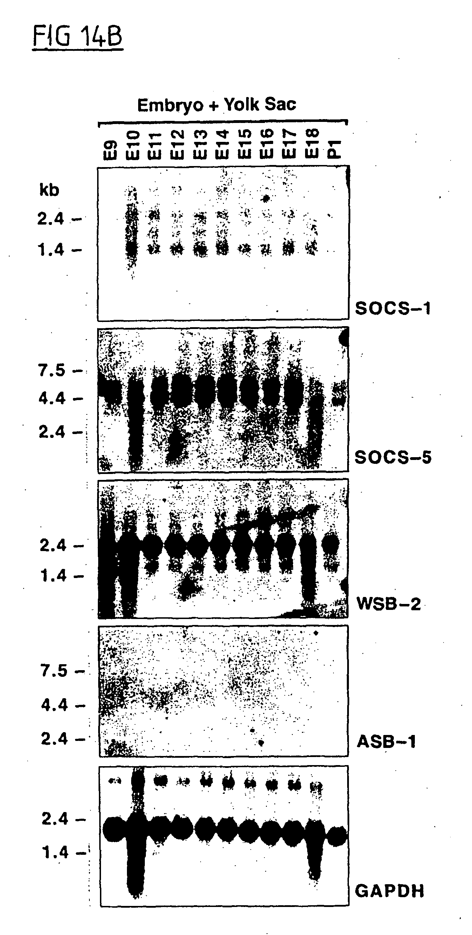

**Figures 14(A) and (B)** are photographic representations showing analysis of mRNA expression of mouse SOCS1 and SOCS5 and SOCS containing a WD-40 repeat (WSB2) and ankyrin repeats (ASB1).

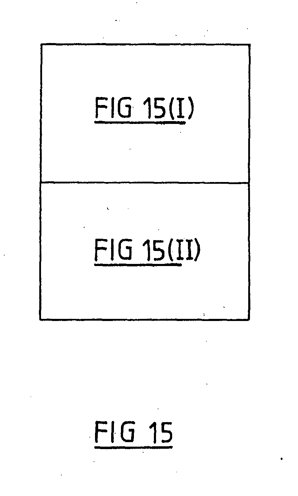

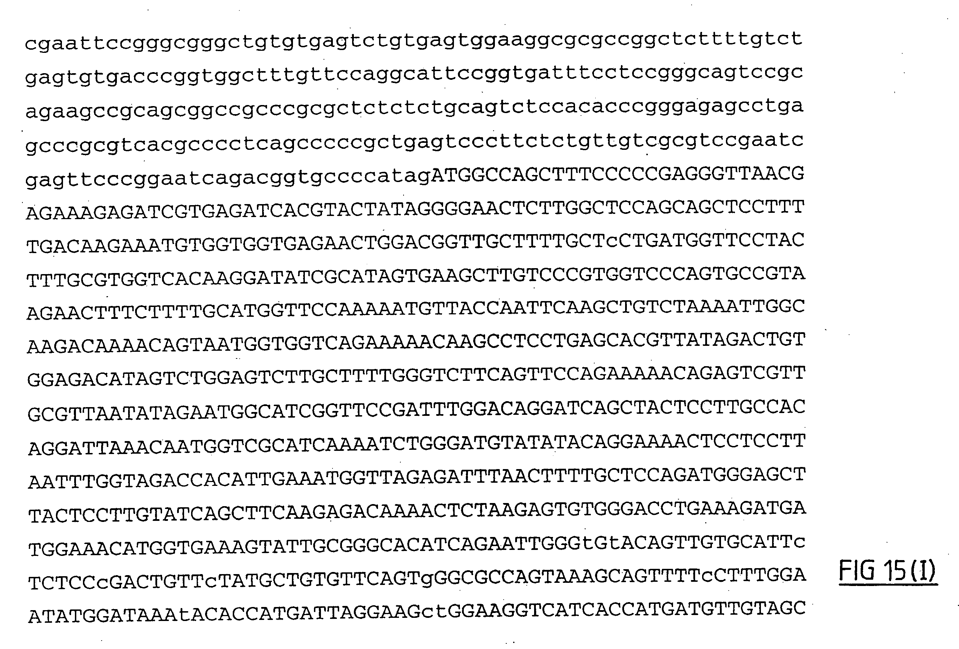

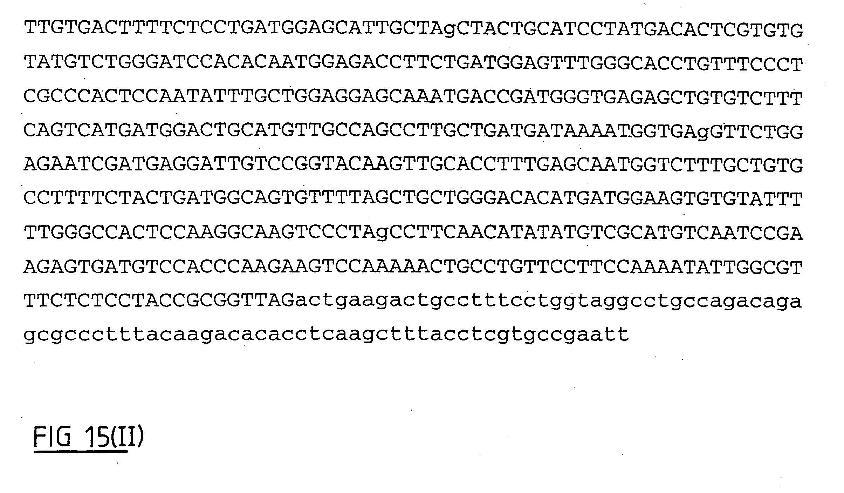

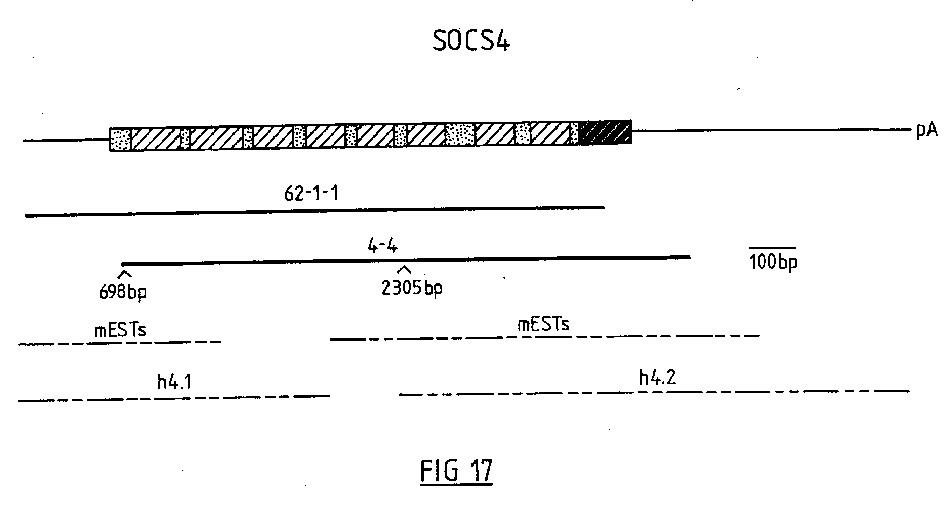

**Figure 15** is a representation showing the nucleotide sequence of the mouse SOCS4 cDNA. The nucleotides encoding the mature coding region from the predicted ATG "start" codon to the stop codon is shown in upper case, while the predicted 5' and 3' untranslated regions are shown in lower case. The relationship of mouse cDNA sequence to mouse and human EST contigs is illustrated in Figure 17.

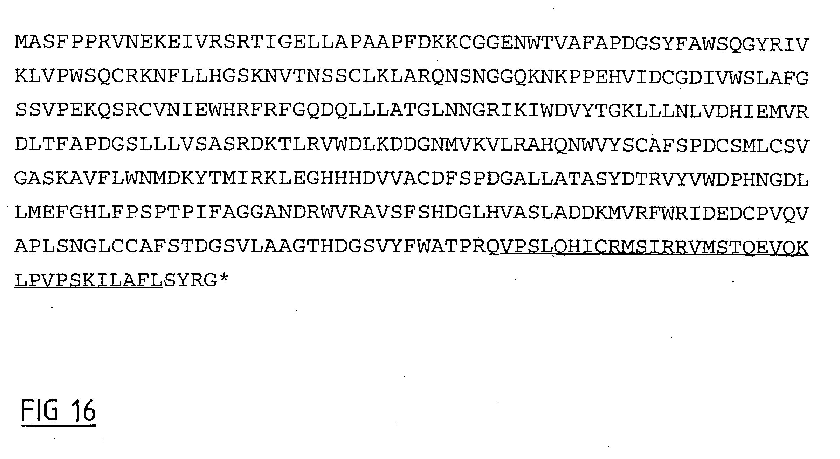

**Figure 16** is a representation showing the predicted amino acid sequence of the mouse SOCS4 protein, derived from the nucleotide sequence in Figure 15. The SOCS box, which also shown in Figure 13, is underlined.

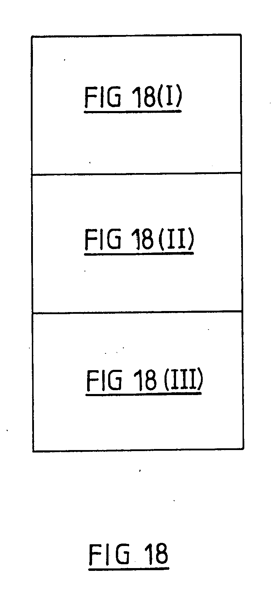

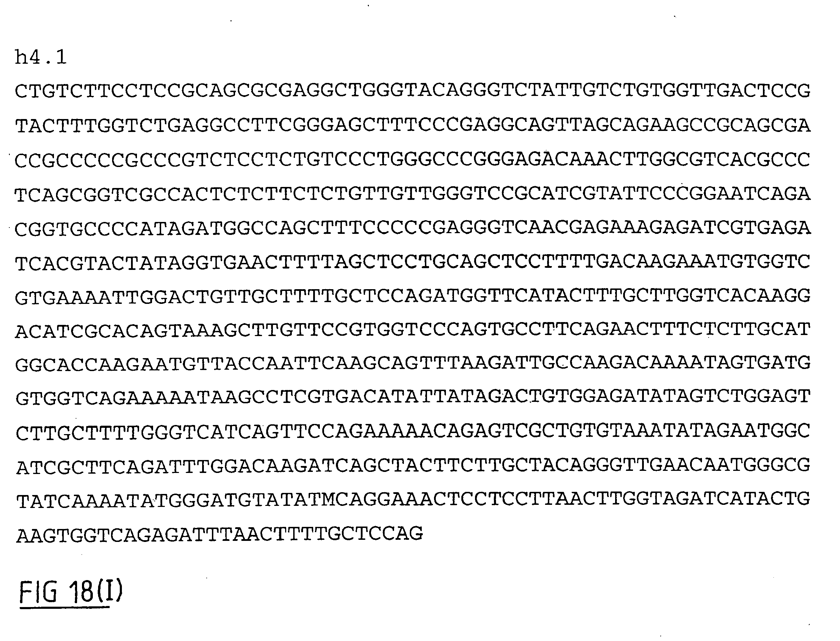

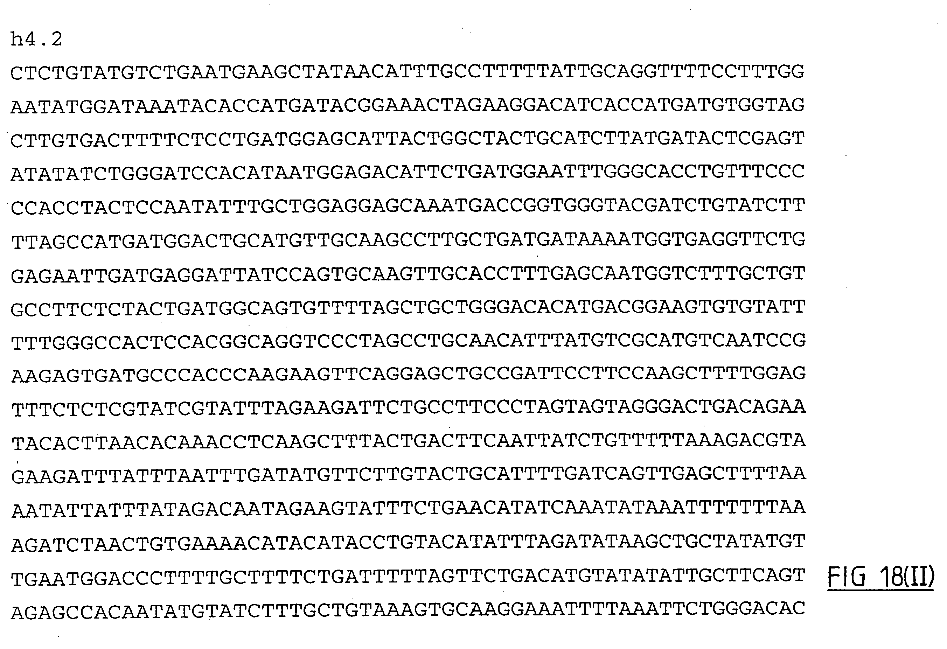

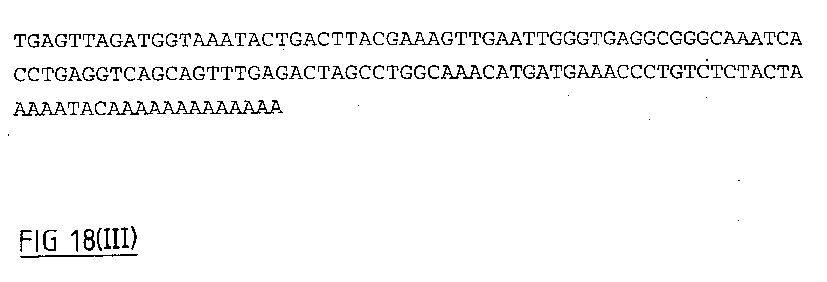

**Figure 18** is a representation showing the nucleotide sequence of human SOCS4 cDNA contigs h4.1 and h4.2, derived from analysis of ESTs listed in Table 4.1. The relationship of these contigs to the mouse cDNA sequence is illustrated in Figure 17.

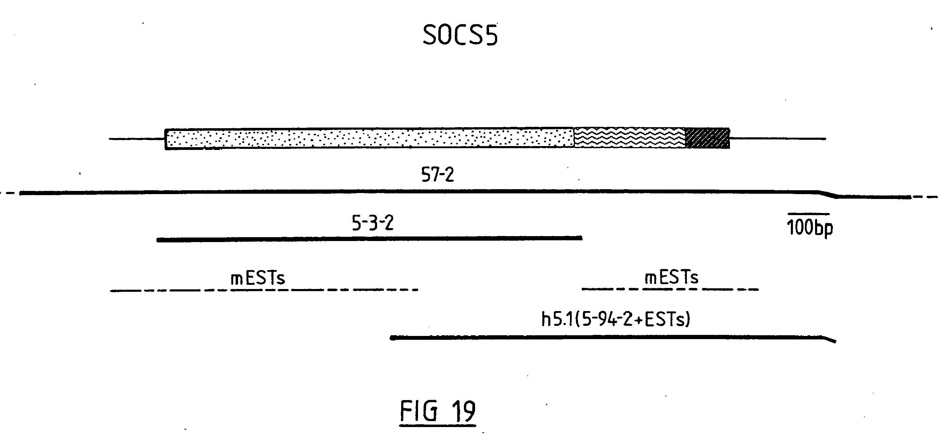

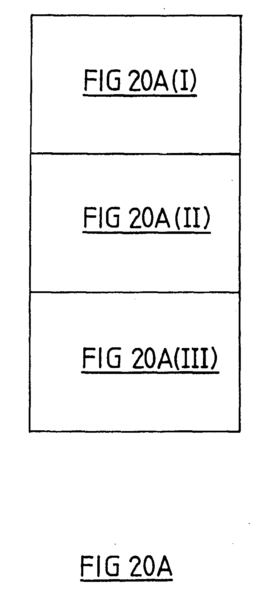

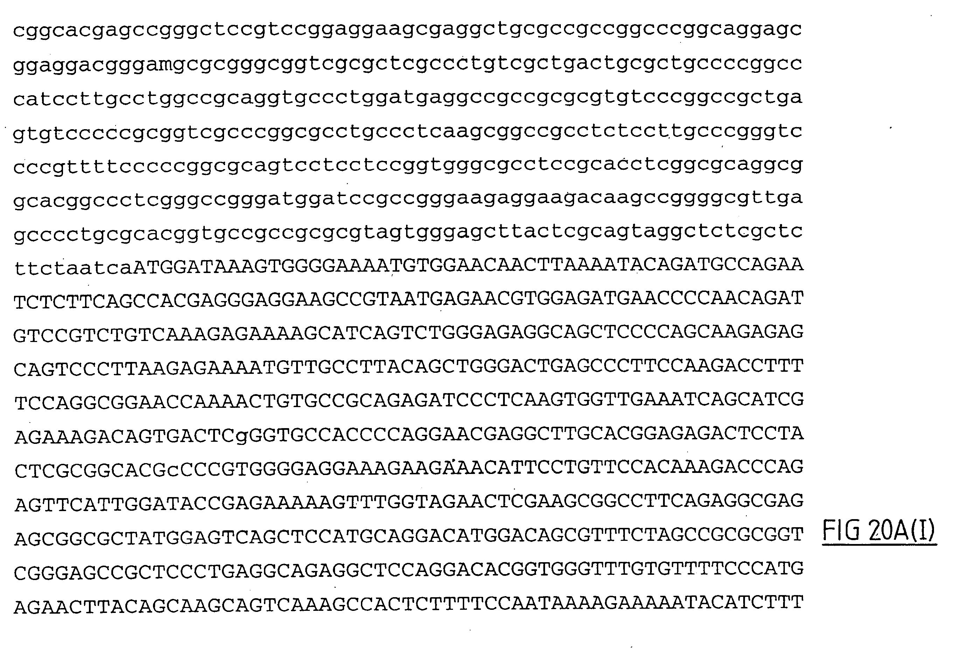

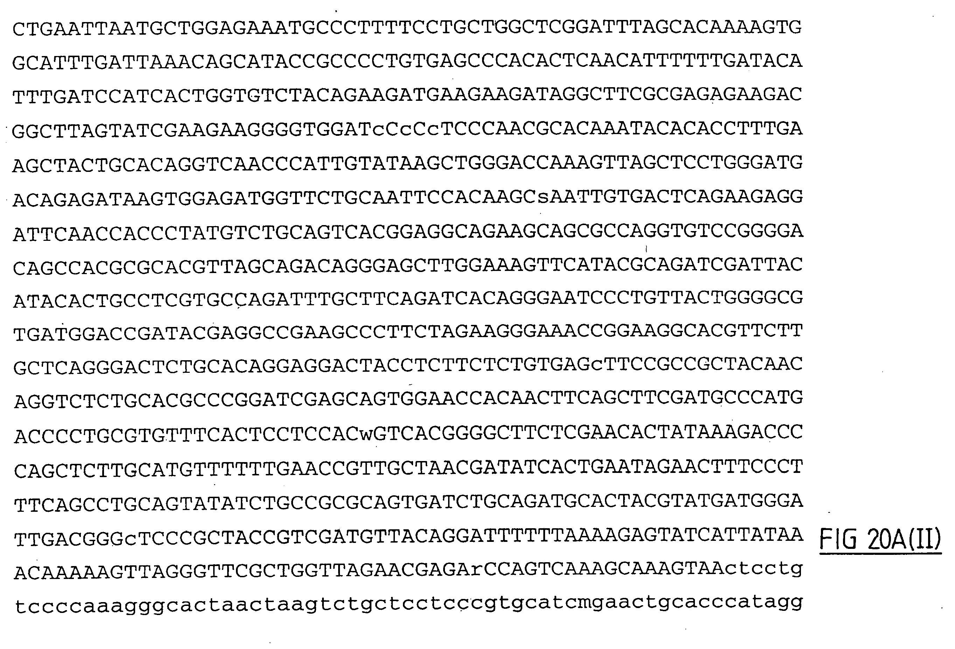

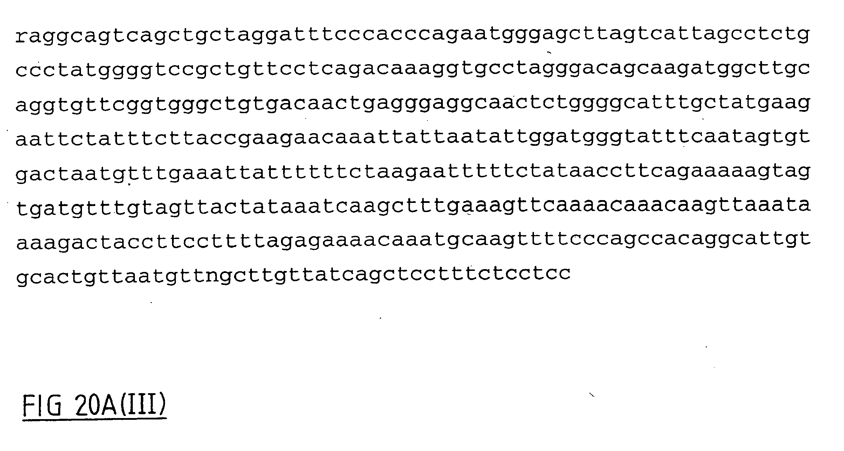

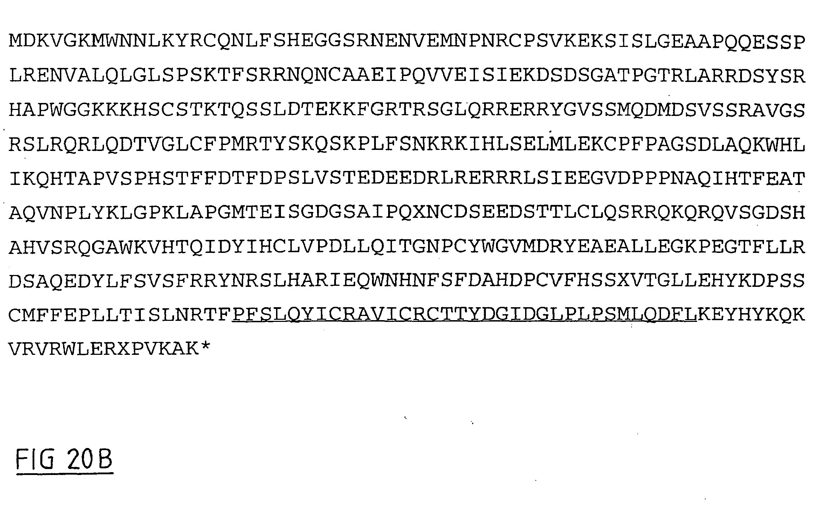

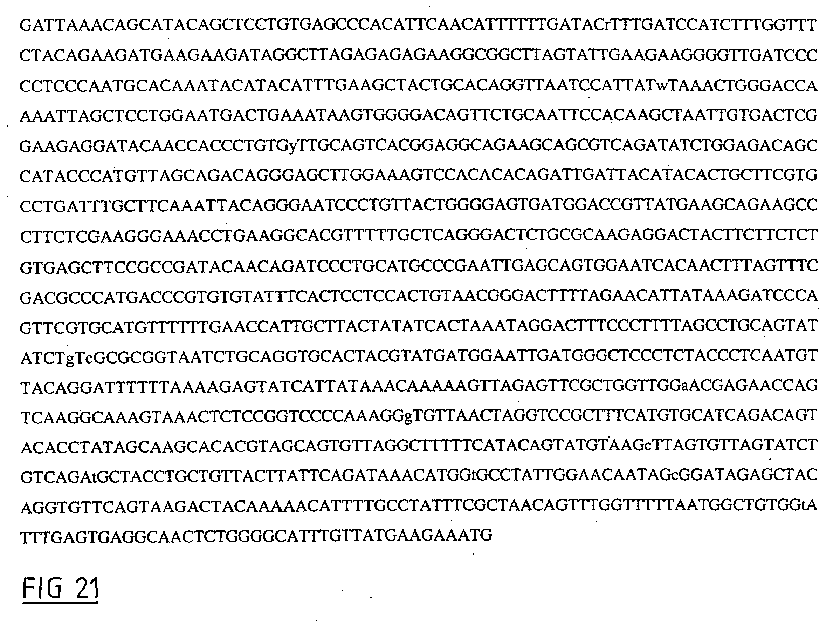

**Figure 19** is a diagrammatic representation showing the relationship of mouse SOCS5 genomic (57-2) and cDNA (5-3-2) clones to contigs derived from analysis of mouse ESTs (Table 5.1) and human cDNA clone (5-94-2) and ESTs (Table 5.2). The nucleotide sequence of the mouse SOCS5 contig is shown in Figure 20, with the sequence of human SOCS5 contig (h5.1) being shown in Figure 21. The deduced amino acid sequence of mouse SOCS5 is shown in Figure 20B. The structure of the protein is shown schematically, with the SH2 domain indicated by ( ) and the SOCS box by ( ). The putative 5' and 3' translated regions are shown by the thin solid line.

**Figure 20A** is a representation showing the nucleotide sequence of the mouse SOCS5 derived from analysis of genomic and cDNA clones. The nucleotides encoding the mature coding region from the predicted ATG "start" codon to the stop codon is shown in upper case, while the predicted 5' and 3' untranslated regions are shown in lower case. The relationship of mouse cDNA sequence to mouse and human EST contigs is illustrated in Figure 19.

**Figure 20B** is a representation of the predicted amino acid sequence of mouse SOCS5 protein, derived from the nucleotide sequence in Figure 20A. The SOCS box, which also shown in Figure 13 is underlined.

**Figure 21** is a representation showing the nucleotide sequence of human SOCS5 cDNA contig h5.1, derived from analysis of cDNA clone 5-94-2 and the ESTs listed in Table 5.2. The relationship of these contigs to the mouse cDNA sequence is illustrated in Figure 19.

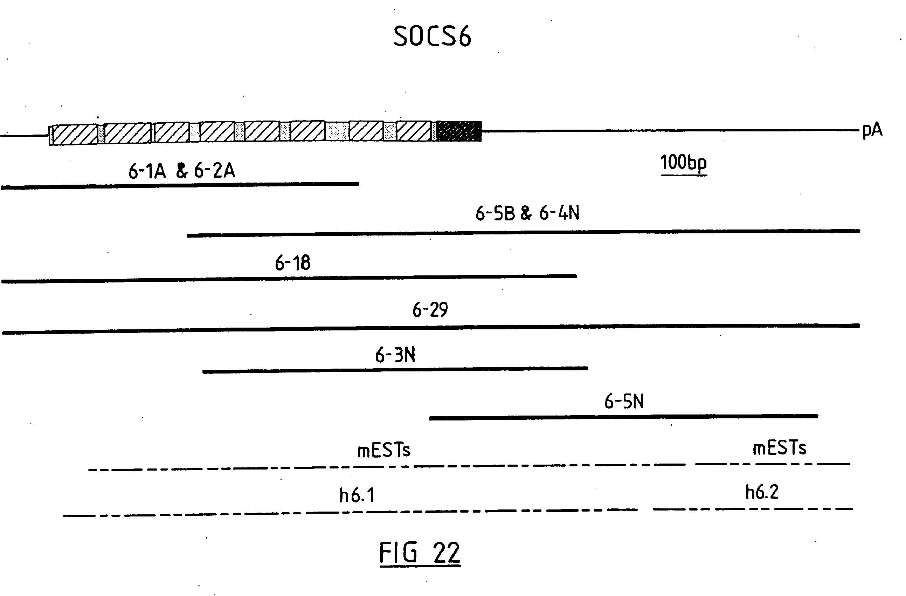

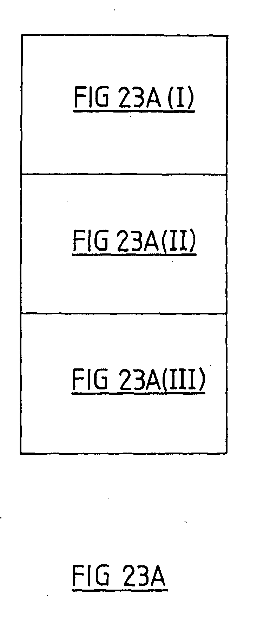

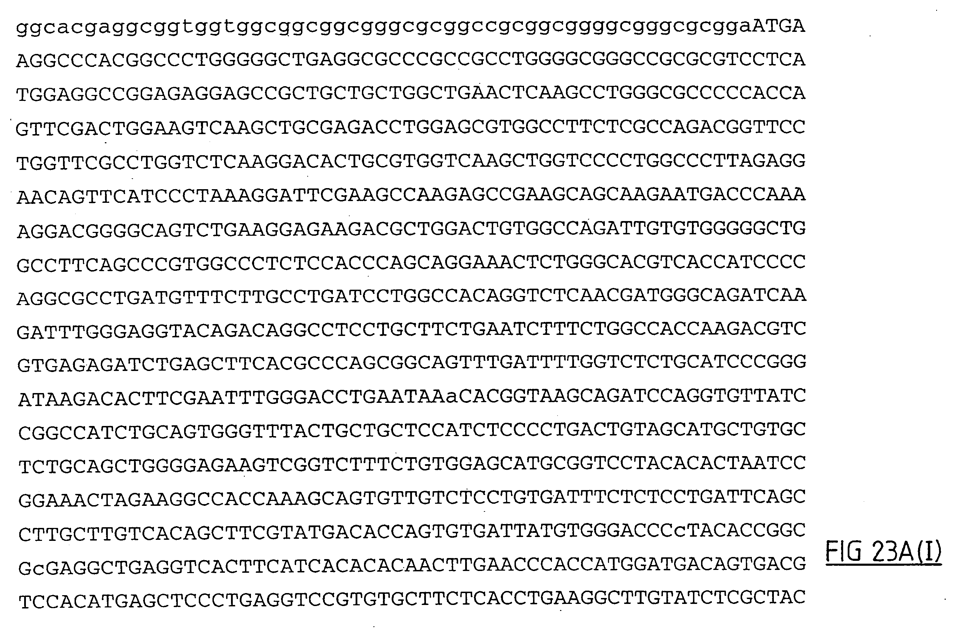

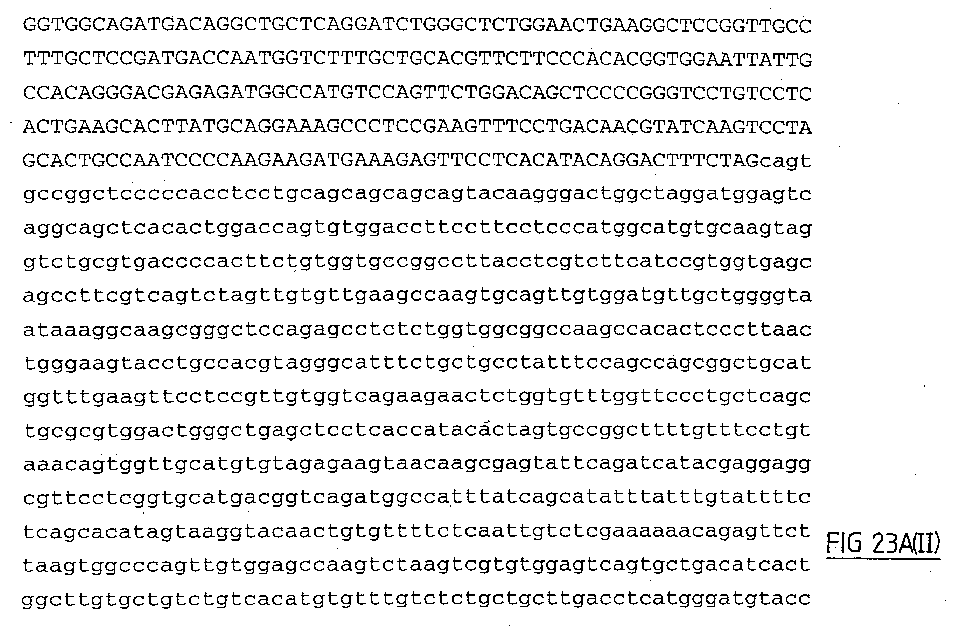

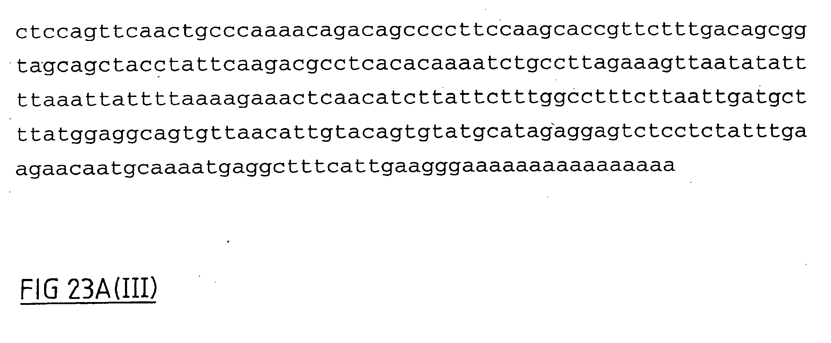

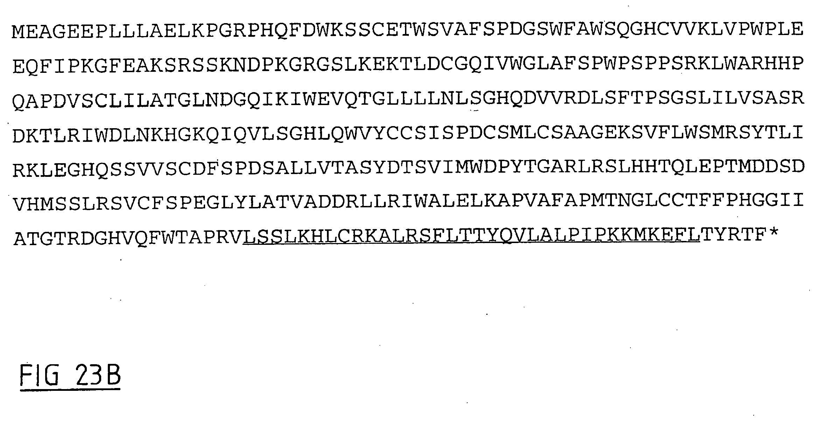

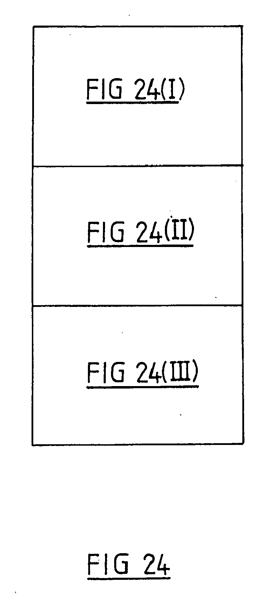

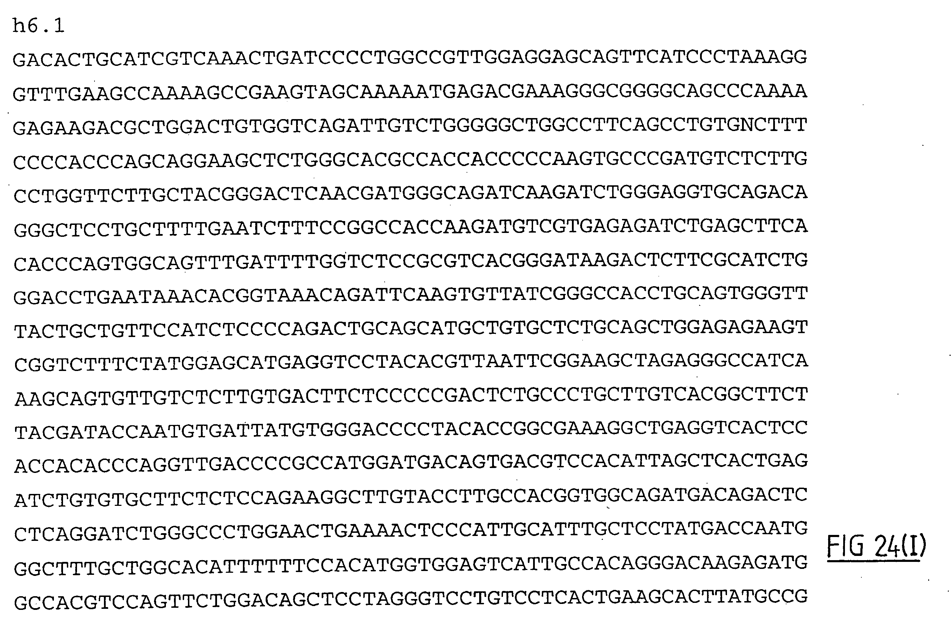

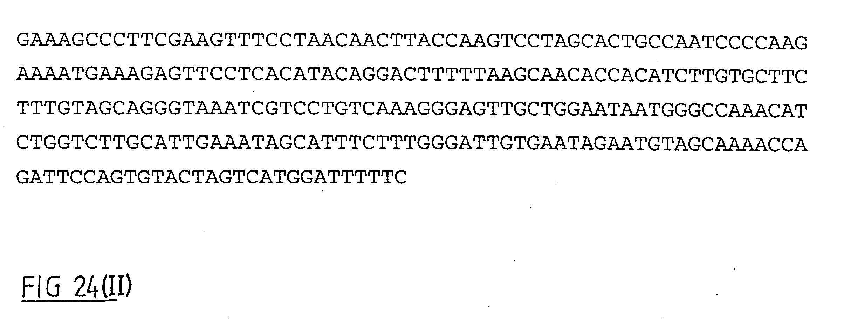

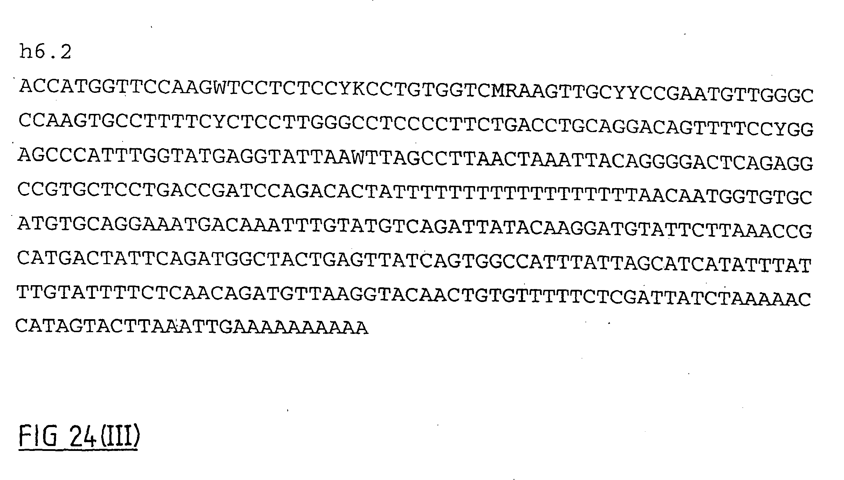

**Figure 22** is a diagrammatic representation showing the relationship of mouse SOCS6 cDNA clones (6-1A, 6-2A, 6-5B, 6-4N, 6-18, 6-29, 6-3N and 6-5N) to contigs derived from analysis of mouse ESTs (Table 6.1) and human ESTs (Table 6.2). The nucleotide sequence of the mouse SOCS-6 contig is shown in Figure 23, with the sequence of human SOCS6 contigs (h6.1 and h6.2) being shown in Figure 24. The deduced amino acid sequence of mouse SOCS6 is shown in Figure 23B. The structure of the protein is shown schematically, while the WD-40 repeats indicated by ( ) and the SOCS box by ( ). The putative 5' and 3' untranslated regions are shown by the thin solid line.

**Figure 23A** is a representation showing the nucleotide sequence of the mouse SOCS6 derived from analysis of cDNA clone 64-10A-11. The nucleotides encoding the part of the predicted coding region, ending in the stop codon are shown in upper case, while the predicted 3' untranslated regions are shown in lower case. The relationship of mouse cDNA sequence to mouse and human EST contigs is illustrated in Figure 22.

**Figure 23B** is a representation showing the predicted amino acid sequence of mouse SOCS6 protein, derived from the nucleotide sequence in Figure 23A. The SOCS box, which also shown in Figure 13 is underlined.

**Figure 24** is a representation showing the nucleotide sequence of human SOCS6 cDNA contig h6.1, derived from analysis of cDNA clone 5-94-2 and the ESTs listed in Table 6.2. The relationship of these contigs to the mouse cDNA sequence is illustrated in Figure 22

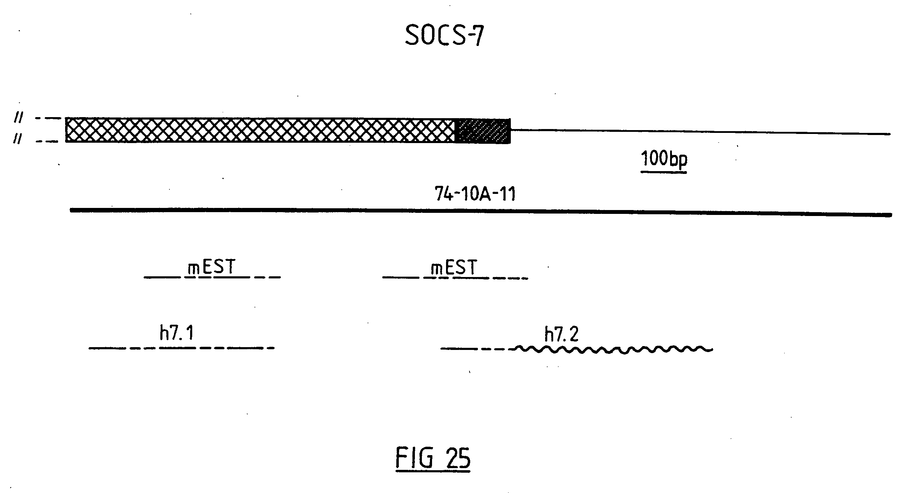

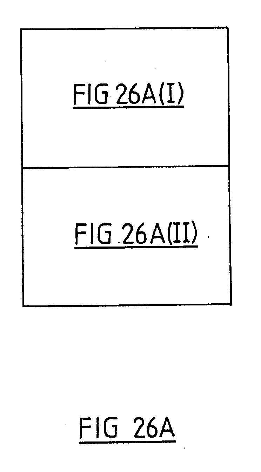

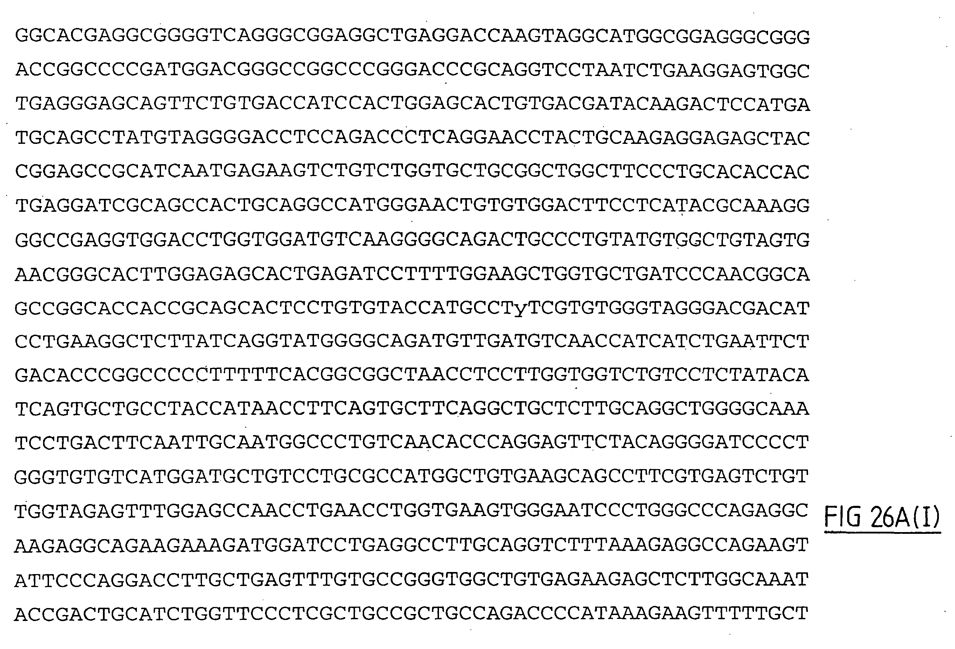

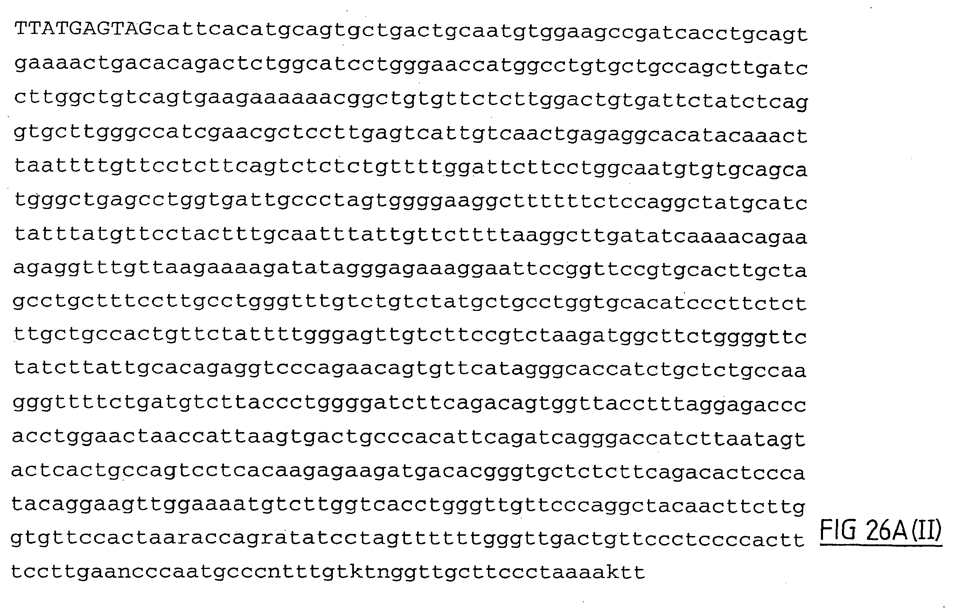

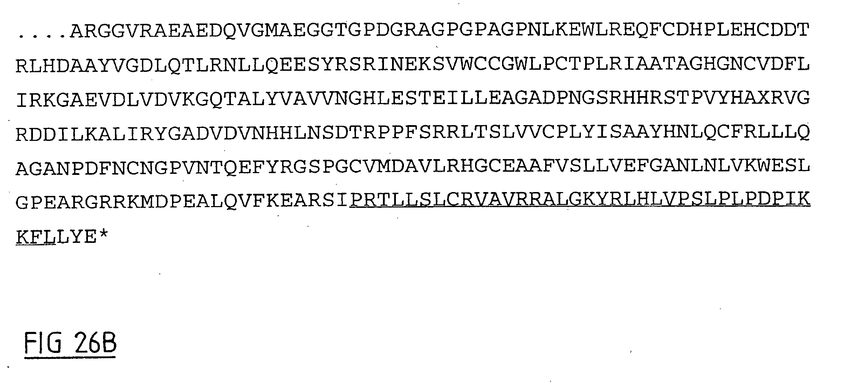

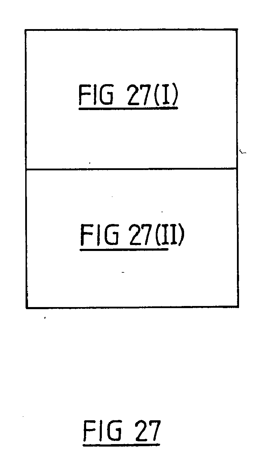

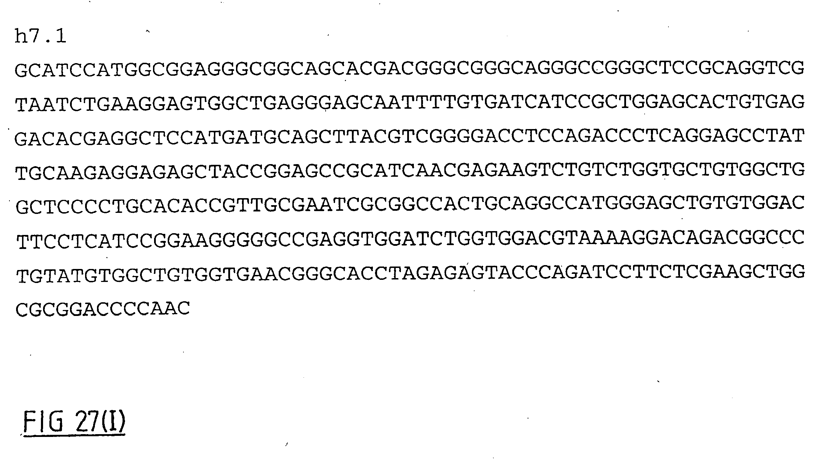

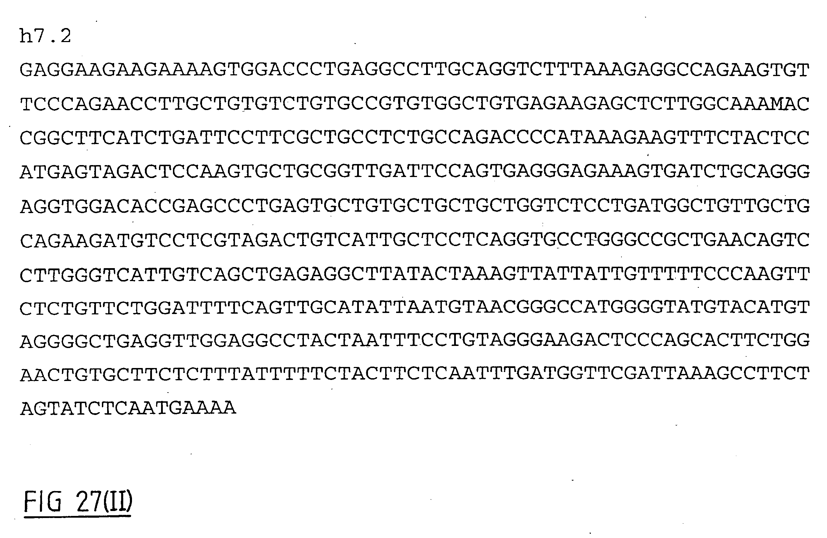

**Figure 25.** is a diagrammatic representation showing the relationship of mouse SOCS7 cDNA clone (74-10A-11) to contigs derived from analysis of mouse ESTs (Table 7.1) and human ESTs (Table 7.2). The nucleotide sequence of the mouse SOCS7 contig is shown in Figure 26 with the sequence of human SOCS7 contigs (h7.1 and h7.2) being shown in Figure 27. The deduced amino acid sequence of mouse SOCS7 is shown in Figure 26B. The structure of the protein is shown schematically, with the ankyrin repeats indicated by () and the SOCS box by (). The putative 5' and 3' untranslated regions are shown by the thin solid line in the mouse and by the wavy line in h7.2. Based on analysis of clones isolated to date and ESTs the 3' untranslated regions of mSOCS7 and hSOCS7 share little similarity.

**Figure 26A** is a representation showing the nucleotide sequence of the mouse SOCS7 derived from analysis of cDNA clone 74-10A-11. The nucleotides encoding the part of the predicted coding region, ending in the stop codon are shown in upper case, while the predicted 3' untranslated regions are shown in lower case. The relationship of mouse cDNA sequence to mouse and human EST contigs is illustrated in Figure 25.

**Figure 26B** is a representation showing the predicted amino acid sequence of mouse SOCS7 protein, derived from the nucleotide sequence in Figure 26A. The SOCS box, which also shown in Figure 13 is underlined.

**Figure 27** is a representation showing the nucleotide sequence of human SOCS7 cDNA contig h7.1 and h7.2 derived from analysis of the ESTs listed in Table 7.2. The relationship of these contigs to the mouse cDNA sequence is illustrated in Figure 25.

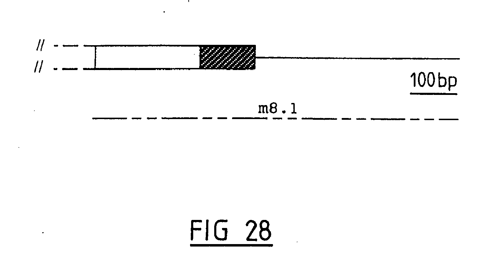

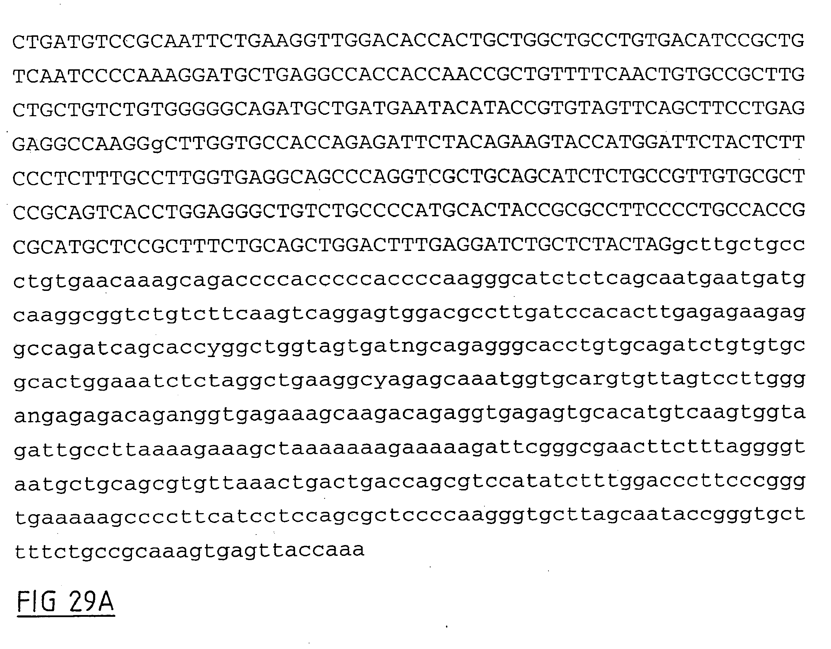

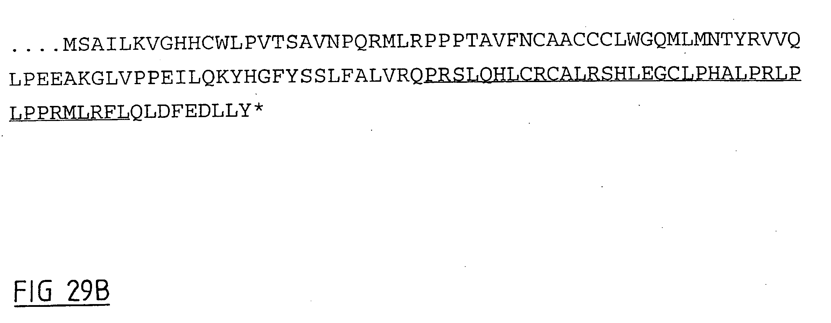

**Figure 28** is a diagrammatic representation of the relationship of sequence derived from analysis of mouse SOCS8 ESTs (Table 8.1 and Figure 29A) to the predicted protein structure of mouse SOCS8. The deduced partial amino acid sequence of mouse SOCS8 is shown in Figure 29B. The structure of the protein is shown schematically with the SOCS box highlighted ( ). The predicted 3' untranslated region is shown by the thin line.

**Figure 29A** is a representation showing the partial nucleotide sequence of mouse SOCS8 cDNA (contig 8.1) derived from analysis of ESTs. The nucleotides encoding the part of the predicted coding region, ending in the STOP codon are shown in upper case, while the predicted 3' untranslated regions are shown in lower case.

**Figure 29B** is a representation showing the partial predicted amino acid sequence of the mouse SOCS8 protein, derived from the nucleotide sequence in Figure 29A. The SOCS box, which also shown in Figure 13 is underlined.

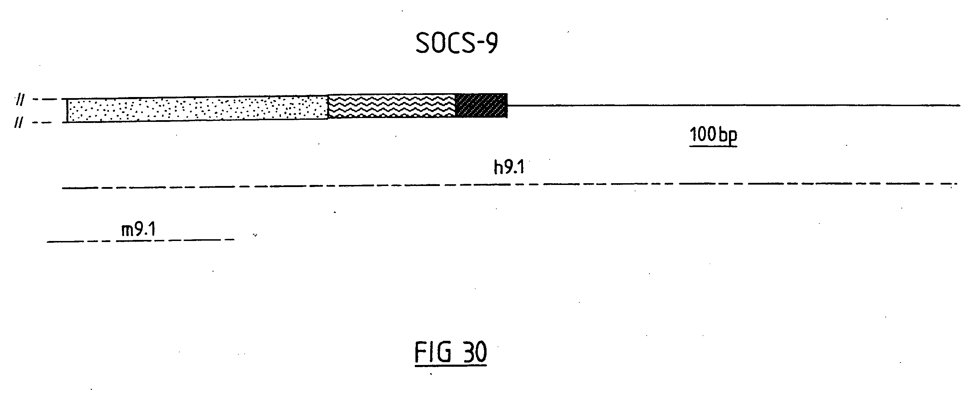

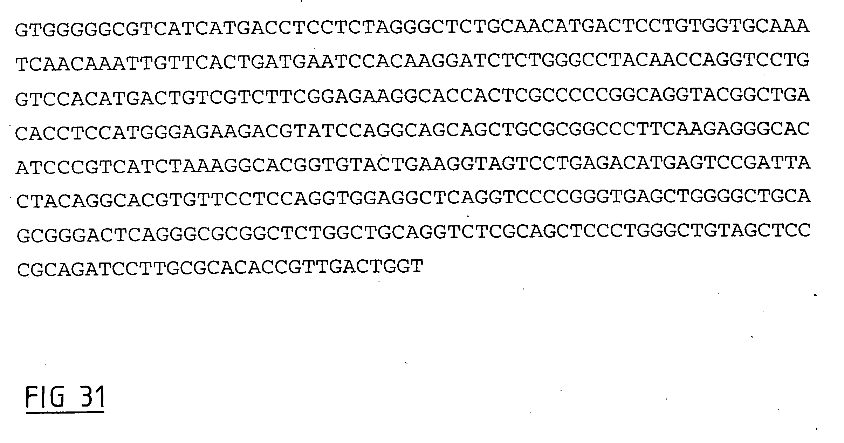

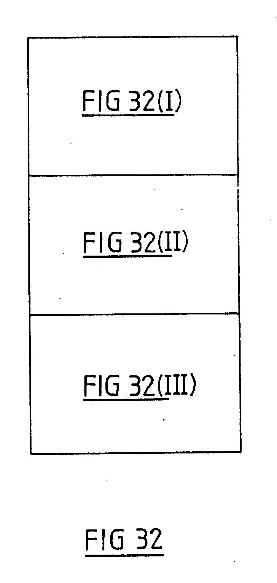

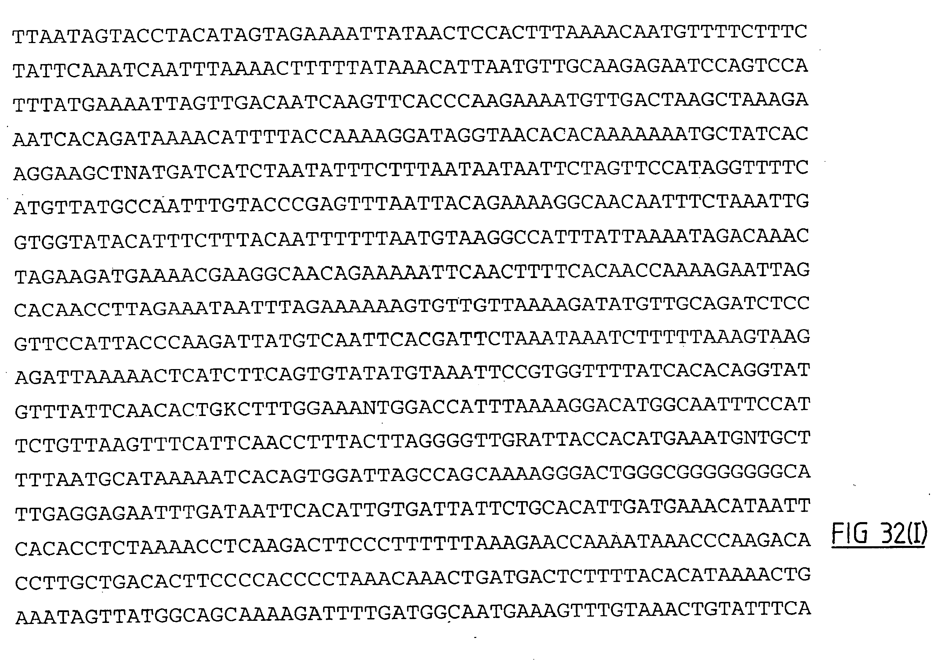

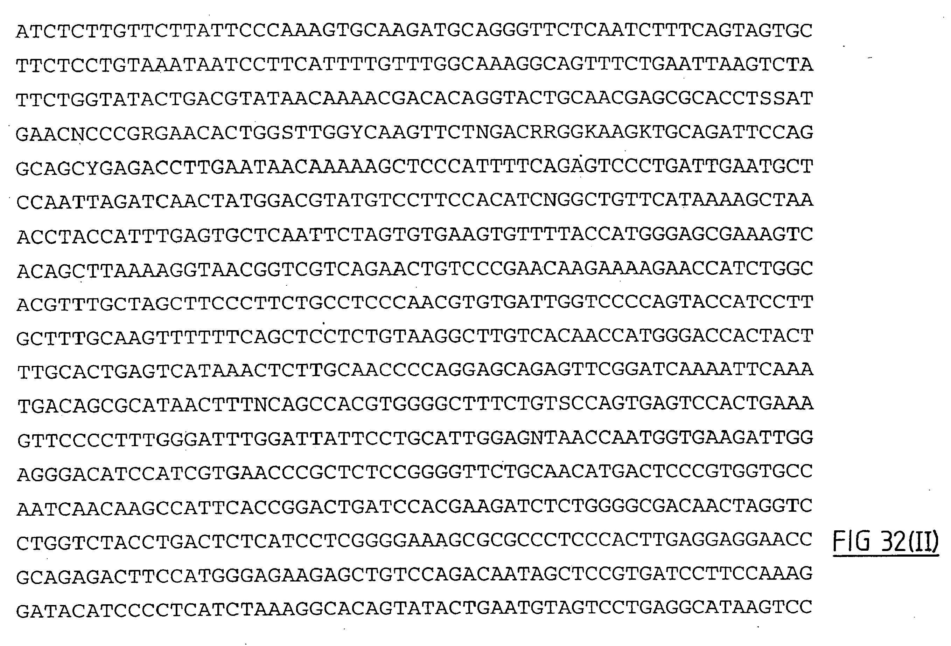

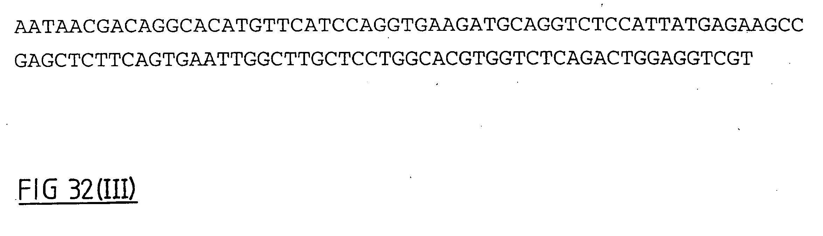

**Figure 30** is a diagrammatic representation showing the relationship of mouse SOCS9 ESTs (Table 9.1) and human SOCS9 ESTs (Table 9.2). The nucleotide sequence of the mouse SOCS9 contig (m9.1) is shown in Figure 31, with the sequence of human SOCS9 contig (h9.1) being shown in Figure 32. The deduced amino acid sequence of human SOCS9 is shown schematically, with the SH2 domain indicated by ( ) and the SOCS box by ( ). The putative 3' untranslated region is shown by the thin solid line.

**Figure 31** is a representation showing the partial nucleotide sequence of mouse SOCS9 cDNA (contig m9.1), derived from analysis of the ESTs listed in Table 9.1. The relationship of these contigs to the mouse cDNA sequence is illustrated in Figure 30.

**Figure 32** is a representation showing the partial nucleotide sequence of human SOCS9 cDNA (contig h9.1), derived from analysis of the ESTs listed in Table 9.2. Although it is clear that contig h9.1 encodes a protein with an SH2 domain and a SOCS box, the quality of the sequence is not high enough to derive a single unambiguous open reading frame. The relationship of these contigs to the mouse cDNA sequence is illustrated in Figure 30.

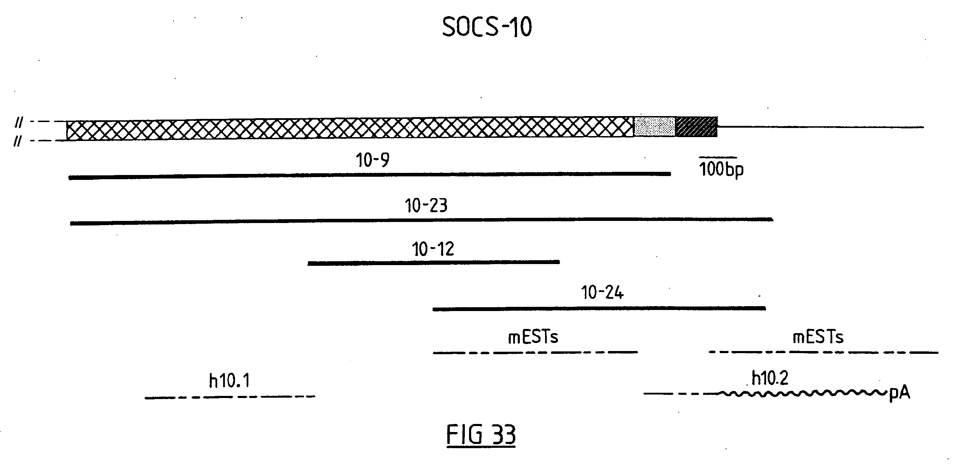

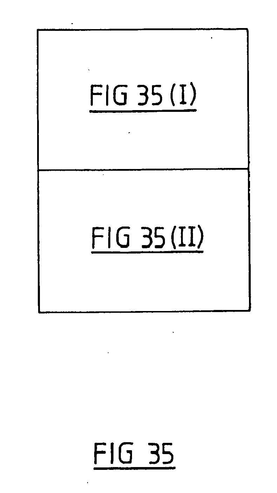

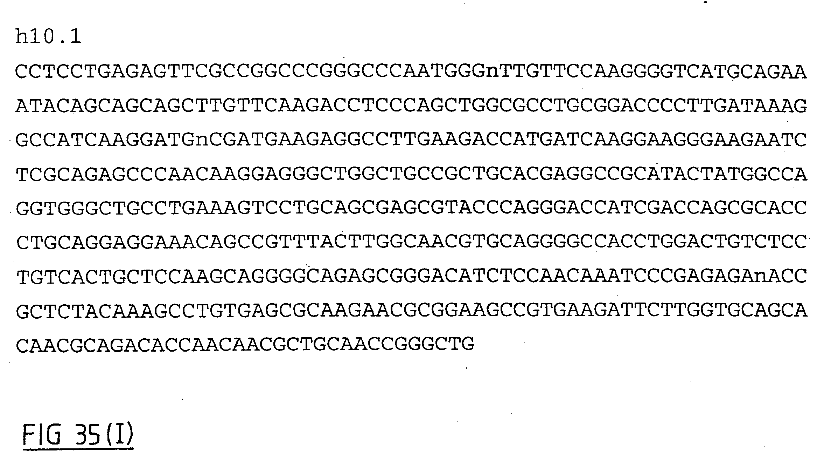

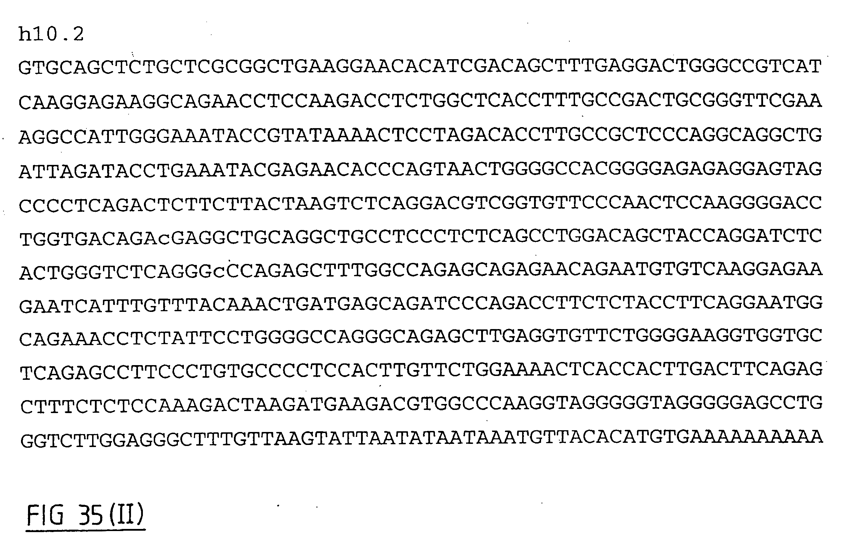

**Figure 33** is a representation showing the relationship of mouse SOCS10 cDNA clones (10-9, 10-12, 10-23 and 10-24) to contigs derived from analysis of mouse ESTs (Table 10.1) and human ESTs (Table 10.2). The nucleotide sequence of the mouse SOCS10 contig is shown in Figure 10.2, with the sequence of human SOCS10 contigs (h10.1 and h10.2) being shown in Figure 35. The predicted structure of the protein is shown schematically, with the ankyrin repeats indicated by ( ) and the SOCS box by ( ). The putative 3' untranslated regions is shown by the thin line solid line in the mouse and by the wavy line in h10.2. Based on analysis of clones isolated to date and ESTs the 3' untranslated regions of mSOCS-10 and hSOCS-10 share little similarity.

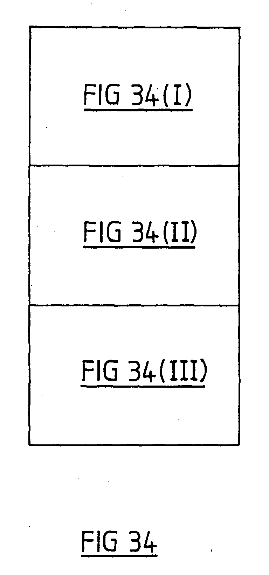

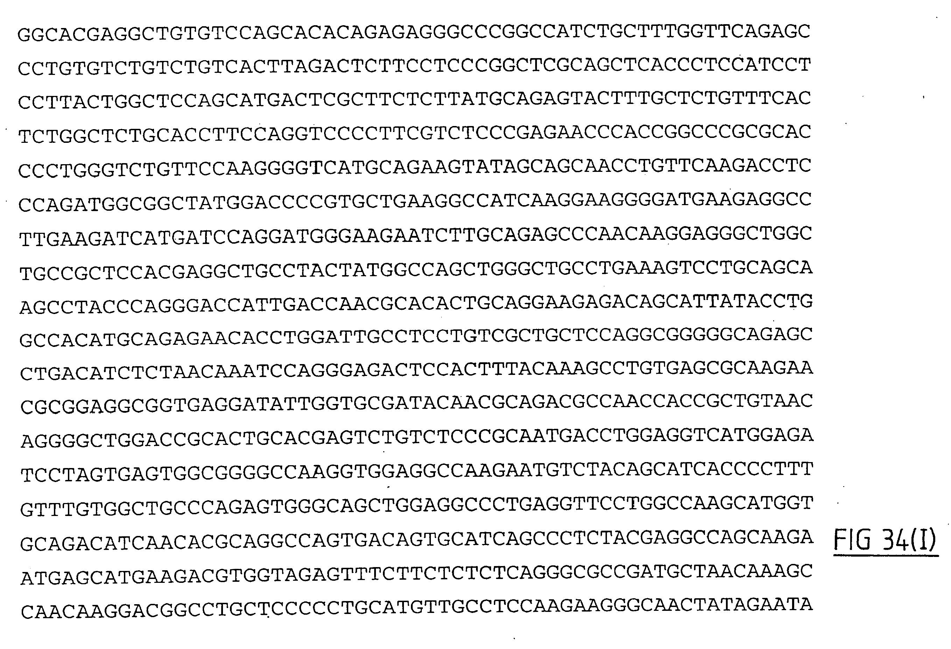

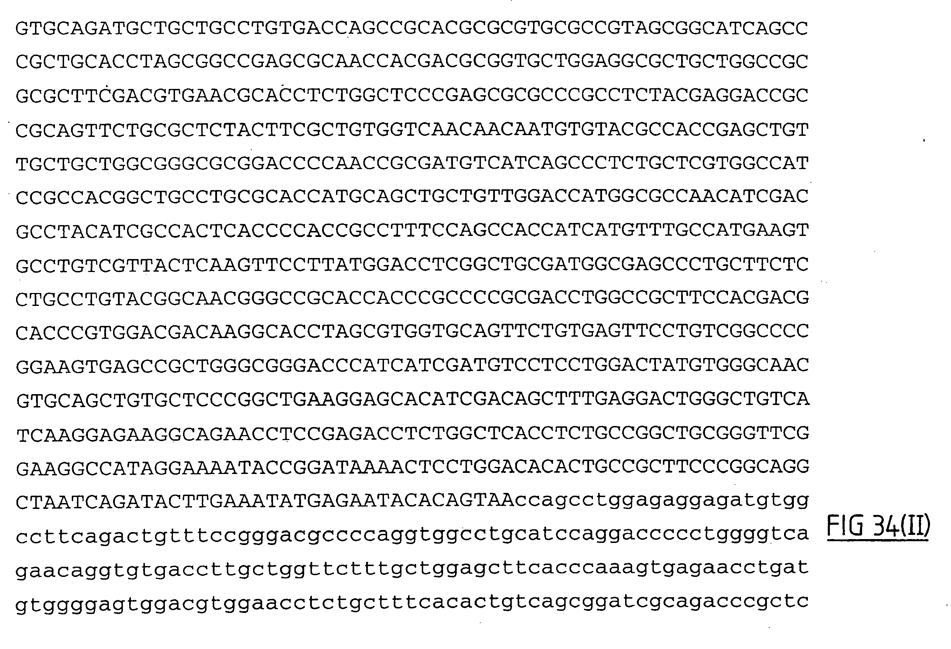

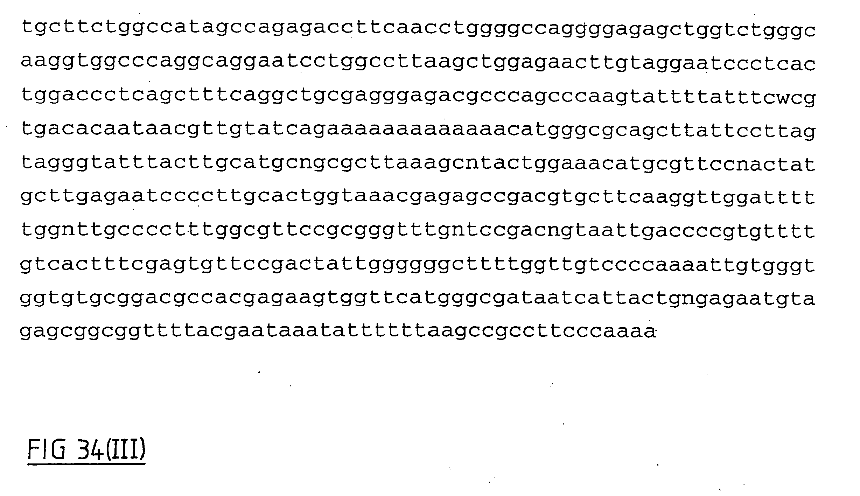

**Figure 34** is a representation showing the nucleotide sequence of the mouse SOCS10 derived from analysis of cDNA clone 10-9, 10-12, 10-23 and 10-24. The nucleotides encoding the part of the predicted coding region, ending in the stop codon are shown in upper case, while the predicted 3' untranslated regions are shown in lower case. Although it is clear that contig m10.1 encodes a protein with a series of ankyrin repeats and a SOCS box, the quality of the sequence is not high enough to derive a single unambiguous open reading frame. The relationship of mouse cDNA sequence to mouse and human EST contigs is illustrated in Figure 33.

**Figure 35** is a representation showing the nucleotide sequence of human SOCS10 cDNA contig h10.2 and h10.2 derived from analysis of the ESTs listed in Table 10.2. The relationship of these contigs to the mouse cDNA sequence is illustrated in Figure 33.

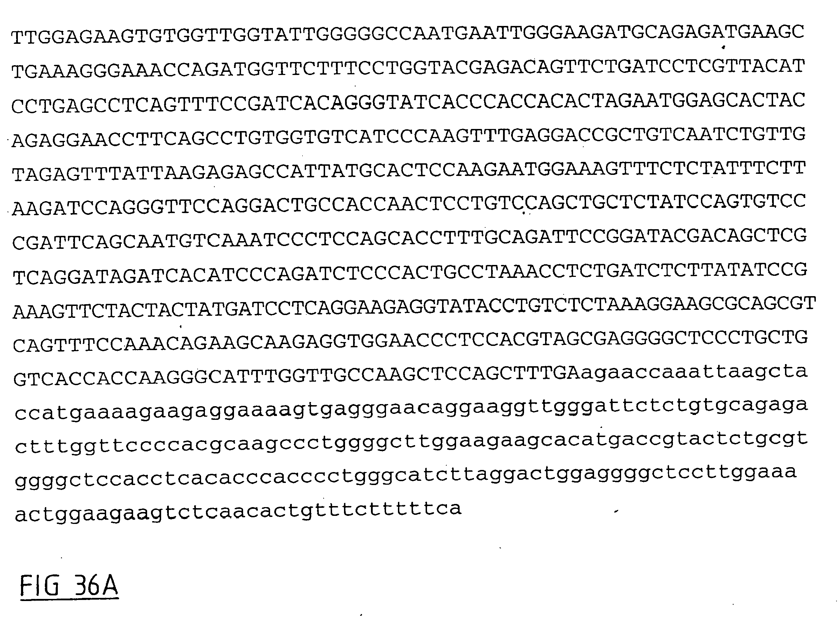

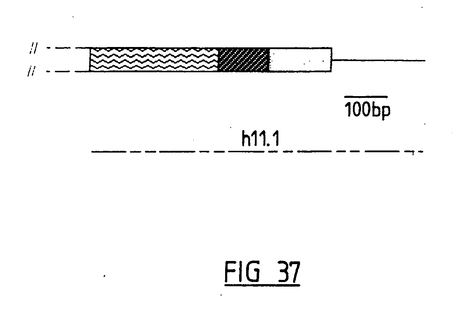

**Figure 36A** is a representation showing the partial nucleotide sequence of the human SOCS11 cDNA derived from analysis of ESTs listed in Table 11.1 The nucleotides encoding the mature coding region from the predicted ATG "start" codon to the stop codon is shown in upper case, while the predicted 5' and 3' untranslated regions are shown in lower case. The relationship of the partial cDNA sequence, derived from ESTs, to the predicted protein is shown in Figure 37.

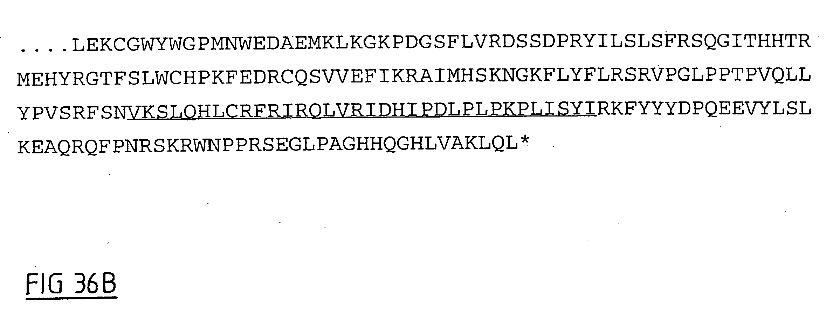

**Figure 36B** is a representation showing the partial predicted amino acid sequence of human SOCS11 protein, derived from the nucleotide sequence in Figure 36A. The SOCS box, which also shown in Figure 13, is underlined.

**Figure 37** is a diagrammatic representation showing the relationship of sequence derived from analysis of human SOCS-11 ESTs (Table 11.1 and Figure 36A) to the predicted protein structure of human SOCS11. The deduced partial amino acid sequence of human SOCS11 is shown in Figure 36B. The structure of the protein is shown schematically with the SH2 domain shown by ( ) and the SOCS box highlighted by ( ). The predicted 3' untranslated region is shown by the thin line.

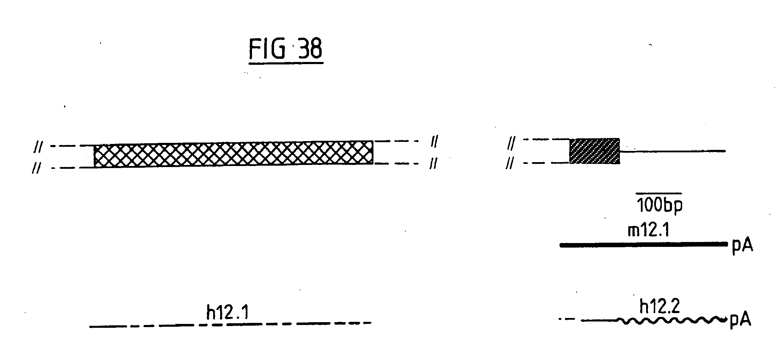

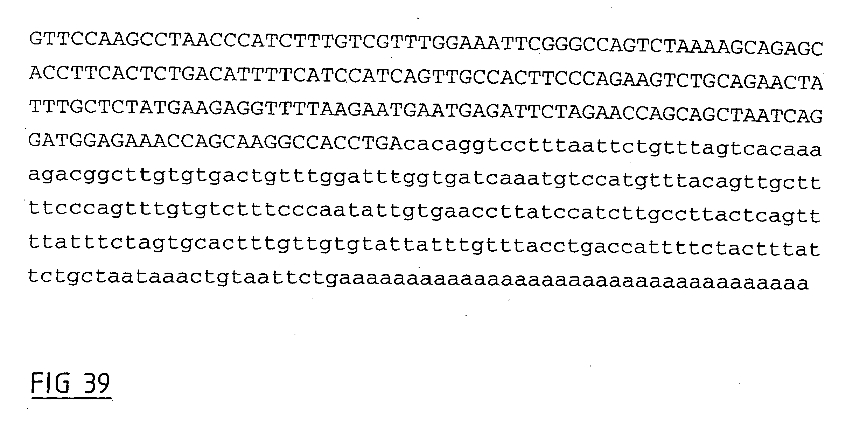

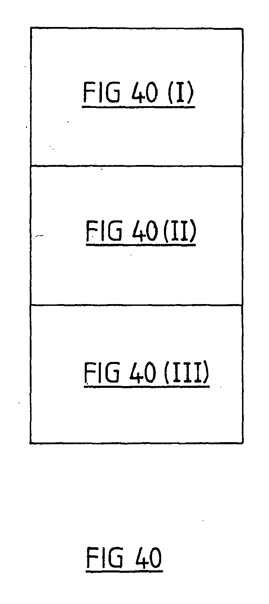

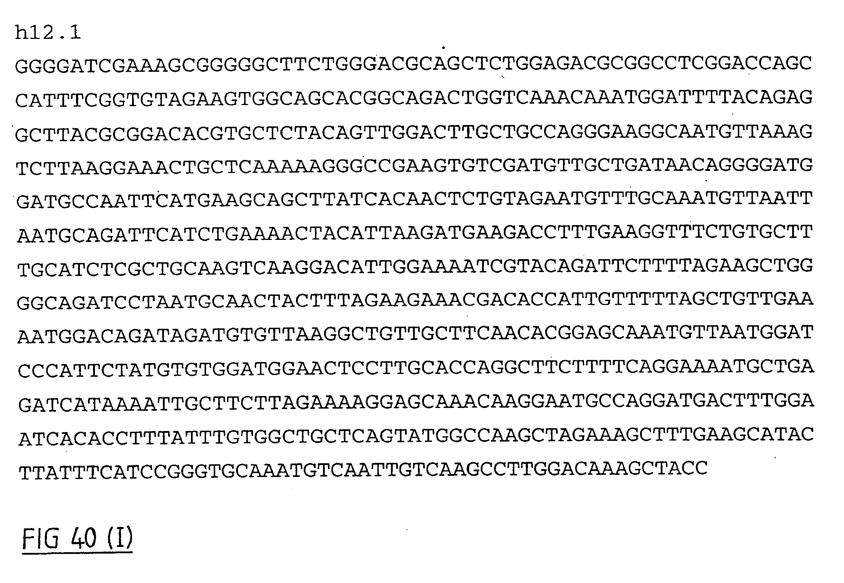

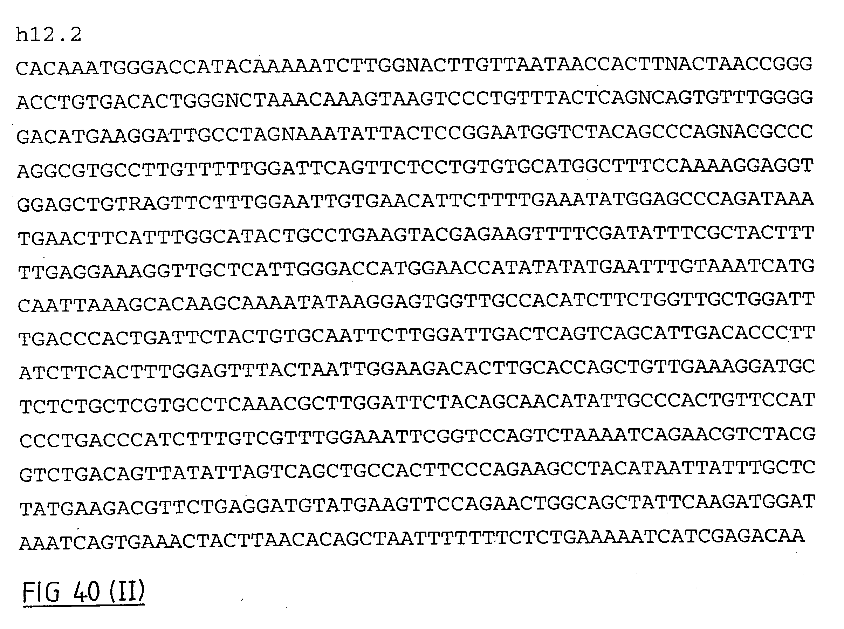

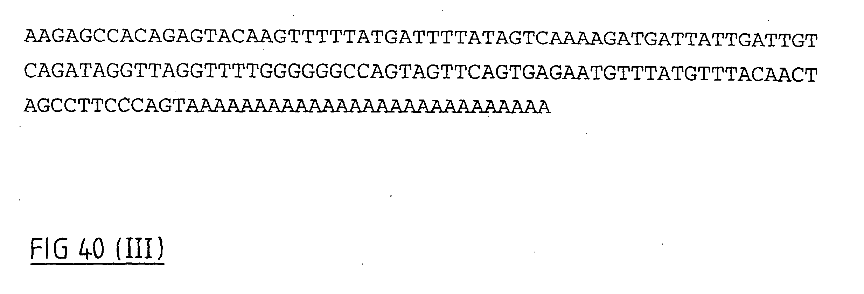

**Figure 38** is a diagrammatic representation showing the relationship of mouse SOCS12 cDNA clones (12-1) to contigs derived from analysis of mouse ESTs (Table 12.1) and human ESTs (Table 12.2). The nucleotide sequence of the mouse SOCS12 contig is shown,in Figure 12.2, with the sequence of human SOCS12 contigs (h12.1 and h12.2) being shown in Figure 40. The deduced partial amino acid sequence of mouse SOCS12 is shown in Figure 39. The structure of the protein is sown schematically, with the ankyrin repeats indicated by ( ) and the SOCS box by ( ). The putative.3' untranslated region is shown by the thin line solid line in the mouse and by the wavy line in h12.2. Based on analysis of clones isolated to date and ESTs the 3' untranslated regions of mSOCS12 and hSOCS12 share little similarity.

**Figure 39** is a representation showing the nucleotide sequence of the mouse SOCS12 derived from analysis of cDNA clone 12-1 and the ESTs listed in Table 12.1. The nucleotides encoding the part of the predicted coding region, including the stop codon are shown in upper case, while the predicted 3' untranslated region is shown in lower case. By homology with human SOCS12 it is clear that contig m12.1 encodes a protein with a series of ankyrin repeats and a SOCS box, the quality of the sequence is not high enough to derive a single unambiguous open reading frame. The relationship of mouse cDNA sequence to mouse and human EST contigs is illustrated in Figure 38.

**Figure 40** is a representation showing the nucleotide sequence of human SOCS12 cDNA contig h12.1 and h12.2 derived from analysis of the ESTs listed in Table 12.2. The relationship of these contigs to the mouse cDNA sequence is illustrated in Figure 38.

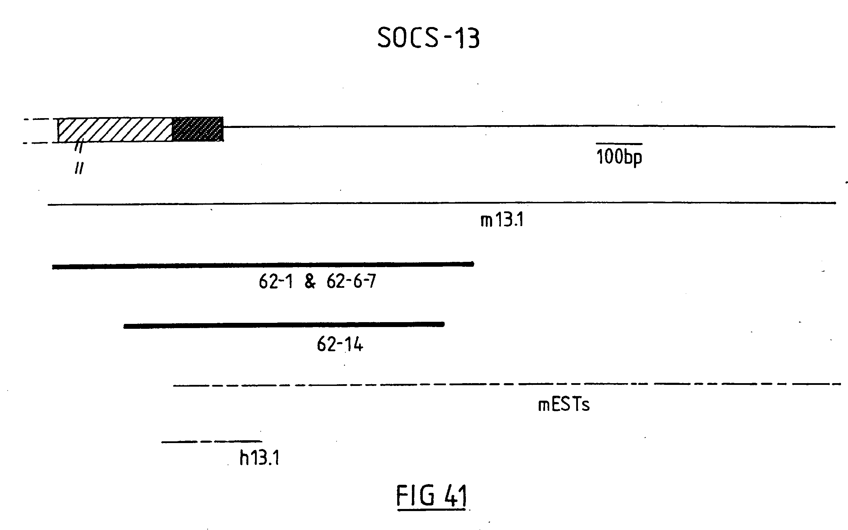

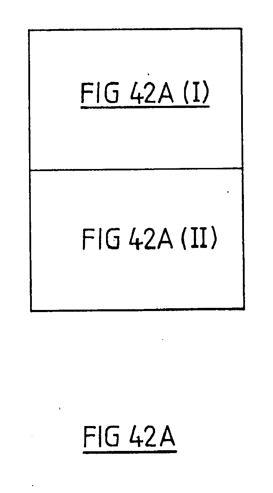

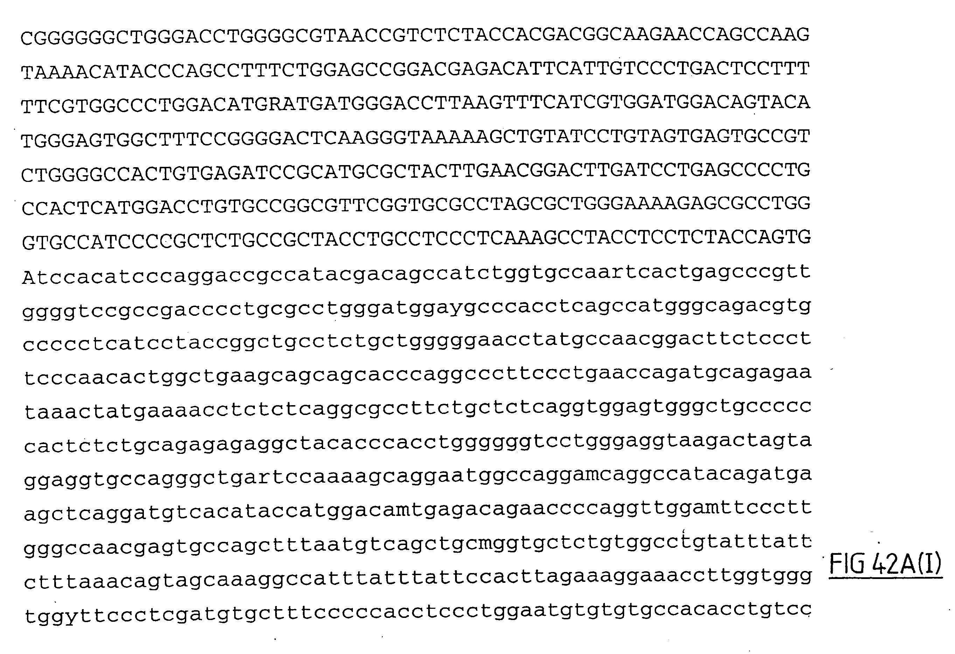

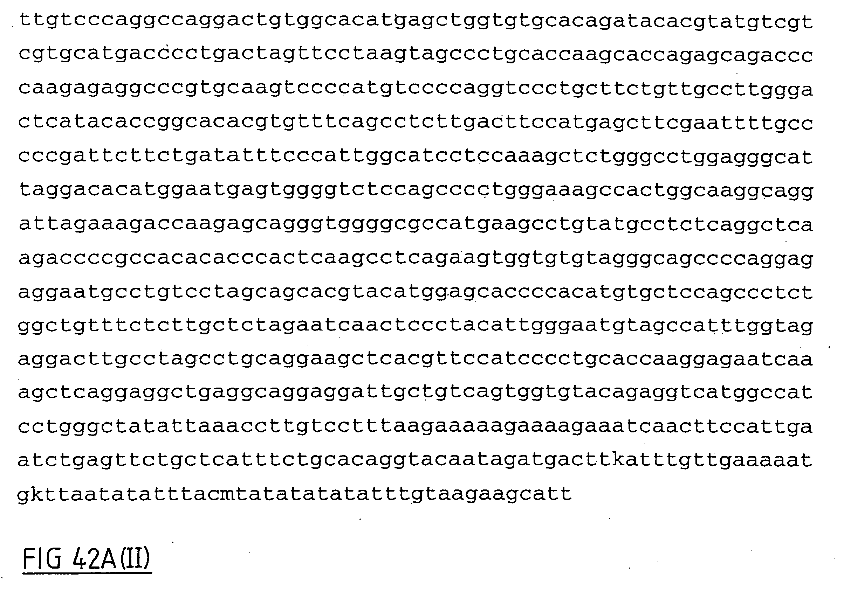

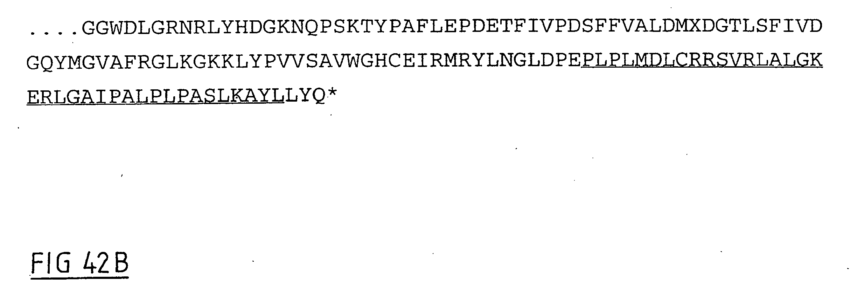

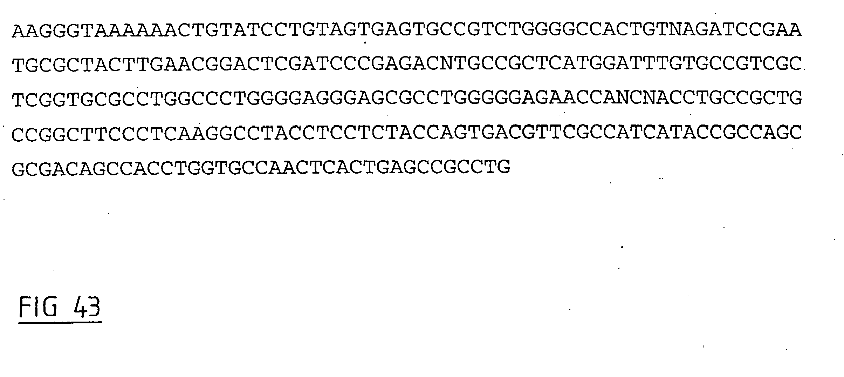

**Figure 41** is a diagrammatic representation showing the relationship of contig m13.1 derived from analysis of mouse SOCS13 cDNA clones (62-1, 62-6-7, 62-14) and mouse ESTs (Table 13.1) to contig h13.1 derived from analysis of human ESTs (Table 13.2). The nucleotide sequence of the mouse SOCS13 contig is shown in Figure 42, with the sequence of human SOCS13 contig (h13.1) being shown in Figure 43. The deduced amino acid sequence of mouse SOCS13 is shown in Figure 42B. The structure of the protein is shown schematically, with the WD-40 repeats highlighted by ( ) and the SOCS box highlighted by ( ). The 3' untranslated region is shown by the thin line solid line.

**Figure 42A** is a representation showing the nucleotide sequence of the mouse SOCS13 derived from analysis of cDNA clones 62-1, 62-6-7 and 62-14. The nucleotides encoding part of the predicted coding region, ending in the stop codon are shown in upper case, while those encoding the predicted 3' untranslated regions are shown in lower case. The relationship of mouse cDNA sequence to mouse and human EST contigs is illustrated in Figure 41.

**Figure 42B** is a representation showing the predicted amino acid sequence of mouse SOCS13 protein, derived from the nucleotide sequence in Figure 42A. The SOCS box, which also shown in Figure 13 is underlined.

**Figure 43** is a representation showing the nucleotide sequence of human SOCS13 cDNA contig h13.1 derived from analysis of the ESTs listed in Table 13.2. The relationship of these contigs to the mouse cDNA sequence is illustrated in Figure 41.

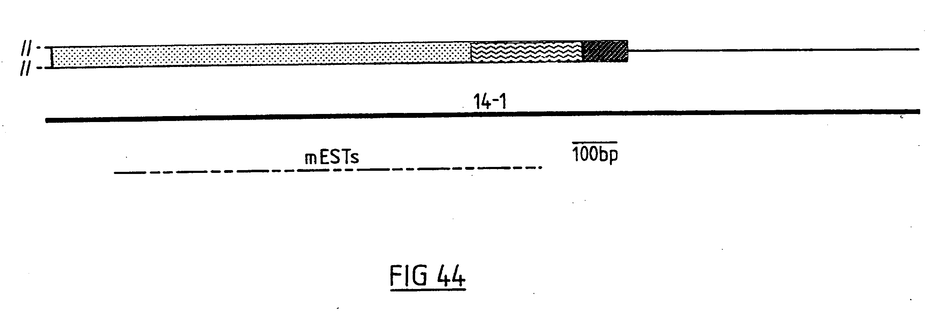

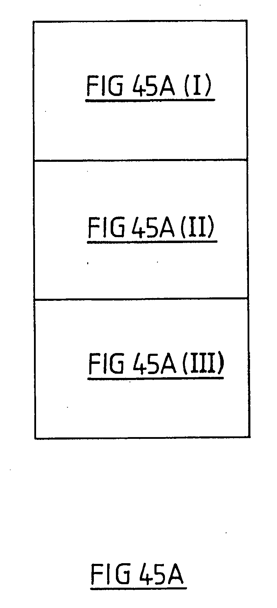

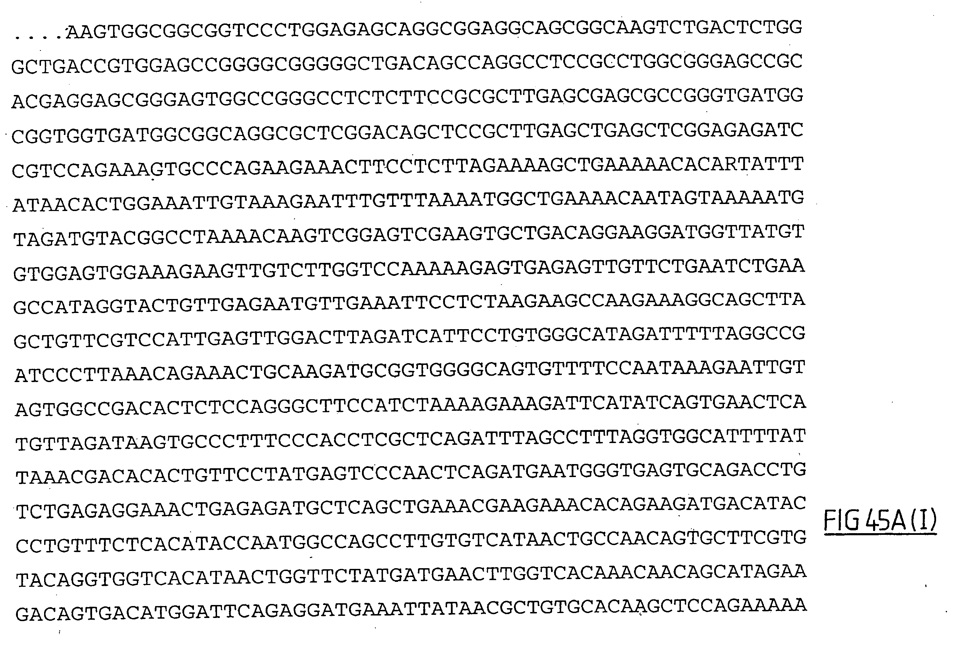

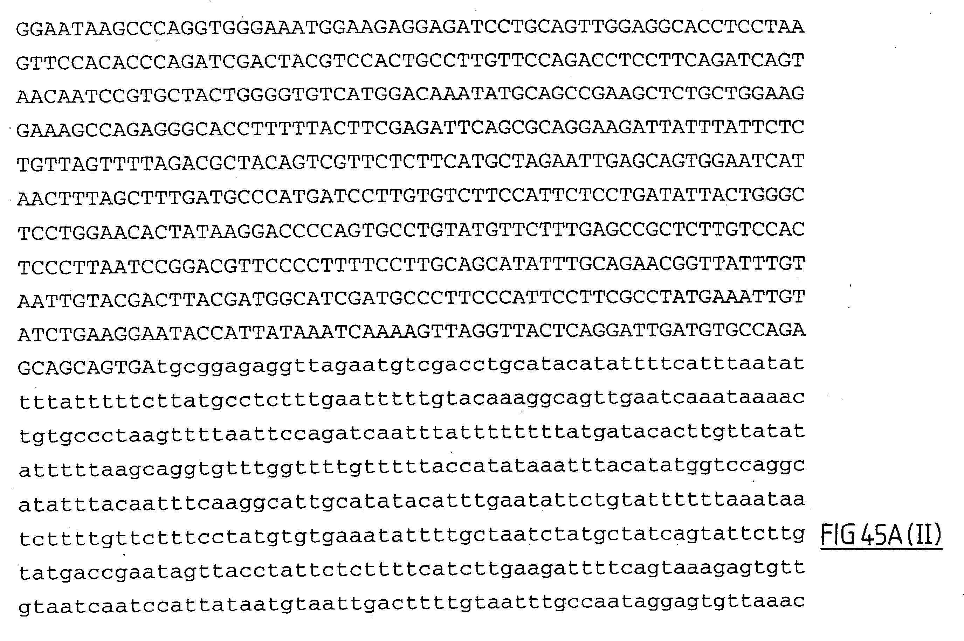

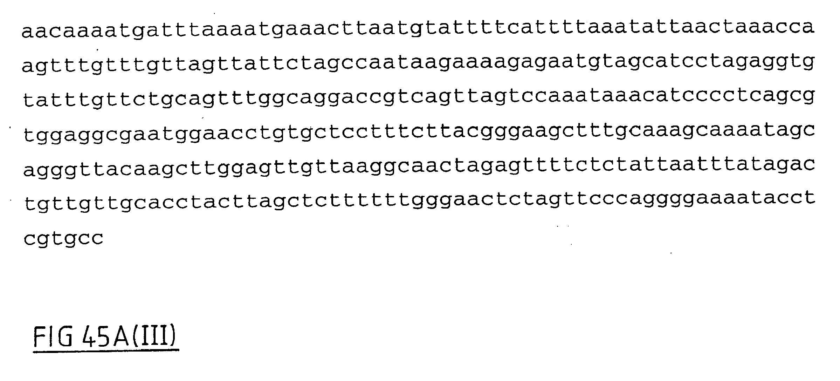

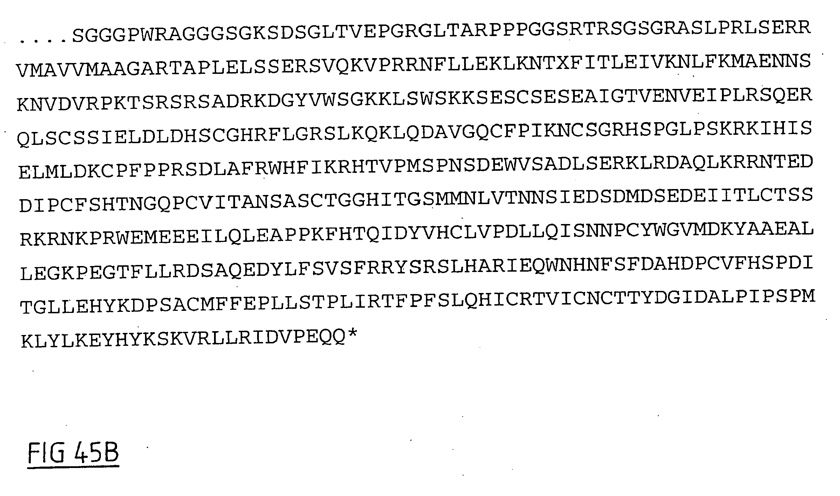

**Figure 44** is a diagrammatic representation showing the relationship of a partial mouse SOCS14 cDNA clone (14-1) to contigs derived from analysis of mouse ESTs (Table 14.1). The nucleotide sequence of the mouse SOCS14 contig is shown in Figure 45. The deduced partial amino acid sequence of mouse SOCS14 is shown in Figure 45B. The structure of the protein is shown schematically, with the SH3 domain indicated by ( ) and the SOCS box by ( ). The putative 3' untranslated region is shown by the thin line.

**Figure 45A** is a representation showing the nucleotide sequence of the mouse SOCS14 derived from analysis of genomic and cDNA clones. The nucleotides encoding the mature coding region from the predicted ATG "start" codon to the stop codon is shown in upper case, while the predicted 5' and 3' untranslated regions are shown in lower case. The relationship of mouse cDNA sequence to mouse and human EST contigs is illustrated in Figure 44.

**Figure 45B** is a representation showing the predicted amino acid sequence of mouse SOCS14 protein, derived from the nucleotide sequence in Figure 45B. The SOCS box, which also shown in Figure 13 is underlined.

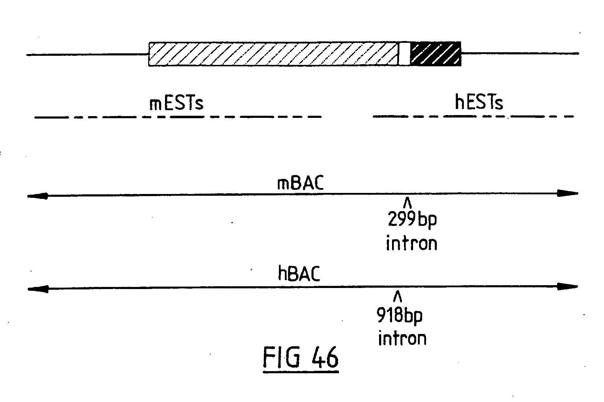

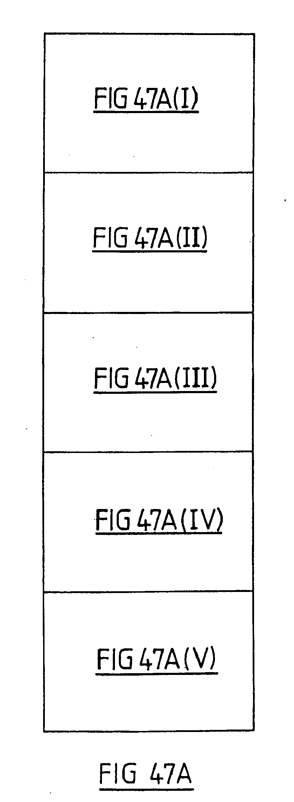

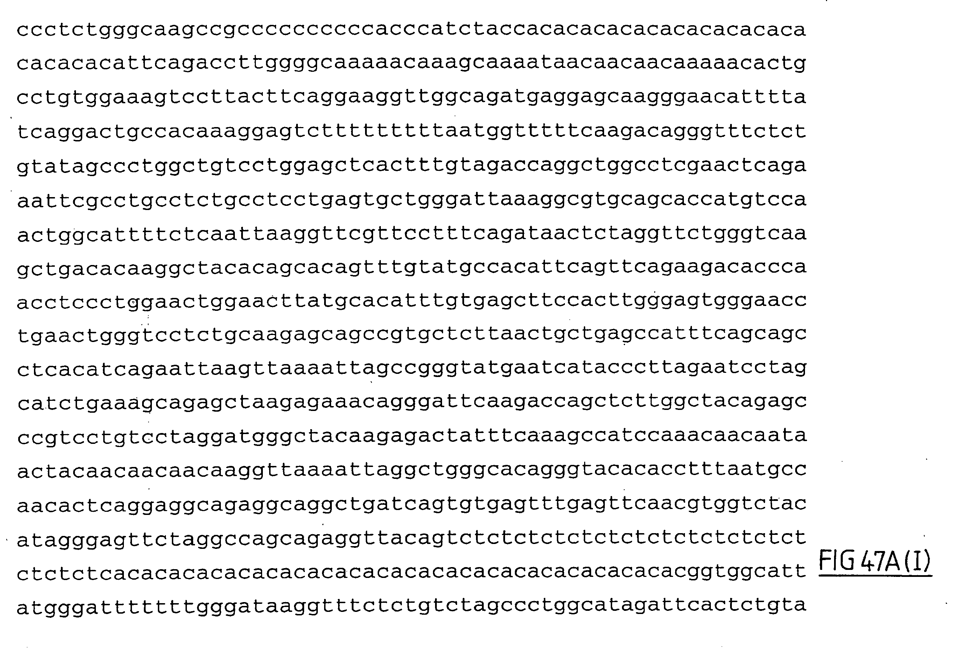

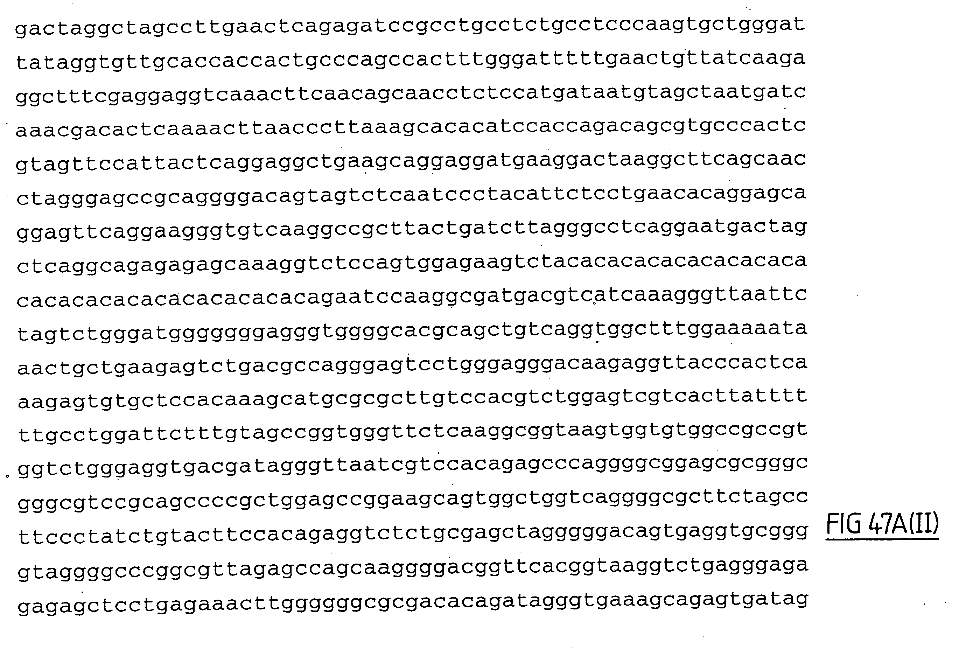

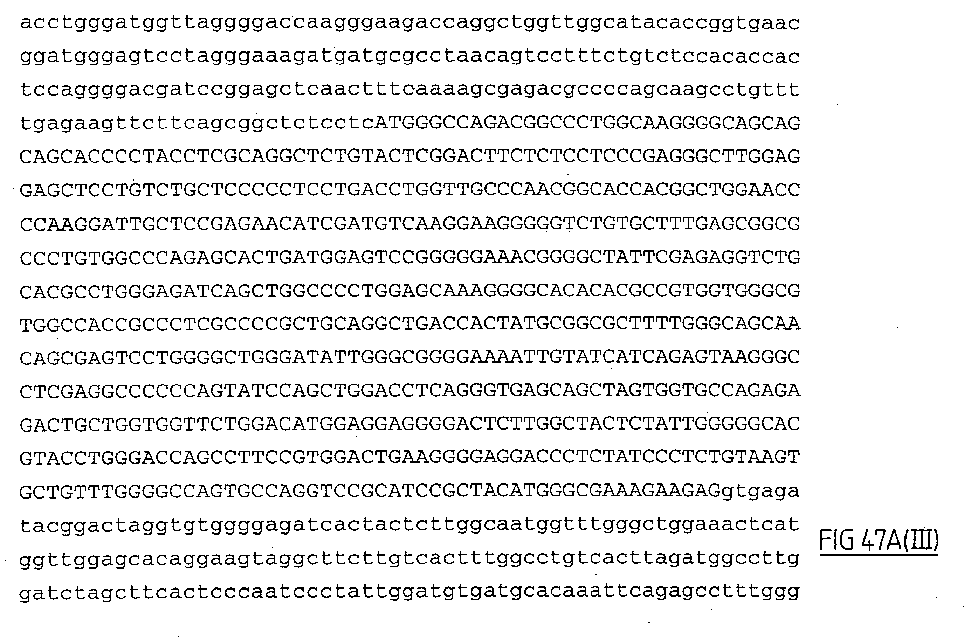

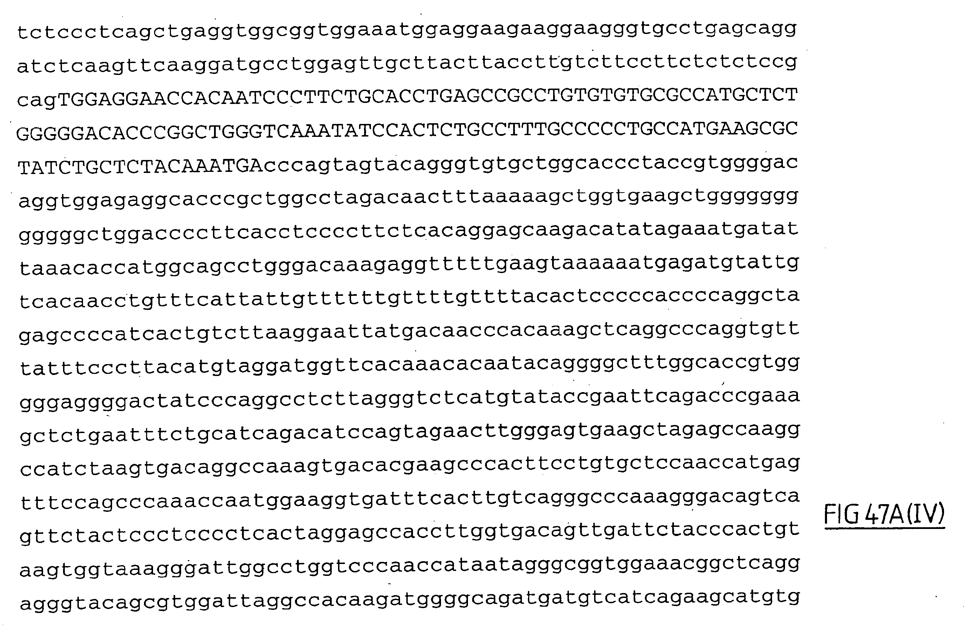

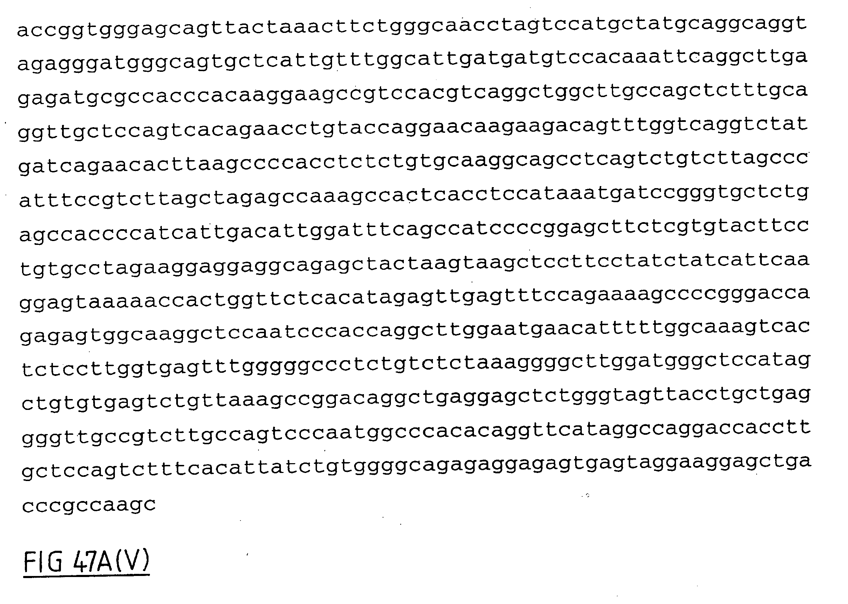

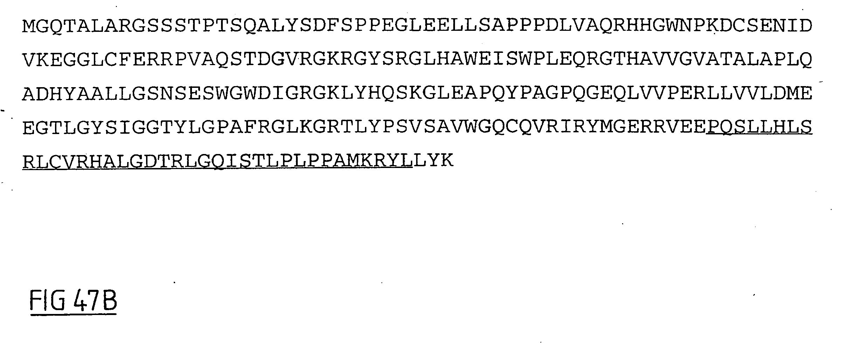

**Figure 46** is a diagrammatic representation showing the relationship of contig m15.1 derived from analysis of mouse BAC and mouse ESTs (Table 15.1) to contig h15.1 derived from analysis of the human BAC and human ESTs (Table 15.2). The nucleotide sequence of the mouse SOCS15 contig is shown in Figure 47, with the sequence of human SOCS15 contig (h15.1) being shown in Figure 47. The deduced amino acid sequence of mouse SOCS15 is shown in Figure 47B. The structure of the protein is shown schematically, with the WD-40 repeats highlighted by ( ) and the SOCS box highlighted by ( ). The 5' and 3' untranslated region are shown by the thin line solid line. The introns which interrupt the coding region are shown by ^.

**Figure 47A** is a representation showing the nucleotide sequence covering the mouse SOCS15 gene derived from analysis the mouse BAC listed in Table 15.1. The nucleotides encoding the predicted coding region, beginning with the ATG and ending in the stop codon are shown in upper case, while those encoding the predicted 5' untranslated region, the introns and the 3' untranslated region are shown in lower case. The relationship of mouse BAC to mouse and human ESTs contigs is illustrated in Figure 46.

**Figure 47B** is a representation showing the predicted amino acid sequence of mouse SOCS15 protein, derived from the nucleotide sequence in Figure 47A. The SOCS box, which also shown in Figure 13 is underlined.

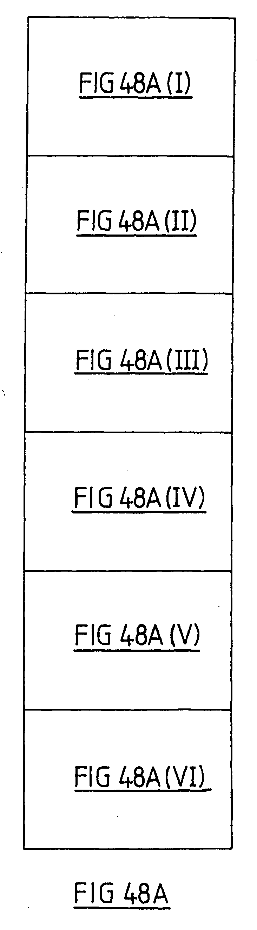

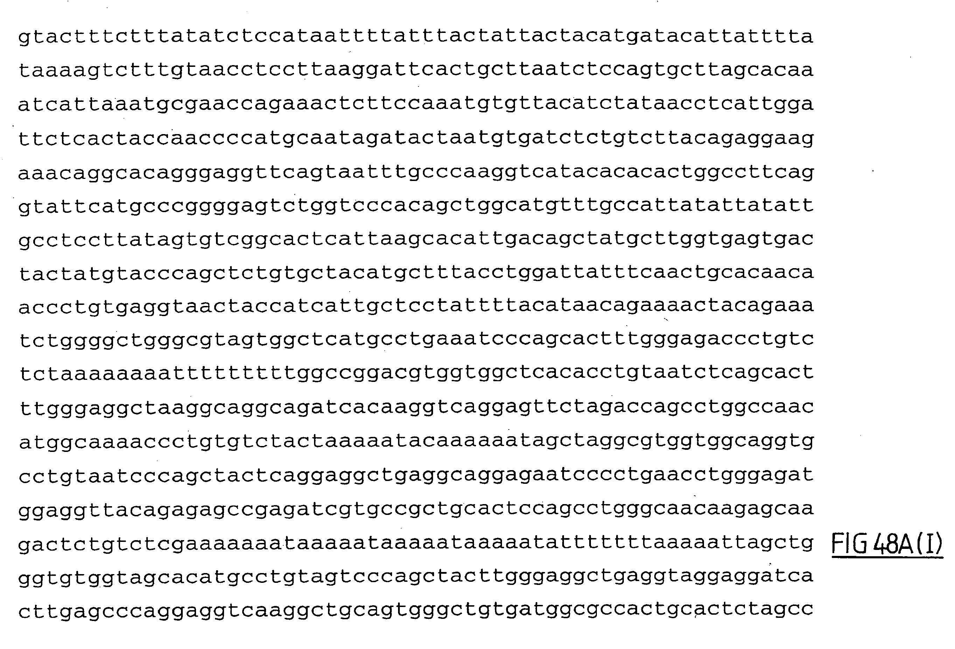

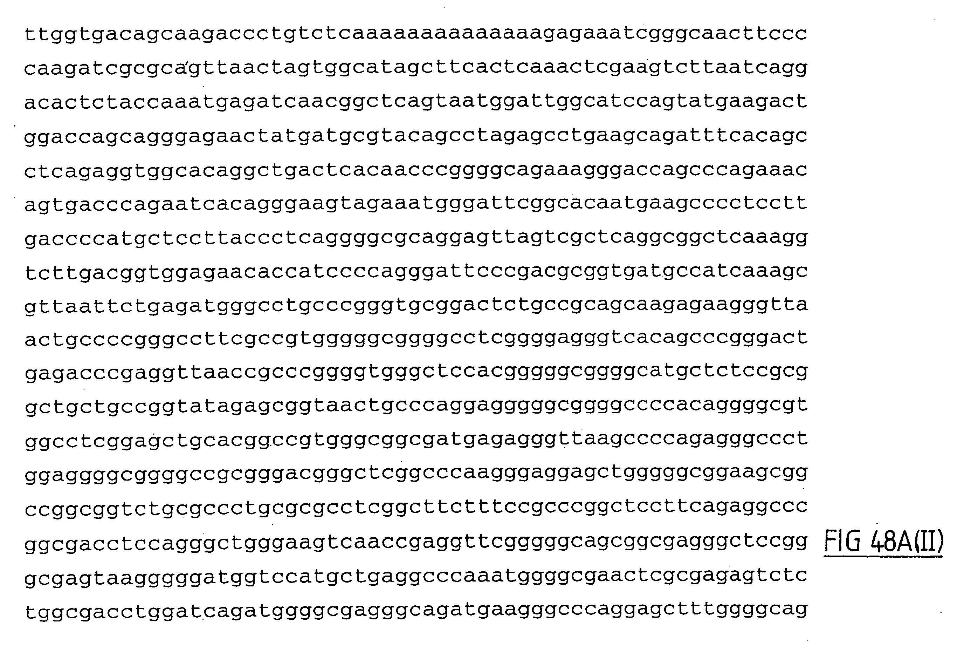

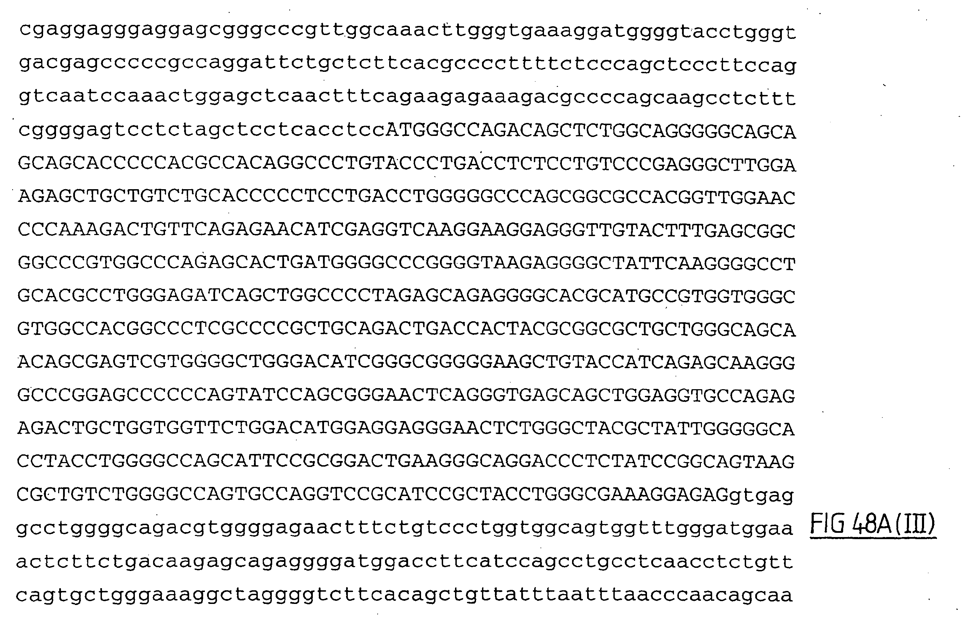

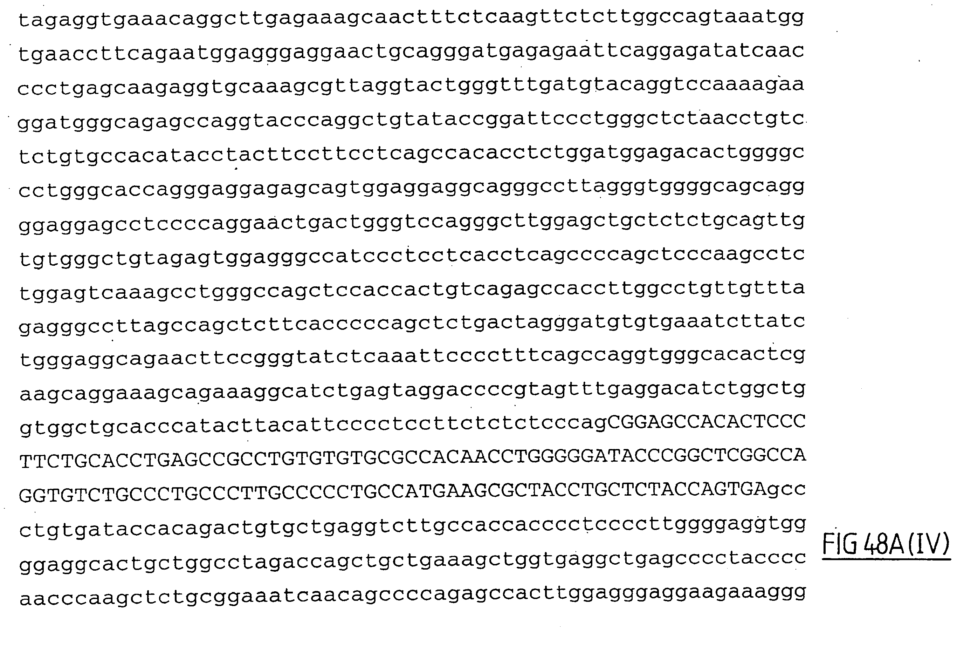

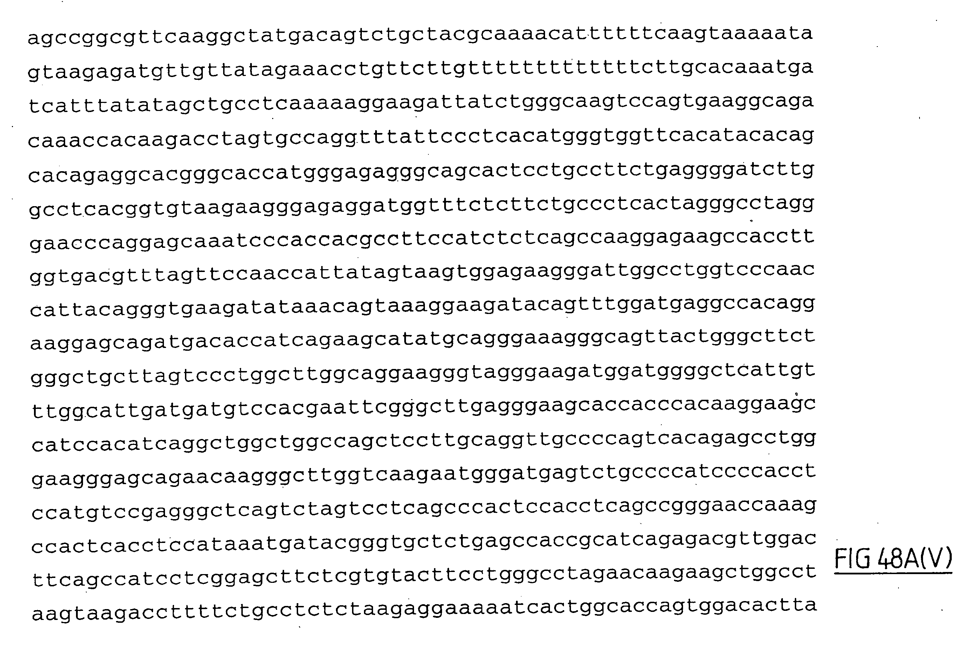

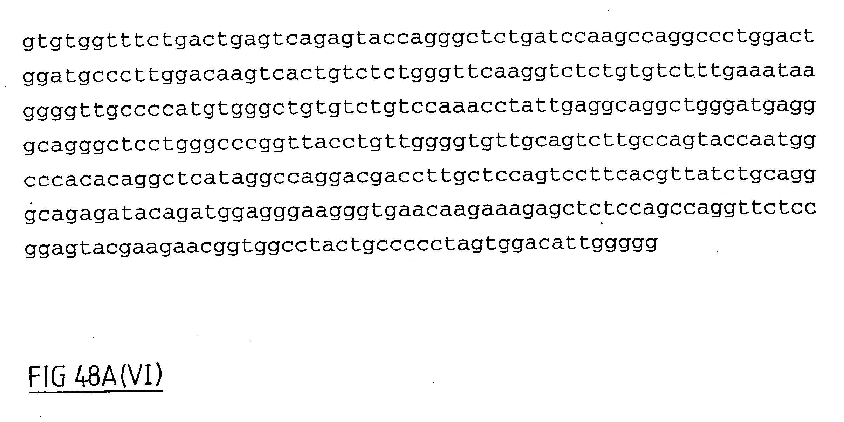

**Figure 48A** is a representation showing the nucleotide sequence covering the human SOCS15 gene derived from analysis the human BAC listed in Table 15.2. The nucleotides encoding the predicted coding region, beginning with the ATG and ending in the stop codon are shown in upper case, while those encoding the predicted 5' untranslated region, the introns and the 3' untranslated region are shown in lower case. The relationship of the human BAC to mouse and human ESTs contigs is illustrated in Figure 46.

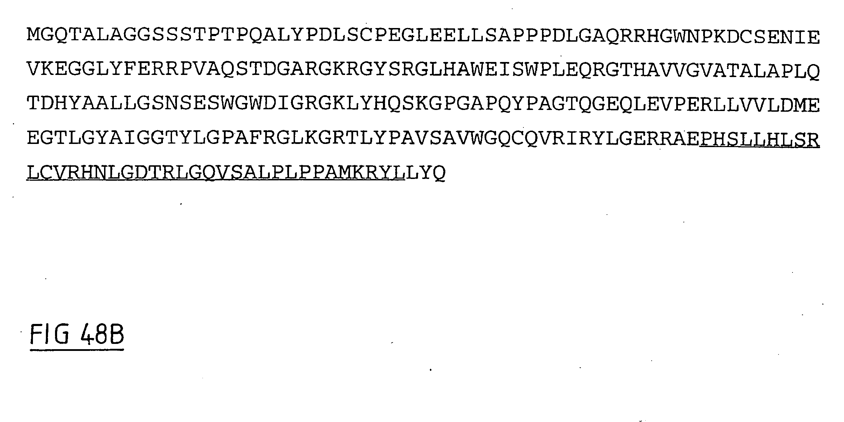

**Figure 48B** is a representation showing the predicted amino acid sequence of human SOCS15 protein, derived from the nucleotide sequence in Figure 48A. The SOCS box, which also shown in Figure 13 is underlined.

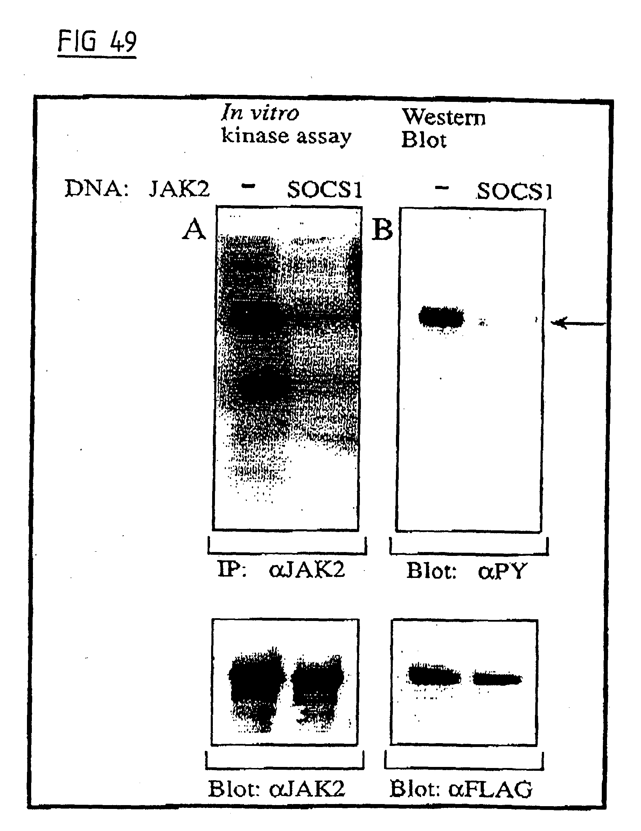

**Figure 49** is a photographic representation showing SOCS1 inhibition of JAK2 kinase activity. (A) Upper panel. Cos M6 cells were transiently transfected with either Flag-tagged mJAK2 and mSOCS-1 DNA (SOCS1) or Flag-mJAK2 DNA alone (-), lysed, JAK2 proteins immunoprecipitated using anti-JAK2 antibody and subjected to an *in vitro* kinase assay. Lower panel. A portion of the JAK2 immunoprecipitates were Western blotted with anti-JAK2 antibody. (B) Upper panel. Cos M6 cells were transiently transfected with Flag- mJAK2 and Flag- mSOCS-1 DNA or Flag-mJAK2 DNA alone, lysed, JAK2 proteins immunoprecipitated using anti-JAK2 (UBI) and separated by SDS/PAGE gel. Immunoprecipitates were then analysed by Western blot with anti-phosphotyrosine antibody. Lower panel; JAK2 expression. Cos cell lysates were separated by SDS/PAGE gel and analysed by Western blot with anti-FLAG antibody (M2).

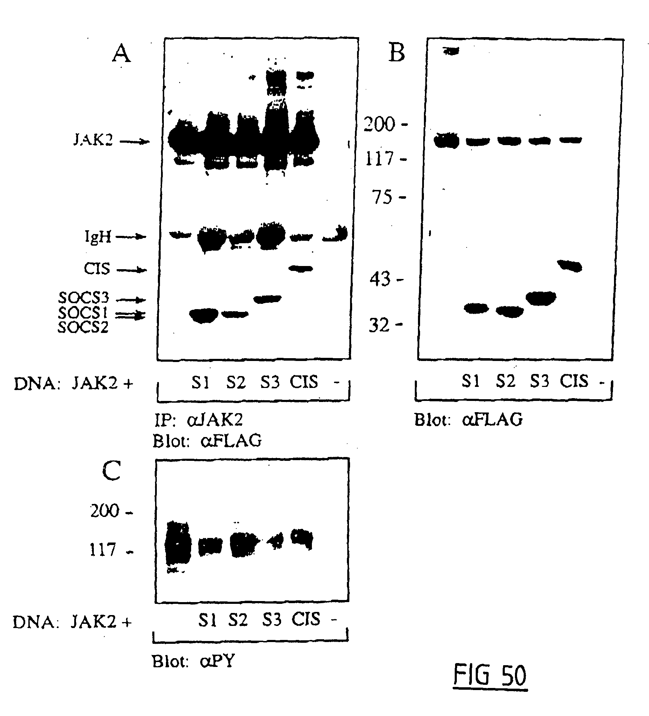

**Figure 50** is a photographic representation showing interaction between JAK2 and SOCS protein. (A) Cos M6 cells were transiently transfected with Flag-tagged mJAK2 and various Flag-tagged SOCS DNAs (SOCS-1;S1, SOCS-2;S2, SOCS-3;S3, CIS) or Flag-mJAK2 alone, lysed, JAK2 proteins immunoprecipitated using anti-JAK2 (UBI) and separated by SDS/PAGE. Immunoprecipitates were then analysed by Western blot with anti-FLAG antibody (M2). (B) Cos cell lysates described in (A) were separated by SDS/PAGE and expression levels of the various proteins were determined by Western blot with anti-FLAG antibody (M2). (C) JAK2 tyrosine phosphorylation. Cos cell lysates described in (A) were separated by SDS/PAGE and proteins analysed by Western blot with anti-phosphotyrosine antibody.

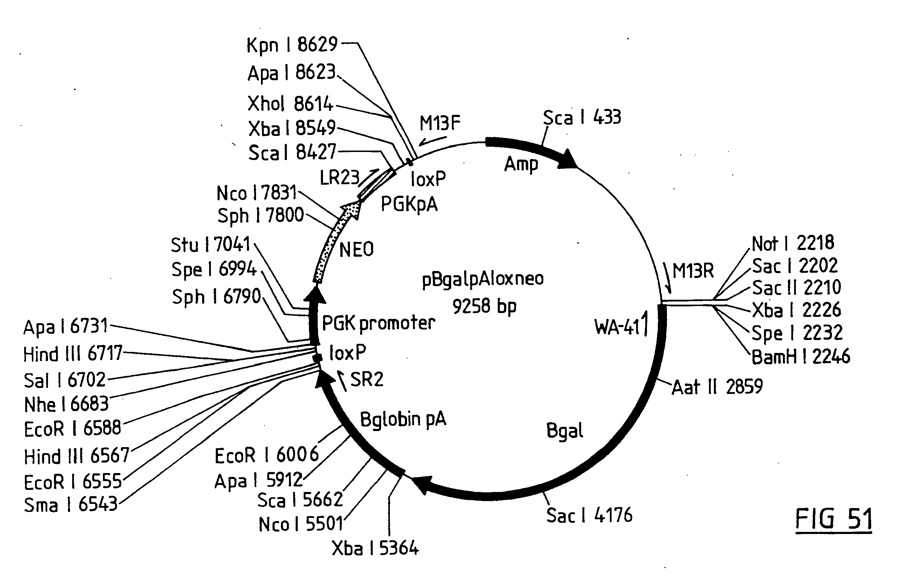

**Figure 51** is a diagrammatic representation of pβgalpAloxneo.

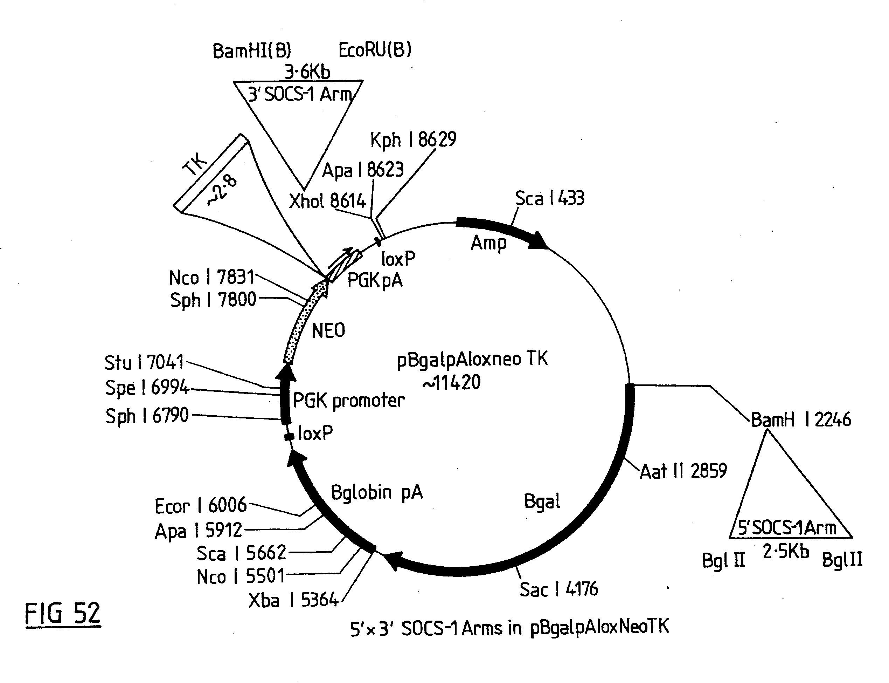

**Figure 52** is a diagrammatic representation of pβgalpAloxneoTK.

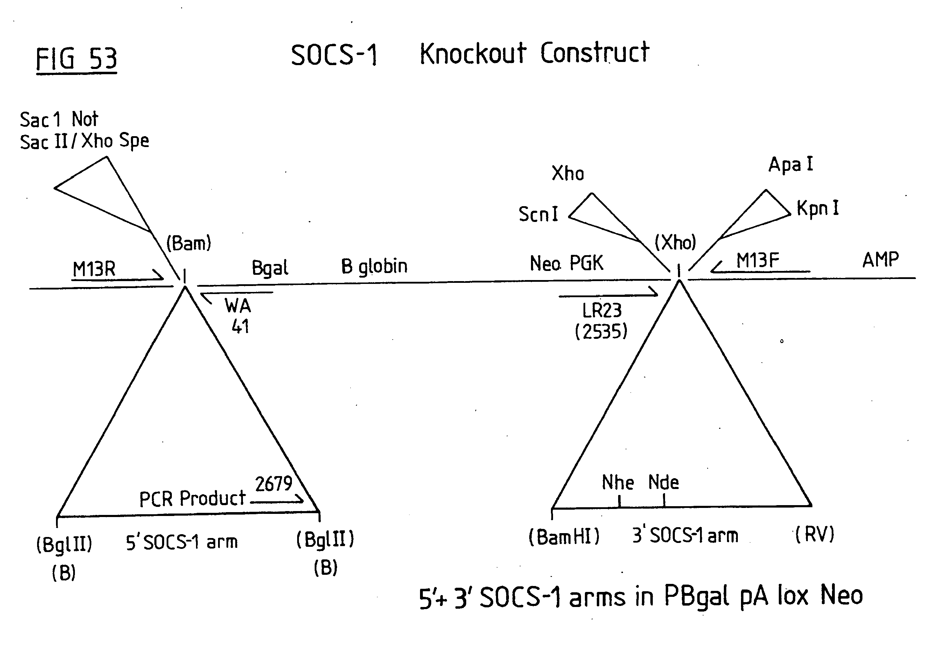

**Figure 53** is a diagrammatic representation of SOCS1 knockout construct.

## DETAILED DESCRIPTION OF PREFERRED EMBODIMENTS

The present invention provides a new family of modulators of signal transduction. As the initial members of this family suppressed cytokine signalling, the family is referred to as the "suppressors of cytokine signalling" family of "SOCS". The SOCS family is defined by the presence of a C-terminal domain referred to as a "SOCS box". Different classes of SOCS molecules are defined by a motif generally but not exclusively located N-terminal to the SOCS box and which is involved by protein:molecule interaction such as protein:DNA or protein:protein interaction. Particularly preferred motifs are selected from an SH2 domain, WD-40 repeats and ankyrin repeats.

WD-40 repeats were originally recognised in the β-subunit of G-proteins. WD-40 repeats appear to form a β-propeller-like structure and may be involved in protein-protein interactions. Ankyrin repeats were originally recognised in the cytoskeletal protein ankryin.

Members of the SOCS family may be identified by any number of means. For example, SOCS1 to SOCS3 were identified by their ability to suppress cytokine-mediated signal transduction and, hence, were identified based on activity. SOCS4 to SOCS15 were identified as nucleotide sequences exhibiting similarity at the level of the SOCS box.

The SOCS box is a conserved motif located in the C-terminal region of the SOCS molecule. In accordance with the present invention, the amino acid sequence of the SOCS box is:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20}$$
$$X_{21} X_{22} X_{23} [Xj]_n X_{24} X_{25} X_{26} X_{27} X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence ofn amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence ofn amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F; and
$X_{28}$ is L, I, V, M, A or P.

As stated above and in accordance with the present invention, SOCS proteins are divided into separate classes based on the presence of a protein:molecule interacting region such as but not limited to an SH2 domain, WD-40 repeats and ankyrin repeats located N-terminal of the SOCS box. The latter three domains are protein:protein interacting domains.

Examples of SH2 containing SOCS proteins include SOCS1, SOCS2, SOCS3, SOCS5, SOCS9, SOCS11 and SOCS14. Examples of SOCS containing WD-40 repeats include SOCS4, SOCS6 and SOCS15. Examples of SOCS containing ankyrin repeats include SOCS7, SOCS10 and SOCS12.

The present invention provides *inter alia* nucleic acid molecules encoding SOCS proteins, purified naturally occurring SOCS proteins as well as recombinant forms of SOCS proteins and methods of modulating signal transduction by modulating activity of SOCS proteins or expression of SOCS genes. Preferably, signal transduction is mediated by a cytokine, examples of which include EPO, TPO, G-CSF, GM-CSF, IL-3, IL-2, IL-4, IL-7, IL-13, IL-6, LIF, IL-12, IFN$\gamma$, TNF$\alpha$, IL-1 and/or M-CSF. Particularly preferred cytokines include IL-6, LIF, OSM, IFN-$\gamma$ and/or thrombopoietin.

Accordingly, one aspect of the present invention provides an isolated nucleic acid molecule comprising a sequence of nucleotides encoding or complementary to a sequence encoding a protein or a derivative, homologue, analogue or mimetic thereof or comprises a nucleotide sequence capable of hybridizing thereto under low stringency conditions at 42°C wherein said protein comprises a SOCS box in its C-terminal region and optionally a protein:molecule interacting domain N-terminal of the SOCS box.

Preferably, the protein:molecule interacting domain is a protein:DNA or protein:protein interacting domain. Most preferably, the protein:molecule interacting domain is one of an SH2 domain, WD-40 repeats and/or ankyrin repeats.

As stated above, preferably the subject SOCS modulate cytokine-mediated signal transduction. The present invention extends, however, to SOCS molecules modulating other effector-mediated signal transduction such as mediated by other endogenous or exogenous molecules, antigens, microbes and microbial products, viruses or components thereof, ions, hormones and parasites. Endogenous molecules in this context are molecules produced within the cell carrying the SOCS molecule. Exogenous molecules are produced by other cells or are introduced to the body.

Preferably, the nucleic acid molecule or SOCS protein is in isolated or purified form. The terms "isolated" and "purified" mean that a molecule has undergone at least one purification step away from other material.

Preferably, the nucleic acid molecule is in isolated form and is DNA such as cDNA or genomic DNA. The DNA may encode the same amino acid sequence as the naturally occurring SOCS or the SOCS may contain one or more amino acid substitutions, deletions and/or additions. The nucleotide sequence may correspond to the genomic coding sequence (including exons and introns) or to the nucleotide sequence in cDNA from mRNA transcribed from the genomic gene or it may carry one or more nucleotide substitutions, deletions and/or additions thereto.

In a preferred embodiment, the nucleic acid molecule comprises a sequence of nucleotide encoding or complementary to a sequence encoding a SOCS protein or a derivative, homologue, analogue or mimetic thereof wherein the amino acid sequence of said SOCS protein is selected from SEQ ID NO:4 (mSOCS1), SEQ ID NO:6 (mSOCS2), SEQ ID NO:8 (mSOCS3), SEQ ID NO:10 (hSOCS1), SEQ ID NO:12 (rSOCS1), SEQ ID NO:14 (mSOCS4), SEQ ID NO:18 (mSOCS5), SEQ ID NO:21 (mSOCS6), SEQ ID NO:25 (mSOCS27), SEQ ID NO:29 (mSOCS8), SEQ ID NO:36 (hSOCS11), SEQ ID NO:41 (mSOCS13), SEQ ID NO:44 (mSOCS14), SEQ ID NO:46 (mSOCS15) and SEQ ID NO:48 (mSOCS15) or encodes an amino acid sequence with a single or multiple amino acid substitution, deletion and/or addition to the listed sequences or is a nucleotide sequence capable of hybridizing to the nucleic acid molecule under low stringency conditions at 42°C.

In an even more preferred embodiment, the present invention provides a nucleic acid molecule comprising a sequence of nucleotides encoding or complementary to a sequence encoding a SOCS protein or a derivative, homologue, analogue or mimetic thereof wherein the nucleotide sequence is selected from a nucleotide sequence substantially set forth in SEQ ID NO:3 (mSOCS1), SEQ ID NO:5 (mSOCS2), SEQ ID NO:7 (mSOCS3), SEQ ID NO:9 (hSOCS11), SEQ ID NO:11 (rSOCS1), SEQ ID NO:13 (mSOCS4), SEQ ID NO:15 and SEQ ID NO:16 (hSOCS4), SEQ ID NO:17 (mSOCS5), SEQ ID NO:19 (hSOCS5), SEQ ID NO:20 (mSOCS6), SEQ ID NO:22 and SEQ ID NO:23 (hSOCS6), SEQ ID NO:24 (mSOCS7), SEQ ID NO:26 and SEQ ID NO:27 (hSOCS7), SEQ ID NO:28 (mSOCS8), SEQ ID NO:30 (mSOCS9), SEQ ID NO:31 (hSOCS9), SEQ ID NO:32 (mSOCS10), SEQ ID NO:33 and SEQ ID NO:34 (hSOCS10), SEQ ID NO:35 (hSOCS11), SEQ ID NO:37 (mSOCS12), SEQ ID NO:38 and SEQ ID NO:39 (hSOCS12), SEQ ID NO:40 (mSOCS13), SEQ ID NO:42 (hSOCS13), SEQ ID NO:43 (mSOCS14), SEQ ID NO:45 (mSOCS15) and SEQ ID NO:47 (hSOCS15) or a nucleotide sequence having at least about 15% siinilarity to all or a region of any of the listed sequences or a nucleic acid molecule capable of hybridizing to any of the listed sequences under low stringency conditions at 42°C.

Reference herein to a low stringency at 42°C includes and encompasses from at least about 1% v/v to at least about 15% v/v formamide and from at least about 1M to at least about 2M salt for hybridisation, and at least about 1M to at least about 2M salt for washing conditions. Alternative stringency conditions may be applied where necessary, such as medium stringency, which includes and encompasses from at least about 16% v/v to at least about 30% v/v formamide and from at least about 0.5M to at least about 0.9M salt for hybridisation, and at least about 0.5M to at least about 0.9M salt for washing conditions, or high stringency, which includes and encompasses from at least about 31% v/v to at least about 50% v/v formamide and from at least about 0.01M to at least about 0.15M salt for hybridisation, and at least about 0.01M to at least about 0.15M salt for washing conditions.

In another embodiment, the present invention is directed to a SOCS protein or a derivative, homologue, analogue or mimetic thereof wherein said SOCS protein is identified as follows:

human SOCS4 characterised by EST81149, EST180909, EST182619, ya99H09, ye70co4, yh53c09, yh77g11, yh87h05, y145h07, yj04e06, yq12h06, yq56a06, yq60e02, yq92g03, yq97h06, yr90f01, yt69c03, yv30a08, yv55f07, yv57h09, yv87h02, yv98e11, yw68d10, yw82a03, yx08a07, yx72h06, yx76b09, yy37h08, yy66b02, za81f08; zb18f07, zc06e08, zd14g06, zd51h12, zd52b09, ze25g11, ze69f02, zf54f03, zh96e07, zv66h12, zs83a08 and zs83g08;

mouse SOCS-4 characterised by mc65f04, mf42e06, mp10c10, mr81g09, and mt19h12;

human SOCS-5 characterised by EST15B103, EST15B105, EST27530 and zf50f01;

mouse SOCS-5 characterised by mc55a01, mh98f09, my26h12 and ve24e06;

human SOCS-6 characterised by yf61e08, yf93a09, yg05f12, yg41f04, yg45c02, yh11f10, yh13b05, zc35a12, ze02h08, z109a03, z169e10, zn39d08 and zo39e06;

mouse SOCS-6 characterised by mc04c05, md48a03, mf31d03, mh26b07, mh78e11, mh88h09, mh94h07, mi27h04 and mj29c05, mp66g04, mw75g03, va53b05, vb34h02, vc55d07, vc59e05, vc67d03, vc68d10, vc97h01, vc99c08, vd07h03, vd08c01, vd09b12, vd 19b02, vd29a04 and vd46d06;

human SOCS-7 characterised by STS WI30171, EST00939, EST12913, yc29b05, yp49f10, zt10f03 and zx73g04;

mouse SOCS-7 characterised by mj39a01 and vi52h07;

mouse SOCS-8 characterised by mj6e09 and vj27a029;

human SOCS-9 characterised by CSRL-82f2-u, EST114054, yy06b07, yy06g06, zr40c09, zr72h01, yx92c08, yx93b08 and hfe0662;

mouse SOCS-9 characterised by me65d05;

human SOCS-10 characterised by aa48h10, zp35h01, zp97h12, zq08h01, zr34g05, EST73000 and HSDHEI005;

mouse SOCS-10 characterised by mb14d12; mb40f06, mg89b11, mq89e12, mp03g12 and vh53c11;

human SOCS-11 characterised by zt24h06 and zr43b02;

human SOCS-13 characterised by EST59161;

mouse SOCS-13 characterised by ma39a09, me60c05, mi78g05, mk10c11, mo48g12, mp94a01, vb57c07 and vh07c11; and

human SOCS-14 characterised by mi75e03, vd29h11 and vd53g07;
or a derivative or homologue of the above ESTs characterised by a nucleic acid molecule being capable of hybridizing to any of the listed ESTs under low stringency conditions at 42°C.

In another embodiment, the nucleotide sequence encodes the following amino acid sequence:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20}$$
$$X_{21} X_{22} X_{23} [Xj]_n X_{24} X_{25} X_{26} X_{27} X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;

$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F; and
$X_{28}$ is L, I, V, M, A or P.

The above sequence comparisons are preferably to the whole molecule but may also be to part thereof. Preferably, the comparisons are made to a contiguous series of at least about 21 nucleotides or at least about 5 amino acids. More preferably, the comparisons are made against at least about 21 contiguous nucleotides or at least 7 contiguous amino acids. Comparisons may also only be made to the SOCS box region or a region encompassing the protein: molecule interacting region such as the SH2 domain WD-40 repeats and/or ankyrin repeats.

Still another embodiment of the present invention contemplates an isolated polypeptide or a derivative, homologue, analogue or mimetic thereof comprising a SOCS box in its C-terminal region.

Preferably the polypeptide further comprises a protein:molecule interacting domain such as a protein:DNA or protein:protein interacting domain. Preferably, this domain is located N-terminal of the SOCS box. It is particularly preferred for the protein:molecule interacting domain to be at least one of an SH2 domain, WD-40 repeats and/or ankyrin repeats.

Preferably, the signal transduction is mediated by a cytokine selected from EPO, TPO, G-CSF, GM-CSF, IL-3, IL-2, IL-4, IL-7, IL-13, IL-6, LIF, IL,-12, IFNγ, TNFα, IL-1 and/or M-CSF. Preferred cytokines are IL-6, LIF, OSM, IFN-γ or thrombopoietin.

More preferably, the protein comprises a SOCS box having the amino acid sequence:

$$X_1 X_2 X_3 X_4 X_5 X_6 X_7 X_8 X_9 X_{10} X_{11} X_{12} X_{13} X_{14} X_{15} X_{16} [X_i]_n X_{17} X_{18} X_{19} X_{20}$$
$$X_{21} X_{22} X_{23} [Xj]_n X_{24} X_{25} X_{26} X_{27} X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W ;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, 1, V, M, A, P, G, C, T or S;
$[X_i]_n$ is a sequence of n amino acids wherein n is from 1 to 50 amino acids and wherein the sequence $X_i$ may comprise

the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F; and
$X_{28}$ is L, I, V, M, A or P.

Still another embodiment provides an isolated polypeptide or a derivative, homologue, analogue or mimetic thereof comprising a sequence of amino acids substantially as set forth in SEQ ID NO:4 (mSOCS1), SEQ ID NO:6 (mSOCS2), SEQ ID NO:8 (mSOCS3), SEQ ID NO:10 (hSOCS1), SEQ ID NO:12 (rSOCS1), SEQ ID NO:14 (mSOCS4), SEQ ID NO:18 (mSOCS5), SEQ ID NO:21 (mSOCS6), SEQ ID NO:25 (mSOCS7), SEQ ID NO:29 (mSOCS8), SEQ ID NO:36 (hSOCS11), SEQ ID NO:41 (mSOCS13), SEQ ID NO:44 (mSOCS14), SEQ ID NO:46 (mSOCS15) and SEQ ID NO:48 (hSOCS15) or an amino acid sequence having at least 15% similarity to all or a part of the listed sequences.
Preferred nucleotide percentage similarities include at least about 20%, at least about 40%, at least about 50%, at least about 60%, at least about 70%, at least about 80%, at least about 90% or above such as 93%, 95%, 98% or 99%.
Preferred amino acid similarities include at least about 20%, at least about 30%, at least about 40%, at least about 50%, at least about 60%, at least about 70%, at least about 80%, at least about 90%, at least about 95%, at least about 97% or 98% or above.
As stated above, similarity may be measured against an entire molecule or a region comprising at least 21 nucleotides or at least 7 amino acids. Preferably, similarity is measured in a conserved region such as SH2 domain, WD-40 repeats, ankyrin repeats or other protein:molecule interacting domains or a SOCS box.
The term "similarity" includes exact identity between sequences or, where the sequence differs, different amino acids are related to each other at the structural, functional, biochemical and/or conformational levels.
The nucleic acid molecule may be isolated from any animal such as humans, primates, livestock animals (e.g. horses, cows, sheep, donkeys, pigs), laboratory test animals (e.g. mice, rats, rabbits, hamsters, guinea pigs), companion animals (e.g. dogs, cats) or captive wild animals (e.g. deer, foxes, kangaroos).
The terms "derivatives" or its singular form "derivative" whether in relation to a nucleic acid molecule or a protein includes parts, mutants, fragments and analogues as well as hybrid or fusion molecules and glycosylation variants. Particularly useful derivatives comprise single or multiple amino acid substitutions, deletions and/or additions to the SOCS amino acid sequence.
Preferably, the derivatives have functional activity or alternatively act as antagonists or agonists. The present invention further extends to homologues of SOCS which include the functionally or structurally related molecule from different animal species. The present invention also encompasses analogues and mimetics. Mimetics include a class of molecule generally but not necessarily having a non-amino acid structure and which functionally are capable of acting in an analogous manner to the protein for which it is a mimic, in this case, a SOCS. Mimetics may comprise a carbohydrate, aromatic ring, lipid or other complex chemical structure or may also be proteinaceous in composition. Mimetics as well as agonists and antagonists contemplated herein are conveniently located through systematic searching of environments, such as coral, marine and freshwater river beds, flora and microorganisms. This is sometimes referred to as natural product screening. Alternatively, libraries of synthetic chemical compounds may be screened for potentially useful molecules.
As stated above, the present invention contemplates agonists and antagonists of the SOCS. One example of an antagonist is an antisense oligonucleotide sequence. Useful oligonucleotides are those which have a nucleotide sequence complementary to at least a portion of the protein-coding or "sense" sequence of the nucleotide sequence. These anti-sense nucleotides can be used to effect the specific inhibition of gene expression. The antisense approach can cause inhibition of gene expression apparently by forming an anti-parallel duplex by complementary base pairing between the antisense construct and the targeted mRNA, presumably resulting in hybridisation arrest of translation. Ribozymes and co-suppression molecules may also be used. Antisense and other nucleic acid molecules may first need to be chemically modified to permit penetration of cell membranes and/or to increase their serum half life or otherwise make them more stable for in vivo administration. Antibodies may also act as either antagonists or agonists although are more useful in diagnostic applications or in the purification of SOCS proteins. Antagonists and agonists may also be identified following natural product screening or screening of libraries of chemical compounds or may be derivatives or analogues of the SOCS molecules.

Accordingly, the present invention extends to analogues of the SOCS proteins of the present invention. Analogues may be used, for example, in the treatment or prophylaxis of cytokine mediated dysfunction such as autoimmunity, immune suppression or hyperactive immunity or other condition including but not limited to dysfunctions in the haemopoietic, endocrine, hepatic and neural systems. Dysfunctions mediated by other signal transducing elements such as hormones or endogenous or exogenous molecules, antigens, microbes and microbial products, viruses or components thereof, ions, hormones and parasites are also contemplated by the present invention.

Analogues of the proteins contemplated herein include, but are not limited to, modification to side chains, incorporating of unnatural amino acids and/or their derivatives during peptide, polypeptide or protein synthesis and the use of crosslinkers and other methods which impose conformational constraints on the proteinaceous molecule or their analogues.

Examples of side chain modifications contemplated by the present invention include modifications of amino groups such as by reductive alkylation by reaction with an aldehyde followed by reduction with $NaBH_4$; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with $NaBH_4$.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides.

Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 3.

**TABLE 3**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | | Chexa L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |

(continued)

| Non-conventional amino acid | Code | Non-conventional amino acid | Code |
|---|---|---|---|
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile. | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |

(continued)

| Non-conventional amino acid | Code | Non-conventional amino acid | Code |
|---|---|---|---|
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-t-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) carbamylmethyl) glycine | Nnbhm | N-(N-(3,3-diphenylpropyl) carbamylmethyl) glycine | Nnbhe |
| 1-carboxy-1-(2,2-diphenylethylamino) cyclopropane | Nmbc | | |

Crosslinkers can be used, for example, to stabilise 3D conformations, using homo-bifunctional crosslinkers such as the bifunctional imido esters having $(CH_2)_n$ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of $C_\alpha$ and $N_\alpha$-methylamino acids, introduction of double bonds between $C_\alpha$ and $C_\beta$ atoms of amino acids and the formation of cyclic peptides or analogues by introducing covalent bonds such as forming an amide bond between the N and C termini, between two side chains or between a side chain and the N or C terminus.

These types of modifications may be important to stabilise the cytokines if administered to an individual or for use as a diagnostic reagent.

Other derivatives contemplated by the present invention include a range of glycosylation variants from a completely unglycosylated molecule to a modified glycosylated molecule. Altered glycosylation patterns may result from expression of recombinant molecules in different host cells.

Another embodiment of the present invention contemplates a method for modulating expression of a SOCS protein in a mammal, said method comprising contacting a gene encoding a SOCS or a factor/element involved in controlling expression of the SOCS gene with an effective amount of a modulator of SOCS expression for a time and under conditions sufficient to up-regulate or down-regulate or otherwise modulate expression of SOCS. An example of a modulator is a cytokine such as IL-6 or other transcription regulators of SOCS expression.

Expression includes transcription or translation or both.

Another aspect of the present invention contemplates a method of modulating activity of SOCS in a human, said method comprising administering to said mammal a modulating effective amount of a molecule for a time and under conditions sufficient to increase or decrease SOCS activity. The molecule may be a proteinaceous molecule or a chemical entity and may also be a derivative of SOCS or a chemical analogue or truncation mutant of SOCS.

A further aspect of the present invention provides a method of inducing synthesis of a SOCS or transcription/ translation of a SOCS comprising contacting a cell containing a SOCS gene with an effective amount of a cytokine capable of inducing said SOCS for a time and under conditions sufficient for said SOCS to be produced. For example, SOCS1 may be induced by IL-6.

Still a further aspect of the present invention contemplates a method of modulating levels of a SOCS protein in a cell said method comprising contacting a cell containing a SOCS gene with an effective amount of a modulator of SOCS gene expression or SOCS protein activity for a time and under conditions sufficient to modulate levels of said SOCS protein.

Yet a further aspect of the present invention contemplates a method of modulating signal transduction in a cell containing a SOCS gene comprising contacting said cell with an effective amount of a modulator of SOCS gene expression or SOCS protein activity for a time sufficient to modulate signal transduction.

Even yet a further aspect of the present invention contemplates a method of influencing interaction between cells wherein at least one cell carries a SOCS gene, said method comprising contacting the cell carrying the SOCS gene with an effective amount of a modulator of SOCS gene expression or SOCS protein activity for a time sufficient to modulate signal transduction.

As stated above, of the present invention contemplates a range of mimetics or small molecules capable of acting as agonists or antagonists of the SOCS. Such molecules may be obtained from natural product screening such as from coral, soil, plants or the ocean or antarctic environments. Alternatively, peptide, polypeptide or protein libraries or chemical libraries may be readily screened. For example, M1 cells expressing a SOCS do not undergo differentiation in the presence of IL-6. This system can be used to screen molecules which permit differentiation in the presence of IL-6 and a SOCS. A range of test cells may be prepared to screen for antagonists and agonists for a range of cytokines. Such molecules are preferably small molecules and may be of amino acid origin or of chemical origin. SOCS molecules interacting with signalling proteins (eg. JAKS) provide molecular screens to detect molecules which interfere or promote this interaction. Once such screening protocol involves natural product screening.

Accordingly, the present invention contemplates a pharmaceutical composition comprising SOCS or a derivative thereof or a modulator of SOCS expression or SOCS activity and one or more pharmaceutically acceptable carriers and/or diluents. These components are referred to as the "active ingredients". These and other aspects of the present invention apply to any SOCS molecules such as but not limited to SOCS1 to SOCS15.

The pharmaceutical forms containing active ingredients suitable for injectable use include sterile aqueous solutions (where water soluble) sterile powders for the extemporaneous preparation of sterile injectable solutions. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as licithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thirmerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets. For oral therapeutic administration, the active compound may be incorporated with excipients and used in the form of ingestible tablets, buccal tablets, troches, capsules, elixirs, suspensions, syrups, wafers and the like. Such compositions and preparations should contain at least 1 % by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 0.1 $\mu$g and 2000 mg of active compound.

The tablets, troches, pills, capsules and the like may also contain the components as listed hereafter. A binder such as gum, acacia, corn starch or gelatin; excipients such as dicalcium phosphate; a disintegrating agent such as corn starch, potato starch, alginic acid and the like; a lubricant such as magnesium stearate; and a sweetening agent such a sucrose, lactose or saccharin may be added or a flavouring agent such as peppermint, oil of wintergreen or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

The present invention also extends to forms suitable for topical application such as creams, lotions and gels.

Pharmaceutically acceptable carriers and/or diluents include any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 0.5 μg to about 2000 mg. Expressed in proportions, the active compound is generally present in from about 0.5 μg to about 2000 mg/ml of carrier. In the case of compositions containing supplementary active ingredients, the dosages are determined by reference to the usual dose and manner of administration of the said ingredients. The effective amount may also be conveniently expressed in terms of an amount per kg of body weight. For example, from about 0.01 ng to about 10,000 mg/kg body weight may be administered.

The pharmaceutical composition may also comprise genetic molecules such as a vector capable of transfecting target cells where the vector carries a nucleic acid molecule capable of modulating SOCS expression or SOCS activity. The vector may, for example, be a viral vector. In this regard, a range of gene therapies are contemplated by the present invention including isolating certain cells, genetically manipulating and returning the cell to the same subject or to a genetically related or similar subject.

Still another aspect of the present invention is directed to antibodies to SOCS and its derivatives. Such antibodies may be monoclonal or polyclonal and may be selected from naturally occurring antibodies to SOCS or may be specifically raised to SOCS or derivatives thereof. In the case of the latter, SOCS or its derivatives may first need to be associated with a carrier molecule. The antibodies and/or recombinant SOCS or its derivatives of the present invention are particularly useful as therapeutic or diagnostic agents.

For example, SOCS and its derivatives can be used to screen for naturally occurring antibodies to SOCS. These may occur, for example in some autoimmune diseases. Alternatively, specific antibodies can be used to screen for SOCS. Techniques for such assays are well known in the art and include, for example, sandwich assays and ELISA. Knowledge of SOCS levels may be important for diagnosis of certain cancers or a predisposition to cancers or monitoring cytokine mediated cellular responsiveness or for monitoring certain therapeutic protocols.

Antibodies to SOCS of the present invention may be monoclonal or polyclonal. Alternatively, fragments of antibodies may be used such as Fab fragments. Furthermore, the present invention extends to recombinant and synthetic antibodies and to antibody hybrids. A "synthetic antibody" is considered herein to include fragments and hybrids of antibodies. The antibodies of this aspect of the present invention are particularly useful for immunotherapy and may also be used as a diagnostic tool for assessing apoptosis or monitoring the program of a therapeutic regimin.

For example, specific antibodies can be used to screen for SOCS proteins. The latter would be important, for example, as a means for screening for levels of SOCS in a cell extract or other biological fluid or purifying SOCS made by recombinant means from culture supernatant fluid. Techniques for the assays contemplated herein are known in the art and include, for example, sandwich assays and ELISA.

It is within the scope of this invention to include any second antibodies (monoclonal, polyclonal or fragments of antibodies or synthetic antibodies) directed to the first mentioned antibodies discussed above. Both the first and second antibodies may be used in detection assays or a first antibody may be used with a commercially available anti-immunoglobulin antibody. An antibody as contemplated herein includes any antibody specific to any region of SOCS.

Both polyclonal and monoclonal antibodies are obtainable by immunization with the enzyme or protein and either type is utilizable for immunoassays. The methods of obtaining both types of sera are well known in the art. Polyclonal sera are less preferred but are relatively easily prepared by injection of a suitable laboratory animal with an effective amount of SOCS, or antigenic parts thereof, collecting serum from the animal, and isolating specific sera by any of the known immunoadsorbent techniques. Although antibodies produced by this method are utilizable in virtually any type of immunoassay, they are generally less favoured because of the potential heterogeneity of the product.

The use of monoclonal antibodies in an immunoassay is particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art.

Another aspect of the present invention contemplates a method for detecting SOCS in a biological sample from a subject said method comprising contacting said biological sample with an antibody specific for SOCS or its derivatives or homologues for a time and under conditions sufficient for an antibody-SOCS complex to form and then detecting said complex.

The presence of SOCS may be accomplished in a number of ways such as by Western blotting and ELISA procedures. A wide range of immunoassay techniques are available as can be seen by reference to US Patent Nos. 4,016,043, 4, 424,279 and 4,018,653. These, of course, include both single-site and two-site or "sandwich" assays of the non-competitive types, as well as in the traditional competitive binding assays. These assays also include direct binding of a labelled antibody to a target.

Sandwich assays are among the most useful and commonly used assays and are favoured for use in the present invention. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of hapten. Variations on the forward assay include a simultaneous assay, in which both sample and labelled antibody are added simultaneously to the bound antibody. These techniques are well known to those skilled in the art, including any minor variations as will be readily apparent. In accordance with the present invention the sample is one which might contain SOCS including cell extract, tissue biopsy or possibly serum, saliva, mucosal secretions, lymph, tissue fluid and respiratory fluid. The sample is, therefore, generally a biological sample comprising biological fluid but also extends to fermentation fluid and supernatant fluid such as from a cell culture.

In the typical forward sandwich assay, a first antibody having specificity for the SOCS or antigenic parts thereof, is either covalently or passively bound to a solid surface. The solid surface Is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. room temperature to 37°C) to allow binding of any subunit present in the antibody. Following the incubation period, the antibody subunit solid phase is washed and dried and incubated with a second antibody specific for a portion of the hapten. The second antibody is linked to a reporter molecule which is used to indicate the binding of the second antibody to the hapten.

An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable colour change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of hapten which was present in the sample. "Reporter molecule" also extends to use of cell agglutination or inhibition of agglutination such as red blood cells on latex beads, and the like.

Alternately, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescene and EIA techniques are both very well established in the art and are particularly preferred for the present method. However, other reporter molecules, such as radioisotope, chemiluminescent or bioluminescent molecules, may also be employed.

The present invention also contemplates genetic assays such as involving PCR analysis to detect SOCS gene or its derivatives. Alternative methods or methods used in conjunction include direct nucleotide sequencing or mutation scanning such as single stranded conformation polymorphisms analysis (SSCP) as specific oligonucleotide hybridisation, as methods such as direct protein truncation tests.

Since cytokines are involved in transcription of some SOCS molecules, the detection of SOCS provides surrogate markers for cytokines or cytokine activity. This may be useful in assessing subjects with a range of conditions such as those will autoimmune diseases, for example, rheumatoid arthritis, diabetes and stiff man syndrome amongst others.

The nucleic acid molecules of the present invention may be DNA or RNA. When the nucleic acid molecule is in DNA form, it may be genomic DNA or cDNA. RNA forms of the nucleic acid molecules of the present invention are generally mRNA.

Although the nucleic acid molecules of the present invention are generally in isolated form, they may be integrated into or ligated to or otherwise fused or associated with other genetic molecules such as vector molecules and in particular expression vector molecules. Vectors and expression vectors are generally capable of replication and, if applicable, expression in one or both of a prokaryotic cell or a eukaryotic cell. Preferably, prokaryotic cells include *E. coli, Bacillus sp* and *Pseudomonas sp.* Preferred eukaryotic cells include yeast, fungal, mammalian and insect cells.

Accordingly, another aspect of the present invention contemplates a genetic construct comprising a vector portion and a mammalian and more particularly a human SOCS gene portion, which SOCS gene portion is capable of encoding a SOCS polypeptide or a functional or immunologically interactive derivative thereof.

Preferably, the SOCS gene portion of the genetic construct is operably linked to a promoter on the vector such that said promoter is capable of directing expression of said SOCS gene portion in an appropriate cell.

In addition, the SOCS gene portion of the genetic construct may comprise all or part of the gene fused to another genetic sequence such as a nucleotide sequence encoding glutathione-S-transferase or part thereof.

The present invention extends to such genetic constructs and to prokaryotic or eukaryotic cells comprising same.

The present invention also extends to any or all derivatives of SOCS including mutants, part, fragments, portions, homologues and analogues or their encoding genetic sequence including single or multiple nucleotide or amino acid substitutions, additions and/or deletions to the naturally occurring nucleotide or amino acid sequence. The present invention also extends to mimetics and agonists and antagonists of SOCS.

The SOCS and its genetic sequence of the present invention will be useful in the generation of a range of therapeutic and diagnostic reagents and will be especially useful in the detection of a cytokine involved in a particular cellular response or a receptor for that cytokine. For example, cells expressing SOCS gene such as M1 cells expressing the SOCS1 gene, will no longer be responsive to a particular cytokine such as, in the case of SOCS1, IL-6. Clearly, the present invention further contemplates cells such as M1 cells expressing any SOCS gene such as from SOCS1 to SOCS15. Furthermore, the present invention provides the use of molecules that regulate or potentiate the ability of therapeutic cytokines. For example, molecules which block some SOCS activity, may act to potential therapeutic cytokine activity (eg. G-CSF).

Soluble SOCS polypeptides are also contemplated to be particularly useful in the treatment of disease, injury or abnormality involving cytokine mediated cellular responsiveness such as hyperimmunity, immunosuppression, allergies, hypertension and the like.

A further aspect of the present invention contemplates the use of SOCS or its functional derivatives in the manufacture of a medicament for the treatment of conditions involving cytokine mediated cellular responsiveness.

The present invention further contemplates transgenic mammalian cells expressing a SOCS gene. Such cells are useful indicator cell lines for assaying for suppression of cytokine function. One example is M1 cells expressing a SOCS gene. Such cell lines may be useful for screening for cytokines or screening molecules such as naturally occurring molecules from plants, coral, microorganisms or bio-organically active soil or water capable of acting as cytokine antagonists or agonists.

The present invention further contemplates hybrids between different SOCS from the same or different animal species. For example, a hybrid may be formed between all or a functional part of mouse SOCS1 and human SOCS1. Alternatively, the hybrid may be between all or part of mouse SOCS1 and mouse SOCS2. All such hybrids are contemplated herein and are particularly useful in developing pleiotropic molecules.

The present invention further contemplates a range of genetic based diagnostic assays screening for individuals with defective SOCS genes. Such mutations may result in cell types not being responsive to a particular cytokine or resulting in over responsiveness leading to a range of conditions. The SOCS genetic sequence can be readily verified using a range of PCR or other techniques to determine whether a mutation is resident in the gene. Appropriate gene therapy or other interventionist therapy may then be adopted.

Preferred embodiments of the invention include:

1. A nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein comprises a SOCS box in its C-terminal region:

2. A nucleic acid molecule according to embodiment 1 wherein the protein further comprises a protein:molecule interacting region;

3. A nucleic acid molecule according to embodiment 1 wherein the protein :molecule interacting region is located in a region N-terminal of the SOCS box;

4. A nucleic acid molecule according to embodiment 2 or embodiment 3 wherein the protein:molecule interacting region is a protein:DNA binding region or a protein:protein binding region;

5. A nucleic acid molecule according to embodiment 4 wherein the protein:molecule interacting region is one or more of an SH2 domain, WD-40 repeats or ankyrin repeats;

6. A nucleic acid molecule according to any one of embodiments 1-5 wherein the SOCS box comprises the amino acid sequence:

$$X_1\ X_2\ X_3\ X_4\ X_5\ X_6\ X_7\ X_8\ X_9\ X_{10}\ X_{11}\ X_{12}\ X_{13}\ X_{14}\ X_{15}\ X_{16}\ [X_i]_n\ X_{17}\ X_{18}\ X_{19}\ X_{20}\ X_{21}\ X_{22}\ X_{23}\ [Xj]_n\ X_{24}\ X_{25}\ X_{26}\ X_{27}\ X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_1$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;

$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F; and
$X_{28}$ is L, I, V, M, A or P;

7. A nucleic acid molecule according to embodiment 6 wherein the protein modulates signal transduction;

8. A nucleic acid molecule according to embodiment 7 wherein the signal transduction is modulated by a cytokine or a hormone, a microbe or a microbial product, a parasite, an antigen or other effector molecule;

9. A nucleic acid molecule according to embodiment 8 wherein the protein modulates cytokine-mediated signal transduction;

10. A nucleic acid molecule according to embodiment 9 wherein the signal transduction is mediated by one or more of the cytokines EPO, TPO, G-CSF, GM-CSF, IL-3, IL-2, IL-4, IL-7, IL-13, IL-6, LIF, IL-12, IFNγ, TNFα, IL-1 and/or M-CSF;

11. A nucleic acid molecule according to embodiment 10 wherein the signal transduction is mediated by one or more of IL-6, LIF, OSM, IFN-γ and/or thrombopoietin;

12. A nucleic acid molecule according to embodiment 11 wherein the signal transduction is mediated by IL-6;

13. A nucleic acid molecule according to embodiment 1 wherein the nucleotide sequence encodes an amino acid sequence substantially as set forth in SEQ ID NO. 4, SEQ ID NO. 6, SEQ ID NO. 8, SEQ ID NO. 10, SEQ ID NO. 12, SEQ ID NO. 14, SEQ ID NO. 18, SEQ ID NO. 21, SEQ ID NO. 25, SEQ ID NO. 29, SEQ ID NO. 36, SEQ ID NO. 41, SEQ ID NO. 44, SEQ ID NO. 46 or SEQ ID NO. 48 or an amino acid sequence having at least about 15% similarity to all or part of the listed sequences or a nucleotide sequence which hybridizes to the nucleic acid molecule under low stringency conditions at 42°C;

14. A nucleic acid molecule according to embodiment 1 wherein the nucleotide sequence is substantially as set forth in SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 16, SEQ ID NO. 17, SEQ ID NO. 20, SEQ ED NO. 22, SEQ ID NO. 23, SEQ ID NO. 24, SEQ ID NO. 26, SEQ ID NO. 27, SEQ ID NO. 28, SEQ ID NO. 30, SEQ ID NO. 31, SEQ ID NO. 32, SEQ ID NO. 33, SEQ ID NO. 34, SEQ ID NO. 35, SEQ ID NO. 37, SEQ ID NO. 38, SEQ ID NO. 39, SEQ ID NO. 40, SEQ ID NO. 42, SEQ ID NO. 43, SEQ ID NO. 45 or SEQ ID NO. 47 or a nucleotide sequence laving at least 15% similarity to all or a part of the listed sequences or a nucleotide sequence capable of hybridizing to the listed sequences under low stringency conditions at 42°C;

15. A nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein exhibits the following characteristics:

(i) comprises a SOCS box in its C-terminal region wherein said SOCS box comprises the amino acid sequence:

$$X_1\ X_2\ X_3\ X_4\ X_5\ X_6\ X_7\ X_8\ X_9\ X_{10}\ X_{11}\ X_{12}\ X_{13}\ X_{14}\ X_{15}\ X_{16}\ [X_i]_n\ X_{17}\ X_{18}\ X_{19}\ X_{20}\ X_{21}\ X_{22}\ X_{23}\ [Xj]_n\ X_{24}\ X_{25}\ X_{26}\ X_{27}\ X_{28}$$

wherein:

$X_1$ is L, I, V, M, A or P;
$X_2$ is any amino acid residue;
$X_3$ is P, T or S;
$X_4$ is L,I,V,M,A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G; $X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F;
$X_{28}$ is L, I, V, M, A or P; and

(ii) comprises at least one of an SH2 domain, WD-40 repeats and/or ankyrin repeats or other protein:molecule interacting domain in a region N-terminal of the SOCS box; and
(iii) modulates signal transduction;

16. An isolated protein or a derivative, homologue or mimetic thereof comprising a SOCS box in its C-terminal region;

17. An isolated protein according to embodiment 16 wherein the protein further comprises a protein:molecule interacting region;

18. An isolated protein according to embodiment 17 wherein the protein:molecule interacting region is located in a region N-terminal of the SOCS box;

19. An isolated protein according to embodiment 16 or embodiment 17 wherein the protein:molecule interacting region is a protein:DNA binding region or a protein:protein binding region;

20. An isolated protein according to embodiment 19 wherein the protein:molecule interacting region is one or more of an SH2 domain, WD-40 repeats or ankyrin repeats;

21. An isolated protein according to any one of embodiments 16-20 wherein the SOCS box comprises the amino acid sequence:

$$X_1\ X_2\ X_3\ X_4\ X_5\ X_6\ X_7\ X_8\ X_9\ X_{10}\ X_{11}\ X_{12}\ X_{13}\ X_{14}\ X_{15}\ X_{16}\ [X_i]_n\ X_{17}\ X_{18}\ X_{19}\ X_{20}\ X_{21}\ X_{22}\ X_{23}\ [Xj]_n\ X_{24}\ X_{25}\ X_{26}\ X_{27}\ X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F; and
$X_{28}$ is L, I, V, M, A or P;

22. An isolated protein according to embodiment 21 wherein the protein modulates signal transduction;

23. An isolated protein according to embodiment 22 wherein the signal transduction is modulated by a cytokine or other endogenous molecule, a hormone, a microbe or a microbial product, a parasite, an antigen or other effector molecule;

24. An isolated protein according to embodiment 23 wherein the protein modulates cytokine-mediated signal transduction;

25. An isolated protein according to embodiment 24 wherein the signal transduction is mediated by one or more of the cytokines EPO, TPO, G-CSF, GM-CSF, IL-3, IL-2, IL-4, IL-7, IL-13, IL-6, LIF, IL-12, IFNγ, TNFα, IL-1 and/or M-CSF;

26. An isolated protein according to embodiment 25 wherein the signal transduction is mediated by one or more of IL-6, LIF, OSM, IFN-γ and/or thrombopoietin;

27. An isolated protein according to embodiment 26 wherein the signal transduction is mediated by IL-6;

28. An isolated protein according to embodiment 16 wherein said protein comprises an amino acid sequence substantially as set forth in SEQ ID NO. 4, SEQ ID NO. 6, SEQ ID NO. 8, SEQ ID NO 10, SEQ ID NO. 12, SEQ ID NO. 14, SEQ ID NO. 18, SEQ ID NO. 21, SEQ ID NO. 25, SEQ ID NO. 29, SEQ ID NO. 36, SEQ ID NO. 41, SEQ ID NO. 44, SEQ ID NO. 46 or SEQ ID NO. 48 or an amino acid sequence having at least about 15% similarity to all or part of the listed sequences;

29. An isolated protein according to embodiment 16 wherein the said protein is encoded by a nucleotide sequence substantially as set forth in SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 16, SEQ ID NO. 17, SEQ ID NO. 20, SEQ ID NO. 22, SEQ ID NO. 23, SEQ ID NO. 24, SEQ ID NO. 26, SEQ ID NO. 27, SEQ ID NO. 28, SEQ ID NO. 30, SEQ ID NO. 31, SEQ ID NO. 32, SEQ ID NO. 33, SEQ ID NO. 34, SEQ ID NO. 35, SEQ ID NO. 37, SEQ ID NO. 38, SEQ ID NO. 39, SEQ ID NO. 40, SEQ ID NO. 42, SEQ ID NO. 43, SEQ ID NO. 45 or SEQ ID NO. 47 or a nucleotide sequence having at least 15% similarity to all or a part of the listed sequences or a nucleotide sequence capable of hybridizing to the listed sequences under low stringency conditions at 42°C;

30. An isolated protein or a derivative, homologue, analogue or mimetic thereof having the following characteristics:

(i) comprises a SOCS box in its C-terminal region wherein said SOCS box comprises the amino acid sequence:

$$X_1\ X_2\ X_3\ X_4\ X_5\ X_6\ X_7\ X_8\ X_9\ X_{10}\ X_{11}\ X_{12}\ X_{13}\ X_{14}\ X_{15}\ X_{16}\ [X_i]_n\ X_{17}\ X_{18}$$
$$X_{19}\ X_{20}\ X_{21}\ X_{22}\ X_{23}\ [Xj]_n\ X_{24}\ X_{25}\ X_{26}\ X_{27}\ X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F;

$X_{28}$ is L, I, V, M, A or P; and

(ii) comprises at least one of an SH2 domain, WD-40 repeats and/or ankyrin repeats or other protein:molecule interacting domain in a region N-terminal of the SOCS box; and
(iii) modulates signal transduction;

31. A method of modulating levels of a SOCS protein in a cell said method comprising contacting a cell containing a SOCS gene with an effective amount of a modulator of SOCS gene expression or SOCS protein activity for a time and under conditions sufficient to modulate levels of said SOCS protein;

32. A method of modulating signal transduction in a cell containing a SOCS gene comprising contacting said cell with an effective amount of a modulator of SOCS gene expression or SOCS protein activity for a time sufficient to modulate signal transduction;

33. A method of influencing interaction between cells wherein at least one cell carries a SOCS gene, said method comprising contacting the cell carrying the SOCS gene with an effective amount of a modulator of SOCS gene expression or SOCS protein activity for a time sufficient to modulate signal transduction;

34. A method according to any one of embodiments 31-33 wherein signal transduction is mediated by a cytokine, a hormone, a microbe or a microbial product, a parasite, an antigen or other effector molecule;

35. A method according to embodiment 34 wherein the cytokine is one or more of EPO, TPO, G-CSF, GM-CSF, IL-3, IL-2, IL-4, IL-7, IL-13, IL-6, LIF, IL-12, IFN$\gamma$, TNF$\alpha$, IL-1 and/or M-CSF;

36. A method according to embodiment 35 wherein the cytokine is one or more of IL-6, LIF, OSM, IFN-$\gamma$ and/or thrombopoietin;

37. A method according to embodiment 36 wherein the cytokine is IL-6;

38. A method according to any one of embodiments 31-37 wherein the SOCS gene encodes a protein having a SOCS box comprising the amino acid sequence:

$$X_1\ X_2\ X_3\ X_4\ X_5\ X_6\ X_7\ X_8\ X_9\ X_{10}\ X_{11}\ X_{12}\ X_{13}\ X_{14}\ X_{15}\ X_{16}\ [X_i]_n\ X_{17}\ X_{18}\ X_{19}$$
$$X_{20}\ X_{21}\ X_{22}\ X_{23}\ [Xj]_n\ X_{24}\ X_{25}\ X_{26}\ X_{27}\ X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$ may comprise the same or different amino acids selected from any amino acid residue;
$X_{17}$ is L, I, V, M, A or P;

$X_{18}$ is any amino acid;
$X_1$ is any amino acid;
$X_{20}$ L, I, V, M, A or P;
$X_{21}$ is P;
$X_{22}$ is L, I, V, M, A, P or G;
$X_{23}$ is P or N;
$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F; and
$X_{28}$ is L, I, V, M, A or P;

39. A method according to embodiment 38 wherein the SOCS gene comprises a nucleotide sequence selected from SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 16, SEQ ID NO. 17, SEQ ID NO. 20, SEQ ID NO. 22, SEQ ID NO. 23, SEQ ID NO. 24, SEQ ID NO. 26, SEQ ID NO. 27, SEQ ID NO. 28, SEQ ID NO. 30, SEQ ID NO. 31, SEQ ID NO. 32, SEQ ID NO. 33, SEQ ID NO. 34, SEQ ID NO. 35, SEQ ID NO. 37, SEQ ID NO. 38, SEQ ID NO. 39, SEQ ID NO. 40, SEQ ID NO. 42, SEQ ID NO. 43, SEQ ID NO. 45 or SEQ ID NO. 47;

40. A method according to embodiment 38 wherein the SOCS gene encodes a protein comprising an amino acid sequence substantially as set forth in SEQ ID NO. 4, SEQ ID NO. 6, SEQ ID NO. 8, SEQ ID NO. 10, SEQ ID NO. 12, SEQ ID NO. 14, SEQ ID NO. 18, SEQ ID NO. 21, SEQ ID NO. 25, SEQ ID NO. 29, SEQ ID NO. 36, SEQ ID NO. 41, SEQ ID NO. 44, SEQ ID NO. 46 or SEQ ID NO. 48.

The present invention is further described by the following non-limiting Examples.

Examples 1-16 relate to SOCS1, SOCS2 and SOCS3 which were identified on the basis of activity. Examples 17-24 relate to various aspects of SOCS4 to SOCS15 which were cloned initially on the basis of sequence similarity. Examples 25-36 relate to specific aspects of SOCS4 to SOCS15, respectively.

**EXAMPLE 1**

**CELL CULTURE AND CYTOKINES**

The M1 cell line was derived from a spontaneously arising leukaemia in SL mice [Ichikawa, 1969]. Parental M1 cells used in this study have been in passage at the Walter and Eliza Hall Institute for Medical Research, Melbourne, Victoria, Australia, for approximately 10 years. M1 cells were maintained by weekly passage in Dulbecco's modified Eagle's medium (DME) containing 10% (v/v) foetal bovine serum (FCS). Recombinant cytokines are generally available from commercial sources or were prepared by published methods. Recombinant murine LIF was produced in *Escherichia coli* and purified, as previously described [Gearing, 1989]. Purified human oncostatin M was purchased from PeproTech Inc (Rocky Hill, NJ, USA), and purified mouse IFN-γ was obtained from Genzyme Diagnostics (Cambridge, MA, USA). Recombinant murine thrombopoietin was produced as a FLAGTM-tagged fusion protein in CHO cells and then purified.

**EXAMPLE 2**

**AGAR COLONY ASSAYS**

In order to assay the differentiation of M1 cells in response to cytokines, 300 cells were cultured in 35 mm Petri dishes containing 1 ml of DME supplemented with 20%(v/v) fital calf serum (FCS), 0.3%(w/v) agar and 0.1 ml of serial dilutions-of IL-6, LIF, OSM, IFN-γ, tpo or dexamethasone (Sigma Chemical Company, St Louis, MI). After 7 days culture at 37°C in a fully humidified atmosphere, containing 10% (v/v) $CO_2$ in air, colonies of M1 cells were counted and classified as differentiated if they were composed of dispersed cells or had a corona of dispersed cells around a tightly packed centre.

**EXAMPLE 3**

**GENERATION OF RETROVIRAL LIBRARY**

A cDNA expression library was constructed from the factor-dependent haemopoietic cell line FDC-P1, essentially as described [Rayner, 1994]. Briefly, cDNA was cloned into the retroviral vector pRUFneo and then transfected into an amphotrophic packaging cell line (PA317). Transiently generated virus was harvested from the cell supernatant at 48 hr posttransfection, and used to infect Y2 ecotropic packaging cells, to generate a high titre virus-producing cell line.

**EXAMPLE 4**

**RETROVIRAL INFECTION OF M1 CELLS**

Pools of $10^6$ infected Ψ2 cells were irradiated (3000 rad) and cocultivated with $10^6$ M1 cells in DME supplemented with 10%(v/v) FCS and 4 μg/ml Polybrene, for 2 days at 37°C. To select for IL-6-unresponsive clones, retrovirally-infected M1 cells were washed once in DME, and cultured at approximately $2x10^4$ cells/ml in 1 ml agar cultures containing 400 μg/ml geneticin (GibcoBRL, Grand Island, NY) and 100 ng/ml IL-6. The efficiency of infection of M1 cells was 1-2%, as estimated by agar plating the infected cells in the presence of geneticin only.

**EXAMPLE 5**

**PCR**

Genomic DNA from retrovirally-infected M1 cells was digested with Sac I and 1 μg of phenol/chloroform extracted DNA was then amplified by polymerase chain reaction (PCR). Primers used for amplification of cDNA inserts from the integrated retrovirus were GAG3 (5' CACGCCGCCCACGTGAAGGC 3' [SEQ ID NO:1]), which corresponds to the vector gag sequence approximately 30 bp 5' of the multiple cloning site, and HSVTK (5' TTCGCCAATGACAAGACGCT 3' [SEQ ID NO:2]), which corresponds to the pMC1neo sequence approximately 200 bp 3' of the multiple cloning site. The PCR entailed an initial denaturation at 94°C for 5 min, 35 cycles of denaturation at 94°C for 1 min, annealing at 56°C for 2 min, and extension at 72°C for 3 min, followed by a final 10 min extension. PCR products were gel purified and then ligated into the pGEM-T plasmid (Promega, Madison, WI), and sequenced using an ABI PRISM Dye Terminator Cycle Sequencing Kit and a Model 373 Automated DNA Sequencer (Applied Biosystems Inc., Foster City, CA).

**EXAMPLE 6**

**CLONING OF cDNAs**

Independent cDNA clones encoding mouse SOCS1 were isolated from a murine thymus cDNA library essentially as described (Hilton *et al,* 1994). The nucleotide and predicted amino acid sequences of mouse SOCS1 cDNA were compared to databases using the BLASTN and TFASTA algorithms (Pearson and Lipman, 1988; Pearson, 1990; Altshcul *et al,* 1990). Oligonucleotides were designed from the ESTs encoding human SOCS1 and mouse SOC-1 and SOCS3 and used to probe commercially available mouse thymus and spleen cDNA libraries. Sequencing was performed using an ABI automated sequencer according to the manufacturer's instructions.

**EXAMPLE 7**

**SOUTHERN AND NORTHERN BLOT ANALYSES AND RT-PCR**

$^{32}P$-labelled probes were generated using a random decanucleotide labelling kit (Bresatec, Adelaide, South Australia) from a 600 bp Pst I fragment encoding neomycin phophotransfease from the plasmid pPGKneo, 1070 bp fragment of the SOCS1 gene obtained by digestion of the 1.4 kbp PCR product with Xho I, SOCS2, SOCS3, CIS and a 1.2 kbp fragment of the chicken glyceraldehyde 3-phosphate dehydrogenase gene [Dugaiczyk, 1983].

Genomic DNA was isolated from cells using a proteinase K-sodium dodecyl sulfate procedure essentially as described. Fifteen micrograms of DNA was digested with either BamH I or Sac I, fractionated on a 0.8%(w/v) agarose gel, transferred to GeneScreenPlus membrane (Du Pont NEN, Boston MA), prehybridised, hybridised with random-primed $^{32}P$-labelled DNA fragments and washed essentially as described [Sambrook, 1989].

Total RNA was isolated from cells and tissues using Trizol Reagent, as recommended by the manufacturer (GibcoBRL,Grand Island, NY). When required polyA+ mRNA was purified essentially as described [Alexander, 1995].

Northern blots were prehybridised, hybridized with random-primed 32P-labelled DNA fragments and washed as described [Alexander, 1995].

To assess the induction of SOCS genes by IL-6, mice (C57BL6) were injected intravenously with 5 μg IL-6 followed by harvest of the liver at the indicated timepoints after injection. M1 cells were cultured in the presence of 20 ng/ml IL-6 and harvested at the indicated times. For RT-PCR analysis, bone marrow cells were harvested as described (Metacalf *et al,* 1995) and stimulated for 1 hr at 37 ° C with 100 ng/ml of a range of cytokines. RT-PCR was performed on total RNA as described (Metcalf *et al,* 1995). PCR products were resolved on an agarose gel and Southern blots were hybridised with probes specific for each SOCS family member. Expression of β-actin was assessed to ensure uniformity of amplification.

## EXAMPLE 8

## DNA CONSTRUCTS AND TRANSFECTION

A cDNA encoding epitope-tagged SOCS1 was generated by subcloning the entire SOCS1 coding region into the pEF-BOS expression vector [Mizushima, 1990], engineered to encode an inframe FLAG epitope downstream of an initiation methionine (pF-SOCS1). Using electroporation as described previously [Hilton, 1994], M1 cells expressing the thrombopoietin receptor (M1.mpl) were transfected with the 20 μg of Aat II-digested pF-SOCS1 expression plasmid and 2 μg of a Sca I-digested plasmid in which transcription of a cDNA encoding puromycin N-acetyl transferase was driven from the mouse phosphoglycerokinase promoter (pPGKPuropA). After 48 hours in culture, transfected cells were selected with 20 μg/ml puromycin (Sigma Chemical Company, St Louis MO), and screened for expression of SOCS1 by Western blotting, using the M2 anti-FLAG monoclonal antibody according to the manafacturer's instructions (Eastman Kodak, Rochester NY). In other experiments M1 cells were transfected with only the pF-SOCS1 plasmid or a control and selected by their ability to grow in agar in the presence of 100 ng/ml of IL-6.

## EXAMPLE 9

## IMMUNOPRECIPITATION AND WESTERN BLOTTING

Prior to either immunoprecipitaion or Western blotting, $10^7$ M1 cells or their derivatives were washed twice, resuspended in 1ml of DME, and incubated at 37°C for 30 min. The cells were then stimulated for 4 min at 37°C with either saline or 100 ng/ml IL-6, after which sodium vanadate (Sigma Chemical Co., St Louis, MI) was added to a concentration of 1 mM. Cells were placed on ice, washed once with saline containing 1 mM sodium vanadate, and then solubilised for 5 min on ice with 300 μl 1% (v/v) Triton X-100, 150 mM NaCl, 2 mM EDTA, 50 mM Tris-HCl pH 7.4, containing Complete protease inhibitors (Boehringer Mannheim, Mannheim, Germany) and 1 mM sodium vanadate. Lysates were cleared by centrifugation and quantitated using a Coomassie Protein Assay Reagent (Pierce, Rockford IL).

For immunoprecipitations, equal concentrations of protein extracts (1-2 mg) were incubated for 1 hr or overnight at 4°C with either 4 μg of anti-gp130 antibody (M20; Santa Cruz Biotechnology Inc., Santa Cruz,CA) or 4 μg of anti-phosphotyrosine antibody (4G10; Upstate Biotechnology Inc., Lake Placid NY), and 15 μl packed volume of Protein G Sepharose (Pharmacia, Uppsala, Sweden) [Hilton *et al,* 1996]. Immunoprecipitates were washed twice in 1% (v/v) NP40, 150 mM NaCl, 50 mM Tris-HCl pH 8.0, containing Complete protease inhibitors (Boehringer Mannheim, Mannheim, Germany and 1 mM sodium vanadate. The samples were heated for 5 min at 95°C in SDS sample buffer (625 mM Tris-HCl pH 6.8, 0.05% (w/v) SDS, 0.1% (v/v) glycerol, bromophenol blue, 0.125% (v/v) 2-mercaptoethanol), fractionated by SDS-PAGE and immunoblotted as described above.

For Western blotting, 10 μg of protein from a cellular extract or material from an immunoprecipitation reaction was loaded onto 4-15% Ready gels (Bio-Rad Laboratories, Hercules CA), and resolved by sodium dodecyl sulfate polyacrylamide gel electrophoresis (SDS-PAGE). Proteins were transferred to PVDF membrane (Micron Separations Inc., Westborough MA) for 1 hr at 100 V. The membranes were probed with the following primary antibodies; anti-tyrosine phosphorylated STAT3 (1:1000 dilution; New England Biolabs, Beverly, MA); anti-STAT3 (C-20; 1:100 dilution; Santa Cruz Biotechnology Inc., Santa Cruz CA); anti-gp130 (M20, 1:100 dilution; Santa Cruz Biotechnology Inc., Santa Cruz CA); anti-phosphotyrosine (horseradish peroxidase-conjugated RC20, 1:5000 dilution; Transduction Laboratories, Lexington KY); anti-tyrosine phosphorylated MAP kinase and anti-MAP kinase antibodies (1:1000 dilution; New England Biolabs, Beverly, MA). Blots were visualised using peroxidase-conjugated secondary antibodies and Enhanced Chemiluminescence (ECL) reagents according to the manafacturer's instructions (Pierce, Rockford IL).

**EXAMPLE 10**

**ELECTROPHORETIC MOBILITY SHIFT ASSAYS**

Assays were performed as described [Novak, 1995], using the high affinity SIF (c-sis- inducible factor) binding site m67 [Wakao, 1994]. Protein extracts were prepared from M1 cells incubated for 4-10 min at 37°C in 10 ml serum-free DME containing either saline, 100 ng/ml IL-6 or 100 ng/ml IFN-γ. The binding reactions contained 4-6 μg protein (constant within a given experiment), 5 ng $^{32}$P-labelled m67 oligonucleotide, and 800 ng sonicated salmon sperm DNA. For certain experiments, protein samples were preincubated with an excess of unlabelled m67 oligonucleotide, or antibodies specific for either STAT1 (Transduction Laboratories, Lexington, KY) or STAT3 (Santa Cruz Biotechnology Inc., Santa Cruz CA), as described [Novak, 1995].

Western blots were performed using anti-tyrosine phosphorylated STAT3 or anti-STAT3 (New England Biolabs, Beverly, MA) or anti-gp130 (Santa Cruz Biotechnology Inc.) as described (Nicola *et al*, 1996). EMSA were performed using the m67 oligonucleotide probe, as described (Novak *et al*, 1995).

**EXAMPLE 11**

**EXPRESSION CLONING OF A NOVEL SUPPRESSOR OF CYTOKINE SIGNAL TRANSDUCTION**

In order to identify cDNAs capable of suppressing cytokine signal transduction, an expression cloning approach was adopted. This strategy centred on M1 cells, a monocytic leukaemia cell line that differentiates into mature macrophages and ceases proliferation in response to the cytokines IL-6, LIF, OSM and IFN-γ, and the steroid dexamethasone. Parental M1 cells were infected with the RUFneo retrovirus, into which cDNAs from the factor-dependent haemopoietic cell line FDC-P1 had been cloned. In this retrovirus, transcription of both the neomycin resistance gene and the cloned cDNA was driven off the powerful constitutive promoter present in the retroviral LTR (Figure 1). When cultured in semi-solid agar, parental M1 cells form large tightly packed colonies. Upon stimulation with IL-6, M1 cells undergo rapid differentiation, resulting in the formation in agar of only single macrophages or small dispersed clusters of cells . Retrovirally-infected M1 cells that were unresponsive to IL-6 were selected in semi-solid agar culture by their ability to form large, tightly packed colonies in the presence of IL-6 and geneticin. A single stable IL-6-unresponsive clone, 4A2, was obtained after examining $10^4$ infected cells.

A fragment of the neomycin phosphotransferase (neo) gene was used to probe a Southern blot of genomic DNA from clone 4A2 and this revealed that the cell line was infected with a single retrovirus containing a cDNA approximately 1.4 kbp in length (Figure 2). PCR amplification using primers from the retroviral vector which flanked the cDNA cloning site enabled recovery of a 1.4 kbp cDNA insert, which we have named suppressor of cytokine signalling-1, or SOCS1. This PCR product was used to probe a similar Southern blot of 4A2 genomic DNA and hybridised to two fragments, one which corresponded to the endogenous SOCS1 gene and the other, which matched the size of the band seen using the neo probe, corresponded to the SOCS1 cDNA cloned into the integrated retrovirus (Figure 2). The latter was not observed in an M1 cell clone infected with a retrovirus containing an irrelevant cDNA. Similarly, Northern blot analysis revealed that SOCS1 mRNA was abundant in the cell line 4A2, but not in the control infected M1 cell clone (Figure 2).

**EXAMPLE 12**

**SOCS1, SOCS2, SOCS3 AND CIS DEFINE A NEW FAMILY OF SH2-CONTAINING PROTEINS**

The SOCS1 PCR product was used as a probe to isolate homologous cDNAs from a mouse thymus cDNA library. The sequence of the cDNAs proved to be identical to the PCR product, suggesting that constitutive or over expression, rather than mutation, of the SOCS1 protein was sufficient for generating an IL-6-unresponsive phenotype. Comparison of the sequence of SOCS1 cDNA with nucleotide sequence databases revealed that it was present on mouse and rat genomic DNA clones containing the protamine gene cluster found on mouse chromosome 16. Closer inspection revealed that the 1.4 kb SOCS1 sequence was not homologous to any of the protamine genes, but rather represented a previously unidentified open reading frame located at the extreme 3' end of these clones (Figure 3). There were no regions of discontinuity between the sequences of the SOCS1 cDNA and genomic locus, suggesting that SOCS1 is encoded by a single exon. In addition to the genomic clone containing the protamine genes, a series of murine and human expressed sequenced tags (ESTs) also revealed large blocks of nucleotide sequence identity to mouse SOCS1. The sequence information provided by the human ESTs allowed the rapid cloning of cDNAs encoding human SOCS1.

The mouse and rat SOCS1 gene encodes a 212 amino acid protein whereas the human SOCS1 gene encodes a 211 amino acid protein. Mouse, rat and human SOCS1 proteins share 95-99% amino acid identity (Figure 9). A search of translated nucleic acid databases with the predicted amino acid sequence of SOCS1 showed that it was most related to a recently cloned cytokine-inducible immediate early gene product, CIS, and two classes of ESTs. Full length cDNAs from the two classes of ESTs were isolated and found to encode proteins of similar length and overall structure to SOCS1 and CIS. These clones were given the names SOCS2 and SOCS3. Each of the four proteins contains a central SH2 domain and a C-terminal region termed the SOCS motif. The SOCS1 proteins exhibit an extremely high level of amino acid sequence similarity (95-99% identity) amongst different species. However, the forms of the SOCS1, SOCS2, SOCS3 and CIS from the same animal, while clearly defining a new family of SH2-containing proteins, exhibited a lower amino acid identity. SOCS2 and CIS exhibit approximately 38% amino acid identity, while the remaining members of the family share approximately 25% amino acid identity (Figure 9). The coding region of the genes for SOCS1 and SOC3 appear to contain no introns while the coding region of the genes for SOCS2 and CIS contain one and two introns, respectively.

The Genbank Accession Numbers for the sequences referred to herein are mouse SOCS1 cDNA (U88325), human SOCS1 cDNA (U88326), mouse SOCS2 cDNA (U88327), mouse SOCS3 cDNA (U88328).

**EXAMPLE 13**

**CONSTITUTIVE EXPRESSION OF SOCS1 SUPPRESSES THE ACTION OF A RANGE OF CYTOKINES**

To formally establish that the phenotype of the 4A2 cell line was directly related to expression of SOCS1, and not to unrelated genetic changes which may have occurred independently in these cells, a cDNA encoding an epitope-tagged version of SOCS1 under the control of the EF1α promoter was transfected into parental M1 cells, and M1 cells expressing the receptor for thrombopoietin, c-mpl (M1.mpl). Transfection of the SOCS1 expression vector into both cell lines resulted in an increase in the frequency of IL-6 unresponsive M1 cells.

Multiple independent clones of M1 cells expression SOCS1, as detected by Western blot, displayed a cytokine-unresponsive phenotype that was indistinguishable from 4A2. Further, if transfectants were not maintained in puromycin, expression of SOCS1 was lost over time and cells regained their cytokine responsiveness. In the absence of cytokine, colonies derived from 4A2 and other SOCS1 expressing clones characteristically grew to a smaller size than colones formed by control M1 cells (Figure 10).

The effect of constitutive SOCS1 expression on the response of M1 cells to a range of cytokines was investigated using the 4A2 cell line and a clone of M1.mpl cells expressing SOCS1 (M1.mpl.SOCS1). Unlike parental M1 cells and M1.mpl cells, the two cell lines expressing SOCS1 continued to proliferate and failed to form differentiated colonies in response to either IL-6, LIF, OSM, IFN-γ or, in the case of the M1.mpl.SOCS1 cell line, thrombopoietin (Figure 4). For both cell lines, however, a normal response to dexamethasone was observed, suggesting that SOCS1 specifically affected cytokine signal transduction rather than differentiation *per se.* Consistent with these data, while parental M1 cells and M1.mpl cells became large and vacuolated in response to IL-6, 4A2 and M1.mpl.SOCS1 cells showed no evidence of morphological differentiation in response to IL-6 or other cytokines (Figure 5).

**EXAMPLE 14**

**SOCS1 INHIBITS A RANGE OF IL-6 SIGNAL TRANSDUCTION PROCESSES, INCLUDING STAT3 PHOSPHORYLATION AND ACTIVATION**

Phosphorylation of the cell surface receptor component gp 130, the cytoplasmic tyrosine kinase JAK1 and the transcription factor STAT3 is thought to play a central role in IL-6 signal transduction. These events were compared in the parental M1 and M1.mpl cell lines and their SOCS1-expressing counterparts. As expected, gp130 was phosphorylated rapidly in response to IL-6 in both parental lines, however, this was reduced five- to ten-fold in the cell lines expressing SOCS1 (Figure 6). Likewise, STAT3 phosphorylation was also reduced by approximately ten-fold in response to IL-6 in those cell lines expressing SOCS1 (Figure 6). Consistent with a reduction in STAT3 phosphorylation, activation of specific STAT DNA binding complexes, as determined by electrophoretic mobility shift assay, was also reduced. Notably, there was a reduction in the formation of SIF-A (containing STAT3), SIF-B (STAT1/STAT3 heterodimer) and SIF-C (containing STAT1), the three STAT complexes induced in M1 cells stimulated with IL-6 (Figure 7). Similarly, constitutive expression of SOCS1 also inhibited IFN-γ-stimulated formation of p91 homodimers (Figure 7). STAT phosphorylation and activation were not the only cytoplasmic processes to be effected by SOCS1 expression, as the phosphorylation of other proteins, including shc and MAP kinase, was reduced to a similar extent (Figure 7).

**EXAMPLE 15**

**TRANSCRIPTION OF THE SOCS1 GENE IS STIMULATED BY IL-6 *IN VITRO* AND *IN VIVO***

Although SOCS1 can inhibit cytokine signal transduction when constitutively expressed in M1 cells, this does.not necessarily indicate that SOCS1 normally functions to negatively regulate an IL-6 response. In order to investigate this possibility the inventors determined whether transcription of the SOCS1 gene is regulated in the response of M1 cells to IL-6 and, because of the critical role IL-6 plays in regulating the acute phase response to injury and infection, the response of the liver to intravenous injection of 5 mg IL-6. In the absence of IL-6, SOCS1 mRNA was undetectable in either M1 cells or in the liver. However, for both cell types, a 1.4 kb SOCS1 transcript was induced within 20 to 40 minutes by IL-6 (Figure 8). For M1 cells, where the IL-6 was present throughout the experiment, the level of SOCS1 mRNA remained elevated (Figure 8). In contrast, IL-6 was administered in vivo by a single intravenous injection and was rapidly cleared from the circulation, resulting in a pulse of IL-6 stimulation to the liver. Consistent with this, transient expression of SOCS1 mRNA was detectable in the liver, peaking approximately 40 minutes after injection and declining to basal levels within 4 hours (Figure 8).

**EXAMPLE 16**

**REGULATION OF SOCS GENES**

Since CIS was cloned as a cytokine-inducible immediate early gene the inventors examined whether SOCS1, SOCS2 and SOCS3 were similarly regulated. The basal pattern of expression of the four SOCS genes was examined by Northern blot analysis of mRNA from a variety of tissues from male and female C57B 1/6 mice (Figure 11A). Constitutive expression of SOCS1 was observed in the thymus and to a lesser extend in the spleen and the lung. SOCS2 expression was restricted primarily to the testis and in some animals the liver and lung; for SOCS3 a low level of expression was observed in the lung, spleen and thymus, while CIS expression was more widespread, including the testis, heart, lung, kidney and, in some animals, the liver.

The inventors sought to determine whether expression of the four SOCS genes was regulated by IL-6. Northern blots of mRNA prepared from the livers of untreated and IL-6-injected mice, or from unstimulated and IL-6-stimulated M1 cells, were hybridised with labelled fragments of SOCS1, SOCS2, SOCS3 and CIS cDNAs (Figure 11B). Expression of all four SOCS genes was increased in the liver following IL-6 injection, however the kinetics of induction appeared to differ. Expression of SOCS1 and SOCS3 was transient in the liver, with mRNA detectable after 20 minutes of IL-6 injection and declining to basal levels within 4 hours for SOCS and 8 hours for SOCS3. Induction of SOCS2 and CIS mRNA in the liver followed similar initial kinetics to that of SOCS1, but was maintained at an elevated level for at least 24 hours. A similar induction of SOCS gene mRNA was observed in other organs, notably the lung and the spleen. In contrast, in M1 cells, while SOCS1 and CIS mRNA were induced by IL-6, no induction of either SOCS2 or SOCS3 expression was detected. This result highlights cell type-specific differences in the expression of the genes of SOCS family members in response to the same cytokine.

In order to examine the spectrum of cytokines that was capable of inducing transcription of the various members of the SOCS gene family, bone marrow cells were stimulated for an hour with a range of cytokines, after which mRNA was extracted and cDNA was synthesised. PCR was then used to assess the expression of SOCS1, SOCS2, SOCS3 and CIS (Figure 11C). In the absence of stimulation, little or no expression of any of the SOCS genes was detectable in bone marrow by PCR. Stimulation of bone marrow cells with a broad array of cytokines appeared capable of up regulating mRNA for one or more members of the SOCS family. IFNγ, for example, induced expression of all four SOCS genes, while erythropoietin, granulocyte colony-stimulating factor, granulocyte-macrophage colony stimulating factor and interleukin-3 induced expression of SOCS2, SOCS3 and CIS. Interestingly, tumor necrosis factor alpha, macrophage colony-stimulating factor and interleukin-1, which act through receptors that do not fall into the type I cytokine receptor class also appeared capable of inducing expression of SOCS3 and CIS, suggesting that SOCS proteins may play a broader role in regulating signal transduction.

As constitutive expression of SOCS1 inhibited the response of M1 cells to a range of cytokines, the inventors examined whether phosphorylation of the cell surface receptor component gp130 and the transcription factor STAT3, which are though to play a central role in IL-6 signal transduction, were affected. These events were compared in the parental M1 and M1.mpl cell lines and their SOCS1-expressing counterparts. As expected, gp130 was phyosphorylated rapidly in response to IL-6 in both parental lines, however, this was reduced in the cell lines expressing SOCS1 (Figure 12A). Likewise, STAT3 phosphorylation was also reduced in response to IL-6 in those cell lines expressing SOCS1 (Figure 12A). Consistent with a reduction in STAT3 phosphorylation, activation of specific STAT/DNA binding complexes, as determined by electrophoretic mobility shift assay, was also reduced. Notably, there was a failure to form SIF-A (containing STAT3) and SIF-B(STAT1/STAT3 heterodimer), the major STAT complexes induced in M1 cells stimulated with IL-6 (Figure 12B). Similarly, constitutive expression of SOCS1 also inhibited IFNγ-stimulating formation of SIF-C (STAT1 homodimer; Figure 12B). These experiments are consistent with the proposal that SOCS1 inhibits signal transduction upstream of receptor and STAT phosphorylation, potentially at the level of the JAK kinases.

The ability of SOCS1 to inhibit signal transduction and ultimately the biological response to cytokines suggest that, like the SH2-containing phosphatase SHP-1 [Ihle *et al*, 1994; Yi *et al,* 1993], the SOCS proteins may play a central role in controlling the intensity and/or duration of a cell's response to a diverse range of extracellular stimuli by suppressing the signal transduction process. The evidence provided here indicates that the SOCS family acts in a classical negative feedback loop for cytokine signal transduction. Like other genes such as OSM, expression of genes encoding the SOCS proteins is induced by cytokines through the activation of STATs. Once expressed, it is proposed that the SOCS proteins inhibit the activity of JAKs and so reduce the phosphorylation of receptors and STATs, thereby suppressing signal transduction and any ensuing biological response. Importantly, inhibition of STAT activation will, over time, lead to a reduction in SOCS gene expression, allowing cells to regain responsiveness to cytokines.

## EXAMPLE 17

## DATABASE SEARCHES

The NCBI genetic sequence database (Genbank), which encompasses the major database of expressed sequence tags (ESTs) and TIGR database of human expressed sequence tags, were searched for sequences with similarity to a concensus SOCS box sequence using the TFASTA and MOTIF/PATTERN algorithms [Pearson, 1990; Cockwell and Giles, 1989]. Using the software package SRS [Etzold *et al,* 1996], ESTs that exhibited similarity to the SOCS box (and their partners derived from sequencing the other end of cDNAs) were retrieved and assembled into contigs using Autoassembler (Applied Biosystems, Foster City, CA). Consensus nucleotide sequences derived from overlapping ESTs were then used to search the various databases using BLASTN [Altschul *et al,* 1990]. Again, positive ESTs were retrieved and added to the contig. This process was repeated until no additional ESTs could be recovered. Final consensus nucleotide sequences were then translated using Sequence Navigator (Applied Biosystems, Foster City, CA).

The ESTs encoding the new SOCS proteins are as follows: **human SOCS4** (EST81149, EST180909, EST182619, ya99H09, ye70co4, yh53c09, yh77g11, yh87h05, yi45h07, yj04e06, yq12h06, yq56a06, yq60e02, yq92g03, yq97h06, yr90f01, yt69c03, yv30a08, yv55f07, yv57h09, yv87h02, yv98e11, yw68d10, yw82a03, yx08a07, yx72h06, yx76b09, yy37h08, yy66b02, za81f08, zb18f07, zc06e08, zd14g06, zd51h12, zd52b09, ze25g11, ze69f02, zf54f03, zh96e07, zv66h12, zs83a08 and zs83g08). **mouse SOCS-4** (mc65f04, mf42e06, mp10c10, mr81g09, and mt19h12). **human SOCS-5** (EST15B103, EST15B105, EST27530 and zf50f01). **mouse SOCS-5** (mc55a01, mh98f09, my26h12 and ve24e06). **human SOCS-6** (yf61e08, yf93a09, yg05f12, yg41f04, yg45c02, yh11f10, yh13b05, zc35a12, ze02h08, z109a03, z169e10, zn39d08 and zo39e06). **mouse SOCS-6** (mc04c05, md48a03, mf31d03, mh26b07, mh78e11, mh88h09, mh94h07, mi27h04 and mj29c05, mp66g04, mw75g03, va53b05, vb34h02, vc5Sd07, vc59e05, vc67d03, vc68d10, vc97h01, vc99c08, vd07h03, vd08c01, vd09b12, vd19b02, vd29a04 and vd46d06). **human SOCS-7** (STS WI30171, EST00939, EST12913, yc29b05, yp49f10, zt10f03 and zx73g04). **mouse SOCS-7** (mj39a01 and vi52h07). **mouse SOCS-8** (mj6e09 and vj27a029). **human SOCS-9** (CSRL-82f2-u, EST114054, yy06b07, yy06g06, zr40c09, zr72h01, yx92c08, yx93b08 and hfe0662). **mouse SOCS-9** (me65d05). **human SOCS-10** (aa48h10, zp35h01, zp97h12, zq08h01, zr34g05, EST73000 and HSDHEI005). **mouse SOCS-10** (mb14d12, mb40f06, mg89b11, mq89e12, mp03g12 and vh53c11). **human SOCS-11** (zt24h06 and zr43b02). **human SOCS-13** (EST59161). **mouse SOCS-13** (ma39a09, me60c05, mi78g05, mk10c11, mo48g12, mp94a01, vb57c07 and vh07c11). **human SOCS-14** (mi75e03, vd29h1 and vd53g07).

## EXAMPLE 18

## cDNA CLONING

Based on the concensus sequences derived from overlapping ESTs, oligonucleotides were designed that were specific to various members of the SOCS family. As described above, oligonucleotides were labelled and used to screen commerically available genomic and cDNA libraries cloned with λ bacteriophage. Genomic and/or cDNA clones covering the entire coding region of mouse SOCS4, mouse SOCS5 and mouse SOCS6 were isolated. The entire gene for SOCS15 is on the human 12p13 BAC (Genbank Accession Number HSU47924) and the mouse chromosome 6 BAC (Genbank Accession Number AC002393). Partial cDNAs for mouse SOCS7, SOCS9, SOCS10, SOCS11, SOCS12, SOCS13 and SOCS14 were also isolated.

## EXAMPLE 19

## NORTHERN BLOTS AND rtPCR

Northern blots were performed as described above. The sources of hybridisation probes were as follows; (i) the entire coding region of the mouse SOCS1 cDNA, (ii) a 1059 bp PCR product derived from coding region of SOCS5 upstream of the SH2 domain, (iii) the entire coding region of the mouse SOCS6 cDNA, (iv) a 790 bp PCR product derived from the coding region of a partial SOCS7 cDNA and (v) a 1200 bp Pst I fragment of the chicken glyceraldehyde 3-phosphate dehydrogenase (GAPDH) cDNA.

## EXAMPLE 20

## ADDITIONAL MEMBERS OF SOCS FAMILY

SOCS1, SOCS2 and SOCS3 are members of the SOCS protein family identified in Examples 1-16. Each contains a central SH2 domain and a conserved motif at the C-terminus, named the SOCS box. In order to isolate further members of this protein family, various DNA databases were searched with the amino acid sequence corresponding to conserved residues of the SOCS box. This search revealed the presence of human and mouse ESTs encoding twelve further members of the SOCS protein family (Figure 13). Using this sequence information cDNAs encoding SOCS4, SOCS5, SOCS6, SOCS7, SOCS9, SOCS10, SOCS11, SOCS12, SOCS13, SOCS14 and SOCS15 have been isolated. Further analysis of contigs derived from ESTs and cDNAs revealed that the SOCS proteins could be placed into three groups according to their predicted structure N-terminal of the SOCS box. The three groups are those with (i) SH2 domains, (ii) WD-40 repeats and (iii) ankyrin repeats.

## EXAMPLE 21

## SOCS PROTEIN WITH SH2 DOMAINS

Eight SOCS proteins with SH2 domains have been identified. These include SOCS1, SOCS2 and SOCS3, SOCS5, SOCS9, SOCS11 and SOCS14 (Figure 13). Full length cDNAs were isolated for mouse SOCS5 and SOCS14 and partial clones encoding mouse SOCS9 and SOCS14. Analysis of primary amino acid sequence and genomic structure suggest that pairs of these proteins (SOCS1 and SOCS3, SOCS2 and CIS, SOCS5 and SOCS14 and SOCS9 and SOCS11) are most closely related (Figure 13). Indeed, the SH2 domains of SOCS5 and SOCS14 are almost identical (Figure 13B), and unlike CIS, SOCS1, SOCS2 and SOCS3, SOCS5 and SOCS14 have an extensive, though less well conserved, N-terminal region preceding their SH2 domains (Figure 13A).

## EXAMPLE 22

## SOCS PROTEINS WITH WD-40 REPEATS

Four SOCS proteins with WD-40 repeats were identified. As with the SOCS proteins with SH2 domains, pairs of these proteins appeared to be closely related. Full length cDNAs of mouse SOCS4 and SOCS6 were isolated and shown to encode proteins containing eight WD-40 repeats N-terminal of the SOCS box (Figure 13) and SOCS4 and SOCS6 share 65% amino acid similarity. SOCS15 was recognised as an open reading frame upon sequencing BACs from human chromosome 12p13 and the syntenic region of mouse chromosome 6 [Ansari-Lari *et al,* 1997]. In the human, chimp and mouse, SOCS15 is encoded by a gene with two coding exons that lies within a few hundred base pairs of the 3' end of the triose phosphate isomerase (TPI) gene, but which is encoded on the opposite strand to TPI (9). In addition to a C-terminal SOCS box, the SOCS15 protein contains four WD-40 repeats. Interestingly, within the EST databases, there is a sequence of a nematode, an insect and a fish relative of SOCS15. SOCS15 appears most closely related to SOCS13.

## EXAMPLE 23

## SOCS PROTEINS WITH ANKYRIN REPEATS

Three SOCS proteins with ankyrin repeats were identified. Analysis of partial cDNAs of mouse SOCS7, SOCS10 and SOCS12 demonstrated the presence of multiple ankyrin repeats.

**EXAMPLE 24**

**EXPRESSION PATTERN OF SOCS PROTEINS**

The expression of mRNA from representative members of each class of SOCS proteins - SOCS1 and SOCS5 from the SH2 domain group, SOCS6 from the WD-40 repeat group and SOCS7 from the ankyrin repeat group was examined. As shown above, SOCS1 mRNA is found in abundance in the thymus and at lower levels in other adult tissues.

Since transcription of the SOCS1 gene is induced by cytokines, the inventors sought to determine whether levels of SOCS5, SOCS6 and SOCS7 mRNA increased upon cytokine stimulation. In the livers of mice injected with IL-6, SOCS1 mRNA is detectable after 20 min and decreases to background levels within 2 hours. In contrast, the kinetics of SOCS5 mRNA expression are quite different, being only detectable 12 to 24 hours after IL-6 injection. SOCS6 mRNA appears to be expressed constitutively while SOCS7 mRNA was not detected in the liver either before injection of IL-6 or at any time after injection.

Expression of these genes was also examined after cytokine stimulation of the factor-dependent cell line FDCP-1 engineered to express bcl-w. Again, while SOCS6 mRNA was expressed constitutively.

**EXAMPLE 25**

**SOCS4**

Mouse and human SOCS4 were recognized through searching EST databases using the SOCS box consensus (Figure 13). Those ESTs derived from mouse and human SOCS4 cDNAs are tabulated below (Tables 4.1 and 4.2). Using sequence information derived from mouse ESTs several oligonucleotides were designed and used to screen, in the conventional manner, a mouse thymus cDNA library cloned into λ-bacteriophage. Two cDNAs encoding mouse SOCS4 were isolated and sequenced in their entirety (Figure 15) and shown to overlap the mouse ESTs identified in the database (Table 4.1 and Figure 17). These cDNAs include a region of 5' untranslated region, the entire mouse SOCS4 coding region and a region of 3' untranslated region (Figure 17). Analysis of the sequence confirms that the SOCS4 cDNA encodes a SOCS Box at its C-terminus and a series of 8 WD-40 repeats before the SOCS Box (Figures 17 and 16). The relationship of the two sequence contigs of human SOCS4 (h4.1 and h4.2) to the experimentally determined mouse SOCS4 cDNA sequence is shown in Figure 17. The nucleotide sequence of the two human contigs is listed in Figure 18.

SEQ ID NO:13 and 14 represent the nucleotide sequence of murine SOCS4 and the corresponding amino acid sequence. SEQ ID NOs: 15 and 16 are SOCS4 cDNA human contigs h4.1 and h4.2, respectively.

**EXAMPLE 26**

**SOCS5**

Mouse and human SOCS5 were recognized through searching EST databases using the SOCS box consensus (Figure 13). Those ESTs derived from mouse and human SOCS5 cDNAs are tabulated below (Tables 5.1 and 5.2). Using sequence information derived from mouse and human ESTs, several oligonucleotides were designed and used to screen, in the conventional manner, a mouse thymus cDNA library, a mouse genomic DNA library and a human thymus cDNA library cloned into λ-bacteriophage. A single genomic DNA clone (57-2) and (5-3-2) cDNA clone encoding mouse SOCS5 were isolated and sequenced in their entirety and shown to overlap with the mouse ESTs identified in the database (Figures 19 and 20A). The entire coding region, in addition to a region of 5' and 3' untranslated regions of mouse SOCS5 appears to be encoded on a single exon (Figure 19). Analysis of the sequence (Figure 20) confirms that SOCS5 genomic and cDNA clones encode a protein with a SOCS box at its C-terminus in addition to an SH2 domain (Figure 19 and 20B). The relationship of the human SOCS5 contig (h5.1; Figure 21) derived from analysis of cDNA clone 5-94-2 and the human SOCS5 ESTs (Table 5.2) to the mouse SOCS5 DNA sequence is shown in Figure 19. The nucleotide sequence and corresponding amino acid sequence of murine SOCS5 are shown in SEQ ID NOs: 17 and 18, respectively. The human SOCS5 nucleotide sequence is shown in SEQ ID NO:19.

**EXAMPLE 27**

**SOCS6**

Mouse and human SOCS6 were recognized through searching EST databases using the SOCS box consensus (Figure 13). Those ESTs derived from mouse and human SOCS6 cDNAs are tabulated below (Tables 6.1 and 6.2).

Using sequence information derived from mouse ESTs, several oligonucleotides were designed and use to screen, in the conventional manner, a mouse thymus cDNA library. Eight cDNA clones (6-1A, 6-2A, 6-5B, 6-4N, 6-18, 6-29, 6-3N, 6-5N) cDNA clone encoding mouse SOCS6 were isolated and sequenced in their entirety and shown to overlap with the mouse ESTs identified in the database (Figures 22 and 23A). Analysis of the sequence (Figure 23) confirms that the mouse SOCS6 cDNA clones encode a protein with a SOCS box at its C-terminus in addition to a eight WD-40 repeats (Figures 22 and 23B). The relationship of the human SOCS-6 contigs (h6.1 and h6.2 ; Figure 24) derived from analysis of human SOCS6 ESTs (Table 6.2) to the mouse SOCS6 DNA sequence is shown in Figure 22. The nucleotide and corresponding amino acid sequences of murine SOCS6 are shown in SEQ ID NOs: 20 and 21, respectively. SOCS6 human contigs h6.1 and h6.2 are shown in SEQ ID NOs: 22 and 23, respectively.

**EXAMPLE 28**

**SOCS7**

Mouse and human SOCS7 were recognized through searching EST databases using the SOCS box consensus (Figure 13). Those ESTs derived from mouse and human SOCS-7 cDNAs are tabulated below (Tables 7.1 and 7.2). Using sequence information derived from mouse ESTs, several oligonucleotides were designed and use to screen, in the conventional manner, a mouse thymus cDNA library. One cDNA clone (74-10A-11) cDNA clone encoding mouse SOCS7 was isolated and sequenced in its entirety and shown to overlap with the mouse ESTs identified in the database (Figures 25 and 26A). Analysis of the sequence (Figure 26) suggests that mouse SOCS7 encodes a protein with a SOCS box at its C-terminus, in addition to several ankyrin repeats (Figure 25 and 26B). The relationship of the human SOCS7 contigs (h7.1 and h7.2 ; Figure 27) derived from analysis of human SOCS7 ESTs (Table 7.2) to the mouse SOCS7 DNA sequence is shown in Figure 25. The nucleotide and corresponding amino acid sequences of murine SOCS7 are shown in SEQ ID NOs: 24 and 25, respectively. The nucleotide sequence of SOCS7 human contigs h7.1 and h7.2 are shown in SEQ ID NOs: 26 and 27, respectively.

**EXAMPLE 29**

**SOCS8**

ESTs derived from mouse SOCS8 cDNAs are tabulated below (Table 8.1). As described for other members of the SOCS family, it is possible to isolate cDNAs for mouse SOCS8 using sequence information derived from mouse ESTs. The relationship of the ESTs to the predicted coding region of SOCS8 is shown in Figure 28. With the nucleotide sequence obtained from the ESTs shown in Figure 29A and the partial amino acid sequence of SOCS8 shown in Figure 29B. The nucleotide sequence and corresponding amino acid sequences for murine SOCS8 are shown in SEQ ID NOs: 28 and 29, respectively.

**EXAMPLE 30**

**SOCS9**

Mouse and human SOCS-9 were recognized through searching EST databases using the SOCS box consensus (Figure 13). Those ESTs derived from mouse and human SOCS9 cDNAs are tabulated below (Tables 9.1 and 9.2). The relationship of the mouse SOCS9 contigs (m9.1; Figure 9.2) derived from analysis of the mouse SOCS9 EST (Table 9.1) to the human SOCS-9 DNA contig (h9.1; Figure 32) derived from analysis of human SOCS9 ESTs (Table 9.2) is shown in Figure 31. Analysis of the sequence (Figure 32) indicates that the human SOCS9 cDNA encodes a protein with a SOCS box at its C-terminus, in addition to an SH2 domain (Figure 30). The nucleotide sequence of muring SOCS9 cDNA is shown in SEQ ID NO:30. The nucleotide sequence of human SOCS9 cDNA is shown in SEQ ID NO:31.

**EXAMPLE 31**

**SOCS10**

Mouse and human SOCS10 were recognized through searching EST databases using the SOCS box consensus (Figure 13). Those ESTs derived from mouse and human SOCS10 cDNAs are tabulated below (Table 10.1 and 10.2). Using sequence information derived from mouse ESTs, several oligonucleotides were designed and use to screen, in the conventional manner, a mouse thymus cDNA library. Four cDNA clones (10-9, 10-12, 10-23 and 10-24) encoding mouse SOCS10 were isolated, sequenced in their entirety and shown to overlap with the mouse and human ESTs identified in the database (Figures 33 and 34). Analysis of the sequence (Figure 34) indicates that the mouse SOCS10 cDNA clone is not full length but that it does encode a protein with a SOCS box at its C-terminus, in addition to several ankyrin repeats (Figure 33). The relationship of the human SOCS10 contigs (h10.1 and h10.2 ; Figure 35) derived from analysis of human SOCS10 ESTs (Table 10.2) to the mouse SOCS10 DNA sequence is shown in Figure 33. Comparison of mouse cDNA clones and ESTs with human ESTs suggests that the 3' untranslated regions of mouse and human SOCS10 differ significantly. The nucleotide sequence of murine SOCS10 is shown in SEQ ID NO:32 and the nucleotide sequence of SOCS10 human contigs h10.1 and h10.2 are shown in SEQ ID NOs:33 and 34, respectively.

**EXAMPLE 32**

**SOCS11**

Human SOCS11 were recognized through searching EST databases using the SOCS box consensus (Figure 13). Those ESTs derived from human SOCS11 cDNAs are tabulated below (Table 11.1 and 11.2). The relationship of the human SOCS11 contigs (h11.1; Figure 36A, B), derived from analysis ESTs (Table 11.2) to the predicted encoded protein, is shown in Figure 37. Analysis of the sequence indicates that the human SOCS11 cDNA encodes a protein with a SOCS box at its C-terminus, in addition to an SH2 domain (Figure 37 and 36B). The nucleotide sequence and corresponding amino acid sequence of human SOCS11 are represented in SEQ ID NOs:35 and 36, respectively.

**EXAMPLE 33**

**SOCS12**

Mouse and human SOCS-12 were recognized through searching EST databases using the SOCS box consensus (Figure 13). Those ESTs derived from mouse and human SOCS12 cDNAs are tabulated below (Tables 12.1 and 12.2). Using sequence information derived from mouse ESTs, several oligonucleotides were designed and use to screen, in the conventional manner, a mouse thymus cDNA library. Four cDNA clones (10-9, 10-12, 10-23 and 10-24) encoding mouse SOCS12 were isolated, sequenced in their entirety and shown to overlap with the mouse and human ESTs identified in the database (Figures 38 and 39). Analysis of the sequence (Figure 39 and 40) indicates that the SOCS12 cDNA clone encodes a protein with a SOCS box at its C-terminus, in addition to several ankyrin repeats (Figure 38). The relationship of the human SOCS12 contigs (h12.1 and h12.2 ; Figure 40) derived from analysis of human SOCS12 ESTs (Table 12.2) to the mouse SOCS12 DNA sequence is shown in Figure 38. Comparison of mouse cDNA clones and ESTs with human ESTs suggests that the 3' untranslated regions of mouse and human SOCS12 differ significantly. The nucleotide sequence of SOCS12 is shown in SEQ ID NO:37. The nucleotide sequence of human SOCS12 contigs h12.1 and h12.2 are shown in SEQ ID NOs:38 and 39, respectively.

**EXAMPLE 34**

**SOCS13**

Mouse and human SOCS-13 were recognized through searching EST databases using the SOCS box consensus (Figure 13). Those ESTs derived from mouse and human SOCS13 cDNAs are tabulated below (Tables 13.1 and 13.2). Using sequence information derived from mouse ESTs, several oligonucleotides were designed and use to screen, in the conventional manner, a mouse thymus and a mouse embryo cDNA library. Three cDNA clones (62-1, 62-6-7 and 62-14) encoding mouse SOCS13 were isolated, sequenced in their entirety and shown to overlap with the mouse ESTs identified in the database (Figure 41 and 42A). Analysis of the sequence (Figure 42) indicates that the mouse SOCS13 cDNA encodes a protein with a SOCS box at its C-terminus, in addition to a potential WD-40 repeat (Figure 41 and 42B). The relationship of the human SOCS13 contigs (h13.1 and h13.2 ; Figure 43) derived from analysis of human SOCS13 ESTs (Table 13.2) to the mouse SOCS13 DNA sequence is shown in Figure 41. The nucleotide sequence and corresponding amino acid sequence of murine SOCS13 and shown in SEQ ID NOs:40 and 41, respectively. The nucleotide sequence of human SOCS13 contig h13.1 is shown in SEQ ID NO:42.

**EXAMPLE 35**

**SOCS14**

Mouse and human SOCS-14 were recognized through searching EST databases using the SOCS box consensus (Figure 13). Those ESTs derived from mouse and human SOCS14 cDNAs are tabulated below (Tables 14.1 and 14.2). Using sequence information derived from mouse and human ESTs, several oligonucleotides were designed and use to screen, in the conventional manner, a mouse thymus cDNA library, a mouse genomic DNA library and a human thymus cDNA library cloned into λ-bacteriophage. A single genomic DNA clone (57-2) and (5-3-2) cDNA clone encoding mouse SOCS14 were isolated and sequenced in their entirety and shown to overlap with the mouse ESTs identified in the database (Figures 44 and 45A). The entire coding region, in addition to a region of 5' and 3' untranslated regions, of mouse SOCS14 appears to be encoded on a single exon (Figure 44). Analysis of the sequence (Figure 45) confirms that SOCS14 genomic and cDNA clones encode a protein with a SOCS box at its C-terminus in addition to an SH2 domain (Figure 44 and 45B). The relationship of the human SOCS14 contig (h14.1; Figure 14.3) derived from analysis of cDNA clone 5-94-2 and the human SOCS14 ESTs (Table 14.2) to the mouse SOCS14 DNA sequence is shown in Figure 44. The nucleotide sequence and corresponding amino acid sequence of murine SOCS14 are shown in SEQ ID NOs: 43 and 44, respectively.

## EXAMPLE 36

## SOCS15

Mouse and human SOCS15 were recognized through searching DNA databases using the SOCS box consensus (Figure 13). Those ESTs derived from mouse and human SOCS15 cDNAs are tabulated below (Tables 15.1 and 15.2), as are a mouse and human BAC that contain the entire mouse and human SOCS-15 genes. Using sequence information derived from the ESTs and the BACs it is possible to predict the entire amino acid sequence of SOCS15 and as described for the other SOCS genes it is feasible to design specific oligonucleotide probes to allow cDNAs to be isolated. The relationship of the BACs to the ESTs is shown in Figure 46 and the nucleotide and predicted amino acid sequence of the SOCS-15, derived from the mouse and human BACs is shown in Figures 47 and 48. The nucleotide sequence and corresponding amino acid sequence of murine SOCS15 are shown in SEQ ID NOs:46 and 47, respectively. The nucleotide and corresponding amino acid sequence of human SOCS15 are shown in SEQ ID NO:48 and 49, respectively.

## EXAMPLE 37

## SOCS INTERACTION WITH JAK2 KINASE

These Examples show interaction between SOCS and JAK2 kinase. Interaction is mediated *via* the SH2 domain of SOCS1, 2, 3 and CIS. The interaction resulted in inhibition of JAK2 kinase activity by SOCS1 (Figure 49). General interaction between JAK2 and SOCS1, 2, 3, and CIS is shown in Figure 50.

The following methods are employed:

**Immunoprecipitation:** Cos 6 cells were transiently transfected by electroporation and cultured for 48 hours. Cells were then lysed on ice in lysis buffer (50 mM Tris/HCL, pH 7.5, 150 mM NaCl, 1% v/v Triton-X-100, 1 mM EDTA, 1 mM Naf, 1 mM $Na_3VO_4$) with the addition of complete protease inhibitors (Boehringer Mannheim), centrifuged at 4°C (14,000 x g, 10 min) and the supernatant retained for immunoprecipitation. JAK2 proteins were immunoprecipitated using 5 $\mu$l anti-JAK2 antibody (UBI). Antigen-antibody complexes were recovered using protein A-Sepharose (30 $\mu$l of a 50% slurry).

**Western blotting:** Immunoprecipitates were analysed by sodium dodecyl sulphate (SDS)-polyacrylamide gel electrophoresis (PAGE) under reducing conditions. Protein was then electrophoretically transferred to nitrocellulose, blocked overnight in 10% w/v skim-milk and washed in PBS/0.1% v/v Tween-20 (Sigma) (wash buffer) prior to incubation with either anti-phosphotyrosine antibody (4G10) (1:5000, UBI), anti-FLAG antibody (1.6 $\mu$g/ml) or anti-JAK2 antibody (1:2000, UBI) diluted in wash buffer/1% w/v BSA for 2 hr. Nitrocellulose blots were washed and primary antibody detected with either peroxidase-conjugated sheep anti-rabbit immunoglobulin (1:5000, Silenus) or peroxidase-conjugated sheep anti-mouse immunoglobulin (1:5000, Silenus) diluted in wash buffer/1% w/v BSA. Blots were washed and antibody binding visualised using the enhanced chemiluminescence (ECL) system (Amersham, UK) according to the manufacturers' instructions.

***In-vitro* kinase assay:** An *in vitro* kinase assy was performed to assess intrinsic JAK2 kinase catalytic activity. JAK2 protein were immunopreciptated as described, washed twice in kinase assay buffer (50 mM NaCl, 5 mM $MgCl_2$, 5 mM MnCl2, 1 mM NaF, 1 mM $Na_3VO_4$, 10 mM HEPES, pH 7.4) and suspended in an equal volume of kinase buffer containing 0.25 μCi/ml (γ-$^{32}$P)-ATP (30 min, room temperature). Excess (γ- P)-ATP was removed and the immunoprecipitates analysed by SDS/PAGE under reducing conditions. Gels were subjected to a mild alkaline hydrolysis by treatment with 1 M KOH (55°C, 2 hours) to remove phosphoserine and phosphothreonine. Radioactive bands were visualised with IMAGEQUANT software on a PhosphorImage system (Molecular Dynamics, Sunnyvale, CA, USA).

**EXAMPLE 38**

**MAKING SOCS-1 KNOCKOUT CONSTRUCTS**

Diagrams of plasmid constructs and knockout constructs are shown in Figures 51-53. The genomic SOCS-1 clone 95-11-10 was digested with the restriction enzymes BamH1 and EcoR1 to obtain a 3.6Kb DNA fragment 3' of the coding region (SOCS-1 exon), which was used as the 3' arm in the SOCS-1 knockout vectors. The ends of this fragment were then blunted. This fragment was then ligated into the following vectors:

pB galpAloxNeo
and pBgalpAloxNeoTK

which had been linearized at the unique Xho1 site and then blunted. This ligation resulted in the formation of the following vectors:

3'SOCS-1 arm in pBgalpAloxNeo
and 3'SOCS-1 arm in pBgalpAloxNeoTK

The 5' arm of the SOCS-1 knockout vectors was constructed by using PCR to generate a 2.5Kb PCR product from the genomic SOCS-1 clone 95-11-10 just 5' of the SOCS-1 coding region (SOCS-1 exon). The oligo's used to generate this product were:

5' oligo (sense) (2465)
AGCT AGA TCT GGA CCC TAC AAT GGC AGC [SEQ ID NO:49]

3' oligo (antisense) (2466)
AGCT AG ATC TGC CAT CCT ACT CGA GGG GCC AGC TGG [SEQ ID NO:50]

The PCR product was then digested with the restriction enzyme BglII, to generate BglII ends to the PCR product. This 5' SOCS-1 PCR product,with BglII, ends was then ligated as follows: 3'SOCS-1 arm in pBgalpAloxNeo and 3'SOCS-1 arm in pBgalpAloxNeoTK, which had been linearized with the unique restriction enzyme BamH1. This resulted in the following vectors being formed:

5'&3'SOCS-1 arms in pBgalpAloxNeo
and 5'&3'SOCS-1 arms in pBgalpAloxNeoTK

These were the final SOCS-1 knockout constructs. Both these constructs lacked the entire SOCS-1 coding region (SOCS-1 EXON), being replaced with portions of the Bgal, B globin polyA, PGK promoter, neomycin and PGK polyA sequences. The 5'&3'SOCS-1 arms in pBgalpAloxNeoTK vector also contained the tymidine kinase gene sequence, between the neomycin and PGK poly A sequences.

The vectors:

5'&3'SOCS-1 arms in pBgalpAloxNeo
and 5'&3'SOCS-1 arms in pBgalpAloxNeoTK

were linearized with the unique restriction enzyme Not1 and then transfected into Embryonic stem cells by electroporation. Clones which were resistant to neomycin were selected and analysed by southern blot to determine if they contained the correctly integrated SOCS-1 targeting sequence. In order to determine if correct integration had occurred, genomic DNA from the neomycin resistant clones was digested with the restriction enzyme EcoR1. The digested DNA was then blotted onto nylon filters and probed with a 1.5Kb EcoR1 /Hind III DNA fragment, which was further 5' of the 5'arm sequence used in the knockout constructs. The band sizes expected for correct integration were:

Wild type SOCS-1 allele 5.4Kb
SOCS-1 knockout allele 8.2Kb in 5'&3'SOCS-1 arms in pBgalpAloxNeo

or 11Kb in 5'&3'SOCS-1 arms in pBgalpAloxNeoTK transfomed cells.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

**Table 4.1**

**Summary of ESTs derived from mouse SOCS-4 cDNAs**

| SOCS | Species | EST name | End | EST no | Library source | Contig |
|---|---|---|---|---|---|---|
| SOCS-4 | Mouse | mc65f04 | 5' | EST0549700 | d13.5-14.5 mouse embryo | m4.1 |
| | | mf42e06 | 5' | EST0593477 | d13.5-14.5 mouse embryo | m4.1 |
| | | mp10c10 | 5' | EST0747905 | d 8.5 mouse embryo | m4.1 |
| | | mr81g09 | 5' | EST0783081 | d13 embryo | m4.1 |
| | | mt19h12 | 5' | EST0816531 | spleen | m4.1 |

**Table 4.2**

**Summary of ESTs derived from human SOCS-4 cDNAs**

| SOCS | Species | EST name | End | EST no | Library source | Contig |
|---|---|---|---|---|---|---|
| SOCS-4 | Human | 27b5 | 5' | EST0534081 | retina | h4.2 |
| | | 30d2 | 5' | EST0534315 | retina | h4.2 |
| | | J0159F | 5' | EST0461188 | foetal heart | h4.2 |
| | | J3802F | 5' | EST0461428 | foetal heart | h4.2 |
| | | EST19523 | 5' | EST0958884 | retina | h4.2 |
| | | EST81149 | 5' | EST1011015 | placenta | h4.2 |
| | | EST180909 | 5' | EST0951375 | Jurkat T-lymphocyte | h4.2 |
| | | EST182619 | 5' | EST0953220 | JurkatT- lymphocyte | h4.1 |
| | | ya99h09 | 3' | EST0103262 | placenta | h4.2 |
| | | ye70c04 | 5' | EST0172673 | foeatl liver/spleen | h4.2 |
| | | yh53c09 | 5' | EST0197390 | placenta | h4.2 |
| | | | 3' | EST0197391 | | h4.2 |
| | | yh77g11 | 5' | EST0203418 | placenta | h4.2 |
| | | | 3' | EST0203419 | | h4.1 |
| | | yh87h05 | 5' | EST0204888 | placenta | h4.1 |
| | | | 3' | EST0204773 | | h4.1 |

(continued)

**Summary of ESTs derived from human SOCS-4 cDNAs**

| SOCS | Species | EST name | End | EST no | Library source | Contig |
|---|---|---|---|---|---|---|
| | | yi45h07 | 5' | EST0246604 | placenta | h4.2 |
| | | yj04e06 | 5' | EST0258541 | placenta | h4.1 |
| | | | 3' | EST0258285 | | h4.1 |
| | | yq12h06 | 5' | EST0309968 | foetal liver spleen | h4.2 |
| | | yq56a06 | 3' | EST0346924 | foetal liver spleen | h4.2 |
| | | yq60e02 | 5' | EST0347259 | foetal liver spleen | h4.2 |
| | | | 3' | EST0347209 | | h4.2 |
| | | yq92g03 | 5' | EST0355932 | foetal liver spleen | h4.2 |
| | | | | 3' EST0355884 | | h4.2 |
| | | yq97h06 | 5' | EST0357618 | foetal liver spleen | h4.2 |
| | | | 3' | EST0357416 | | h4.2 |
| | | yr90fD1 | 5' | EST0372402 | foetal liver spleen | h4.2 |
| | | yt69c03 | 5' | EST0338395 | foetal liver spleen | h4.2 |
| | | | 3' | EST0338303 | | h4.2 |
| | | yv30a08 8 | 3' | EST0458506 | foetal liver spleen | h4.2 |
| | | yv55f07 | 5' | EST0465391 | foetal liver spleen | h4.2 |
| | | | 3' | EST0463331 | | h4.2 |
| | | yv57h09 | 5' | EST0464336 | foetal liver spleen | h4.2 |
| | | | 3' | EST0458765 | | h4.2 |
| | | yv87h02 | 5' | EST0388085 | melanocyte | h4.2 |
| | | yv98e11 | 5' | EST0400679 | melanocyte | h4.2 |
| | | | 3' | EST0400680 | | h4.2 |
| | | yw68d10 | 5' | EST0441370 | placenta (8-9 wk) | h4.2 |
| | | yw82a03 | 5' | EST0463005 | placenta (8-9 wk) | h4.2 |
| | | | 3' | EST0433678 | | h4.1 |
| | | yx08a07 | 3' | EST0407016 | melanoocyte | h4.1 |
| | | yx72h06 | 5' | EST0435158 | melanoocyte | h4.2 |
| | | | 3' | EST0422871 | melanoocyte | h4.1 |
| | | yx76b09 | 5' | EST0434011 | melanoocyte | h4.2 |

(continued)

**Summary of ESTs derived from human SOCS-4 cDNAs**

| SOCS | Species | EST name | End | EST no | Library source | Contig |
|---|---|---|---|---|---|---|
| | | yy37h08 | 5' | EST0451704 | melanoocyte | h4.2 |
| | | yy66b02 | 5' | EST0505446 | multiple sclerosis lesion | h4.2 |
| | | za81f08 | 5' | EST0511777 | foetal lung | h4.2 |
| | | zb18f07 | 3' | EST0485315 | foetal lung | h4.1 |
| | | zc06e08 | 5' | EST0540473 | parathyroid tumor | h4.1 |
| | | | 3' | EST0540354 | | h4,1 |
| | | zd14g06 | 3' | EST0564666 | foetal heart | h4.1 |
| | | zd51h12 | 3' | EST0578099 | foetal heart | h4.1 |
| | | zd52b09 | 5' | EST0582012 | foetal heart | h4,1 |
| | | | 3' | EST0581958 | | h4. 1 |
| | | ze25g11 | 3' | EST0679543 | foetal heart | h4.1 |
| | | ze69f02 | 5' | EST0635563 | retina | h4,2 |
| | | | 3' | EST0635472 | | h4.1 |
| | | zf54f03 | 5' | EST0680111 | retina | h4.2 |
| | | zh96e07 | 5' | EST0616241 | foetal liver spleen | h4.2 |
| | | | 3' | EST0615745 | | h4.2 |
| | | zv66h12 | 5' | EST1043265 | 8-9w foetus | h4.2 |
| | | zs83a08 | 5' | EST0920072 | germinal centre B cell | h4.1 |
| | | | 3' | EST0920016 | | h4.1 |
| | | zs83g08 | 5' | EST0920121 | germinal centre B cell | h4.1 |
| | | | 3' | EST0920122 | | h4.1 |

**Table 5.1**

**Summary of ESTs derived from mouse SOCS-5 cDNAs**

| SOCS | Species | EST name | End | EST no | Library source | Contig |
|---|---|---|---|---|---|---|
| SOCS-5 | Mouse | mc55a01 | 5' | EST0541556 | d13.5-14.5 mouse embryo | m5.1 |
| | | mh98f09 | 5' | EST0638237 | placenta | m5.1 |
| | | my26h12 | 5' | EST0859939 | mixed organs | m5.1 |
| | | ve24e06 | 5' | EST0819106 | heart | m5.1 |

**Table 5.2**

**Summary of ESTs derived from human SOCS-5 cDNAs**

| SOCS | Species | EST name | End | EST no | Library source | Contig |
|---|---|---|---|---|---|---|
| SOCS-5 | Human | EST15B103 | ? | EST0258029 | adipose tissue | h5.1 |
| | | EST15B105 | ? | EST0258028 | adipose tissue | h5.1 |
| | | EST27530 | 5' | EST0965892 | cerebellum | h5.1 |
| | | zf50f01 | 5' | EST0679820 | retina | h5.1 |

**Table 6.1**

**Summary of ESTs derived from mouse SOCS-6 cDNAs**

| SOCS | Species | EST name | End | EST no | Library source | Contig |
|---|---|---|---|---|---|---|
| SOCS-6 | Mouse | mco4c05 | 5' | EST0525832 | d19.5 embryo | m6.1 |
| | | md48a03 | 5' | EST0566730 | d13.5-14.5 embryo | m6.1 |
| | | mf31d03 | 5' | EST0675970 | d13.5-14.5 embryo | m6.1 |
| | | mh26b07 | 5' | EST0628752 | d13.5-14.5 placenta | m6.1 |
| | | mh78e11 | 5' | EST0637608 | d13.5-14.5 placenta | m6.1 |
| | | mh88h09 | 5' | EST0644383 | d13.5-14.5 placenta | m6.1 |
| | | mh94h07 | 5' | EST0638078 | d13.5-14.5 placenta | m6.1 |
| | | m127h04 | 5' | EST0644252 | d13.5-14.5 embryo | m6.1 |
| | | mj29c05 | 5' | EST0664093 | d13.5-14.5 embryo | m6.1 |
| | | mp66g04 | 5' | EST0757905 | thymus | m6.1 |
| | | mw75g03 | 5' | EST0847938 | liver | m6.1 |
| | | va53b05 | 5' | EST0901540 | d12.5 embryo | m6.1 |
| | | vb34h02 | 5' | EST0930132 | lymph node | m6.1 |
| | | vc55d07 | 3' | EST1057735 | 2 cell embryo | m6.1 |
| | | vc59e05 | 3' | EST10S8201 | 2 cell embryo | m6.1 |
| | | vc67d03 | 3' | EST1057849 | 2 cell embryo | m6.1 |
| | | vc68d10 | 3' | EST1058663 | 2 cell embryo | m6.1 |
| | | vc97h01 | 3' | EST1059343 | 2 cell embryo | m6.1 |

(continued)

Summary of ESTs derived from mouse SOCS-6 cDNAs

| SOCS | Species | EST name | End | EST no | Library source | Contig |
|---|---|---|---|---|---|---|
| | | vc99c08 | 3' | EST1059410 | 2 cell embryo | m6.1 |
| | | vd07h03 | 3' | EST1058173 | 2 cell embryo | m6.1 |
| | | vd08c01 | 3' | ESTI058275 | 2 cell embryo | m6.1 |
| | | vd09b12 | 3' | EST1058632 | 2 cell embryo | m6.1 |
| | | vd19b02 | 3' | EST1059723 | 2 cell embryo | m6.1 |
| | | vd29a04 | 3' | ? none found | | m6.1 |
| | | vd46d06 | 3' | ? none found | | m6.1 |

**Table 6.2**

**Summary of ESTs derived from human SOCS-5 cDNAs**

| SOCS | Species | EST name | End | EST no | Library source | Contig |
|---|---|---|---|---|---|---|
| SOCS-6 | Human | | | | | |
| | | yf61e08 | 5' | EST0184387 | d73 infant brain | h6.1 |
| | | yf93a09 | 5' | EST0186084 | d73 infant brain | h6.1 |
| | | yg05f12 | 5' | EST0191486 | d73 infant brain | h6.1 |
| | | yg41f04 | 5' | EST0195017 | d73 infant brain | h6.1 |
| | | yg45c02 | 5' | EST0185308 | d73 infant brain | h6.1 |
| | | yh11f10 | 5' | EST0236705 | d73 infant brain | h6.1 |
| | | yh13b05 | 5' | EST0237191 | d73 infant brain | h6.1 |
| | | | 3' | EST0236958 | | h6.2 |
| | | zc35a12 | 5' | EST0555518 | senescent fibroblasts | h6.1 |
| | | ze02h08 | 5' | EST0603826 | foetal heart | h6.1 |
| | | | 3' | EST0603718 | | h6.2 |
| | | z109a03 | 5' | EST0773936 | pregnant uterus | h6.1 |
| | | | 3' | EST0773892 | | h6.1 |
| | | z169e10 | 5' | EST0683363 | colon | h6.1 |
| | | zn39d08 | 5' | EST0718885 | endothelial cell | h6.1 |
| | | zo39e06 | 5' | EST0785947 | endothelial cell | h6.1 |

**Table 7.1**

**Summary of ESTs derived from mouse SOCS-7 cDNAs**

| SOCS | Species | EST name | End | EST no | Library source | Contig |
|---|---|---|---|---|---|---|
| SOCS-7 | Mouse | mj39a01 | 5' | EST0665627 | d13.5/14.5 embryo | m7.1 |
| | | vi52h07 | 5' | EST1267404 | d7.5 embryo | m7.1 |

Table 7.2

**Summary of ESTs derived from human SOCS-5 cDNAs**

| SOCS | Species | EST name | End | EST no | Library source | Contig |
|---|---|---|---|---|---|---|
| SOCS-7 | HUMAN | STS WI-30171 | | (G21563) | Chromosome 2 | h7.2 |
| | | EST00939 | 5' | EST0000906 | hippocampus | h7.1 |
| | | EST12913 | 3' | EST0944382 | uterus | h7.2 |
| | | yc29b05 | 3' | EST0128727 | liver | h7.2 |
| | | yp49f10 | 3' | EST0301914 | retina | h7.2 |
| | | zt10f03 | 5' | EST0922932 | germinal centre B cell | h7.2 |
| | | | 3' | EST0921231 | | h7.1 |
| | | zx73g04 | 3' | EST1102975 | ovarian tumour | h7.1 |

**Table 8.1**

**Summary of ESTs derived from mouse SOCS-8 cDNAs**

| SOCS | Species | EST name | End | EST no | Library source | Contig |
|---|---|---|---|---|---|---|
| SOCS-8 | Mouse | mj16e09 | r1 | EST0666240 | d13.5/14.5 embryo | m8.1 |
| | | vj27a029 | r1 | EST1155973 | heart | m8.1 |

**Table 9.1**

**Summary of ESTs derived from mouse SOCS-9 cDNAs**

| SOCS | Species | EST name | End | EST no | Library source | Contig |
|---|---|---|---|---|---|---|
| | Mouse | me65d05 | 5' | EST0585211 | d 13.5/14.5 embryo | m9.1 |

**Table 9.2**

**Summary of ESTs derived from human SOCS-5 cDNAs**

| SOCS | Species | EST name | End | EST no | Library source | Contig |
|---|---|---|---|---|---|---|
| SOCS-9 | Human | CSRL-83f2-u | | (B06659) | chromsome 11 | h9.1 |
| | | EST114054 | 5' | EST0939759 | placenta | h9.1 |
| | | yy06b07 | 3' | EST0434504 | melanocyte | h9.1 |
| | | yy06g06 | 5' | EST0443783 | melanocyte | h9.1 |

(continued)

Summary of ESTs derived from human SOCS-5 cDNAs

| SOCS | Species | EST name | End | EST no | Library source | Contig |
|---|---|---|---|---|---|---|
| | | zr40c09 | 5' | EST0832461 | melanocyte, heart, uterus | h9.1 |
| | | zr72h01 | 5' 3' | EST0892025 | melanocyte, heart, uterus | h9.1 |
| | | | 3' | EST0892026 | | h9.1 |
| | | yx92c08 | 5' | EST0441160 | melanocyte | h9.1 |
| | | yx93b08 | 5' | EST0441260 | melanocyte | h9.1 |
| | | hfe0662 | 5' | EST0889611 | foetal heart | h9.1 |

Table 10.1

Summary of ESTs derived from mouse SOCS-10 cDNAs

| SOCS | Species | EST name | End | EST no | Library source | Contig |
|---|---|---|---|---|---|---|
| | Mouse | mb14d12 | 5' | EST0549887 | d19.5 embryo | m10.1 |
| | | mb40f06 | 5' | EST0515064 | d19.5 embryo | m10.1 |
| | | mg89b11 | 5' | EST0630631 | d13.5-14.5 embryo | m10.1 |
| | | mq89e12 | 5' | EST0776015 | heart | m10.1 |
| | | mp03g12 | 5' | EST0741991 | heart | m10.1 |
| | | vh53c11 | 5' | EST1154634 | mammary gland | m10.1 |

Table 10.2

Summary of ESTs derived from human SOCS-5 cDNAs

| SOCS | Species | EST name | End | EST no | Library source | Contig |
|---|---|---|---|---|---|---|
| SOCS-10 | Human | aa48h10 | 3' | EST1135220 | germinal centre B cell | h10.2 |
| | | zp35h01 | 3' | EST0819137 | muscle | h10.2 |
| | | zp97h12 | 5'<br>3' | EST0835442<br>EST0831211 | muscle | h10.2<br>h10.2 |
| | | zq08h01 | 5' | EST0835907 | muscle | h10.1 |
| | | zr34g05 | 5' | EST0834251 | melanocyte, heart, uterus | h10.2 |
| | | | 3' | EST0834440 | | h10.2 |
| | | EST73000 | 5 | EST1004491 | ovary | h10.2 |
| | | HSDHEI005 | ? | EST0013906 | heart | h10.2 |

**Table 11.1**

**Summary of ESTs derived from human SOCS-5 cDNAs**

| SOCS | Species | EST name | End | EST no | Library source | Contig |
|---|---|---|---|---|---|---|
| SOCS-11 | Human | zt24h06 | r1 | EST0925023 | ovarian tumor | 11.1 |
| | | zr43b02 | r1 | EST0873006 | melanocyte, heart, uterus | 11.1 |
| | | | s1 | EST0872954 | | 11.1 |

**Table 12.1**

**Summary of ESTs derived from mouse SOCS-12 cDNAs**

| SOCS | Species | EST name | End | EST no | Library source | Contig |
|---|---|---|---|---|---|---|
| SOCS-12 | Mouse | EST03803 | 5’ | EST1054173 | day 7.5 emb ectoplacental cone | m12.1 |
| | | mt18f02 | 5’ | EST0817652 | 3NbMS spleen | m12.1 |
| | | mz60g10 | 5’ | EST0890872 | lymph node | m12.1 |
| | | va05c11 | 5’ | EST0909449 | lymph node | m12.1 |

**Table 12.2**

**Summary of ESTs derived from human SOCS-5 cDNAs**

| SOCS | Species | EST name | End | EST no | Library source | Contig |
|---|---|---|---|---|---|---|
| SOCS-12 | Human | STS-SHGC-13867 | | | Chromosome 2 | h12.2 |
| | | EST177695 | 5’ | EST0948071 | Jurkat cells | h12.1 |
| | | EST64550 | 5’ | EST0997367 | Jurkat cells | h12.1 |
| | | EST76868 | 5’ | EST1007291 | pineal body | h12.2 |
| | | PMY2369 | 5’ | EST1115998 | KG-1 | h12.1 |
| | | yb38f04 | 5’ | EST0108807 | foetal spleen | h12.1 |
| | | | 3’ | | | h12.2 |
| | | yg74e12 | 5’ | EST0224407 | d73 brain | h12.1 |
| | | yh13g04 | 5’ | EST0237226 | d73 brain | h12.1 |
| | | | 3’ | EST0236992 | | h12.2 |
| | | yh48b06 | 5’ | yh48b06 | placenta | h12.2 |
| | | yh53a05 | 5’ | EST0197282 | placenta | h12.2 |
| | | | 3’ | EST0197486 | | h12.2 |
| | | yn48h09 | 5’ | EST0278258. | brain | h12.2 |
| | | | 3’ | EST0278259 | | h12.2 |
| | | yn90a09 | 3’ | EST0302557 | brain | h12.2 |

(continued)

Summary of ESTs derived from human SOCS-5 cDNAs

| SOCS | Species | EST name | End | EST no | Library source | Contig |
|---|---|---|---|---|---|---|
| | | yo08f03 | 5' | EST0301790 | brain | h12.2 |
| | | | 3' | EST0302059 | | h12.2 |
| | | yo11e01 | 3' | ? none found | | h12.2 |
| | | yo63b12 | 5' | EST0303606 | breast | h12.2 |
| | | | 3' | EST0304085 | | h12.2 |
| | | yq56g02 | 3' | EST0346935 | foetal liver spleen | h12.1 |
| | | zh57c04 | 3' | EST0594201 | foetal liver spleen | h12.2 |
| | | zh79h01 | 3' | EST0598945 | foetal liver spleen | h12.2 |
| | | zh99a11 | 3' | EST0618570 | foetal liver spleen | h12.2 |
| | | zo92h12 | 5' | EST0803392 | ovarian cancer | h12.1 |
| | | | 3' | EST0803393 | | h12.2 |
| | | zs48c01 | 5' | EST0925714 | germinal centre B cell | h12.1 |
| | | | 3' | EST0925530 | | h12.2 |
| | | zs45h02 | 3' | EST0932296 | germinal centre B cell | h12.2 |

**Table 13.1**

Summary of ESTs derived from mouse SOCS-13 cDNAs

| SOCS | Species | EST name | End | EST no | Library source | Contig |
|---|---|---|---|---|---|---|
| SOCS-13 | Mouse | ma39c09 | 5' | EST0517875 | day 19.5 embryo | m13.1 |
| | | me60c05 | 5' | EST0584950 | day 13.5/14.5 embryo | m13.1 |
| | | mi78g05 | 5' | EST0653834 | day 19.5 embryo | m13.1 |
| | | mk10c11 | 5' | EST0735158 | day 19.5 embryo | m13.1 |
| | | mo48g12 | 5' | EST0745111 | day 10.5 embryo | m13.1 |
| | | mp94a01 | 5' | EST0762827 | thymus | m13.1 |
| | | vb57c07 | 5' | EST1028976 | day 11.5 embryo | m13.1 |
| | | vh07c11 | 5' | EST1117269 | mammary gland | m13.1 |

**Table 13.2**

**Summary of ESTs derived from human SOCS-13 cDNAs**

| SOCS | Species | EST name | End | EST no | Library source | Contig |
|---|---|---|---|---|---|---|
| SOCS-13 | Human | EST59161 | 5' | EST0992726 | infant brain | h13.1 |

**Table 14.1**

**Summary of ESTs derived from mouse SOCS-14 cDNAs**

| SOCS | Species | EST name | End | EST no | Library source | Contig |
|---|---|---|---|---|---|---|
| SOCS-14 | mouse | mi75e03 | 5' | EST0651892 | d19.5 embryo | m14.1 |
| | | vd29h11 | 5' | EST1067080 | 2 cell embryo | m14.1 |
| | | vd53g07 | 5' | EST1119627 | 2 cell embryo | m14.1 |

Table 15.1

**Summary of ESTs derived from mouse SOCS-15 cDNAs**

| SOCS | Species | EST name | End | EST no | Library source | Contig |
|---|---|---|---|---|---|---|
| SOCS-15 | Mouse | mh29b05 | 5' | EST0628834 | placenta | m15.1 |
| | | mh98h09 | 5' | EST0638243 | placenta | m15.1 |
| | | m145a02 | 5' | EST0687171 | testis | m15.1 |
| | | mu43a10 | 5' | EST851588 | thymus | m15.1 |
| | | my38c09 | 5' | EST878461 | pooled organs | m15.1 |
| | | vj37h07 | 5' | EST1174791 | diaphragm | m15.1 |
| | | AC002393 | | | Chromosome 6 BAC | m15.1 |

**Table 15.2**

**Summary of ESTs derived from human SOCS-15 cDNAs**

| SOCS | Species | EST name | End | EST no | Library source | Contig |
|---|---|---|---|---|---|---|
| SOCS-15 | Human | EST98889 | 5' | EST1026568 | thyroid | h15.1 |
| | | ne48bo5 | 3' | EST1138057 | colon tumour | h15.1 |
| | | yb12h12 | 5' | EST0098885 | placenta | h15.1 |
| | | | 3' | EST0098886 | | h15.1 |
| | | HSU47924 | | | Chromosome 12 BAC | h15.1 |

BIBLIOGRAPHY:

Alexander WS, Metcalf D and Dunn AR (1995). Embo Journal 14, 5569-78.

Altschul, S.F. Gish, W. Miller, W. Myers, E.W. & Lipman, D.J. (1990) J. Mol. Biol. 215, 403-10.

Ansari-Lari, M.A., Shen, Y., Munzy, D.M., Lee, W. and Gibbs, R.A. (1997) Genome. Res. 7, 268-280.

Bazan JF (1990). [Review]. Immunology Today 11, 350-4.

Bork, P. (1993) Proteins: Struct. Funct. Genet. 17, 363-374.

Cockwell, L.Y. and Giles, I.G. (1989) Comp. Appl. Biosci. 5, 227-232.

Cutler RL, Liu L, Damen JE and Krystal G (1993). Journal of Biological Chemistry 268, 21463-5.

Darnell J Jr., Kerr IM and Stark GR (1994). Science 264, 1415-21.

David M, Petricoin E3, Benjamin C, Pine R, Weber MJ and Larner AC (1995). Science 269, 1721-3.

David M, Wong L, Flavell R, Thompson SA, Wells A, Larner AC and Johnson GR (1996). Journal of Biological Chemistry 271, 9185-8.

Dugaiczyk A, Haron JA, Stone EM, Dennison OE, Rothblum KN and Schwartz RJ (1983). Biochemistry 22, 1605-13.

Durbin JE, Hackenmiller R, Simon MC and Levy DE (1996). Cell 84, 443-50.

Etzold, T., Ulyanov, A. and Argos, P. (1996) Methods Enzymol. 266, 114-28.

Gearing DP, Nicola NA, Metcalf D, Foote S, Willson TA, Gough NM and Williams L (1989). BioTechnology 7, 1157-1161.

Gupta S, Yan H, Wong LH, Ralph S, Krolewski J and Schindler C (1996). Embo Journal 15, 1075-84.

Hilton DJ (1994). An introduction to cytokine receptors, p8-16 in Guidebook to Cytokines and Their Receptors, Eds: N. A. Nicola. Oxford University Press: Oxford.

Hilton DJ, Hilton AA, Raicevic A, Rakar S, Harrison-Smith M, Gough NM, Begley CG, Metcalf D, Nicola NA and Willson TA (1994). Embo Journal 13, 4765-75.

Hilton DJ, Watowich SS, Katz L and Lodish HF (1996). J. Biol. Chem. 271, 4699-4708.

Ichikawa Y (1969). Journal of Cellular Physiology 74, 223-34.

Ihle JN (1995). Nature 377, 591-4.

Ihle JN, Witthuhn BA, Quelle FW, Yamamoto K and Silvennoinen O (1995). Annual Review of Immunology 13, 369-98.

Kaplan MH, Schindler U, Smiley ST and Grusby MJ (1996a). Immunity 4, 313-9.

Kaplan MH, Sun YL, Hoey T and Grusby MJ (1996b). Nature 382, 174-179.

Levy DE and Stark GR (1996). Molecular & Cellular Biology 16, 369-75.

Metcalf D, Wilson TA, Hilton DJ, DiRago L and Mifsud S. (1995) Leukaemia 9, 1556-1564.

Meraz MA, White JM, Sheehan KC, Bach EA, Rodig SJ, Dighe AS, Kaplan DH, Riley JK, Greenlund AC, Campbell D, Carver-Moore K, DuBois RN, Clark R, Aguet M and Schreiber RD (1996). Cell 84, 431-42.

Mizushima S and Nagata S (1990). Nucleic Acids Research 18, 5322.

Murakami M, Narazaki M, Hibi M, Yawata H, Yasukawa K, Hamaguchi M, Taga T and Kishimoto T (1991). Proc.

Natl. Acad. Sci. USA 88, 11349-11353.

Neer, E.J., Schmidt, C.J., Nambudripad, R. and Smith, T.F. (1994) Nature 371, 297-300.

Nicola NA( (1994). Guidebook to Cytokines and Their Receptors. Oxford University Press: Oxford.

Nicola, NV, Viney E, Hilton DJ, Roberts B and Wilson T. (1996) Growth Factors 13, 141-149.

Novak U, Harpur AG, Paradiso L, Kanagasundaram V, Jaworowski A, Wilks AF and Hamilton JA (1995). Blood 86, 2948-56.

Pearson WR and Lipman DJ. (1988) Proc. Natl. Acad. Sci. USA 85, 2444-8.

Pearson WR. (1990) Methods Enzymol. 183, 63-98.

Rayner JR and Gonda TJ (1994). Molecular & Cellular Biology 14, 880-7.

Sambrook J, Fritsch EF and Maniatis T (1989). Molecular Cloning, A Laboratory Manual. Cold Spring Harbour Laboratory Press, Cold Spring Harbour USA.

Sato N, Sakamaki K, Terada N, Arai K and Miyajima A (1993). Embo Journal 12, 4181-9.

Shimoda K, van Deursen J, Sangster MY; Sarawar SR, Carson RT, Tripp RA, Chu C, Quelle FW, Nosaka T, Vignali DA, Doherty PC, Grosveld G, Paul WE and Ihle JN (1996). Nature 380, 630-3.

Shual K, Ziemiecki A, Wilks AF, Harpur AG, Sadowski HB, Gilman MZ and Darnell JE (1993). Nature 366, 580-3.

Sprang SR and Bazan JF (1993). Curr. Opin. Structural Biol. 3, 815-827.

Takeda K, Tanaka T, Shi W, Matsumoto M, Minami M, Kashiwamura S, Nakanishi K, Yoshida N, Kishimoto T and Akira S (1996). Nature 380, 627-30.

Thierfelder WE, Vandeursen JM, Yamamoto K, Tripp RA, Sarawar SR, Carson RT, Sangster MY, Vignali DDA, Doherty PC, Grosveld GC and Ihle JN (1996). Nature 382, 171-174.

Wakao H, Gouilleux F and Groner B (1994). Embo Journal 13, 2182-91.

Wen Z, Zhong Z and Darnell J Jr. (1995). Cell 82, 241-50.

Yi T, Mui AL, Krystal G and Ihle JN (1993). Molecular & Cellular Biology 13, 7577-86.

Yoshimura A, Ohkubo T, Kiguchi T, Jenkins NA, Gilbert DJ, Copeland NG, Hara T and Miyajima A (1995). Embo Journal 14, 2816-26.

## Claims

1. A nucleic acid molecule comprising a sequence of nucleotides encoding or complementary to a sequence encoding a protein or a derivative, homologue, analogue or mimetic thereof or a nucleotide sequence capable of hybridizing thereto under low stringency conditions at 42°C wherein said protein comprises in its C-terminal region a SOCS box comprising the amino acid sequence:

$$X_1\ X_2\ X_3\ X_4\ X_5\ X_6\ X_7\ X_8\ X_9\ X_{10}\ X_{11}\ X_{12}\ X_{13}\ X_{14}\ X_{15}\ X_{16}\ [X_i]_n\ X_{17}\ X_{18}\ X_{19}\ X_{20}\ X_{21}\ X_{22}\ X_{23}\ [X_j]_n\ X_{24}\ X_{25}\ X_{26}\ X_{27}\ X_{28}$$

wherein:

$X_1$ is L, I, V, M, A or P;
$X_2$ is any amino acid residue;
$X_3$ is P, T or S;
$X_4$ is L, I, V, M, A or P;
$X_5$ is any amino acid;
$X_6$ is any amino acid;
$X_7$ is L, I, V, M, A, F, Y or W;
$X_8$ is C, T or S;
$X_9$ is R, K or H;
$X_{10}$ is any amino acid;
$X_{11}$ is any amino acid;
$X_{12}$ is L, I, V, M, A or P;
$X_{13}$ is any amino acid;
$X_{14}$ is any amino acid;
$X_{15}$ is any amino acid;
$X_{16}$ is L, I, V, M, A, P, G, C, T or S;
$[X_i]_n$ is a sequence of n amino acids wherein n is from 1 to 50 amino acids and wherein the sequence $X_i$, may comprise the same or different amino acids selected from any amino acid residue;
$X_{17}$ is L, I, V, M, A or P;
$X_{18}$ is any amino acid;
$X_{19}$ is any amino acid;
$X_{20}$ L, I, V, M, A or P;
$X_{21}$ is P;
$X_{22}$ is L, I, V, M, A, P or G;
$X_{23}$ is P or N;

$[X_j]_n$ is a sequence of n amino acids wherein n is from 1 to 50 amino acids and wherein the sequence $X_j$ may comprise the same or different amino acids selected from any amino acid residue;
$X_{24}$ is L, I, V, M, A or P;
$X_{25}$ is any amino acid;
$X_{26}$ is any amino acid;
$X_{27}$ is Y or F; and
$X_{28}$ is L, I, V, M, A or P.

**2.** A nucleic acid molecule according to claim 1 wherein said nucleic acid molecule comprises a nucleotide sequence encoding, or complementary to a sequence encoding, a protein which comprises the amino acid sequence of any one of SEQ ID NOS. 4, 6, 10, 12, 14, 16, 18, 21, 25, 29, 36, 41, 44, 46 or 48 or an amino acid sequence having at least 70% similarity to any one of SEQ ID NOS. 4, 6, 10, 12, 14, 16, 18, 21, 25, 29, 36, 41, 44, 46 or 48, wherein said protein comprises a SOCS box in its C-terminal region and modulates signal transduction.

**3.** A nucleic acid molecule according to claim 1 or claim 2 wherein the nucleotide sequence is as set forth in any one of SEQ ID NOS. 3, 5, 7, 9, 11, 13, 15, 16, 17, 20, 22, 23, 24, 26, 27, 28, 30, 31, 32, 33, 34, 35, 37, 38, 39, 40, 42, 43, 45 or 47, or has at least 70% similarity to SEQ ID NOS. 3, 5, 7, 9, 11, 13, 15, 16, 17, 20, 22, 23, 24, 26, 27, 28, 30, 31, 32, 33, 34, 35, 37, 38, 39, 40, 42, 43, 45, or is capable of hybridizing to 3, 5, 7, 9, 11, 13, 15, 16, 17, 20, 22, 23, 24, 26, 27, 28, 30, 31, 32, 33, 34, 35, 37, 38, 39, 40, 42, 43, 45 or 47 under medium stringency conditions at 42°C.

**4.** An isolated protein comprising the amino acid sequence of any one of SEQ ID NOS. 4, 6, 10, 12, 14, 16, 18, 21, 25, 29, 36, 41, 44, 46 or 48 or an amino acid sequence having at least 70% similarity to any one of SEQ ID NOS. 4, 6, 10, 12, 14, 16, 18, 21, 25, 29, 36, 41, 44, 46 or 48, wherein the protein comprises a SOCS box in its C-terminal region and modulates signal transduction.

**5.** A nucleic acid molecule according to any one of claims 1 to 3 or a protein according to claim 4, wherein the protein comprises a protein:molecule interacting region, preferably a protein:DNA binding region or a protein:protein binding region, and more preferably an SH2 domain, WD-40 repeat or ankyrin repeat, in a region N-terminal of the SOCS box.

**6.** A nucleic acid molecule according to any one of claims 1 to 3 or 5 or a protein according to any one of claims 4 or 5, wherein the signal transduction is mediated by a cytokine or other endogenous molecule, a hormone, a microbe or a microbial product, a parasite, an antigen or other effector molecule.

**7.** A nucleic acid molecule or a protein according to claim 6 wherein the protein modulates cytokine-mediated signal transduction.

**8.** A nucleic acid molecule or a protein according to claim 7 wherein the signal transduction is mediated by one or more of the cytokines EPO, TPO, G-CSF, GM-CSF, IL-3, IL-2, IL-4, IL-7, IL-13, IL-6, LIF, IL-12, IFN-$\gamma$, TNF$\alpha$, IL-1, OSM, thrombopoietin and/or M-CSF.

**9.** An *in vitro* method of modulating levels of a SOCS protein in a cell said method comprising contacting a cell containing a SOCS gene with an effective amount of a modulator of the expression of a SOCS gene which comprises a nucleic acid molecule as defined in any one of claims 1 to 3 and 5 to 8 or a modulator of the activity of a SOCS protein as defined in any one of claims 4 to 8 for a time and under conditions sufficient to modulate levels of said SOCS protein.

**10.** An *in vitro* method of modulating signal transduction in a cell containing a SOCS gene comprising contacting said cell with an effective amount of a modulator of the expression of a SOCS gene which comprises a nucleic acid molecule as defined in any one of claims 1 to 3 and 5 to 8 or a modulator of the activity of a SOCS protein as defined in any one of claims 4 to 8 for a time sufficient to modulate signal transduction.

**11.** An *in vitro* method of influencing interaction between cells wherein at least one cell carries a SOCS gene, said method comprising contacting the cell carrying the SOCS gene with an effective amount of a modulator of the expression of a SOCS gene which comprises a nucleic acid molecule as defined in any one of claims 1 to 3 and 5 to 8 or a modulator of the activity of a SOCS protein as defined in any one of claims 4 to 8 for a time sufficient to modulate signal transduction.

**12.** A method according to any one of claims 9 to 11 wherein signal transduction is mediated by a mediator as defined in any one of claims 6 to 8.

13. An antisense oligonucleotide having a nucleotide sequence complementary to at least a portion of the "sense" sequence of a nucleic acid molecule as defined in any one of claims 1 to 3 or 6 to 8, said antisense oligonucleotide being an antagonist of SOCS capable of inhibiting SOCS gene expression.

14. A pharmaceutical composition comprising a protein as defined in any one of claims 3 to 8 and one or more pharmaceutically acceptable carriers and/or diluents.

15. A vector comprising a nucleic acid molecule as defined in any one of claims 1 to 3 or 5 to 8.

16. A transgenic mammalian cell line expressing a nucleic acid molecule as defined in any one of claims 1 to 3 or 5 to 8.

17. Use of a protein as defined in any one of claims 4 to 8 in the manufacture of a medicament for the treatment of hyperimmunity, immunosuppression, allergies and hypertension.

18. Use of a modulator of the expression of a SOCS gene comprising a nucleotide sequence as defined in any one of claims 1 to 3 or 5 to 8 or of the activity of a SOCS protein as defined in any one of claims 4 to 8, in the manufacture of a medicament for treatment of hyperimmunity, immunosuppression, allergies and hypertension.

19. An antibody or fragment thereof, that is specific for a protein according to any one of claims 4 to 8.

20. An antibody or fragment thereof according to claim 19 wherein said antibody is a polyclonal antibody, a monoclonal antibody, a synthetic antibody or a recombinant antibody.

21. An antibody or fragment thereof that is specific for an antibody or fragment according to claim 19 or claim 20 and that is optionally labelled with a reporter molecule.

22. A method for detecting a protein according to any one of claims 4 to 8 in a sample, said method comprising contacting said sample with an antibody or fragment thereof according to claim 19 or claim 20 for a time and under conditions sufficient for a complex to form between said protein and said antibody or antibody fragment and then detecting said complex.

23. A method according to claim 22 wherein the sample is a biological sample or is fermentation fluid or supernatant fluid such as from a cell culture.

24. A method according to claim 23 wherein the biological sample is a cell extract or is a biological fluid.

25. A method according to any one of claims 22 to 24 wherein the method is an immunoassay.

26. A method according to claim 25 wherein the immunoassay is an ELISA or a sandwich assay or a Western blot.

27. Use of an antibody or fragment thereof according to any one of claims 19 to 21 for purifying a protein according to any one of claims 4 to 8.

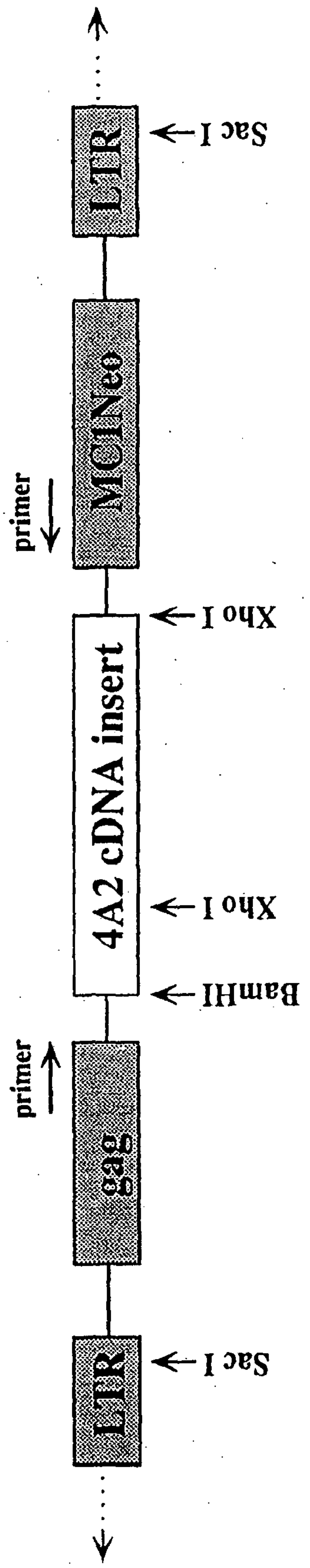
LTR
Sac I
MC1Neo
primer
Xho I
4A2 cDNA insert
Xho I
BamHI
primer
gag
LTR
Sac I

FIGURE 1

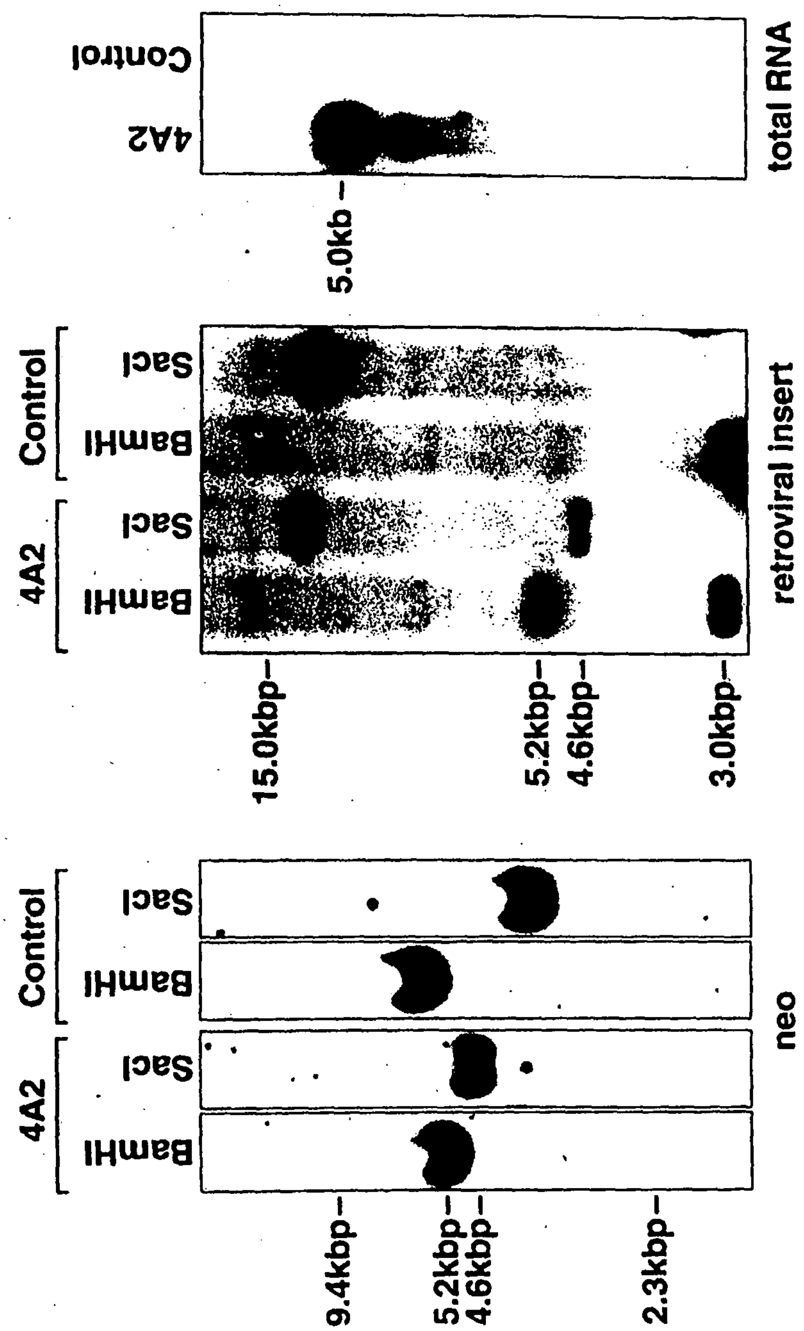
4A2
Control
total RNA
5.0kb
BamHI
SacI
retroviral insert
15.0kbp
5.2kbp
4.6kbp
3.0kbp
neo
9.4kbp
2.3kbp

FIG 2

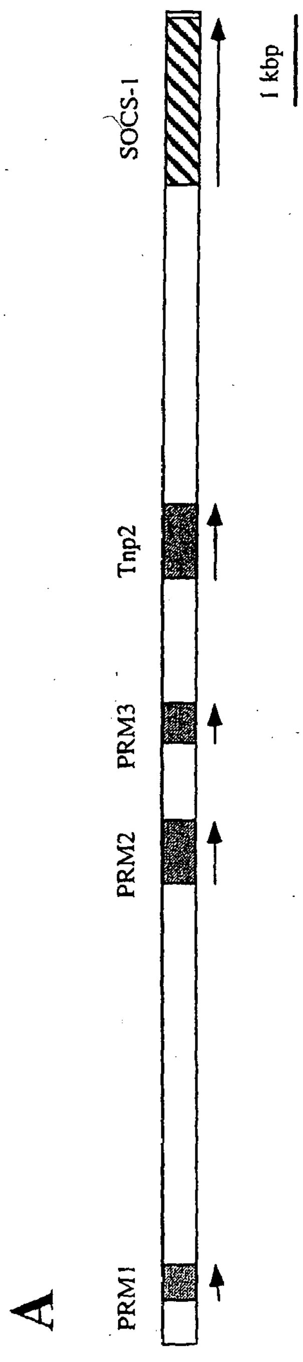


FIG 3A

```
-159                              cgaggctcaagctccgggcggattctgcgtgccgctctcg
-120 ctccttggggtctgttggccggcctgtgccacccggacgcccggctcactgcctctgtct
-60  ccccccatcagcgcagccccggacgctatggcccacccctccagctggcccctcgagtagg

1    M  V  A  R  N  Q  V  A  A  D  N  A  I  S  P  A  A  E  P  R
1    ATGGTAGCACGCAACCAGGTGGCAGCCGACAATGCGATCTCCCCGGCAGCAGAGCCCCGA

21   R  R  S  E  P  S  S  S  S  S  S  S  P  A  A  P  V  R  P
61   CGGCGGTCAGAGCCCTCCTCGTCCTCGTCTTCGTCCTCGCCAGCGGCCCCCGTGCGTCCC

41   R  P  C  P  A  V  P  A  P  A  P  G  D  T  H  F  R  T  F  R
121  CGGCCCTGCCCGGCGGTCCCAGCCCCAGCCCCTGGCGACACTCACTTCCGCACCTTCCGC

61   S  H  S  D  Y  R  R  I  T  R  T  S  A  L  L  D  A  C  G  F
181  TCCCACTCCGATTACCGGCGCATCACGCGGACCAGCGCGCTCCTGGACGCCTGCGGCTTC

81   Y  W  G  P  L  S  V  H  G  A  H  E  R  L  R  A  E  P  V  G
241  TATTGGGGACCCCTGAGCGTGCACGGGGCGCACGAGCGGCTGCGTGCCGAGCCCGTGGGC

101  T  F  L  V  R  D  S  R  Q  R  N  C  F  F  A  L  S  V  K  M
301  ACCTTCTTGGTGCGCGACAGTCGTCAACGGAACTGCTTCTTCGCGCTCAGCGTGAAGATG

121  A  S  G  P  T  S  I  R  V  H  F  Q  A  G  R  F  H  L  D  G
361  GCTTCGGGCCCCACGAGCATCCGCGTGCACTTCCAGGCCGGCCGCTTCCACTTGGACGGC

141  S  R  E  T  F  D  C  L  F  E  L  L  E  H  Y  V  A  A  P  R
421  AGCCGCGAGACCTTCGACTGCCTTTTCGAGCTGCTGGAGCAcTACGTGGCGGCGCCGCGC

161  R  M  L  G  A  P  L  R  Q  R  R  V  R  P  L  Q  E  L  C  R
481  CGCATGTTGGGGGCCCcGCTGCGCCAGCGCCGCGTGCGGCCGCTGCAGGAGCTGTGTCGC

181  Q  R  I  V  A  A  V  G  R  E  N  L  A  R  I  P  L  N  P  V
541  CAGCGCATCGTGGCCGCCGTGGGTCGCGAGAACCTGGCGCGCATCCcTCTTAACCCGGTA

201  L  R  D  Y  L  S  S  F  P  F  Q  I  *
601  CTCCGTGACTACCTGAGTTCCTTCCCCTTCCAGATCtgaccggctgccgctgtgccgcag

661  cattaagtgggggcgccttattatttcttattattaattattattatttttctggaacca
721  cgtgggagccctccccgcctgggtcggagggagtggttgtggagggtgagatgcctccca
781  cttctggctggagacctcatcccacctctcaggggtgggggtgctcccctcctggtgctc
841  cctccgggtccccccctggttgtagcagcttgtgtctggggccaggacctgaattccactc
901  ctacctctccatgtttacatattcccagtatctttgcacaaaccaggggtcggggagggt
961  ctctggcttcatttttctgctgtgcagaatatcctattttatatttttacagccagttta
1021 ggtaataaactttattatgaaagtttttttttaaaagaaaaaaaaaaaaaaaaa
```

FIG 3B

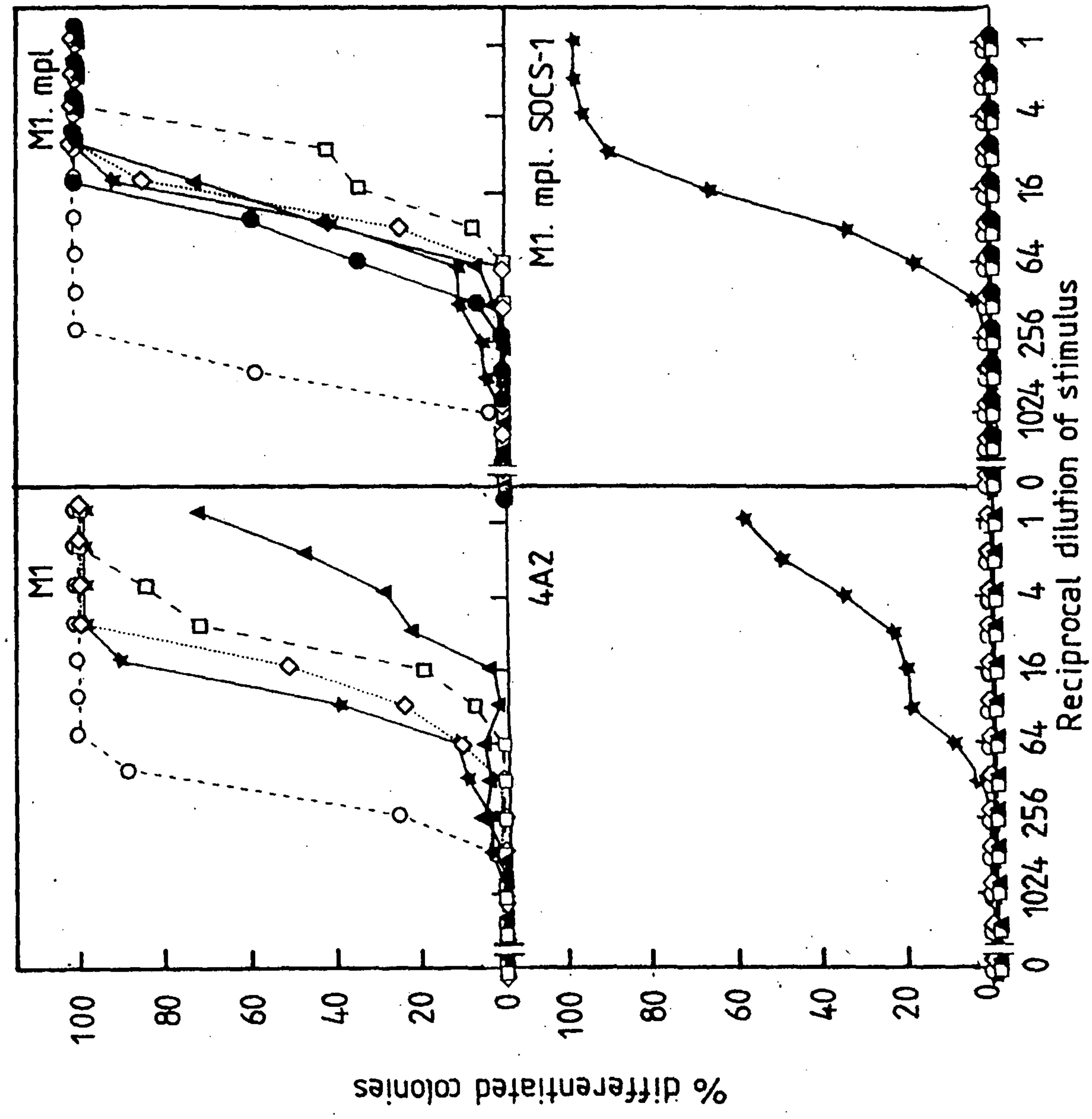
M1
M1. mpl
4A2
M1. mpl. SOCS-1
% differentiated colonies
100
80
60
40
20
0
0
1024
256
64
16
4
1
Reciprocal dilution of stimulus

FIG 4

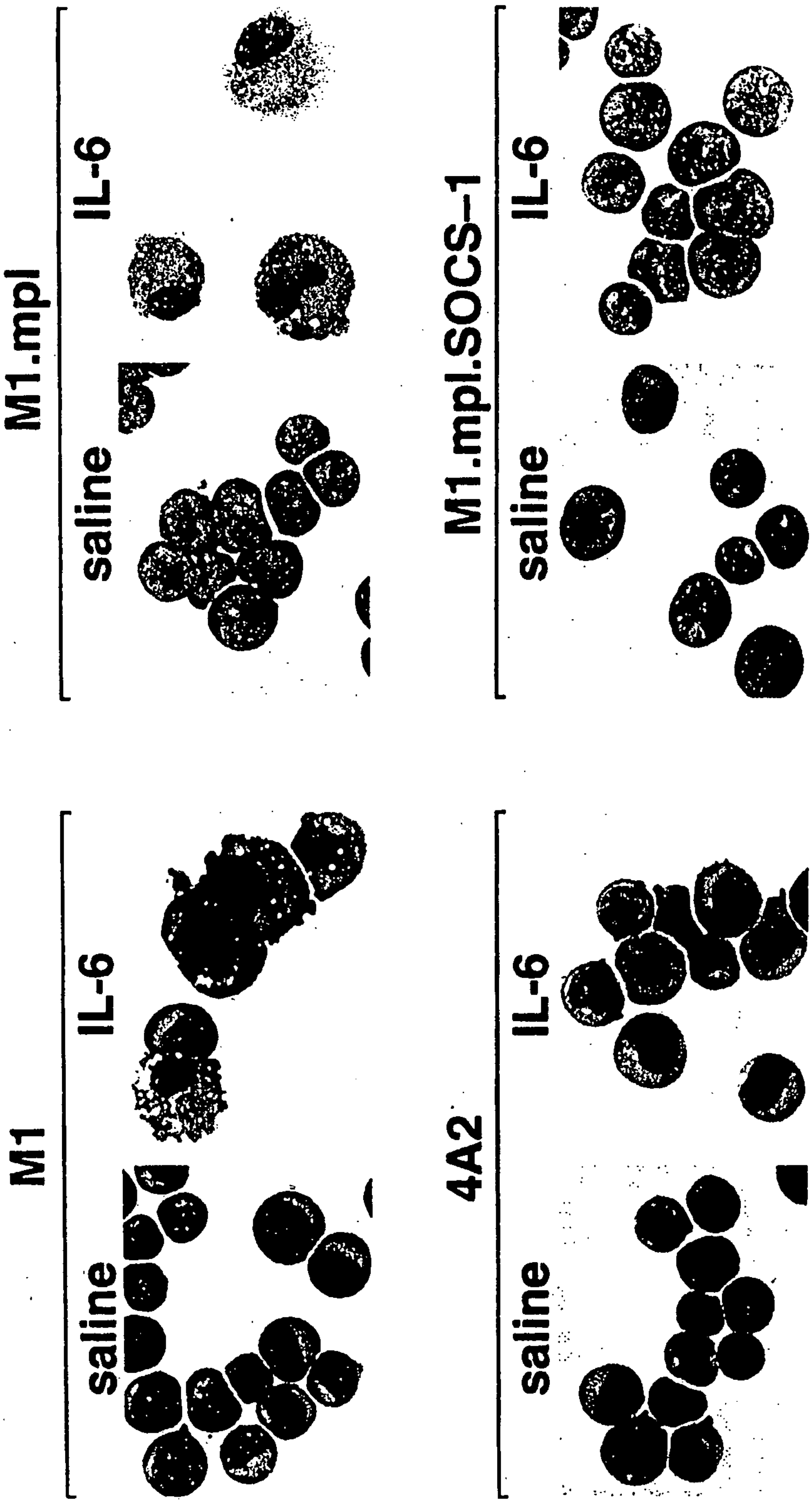
M1.mpl
saline
IL-6
M1.mpl.SOCS–1
saline
IL-6
M1
saline
IL-6
4A2
saline
IL-6

FIG 5

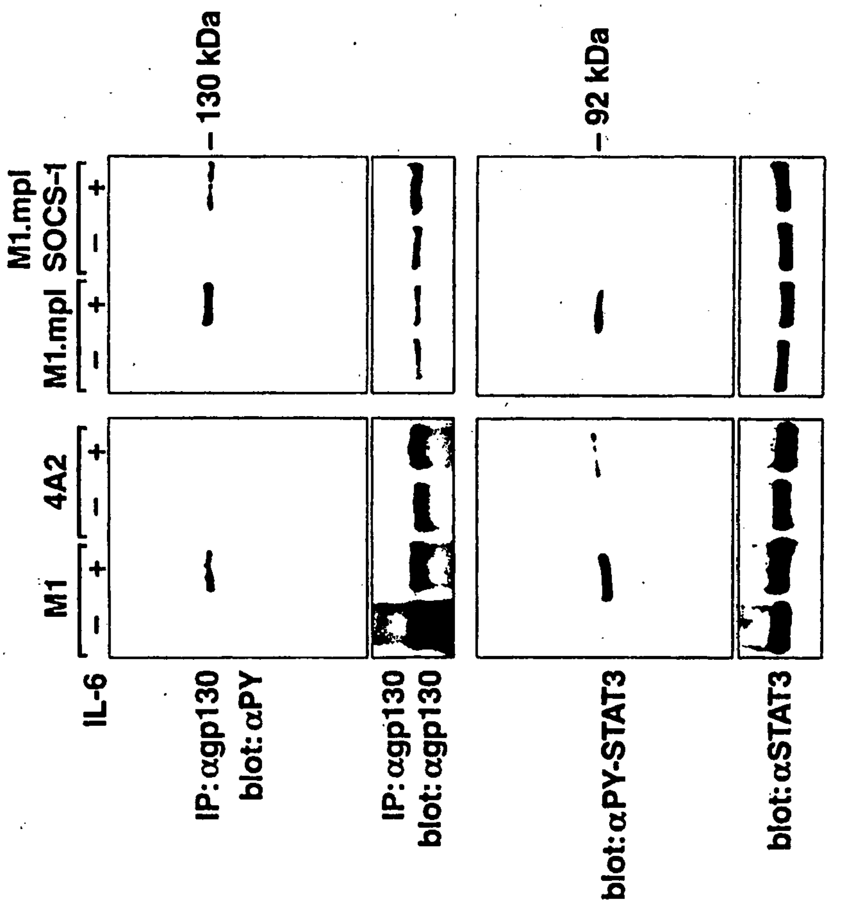
IL-6
M1
4A2
M1.mpl
M1.mpl SOCS-1
- + - + - + - +
IP: αgp130
blot: αPY
IP: αgp130
blot: αgp130
blot: αPY-STAT3
blot: αSTAT3
130 kDa
92 kDa

FIG 6

FIG 7A

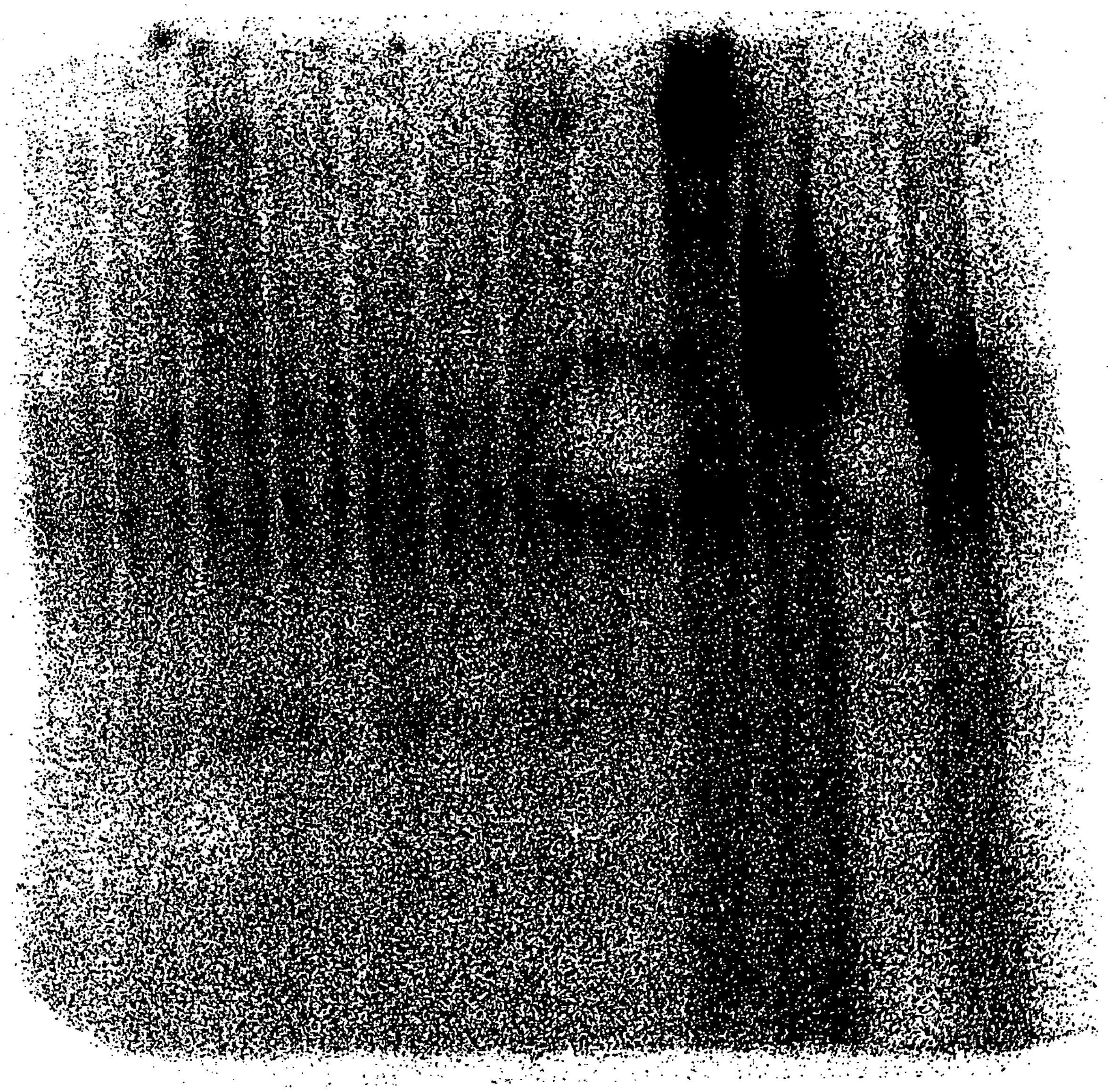

FIG 7B

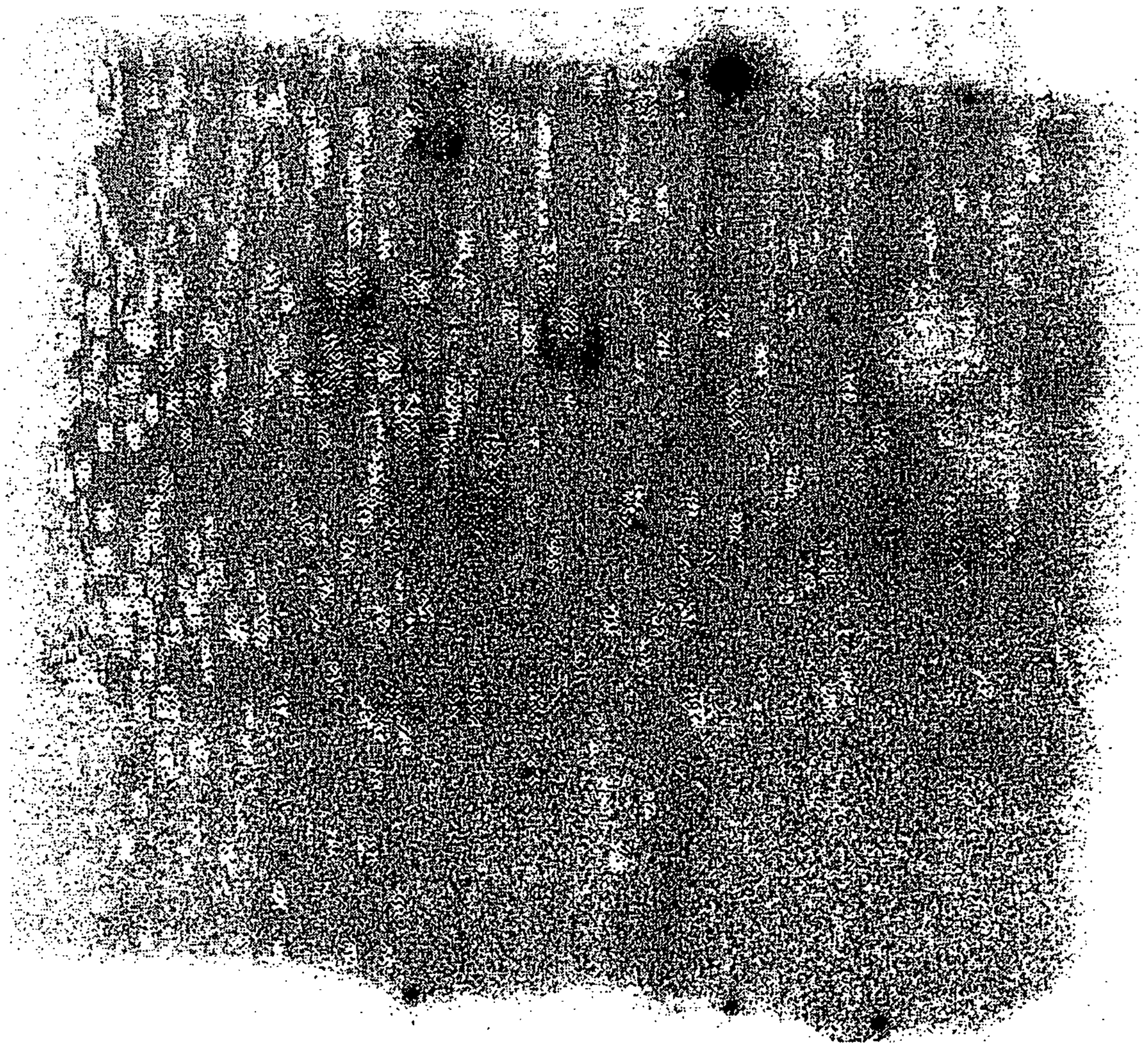

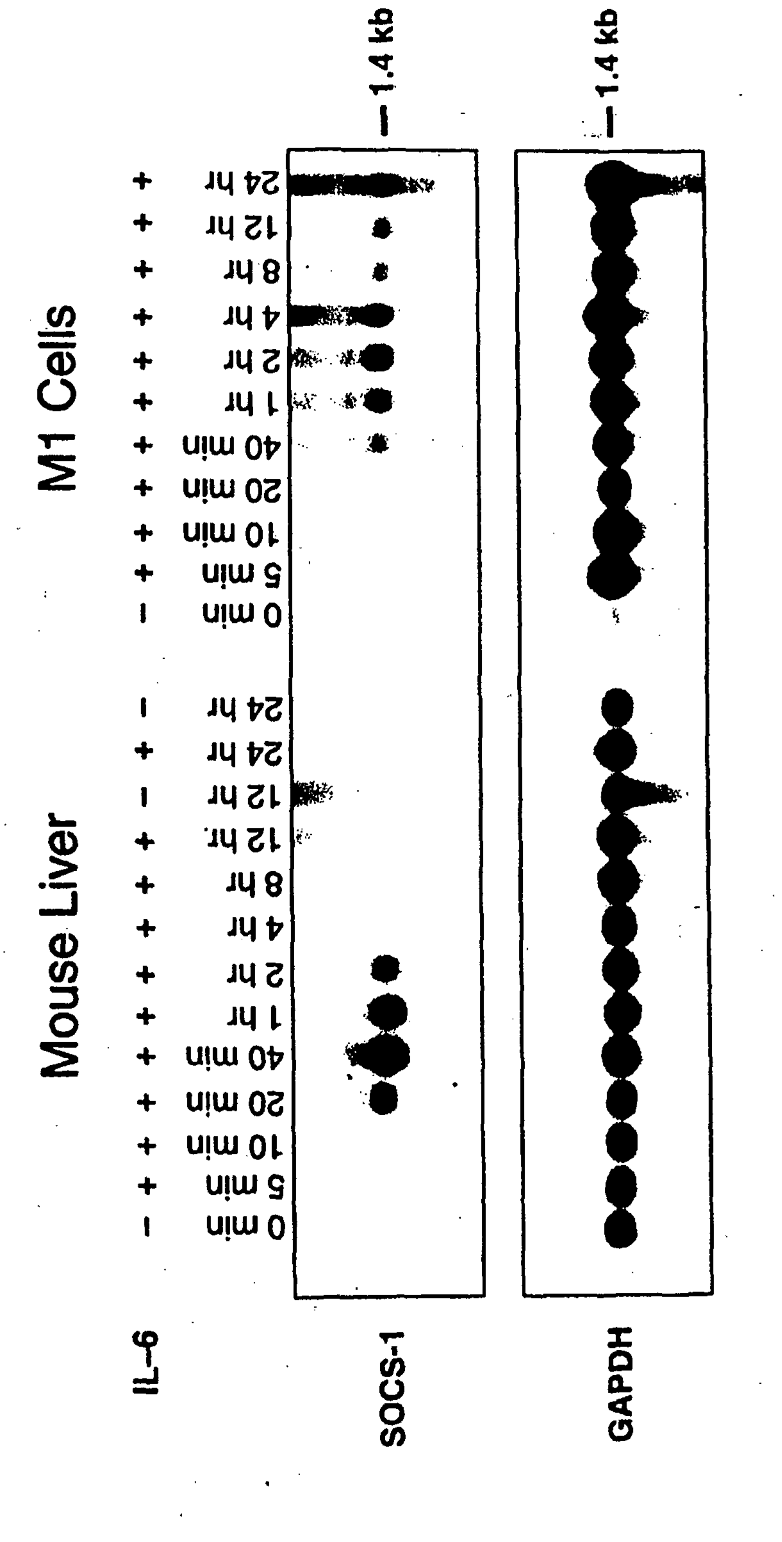

Mouse Liver
M1 Cells
IL-6
SOCS-1
GAPDH
1.4 kb
1.4 kb
0 min
5 min
10 min
20 min
40 min
1 hr
2 hr
4 hr
8 hr
12 hr
12 hr
24 hr
24 hr
0 min
5 min
10 min
20 min
40 min
1 hr
2 hr
4 hr
8 hr
12 hr
24 hr


FIG 8

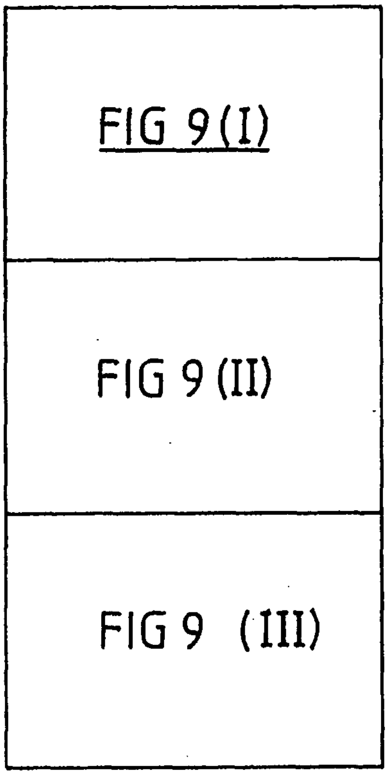


FIG 9

```
hs SOCS-1  (1)  MVAHNQVAADNAVSTAAEPRRRPEPSSSSSSS-PAAPARPRPCPAVPAPA  (49)
rr SOCS-1  (1)  MVARNQVEADNAISPASEPRRRPEPSSSSSSSSPAAPARPRPCPVVPAPA  (50)
mm SOCS-1  (1)  MVARNQVAADNAISPAAEPRRRSEPSSSSSSSSPAAPVRPRPCPAVPAPA  (50)
mm SOCS-2  (1)  MTLRCLEPSGNGADRTRSQWGTAGLPEEQSPEA-----------------  (33)
mm SOCS-3  (1)  MVTHSKFPAAGMSR------------------------------------  (14)
mm CIS     (1)  MVLCVQGSCPLLAVEQIGRRPLWAQSLELPGPAMQPLPTGAFPEEVTEET  (50)

hs SOCS-1  (50) PGDTHF--RTFRSHADYRRITRASALLDACGFYWGPLSVHGAHERLRAEP  (97)
rr SOCS-1  (51) PGDTHF--RTFRSHSDYRRITRTSALLDACGFYWGPLSVHGAHERLRSEP  (98)
mm SOCS-1  (51) PGDTHF--RTFRSHSDYRRITRTSALLDACGFYWGPLSVHGAHERLRAEP  (98)
mm SOCS-2  (34) -----------------ARLAKALRELSQTGWYWGSMTVNEAKEKLKEAP  (66)
mm SOCS-3  (15) PLDTSLRLKTFSSKSEYQLVVNAVRKLQESGFYWSAVTGGEANLLLSAEP  (64)
mm CIS     (51) PVQAENEPKVLDPEGDLLCIAKTFSYLRESGWYWGSITASEARQHLQKMP (100)
```

FIG 9 (I)

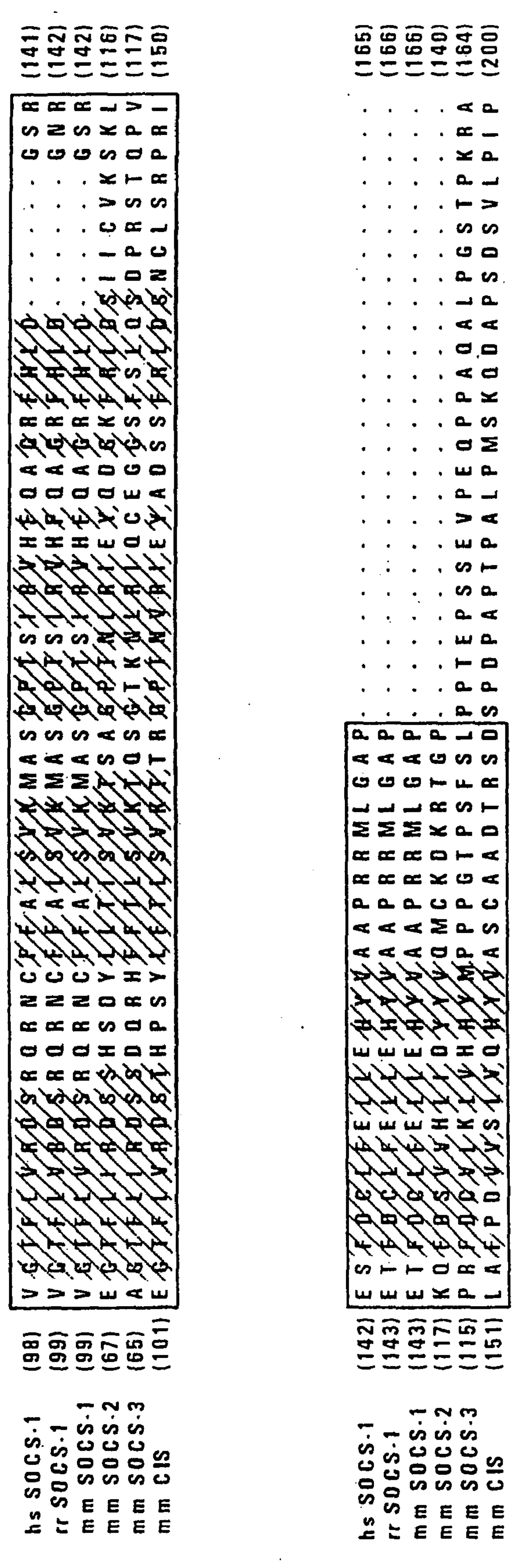

FIG 9(II)

```
hs SOCS-1  (166) - - - - - - - - - - - - - - - - - - L R Q R R V R P L Q E L C R Q R I V A T V G R - E N L A R I P L N P (198)
rr SOCS-1  (167) - - - - - - - - - - - - - - - - - - L R Q R R V R P L Q E L C R Q R I V A A V G R - E N L A R I F L N P (199)
mm SOCS-1  (167) - - - - - - - - - - - - - - - - - - L R Q R R V R P L Q E L C R Q R I V A A V G R - E N L A R I F L N P (199)
mm SOCS-2  (141) - - E A P R N G T V H L Y L T K P L Y T S A P T L Q H F C R L A I N K C T G T - - - I W G L P L P T (185)
mm SOCS-3  (165) Y Y I Y S G G E K I P L V L S R P L S S N V A T L Q H L C R K T V N G H L D S Y E K V T Q L P G P - (213)
mm CIS     (201) - - - V A T A V H L K L V Q P F V R R S S A R S L D H L C R L V I N R L V A D - - - V D C L P I P R (244)

hs SOCS-1  (199) V L R D Y L S S F P F Q I * (211)
rr SOCS-1  (200) V L R D Y L S S F P F Q I * (212)
mm SOCS-1  (200) V L R D Y L S S F P F Q I * (212)
mm SOCS-2  (186) R L K D Y L E E Y K F Q V * (198)
mm SOCS-3  (214) - I R E F L D Q Y D A P L * (225)
mm CIS     (245) R M A D Y L R Q Y P F Q L * (257)
```

FIG 9(III)

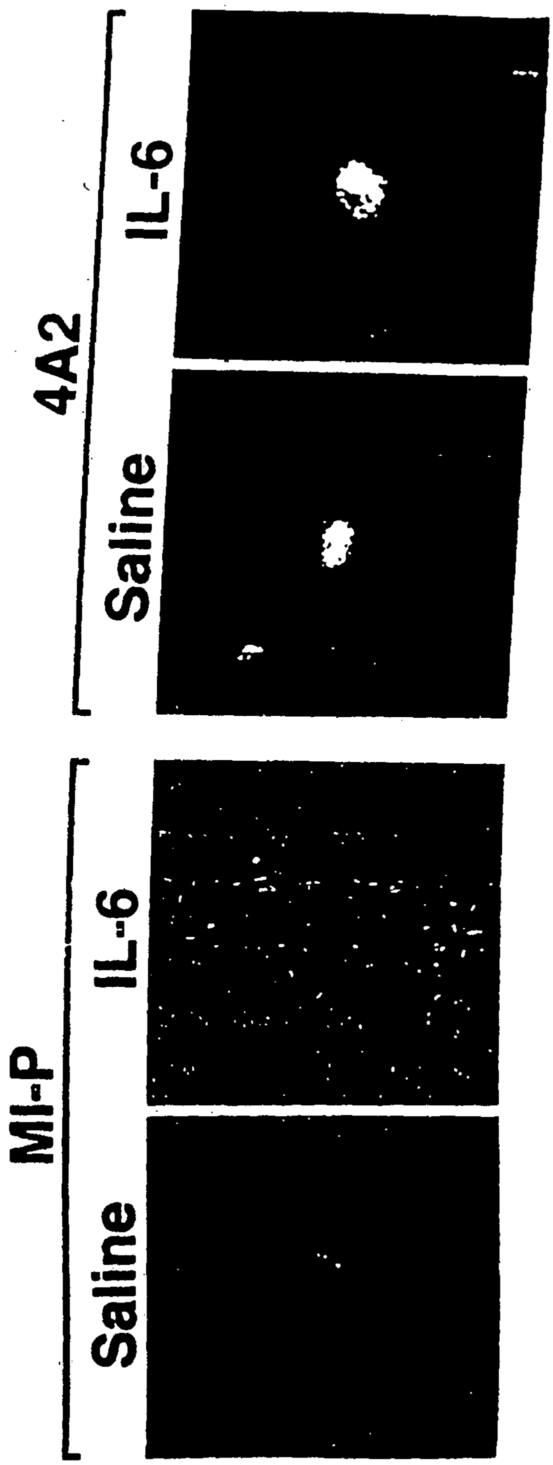


FIG 10

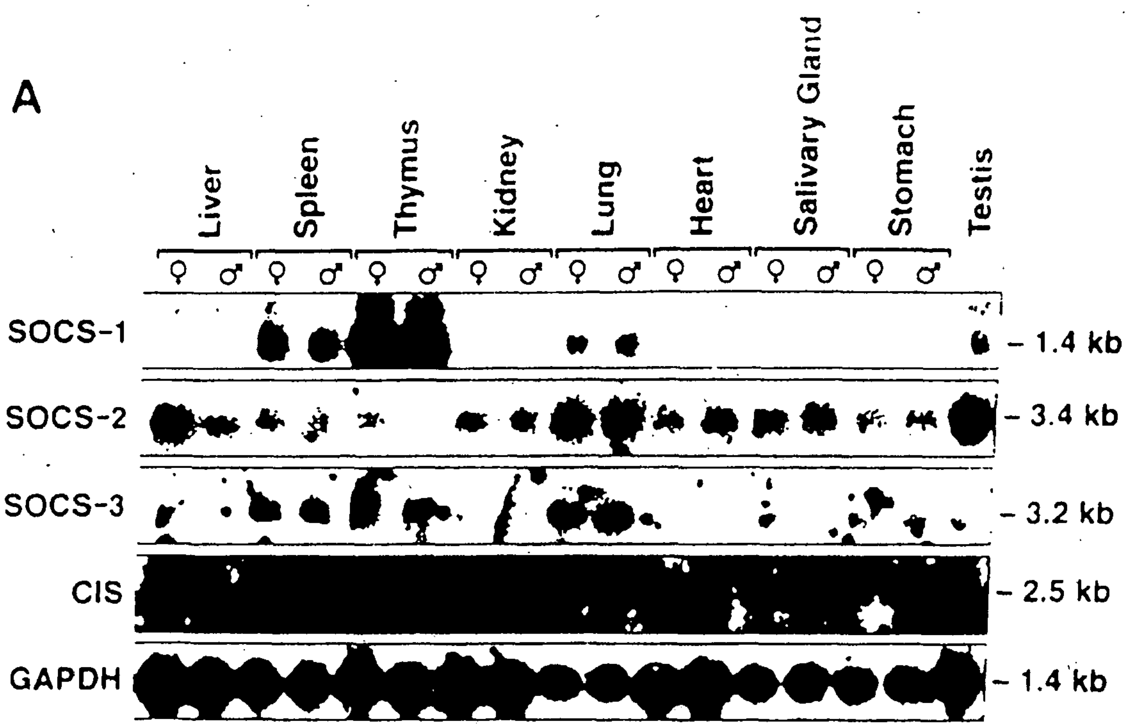
A
Liver
Spleen
Thymus
Kidney
Lung
Heart
Salivary Gland
Stomach
Testis
♀ ♂ ♀ ♂ ♀ ♂ ♀ ♂ ♀ ♂ ♀ ♂ ♀ ♂ ♀ ♂
SOCS-1
SOCS-2
SOCS-3
CIS
GAPDH
– 1.4 kb
– 3.4 kb
– 3.2 kb
– 2.5 kb
– 1.4 kb

FIG 11A

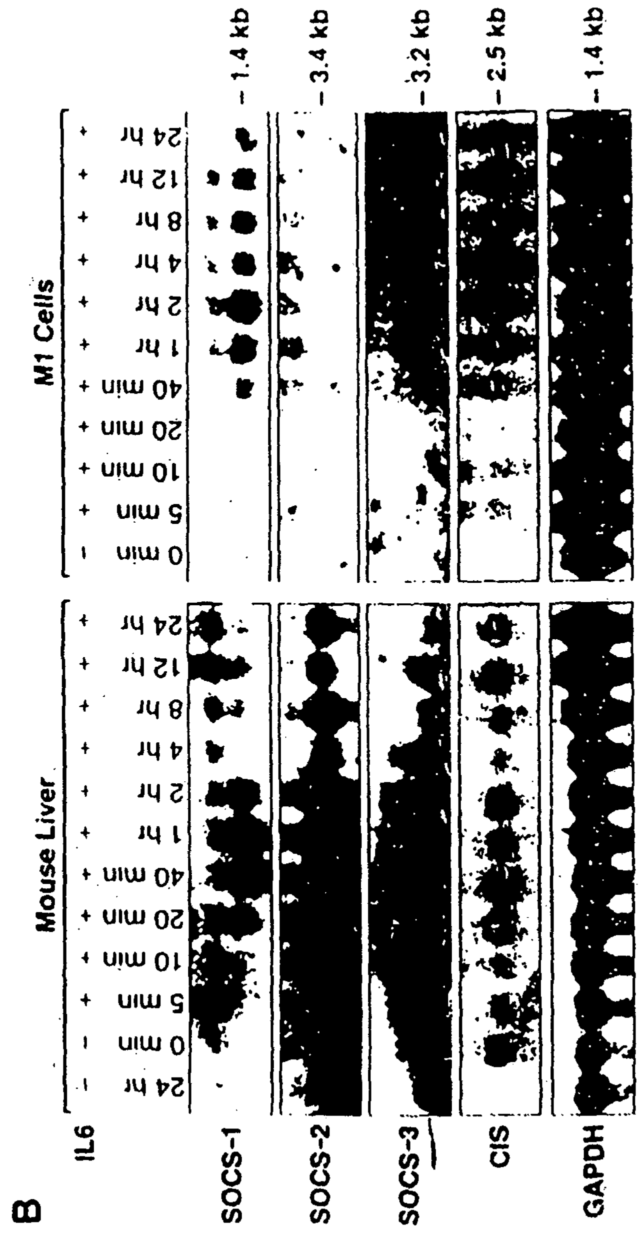


FIG 11B

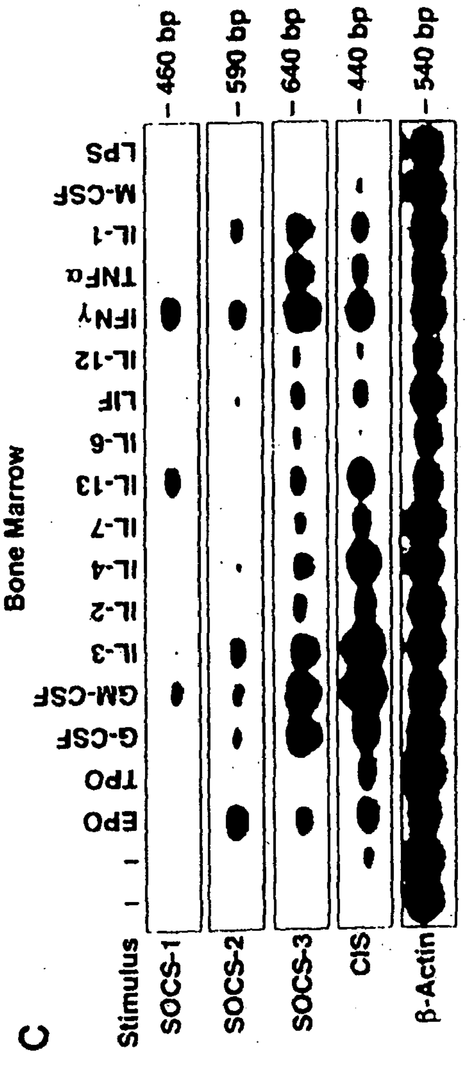


FIG 11C

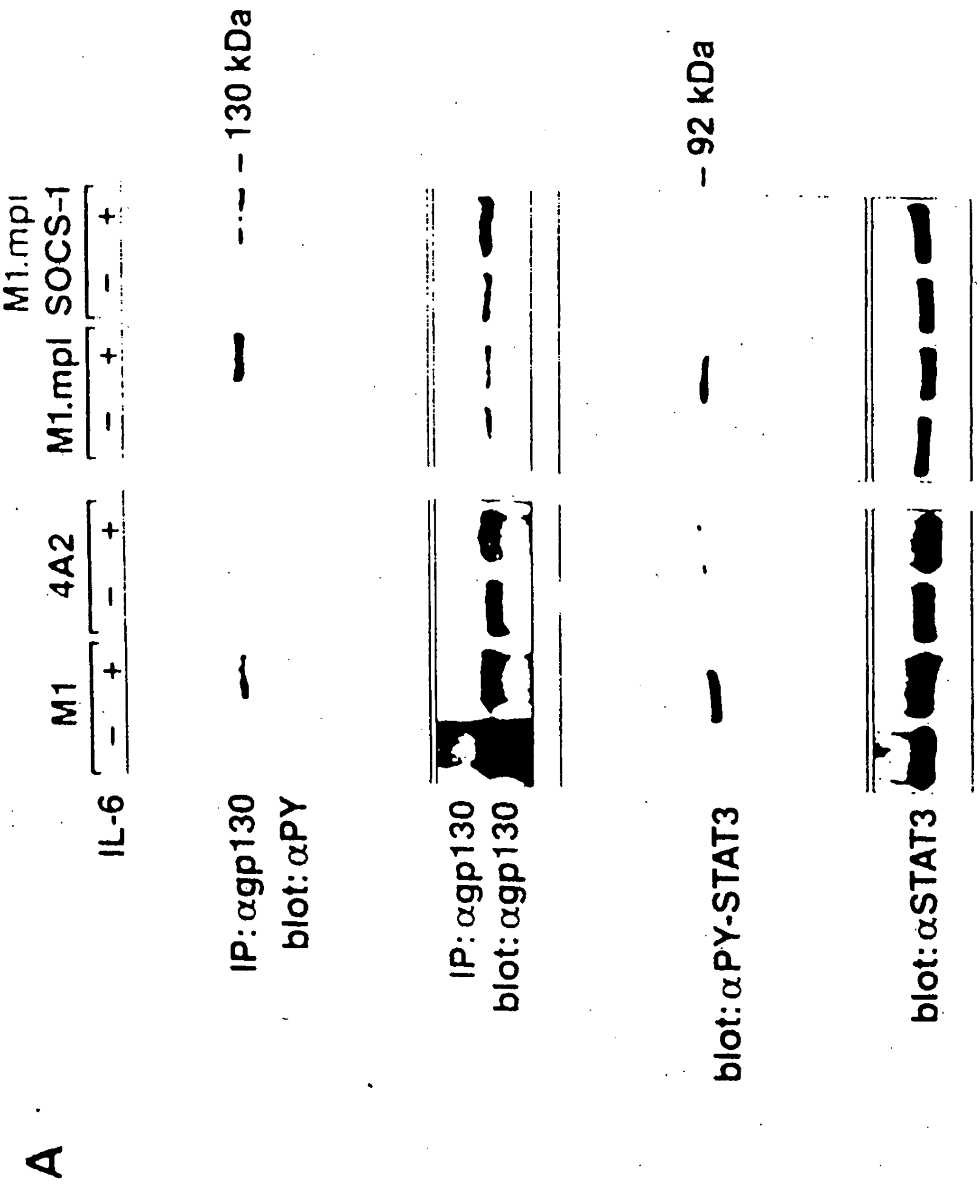
A
IL-6
M1
4A2
M1.mpl
M1.mpl SOCS-1
- + - + - + - +
IP: αgp130 blot: αPY
- 130 kDa
IP: αgp130 blot: αgp130
blot: αPY-STAT3
- 92 kDa
blot: αSTAT3

FIG 12A

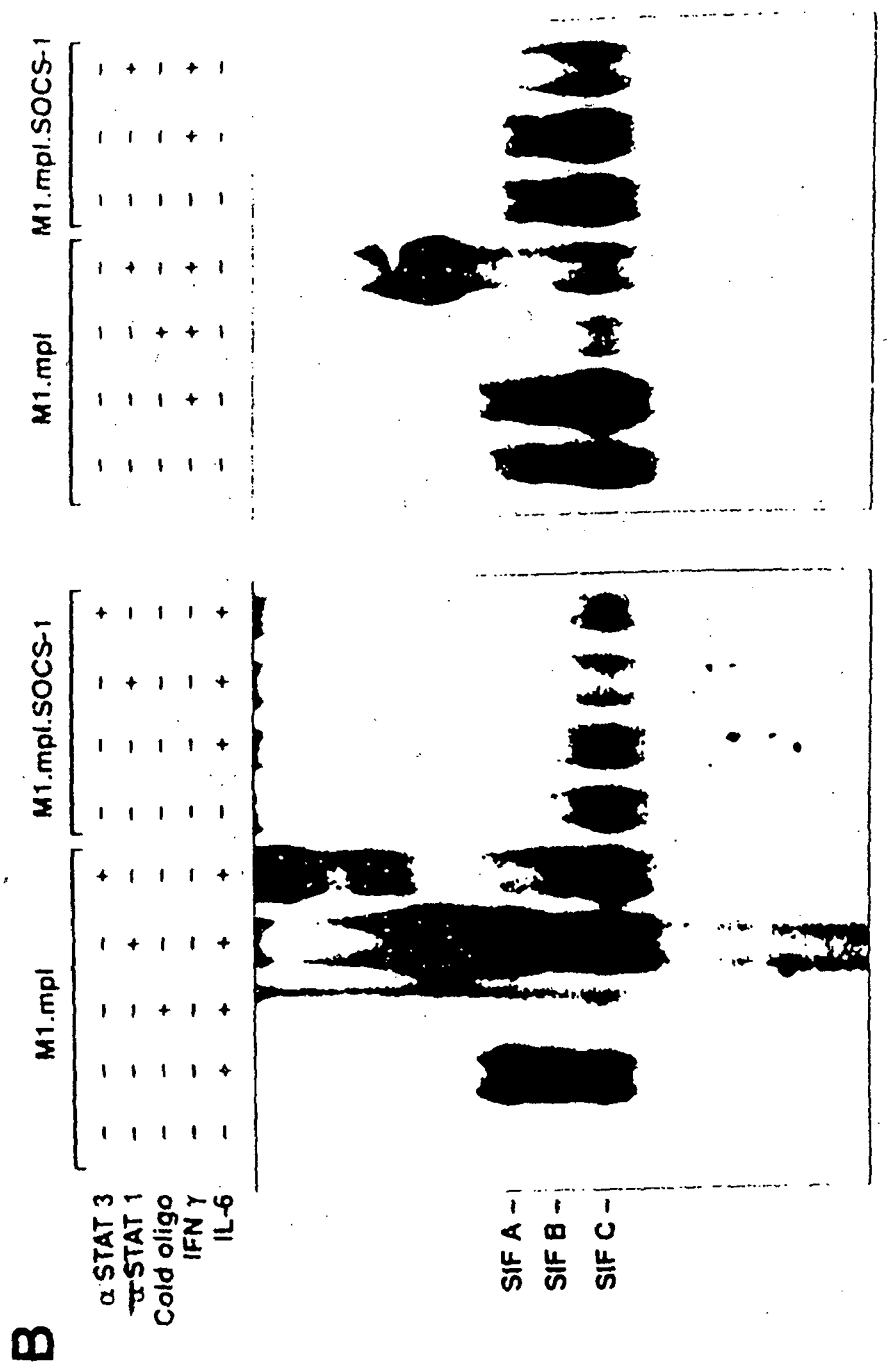
B
α STAT 3
α STAT 1
Cold oligo
IFN γ
IL-6
M1.mpl
M1.mpl.SOCS-1
M1.mpl
M1.mpl.SOCS-1
SIF A
SIF B
SIF C

FIG 12B

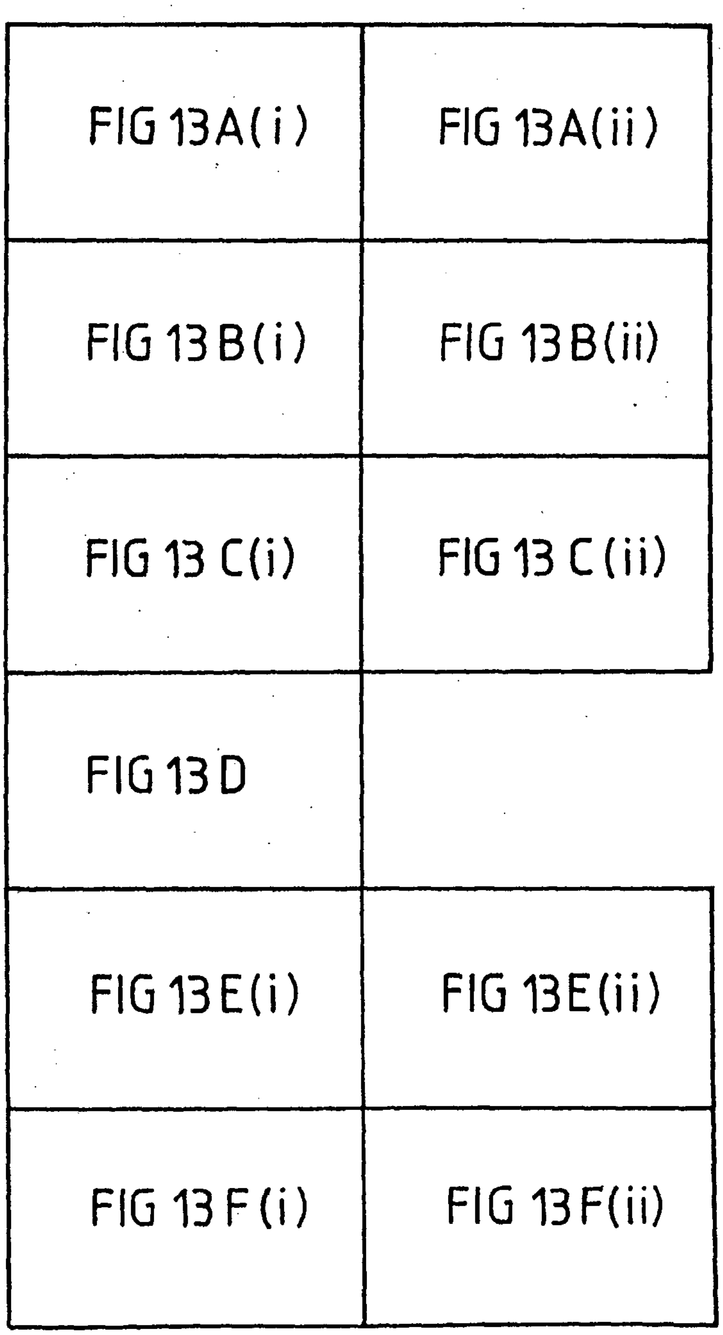
FIG 13A(i)
FIG 13A(ii)
FIG 13B(i)
FIG 13B(ii)
FIG 13C(i)
FIG 13C(ii)
FIG 13D
FIG 13E(i)
FIG 13E(ii)
FIG 13F(i)
FIG 13F(ii)

FIG 13

A

| | | |
|---|---|---|
| mmSOCS-1 | SH2 | MVARNQVAADNAISPAAEPRRRSEPSSSSSSSSPAAPVRP |
| mSOCS-3 | SH2 | MVTHSKFPAAGMSR.......................... |
| mSOCS-2 | SH2 | MTLRCLEPSGNGADRTRSQWGTAGLPEEQSPEA....... |
| mCIS | SH2 | MVLCVQGSCPLLAVEQIGRRPLWAQSLELPGPAMQPLPTG |
| mSOCS-5 | SH2 | MDKVGKMWNNLKYRCQNLFSHEGGSRNENVEMNPNRCPSV |
| mSOCS-14 | SH2 | SGGGPWRAGGGSGKSDSGLTVEPGRGLTARPPPGGSRTRS |
| mmSOCS-4 | WD | MASFPPRVNE |
| mmSOCS-6 | WD | ME |
| mmSOCS-15 | WD | MGQTAL |

| | | |
|---|---|---|
| mSOCS-5 | SH2 | AEIPQVVEISIEKDSDSGATPGTRLARRDSYSRHAPWGGK |
| mSOCS-14 | SH2 | NFLLEKLKNTVFITLEIVKNLFKMAENNSKNVDVRPKTSR |
| mSOCS-5 | SH2 | VSSRAVGSRSLRQRLQDTVGLCFPMRTYSKQSKPLFSNKR |
| mSOCS-14 | SH2 | SQERQLSCSSIELDLDHSCGHRFLGRSLKQKLQDAVGQCF |
| mSOCS-5 | SH2 | TFFDTFDPSLVSTEDEEDRLRERRRLSIEEGVDPPPNAQI |
| mSOCS-14 | SH2 | IKRHTVPMSPNSDEWVSADLSERKLRDAQLKRRNTEDDIP |
| mSOCS-5 | SH2 | SEEDSTTLCLQSRHQKQRQVSGDSHAHVSRQGAWKVHTQI |
| mSOCS-14 | SH2 | SEDEIIFLCTSSRKRNKPRWEMEEEILQLEAPP KFHTQI |

FIG. 13 A (i)

**A**

| | | |
|---|---|---|
| mmSOCS-1 | SH2 | R P C P A V P A P A |
| mSOCS-3 | SH2 | . . . . . . . . . . |
| mSOCS-2 | SH2 | . . . . . . . . . . |
| mCIS | SH2 | . . . . . . . . . . |
| mSOCS-5 | SH2 | K E K S I S L G E A A P Q Q E S S P L R E N V A L Q L G L S P S K T F S R R N Q N C A |
| mSOCS-14 | SH2 | G S G R A S L P R L S E R R V M A V V M A A G A R T A P L E L S S E R S V Q K V P R R |
| mmSOCS-4 | WD | |
| mmSOCS-6 | WD | |
| mmSOCS-15 | WD | |
| mSOCS-5 | SH2 | K K H S C S T K T Q S S L D T E K K F G R T R S G L Q R R E R R Y G V S S M Q D M D S |
| mSOCS-14 | SH2 | S R S A D R K D G Y V W S G K K L S W S K K S E S C S E S E A I G T V E N V E I P L R |
| mSOCS-5 | SH2 | K I H L S E L M L E K C P F P A G S D L A Q K W H L I K Q H T A P V S P H S |
| mSOCS-14 | SH2 | P I K N C S G R H S P G L P S K R K I H I S E L M L D K C P F P P R S D L A F R W H F |
| mSOCS-5 | SH2 | H T F E A T A Q V N P L Y K G P K L A P G M T E I S G D G S A I P Q X N C D |
| mSOCS-14 | SH2 | C F S H T N G Q P C V I T A N S A S C T G G H I T G S M M N L V T N N S I E D S D M D |
| mSOCS-5 | SH2 | D Y I H C L V P D L L Q I T G N P |
| mSOCS-14 | SH2 | D Y V H C L V P D L L Q I S N N P |

FIG. 13 A (ii)

B

| | | |
|---|---|---|
| | | Y F H T T D T |
| CONSENSUS | | G W Y o G o S o E b a L G S F L o R E S o S o |
| mSOCS-1 | SH2 | G F Y W G P L S V H G A H E R L R A E P V G T F L V R D S R Q R N C F A L S V K |
| mSOCS-3 | SH2 | G F Y W S A V T G G E A N L L L S A E P A G T F L I R D S S D Q R H F T L S V K |
| mSOCS-2 | SH2 | G W Y W G S M T V N E A K E K L K E A P E G T F L I R D S S H S D Y L T I S V K |
| mCIS | SH2 | G W Y W G S I T A S E A R Q H L Q K M P E G T F L V R D S T H P S Y F T L S V K |
| mSOCS-5 | SH2 | P C Y W G V M D R Y E A E A L L E G K P E G T F L L R D S A Q E D Y F S V S F R |
| mSOCS-14 | SH2 | P C Y W G V M D X Y A A E A L L E G K P E G T F L L R D S A Q E D Y F S V S F R |
| hSOCS-9 | SH2 | G W Y W G P I T R W E A E G K L A N V P D G S F L V R D S S D D R Y L S C D F R |
| hSOCS-11 | SH2 | G W Y W G P M N W E D A E M K L K G K P D G S F L V R D S S D P R Y L S L S F R |

FIG. 13 B (i)

**B**

```
                                R          T                                     E       Y H
CONSENSUS                 G   o K o    G  F S o                                  D o o   H Y
mSOCS-1     SH2  M A S G P T S I R V H F Q A G R F H L D G S R E T F             D C L F E L L E H Y
mSOCS-3     SH2  T Q S G T K N L R I Q C E G G S F S L Q S D P R S T Q P V P R F D C V L K L V H H Y
mSOCS-2     SH2  T S A G P T N L R I E Y Q D G K F R L D S I I C V K S K L K Q F D S V V H L I D Y Y
mCIS        SH2  T T R G P T N V R I E Y A D S S F R L D S N C L S R P R I L A F P D V V   S L V Q H Y
mSOCS-5     SH2  R Y N R S L H A R I E Q W N H N F S F D A H D P C V F H S S       T V T G   I L E H Y
mSOCS-14    SH2  R Y S R S L H A R I E Q W N H N F S F D A H D P C V F H S P       Q I T G   I L E H Y
hSOCS-9     SH2  S H G K T L H T R I E H S N G R F S F Y E Q X D V E G H T S I V   D L I G A F N Q G L
hSOCS-11    SH2  S Q G I T H H T R M E H Y R G T F S L W C H P K F E D R C Q S V V E F I K R A I M H S
```

FIG. 13 B (ii)

C

```
             V                                  D  D            AAA
             A       S                          G  G            GGG
             S    C  C                          N  N         CCCTTT      C
CONSENSUS    GHXXXϕXXϕxxxXxxϕxxxPxxP.xxxxϕϕϕSSSXDXXϕX
mmSOCS-4     KEIVRSRTIGELLAPAAPFDKKC GGENWTVAFAPDGSYF
mmSOCS-6     AGEEPLLLAELKPGRPHQFDWKS.SCETWSVAFSPDGSWF

mmSOCS-4     VPWSQCRKNFLLHGSKNVTNSSCLKLARQNSNGGQKNKPP
mmSOCS-6     VPWPLEEQFIPKGFEAKSRSSKNDPKGRGSLKEKTL

mmSOCS-4     GSSVPEKQSRCVNIEWHRFRFGQDQ      LLLATGLNNGRI
mmSOCS-6     SPWPSPPSRKLWARHPQA.P..D..VSCLILLATGLNDGQI

mmSOCS-4     AHIEMVRDL   T  F   A PD   GSLLLVSASRDKTLR
mmSOCS-6     GHQDVVRDL...S..F..TP.SG..  SLILVSASRDKTLR

mmSOCS-4      HQNWVYSC   A  F   SP  D   CS MLCSVGASKAVF
mmSOCS-6     GHLQWVYCC...S..I..SP..D..CS MLCSAAGEKSVF

mmSOCS-4     GHHHDVVAC...D..F..SP..D..GA.LLATASYDTRVY
mmSOCS-6     GHQSSVVSC...D..F..SP..D..SA.LLVTASYDTSVI

mmSOCS-4     GANDRWVRA  VS  F  SH  D  GL HVASLADDKMVR
mmSOCS-6     VHMSSLRSV...C..F..SP..E..GL.YLATVADDRLLR

mmSOCS-4     LSNGLCCAF...S..T...D..G..S..VLAAGTHDGSVY
mmSOCS-6     MTNGLCCTF...   ..F.PHG..G.....IIATGTRDGHVQ

mmSOCS-15    ARGSSSTPTSQALYSDFSPPEGLEELLSAPPPDLVAQRHH

mmSOCS-15    PKDCSENIDVKEGGLCFERRPVAQSTDGVRGKRGYSRGLH

mmSOCS-13                                          •
mmSOCS-15    LEQRGTH     AVVGVATALAPLQADHYAALLGSNSESW

mmSOCS-13      YHDGK   NQPSKTYPAFLEPDE   TFIVPDSFFVALD
mmSOCS-15      YHQSKGLEAPQYPAGPQGEQLVVPERLLVVLDMEEGTL
```

FIG. 13 C (i)

C

```
                 K
               Y N
             C F R
CONSENSUS    ϕ W D
mmSOCS-4     A W S Q G Y R I V K L
mmSOCS-6     A W S Q G H C V V K L

mmSOCS-4     E H V I D C G D I V W S L A F
mmSOCS-6             D C G Q I V W G V A F

mmSOCS-4     K I W D V Y T G K L L L L N L V D
mmSOCS-6     K I W E V Q T G L   L L L N L S

mmSOCS-4     V W D L K D D G N M V K V L R
mmSOCS-6     I W D L N K H G K Q I Q V L S

mmSOCS-4     L W N M D K Y T M I R K L E
mmSOCS-6     L W S M R S Y T L I R K L E

mmSOCS-4     V W D P H N G D L L M E F G H L F P S P T P I F A G
mmSOCS-6     M W D P Y T G A R L R S L H H T Q L E P T M D D S D

mmSOCS-4     F Y R I D E D C P V Q V A P
mmSOCS-6     I W A - L E L K A P V A F A P

mmSOCS-4     F W A
mmSOCS-6     F W T

mmSOCS-15    G W N

mmSOCS-15    A W E

mmSOCS-13    G W D I S W P L G R N R L
mmSOCS-15    G W D         I G R G K L

mmSOCS-13    M X D
mmSOCS-15    G Y S
```

FIG. 13 C (ii)

D

```
                              T                                po
CONSENSUS                     S  L H φ A φ               φ φ φ    G  po
mSOCS-7      ANK   • P C T P L R I A A T A G H G N C V D F L I R K G A E V D L V D V
mSOCS-7      ANK   K G Q T A L Y V A V V N G H L E S T E I L L E A G A D P N G S R H
mSOCS-7      ANK   H R S T P V Y H A X R V G R D D I L K A L I R Y G A D V D V N H H L N S D T R P P F S R R L T S
mSOCS-7      ANK   L V V C P L Y I S A A Y H N L Q C F R L L L Q A G A N P D F N C N G P V N T Q E F Y R G S
mSOCS-7      ANK   P G C V M D A V L R H G C E A A F V S L L V E F G A N L N

mSOCS-10     ANK   • E E T A L Y L A T C R E H L D C L L S L L Q A G A E P D I S N K
mSOCS-10     ANK   S R E T P L Y K A C E R K N A E A V R I L V R Y N A D A N H R C N
mSOCS-10     ANK   R G W T A L H E S V S R N D L E V M E I L V S G G A K V E A K N V
mSOCS-10     ANK   Y S I T P L F V A A Q S G Q L E A L R F L A K H G A D I N T Q A S
mSOCS-10     ANK   D S A S A L Y E A S K N E H E D V V E F L L S Q G A D A N K A N K
mSOCS-10     ANK   D G L L P L H V A S K K G N Y R I V Q M L L P V T S R T R V R R
mSOCS-10     ANK   S G I S P L H L A A E R N H D A V L E A L L A A R F D V N A P L A P E R A R L Y
mSOCS-10     ANK   E D R R S S A L Y F A V N N V Y A T E L L L L A G A D P N R
mSOCS-10     ANK   D V I S P L L V A I R H G C L R T M Q L L L D H G A N I D A •
```

FIG. 13 D

E

| | | |
|---|---|---|
| mSOCS-1 | SH2 | |
| mSOCS-3 | SH2 | MPPPGTPSFSLPPTEPSSEVPEQPPAQALPGSTPKRAYYI |
| mSOCS-2 | SH2 | VQMCKDKRTGPE |
| mCIS | SH2 | VASCAADTRSDSPDPAPTPALPMSKQDAPSDSVLPIP |
| mSOCS-5 | SH2 | KDP |
| mSOCS-14 | SH2 | KDP |
| hSOCS-9 | SH2 | .... CYSRSQLP |
| hSOCS-11 | SH2 | KNGKFLYFLRSRVP |
| mSOCS-4 | WD | |
| mSOCS-6 | WD | |
| mSOCS-13 | WD | GTLSFIVDGQYMGVAFRGLKGKKLYPVVSAV |
| mSOCS-15 | WD | IGGTYLGPAFRGLKGRTLYPSVSAV |
| mSOCS-7 | ANK | LVKWESLGPEARGRRKMDPEALQVFKEARSI |

FIG. 13 E (i)

E

| | | |
|---|---|---|
| | | Q R R |
| mSOCS-1 | SH2 | S S N |
| mSOCS-3 | SH2 | V A A P R R M L G A P L R Y T S |
| mSOCS-2 | SH2 | Y S G G E K I P L V L S R P L R S S |
| mCIS | SH2 | A P R N G T V H L Y L T K P L R T F |
| mSOCS-5 | SH2 | V A T A V H L K L V Q P F V R R T F |
| mSOCS-14 | SH2 | S S C M F F E P L L T I S L N F X X |
| hSOCS-9 | SH2 | S A C M F F E P L L S T P L I F S N |
| hSOCS-11 | SH2 | G S A T Y L V R L A K P V S R P R Q |
| mSOCS-4 | WD | G L P P T P V Q L L Y P V S R P R V |
| mSOCS-6 | WD | T D P E |
| mSOCS-13 | WD | T A V E E |
| mSOCS-15 | WD | W G H C E I R M R Y L N G L |
| mSOCS-7 | ANK | W G Q C Q V R I R Y M G E R R |

FIG. 13 E (ii)

F

```
                    P
                P   T     H                 T                                           F

CONSENSUS       φ   S L   Y φ C R     φ       φ                     φ     φ P φ P     φ     Y φ
mSOCS-1    SH2  V R P L Q E L C R Q R I V A A V G R E N - - - - - - L A R I P L N P V L R D Y L
mSOCS-3    SH2  V A T L Q H L C R K T V N G H L D S Y E K - - - - - V T Q L P G P     I R E F L
mSOCS-2    SH2  A P T L Q H F C R L A I N K C T G T - - - - - - - - I W G L P L P T R L K D Y L
mCIS       SH2  A R S L Q H L C R L V I N R L V A D - - - - - - - - V D C L P L P R R M A D Y L
mSOCS-5    SH2  P F S L Q Y I C R A V I C R C T T Y D G - - - - - - I D G L P L P S M L Q D F L
mSOCS-14   SH2  P F S L Q H I C R T V I C N C T T Y D G - - - - - - I D A L P I P S P M K L Y L
hSOCS-9    SH2  V R S L Q Y L C R F V I C Q Y T R I D L - - - - - - I Q K L P L P N K M K D Y L
hSOCS-11   SH2  V K S L Q H L C R F R I R Q Y T R I D H - - - - - - I P D L P L P K P L I S Y I
mSOCS-4    WD   V P S L Q H I C R M S I R R V M S T Q E - - - - - - V Q K L P V P S K I L A F L
mSOCS-6    WD   L S S L K H L C R K A L R S F L T T Y Q - - - - - - V L A L P I P K K M K E F L
mSOCS-13   WD   P L P L M D L C R R S V R L A L G K E R L G A - - - I P A L P L P A S L K A Y L
mSOCS-15   WD   P Q S L L H L S R L C V R H A L G D T R L G Q - - - I S T L P L P P A M K R Y L
mSOCS-7    ANK  P R T L L S L C R V A V R R A L G K Y R L H L - - - V P S L P L P D P I K K F L
mSOCS-10   ANK  P R P L A H L C R L R V R K A I G K Y R I K L - - - L D T L P L P G R L I R Y L
mSOCS-12   ANK  V P S L T H L C R L E I R A S L K A E H L H S D I F I H Q L P L P R S L Q N Y L
mSOCS-8    ?    P R S L Q H L C R C A L R S H L E G C L P H A - - - L P R L P L P P R M L R F L
```

FIG. 13 F (i)

F

| CONSENSUS | | |
|---|---|---|
| mSOCS-1 | SH2 | SSFPFQI* |
| mSOCS-3 | SH2 | DQYDAP* |
| mSOCS-2 | SH2 | EEYKFQV* |
| mCIS | SH2 | RQYPFQL* |
| mSOCS-5 | SH2 | KEYHYKQKVRVRWLE |
| mSOCS-14 | SH2 | KEYHYKSKVRLLRIDLKEAQRQFPNRSKRWNPPRSEGLPAGHHQGHLV* |
| hSOCS-9 | SH2 | QEKHY* |
| hSOCS-11 | SH2 | RKFYYYDPQEEVYLS |
| mSOCS-4 | WD | SYRG* |
| mSOCS-6 | WD | TYRTF* |
| mSOCS-13 | WD | LYQ* |
| mSOCS-15 | WD | LY* |
| mSOCS-7 | ANK | YE* |
| mSOCS-10 | ANK | KYENTQ* |
| mSOCS-12 | ANK | LYEEVLRMNEILEPA |
| mSOCS-8 | ? | QLDFEDLLY* |

FIG. 13 F (ii)

FIG 14A

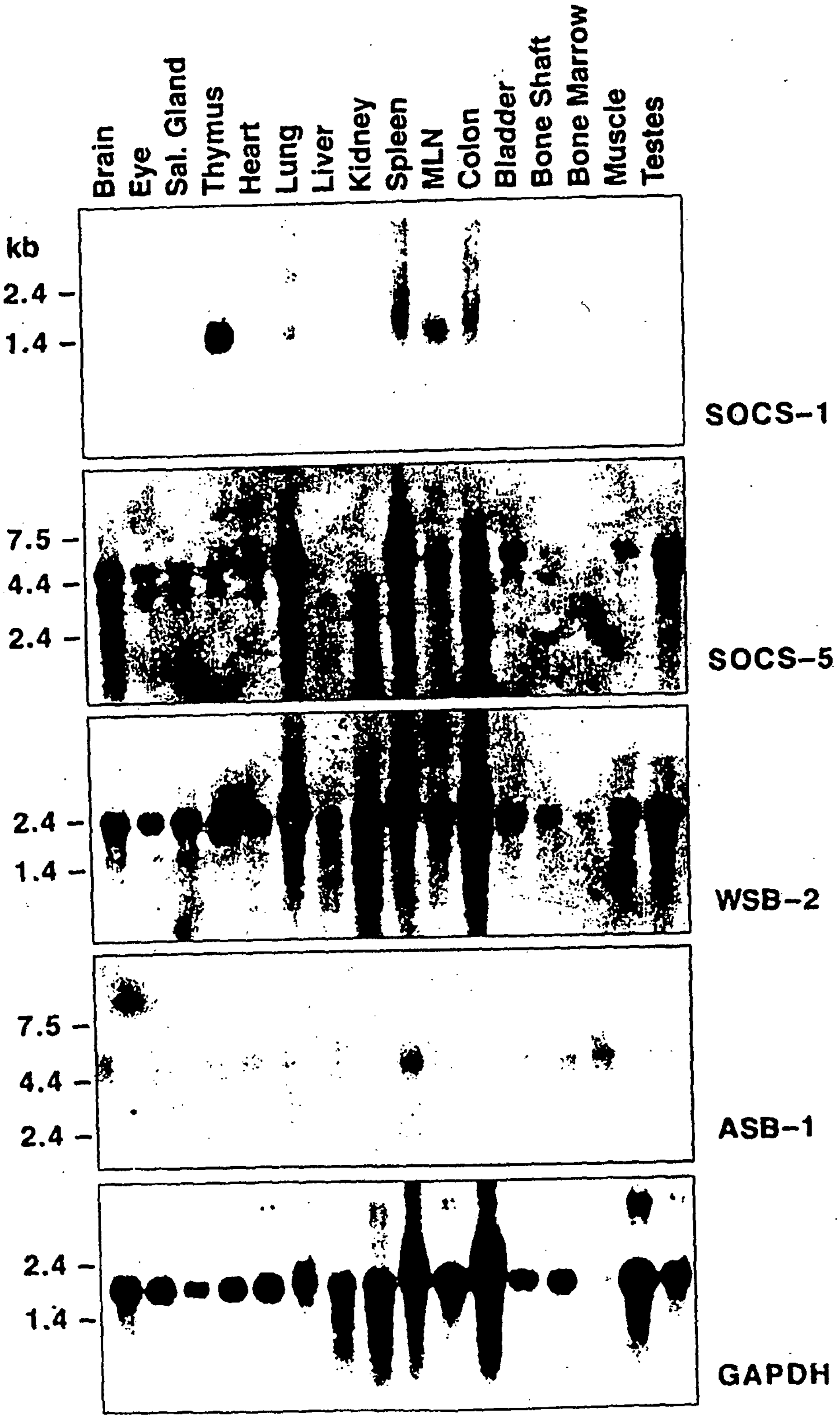
Brain
Eye
Sal. Gland
Thymus
Heart
Lung
Liver
Kidney
Spleen
MLN
Colon
Bladder
Bone Shaft
Bone Marrow
Muscle
Testes
kb
2.4
1.4
SOCS-1
7.5
4.4
2.4
SOCS-5
2.4
1.4
WSB-2
7.5
4.4
2.4
ASB-1
2.4
1.4
GAPDH

FIG 14B

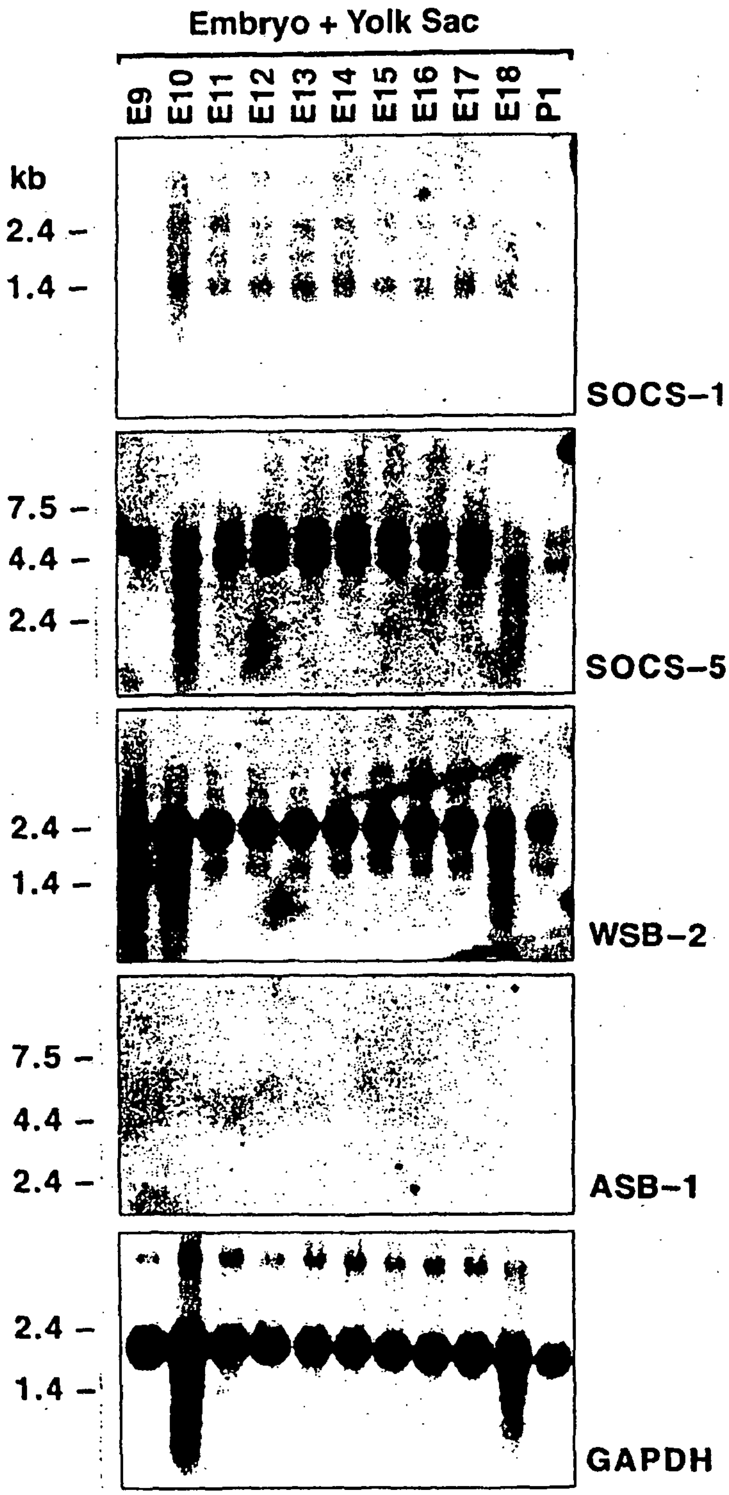
Embryo + Yolk Sac
E9
E10
E11
E12
E13
E14
E15
E16
E17
E18
P1
kb
2.4 -
1.4 -
SOCS-1
7.5 -
4.4 -
2.4 -
SOCS-5
2.4 -
1.4 -
WSB-2
7.5 -
4.4 -
2.4 -
ASB-1
2.4 -
1.4 -
GAPDH

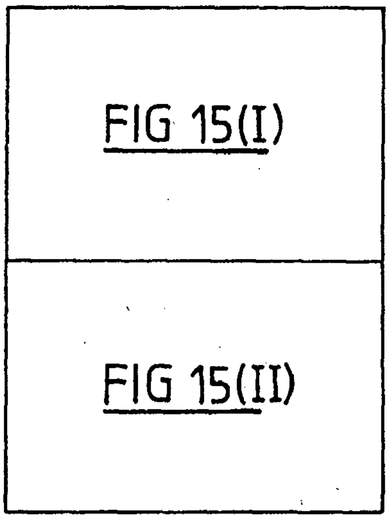


FIG 15

cgaattccgggcgggctgtgtgagtctgtgagtggaaggcgcgccggctcttttgtct
gagtgtgacccggtggctttgttccaggcattccggtgatttcctccgggcagtccgc
agaagccgcagcggccgcccgcgctctctctgcagtctccacacccgggagagcctga
gcccgcgtcacgcccctcagcccccgctgagtcccttctctgttgtcgcgtccgaatc
gagttcccggaatcagacggtgccccatagATGGCCAGCTTTCCCCCGAGGGTTAACG
AGAAAGAGATCGTGAGATCACGTACTATAGGGGAACTCTTGGCTCCAGCAGCTCCTTT
TGACAAGAAATGTGGTGGTGAGAACTGGACGGTTGCTTTTGCTcCTGATGGTTCCTAC
TTTGCGTGGTCACAAGGATATCGCATAGTGAAGCTTGTCCCGTGGTCCCAGTGCCGTA
AGAACTTTCTTTTGCATGGTTCCAAAAATGTTACCAATTCAAGCTGTCTAAAATTGGC
AAGACAAAACAGTAATGGTGGTCAGAAAAACAAGCCTCCTGAGCACGTTATAGACTGT
GGAGACATAGTCTGGAGTCTTGCTTTTGGGTCTTCAGTTCCAGAAAAACAGAGTCGTT
GCGTTAATATAGAATGGCATCGGTTCCGATTTGGACAGGATCAGCTACTCCTTGCCAC
AGGATTAAACAATGGTCGCATCAAAATCTGGGATGTATATACAGGAAAACTCCTCCTT
AATTTGGTAGACCACATTGAAATGGTTAGAGATTTAACTTTTGCTCCAGATGGGAGCT
TACTCCTTGTATCAGCTTCAAGAGACAAAACTCTAAGAGTGTGGGACCTGAAAGATGA
TGGAAACATGGTGAAAGTATTGCGGGCACATCAGAATTGGGtGtACAGTTGTGCATTc
TCTCCcGACTGTTcTATGCTGTGTTCAGTgGGCGCCAGTAAAGCAGTTTTcCTTTGGA
ATATGGATAAAtACACCATGATTAGGAAGctGGAAGGTCATCACCATGATGTTGTAGC

FIG 15(I)

TTGTGACTTTTCTCCTGATGGAGCATTGCTAgCTACTGCATCCTATGACACTCGTGTG
TATGTCTGGGATCCACACAATGGAGACCTTCTGATGGAGTTTGGGCACCTGTTTCCCT
CGCCCACTCCAATATTTGCTGGAGGAGCAAATGACCGATGGGTGAGAGCTGTGTCTTT
CAGTCATGATGGACTGCATGTTGCCAGCCTTGCTGATGATAAAATGGTGAgGTTCTGG
AGAATCGATGAGGATTGTCCGGTACAAGTTGCACCTTTGAGCAATGGTCTTTGCTGTG
CCTTTTCTACTGATGGCAGTGTTTTAGCTGCTGGGACACATGATGGAAGTGTGTATTT
TTGGGCCACTCCAAGGCAAGTCCCTAgCCTTCAACATATATGTCGCATGTCAATCCGA
AGAGTGATGTCCACCCAAGAAGTCCAAAAACTGCCTGTTCCTTCCAAAATATTGGCGT
TTCTCTCCTACCGCGGTTAGactgaagactgcctttcctggtaggcctgccagacaga
gcgccctttacaagacacacctcaagctttacctcgtgccgaatt

FIG 15(II)

MASFPPRVNEKEIVRSRTIGELLAPAAPFDKKCGGENWTVAFAPDGSYFAWSQGYRIV
KLVPWSQCRKNFLLHGSKNVTNSSCLKLARQNSNGGQKNKPPEHVIDCGDIVWSLAFG
SSVPEKQSRCVNIEWHRFRFGQDQLLLATGLNNGRIKIWDVYTGKLLLNLVDHIEMVR
DLTFAPDGSLLLVSASRDKTLRVWDLKDDGNMVKVLRAHQNWVYSCAFSPDCSMLCSV
GASKAVFLWNMDKYTMIRKLEGHHHDVVACDFSPDGALLATASYDTRVYVWDPHNGDL
LMEFGHLFPSPTPIFAGGANDRWVRAVSFSHDGLHVASLADDKMVRFWRIDEDCPVQV
APLSNGLCCAFSTDGSVLAAGTHDGSVYFWATPRQVPSLQHICRMSIRRVMSTQEVQK
LPVPSKILAFLSYRG*

FIG 16

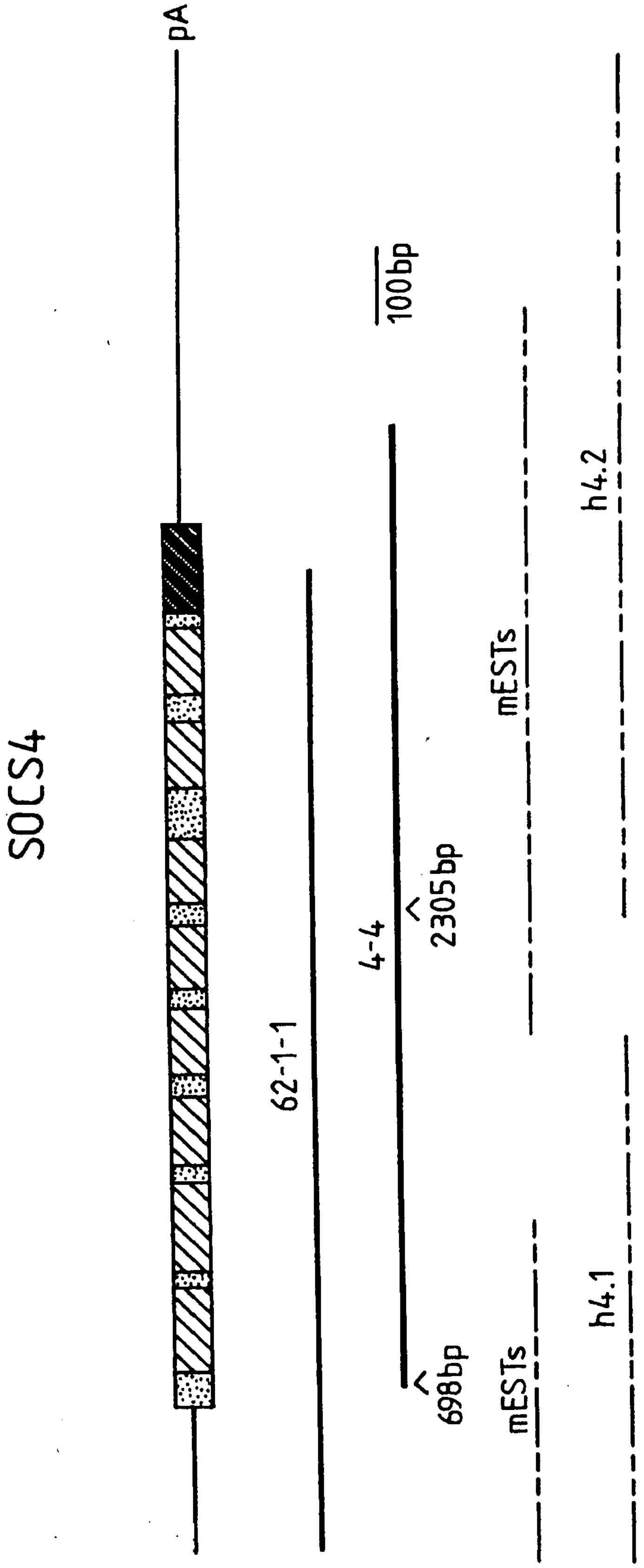


FIG 17

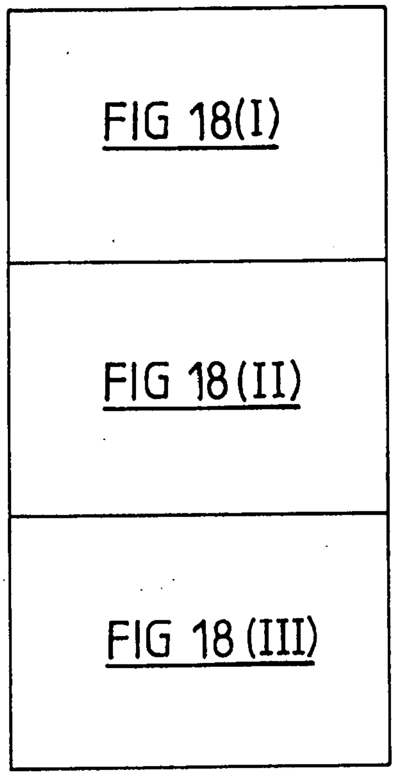


FIG 18

h4.1

CTGTCTTCCTCCGCAGCGCGAGGCTGGGTACAGGGTCTATTGTCTGTGGTTGACTCCG
TACTTTGGTCTGAGGCCTTCGGGAGCTTTCCCGAGGCAGTTAGCAGAAGCCGCAGCGA
CCGCCCCCGCCCGTCTCCTCTGTCCCTGGGCCCGGGAGACAAACTTGGCGTCACGCCC
TCAGCGGTCGCCACTCTCTTCTCTGTTGTTGGGTCCGCATCGTATTCCCGGAATCAGA
CGGTGCCCCATAGATGGCCAGCTTTCCCCCGAGGGTCAACGAGAAAGAGATCGTGAGA
TCACGTACTATAGGTGAACTTTTAGCTCCTGCAGCTCCTTTTGACAAGAAATGTGGTC
GTGAAAATTGGACTGTTGCTTTTGCTCCAGATGGTTCATACTTTGCTTGGTCACAAGG
ACATCGCACAGTAAAGCTTGTTCCGTGGTCCCAGTGCCTTCAGAACTTTCTCTTGCAT
GGCACCAAGAATGTTACCAATTCAAGCAGTTTAAGATTGCCAAGACAAAATAGTGATG
GTGGTCAGAAAAATAAGCCTCGTGACATATTATAGACTGTGGAGATATAGTCTGGAGT
CTTGCTTTTGGGTCATCAGTTCCAGAAAAACAGAGTCGCTGTGTAAATATAGAATGGC
ATCGCTTCAGATTTGGACAAGATCAGCTACTTCTTGCTACAGGGTTGAACAATGGGCG
TATCAAAATATGGGATGTATATMCAGGAAACTCCTCCTTAACTTGGTAGATCATACTG
AAGTGGTCAGAGATTTAACTTTTGCTCCAG

FIG 18(I)

h4.2

CTCTGTATGTCTGAATGAAGCTATAACATTTGCCTTTTTATTGCAGGTTTTCCTTTGG
AATATGGATAAATACACCATGATACGGAAACTAGAAGGACATCACCATGATGTGGTAG
CTTGTGACTTTTCTCCTGATGGAGCATTACTGGCTACTGCATCTTATGATACTCGAGT
ATATATCTGGGATCCACATAATGGAGACATTCTGATGGAATTTGGGCACCTGTTTCCC
CCACCTACTCCAATATTTGCTGGAGGAGCAAATGACCGGTGGGTACGATCTGTATCTT
TTAGCCATGATGGACTGCATGTTGCAAGCCTTGCTGATGATAAAATGGTGAGGTTCTG
GAGAATTGATGAGGATTATCCAGTGCAAGTTGCACCTTTGAGCAATGGTCTTTGCTGT
GCCTTCTCTACTGATGGCAGTGTTTTAGCTGCTGGGACACATGACGGAAGTGTGTATT
TTTGGGCCACTCCACGGCAGGTCCCTAGCCTGCAACATTTATGTCGCATGTCAATCCG
AAGAGTGATGCCCACCCAAGAAGTTCAGGAGCTGCCGATTCCTTCCAAGCTTTTGGAG
TTTCTCTCGTATCGTATTTAGAAGATTCTGCCTTCCCTAGTAGTAGGGACTGACAGAA
TACACTTAACACAAACCTCAAGCTTTACTGACTTCAATTATCTGTTTTTAAAGACGTA
GAAGATTTATTTAATTTGATATGTTCTTGTACTGCATTTTGATCAGTTGAGCTTTTAA
AATATTATTTATAGACAATAGAAGTATTTCTGAACATATCAAATATAAATTTTTTTAA
AGATCTAACTGTGAAAACATACATACCTGTACATATTTAGATATAAGCTGCTATATGT
TGAATGGACCCTTTTGCTTTTCTGATTTTTAGTTCTGACATGTATATATTGCTTCAGT
AGAGCCACAATATGTATCTTTGCTGTAAAGTGCAAGGAAATTTTAAATTCTGGACAC

FIG 18(II)

TGAGTTAGATGGTAAATACTGACTTACGAAAGTTGAATTGGGTGAGGCGGGCAAATCA
CCTGAGGTCAGCAGTTTGAGACTAGCCTGGCAAACATGATGAAACCCTGTCTCTACTA
AAAATACAAAAAAAAAAAAA

FIG 18(III)

SOCS5

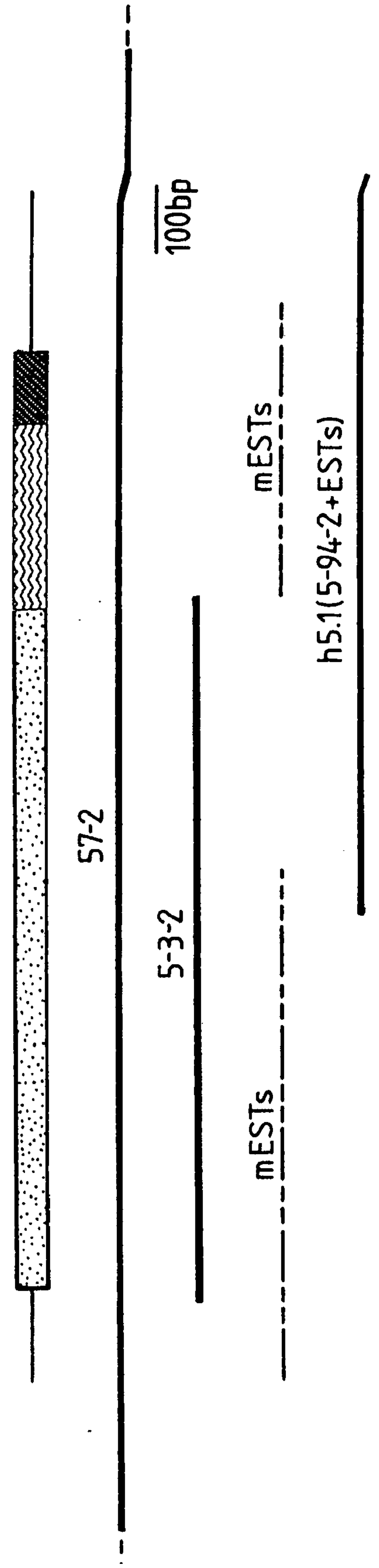


FIG 19

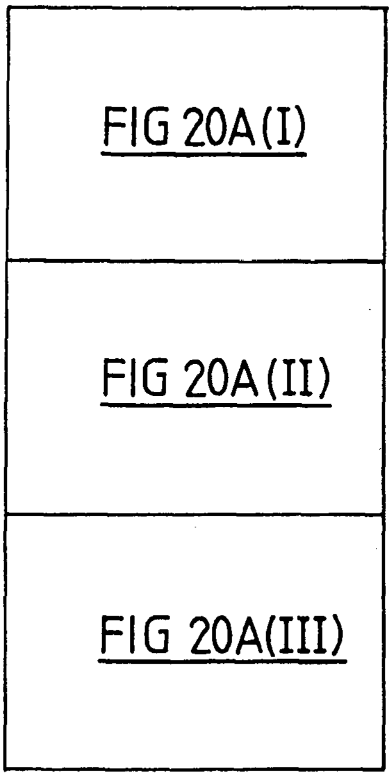


FIG 20A

cggcacgagccgggctccgtccggaggaagcgaggctgcgccgccggcccggcaggagc
ggaggacgggamgcgcgggcggtcgcgctcgccctgtcgctgactgcgctgccccggcc
catccttgcctggccgcaggtgccctggatgaggccgccgcgtgtcccggccgctga
gtgtcccccgcggtcgcccggcgcctgccctcaagcggccgcctctccttgcccgggtc
cccgtttcccccggcgcagtcctcctccggtgggcgcctccgcacctcggcgcaggcg
gcacggccctcgggccgggatggatccgccgggaagaggaagacaagccggggcgttga
gcccctgcgcacggtgccgccgcgcgtagtgggagcttactcgcagtaggctctcgctc
ttctaatcaATGGATAAAGTGGGAAAATGTGGAACAACTTAAAATACAGATGCCAGAA
TCTCTTCAGCCACGAGGGAGGAAGCCGTAATGAGAACGTGGAGATGAACCCCAACAGAT
GTCCGTCTGTCAAAGAGAAAAGCATCAGTCTGGGAGAGGCAGCTCCCCAGCAAGAGAG
CAGTCCCTTAAGAGAAAATGTTGCCTTACAGCTGGGACTGAGCCCTTCCAAGACCTTT
TCCAGGCGGAACCAAAACTGTGCCGCAGAGATCCCTCAAGTGGTTGAAATCAGCATCG
AGAAAGACAGTGACTCgGGTGCCACCCCAGGAACGAGGCTTGCACGGAGAGACTCCTA
CTCGCGGCACGcCCCGTGGGGAGGAAAGAAGAAACATTCCTGTTCCACAAAGACCCAG
AGTTCATTGGATACCGAGAAAAAGTTTGGTAGAACTCGAAGCGGCCTTCAGAGGCGAG
AGCGGCGCTATGGAGTCAGCTCCATGCAGGACATGGACAGCGTTTCTAGCCGCGCGGT
CGGGAGCCGCTCCCTGAGGCAGAGGCTCCAGGACACGGTGGGTTTGTGTTTTCCCATG
AGAACTTACAGCAAGCAGTCAAAGCCACTCTTTTCCAATAAAAGAAAAATACATCTTT

FIG 20A(I)

CTGAATTAATGCTGGAGAAATGCCCTTTTCCTGCTGGCTCGGATTTAGCACAAAAGTG
GCATTTGATTAAACAGCATACCGCCCCTGTGAGCCCACACTCAACATTTTTTGATACA
TTTGATCCATCACTGGTGTCTACAGAAGATGAAGAAGATAGGCTTCGCGAGAGAAGAC
GGCTTAGTATCGAAGAAGGGGTGGATcCcCcTCCCAACGCACAAATACACACCTTTGA
AGCTACTGCACAGGTCAACCCATTGTATAAGCTGGGACCAAAGTTAGCTCCTGGGATG
ACAGAGATAAGTGGAGATGGTTCTGCAATTCCACAAGCsAATTGTGACTCAGAAGAGG
ATTCAACCACCCTATGTCTGCAGTCACGGAGGCAGAAGCAGCGCCAGGTGTCCGGGGA
CAGCCACGCGCACGTTAGCAGACAGGGAGCTTGGAAAGTTCATACGCAGATCGATTAC
ATACACTGCCTCGTGCCAGATTTGCTTCAGATCACAGGGAATCCCTGTTACTGGGGCG
TGATGGACCGATACGAGGCCGAAGCCCTTCTAGAAGGGAAACCGGAAGGCACGTTCTT
GCTCAGGGACTCTGCACAGGAGGACTACCTCTTCTCTGTGAGcTTCCGCCGCTACAAC
AGGTCTCTGCACGCCCGGATCGAGCAGTGGAACCACAACTTCAGCTTCGATGCCCATG
ACCCCTGCGTGTTTCACTCCTCCACwGTCACGGGGCTTCTCGAACACTATAAAGACCC
CAGCTCTTGCATGTTTTTTGAACCGTTGCTAACGATATCACTGAATAGAACTTTCCCT
TTCAGCCTGCAGTATATCTGCCGCGCAGTGATCTGCAGATGCACTACGTATGATGGGA
TTGACGGGcTCCCGCTACCGTCGATGTTACAGGATTTTTTAAAAGAGTATCATTATAA
ACAAAAAGTTAGGGTTCGCTGGTTAGAACGAGArCCAGTCAAAGCAAAGTAActcctg
tccccaaagggcactaactaagtctgctcctcccgtgcatcmgaactgcacccatagg

FIG 20A(II)

raggcagtcagctgctaggatttcccacccagaatgggagcttagtcattagcctctg
ccctatggggtccgctgttcctcagacaaaggtgcctagggacagcaagatggcttgc
aggtgttcggtgggctgtgacaactgagggaggcaactctggggcatttgctatgaag
aattctatttcttaccgaagaacaaattattaatattggatgggtatttcaatagtgt
gactaatgtttgaaattatttttctaagaattttctataaccttcagaaaaagtag
tgatgtttgtagttactataaatcaagctttgaaagttcaaaacaaacaagttaaata
aaagactaccttcctttagagaaaacaaatgcaagttttcccagccacaggcattgt
gcactgttaatgttngcttgttatcagctcctttctcctcc

FIG 20A(III)

MDKVGKMWNNLKYRCQNLFSHEGGSRNENVEMNPNRCPSVKEKSISLGEAAPQQESSP
LRENVALQLGLSPSKTFSRRNQNCAAEIPQVVEISIEKDSDSGATPGTRLARRDSYSR
HAPWGGKKKHSCSTKTQSSLDTEKKFGRTRSGLQRRERRYGVSSMQDMDSVSSRAVGS
RSLRQRLQDTVGLCFPMRTYSKQSKPLFSNKRKIHLSELMLEKCPFPAGSDLAQKWHL
IKQHTAPVSPHSTFFDTFDPSLVSTEDEEDRLRERRRLSIEEGVDPPPNAQIHTFEAT
AQVNPLYKLGPKLAPGMTEISGDGSAIPQXNCDSEEDSTTLCLQSRRQKQRQVSGDSH
AHVSRQGAWKVHTQIDYIHCLVPDLLQITGNPCYWGVMDRYEAEALLEGKPEGTFLLR
DSAQEDYLFSVSFRRYNRSLHARIEQWNHNFSFDAHDPCVFHSSXVTGLLEHYKDPSS
CMFFEPLLTISLNRTFPFSLQYICRAVICRCTTYDGIDGLPLPSMLQDFLKEYHYKQK
VRVRWLERXPVKAK*

FIG 20B

GATTAAACAGCATACAGCTCCTGTGAGCCCACATTCAACATTTTTTGATACrTTTGATCCATCTTTGGTTT
CTACAGAAGATGAAGAAGATAGGCTTAGAGAGAGAAGGCGGCTTAGTATTGAAGAAGGGGTTGATCCC
CCTCCCAATGCACAAATACATACATTTGAAGCTACTGCACAGGTTAATCCATTATwTAAACTGGGACCA
AAATTAGCTCCTGGAATGACTGAAATAAGTGGGGACAGTTCTGCAATTCCACAAGCTAATTGTGACTCG
GAAGAGGATACAACCACCCTGTGyTTGCAGTCACGGAGGCAGAAGCAGCGTCAGATATCTGGAGACAGC
CATACCCATGTTAGCAGACAGGGAGCTTGGAAAGTCCACACACAGATTGATTACATACACTGCTTCGTG
CCTGATTTGCTTCAAATTACAGGGAATCCCTGTTACTGGGGAGTGATGGACCGTTATGAAGCAGAAGCC
CTTCTCGAAGGGAAACCTGAAGGCACGTTTTTGCTCAGGGACTCTGCGCAAGAGGACTACTTCTTCTCT
GTGAGCTTCCGCCGATACAACAGATCCCTGCATGCCCGAATTGAGCAGTGGAATCACAACTTTAGTTTC
GACGCCCATGACCCGTGTGTATTTCACTCCTCCACTGTAACGGGACTTTTAGAACATTATAAAGATCCCA
GTTCGTGCATGTTTTTTGAACCATTGCTTACTATATCACTAAATAGGACTTTCCCTTTTAGCCTGCAGTAT
ATCTgTcGCGCGGTAATCTGCAGGTGCACTACGTATGATGGAATTGATGGGCTCCCTCTACCCTCAATGT
TACAGGATTTTTTAAAAGAGTATCATTATAAACAAAAAGTTAGAGTTCGCTGGTTGGaACGAGAACCAG
TCAAGGCAAAGTAAACTCTCCGGTCCCCAAAGGgTGTTAACTAGGTCCGCTTTCATGTGCATCAGACAGT
ACACCTATAGCAAGCACACGTAGCAGTGTTAGGCTTTTTCATACAGTATGTAAGcTTAGTGTTAGTATCT
GTCAGAtGCTACCTGCTGTTACTTATTCAGATAAACATGGtGCCTATTGGAACAATAGcGGATAGAGCTAC
AGGTGTTCAGTAAGACTACAAAAACATTTTGCCTATTTCGCTAACAGTTTGGTTTTTAATGGCTGTGGtA
TTTGAGTGAGGCAACTCTGGGGCATTTGTTATGAAGAAATG

FIG 21

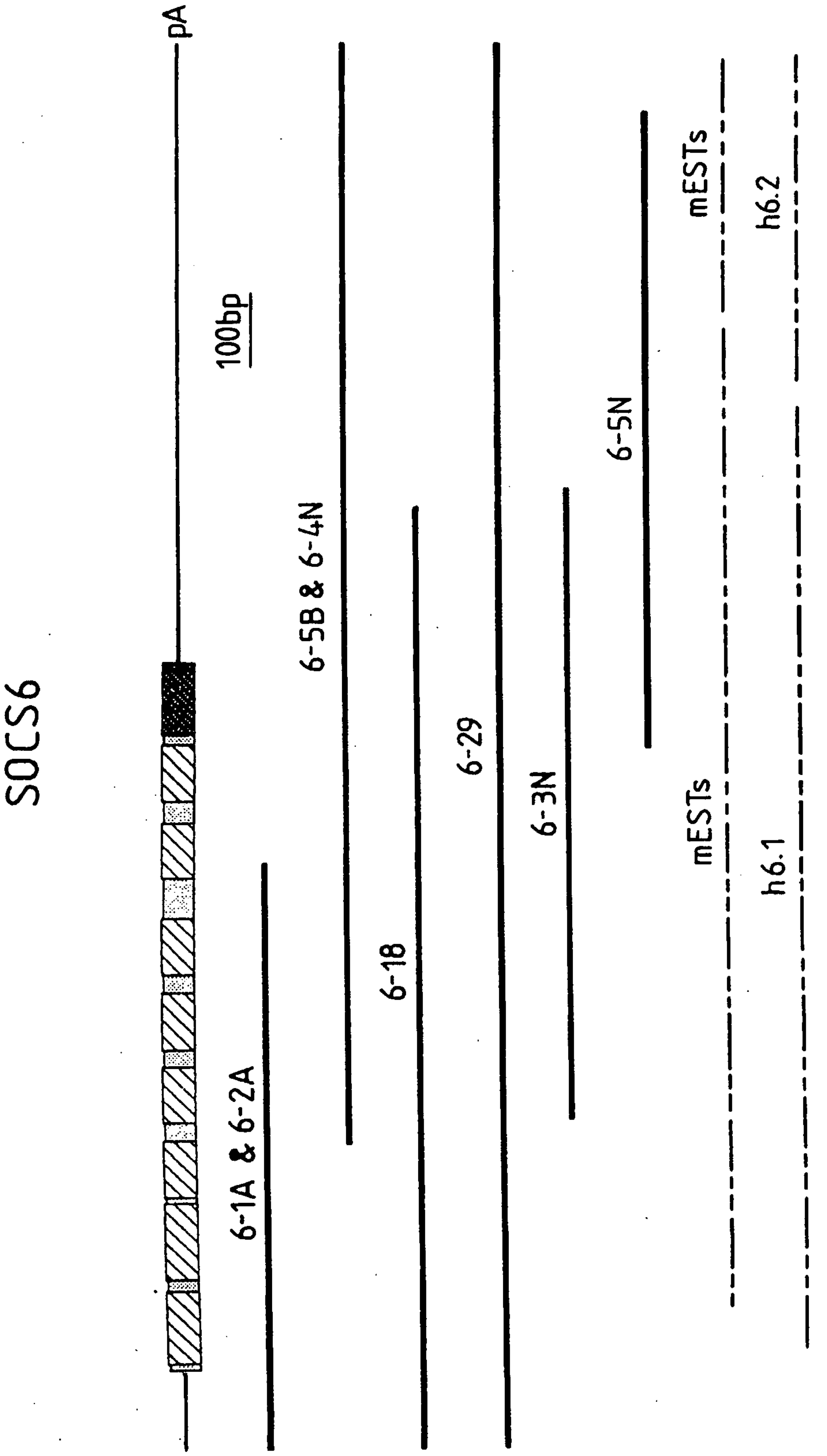
SOCS6
pA
100bp
6-1A & 6-2A
6-5B & 6-4N
6-18
6-29
6-3N
6-5N
mESTs
mESTs
h6.1
h6.2

FIG 22

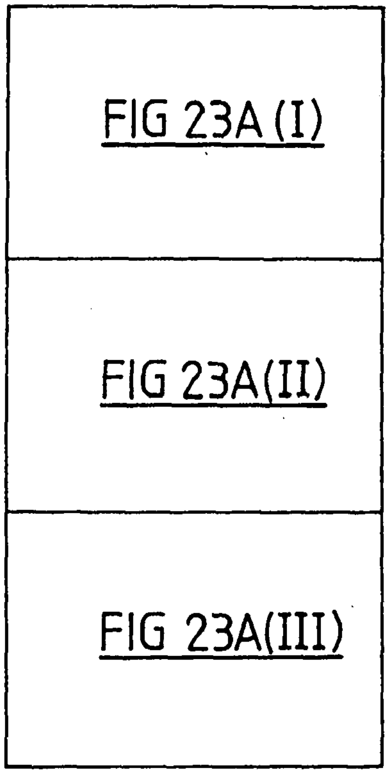


FIG 23A

ggcacgaggcggtggtggcggcggcgggcgcggccgcggcggggcgggcgcggaATGA
AGGCCCACGGCCCTGGGGGCTGAGGCGCCCGCCGCCTGGGGCGGGCCGCGCGTCCTCA
TGGAGGCCGGAGAGGAGCCGCTGCTGCTGGCTGAACTCAAGCCTGGGCGCCCCCACCA
GTTCGACTGGAAGTCAAGCTGCGAGACCTGGAGCGTGGCCTTCTCGCCAGACGGTTCC
TGGTTCGCCTGGTCTCAAGGACACTGCGTGGTCAAGCTGGTCCCCTGGCCCTTAGAGG
AACAGTTCATCCCTAAAGGATTCGAAGCCAAGAGCCGAAGCAGCAAGAATGACCCAAA
AGGACGGGGCAGTCTGAAGGAGAAGACGCTGGACTGTGGCCAGATTGTGTGGGGGCTG
GCCTTCAGCCCGTGGCCCTCTCCACCCAGCAGGAAACTCTGGGCACGTCACCATCCCC
AGGCGCCTGATGTTTCTTGCCTGATCCTGGCCACAGGTCTCAACGATGGGCAGATCAA
GATTTGGGAGGTACAGACAGGCCTCCTGCTTCTGAATCTTTCTGGCCACCAAGACGTC
GTGAGAGATCTGAGCTTCACGCCCAGCGGCAGTTTGATTTTGGTCTCTGCATCCCGGG
ATAAGACACTTCGAATTTGGGACCTGAATAAaCACGGTAAGCAGATCCAGGTGTTATC
CGGCCATCTGCAGTGGGTTTACTGCTGCTCCATCTCCCCTGACTGTAGCATGCTGTGC
TCTGCAGCTGGGGAGAAGTCGGTCTTTCTGTGGAGCATGCGGTCCTACACACTAATCC
GGAAACTAGAAGGCCACCAAAGCAGTGTTGTCTCCTGTGATTTCTCTCCTGATTCAGC
CTTGCTTGTCACAGCTTCGTATGACACCAGTGTGATTATGTGGGACCCcTACACCGGC
GcGAGGCTGAGGTCACTTCATCACACACAACTTGAACCCACCATGGATGACAGTGACG
TCCACATGAGCTCCCTGAGGTCCGTGTGCTTCTCACCTGAAGGCTTGTATCTCGCTAC

FIG 23A(I)

GGTGGCAGATGACAGGCTGCTCAGGATCTGGGCTCTGGAACTGAAGGCTCCGGTTGCC
TTTGCTCCGATGACCAATGGTCTTTGCTGCACGTTCTTCCCACACGGTGGAATTATTG
CCACAGGGACGAGAGATGGCCATGTCCAGTTCTGGACAGCTCCCCGGGTCCTGTCCTC
ACTGAAGCACTTATGCAGGAAAGCCCTCCGAAGTTTCCTGACAACGTATCAAGTCCTA
GCACTGCCAATCCCCAAGAAGATGAAAGAGTTCCTCACATACAGGACTTTCTAGcagt
gccggctcccccacctcctgcagcagcagcagtacaagggactggctaggatggagtc
aggcagctcacactggaccagtgtggaccttccttcctcccatggcatgtgcaagtag
gtctgcgtgaccccacttctgtggtgccggccttacctcgtcttcatccgtggtgagc
agccttcgtcagtctagttgtgttgaagccaagtgcagttgtggatgttgctggggta
ataaaggcaagcgggctccagagcctctctggtggcggccaagccacactcccttaac
tgggaagtacctgccacgtagggcatttctgctgcctatttccagccagcggctgcat
ggtttgaagttcctccgttgtggtcagaagaactctggtgtttggttccctgctcagc
tgcgcgtggactgggctgagctcctcaccatacactagtgccggcttttgtttcctgt
aaacagtggttgcatgtgtagagaagtaacaagcgagtattcagatcatacgaggagg
cgttcctcggtgcatgacggtcagatggccatttatcagcatatttatttgtattttc
tcagcacatagtaaggtacaactgtgttttctcaattgtctcgaaaaacagagttct
taagtggcccagttgtggagccaagtctaagtcgtgtggagtcagtgctgacatcact
ggcttgtgctgtctgtcacatgtgtttgtctctgctgcttgacctcatgggatgtacc

FIG 23A(II)

ctccagttcaactgcccaaaacagacagccccttccaagcaccgttctttgacagcgg
tagcagctacctattcaagacgcctcacacaaaatctgccttagaaagttaatatatt
ttaaattattttaaaagaaactcaacatcttattctttggcctttcttaattgatgct
ttatggaggcagtgttaacattgtacagtgtatgcatagaggagtctcctctatttga
agaacaatgcaaaatgaggctttcattgaagggaaaaaaaaaaaaaaa

FIG 23A(III)

MEAGEEPLLLAELKPGRPHQFDWKSSCETWSVAFSPDGSWFAWSQGHCVVKLVPWPLE
EQFIPKGFEAKSRSSKNDPKGRGSLKEKTLDCGQIVWGLAFSPWPSPPSRKLWARHHP
QAPDVSCLILATGLNDGQIKIWEVQTGLLLLNLSGHQDVVRDLSFTPSGSLILVSASR
DKTLRIWDLNKHGKQIQVLSGHLQWVYCCSISPDCSMLCSAAGEKSVFLWSMRSYTLI
RKLEGHQSSVVSCDFSPDSALLVTASYDTSVIMWDPYTGARLRSLHHTQLEPTMDDSD
VHMSSLRSVCFSPEGLYLATVADDRLLRIWALELKAPVAFAPMTNGLCCTFFPHGGII
ATGTRDGHVQFWTAPRV<u>LSSLKHLCRKALRSFLTTYQVLALPIPKKMKEFL</u>TYRTF*

FIG 23B

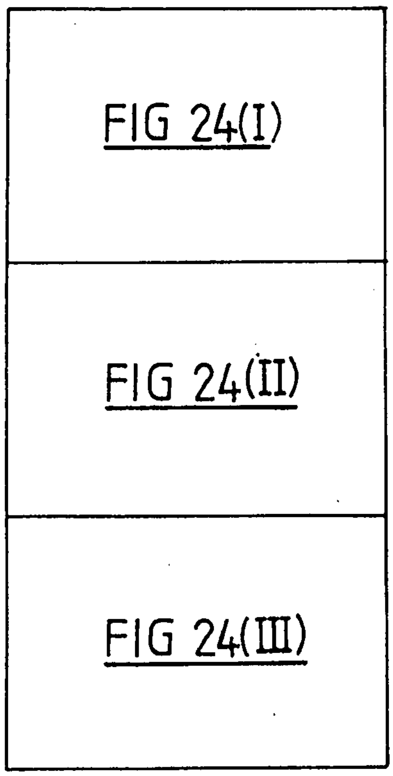


FIG 24

h6.1

GACACTGCATCGTCAAACTGATCCCCTGGCCGTTGGAGGAGCAGTTCATCCCTAAAGG
GTTTGAAGCCAAAAGCCGAAGTAGCAAAAATGAGACGAAAGGGCGGGGCAGCCCAAAA
GAGAAGACGCTGGACTGTGGTCAGATTGTCTGGGGGCTGGCCTTCAGCCTGTGNCTTT
CCCCACCCAGCAGGAAGCTCTGGGCACGCCACCACCCCCAAGTGCCCGATGTCTCTTG
CCTGGTTCTTGCTACGGGACTCAACGATGGGCAGATCAAGATCTGGGAGGTGCAGACA
GGGCTCCTGCTTTTGAATCTTTCCGGCCACCAAGATGTCGTGAGAGATCTGAGCTTCA
CACCCAGTGGCAGTTTGATTTTGGTCTCCGCGTCACGGGATAAGACTCTTCGCATCTG
GGACCTGAATAAACACGGTAAACAGATTCAAGTGTTATCGGCCACCTGCAGTGGGTT
TACTGCTGTTCCATCTCCCCAGACTGCAGCATGCTGTGCTCTGCAGCTGGAGAGAAGT
CGGTCTTTCTATGGAGCATGAGGTCCTACACGTTAATTCGGAAGCTAGAGGGCCATCA
AAGCAGTGTTGTCTCTTGTGACTTCTCCCCCGACTCTGCCCTGCTTGTCACGGCTTCT
TACGATACCAATGTGATTATGTGGACCCCTACACCGGCGAAAGGCTGAGGTCACTCC
ACCACACCCAGGTTGACCCCGCCATGGATGACAGTGACGTCCACATTAGCTCACTGAG
ATCTGTGTGCTTCTCTCCAGAAGGCTTGTACCTTGCCACGGTGGCAGATGACAGACTC
CTCAGGATCTGGGCCCTGGAACTGAAAACTCCCATTGCATTTGCTCCTATGACCAATG
GGCTTTGCTGGCACATTTTTTCCACATGGTGGAGTCATTGCCACAGGACAAGAGATG
GCCACGTCCAGTTCTGGACAGCTCCTAGGGTCCTGTCCTCACTGAAGCACTTATGCCG

FIG 24(I)

GAAAGCCCTTCGAAGTTTCCTAACAACTTACCAAGTCCTAGCACTGCCAATCCCCAAG
AAAATGAAAGAGTTCCTCACATACAGGACTTTTTAAGCAACACCACATCTTGTGCTTC
TTTGTAGCAGGGTAAATCGTCCTGTCAAAGGGAGTTGCTGGAATAATGGGCCAAACAT
CTGGTCTTGCATTGAAATAGCATTTCTTTGGGATTGTGAATAGAATGTAGCAAAACCA
GATTCCAGTGTACTAGTCATGGATTTTTC

FIG 24(II)

h6.2

ACCATGGTTCCAAGWTCCTCTCCYKCCTGTGGTCMRAAGTTGCYYCCGAATGTTGGGC
CCAAGTGCCTTTTCYCTCCTTGGGCCTCCCCTTCTGACCTGCAGGACAGTTTTCCYGG
AGCCCATTTGGTATGAGGTATTAAWTTAGCCTTAACTAAATTACAGGGGACTCAGAGG
CCGTGCTCCTGACCGATCCAGACACTATTTTTTTTTTTTTTTTTAACAATGGTGTGC
ATGTGCAGGAAATGACAAATTTGTATGTCAGATTATACAAGGATGTATTCTTAAACCG
CATGACTATTCAGATGGCTACTGAGTTATCAGTGGCCATTTATTAGCATCATATTTAT
TTGTATTTTCTCAACAGATGTTAAGGTACAACTGTGTTTTTCTCGATTATCTAAAAAC
CATAGTACTTAAATTGAAAAAAAAAA

FIG 24(III)

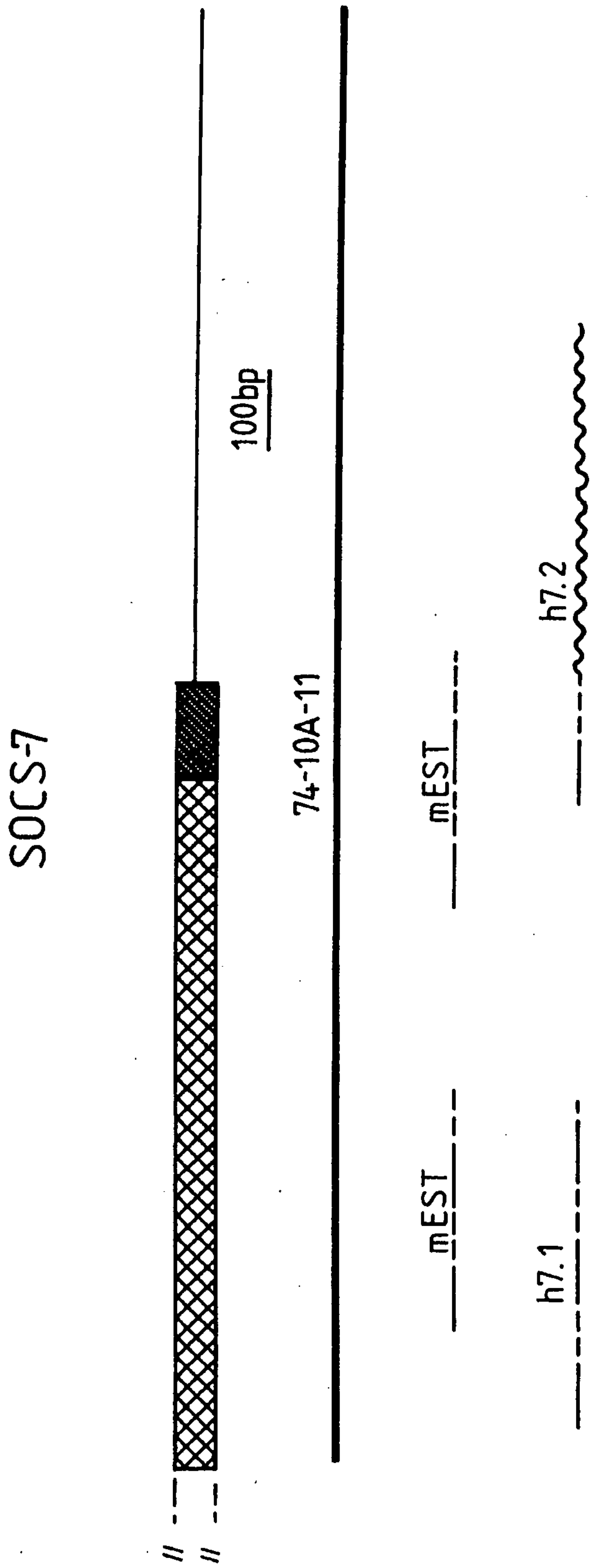


FIG 25

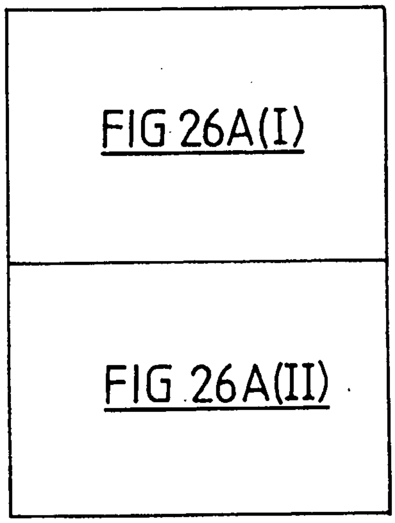


FIG 26A

GGCACGAGGCGGGGTCAGGGCGGAGGCTGAGGACCAAGTAGGCATGGCGGAGGCGGG
ACCGGCCCCGATGGACGGGCCGGCCCGGGACCCGCAGGTCCTAATCTGAAGGAGTGGC
TGAGGGAGCAGTTCTGTGACCATCCACTGGAGCACTGTGACGATACAAGACTCCATGA
TGCAGCCTATGTAGGGGACCTCCAGACCCTCAGGAACCTACTGCAAGAGGAGAGCTAC
CGGAGCCGCATCAATGAGAAGTCTGTCTGGTGCTGCGGCTGGCTTCCCTGCACACCAC
TGAGGATCGCAGCCACTGCAGGCCATGGGAACTGTGTGGACTTCCTCATACGCAAAGG
GGCCGAGGTGGACCTGGTGGATGTCAAGGGGCAGACTGCCCTGTATGTGGCTGTAGTG
AACGGGCACTTGGAGAGCACTGAGATCCTTTTGGAAGCTGGTGCTGATCCCAACGGCA
GCCGGCACCACCGCAGCACTCCTGTGTACCATGCCTyTCGTGTGGGTAGGGACGACAT
CCTGAAGGCTCTTATCAGGTATGGGGCAGATGTTGATGTCAACCATCATCTGAATTCT
GACACCCGGCCCCCTTTTTCACGGCGGCTAACCTCCTTGGTGGTCTGTCCTCTATACA
TCAGTGCTGCCTACCATAACCTTCAGTGCTTCAGGCTGCTCTTGCAGGCTGGGGCAAA
TCCTGACTTCAATTGCAATGGCCCTGTCAACACCCAGGAGTTCTACAGGGGATCCCCT
GGGTGTGTCATGGATGCTGTCCTGCGCCATGGCTGTGAAGCAGCCTTCGTGAGTCTGT
TGGTAGAGTTTGGAGCCAACCTGAACCTGGTGAAGTGGGAATCCCTGGCCCAGAGGC
AAGAGGCAGAAGAAAGATGGATCCTGAGGCCTTGCAGGTCTTTAAAGAGGCCAGAAGT
ATTCCCAGGACCTTGCTGAGTTTGTGCCGGGTGGCTGTGAGAAGAGCTCTTGGCAAAT
ACCGACTGCATCTGGTTCCCTCGCTGCCGCTGCCAGACCCCATAAAGAAGTTTTTGCT

FIG 26A(I)

TTATGAGTAGcattcacatgcagtgctgactgcaatgtggaagccgatcacctgcagt
gaaaactgacacagactctggcatcctgggaaccatggcctgtgctgccagcttgatc
cttggctgtcagtgaagaaaaacggctgtgttctcttggactgtgattctatctcag
gtgcttgggccatcgaacgctccttgagtcattgtcaactgagaggcacatacaaact
taattttgttcctcttcagtctctctgttttggattcttcctggcaatgtgtgcagca
tgggctgagcctggtgattgccctagtggggaaggctttttctccaggctatgcatc
tatttatgttcctactttgcaatttattgttcttttaaggcttgatatcaaaacagaa
agaggtttgttaagaaaagatatagggagaaaggaattccggttccgtgcacttgcta
gcctgctttccttgcctgggtttgtctgtctatgctgcctggtgcacatcccttctct
ttgctgccactgttctatttgggagttgtcttccgtctaagatggcttctggggttc
tatcttattgcacagaggtcccagaacagtgttcatagggcaccatctgctctgccaa
gggttttctgatgtcttaccctggggatcttcagacagtggttacctttaggagaccc
acctggaactaaccattaagtgactgcccacattcagatcagggaccatcttaatagt
actcactgccagtcctcacaagagaagatgacacgggtgctctcttcagacactccca
tacaggaagttggaaaatgtcttggtcacctgggttgttcccaggctacaacttcttg
gtgttccactaaraccagratatcctagtttttgggttgactgttccctccccactt
tccttgaancccaatgcccntttgtktnggttgcttccctaaaaktt

FIG 26A(II)

....ARGGVRAEAEDQVGMAEGGTGPDGRAGPGPAGPNLKEWLREQFCDHPLEHCDDT
RLHDAAYVGDLQTLRNLLQEESYRSRINEKSVWCCGWLPCTPLRIAATAGHGNCVDFL
IRKGAEVDLVDVKGQTALYVAVVNGHLESTEILLEAGADPNGSRHHRSTPVYHAXRVG
RDDILKALIRYGADVDVNHHLNSDTRPPFSRRLTSLVVCPLYISAAYHNLQCFRLLLQ
AGANPDFNCNGPVNTQEFYRGSPGCVMDAVLRHGCEAAFVSLLVEFGANLNLVKWESL
GPEARGRRKMDPEALQVFKEARSIPRTLLSLCRVAVRRALGKYRLHLVPSLPLPDPIK
KFLLYE*

FIG 26B

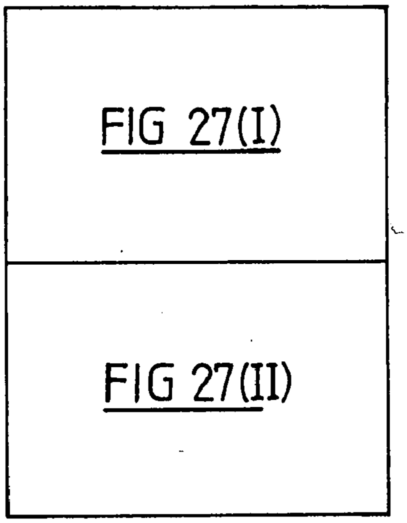


FIG 27

h7.1

GCATCCATGGCGGAGGGCGGCAGCACGACGGGCGGGCAGGGCCGGGCTCCGCAGGTCG
TAATCTGAAGGAGTGGCTGAGGGAGCAATTTTGTGATCATCCGCTGGAGCACTGTGAG
GACACGAGGCTCCATGATGCAGCTTACGTCGGGGACCTCCAGACCCTCAGGAGCCTAT
TGCAAGAGGAGAGCTACCGGAGCCGCATCAACGAGAAGTCTGTCTGGTGCTGTGGCTG
GCTCCCCTGCACACCGTTGCGAATCGCGGCCACTGCAGGCCATGGGAGCTGTGTGGAC
TTCCTCATCCGGAAGGGGGCCGAGGTGGATCTGGTGGACGTAAAAGGACAGACGGCCC
TGTATGTGGCTGTGGTGAACGGGCACCTAGAGAGTACCCAGATCCTTCTCGAAGCTGG
CGCGGACCCCAAC

FIG 27(I)

h7.2

GAGGAAGAAGAAAAGTGGACCCTGAGGCCTTGCAGGTCTTTAAAGAGGCCAGAAGTGT
TCCCAGAACCTTGCTGTGTCTGTGCCGTGTGGCTGTGAGAAGAGCTCTTGGCAAAMAC
CGGCTTCATCTGATTCCTTCGCTGCCTCTGCCAGACCCCATAAAGAAGTTTCTACTCC
ATGAGTAGACTCCAAGTGCTGCGGTTGATTCCAGTGAGGGAGAAAGTGATCTGCAGGG
AGGTGGACACCGAGCCCTGAGTGCTGTGCTGCTGCTGGTCTCCTGATGGCTGTTGCTG
CAGAAGATGTCCTCGTAGACTGTCATTGCTCCTCAGGTGCCTGGGCCGCTGAACAGTC
CTTGGGTCATTGTCAGCTGAGAGGCTTATACTAAAGTTATTATTGTTTTTCCCAAGTT
CTCTGTTCTGGATTTTCAGTTGCATATTAATGTAACGGGCCATGGGGTATGTACATGT
AGGGGCTGAGGTTGGAGGCCTACTAATTTCCTGTAGGGAAGACTCCCAGCACTTCTGG
AACTGTGCTTCTCTTTATTTTTCTACTTCTCAATTTGATGGTTCGATTAAAGCCTTCT
AGTATCTCAATGAAAA

FIG 27(II)

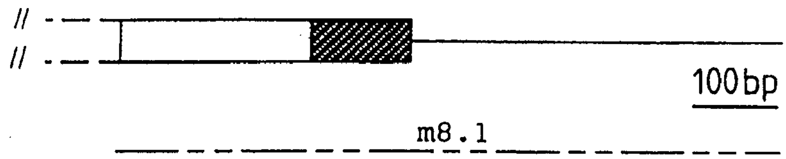


FIG 28

CTGATGTCCGCAATTCTGAAGGTTGGACACCACTGCTGGCTGCCTGTGACATCCGCTG
TCAATCCCCAAAGGATGCTGAGGCCACCACCAACCGCTGTTTTCAACTGTGCCGCTTG
CTGCTGTCTGTGGGGGCAGATGCTGATGAATACATACCGTGTAGTTCAGCTTCCTGAG
GAGGCCAAGGgCTTGGTGCCACCAGAGATTCTACAGAAGTACCATGGATTCTACTCTT
CCCTCTTTGCCTTGGTGAGGCAGCCCAGGTCGCTGCAGCATCTCTGCCGTTGTGCGCT
CCGCAGTCACCTGGAGGGCTGTCTGCCCCATGCACTACCGCGCCTTCCCCTGCCACCG
CGCATGCTCCGCTTTCTGCAGCTGGACTTTGAGGATCTGCTCTACTAGgcttgctgcc
ctgtgaacaaagcagaccccacccccaccccaagggcatctctcagcaatgaatgatg
caaggcggtctgtcttcaagtcaggagtggacgccttgatccacacttgagagaagag
gccagatcagcaccyggctggtagtgatngcagagggcacctgtgcagatctgtgtgc
gcactggaaatctctaggctgaaggcyagagcaaatggtgcargtgttagtccttggg
angagagacaganggtgagaaagcaagacagaggtgagagtgcacatgtcaagtggta
gattgccttaaaagaaagctaaaaaagaaaaagattcgggcgaacttctttaggggt
aatgctgcagcgtgttaaactgactgaccagcgtccatatctttggacccttcccggg
tgaaaaagccccttcatcctccagcgctccccaagggtgcttagcaataccgggtgct
tttctgccgcaaagtgagttaccaaa

FIG 29A

....MSAILKVGHHCWLPVTSAVNPQRMLRPPPTAVFNCAACCCLWGQMLMNTYRVVQ
LPEEAKGLVPPEILQKYHGFYSSLFALVRQ<u>PRSLQHLCRCALRSHLEGCLPHALPRLP</u>
<u>LPPRMLRFL</u>QLDFEDLLY*

FIG 29B

SOCS-9

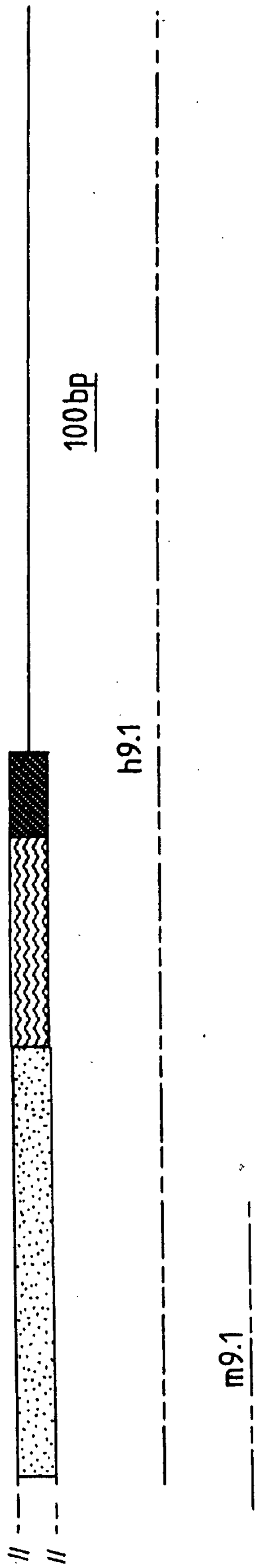


FIG 30

GTGGGGGCGTCATCATGACCTCCTCTAGGGCTCTGCAACATGACTCCTGTGGTGCAAA
TCAACAAATTGTTCACTGATGAATCCACAAGGATCTCTGGGCCTACAACCAGGTCCTG
GTCCACATGACTGTCGTCTTCGGAGAAGGCACCACTCGCCCCCGGCAGGTACGGCTGA
CACCTCCATGGGAGAAGACGTATCCAGGCAGCAGCTGCGCGGCCCTTCAAGAGGGCAC
ATCCCGTCATCTAAAGGCACGGTGTACTGAAGGTAGTCCTGAGACATGAGTCCGATTA
CTACAGGCACGTGTTCCTCCAGGTGGAGGCTCAGGTCCCCGGGTGAGCTGGGGCTGCA
GCGGGACTCAGGGCGCGGCTCTGGCTGCAGGTCTCGCAGCTCCCTGGGCTGTAGCTCC
CGCAGATCCTTGCGCACACCGTTGACTGGT

FIG 31

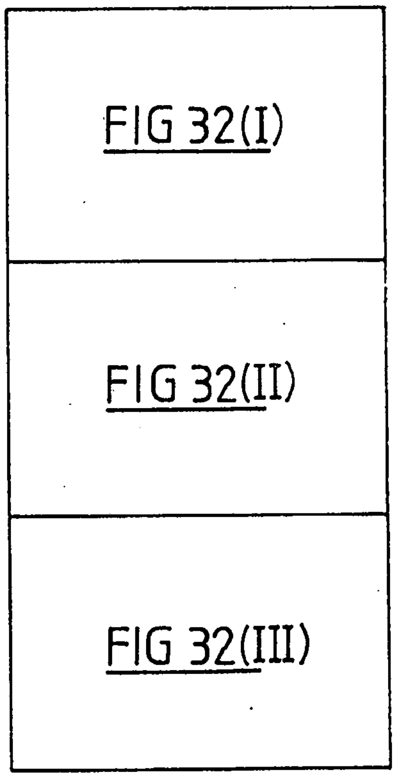


FIG 32

TTAATAGTACCTACATAGTAGAAAATTATAACTCCACTTTAAAACAATGTTTTCTTTC
TATTCAAATCAATTTAAAACTTTTTATAAACATTAATGTTGCAAGAGAATCCAGTCCA
TTTATGAAAATTAGTTGACAATCAAGTTCACCCAAGAAAATGTTGACTAAGCTAAAGA
AATCACAGATAAAACATTTTACCAAAAGGATAGGTAACACACAAAAAAATGCTATCAC
AGGAAGCTNATGATCATCTAATATTTCTTTAATAATAATTCTAGTTCCATAGGTTTTC
ATGTTATGCCAATTTGTACCCGAGTTTAATTACAGAAAAGGCAACAATTTCTAAATTG
GTGGTATACATTTCTTTACAATTTTTTAATGTAAGGCCATTTATTAAAATAGACAAAC
TAGAAGATGAAAACGAAGGCAACAGAAAAATTCAACTTTTCACAACCAAAAGAATTAG
CACAACCTTAGAAATAATTTAGAAAAAAGTGTTGTTAAAAGATATGTTGCAGATCTCC
GTTCCATTACCCAAGATTATGTCAATTCACGATTCTAAATAAATCTTTTTAAAGTAAG
AGATTAAAAACTCATCTTCAGTGTATATGTAAATTCCGTGGTTTTATCACACAGGTAT
GTTTATTCAACACTGKCTTTGGAAANTGGACCATTTAAAAGGACATGGCAATTTCCAT
TCTGTTAAGTTTCATTCAACCTTTACTTAGGGGTTGRATTACCACATGAAATGNTGCT
TTTAATGCATAAAAATCACAGTGGATTAGCCAGCAAAAGGGACTGGCGGGGGGGGCA
TTGAGGAGAATTTGATAATTCACATTGTGATTATTCTGCACATTGATGAAACATAATT
CACACCTCTAAAACCTCAAGACTTCCCTTTTTTAAAGAACCAAAATAAACCCAAGACA
CCTTGCTGACACTTCCCCACCCCTAAACAAACTGATGACTCTTTTACACATAAAACTG
AAATAGTTATGGCAGCAAAAGATTTTGATGGCAATGAAAGTTTGTAAACTGTATTTCA

FIG 32(I)

ATCTCTTGTTCTTATTCCCAAAGTGCAAGATGCAGGGTTCTCAATCTTTCAGTAGTGC
TTCTCCTGTAAATAATCCTTCATTTTGTTTGGCAAAGGCAGTTTCTGAATTAAGTCTA
TTCTGGTATACTGACGTATAACAAAACGACACAGGTACTGCAACGAGCGCACCTSSAT
GAACNCCCGRGAACACTGGSTTGGYCAAGTTCTNGACRRGGKAAGKTGCAGATTCCAG
GCAGCYGAGACCTTGAATAACAAAAAGCTCCCATTTTCAGAGTCCCTGATTGAATGCT
CCAATTAGATCAACTATGGACGTATGTCCTTCCACATCNGGCTGTTCATAAAAGCTAA
ACCTACCATTTGAGTGCTCAATTCTAGTGTGAAGTGTTTTACCATGGGAGCGAAAGTC
ACAGCTTAAAAGGTAACGGTCGTCAGAACTGTCCCGAACAAGAAAAGAACCATCTGGC
ACGTTTGCTAGCTTCCCTTCTGCCTCCCAACGTGTGATTGGTCCCCAGTACCATCCTT
GCTTTGCAAGTTTTTTCAGCTCCTCTGTAAGGCTTGTCACAACCATGGGACCACTACT
TTGCACTGAGTCATAAACTCTTGCAACCCCAGGAGCAGAGTTCGGATCAAAATTCAAA
TGACAGCGCATAACTTTNCAGCCACGTGGGGCTTTCTGTSCCAGTGAGTCCACTGAAA
GTTCCCCTTTGGGATTTGGATTATTCCTGCATTGGAGNTAACCAATGGTGAAGATTGG
AGGGACATCCATCGTGAACCCGCTCTCCGGGGTTCTGCAACATGACTCCCGTGGTGCC
AATCAACAAGCCATTCACCGGACTGATCCACGAAGATCTCTGGGGCGACAACTAGGTC
CTGGTCTACCTGACTCTCATCCTCGGGGAAAGCGCGCCCTCCCACTTGAGGAGGAACC
GCAGAGACTTCCATGGGAGAAGAGCTGTCCAGACAATAGCTCCGTGATCCTTCCAAAG
GATACATCCCCTCATCTAAAGGCACAGTATACTGAATGTAGTCCTGAGGCATAAGTCC

FIG 32(II)

AATAACGACAGGCACATGTTCATCCAGGTGAAGATGCAGGTCTCCATTATGAGAAGCC
GAGCTCTTCAGTGAATTGGCTTGCTCCTGGCACGTGGTCTCAGACTGGAGGTCGT

FIG 32(III)

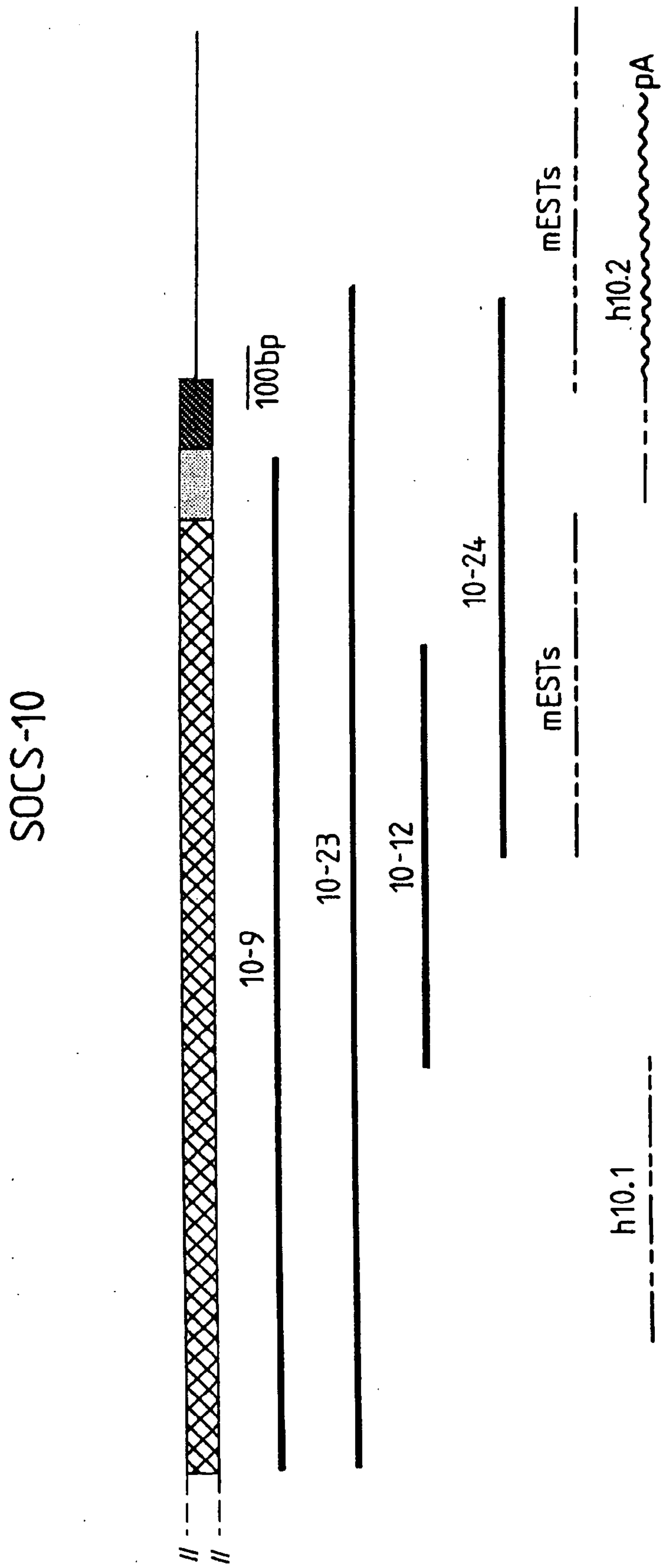


FIG 33

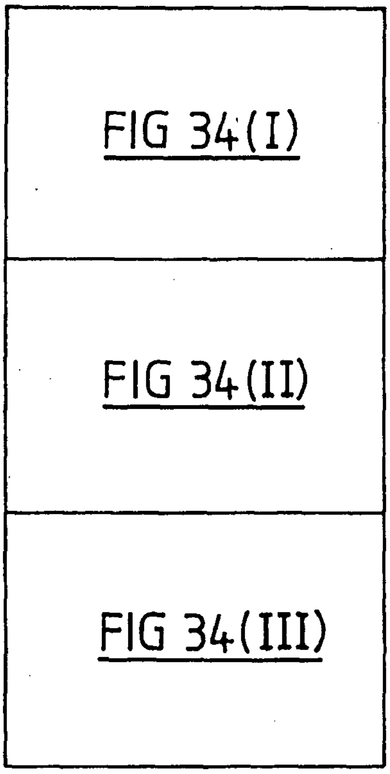


FIG 34

GGCACGAGGCTGTGTCCAGCACACAGAGAGGGCCCGGCCATCTGCTTTGGTTCAGAGC
CCTGTGTCTGTCTGTCACTTAGACTCTTCCTCCCGGCTCGCAGCTCACCCTCCATCCT
CCTTACTGGCTCCAGCATGACTCGCTTCTCTTATGCAGAGTACTTTGCTCTGTTTCAC
TCTGGCTCTGCACCTTCCAGGTCCCCTTCGTCTCCCGAGAACCCACCGGCCCGCGCAC
CCCTGGGTCTGTTCCAAGGGGTCATGCAGAAGTATAGCAGCAACCTGTTCAAGACCTC
CCAGATGGCGGCTATGGACCCCGTGCTGAAGGCCATCAAGGAAGGGGATGAAGAGGCC
TTGAAGATCATGATCCAGGATGGGAAGAATCTTGCAGAGCCCAACAAGGAGGGCTGGC
TGCCGCTCCACGAGGCTGCCTACTATGGCCAGCTGGGCTGCCTGAAAGTCCTGCAGCA
AGCCTACCCAGGGACCATTGACCAACGCACACTGCAGGAAGAGACAGCATTATACCTG
GCCACATGCAGAGAACACCTGGATTGCCTCCTGTCGCTGCTCCAGGCGGGGGCAGAGC
CTGACATCTCTAACAAATCCAGGGAGACTCCACTTTACAAAGCCTGTGAGCGCAAGAA
CGCGGAGGCGGTGAGGATATTGGTGCGATACAACGCAGACGCCAACCACCGCTGTAAC
AGGGGCTGGACCGCACTGCACGAGTCTGTCTCCCGCAATGACCTGGAGGTCATGGAGA
TCCTAGTGAGTGGCGGGGCCAAGGTGGAGGCCAAGAATGTCTACAGCATCACCCCTTT
GTTTGTGGCTGCCCAGAGTGGGCAGCTGGAGGCCCTGAGGTTCCTGGCCAAGCATGGT
GCAGACATCAACACGCAGGCCAGTGACAGTGCATCAGCCCTCTACGAGGCCAGCAAGA
ATGAGCATGAAGACGTGGTAGAGTTTCTTCTCTCAGGGCGCCGATGCTAACAAAGC
CAACAAGGACGGCCTGCTCCCCCTGCATGTTGCCTCCAAGAAGGGCAACTATAGAATA

FIG 34(I)

GTGCAGATGCTGCTGCCTGTGACCAGCCGCACGCGCGTGCGCCGTAGCGGCATCAGCC
CGCTGCACCTAGCGGCCGAGCGCAACCACGACGCGGTGCTGGAGGCGCTGCTGGCCGC
GCGCTTCGACGTGAACGCACCTCTGGCTCCCGAGCGCGCCCGCCTCTACGAGGACCGC
CGCAGTTCTGCGCTCTACTTCGCTGTGGTCAACAACAATGTGTACGCCACCGAGCTGT
TGCTGCTGGCGGGCGCGGACCCCAACCGCGATGTCATCAGCCCTCTGCTCGTGGCCAT
CCGCCACGGCTGCCTGCGCACCATGCAGCTGCTGTTGGACCATGGCGCCAACATCGAC
GCCTACATCGCCACTCACCCCACCGCCTTTCCAGCCACCATCATGTTTGCCATGAAGT
GCCTGTCGTTACTCAAGTTCCTTATGGACCTCGGCTGCGATGGCGAGCCCTGCTTCTC
CTGCCTGTACGGCAACGGGCCGCACCACCCGCCCCGCGACCTGGCCGCTTCCACGACG
CACCCGTGGACGACAAGGCACCTAGCGTGGTGCAGTTCTGTGAGTTCCTGTCGGCCCC
GGAAGTGAGCCGCTGGGCGGGACCCATCATCGATGTCCTCCTGGACTATGTGGGCAAC
GTGCAGCTGTGCTCCCGGCTGAAGGAGCACATCGACAGCTTTGAGGACTGGGCTGTCA
TCAAGGAGAAGGCAGAACCTCCGAGACCTCTGGCTCACCTCTGCCGGCTGCGGGTTCG
GAAGGCCATAGGAAAATACCGGATAAAACTCCTGGACACACTGCCGCTTCCCGGCAGG
CTAATCAGATACTTGAAATATGAGAATACACAGTAAccagcctggagaggagatgtgg
ccttcagactgtttccgggacgccccaggtggcctgcatccaggaccccctggggtca
gaacaggtgtgaccttgctggttctttgctggagcttcacccaaagtgagaacctgat
gtggggagtggacgtggaacctctgctttcacactgtcagcggatcgcagacccgctc

FIG 34(II)

tgcttctggccatagccagagaccttcaacctggggccagggagagctggtctgggc
aaggtggcccaggcaggaatcctggccttaagctggagaacttgtaggaatccctcac
tggaccctcagctttcaggctgcgagggagacgcccagcccaagtatttatttcwcg
tgacacaataacgttgtatcagaaaaaaaaaaaacatgggcgcagcttattccttag
tagggtatttacttgcatgcngcgcttaaagcntactggaaacatgcgttccnactat
gcttgagaatccccttgcactggtaaacgagagccgacgtgcttcaaggttggatttt
tggnttgccccttggcgttccgcgggtttgntccgacngtaattgaccccgtgtttt
gtcactttcgagtgttccgactattgggggcttttggttgtccccaaaattgtgggt
ggtgtgcggacgccacgagaagtggttcatgggcgataatcattactgngagaatgta
gagcggcggttttacgaataaatatttttaagccgccttcccaaaa

FIG 34(III)

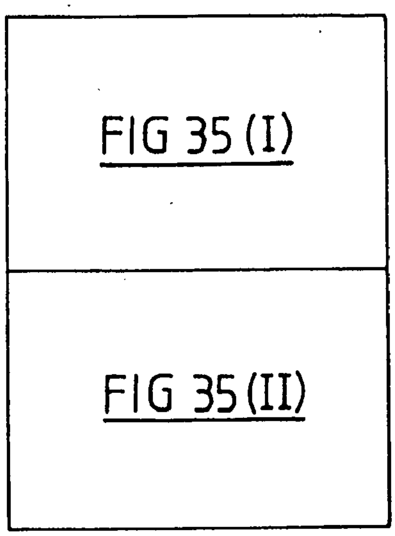


FIG 35

h10.1

CCTCCTGAGAGTTCGCCGGCCCGGGCCCAATGGGnTTGTTCCAAGGGGTCATGCAGAA
ATACAGCAGCAGCTTGTTCAAGACCTCCCAGCTGGCGCCTGCGGACCCCTTGATAAAG
GCCATCAAGGATGnCGATGAAGAGGCCTTGAAGACCATGATCAAGGAAGGGAAGAATC
TCGCAGAGCCCAACAAGGAGGGCTGGCTGCCGCTGCACGAGGCCGCATACTATGGCCA
GGTGGGCTGCCTGAAAGTCCTGCAGCGAGCGTACCCAGGGACCATCGACCAGCGCACC
CTGCAGGAGGAAACAGCCGTTTACTTGGCAACGTGCAGGGGCCACCTGGACTGTCTCC
TGTCACTGCTCCAAGCAGGGGCAGAGCGGGACATCTCCAACAAATCCCGAGAGAnACC
GCTCTACAAAGCCTGTGAGCGCAAGAACGCGGAAGCCGTGAAGATTCTTGGTGCAGCA
CAACGCAGACACCAACAACGCTGCAACCGGGCTG

FIG 35(I)

h10.2

GTGCAGCTCTGCTCGCGGCTGAAGGAACACATCGACAGCTTTGAGGACTGGGCCGTCAT
CAAGGAGAAGGCAGAACCTCCAAGACCTCTGGCTCACCTTTGCCGACTGCGGGTTCGAA
AGGCCATTGGGAAATACCGTATAAAACTCCTAGACACCTTGCCGCTCCCAGGCAGGCTG
ATTAGATACCTGAAATACGAGAACACCCAGTAACTGGGGCCACGGGGAGAGAGGAGTAG
CCCCTCAGACTCTTCTTACTAAGTCTCAGGACGTCGGTGTTCCCAACTCCAAGGGGACC
TGGTGACAGAcGAGGCTGCAGGCTGCCTCCCTCTCAGCCTGGACAGCTACCAGGATCTC
ACTGGGTCTCAGGGcCCAGAGCTTTGGCCAGAGCAGAGAACAGAATGTGTCAAGGAGAA
GAATCATTTGTTTACAAACTGATGAGCAGATCCCAGACCTTCTCTACCTTCAGGAATGG
CAGAAACCTCTATTCCTGGGGCCAGGGCAGAGCTTGAGGTGTTCTGGGGAAGGTGGTGC
TCAGAGCCTTCCCTGTGCCCCTCCACTTGTTCTGGAAAACTCACCACTTGACTTCAGAG
CTTTCTCTCCAAAGACTAAGATGAAGACGTGGCCCAAGGTAGGGGGTAGGGGGAGCCTG
GGTCTTGGAGGGCTTTGTTAAGTATTAATATAATAAATGTTACACATGTGAAAAAAAAA

FIG 35(II)

TTGGAGAAGTGTGGTTGGTATTGGGGGCCAATGAATTGGGAAGATGCAGAGATGAAGC
TGAAAGGGAAACCAGATGGTTCTTTCCTGGTACGAGACAGTTCTGATCCTCGTTACAT
CCTGAGCCTCAGTTTCCGATCACAGGGTATCACCCACCACACTAGAATGGAGCACTAC
AGAGGAACCTTCAGCCTGTGGTGTCATCCCAAGTTTGAGGACCGCTGTCAATCTGTTG
TAGAGTTTATTAAGAGAGCCATTATGCACTCCAAGAATGGAAAGTTTCTCTATTTCTT
AAGATCCAGGGTTCCAGGACTGCCACCAACTCCTGTCCAGCTGCTCTATCCAGTGTCC
CGATTCAGCAATGTCAAATCCCTCCAGCACCTTTGCAGATTCCGGATACGACAGCTCG
TCAGGATAGATCACATCCCAGATCTCCCACTGCCTAAACCTCTGATCTCTTATATCCG
AAAGTTCTACTACTATGATCCTCAGGAAGAGGTATACCTGTCTCTAAAGGAAGCGCAGCGT
CAGTTTCCAAACAGAAGCAAGAGGTGGAACCCTCCACGTAGCGAGGGGCTCCCTGCTG
GTCACCACCAAGGGCATTTGGTTGCCAAGCTCCAGCTTTGAagaaccaaattaagcta
ccatgaaaagaagaggaaaagtgagggaacaggaaggttgggattctctgtgcagaga
ctttggttccccacgcaagccctggggcttggaagaagcacatgaccgtactctgcgt
ggggctccacctcacacccacccctgggcatcttaggactggaggggctccttggaaa
actggaagaagtctcaacactgtttcttttca

FIG 36A

....LEKCGWYWGPMNWEDAEMKLKGKPDGSFLVRDSSDPRYILSLSFRSQGITHHTR

MEHYRGTFSLWCHPKFEDRCQSVVEFIKRAIMHSKNGKFLYFLRSRVPGLPPTPVQLL

YPVSRFSNVKSLQHLCRFRIRQLVRIDHIPDLPLPKPLISYIRKFYYYDPQEEVYLSL

KEAQRQFPNRSKRWNPPRSEGLPAGHHQGHLVAKLQL*

FIG 36B

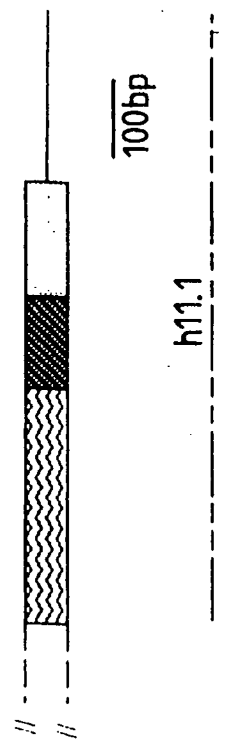


FIG 37

FIG 38

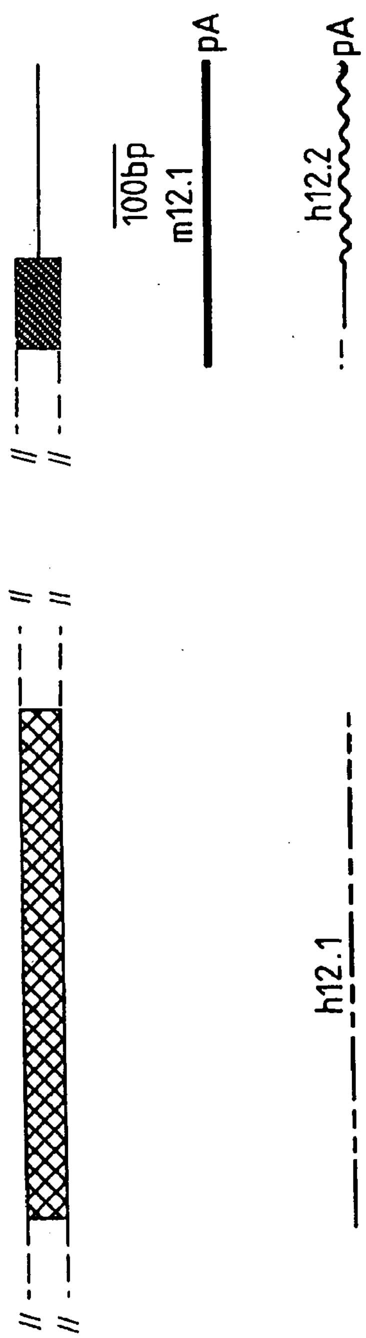

GTTCCAAGCCTAACCCATCTTTGTCGTTTGGAAATTCGGGCCAGTCTAAAAGCAGAGC
ACCTTCACTCTGACATTTTCATCCATCAGTTGCCACTTCCCAGAAGTCTGCAGAACTA
TTTGCTCTATGAAGAGGTTTTAAGAATGAATGAGATTCTAGAACCAGCAGCTAATCAG
GATGGAGAAACCAGCAAGGCCACCTGAcacaggtcctttaattctgtttagtcacaaa
agacggcttgtgtgactgtttggatttggtgatcaaatgtccatgtttacagttgctt
ttcccagtttgtgtctttcccaatattgtgaaccttatccatcttgccttactcagtt
ttatttctagtgcactttgttgtgtattatttgtttacctgaccattttctactttat
tctgctaataaactgtaattctgaaaaaaaaaaaaaaaaaaaaaaaaaaaaaaaaaaaa

FIG 39

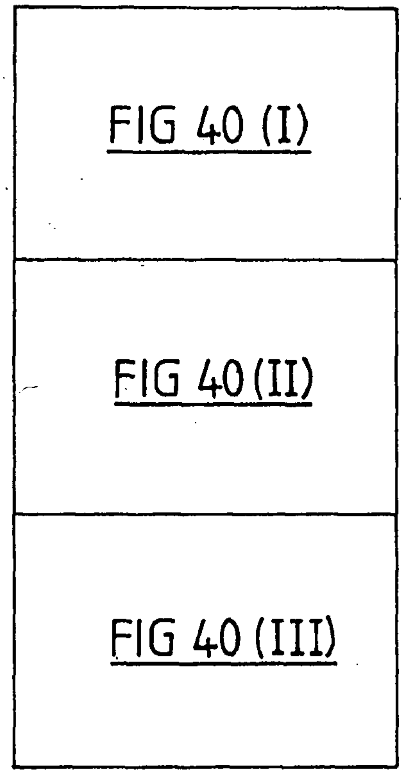


FIG 40

h12.1

GGGGATCGAAAGCGGGGGCTTCTGGGACGCAGCTCTGGAGACGCGGCCTCGGACCAGC
CATTTCGGTGTAGAAGTGGCAGCACGGCAGACTGGTCAAACAAATGGATTTTACAGAG
GCTTACGCGGACACGTGCTCTACAGTTGGACTTGCTGCCAGGGAAGGCAATGTTAAAG
TCTTAAGGAAACTGCTCAAAAAGGGCCGAAGTGTCGATGTTGCTGATAACAGGGGATG
GATGCCAATTCATGAAGCAGCTTATCACAACTCTGTAGAATGTTTGCAAATGTTAATT
AATGCAGATTCATCTGAAAACTACATTAAGATGAAGACCTTTGAAGGTTTCTGTGCTT
TGCATCTCGCTGCAAGTCAAGGACATTGGAAAATCGTACAGATTCTTTTAGAAGCTGG
GGCAGATCCTAATGCAACTACTTTAGAAGAAACGACACCATTGTTTTTAGCTGTTGAA
AATGGACAGATAGATGTGTTAAGGCTGTTGCTTCAACACGGAGCAAATGTTAATGGAT
CCCATTCTATGTGTGGATGGAACTCCTTGCACCAGGCTTCTTTTCAGGAAAATGCTGA
GATCATAAAATTGCTTCTTAGAAAAGGAGCAAACAAGGAATGCCAGGATGACTTTGGA
ATCACACCTTTATTTGTGGCTGCTCAGTATGGCCAAGCTAGAAAGCTTTGAAGCATAC
TTATTTCATCCGGGTGCAAATGTCAATTGTCAAGCCTTGGACAAAGCTACC

FIG 40 (I)

h12.2

CACAAATGGGACCATACAAAAATCTTGGNACTTGTTAATAACCACTTNACTAACCGGG
ACCTGTGACACTGGGNCTAAACAAAGTAAGTCCCTGTTTACTCAGNCAGTGTTTGGGG
GACATGAAGGATTGCCTAGNAAATATTACTCCGGAATGGTCTACAGCCCAGNACGCCC
AGGCGTGCCTTGTTTTTGGATTCAGTTCTCCTGTGTGCATGGCTTTCCAAAAGGAGGT
GGAGCTGTRAGTTCTTTGGAATTGTGAACATTCTTTTGAAATATGGAGCCCAGATAAA
TGAACTTCATTTGGCATACTGCCTGAAGTACGAGAAGTTTTCGATATTTCGCTACTTT
TTGAGGAAAGGTTGCTCATTGGGACCATGGAACCATATATATGAATTTGTAAATCATG
CAATTAAAGCACAAGCAAAATATAAGGAGTGGTTGCCACATCTTCTGGTTGCTGGATT
TGACCCACTGATTCTACTGTGCAATTCTTGGATTGACTCAGTCAGCATTGACACCCTT
ATCTTCACTTTGGAGTTTACTAATTGGAAGACACTTGCACCAGCTGTTGAAAGGATGC
TCTCTGCTCGTGCCTCAAACGCTTGGATTCTACAGCAACATATTGCCCACTGTTCCAT
CCCTGACCCATCTTTGTCGTTTGGAAATTCGGTCCAGTCTAAAATCAGAACGTCTACG
GTCTGACAGTTATATTAGTCAGCTGCCACTTCCCAGAAGCCTACATAATTATTTGCTC
TATGAAGACGTTCTGAGGATGTATGAAGTTCCAGAACTGGCAGCTATTCAAGATGGAT
AAATCAGTGAAACTACTTAACACAGCTAATTTTTTTCTCTGAAAAATCATCGAGACAA

FIG 40 (II)

AAGAGCCACAGAGTACAAGTTTTTATGATTTTATAGTCAAAAGATGATTATTGATTGT
CAGATAGGTTAGGTTTTGGGGGCCAGTAGTTCAGTGAGAATGTTTATGTTTACAACT
AGCCTTCCCAGTAAAAAAAAAAAAAAAAAAAAAAAAAAAA

FIG 40 (III)

SOCS-13

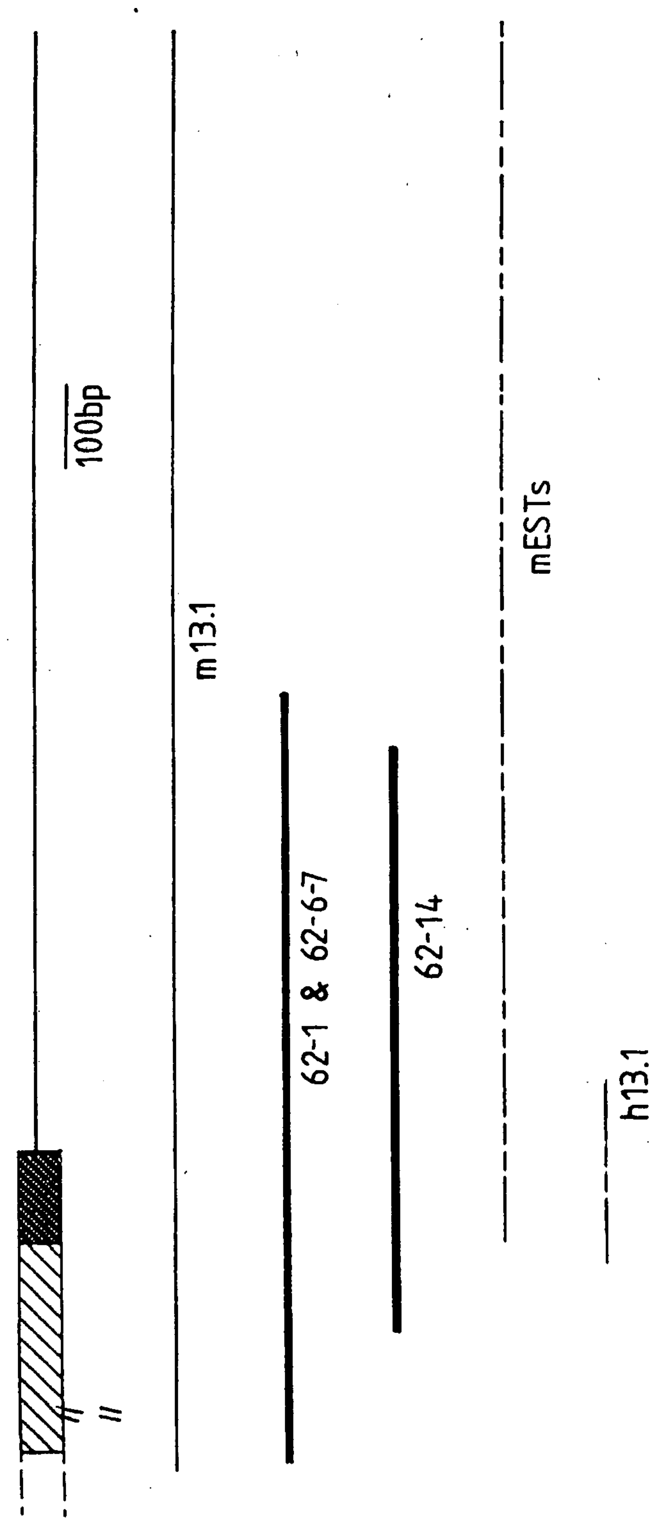


FIG 41

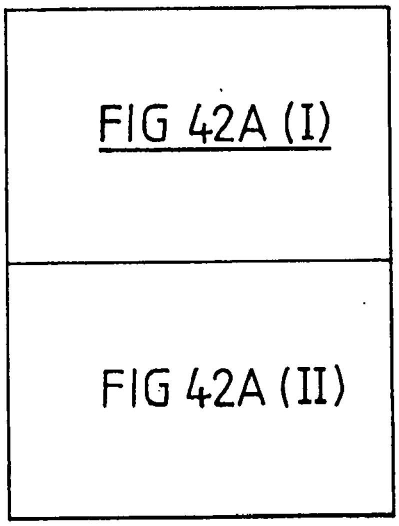


FIG 42A

CGGGGGGCTGGGACCTGGGGCGTAACCGTCTCTACCACGACGGCAAGAACCAGCCAAG
TAAAACATACCCAGCCTTTCTGGAGCCGGACGAGACATTCATTGTCCCTGACTCCTTT
TTCGTGGCCCTGGACATGRATGATGGGACCTTAAGTTTCATCGTGGATGGACAGTACA
TGGGAGTGGCTTTCCGGGGACTCAAGGGTAAAAAGCTGTATCCTGTAGTGAGTGCCGT
CTGGGGCCACTGTGAGATCCGCATGCGCTACTTGAACGGACTTGATCCTGAGCCCCTG
CCACTCATGGACCTGTGCCGGCGTTCGGTGCGCCTAGCGCTGGGAAAAGAGCGCCTGG
GTGCCATCCCCGCTCTGCCGCTACCTGCCTCCCTCAAAGCCTACCTCCTCTACCAGTG
Atccacatcccaggaccgccatacgacagccatctggtgccaartcactgagcccgtt
ggggtccgccgacccctgcgcctgggatggaygcccacctcagccatgggcagacgtg
ccccctcatcctaccggctgcctctgctgggggaacctatgccaacggacttctccct
tcccaacactggctgaagcagcagcacccaggcccttccctgaaccagatgcagagaa
taaactatgaaaacctctctcaggcgccttctgctctcaggtggagtgggctgccccc
cactctctgcagagagaggctacacccacctgggggtcctgggaggtaagactagta
ggaggtgccagggctgartccaaaagcaggaatggccaggamcaggccatacagatga
agctcaggatgtcacataccatggacamtgagacagaaccccaggttggamttccctt
gggccaacgagtgccagctttaatgtcagctgcmggtgctctgtggcctgtatttatt
ctttaaacagtagcaaaggccatttatttattccacttagaaaggaaaccttggtggg
tggyttccctcgatgtgctttcccccacctccctggaatgtgtgtgccacacctgtcc

FIG 42A(I)

ttgtcccaggccaggactgtggcacatgagctggtgtgcacagatacacgtatgtcgt
cgtgcatgaccctgactagttcctaagtagccctgcaccaagcaccagagcagaccc
caagagaggcccgtgcaagtccccatgtccccaggtccctgcttctgttgccttggga
ctcatacaccggcacacgtgtttcagcctcttgacttccatgagcttcgaattttgcc
cccgattcttctgatatttcccattggcatcctccaaagctctgggcctggagggcat
taggacacatggaatgagtggggtctccagcccctgggaaagccactggcaaggcagg
attagaaagaccaagagcagggtggggcgccatgaagcctgtatgcctctcaggctca
agaccccgccacacacccactcaagcctcagaagtggtgtgtagggcagccccaggag
aggaatgcctgtcctagcagcacgtacatggagcaccccacatgtgctccagccctct
ggctgtttctcttgctctagaatcaactccctacattgggaatgtagccatttggtag
aggacttgcctagcctgcaggaagctcacgttccatcccctgcaccaaggagaatcaa
agctcaggaggctgaggcaggaggattgctgtcagtggtgtacagaggtcatggccat
cctgggctatattaaaccttgtcctttaagaaaaagaaaagaaatcaacttccattga
atctgagttctgctcatttctgcacaggtacaatagatgacttkatttgttgaaaaat
gkttaatatatttacmtatatatatatttgtaagaagcatt

FIG 42A(II)

....GGWDLGRNRLYHDGKNQPSKTYPAFLEPDETFIVPDSFFVALDMXDGTLSFIVD
GQYMGVAFRGLKGKKLYPVVSAVWGHCEIRMRYLNGLDPEPLPLMDLCRRSVRLALGK
ERLGAIPALPLPASLKAYLLYQ*

FIG 42B

AAGGGTAAAAAACTGTATCCTGTAGTGAGTGCCGTCTGGGGCCACTGTNAGATCCGAA
TGCGCTACTTGAACGGACTCGATCCCGAGACNTGCCGCTCATGGATTTGTGCCGTCGC
TCGGTGCGCCTGGCCCTGGGGAGGGAGCGCCTGGGGGAGAACCANCNACCTGCCGCTG
CCGGCTTCCCTCAAGGCCTACCTCCTCTACCAGTGACGTTCGCCATCATACCGCCAGC
GCGACAGCCACCTGGTGCCAACTCACTGAGCCGCCTG

FIG 43

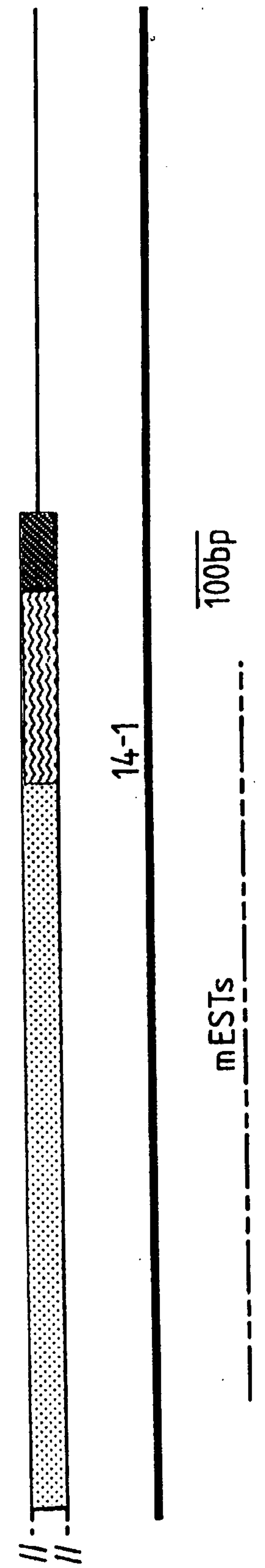


FIG 44

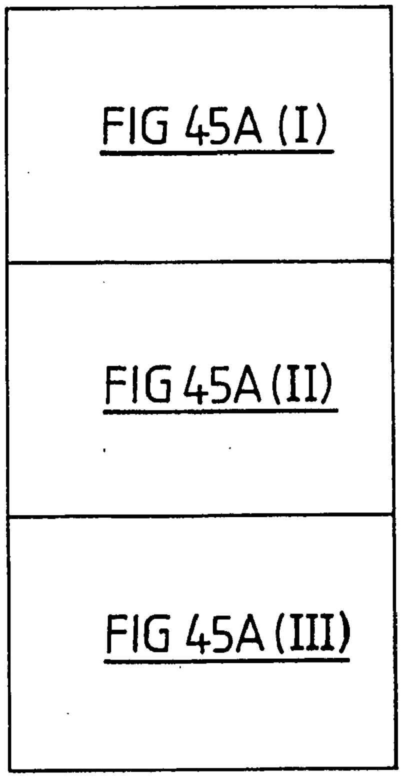


FIG 45A

....AAGTGGCGGCGGTCCCTGGAGAGCAGGCGGAGGCAGCGGCAAGTCTGACTCTGG
GCTGACCGTGGAGCCGGGGCGGGGGCTGACAGCCAGGCCTCCGCCTGGCGGGAGCCGC
ACGAGGAGCGGGAGTGGCCGGGCCTCTCTTCCGCGCTTGAGCGAGCGCCGGGTGATGG
CGGTGGTGATGGCGGCAGGCGCTCGGACAGCTCCGCTTGAGCTGAGCTCGGAGAGATC
CGTCCAGAAAGTGCCCAGAAGAAACTTCCTCTTAGAAAAGCTGAAAAACACARTATTT
ATAACACTGGAAATTGTAAAGAATTTGTTTAAAATGGCTGAAAACAATAGTAAAAATG
TAGATGTACGGCCTAAAACAAGTCGGAGTCGAAGTGCTGACAGGAAGGATGGTTATGT
GTGGAGTGGAAAGAAGTTGTCTTGGTCCAAAAAGAGTGAGAGTTGTTCTGAATCTGAA
GCCATAGGTACTGTTGAGAATGTTGAAATTCCTCTAAGAAGCCAAGAAAGGCAGCTTA
GCTGTTCGTCCATTGAGTTGGACTTAGATCATTCCTGTGGGCATAGATTTTTAGGCCG
ATCCCTTAAACAGAAACTGCAAGATGCGGTGGGGCAGTGTTTTCCAATAAAGAATTGT
AGTGGCCGACACTCTCCAGGGCTTCCATCTAAAAGAAAGATTCATATCAGTGAACTCA
TGTTAGATAAGTGCCCTTTCCCACCTCGCTCAGATTTAGCCTTTAGGTGGCATTTTAT
TAAACGACACACTGTTCCTATGAGTCCCAACTCAGATGAATGGGTGAGTGCAGACCTG
TCTGAGAGGAAACTGAGAGATGCTCAGCTGAAACGAAGAAACACAGAAGATGACATAC
CCTGTTTCTCACATACCAATGGCCAGCCTTGTGTCATAACTGCCAACAGTGCTTCGTG
TACAGGTGGTCACATAACTGGTTCTATGATGAACTTGGTCACAAACAACAGCATAGAA
GACAGTGACATGGATTCAGAGGATGAAATTATAACGCTGTGCACAAGCTCCAGAAAAA

FIG 45A(I)

GGAATAAGCCCAGGTGGGAAATGGAAGAGGAGATCCTGCAGTTGGAGGCACCTCCTAA
GTTCCACACCCAGATCGACTACGTCCACTGCCTTGTTCCAGACCTCCTTCAGATCAGT
AACAATCCGTGCTACTGGGGTGTCATGGACAAATATGCAGCCGAAGCTCTGCTGGAAG
GAAAGCCAGAGGGCACCTTTTTACTTCGAGATTCAGCGCAGGAAGATTATTTATTCTC
TGTTAGTTTTAGACGCTACAGTCGTTCTCTTCATGCTAGAATTGAGCAGTGGAATCAT
AACTTTAGCTTTGATGCCCATGATCCTTGTGTCTTCCATTCTCCTGATATTACTGGGC
TCCTGGAACACTATAAGGACCCCAGTGCCTGTATGTTCTTTGAGCCGCTCTTGTCCAC
TCCCTTAATCCGGACGTTCCCCTTTTCCTTGCAGCATATTTGCAGAACGGTTATTTGT
AATTGTACGACTTACGATGGCATCGATGCCCTTCCCATTCCTTCGCCTATGAAATTGT
ATCTGAAGGAATACCATTATAAATCAAAAGTTAGGTTACTCAGGATTGATGTGCCAGA
GCAGCAGTGAtgcggagaggttagaatgtcgacctgcatacatattttcatttaatat
tttatttttcttatgcctctttgaatttttgtacaaaggcagttgaatcaaataaaac
tgtgccctaagttttaattccagatcaatttatttttttatgatacacttgttatat
atttttaagcaggtgtttggttttgtttttaccatataaatttacatatggtccaggc
atatttacaatttcaaggcattgcatatacatttgaatattctgtatttttaaataa
tcttttgttctttcctatgtgtgaaatattttgctaatctatgctatcagtattcttg
tatgaccgaatagttacctattctcttttcatcttgaagattttcagtaaagagtgtt
gtaatcaatccattataatgtaattgactttttgtaatttgccaataggagtgttaaac

FIG 45A (II)

aacaaaatgatttaaaatgaaacttaatgtattttcattttaaatattaactaaacca
agtttgtttgttagttattctagccaataagaaaagagaatgtagcatcctagaggtg
tatttgttctgcagtttggcaggaccgtcagttagtccaaataaacatcccctcagcg
tggaggcgaatggaacctgtgctcctttcttacgggaagctttgcaaagcaaaatagc
agggttacaagcttggagttgttaaggcaactagagttttctctattaatttatagac
tgttgttgcacctacttagctctttttgggaactctagttcccaggggaaaatacct
cgtgcc

FIG 45A(III)

....SGGGPWRAGGGSGKSDSGLTVEPGRGLTARPPPGGSRTRSGSGRASLPRLSERR
VMAVVMAAGARTAPLELSSERSVQKVPRRNFLLEKLKNTXFITLEIVKNLFKMAENNS
KNVDVRPKTSRSRSADRKDGYVWSGKKLSWSKKSESCSESEAIGTVENVEIPLRSQER
QLSCSSIELDLDHSCGHRFLGRSLKQKLQDAVGQCFPIKNCSGRHSPGLPSKRKIHIS
ELMLDKCPFPPRSDLAFRWHFIKRHTVPMSPNSDEWVSADLSERKLRDAQLKRRNTED
DIPCFSHTNGQPCVITANSASCTGGHITGSMMNLVTNNSIEDSDMDSEDEIITLCTSS
RKRNKPRWEMEEEILQLEAPPKFHTQIDYVHCLVPDLLQISNNPCYWGVMDKYAAEAL
LEGKPEGTFLLRDSAQEDYLFSVSFRRYSRSLHARIEQWNHNFSFDAHDPCVFHSPDI
TGLLEHYKDPSACMFFEPLLSTPLIRTFPFSLQHICRTVICNCTTYDGIDALPIPSPM
KLYLKEYHYKSKVRLLRIDVPEQQ*

FIG 45B

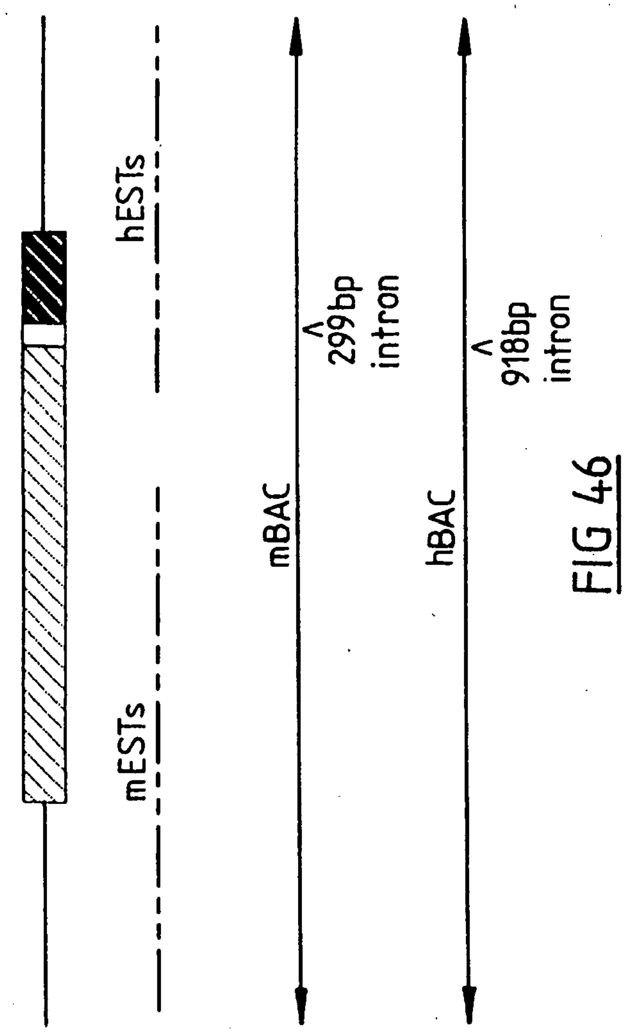


FIG 46

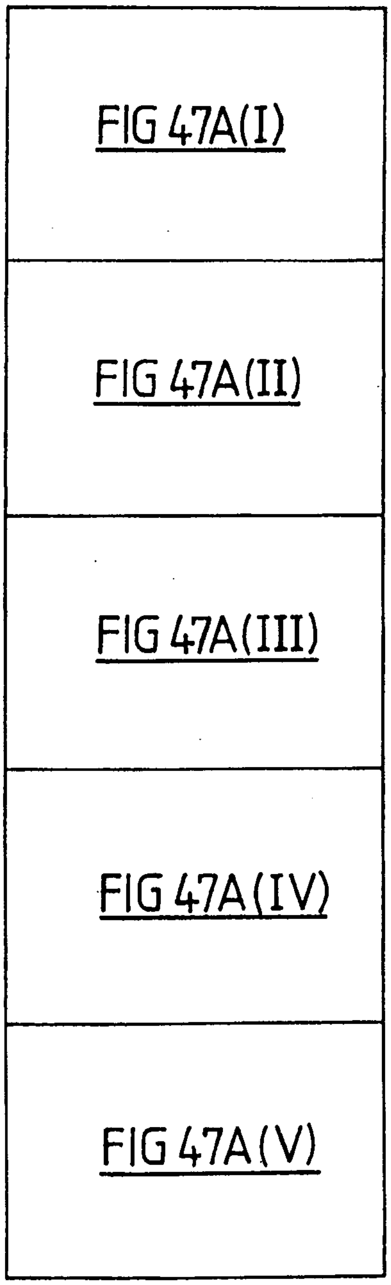


FIG 47A

ccctctgggcaagccgccccccccccacccatctaccacacacacacacacacacacaca
cacacacattcagaccttggggcaaaaacaaagcaaaataacaacaacaaaaacactg
cctgtggaaagtccttacttcaggaaggttggcagatgaggagcaaggaacattta
tcaggactgccacaaaggagtctttttttttaatggttttcaagacagggtttctct
gtatagccctggctgtcctggagctcactttgtagaccaggctggcctcgaactcaga
aattcgcctgcctctgcctcctgagtgctgggattaaaggcgtgcagcaccatgtcca
actggcattttctcaattaaggttcgttcctttcagataactctaggttctgggtcaa
gctgacacaaggctacacagcacagtttgtatgccacattcagttcagaagacaccca
acctccctggaactggaacttatgcacatttgtgagcttccacttgggagtgggaacc
tgaactgggtcctctgcaagagcagccgtgctcttaactgctgagccatttcagcagc
ctcacatcagaattaagttaaaattagccgggtatgaatcatacccttagaatcctag
catctgaaagcagagctaagagaaacagggattcaagaccagctcttggctacagagc
ccgtcctgtcctaggatgggctacaagagactatttcaaagccatccaaacaacaata
actacaacaacaacaaggttaaaattaggctgggcacagggtacacacctttaatgcc
aacactcaggaggcagaggcaggctgatcagtgtgagtttgagttcaacgtggtctac
atagggagttctaggccagcagaggttacagtctctctctctctctctctctctctct
ctctctcacacacacacacacacacacacacacacacacacacacacggtggcatt
atgggatttttttgggataaggtttctctgtctagccctggcatagattcactctgta

FIG 47A(I)

gactaggctagccttgaactcagagatccgcctgcctctgcctcccaagtgctgggat
tataggtgttgcaccaccactgcccagccactttgggattttgaactgttatcaaga
ggctttcgaggaggtcaaacttcaacagcaacctctccatgataatgtagctaatgatc
aaacgacactcaaaacttaacccttaaagcacacatccaccagacagcgtgcccactc
gtagttccattactcaggaggctgaagcaggaggatgaaggactaaggcttcagcaac
ctagggagccgcaggggacagtagtctcaatccctacattctcctgaacacaggagca
ggagttcaggaagggtgtcaaggccgcttactgatcttagggcctcaggaatgactag
ctcaggcagagagagcaaaggtctccagtggagaagtctacacacacacacacacaca
cacacacacacacacacacagaatccaaggcgatgacgtcatcaaagggttaattc
tagtctgggatgggggggagggtggggcacgcagctgtcaggtggctttggaaaaata
aactgctgaagagtctgacgccagggagtcctgggagggacaagaggttacccactca
aagagtgtgctccacaaagcatgcgcgcttgtccacgtctggagtcgtcacttatttt
ttgcctggattctttgtagccggtgggttctcaaggcggtaagtggtgtggccgccgt
ggtctgggaggtgacgatagggttaatcgtccacagagcccaggggcggagcgcgggc
gggcgtccgcagccccgctggagccggaagcagtggctggtcaggggcgcttctagcc
ttccctatctgtacttccacagaggtctctgcgagctaggggacagtgaggtgcggg
gtaggggcccggcgttagagccagcaaggggacggttcacggtaaggtctgagggaga
gagagctcctgagaaacttgggggcgcgacacagatagggtgaaagcagagtgatag

FIG 47A(II)

acctgggatggttaggggaccaagggaagaccaggctggttggcatacaccggtgaac
ggatgggagtcctagggaaagatgatgcgcctaacagtcctttctgtctccacaccac
tccaggggacgatccggagctcaactttcaaaagcgagacgccccagcaagcctgttt
tgagaagttcttcagcggctctcctcATGGCCAGACGGCCCTGGCAAGGGGCAGCAG
CAGCACCCCTACCTCGCAGGCTCTGTACTCGGACTTCTCTCCTCCCGAGGGCTTGGAG
GAGCTCCTGTCTGCTCCCCCTCCTGACCTGGTTGCCCAACGGCACCACGGCTGGAACC
CCAAGGATTGCTCCGAGAACATCGATGTCAAGGAAGGGGGTCTGTGCTTTGAGCGGCG
CCCTGTGGCCCAGAGCACTGATGGAGTCCGGGGGAAACGGGGCTATTCGAGAGGTCTG
CACGCCTGGGAGATCAGCTGGCCCCTGGAGCAAAGGGGCACACACGCCGTGGTGGGCG
TGGCCACCGCCCTCGCCCCGCTGCAGGCTGACCACTATGCGGCGCTTTTGGGCAGCAA
CAGCGAGTCCTGGGGCTGGGATATTGGGCGGGGAAAATTGTATCATCAGAGTAAGGGC
CTCGAGGCCCCCCAGTATCCAGCTGGACCTCAGGGTGAGCAGCTAGTGGTGCCAGAGA
GACTGCTGGTGGTTCTGGACATGGAGGAGGGGACTCTTGGCTACTCTATTGGGGGCAC
GTACCTGGGACCAGCCTTCCGTGGACTGAAGGGGAGGACCCTCTATCCCTCTGTAAGT
GCTGTTTGGGGCCAGTGCCAGGTCCGCATCCGCTACATGGGCGAAAGAAGAGgtgaga
tacggactaggtgtggggagatcactactcttggcaatggtttgggctggaaactcat
ggttggagcacaggaagtaggcttcttgtcactttggcctgtcacttagatggccttg
gatctagcttcactcccaatccctattggatgtgatgcacaaattcagagcctttggg

FIG 47A(III)

tctccctcagctgaggtggcggtggaaatggaggaagaaggaagggtgcctgagcagg
atctcaagttcaaggatgcctggagttgcttacttaccttgtcttccttctctctccg
cagTGGAGGAACCACAATCCCTTCTGCACCTGAGCCGCCTGTGTGTGCGCCATGCTCT
GGGGGACACCCGGCTGGGTCAAATATCCACTCTGCCTTTGCCCCCTGCCATGAAGCGC
TATCTGCTCTACAAATGAcccagtagtacagggtgtgctggcaccctaccgtggggac
aggtggagaggcacccgctggcctagacaactttaaaagctggtgaagctgggggg
gggggctggaccccttcacctccccttctcacaggagcaagacatatagaaatgatat
taaacaccatggcagcctgggacaaagaggttttgaagtaaaaaatgagatgtattg
tcacaacctgtttcattattgtttttgttttgttttacactcccccaccccaggcta
gagccccatcactgtcttaaggaattatgacaacccacaaagctcaggcccaggtgtt
tatttcccttacatgtaggatggttcacaaacacaatacaggggctttggcaccgtgg
gggaggggactatcccaggcctcttagggtctcatgtataccgaattcagacccgaaa
gctctgaatttctgcatcagacatccagtagaacttgggagtgaagctagagccaagg
ccatctaagtgacaggccaaagtgacacgaagcccacttcctgtgctccaaccatgag
tttccagcccaaaccaatggaaggtgatttcacttgtcagggcccaaagggacagtca
gttctactccctcccctcactaggagccaccttggtgacagttgattctacccactgt
aagtggtaaagggattggcctggtcccaaccataatagggcggtggaaacggctcagg
agggtacagcgtggattaggccacaagatggggcagatgatgtcatcagaagcatgtg

FIG 47A(IV)

accggtgggagcagttactaaacttctgggcaacctagtccatgctatgcaggcaggt
agagggatgggcagtgctcattgtttggcattgatgatgtccacaaattcaggcttga
gagatgcgccacccacaaggaagccgtccacgtcaggctggcttgccagctctttgca
ggttgctccagtcacagaacctgtaccaggaacaagaagacagtttggtcaggtctat
gatcagaacacttaagccccacctctctgtgcaaggcagcctcagtctgtcttagccc
atttccgtcttagctagagccaaagccactcacctccataaatgatccgggtgctctg
agccaccccatcattgacattggatttcagccatccccggagcttctcgtgtacttcc
tgtgcctagaaggaggaggcagagctactaagtaagctccttcctatctatcattcaa
ggagtaaaaaccactggttctcacatagagttgagtttccagaaaagccccgggacca
gagagtggcaaggctccaatcccaccaggcttggaatgaacattttggcaaagtcac
tctccttggtgagtttgggggccctctgtctctaaaggggcttggatgggctccatag
ctgtgtgagtctgttaaagccggacaggctgaggagctctgggtagttacctgctgag
gggttgccgtcttgccagtcccaatggcccacacaggttcataggccaggaccacctt
gctccagtctttcacattatctgtggggcagagaggagagtgagtaggaaggagctga
cccgccaagc

FIG 47A(V)

MGQTALARGSSSTPTSQALYSDFSPPEGLEELLSAPPPDLVAQRHHGWNPKDCSENID
VKEGGLCFERRPVAQSTDGVRGKRGYSRGLHAWEISWPLEQRGTHAVVGVATALAPLQ
ADHYAALLGSNSESWGWDIGRGKLYHQSKGLEAPQYPAGPQGEQLVVPERLLVVLDME
EGTLGYSIGGTYLGPAFRGLKGRTLYPSVSAVWGQCQVRIRYMGERRVEEPQSLLHLS
RLCVRHALGDTRLGQISTLPLPPAMKRYLLYK

FIG 47B

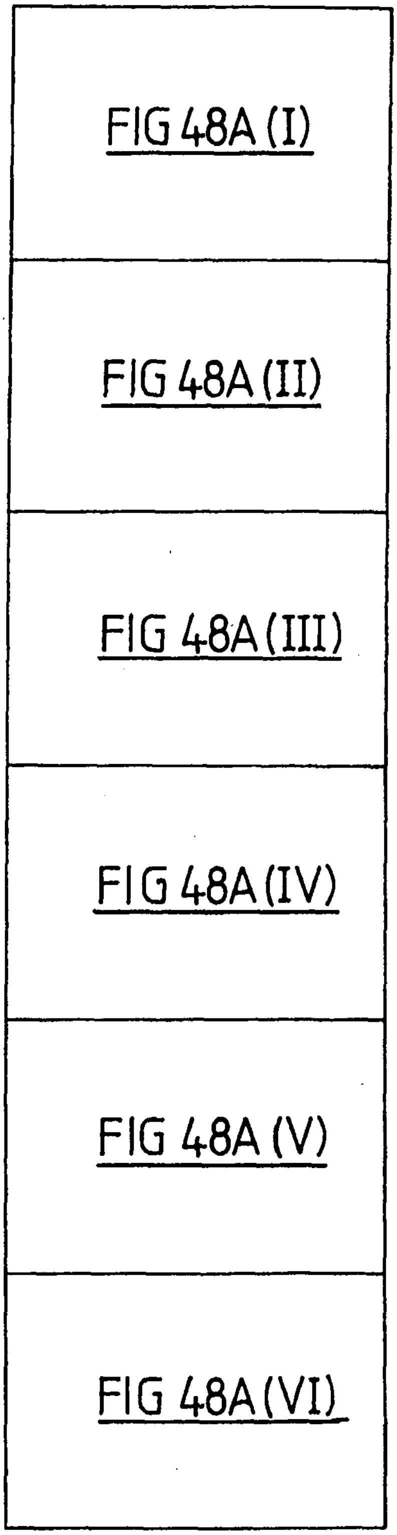


FIG 48A

gtactttctttatatctccataattttatttactattactacatgatacattatttta
taaaagtctttgtaacctccttaaggattcactgcttaatctccagtgcttagcacaa
atcattaaatgcgaaccagaaactcttccaaatgtgttacatctataacctcattgga
ttctcactaccaaccccatgcaatagatactaatgtgatctctgtcttacagaggaag
aaacaggcacagggaggttcagtaatttgcccaaggtcatacacacactggccttcag
gtattcatgcccggggagtctggtcccacagctggcatgtttgccattatattatatt
gcctccttatagtgtcggcactcattaagcacattgacagctatgcttggtgagtgac
tactatgtacccagctctgtgctacatgctttacctggattatttcaactgcacaaca
accctgtgaggtaactaccatcattgctcctatttacataacagaaaactacagaaa
tctggggctgggcgtagtggctcatgcctgaaatcccagcactttgggagaccctgtc
tctaaaaaaatttttttttggccggacgtggtggctcacacctgtaatctcagcact
ttgggaggctaaggcaggcagatcacaaggtcaggagttctagaccagcctggccaac
atggcaaaaccctgtgtctactaaaaatacaaaaaatagctaggcgtggtggcaggtg
cctgtaatcccagctactcaggaggctgaggcaggagaatcccctgaacctgggagat
ggaggttacagagagccgagatcgtgccgctgcactccagcctgggcaacaagagcaa
gactctgtctcgaaaaaaataaaaataaaaataaaaatatttttttaaaaattagctg
ggtgtggtagcacatgcctgtagtcccagctacttgggaggctgaggtaggaggatca
cttgagcccaggaggtcaaggctgcagtgggctgtgatggcgccactgcactctagcc

FIG 48A(I)

ttggtgacagcaagaccctgtctcaaaaaaaaaaaaaaaagagaaatcgggcaacttccc
caagatcgcgcagttaactagtggcatagcttcactcaaactcgaagtcttaatcagg
acactctaccaaatgagatcaacggctcagtaatggattggcatccagtatgaagact
ggaccagcagggagaactatgatgcgtacagcctagagcctgaagcagatttcacagc
ctcagaggtggcacaggctgactcacaacccggggcagaaaggaccagcccagaaac
agtgacccagaatcacagggaagtagaaatgggattcggcacaatgaagcccctcctt
gaccccatgctccttaccctcaggggcgcaggagttagtcgctcaggcggctcaaagg
tcttgacggtggagaacaccatccccagggattcccgacgcggtgatgccatcaaagc
gttaattctgagatgggcctgcccgggtgcggactctgccgcagcaagagaagggtta
actgccccgggccttcgccgtgggggcggggcctcggggagggtcacagcccgggact
gagacccgaggttaaccgcccggggtgggctccacgggggcggggcatgctctccgcg
gctgctgccggtatagagcggtaactgcccaggagggggcggggccccacaggggcgt
ggcctcggagctgcacggccgtgggcggcgatgagagggttaagccccagagggccct
ggagggggcggggccgcgggacgggctcggcccaaggaggagctgggggcggaagcgg
ccggcggtctgcgccctgcgcgcctcggcttctttccgcccggctccttcagaggccc
ggcgacctccagggctgggaagtcaaccgaggttcgggggcagcggcgagggctccgg
gcgagtaaggggggatggtccatgctgaggcccaaatggggcgaactcgcgagagtctc
tggcgacctggatcagatggggcgagggcagatgaagggcccaggagctttggggcag

FIG 48A(II)

cgaggagggaggagcgggcccgttggcaaacttgggtgaaaggatggggtacctgggt
gacgagcccccgccaggattctgctcttcacgcccttttctcccagctcccttccag
gtcaatccaaactggagctcaactttcagaagagaaagacgccccagcaagcctcttt
cggggagtcctctagctcctcacctccATGGCCAGACAGCTCTGGCAGGGGGCAGCA
GCAGCACCCCCACGCCACAGGCCCTGTACCCTGACCTCTCCTGTCCCGAGGGCTTGGA
AGAGCTGCTGTCTGCACCCCCTCCTGACCTGGGGGCCCAGCGGCGCCACGGTTGGAAC
CCCAAAGACTGTTCAGAGAACATCGAGGTCAAGGAAGGAGGGTTGTACTTTGAGCGGC
GGCCCGTGGCCCAGAGCACTGATGGGGCCCGGGGTAAGAGGGGCTATTCAAGGGGCCT
GCACGCCTGGGAGATCAGCTGGCCCCTAGAGCAGAGGGGCACGCATGCCGTGGTGGGC
GTGGCCACGGCCCTCGCCCCGCTGCAGACTGACCACTACGCGGCGCTGCTGGGCAGCA
ACAGCGAGTCGTGGGGCTGGGACATCGGGCGGGGGAAGCTGTACCATCAGAGCAAGGG
GCCCGGAGCCCCCCAGTATCCAGCGGGAACTCAGGGTGAGCAGCTGGAGGTGCCAGAG
AGACTGCTGGTGGTTCTGGACATGGAGGAGGGAACTCTGGGCTACGCTATTGGGGGCA
CCTACCTGGGGCCAGCATTCCGCGGACTGAAGGGCAGGACCCTCTATCCGGCAGTAAG
CGCTGTCTGGGGCCAGTGCCAGGTCCGCATCCGCTACCTGGGCGAAAGGAGAGgtgag
gcctggggcagacgtggggagaactttctgtccctggtggcagtggtttgggatggaa
actcttctgacaagagcagaggggatggaccttcatccagcctgcctcaacctctgtt
cagtgctgggaaaggctaggggtcttcacagctgttatttaatttaacccaacagcaa

FIG 48A(III)

tagaggtgaaacaggcttgagaaagcaactttctcaagttctcttggccagtaaatgg
tgaaccttcagaatggagggaggaactgcagggatgagagaattcaggagatatcaac
ccctgagcaagaggtgcaaagcgttaggtactgggtttgatgtacaggtccaaaagaa
ggatgggcagagccaggtacccaggctgtataccggattccctgggctctaacctgtc
tctgtgccacatacctacttccttcctcagccacacctctggatggagacactggggc
cctgggcaccagggaggagagcagtggaggaggcagggccttagggtggggcagcagg
ggaggagcctccccaggaactgactgggtccagggcttggagctgctctctgcagttg
tgtgggctgtagagtggagggccatccctcctcacctcagccccagctcccaagcctc
tggagtcaaagcctgggccagctccaccactgtcagagccaccttggcctgttgttta
gagggccttagccagctcttcacccccagctctgactagggatgtgtgaaatcttatc
tgggaggcagaacttccgggtatctcaaattcccctttcagccaggtgggcacactcg
aagcaggaaagcagaaaggcatctgagtaggaccccgtagtttgaggacatctggctg
gtggctgcacccatacttacattcccctccttctctcccagCGGAGCCACACTCCC
TTCTGCACCTGAGCCGCCTGTGTGTGCGCCACAACCTGGGGGATACCCGGCTCGGCCA
GGTGTCTGCCCTGCCCTTGCCCCCTGCCATGAAGCGCTACCTGCTCTACCAGTGAgcc
ctgtgataccacagactgtgctgaggtcttgccaccacccctccccttggggaggtgg
ggaggcactgctggcctagaccagctgctgaaagctggtgaggctgagcccctacccc
aacccaagctctgcggaaatcaacagccccagagccacttggaggaggaagaaaggg

FIG 48A(IV)

agccggcgttcaaggctatgacagtctgctacgcaaaacattttttcaagtaaaata
gtaagagatgttgttatagaaacctgttcttgttttttttttttttcttgcacaaatga
tcatttatatagctgcctcaaaaaggaagattatctgggcaagtccagtgaaggcaga
caaaccacaagacctagtgccaggtttattccctcacatgggtggttcacatacacag
cacagaggcacgggcaccatgggagagggcagcactcctgccttctgaggggatcttg
gcctcacggtgtaagaagggagaggatggtttctcttctgccctcactagggcctagg
gaacccaggagcaaatcccaccacgccttccatctctcagccaaggagaagccacctt
ggtgacgtttagttccaaccattatagtaagtggagaagggattggcctggtcccaac
cattacagggtgaagatataaacagtaaaggaagatacagtttggatgaggccacagg
aaggagcagatgacaccatcagaagcatatgcagggaaagggcagttactgggcttct
gggctgcttagtccctggcttggcaggaagggtagggaagatggatggggctcattgt
ttggcattgatgatgtccacgaattcgggcttgagggaagcaccacccacaaggaagc
catccacatcaggctggctggccagctccttgcaggttgccccagtcacagagcctgg
gaagggagcagaacaagggcttggtcaagaatgggatgagtctgccccatccccacct
ccatgtccgagggctcagtctagtcctcagcccactccacctcagccgggaaccaaag
ccactcacctccataaatgatacgggtgctctgagccaccgcatcagagacgttggac
ttcagccatcctcggagcttctcgtgtacttcctgggcctagaacaagaagctggcct
aagtaagaccttttctgcctctaagaggaaaatcactggcaccagtggacactta

FIG 48A(V)

gtgtggtttctgactgagtcagagtaccagggctctgatccaagccaggccctggact
ggatgcccttggacaagtcactgtctctgggttcaaggtctctgtgtctttgaaataa
ggggttgccccatgtgggctgtgtctgtccaaacctattgaggcaggctgggatgagg
gcagggctcctgggcccggttacctgttggggtgttgcagtcttgccagtaccaatgg
cccacacaggctcataggccaggacgaccttgctccagtccttcacgttatctgcagg
gcagagatacagatggagggaagggtgaacaagaaagagctctccagccaggttctcc
ggagtacgaagaacggtggcctactgccccctagtggacattggggg

FIG 48A(VI)

MGQTALAGGSSSTPTPQALYPDLSCPEGLEELLSAPPPDLGAQRRHGWNPKDCSENIE
VKEGGLYFERRPVAQSTDGARGKRGYSRGLHAWEISWPLEQRGTHAVVGVATALAPLQ
TDHYAALLGSNSESWGWDIGRGKLYHQSKGPGAPQYPAGTQGEQLEVPERLLVVLDME
EGTLGYAIGGTYLGPAFRGLKGRTLYPAVSAVWGQCQVRIRYLGERRAEPHSLLHLSR
LCVRHNLGDTRLGQVSALPLPPAMKRYLLYQ

FIG 48B

FIG 49

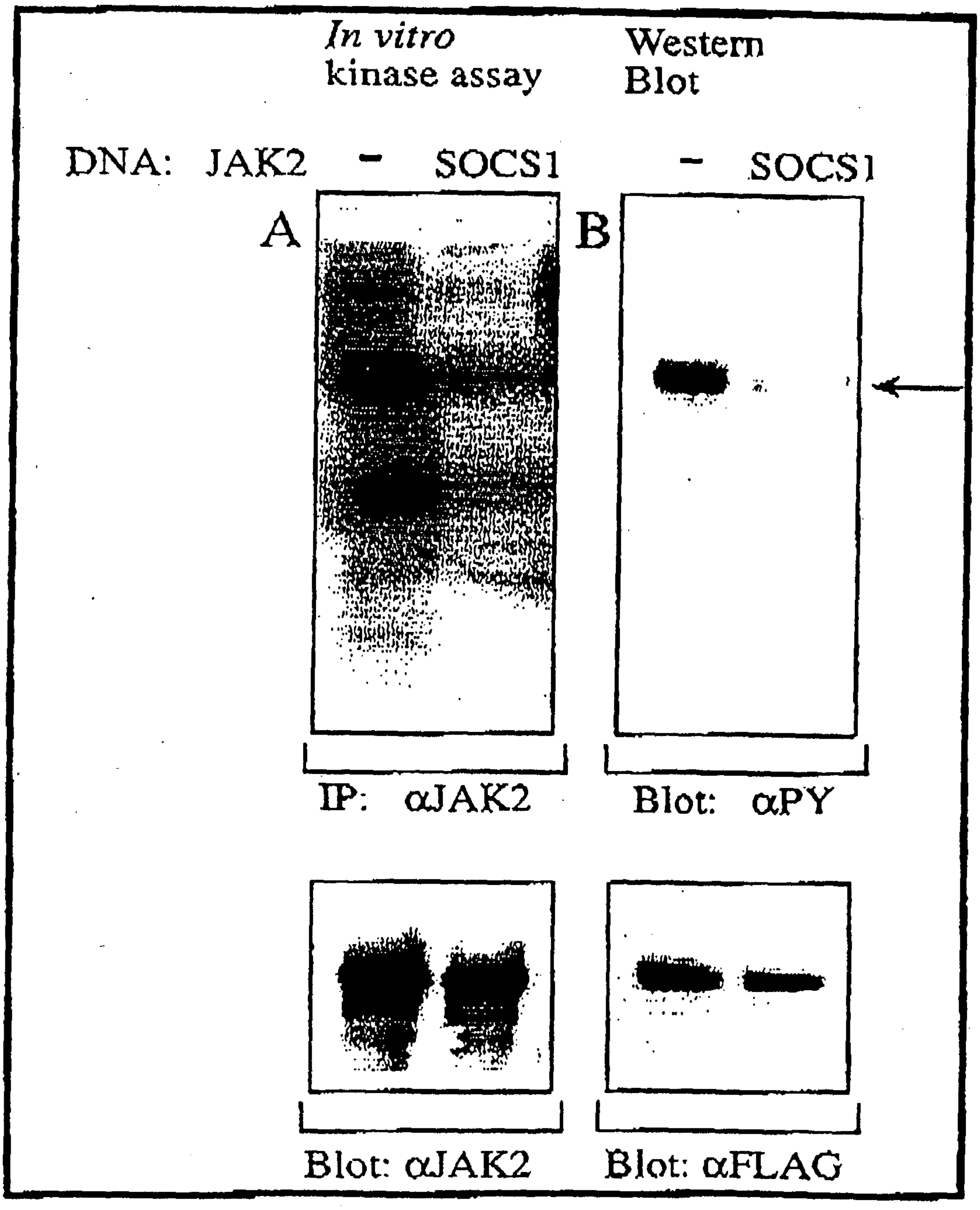
In vitro
kinase assay
Western
Blot
DNA: JAK2 - SOCS1 - SOCS1
A
B
IP: αJAK2
Blot: αPY
Blot: αJAK2
Blot: αFLAG

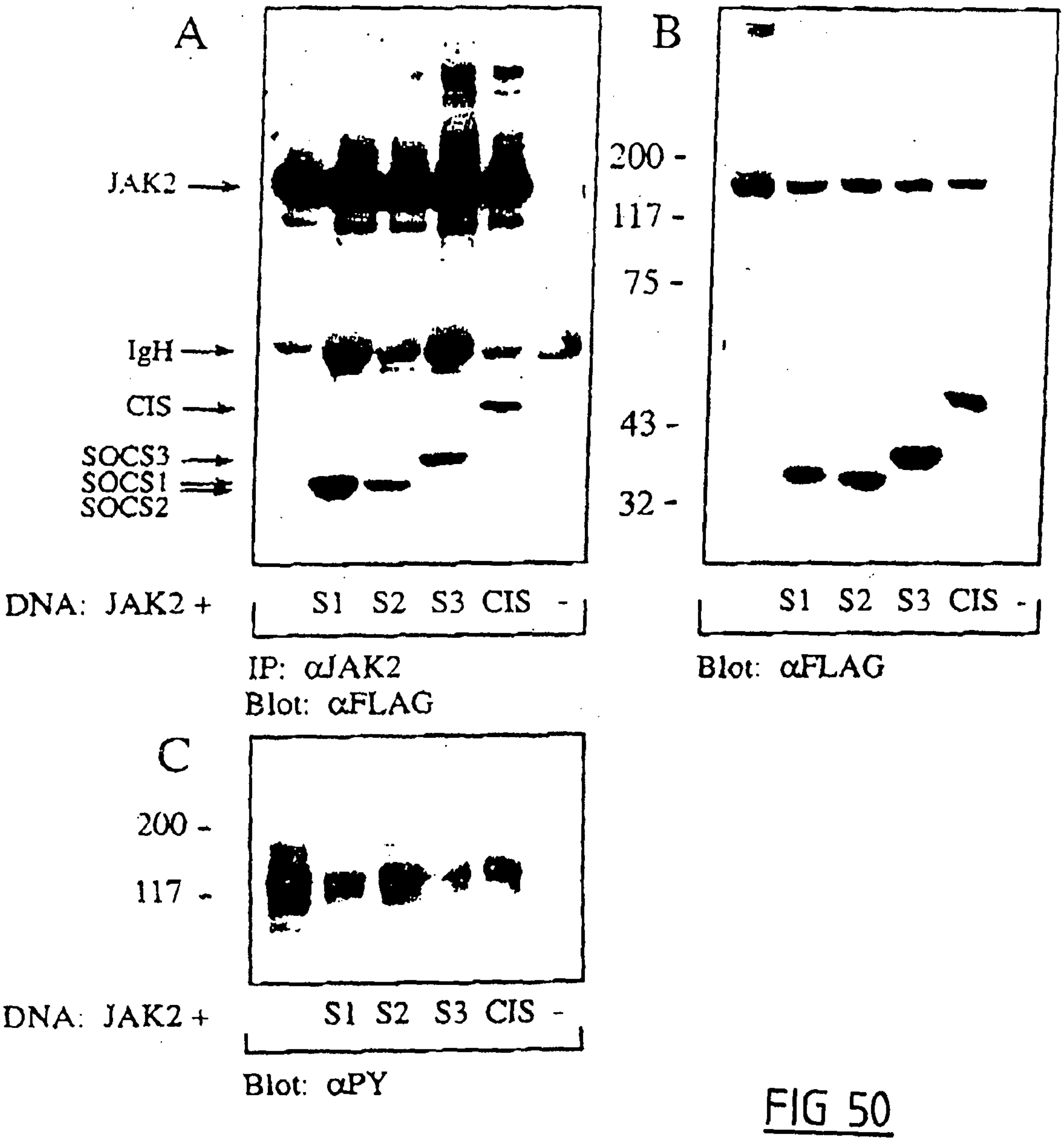
A
B
JAK2
IgH
CIS
SOCS3
SOCS1
SOCS2
200 -
117 -
75 -
43 -
32 -
DNA: JAK2 + S1 S2 S3 CIS -
IP: αJAK2
Blot: αFLAG
S1 S2 S3 CIS -
Blot: αFLAG
C
200 -
117 -
DNA: JAK2 + S1 S2 S3 CIS -
Blot: αPY

FIG 50

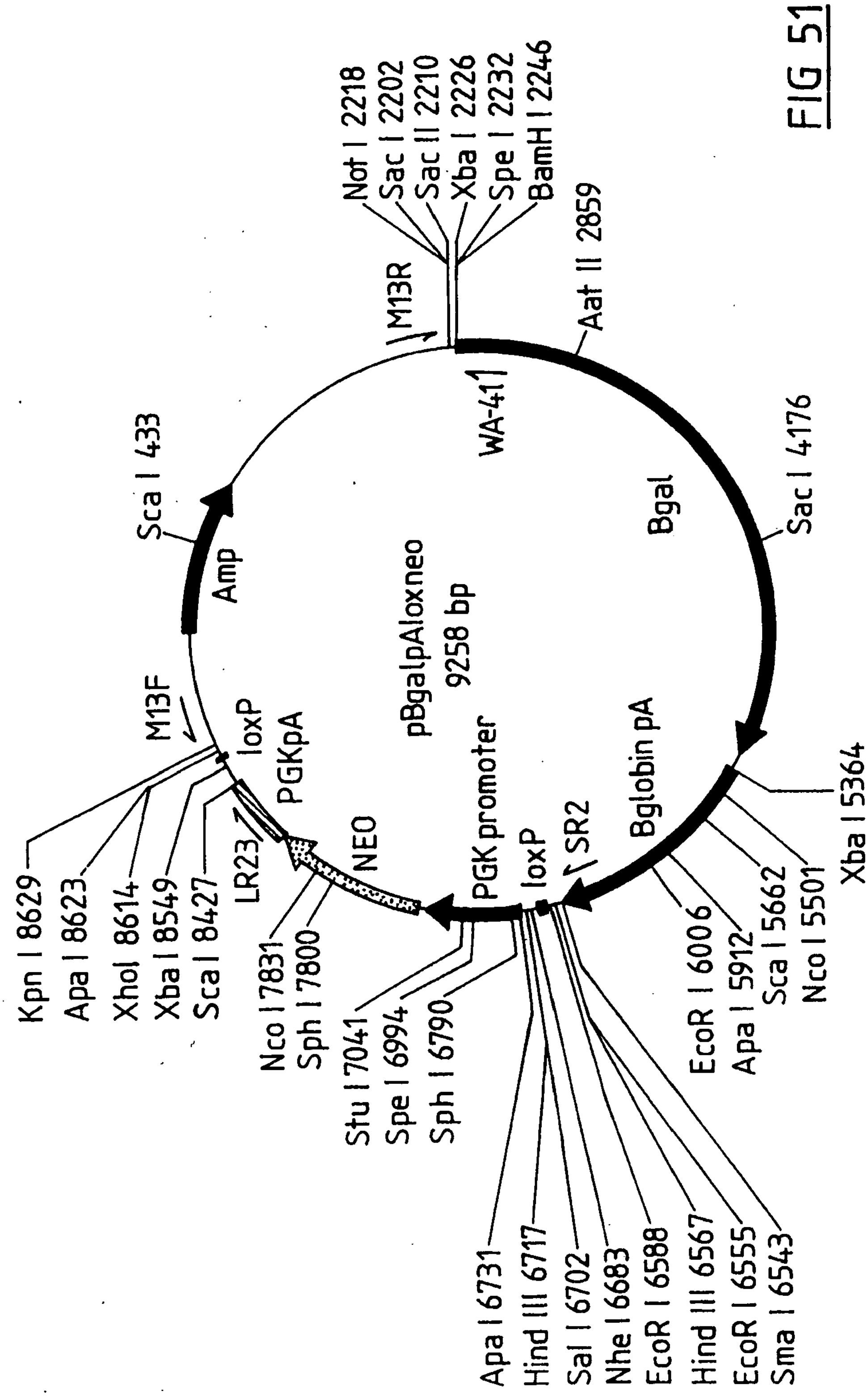
pBgalpAloxneo
9258 bp
Not I 2218
Sac I 2202
Sac II 2210
Xba I 2226
Spe I 2232
BamH I 2246
M13R
Aat II 2859
WA-41
Sac I 4176
Bgal
Xba I 5364
Bglobin pA
Nco I 5501
Sca I 5662
Apa I 5912
EcoR I 6006
SR2
loxP
Sma I 6543
EcoR I 6555
Hind III 6567
EcoR I 6588
Nhe I 6683
Sal I 6702
Hind III 6717
Apa I 6731
PGK promoter
Sph I 6790
Spe I 6994
Stu I 7041
NEO
Sph I 7800
Nco I 7831
LR23
PGKpA
loxP
Sca I 8427
Xba I 8549
XhoI 8614
Apa I 8623
Kpn I 8629
M13F
Amp
Sca I 433

FIG 51

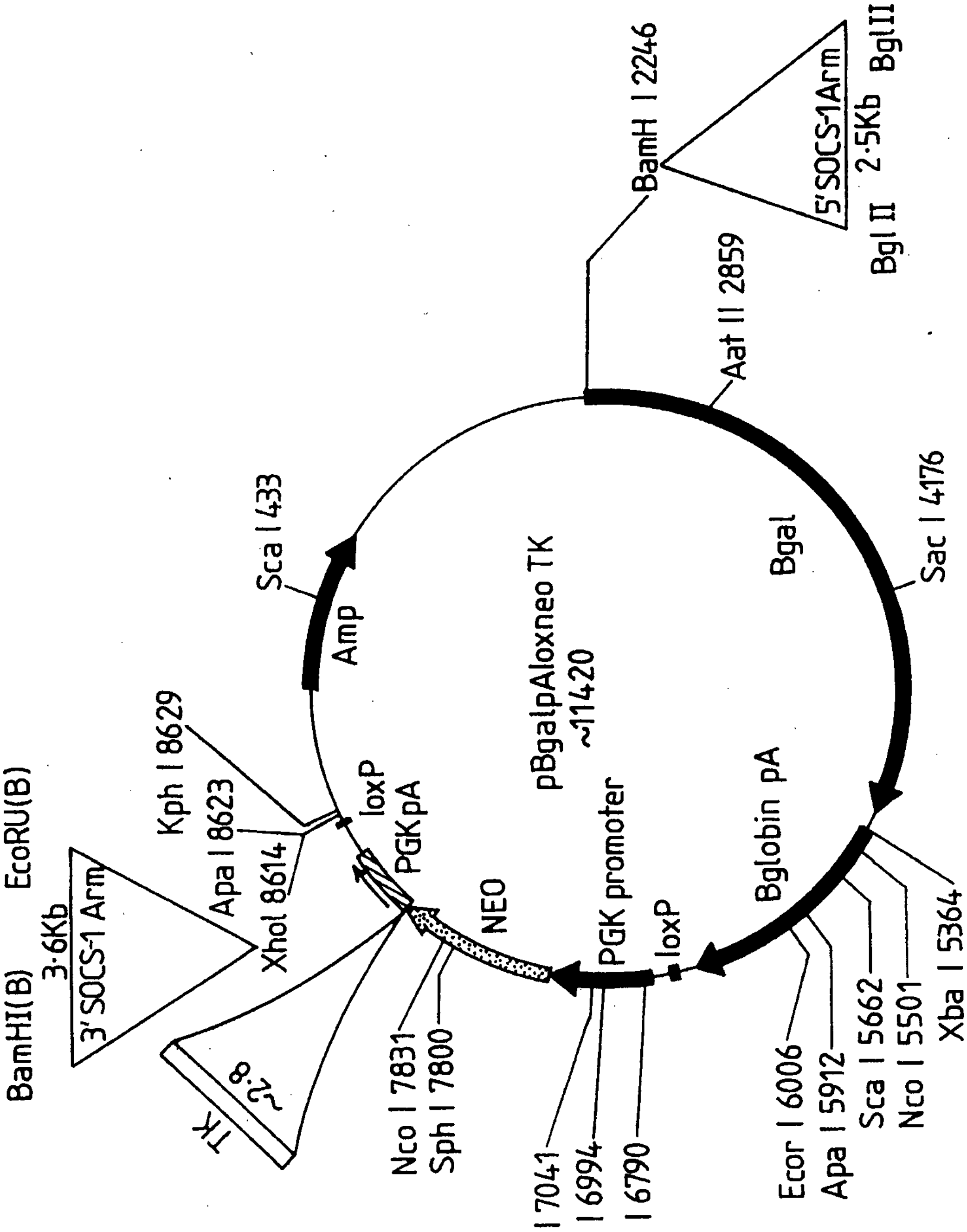


5'x3' SOCS-1 Arms in pBgalpAloxNeoTK

FIG 52

FIG 53

SOCS-1 Knockout Construct

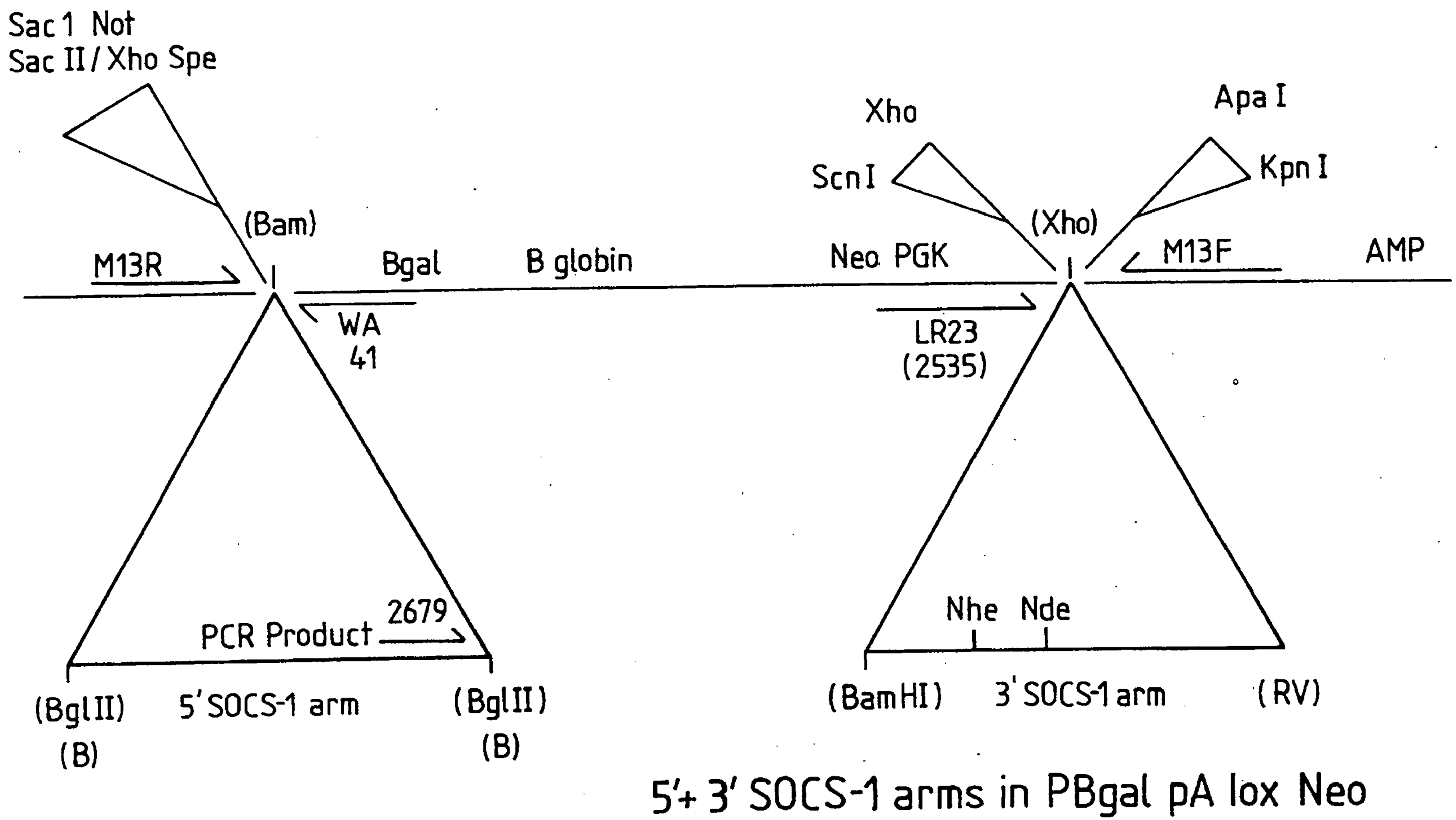


5'+ 3' SOCS-1 arms in PBgal pA lox Neo

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4016043 A
- US 4424279 A
- US 4018653 A

### Non-patent literature cited in the description


- **ALEXANDER WS ; METCALF D ; DUNN AR.** *Embo Journal,* 1995, vol. 14, 5569-78
- **ALTSCHUL, S.F. GISH ; W. MILLER ; W. MYERS ; E.W. & LIPMAN, D.J.** *J. Mol. Biol.,* 1990, vol. 215, 403-10
- **ANSARI-LARI, M.A. ; SHEN, Y. ; MUNZY, D.M. ; LEE, W. ; GIBBS, R.A.** *Genome. Res.,* 1997, vol. 7, 268-280
- **BAZAN JF.** *Immunology Today,* 1990, vol. 11, 350-4
- **BORK, P.** *Proteins: Struct. Funct. Genet.,* 1993, vol. 17, 363-374
- **COCKWELL, L.Y. ; GILES, I.G.** *Comp. Appl. Biosci.,* 1989, vol. 5, 227-232
- **CUTLER RL ; LIU L ; DAMEN JE ; KRYSTAL G.** *Journal of Biological Chemistry,* 1993, vol. 268, 21463-5
- **DARNELL J JR. ; KERR IM ; STARK GR.** *Science,* 1994, vol. 264, 1415-21
- **DAVID M ; PETRICOIN E3 ; BENJAMIN C ; PINE R ; WEBER MJ ; LARNER AC.** *Science,* 1995, vol. 269, 1721-3
- **DAVID M ; WONG L ; FLAVELL R ; THOMPSON SA ; WELLS A ; LARNER AC ; JOHNSON GR.** *Journal of Biological Chemistry,* 1996, vol. 271, 9185-8
- **DUGAICZYK A ; HARON JA ; STONE EM ; DENNISON OE ; ROTHBLUM KN ; SCHWARTZ RJ.** *Biochemistry,* 1983, vol. 22, 1605-13
- **DURBIN JE ; HACKENMILLER R ; SIMON MC ; LEVY DE.** *Cell,* 1996, vol. 84, 443-50
- **ETZOLD, T. ; ULYANOV, A. ; ARGOS, P.** *Methods Enzymol.,* 1996, vol. 266, 114-28
- **GEARING DP ; NICOLA NA ; METCALF D ; FOOTE S ; WILLSON TA ; GOUGH NM ; WILLIAMS L.** *BioTechnology,* 1989, vol. 7, 1157-1161
- **GUPTA S ; YAN H ; WONG LH ; RALPH S ; KROLEWSKI J ; SCHINDLER C.** *Embo Journal,* 1996, vol. 15, 1075-84
- An introduction to cytokine receptors. **HILTON DJ.** Guidebook to Cytokines and Their Receptors. Oxford University Press, 1994, 8-16
- **HILTON DJ ; HILTON AA ; RAICEVIC A ; RAKAR S ; HARRISON-SMITH M ; GOUGH NM ; BEGLEY CG ; METCALF D ; NICOLA NA ; WILLSON TA.** *Embo Journal,* 1994, vol. 13, 4765-75
- **HILTON DJ ; WATOWICH SS ; KATZ L ; LODISH HF.** *J. Biol. Chem.,* 1996, vol. 271, 4699-4708
- **ICHIKAWA Y.** *Journal of Cellular Physiology,* 1969, vol. 74, 223-34
- **IHLE JN.** *Nature,* 1995, vol. 377, 591-4
- **IHLE JN ; WITTHUHN BA ; QUELLE FW ; YAMAMOTO K ; SILVENNOINEN O.** *Annual Review of Immunology,* 1995, vol. 13, 369-98
- **KAPLAN MH ; SCHINDLER U ; SMILEY ST ; GRUSBY MJ.** *Immunity,* 1996, vol. 4, 313-9
- **KAPLAN MH ; SUN YL ; HOEY T ; GRUSBY MJ.** *Nature,* 1996, vol. 382, 174-179
- **LEVY DE ; STARK GR.** *Molecular & Cellular Biology,* 1996, vol. 16, 369-75
- **METCALF D ; WILSON TA ; HILTON DJ ; DIRAGO L ; MIFSUD S.** *Leukaemia,* 1995, vol. 9, 1556-1564
- **MERAZ MA ; WHITE JM ; SHEEHAN KC ; BACH EA ; RODIG SJ ; DIGHE AS ; KAPLAN DH ; RILEY JK ; GREENLUND AC ; CAMPBELL D.** *Cell,* 1996, vol. 84, 431-42
- **MIZUSHIMA S ; NAGATA S.** *Nucleic Acids Research,* 1990, vol. 18, 5322
- **MURAKAMI M ; NARAZAKI M ; HIBI M ; YAWATA H ; YASUKAWA K ; HAMAGUCHI M ; TAGA T ; KISHIMOTO T.** *Proc. Natl. Acad. Sci. USA,* 1991, vol. 88, 11349-11353
- **NEER, E.J. ; SCHMIDT, C.J. ; NAMBUDRIPAD, R. ; SMITH, T.F.** *Nature,* 1994, vol. 371, 297-300
- **NICOLA NA.** Guidebook to Cytokines and Their Receptors. Oxford University Press, 1994
- **NICOLA, NV ; VINEY E ; HILTON DJ ; ROBERTS B ; WILSON T.** *Growth Factors,* 1996, vol. 13, 141-149
- **NOVAK U ; HARPUR AG ; PARADISO L ; KANAGASUNDARAM V ; JAWOROWSKI A ; WILKS AF ; HAMILTON JA.** *Blood,* 1995, vol. 86, 2948-56

- **PEARSON WR ; LIPMAN DJ.** *Proc. Natl. Acad. Sci. USA,* 1988, vol. 85, 2444-8
- **PEARSON WR.** *Methods Enzymol.,* 1990, vol. 183, 63-98
- **RAYNER JR ; GONDA TJ.** *Molecular & Cellular Biology,* 1994, vol. 14, 880-7
- **SAMBROOK J ; FRITSCH EF ; MANIATIS T.** Molecular Cloning, A Laboratory Manual. Cold Spring Harbour Laboratory Press, Cold Spring Harbour, 1989
- **SATO N ; SAKAMAKI K ; TERADA N ; ARAI K ; MIYAJIMA A.** *Embo Journal,* 1993, vol. 12, 4181-9
- **SHIMODA K ; VAN DEURSEN J ; SANGSTER MY ; SARAWAR SR ; CARSON RT ; TRIPP RA ; CHU C ; QUELLE FW ; NOSAKA T ; VIGNALI DA.** *Nature,* 1996, vol. 380, 630-3
- **SHUAL K ; ZIEMIECKI A ; WILKS AF ; HARPUR AG ; SADOWSKI HB ; GILMAN MZ ; DARNELL JE.** *Nature,* 1993, vol. 366, 580-3
- **SPRANG SR ; BAZAN JF.** *Curr. Opin. Structural Biol.,* 1993, vol. 3, 815-827
- **TAKEDA K ; TANAKA T ; SHI W ; MATSUMOTO M ; MINAMI M ; KASHIWAMURA S ; NAKANISHI K ; YOSHIDA N ; KISHIMOTO T ; AKIRA S.** *Nature,* 1996, vol. 380, 627-30
- **THIERFELDER WE ; VANDEURSEN JM ; YAMAMOTO K ; TRIPP RA ; SARAWAR SR ; CARSON RT ; SANGSTER MY ; VIGNALI DDA ; DOHERTY PC ; GROSVELD GC.** *Nature,* 1996, vol. 382, 171-174
- **WAKAO H ; GOUILLEUX F ; GRONER B.** *Embo Journal,* 1994, vol. 13, 2182-91
- **WEN Z ; ZHONG Z ; DARNELL J JR.** *Cell,* 1995, vol. 82, 241-50
- **YI T ; MUI AL ; KRYSTAL G ; IHLE JN.** *Molecular & Cellular Biology,* 1993, vol. 13, 7577-86
- **YOSHIMURA A ; OHKUBO T ; KIGUCHI T ; JENKINS NA ; GILBERT DJ ; COPELAND NG ; HARA T ; MIYAJIMA A.** *Embo Journal,* 1995, vol. 14, 2816-26